(19) Europäisches Patentamt
European Patent Office
Office européen des brevets

(11) Publication number: 0 454 444 A1

# (12) EUROPEAN PATENT APPLICATION

(21) Application number: 91303698.4

(22) Date of filing: 24.04.91

(51) Int. Cl.[5]: C07D 211/88, C07D 221/20, C07D 265/36, C07D 271/107, C07D 273/02, C07D 401/10, C07D 413/04, C07D 413/10, C07C 233/15, C07C 233/13, C07C 233/43

(30) Priority: 24.04.90 JP 108236/90
27.03.91 JP 89986/91

(43) Date of publication of application:
30.10.91 Bulletin 91/44

(84) Designated Contracting States:
AT BE CH DE DK ES FR GB GR IT LI LU NL SE

(71) Applicant: NISSAN CHEMICAL INDUSTRIES LTD.
7-1, 3-chome Kanda-Nishiki-cho
Chiyoda-ku Tokyo (JP)

(72) Inventor: Satow, Jun, c/o Nissan Chemical Ind. Ltd.
Central Research Institute, 722-1, Tsuboi-cho
Funabashi-shi, Chiba-ken (JP)
Inventor: Fukuda, Kenzou, c/o Nissan Chemical Ind. Ltd.
Central Research Institute, 722-1, Tsuboi-cho
Funabashi-shi, Chiba-ken (JP)
Inventor: Itoh, Kaoru, c/o Nissan Chemical Ind. Ltd.
Central Research Institute, 722-1, Tsuboi-cho
Funabashi-shi, Chiba-ken (JP)
Inventor: Kita, Hiroshi, c/o Nissan Chemical Ind. Ltd.
Central Research Institute, 722-1, Tsuboi-cho
Funabashi-shi, Chiba-ken (JP)
Inventor: Kawamura, Yasuo, c/o Nissan Chemical Ind. Ltd.
Central Research Institute, 722-1, Tsuboi-cho
Funabashi-shi, Chiba-ken (JP)
Inventor: Suzuki, Koichi, c/o Nissan Chemical Ind. Ltd.
1470, Oaza Shiraoka, Shiraoka-machi
Minamisaitama-gun, Saitama-ken (JP)
Inventor: Nawamaki, Tsutomu, c/o Nissan Chemical Ind. Ltd.
1470, Oaza Shiraoka, Shiraoka-machi
Minamisaitama-gun, Saitama-ken (JP)
Inventor: Watanabe, Shigeomi, c/o Nissan Chemical Ind. Ltd.
1470, Oaza Shiraoka, Shiraoka-machi
Minamisaitama-gun, Saitama-ken (JP)
Inventor: Endo, Toshiharu, c/o Nissan Chemical Ind. Ltd.
1470, Oaza Shiraoka, Shiraoka-machi
Minamisaitama-gun, Saitama-ken (JP)

(74) Representative: Woods, Geoffrey Corlett et al
J.A. KEMP & CO. 14 South Square Gray's Inn
London WC1R 5LX (GB)

## (54) Glutarimide derivatives and herbicides.

(57) Glutarimide derivatives of formula (I):

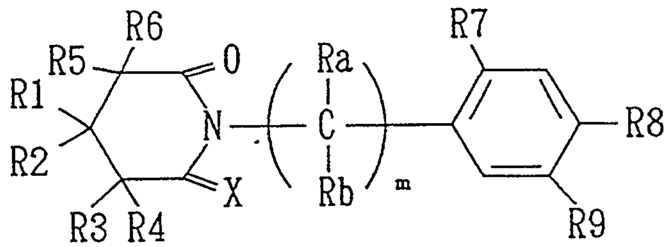

(I)

and glutaric acid amide derivatives of formula (VII):

EP 0 454 444 A1

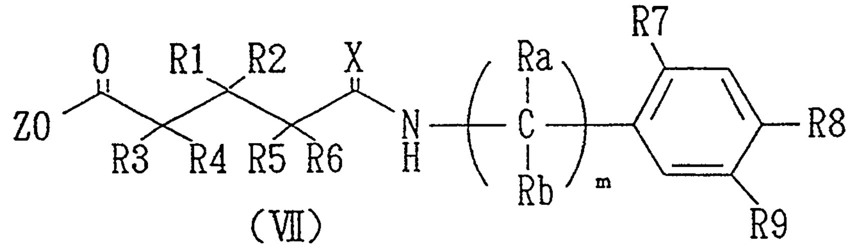

(VII)

have a herbicidal effect.

BACKGROUND OF THE INVENTION

This invention relates to novel glutarimide derivatives and selective herbicides containing said derivatives as active ingredients, and novel glutaric acid amide derivatives and selective herbicides containing said derivatives as active ingredients. The glutarimide derivative is effective as an active ingredient of a herbicide for a soil and paddy field and the glutaric acid amide derivative is effective as an active ingredient of a herbicide for a soil and paddy field as well as an intermediate for the former one, an intermediate for medicine and an intermediate for agricultural chemicals.

Many herbicides have been practiced in order to protect important crop plants such as rice, soybean, wheat, corn, cotton and beet from weeds, and to increase productivity thereof. These herbicides can be roughly classified into three classes of for upland cropping, paddy field and non-arable land. Further, in either of classes, it can be classified into the soil-incorporation treatment type, pre-emergence type and post-emergence treatment (foliage treatment) types according to the method of application, respectively.

In recent years, accompanying with increase of global population, it would be clear that productivity of important crop plants affects to food economy in the respective countries. In accordance with these changes, it would be certain that the conventional agricultural forms will be changed approaching 21st century. In fact, for farmers engaged in agriculture, development of herbicides which can economically and effectively kill weeds or control the growth of weeds which become hindrance during crop plants cultivation has been increasingly required.

As such a herbicide, development of chemicals which can satisfy the following requirements has earnestly been desired.

Desired are (1) those having high herbicidal activity with a small amount of chemicals (it is necessary to kill weeds with spreading chemicals as small amount as possible particularly in view of environmental protection), (2) those having a moderate residual activity (in recent years, the problem is pointed out that chemicals which remain in the soil for a long period of time give damage to the next crop plants so that it is important to show moderate residual activity after spreading), (3) those which promptly kill weeds after application (after chemical treatment, it is possible to perform seeding and transplantation of the next crop plants within a short term), (4) those with a little treatment times (application times) of chemicals (it is important for farmers engaged in agriculture to decrease the times of work for prevention and removing weeds), (5) those having wide range of preventable and removable weeds (it is desired that different kinds of weeds having different natures such as broad-leaved weeds, graminaceous weeds, perennial weeds, etc. can be prevented and removed with one kind of chemical), (6) those which can be applied with many kinds of application method (more potent herbicial activities can be obtained if the chemical has soil treatment effect, foliage treatment effect, etc. in combination) and (7) those showing no damage to crop plants, which arises problems (it is preferred to selectively kill weeds in a cultivated field where both crop plants and weeds in combination).

However, the herbicides which have already been used do not necessarily satisfy the above requirements.

As a glutarimide derivative, it has been known that Bemegrid represented by the following formula:

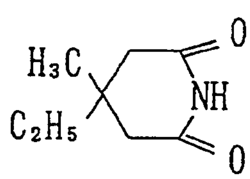

has a function of stimulant as a medicine, and as a derivative thereof in which a substituent at the N-position is changed, synthesis of an N-phenyl derivative has been disclosed in Australia Journal Chemistry, vol. 30, p. 1157 (1977), but no herbicidal activity has been disclosed. Also, in German Patent DE 2,100,800, it has been disclosed that a compound in which phenyl group wherein 3- and 5-position thereof are substituted is substituted on the N-position has a fungicidal activity but there is no description with regard to the herbicidal activity. Further, in U.S. Patent No. 4,400,202, there is disclosed a herbicide containing glutarimide but the substituent position of phenyl group substituted on the N-position is the meta-position and the other substituents are also different from those of the present invention.

It has been described that the compound having a substituent at the para-position of penyl group substituted on the nitrogen atom of glutarimide has herbicidal activity in Japanese Patent Application Laid-Open (KOKAI) No. 290850/1990 (corresponding to EP391847 and CA2013606). However, weeds killing activity thereof against weeds of *Gramineae* is insufficient.

In view of the above circumstances, as a result of the present inventors' earnest studies in order to develop

herbicides which show selectivity to important crop plants, have excellent herbicidal activities against many weeds with a little dosage and also have both soil treatment and foliage treatment effects, it has been found that glutarimide derivatives obtained by (1) reacting a glutaric anhydride derivative with an amine compound and then subjecting the resulting glutaramide derivative to dehydration cyclization or by (2) subjecting to addition, dehydration and cyclization with an amine compound, shows potent weeds killing activity against various weeds, particularly paddy weeds with a low dosage and show no phytotoxicity to important crop plants such as rice, beet, wheat, barley, sorghum, peanut, corn, soybean and cotton. The present invention has been accomplished on the basis of the above findings.

## SUMMARY OF THE INVENTION

In a first aspect of the present invention, there is provided glutarimide derivatives represented by the formula (I):

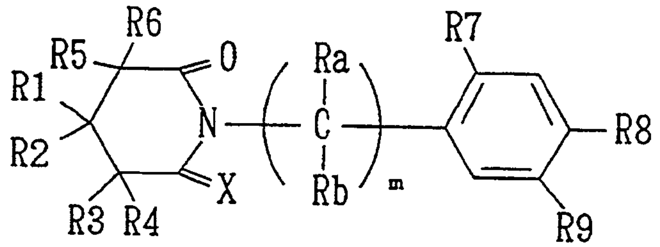

(I)

wherein X represents oxygen atom or sulfur atom;

R1, R2, R3, R4, R5 and R6 each independently represent hydrogen atom, a halogen atom, cyano group, a($C_1$ to $C_6$)alkyl group, a($C_3$ to $C_6$)cycloalkyl group, a($C_3$ to $C_6$)alkenyl group, a($C_3$ to $C_6$)alkynyl group, a($C_1$ to $C_6$)haloalkyl group, a($C_1$ to $C_6$)alkoxy group, a($C_1$ to $C_6$)haloalkoxy group, a($C_1$ to $C_6$)alkylthio group, a($C_1$ to $C_6$)haloalkylthio group, a($C_3$ to $C_6$)halocycloalkyl group, a phenyl group which may be substituted one or more substituent (the substituent is at least one selected from a halogen atom, a($C_1$ to $C_4$)alkyl group, a($C_1$ to $C_4$)haloalkyl group, a($C_1$ to $C_4$)alkoxy group and a($C_1$ to $C_4$)haloalkoxy group) or a benzyl group which may be substituted one or more substituent (the substituent is at least one selected from a halogen atom, a($C_1$ to $C_4$-)alkyl group, a($C_1$ to $C_4$)haloalkyl group, a($C_1$ to $C_4$)alkoxy group and a($C_1$ to $C_4$)haloalkoxy group), (two substituent groups on the same carbon atom may form a spiro ring by 2 to 5 methylene chains which may be optionally susbtituted by a halogen atom or two substituent groups on the adjacent carbon atoms may form a 5- to 6-membered ring by 1 to 4 methylene chains which may be optionally susbtituted by a halogen atom);

m is an integer of 0 to 3;

Ra and Rb each independently represent hydrogen atom or a($C_1$ to $C_4$)alkyl group;

R7 represents hydrogen atom or a halogen atom;

(i) when R8 and R9 are taken separately,

R8 represents a halogen atom, a($C_1$ to $C_4$)alkyl group, hydrogen atom, cyano group or nitro group;

R9 represents hydrogen atom, a halogen atom, a($C_1$ to $C_6$)alkyl group, cyano group, a($C_1$ to $C_4$)haloalkyl group, nitro group, amino group, hydroxyl group, mercapto group, carboxyl group, OR11 [wherein R11 represents a($C_1$ to $C_6$)alkyl group, a($C_3$ to $C_6$)cycloalkyl group, a($C_3$ to $C_6$)cycloalkyl($C_1$ to $C_2$)alkyl group, a($C_2$ to $C_6$)alkenyl group, a($C_3$ to $C_6$)alkynyl group, a($C_1$ to $C_4$)haloalkyl group, phenyl group or benzyl group],

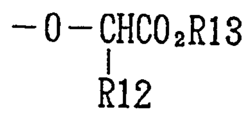

[wherein R12 represents hydrogen atom, a($C_1$ to $C_3$)alkyl group, methylthio group, phenylthio group or methoxymethyl group, R13 represents hydrogen atom, sodium atom, potassium atom, ammonium group, a($C_1$ to $C_6$)alkyl group, a($C_2$ to $C_6$)alkenyl group, a($C_3$ to $C_6$)alkynyl group, a($C_3$ to $C_6$)cycloalkyl group, a($C_3$ to $C_6$)cycloalkyl($C_1$ to $C_2$)alkyl group, a($C_2$ to $C_4$)haloalkyl group, phenyl group, phenethyl group or benzyl group], $-CO_2R14$ [wherein R14 represents a($C_1$ to $C_6$)alkyl group, a($C_2$ to $C_6$)alkenyl group, a($C_3$ to $C_5$)alkynyl group, a($C_3$ to $C_6$)cycloalkyl group, a($C_2$ to $C_4$)haloalkyl group, phenyl group or benzyl group], $-CO_2CH_2CO_2R15$ [wherein R15 represents hydrogen atom or a($C_1$ to $C_4$)alkyl group],

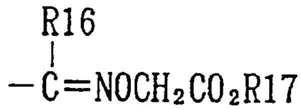

[wherein R16 represents hydrogen atom, a($C_1$ to $C_3$)alkyl group, a($C_1$ to $C_3$)alkoxy group or a($C_1$ to $C_3$)alkylthio group, R17 represents hydrogen atom, a($C_1$ to $C_6$)alkyl group, a($C_3$ to $C_6$)cycloalkyl group, a($C_2$ to $C_3$)haloalkyl group, phenyl group or benzyl group],

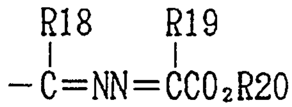

[wherein R18 represents hydrogen atom, a($C_1$ to $C_3$)alkyl group, a($C_1$ to $C_3$)alkoxy group or a($C_1$ to $C_3$)alkylthio group, R19 represents hydrogen atom or methyl group, R20 represents hydrogen atom, a($C_1$ to $C_4$)alkyl group, a($C_3$ to $C_6$)cycloalkyl group, a($C_2$ to $C_4$)haloalkyl group, phenyl group or benzyl group],

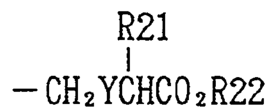

[wherein Y represents oxygen atom or sulfur atom, R21 represents hydrogen or a($C_1$ to $C_3$)alkyl group, R22 represents hydrogen atom, a($C_1$ to $C_6$)alkyl group, a($C_3$ to $C_6$)cycloalkyl group, a($C_2$ to $C_4$)haloalkyl group, phenyl group or benzyl group], -SR23 [wherein R23 represents a($C_1$ to $C_6$)alkyl group, a($C_3$ to $C_6$)cycloalkyl group, a($C_2$ to $C_6$)alkenyl group, a($C_3$ to $C_6$)alkynyl group, cyanomethyl group, hydroxymethyl group or chloromethyl group],

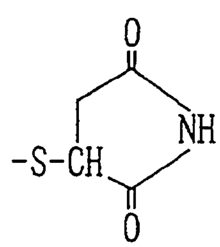 、 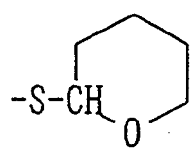 、

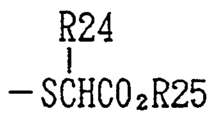

[wherein R24 represents hydrogen atom, a($C_1$ to $C_3$)alkyl group, methylthio group, phenylthio group or methoxymethyl group, R25 represents hydrogen atom, sodium atom, potassium atom, ammonium group, diisopropylammonium group, a($C_1$ to $C_6$)alkyl group, a($C_2$ to $C_6$)alkenyl group, a($C_3$ to $C_6$)alkynyl group, a($C_3$ to $C_6$)cycloalkyl group, a($C_3$ to $C_6$)cycloalkyl($C_1$ to $C_2$)alkyl group, a($C_2$ to $C_4$)haloalkyl group, phenyl group, benzyl group, α-methylbenzyl group, $-CH_2CH_2CH_2N(CH_3)_2$, a phenyl group which may be substituted one or more substituent (the substituent is at least one selected from a halogen atom, a($C_1$ to $C_4$)alkyl group, a($C_1$ to $C_4$)alkoxy group, a($C_1$ to $C_2$)alkylamino group, dimethylamino group, a($C_1$ to $C_2$)alkoxycarbonyl group, a($C_1$ to $C_2$)alkylcarbonyl group, a($C_1$ to $C_4$)haloalkyl group and a($C_1$ to $C_4$)haloalkyloxy group), or a benzyl group which may be substituted one or more substituent (the substituent is at least one selected from a halogen atom, a($C_1$ to $C_4$)alkyl group, a($C_1$ to $C_3$)alkoxy group, a($C_1$ to $C_2$)alkylamino group, dimethylamino group, a($C_1$ to $C_2$)alkylcarbonyl group, $-NHCOCF_3$, a($C_1$ to $C_2$)alkoxycarbonyl group, methylsulfonyl group, trifluoromethylsulfonyl group, a($C_1$ to $C_2$)haloalkyl group and a($C_1$ to $C_2$)haloalkyloxy group)], $-SCH_2(CH_2)_nCO_2R25$ [wherein n is an integer of 1 to 4, R25 is the same meaning as defined above], $-SCH_2$-Y-R26 [wherein Y is the same meaning as defined above, R26 represents hydrogen atom, a($C_1$ to $C_5$)alkyl group, cyanomethyl group, a($C_1$ to $C_4$)alkylcarbonyl group, $-COCH_2Cl$ or $CON(CH_3)_2$], $-SCH_2$-Y-CH(R24)$CO_2$R25 [wherein Y, R24 and R25 are the same

meanings as defined above], $-SCH_2-Y-CH_2-(CH_2)_n-CO_2R25$ [wherein Y, n and R25 are the same meanings as defined above],

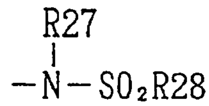

[wherein R27 represents hydrogen atom, sodium atom, a($C_1$ to $C_4$)alkyl group, a($C_1$ to $C_4$)alkylsulfonyl group or a($C_1$ to $C_3$)alkoxy($C_1$ to $C_2$)alkyl group, and R28 represents a($C_1$ to $C_4$)alkyl group or a($C_1$ to $C_3$)haloalkyl group], $-N(R27)CO_2R25$ [wherein R27 and R25 are the same meanings as defined above], $-N(R26)CH(R12)CO_2R25$ [wherein R12, R25 and R26 are the same meanings as defined above], -N(R27)COR11 [wherein R11 and R27 are the same meanings as defined above],

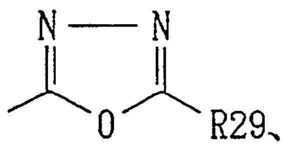,
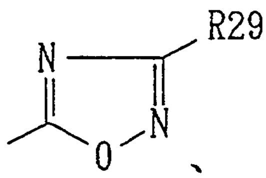,
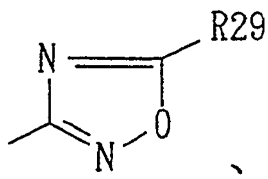,

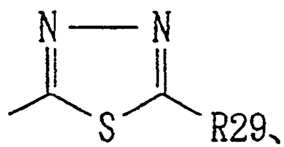,
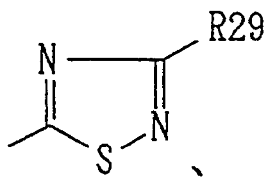,
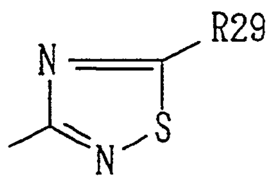

[wherein R29 represents hydrogen atom, a($C_1$ to $C_6$)alkyl group, a($C_3$ to $C_6$)cycloalkyl group, a($C_1$ to $C_3$)haloalkyl group, a($C_1$ to $C_5$)alkoxy group, a($C_1$ to $C_6$)alkylthio group, a($C_1$ to $C_4$)alkoxycarbonyl group, mercapto group, hydroxyl group, amino group, a($C_1$ to $C_3$)alkoxy($C_1$ to $C_2$)alkyl group, a($C_1$ to $C_6$)alkylamino group, NR30(R31) {wherein R30 and R31 each independently represent a($C_1$ to $C_6$)alkyl group}, $-CON(CH_3)_2$, $-CONHSO_2CH_3$, a($C_1$ to $C_6$)alkylsulfonyl group, a halogen atom, phenyl group, benzyl group or $-N(R32)SO_2R33$ {wherein R32 represents hydrogen atom, sodium atom, a($C_1$ to $C_6$)alkyl group, a($C_1$ to $C_4$)alkylsulfonyl group, a($C_1$ to $C_3$)alkoxy($C_1$ to $C_2$)alkyl group, and R33 represents a($C_1$ to $C_6$)alkyl group or a($C_1$ to $C_4$)haloalkyl group}]

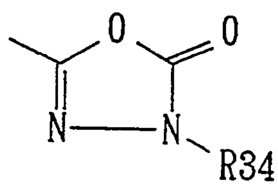

[wherein R34 represents a($C_3$ to $C_6$)alkyl group or a($C_1$ to $C_6$)cycloalkyl group],

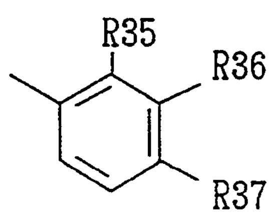

[wherein R35, R36 and R37 each independently represent hydrogen atom or a($C_1$ to $C_6$)alkyl group],

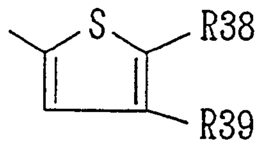 , 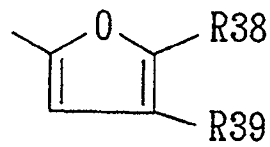

[wherein R38 and R39 each independently represent a halogen atom, hydrogen atom, a($C_1$ to $C_6$)alkyl group or dimethylamino group],

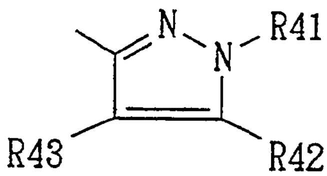 , 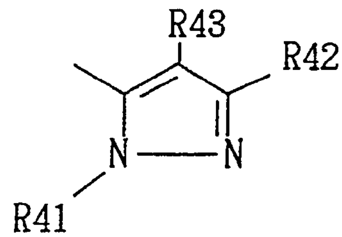

[wherein R41 represents hydrogen atom, a($C_1$ to $C_6$)alkyl group, a($C_3$ to $C_6$)cycloalkyl group, phenyl group, benzyl group or 2-pyridyl group, R42 represents hydrogen atom, a halogen atom, hydroxyl group, amino group, mercapto group, a($C_1$ to $C_6$)alkyl group, a($C_1$ to $C_4$)alkylamino group, -NR30(R31) {wherein R 30 and R31 are the same meanings as defined above}, a($C_1$ to $C_4$)alkylthio group, $-CO_2R30$ {wherein R30 is the same meaning as defined above}, a($C_1$ to $C_4$)alkylsulfonyl group, a($C_1$ to $C_4$)alkoxy group or $N(R27)SO_2R28$ {wherein R27 and R28 are the same meanings as defined above}, R43 represents hydrogen atom, a halogen atom, nitro group, amino group, cyano group, a($C_1$ to $C_4$)alkylamino group, -NR30(R31) {wherein R30 and R31 are the same meanings as defined above}, a($C_1$ to $C_4$)alkylcarbonyl group or a($C_1$ to $C_4$)alkoxycarbonyl group],

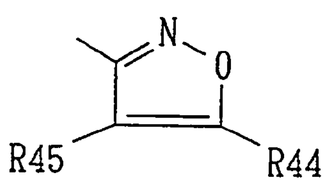 , 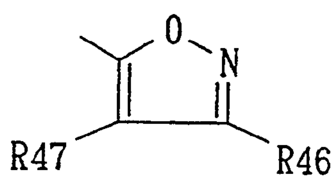

[wherein R44 represents hydrogen atom, hydroxyl group, methoxy group, amino group, benzoyl group or a($C_1$ to $C_6$)alkyl group, R45 represents hydrogen atom, cyano group, acetyl group, a($C_1$ to $C_6$)alkyl group, carboxyl group or a($C_1$ to $C_4$)alkoxycarbonyl group, R46 and R47 each independently represent hydrogen atom, phenyl group, hydroxyl group, methoxy group or a($C_1$ to $C_6$)alkyl group],

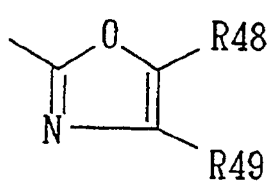

[wherein R48 and R49 each independently represent hydrogen atom or a($C_1$ to $C_6$)alkyl group], or

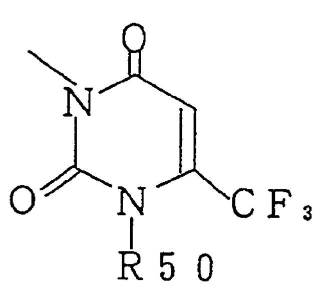

[wherein R50 represents hydrogen atom, sodium atom or a($C_1$ to $C_4$)alkyl group];

(ii) when R8 and R9 form a ring,

the glutarimide derivatives are represented by the formula (II), formula (III), formula (IV), formula (V) or formula (VI):

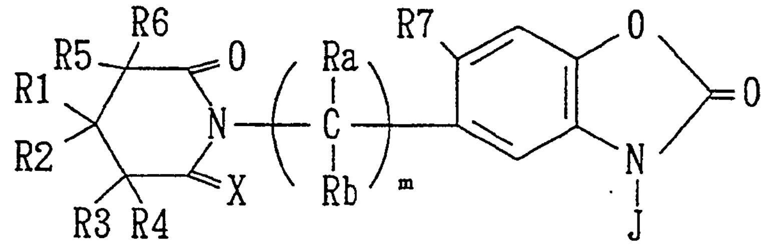

(II)

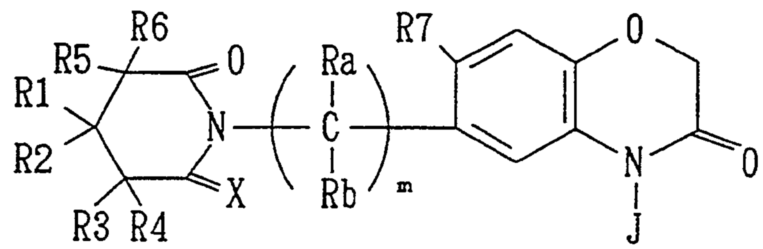

(III)

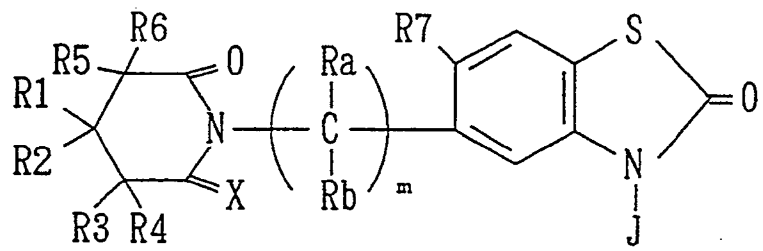

(IV)

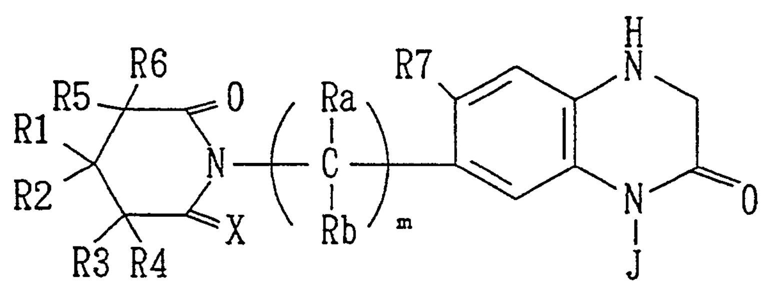

(V)

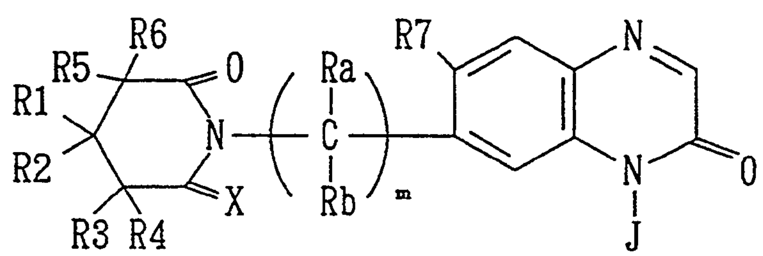

(VI)

wherein Ra, Rb, m, R1, R2, R3, R4, R5, R6, R7 and X are the same meanings as defined above;

J represents hydrogen atom, a($C_1$ to $C_6$)alkyl group, a($C_2$ to $C_6$)alkenyl group, a($C_3$ to $C_6$)alkynyl group, hydroxymethyl group, chloromethyl group, $-CH_2CONH_2$, cyanomethyl group, $-CH_2CO_2$-($C_1$ to $C_4$)alkyl, a($C_3$ to $C_6$)cycloalkyl group, a($C_3$ to $C_6$)cycloalkyl($C_1$ to $C_2$)alkyl group, a($C_1$ to $C_2$)alkoxy($C_1$ to $C_2$)alkyl group, a phenyl group which may be substituted one or more substituent (the substituent is at least one selected from a($C_1$ to $C_6$)alkyl group, a($C_1$ to $C_6$)alkoxy group, a halogen atom, a($C_1$ to $C_6$)haloalkyl group, a($C_1$ to $C_6$)haloalkoxy group, a($C_3$ to $C_6$)cycloalkyl group, a($C_1$ to $C_4$)alkylamino group, a($C_1$ to $C_4$)alkoxycarbonyl group, a($C_2$ to $C_4$)alkylcarbonyl group, a($C_1$ to $C_4$)alkylsulfonyl group, a($C_2$ to $C_6$)alkenyl group and a($C_2$ to $C_6$)alkynyl group) or a benzyl group which may be substituted one or more substituent (the substituent is at least one selected

from a($C_1$ to $C_6$)alkyl group, a($C_1$ to $C_6$)alkoxy group, a halogen atom, a($C_1$ to $C_6$)haloalkyl group, a($C_1$ to $C_6$)haloalkoxy group, a($C_3$ to $C_6$)cycloalkyl group, a($C_1$ to $C_4$)alkylamino group, a($C_1$ to $C_4$)alkoxycarbonyl group, a($C_2$ to $C_4$)alkylcarbonyl group, a($C_1$ to $C_4$)alkylsulfonyl group, a($C_2$ to $C_6$)alkenyl group and a($C_2$ to $C_6$-)alkynyl group),

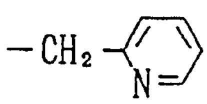 or 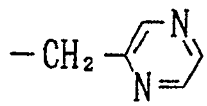 ;

provided that the glutarimide derivatives represented by the formula (A) are excluded,

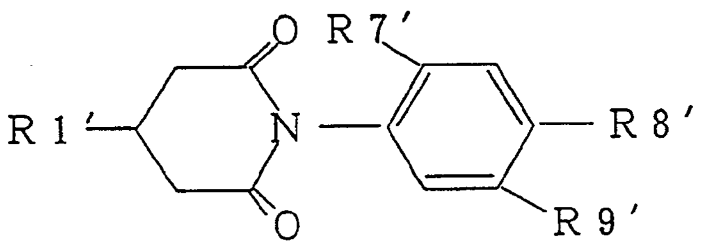

(A)

wherein R1′ represents hydrogen atom, a($C_1$ to $C_6$)alkyl group, a($C_3$ to $C_7$)cycloalkyl group, a($C_1$ to $C_6$)haloalkyl group, a($C_3$ to $C_6$)alkenyl group or a($C_3$ to $C_6$)alkynyl group;

R7′ represents hydrogen atom or a halogen atom;

R8′ represents hydrogen atom, cyano group, nitro group or a halogen atom;

R9′ represents hydrogen atom, cyano group, nitro group or hydroxyl group; -OR11 [wherein R11 is the same meaning as defined above], -O-CH(R12)$CO_2$R13 [wherein R12 and R13 are the same meanings as defined above], -$CO_2$R14 [wherein R14 is the same meaning as defined above], -C(R16′)=$NOCH_2CO_2$R17 [wherein R16′ represents hydrogen atom or a($C_1$ to $C_3$)alkyl group, and R17 is the same meaning as defined above], -SR23′ [wherein R23′ represents a($C_1$ to $C_6$)alkyl group, a ($C_3$ to $C_6$)cycloalkyl group, a($C_2$ to $C_6$)alkenyl group, a($C_3$ to $C_6$)alkynyl group or cyanomethyl group], -SCH(R24)$CO_2$R25 [wherein R24 and R25 are the same meanings as defined above], or -$SCH_2(CH_2)_{n'}CO_2$R25 [wherein n′ is an integer of 1 to 3 and R25 is the same meaning as defined above, and provided that when a compound of an optically active site, or a stereoisomer or geometric isomer is included in the glutarimide derivative, all these forms are included.

In a second aspect of the present invention, there is provided glutarc acid amide derivatives represented by the formula (VII):

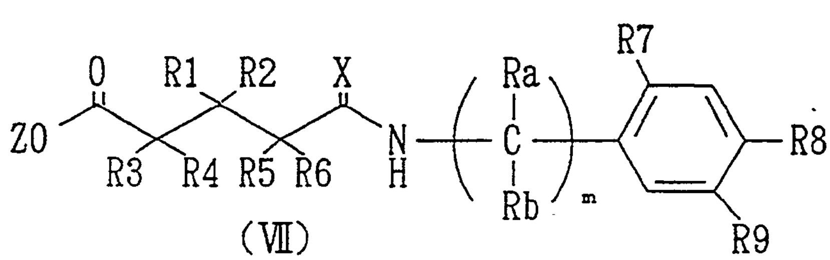

(VII)

wherein X represents oxygen atom or sulfur atom;

Z represents hydrogen atom, a($C_1$ to $C_6$)alkyl group, phenyl group or benzyl group;

R1, R2, R3, R4, R5 and R6 each independently represent hydrogen atom, a halogen atom, cyano group, a($C_1$ to $C_6$)alkyl group, a($C_3$ to $C_6$)cycloalkyl group, a($C_3$ to $C_6$)alkenyl group, a($C_3$ to $C_6$)alkynyl group, a($C_1$ to $C_6$)haloalkyl group, a($C_1$ to $C_6$)alkoxy group, a($C_1$ to $C_6$)haloalkoxy group, a($C_1$ to $C_6$)alkylthio group, a($C_1$ to $C_6$)haloalkylthio group, a($C_3$ to $C_6$)halocycloalkyl group, a phenyl group which may be substituted one or more substituent (the substituent is at least one selected from a halogen atom, a($C_1$ to $C_4$)alkyl group, a($C_1$ to $C_4$)haloalkyl group, a($C_1$ to $C_4$)alkoxy group and a($C_1$ to $C_4$)haloalkoxy group) or a benzyl group which may be substituted one or more substituent (the substituent is at least one selected from a halogen atom, a($C_1$ to $C_4$-)alkyl group, a($C_1$ to $C_4$)haloalkyl group, a($C_1$ to $C_4$)alkoxy group and a($C_1$ to $C_4$)haloalkoxy group), (two substituent groups on the same carbon atom may form a spiro ring by 2 to 5 methylene chains which may be optionally susbtituted by a halogen atom or two substituent groups on the adjacent carbon atom may form a

5- to 6-membered ring by 1 to 4 methylene chains which may be optionally susbtituted by a halogen atom);

m is an integer of 0 to 3;

Ra and Rb each independently represent hydrogen atom or a($C_1$ to $C_4$)alkyl group;

R7 represents hydrogen atom or a halogen atom;

(i) when R8 and R9 are taken separately,

R8 represents a halogen atom, a($C_1$ to $C_4$)alkyl group, hydrogen atom, cyano group or nitro group;

R9 represents hydrogen atom, a halogen atom, a($C_1$ to $C_6$)alkyl group, cyano group, a($C_1$ to $C_4$)haloalkyl group, nitro group, amino group, hydroxyl group, mercapto group, carboxyl group, OR11 [wherein R11 represents a($C_1$ to $C_6$)alkyl group, a($C_3$ to $C_6$)cycloalkyl group, a($C_3$ to $C_6$)cycloalkyl($C_1$ to $C_2$)alkyl group, a($C_2$ to $C_6$)alkenyl group, a($C_3$ to $C_6$)alkynyl group, a($C_1$ to $C_4$)haloalkyl group, phenyl group or benzyl group],

$$-O-\underset{R12}{\underset{|}{C}}HCO_2R13$$

[wherein R12 represents hydrogen atom, a($C_1$ to $C_3$)alkyl group, methylthio group, phenylthio group or methoxymethyl group, R13 represents hydrogen atom, sodium atom, potassium atom, ammonium group, a($C_1$ to $C_6$)alkyl group, a($C_2$ to $C_6$)alkenyl group, a($C_3$ to $C_6$)alkynyl group, a($C_3$ to $C_6$)cycloalkyl group, a($C_3$ to $C_6$)cycloalkyl($C_1$ to $C_2$)alkyl group, a($C_2$ to $C_4$)haloalkyl group, phenyl group, phenethyl group or benzyl group], -$CO_2$R14 [wherein R14 represents a($C_1$ to $C_6$)alkyl group, a($C_2$ to $C_6$)alkenyl group, a($C_3$ to $C_5$)alkynyl group, a($C_3$ to $C_6$)cycloalkyl group, a($C_2$ to $C_4$)haloalkyl group, phenyl group or benzyl group], -$CO_2CH_2CO_2$R15 [wherein R15 represents hydrogen atom or a($C_1$ to $C_4$)alkyl group],

$$-\overset{R16}{\overset{|}{C}}=NOCH_2CO_2R17$$

[wherein R16 represents hydrogen atom, a($C_1$ to $C_3$)alkyl group, a($C_1$ to $C_3$)alkoxy group or a($C_1$ to $C_3$)alkylthio group, R17 represents hydrogen atom, a($C_1$ to $C_6$)alkyl group, a($C_3$ to $C_6$)cycloalkyl group, a($C_2$ to $C_3$)haloalkyl group, phenyl group or benzyl group],

$$-\overset{R18}{\overset{|}{C}}=NN=\overset{R19}{\overset{|}{C}}CO_2R20$$

[wherein R18 represents hydrogen atom, a($C_1$ to $C_3$)alkyl group, a($C_1$ to $C_3$)alkoxy group or a($C_1$ to $C_3$)alkylthio group, R19 represents hydrogen atom or methyl group, R20 represents hydrogen atom, a($C_1$ to $C_4$)alkyl group, a($C_3$ to $C_6$)cycloalkyl group, a($C_2$ to $C_4$)haloalkyl group, phenyl group or benzyl group],

$$-CH_2Y\overset{R21}{\overset{|}{C}}HCO_2R22$$

[wherein Y represents oxygen atom or sulfur atom, R21 represents hydrogen or a($C_1$ to $C_3$)alkyl group, R22 represents hydrogen atom, a($C_1$ to $C_6$)alkyl group, a($C_3$ to $C_6$)cycloalkyl group, a($C_2$ to $C_4$)haloalkyl group, phenyl group or benzyl group], -SR23 [wherein R23 represents a($C_1$ to $C_6$)alkyl group, a($C_3$ to $C_6$)cycloalkyl group, a($C_2$ to $C_6$)alkenyl group, a($C_3$ to $C_6$)alkynyl group, cyanomethyl group, hydroxymethyl group or chloromethyl group],

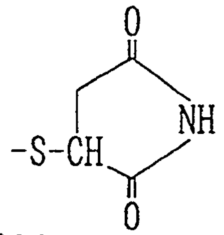 、 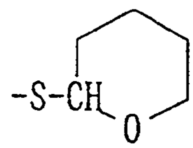 、

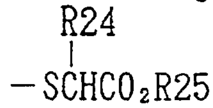

[wherein R24 represents hydrogen atom, a($C_1$ to $C_3$)alkyl group, methylthio group, phenylthio group or methoxymethyl group, R25 represents hydrogen atom, sodium atom, potassium atom, ammonium group, diisopropylammonium group, a($C_1$ to $C_6$)alkyl group, a($C_2$ to $C_6$)alkenyl group, a($C_3$ to $C_6$)alkynyl group, a($C_3$ to $C_6$)cycloalkyl group, a($C_3$ to $C_6$)cycloalkyl($C_1$ to $C_2$)alkyl group, a($C_2$ to $C_4$)haloalkyl group, phenyl group, benzyl group, α-methylbenzyl group, $-CH_2CH_2CH_2N(CH_3)_2$, a phenyl group which may be substituted one or more substituent (the substituent is at least one selected from a halogen atom, a($C_1$ to $C_4$)alkyl group, a($C_1$ to $C_4$)alkoxy group, a($C_1$ to $C_2$)alkylamino group, dimethylamino group, a($C_1$ to $C_2$)alkoxycarbonyl group, a($C_1$ to $C_2$)alkylcarbonyl group, a($C_1$ to $C_4$)haloalkyl group and a($C_1$ to $C_4$)haloalkyloxy group); or a benzyl group which may be substituted one or more substituent (the substituent is at least one selected from a halogen atom, a($C_1$ to $C_4$)alkyl group, a($C_1$ to $C_3$)alkoxy group, a($C_1$ to $C_2$)alkylamino group, dimethylamino group, a($C_1$ to $C_2$)alkylcarbonyl group, $-NHCOCF_3$, a($C_1$ to $C_2$)alkoxycarbonyl group, methylsulfonyl group, trifluoromethylsulfonyl group, a($C_1$ to $C_2$)haloalkyl group and a($C_1$ to $C_2$)haloalkyloxy group)],$-SCH_2(CH_2)_nCO_2R25$ [wherein n is an integer of 1 to 4, R25 is the same meaning as defined above], $-SCH_2$-Y-R26 [wherein Y is the same meaning as defined above, R26 represents hydrogen atom, a($C_1$ to $C_5$)alkyl group, cyanomethyl group, a($C_1$ to $C_4$)alkylcarbonyl group, $-COCH_2Cl$ or $CON(CH_3)_2$], $-SCH_2$-Y-CH(R24)$CO_2$R25 [wherein Y, R24 and R25 are the same meanings as defined above], $-SCH_2$-Y-$CH_2$-$(CH_2)_n$-$CO_2$R25 [wherein Y, n and R25 are the same meanings as defined above],

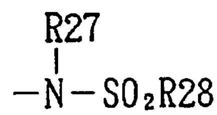

[wherein R27 represents hydrogen atom, sodium atom, a($C_1$ to $C_4$)alkyl group, a($C_1$ to $C_4$)alkylsulfonyl group or a($C_1$ to $C_3$)alkoxy($C_1$ to $C_2$)alkyl group, and R28 represents a($C_1$ to $C_4$)alkyl group or a($C_1$ to $C_3$)haloalkyl group], -N(R27)$CO_2$R25 [wherein R27 and R25 are the same meanings as defined above], -N(R26)CH(R12)$CO_2$R25 [wherein R12, R25 and R26 are the same meanings as defined above], -N(R27)COR11 [wherein R11 and R27 are the same meanings as defined above],

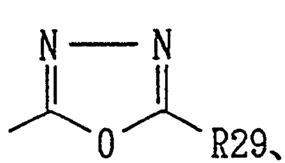 、 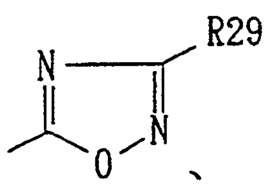 、 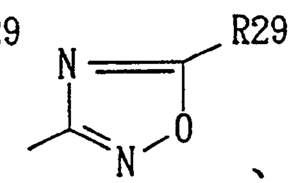 、

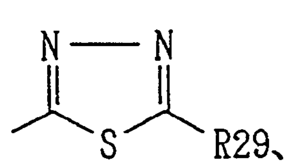 、 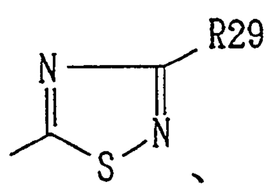 、 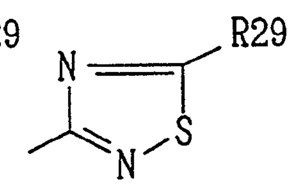

[wherein R29 represents hydrogen atom, a($C_1$ to $C_6$)alkyl group, a($C_3$ to $C_6$)cycloalkyl group, a($C_1$ to $C_3$)haloalkyl group, a($C_1$ to $C_5$)alkoxy group, a($C_1$ to $C_6$)alkylthio group, a($C_1$ to $C_4$)alkoxycarbonyl group, mercapto group, hydroxyl group, amino group, a($C_1$ to $C_3$)alkoxy($C_1$ to $C_2$)alkyl group, a($C_1$ to $C_6$)alkylamino group, NR30(R31)

{wherein R30 and R31 each independently represent a($C_1$ to $C_6$)alkyl group}, -CON($CH_3$)$_2$, -CONH$SO_2CH_3$, a($C_1$ to $C_6$)alkylsulfonyl group, a halogen atom, phenyl group, benzyl group or -N(R32)$SO_2$R33 (wherein R32 represents hydrogen atom, sodium atom, a($C_1$ to $C_6$)alkyl group, a($C_1$ to $C_4$)alkylsulfonyl group, a($C_1$ to $C_3$)alkoxy($C_1$ to $C_2$)alkyl group, and R33 represents a($C_1$ to $C_6$)alkyl group or a($C_1$ to $C_4$)haloalkyl group}],

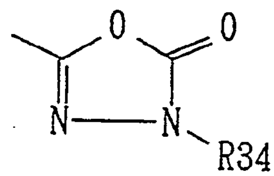

[wherein R34 represents a($C_1$ to $C_5$)alkyl group or a($C_3$ to $C_6$)cycloalkyl group],

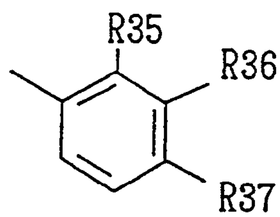

[wherein R35, R36 and R37 each independently represent hydrogen atom or a($C_1$ to $C_6$)alkyl group],

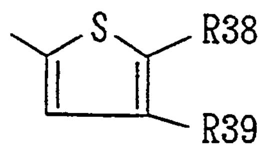 , 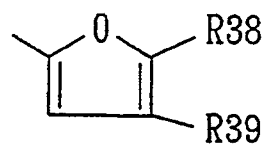

[wherein R38 and R39 each independently represent a halogen atom, hydrogen atom, a($C_1$ to $C_6$)alkyl group or dimethylamino group],

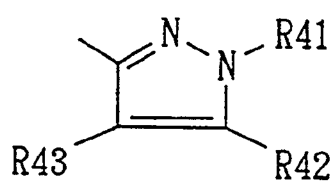 , 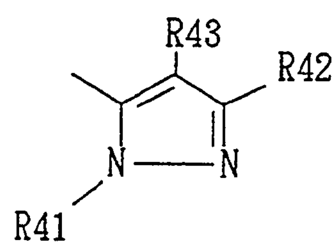

[wherein R41 represents hydrogen atom, a($C_1$ to $C_6$)alkyl group, a($C_3$ to $C_6$)cycloalkyl group, phenyl group, benzyl group or 2-pyridyl group, R42 represents hydrogen atom, a halogen atom, hydroxyl group, amino group, mercapto group, a($C_1$ to $C_6$)alkyl group, a($C_1$ to $C_4$)alkylamino group, -NR30(R31) {wherein R 30 and R31 are the same meanings as defined above}, a($C_1$ to $C_4$)alkylthio group, -$CO_2$R30 {wherein R30 is the same meaning as defined above}, a($C_1$ to $C_4$)alkylsulfonyl group, a($C_1$ to $C_4$)alkoxy group or N(R27)$SO_2$R28 {wherein R27 and R28 are the same meanings as defined above}, R43 represents hydrogen atom, a halogen atom, nitro group, amino group, cyano group, a($C_1$ to $C_4$)alkylamino group, -NR30(R31) {wherein R30 and R31 are the same meanings as defined above}, a($C_1$ to $C_4$)alkylcarbonyl group or a($C_1$ to $C_4$)alkoxycarbonyl group],

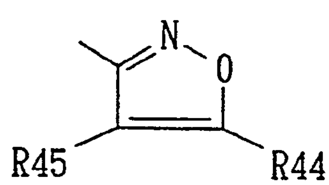 , 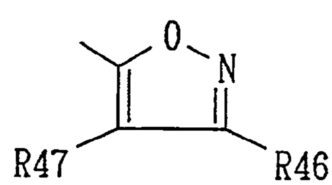

[wherein R44 represents hydrogen atom, hydroxyl group, methoxy group, amino group, benzoyl group or a($C_1$ to $C_6$)alkyl group, R45 represents hydrogen atom, cyano group, acetyl group, a($C_1$ to $C_6$)alkyl group, carboxyl group or a($C_1$ to $C_4$)alkoxycarbonyl group, R46 and R47 each independently represent hydrogen atom, phenyl group, hydroxyl group, methoxy group or a($C_1$ to $C_6$)alkyl group],

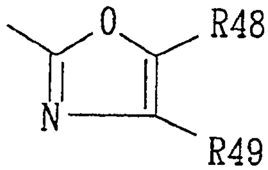

[wherein R48 and R49 each independently represent hydrogen atom or a($C_1$ to $C_6$)alkyl group], or

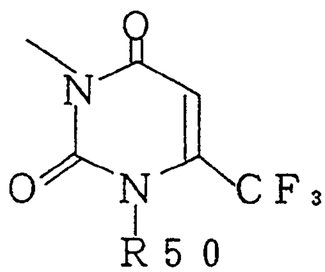

wherein R50 represents hydrogen atom, sodium atom or a($C_1$ to $C_4$)alkyl group;

(ii) when R8 and R9 form a ring,

the glutaric acid amide derivatives are represented by the formula (VIII), formula (IX), formula (X), formula (XI) or formula (XII):

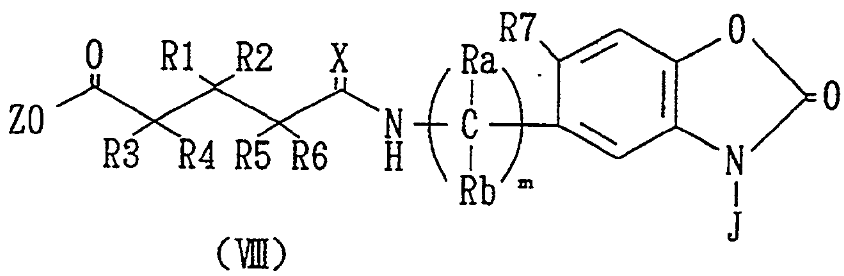

(VIII)

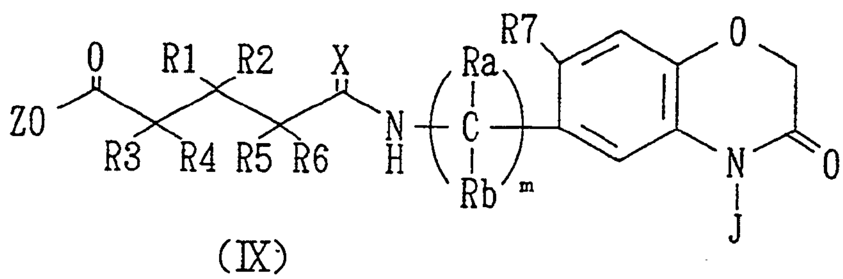

(IX)

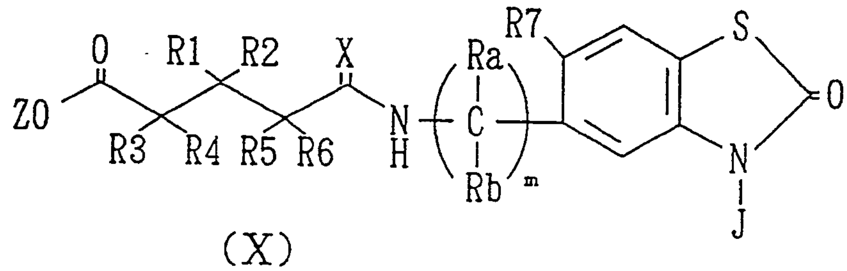

(X)

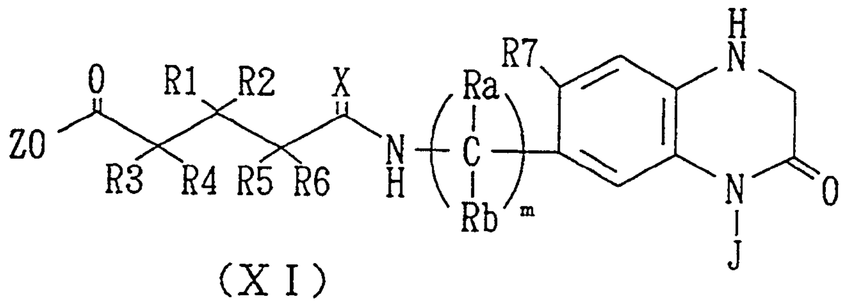

(X I)

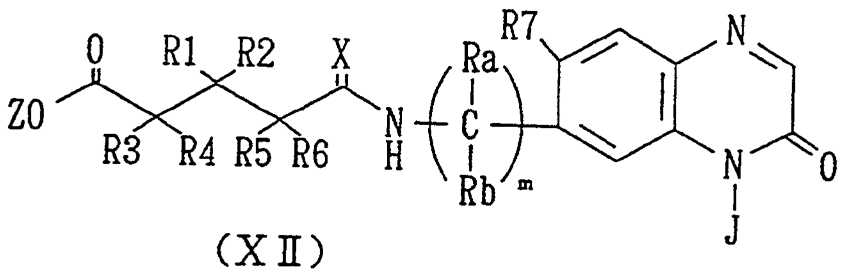

(X II)

wherein Ra, Rb, m, R1, R2, R3, R4, R5, R6, R7, X and Z are the same meanings as defined above;

J represents hydrogen atom, a($C_1$ to $C_6$)alkyl group, a($C_2$ to $C_6$)alkenyl group, a($C_3$ to $C_6$)alkynyl group, hydroxymethyl group, chloromethyl group, $-CH_2CONH_2$, cyanomethyl group, $-CH_2CO_2$($C_1$ to $C_4$)alkyl, a($C_3$ to $C_6$)cycloalkyl group, a($C_3$ to $C_6$)cycloalkyl($C_1$ to $C_2$)alkyl group, a($C_1$ to $C_2$)alkoxy($C_1$ to $C_2$)alkyl group, a phenyl group which may be substituted one or more substituent (the substituent is at least one selected from a($C_1$ to $C_6$)alkyl group, a($C_1$ to $C_6$)alkoxy group, a halogen atom, a($C_1$ to $C_6$)haloalkyl group, a($C_1$ to $C_6$) haloalkoxy group, a($C_3$ to $C_6$)cycloalkyl group, a($C_1$ to $C_4$)alkylamino group, a($C_1$ to $C_4$)alkoxycarbonyl group, a($C_2$ to $C_4$)alkylcarbonyl group, a($C_1$ to $C_4$)alkylsulfonyl group, a($C_2$ to $C_6$)alkenyl group and a($C_2$ to $C_6$)alkynyl group) or a benzyl group which may be substituted one or more substituent (the substituent is at least one selected from a($C_1$ to $C_6$)alkyl group, a($C_1$ to $C_6$)alkoxy group, a halogen atom, a($C_1$ to $C_6$)haloalkyl group, a($C_1$ to $C_6$)haloalkoxy group, a($C_3$ to $C_6$)cycloalkyl group, a($C_1$ to $C_4$)alkylamino group, a($C_1$ to $C_4$)alkoxycarbonyl group, a($C_2$ to $C_4$)alkylcarbonyl group, a($C_1$ to $C_4$)alkylsulfonyl group, a($C_2$ to $C_6$)alkenyl group and a($C_2$ to $C_6$-)alkynyl group),

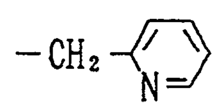 or 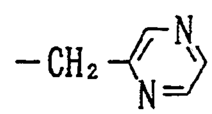 ;

provided that the glutaric acid amide derivatives represented by the formula (B) are excluded;

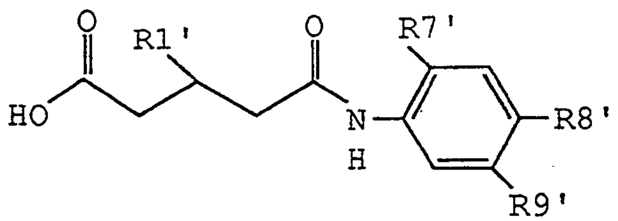

(B)

wherein R1′ represents hydrogen atom, a($C_1$ to $C_6$)alkyl group, a($C_3$ to $C_7$)cycloalkyl group, a($C_1$ to $C_6$)haloalkyl group, a($C_3$ to $C_6$)alkenyl group or a($C_3$ to $C_6$)alkynyl group;

R7′ represents hydrogen atom or a halogen atom;

R8′ represents hydrogen atom, cyano group, nitro group or a halogen atom;

R9′ represents hydrogen atom, cyano group, nitro group or hydroxyl group, -OR11 [wherein R11 is the same meaning as defined above], -O-CH(R12)$CO_2$R13 [wherein R12 and R13 are the same meanings as defined above], -$CO_2$R14 [wherein R14 is the same meaning as defined above], -C(R16′)=NO$CH_2CO_2$R17 [wherein R16′ represents hydrogen atom or a($C_1$ to $C_3$)alkyl group, and R17 is the same meaning as defined above], -SR23′ [wherein R23′ represents a($C_1$ to $C_6$)alkyl group, a($C_3$ to $C_6$)cycloalkyl group, a($C_2$ to $C_6$)alkenyl group, a($C_3$ to $C_6$)alkynyl group or cyanomethyl group], -SCH(R24)$CO_2$R25 [wherein R24 and R25 are the same meanings as defined above], or -$SCH_2(CH_2)_{n'}CO_2$R25 [wherein n′ is an integer of 1 to 3 and R25 is the same meaning as defined above], and provided that when a compound of an optically active site, or a stereoisomer or geometric isomer is included in the glutaric acid amide derivative, all these forms are included.

In a third aspect of the present invention, there is provided a herbicide which comprises a herbicidally effective amount of the glutarimide derivatives as defined in 1st aspect as an effective ingredient, and a herbicidally acceptable carrier or diluent therefor.

In a fourth aspect of the present invention, there is provided a herbicide which comprises a herbicidally effective amount of the glutaric acid amide derivatives as defined in 2nd aspect as an effective ingredient, and a herbicidally acceptable carrier or diluent therefor.

## DETAILED DESCRIPTION OF THE INVENTION

The glutarimide derivatives of the present invention represented by the formula (I) and the glutaric acid amide derivatives of the present invention represented by the formula (VII) have conventionally never been known and are novel compounds.

The compound of the present invention can be synthesized, for example, by the methods shown in Schemes 1, 2 and 3 (R1 to R9, Ra, Rb and m in Schemes 1, 2 and 3 are the same meanings as defined above, and R51 represents a $C_1$ to $C_6$, for example a $C_1$ to $C_5$, alkyl group, a phenyl group or a benzyl group).

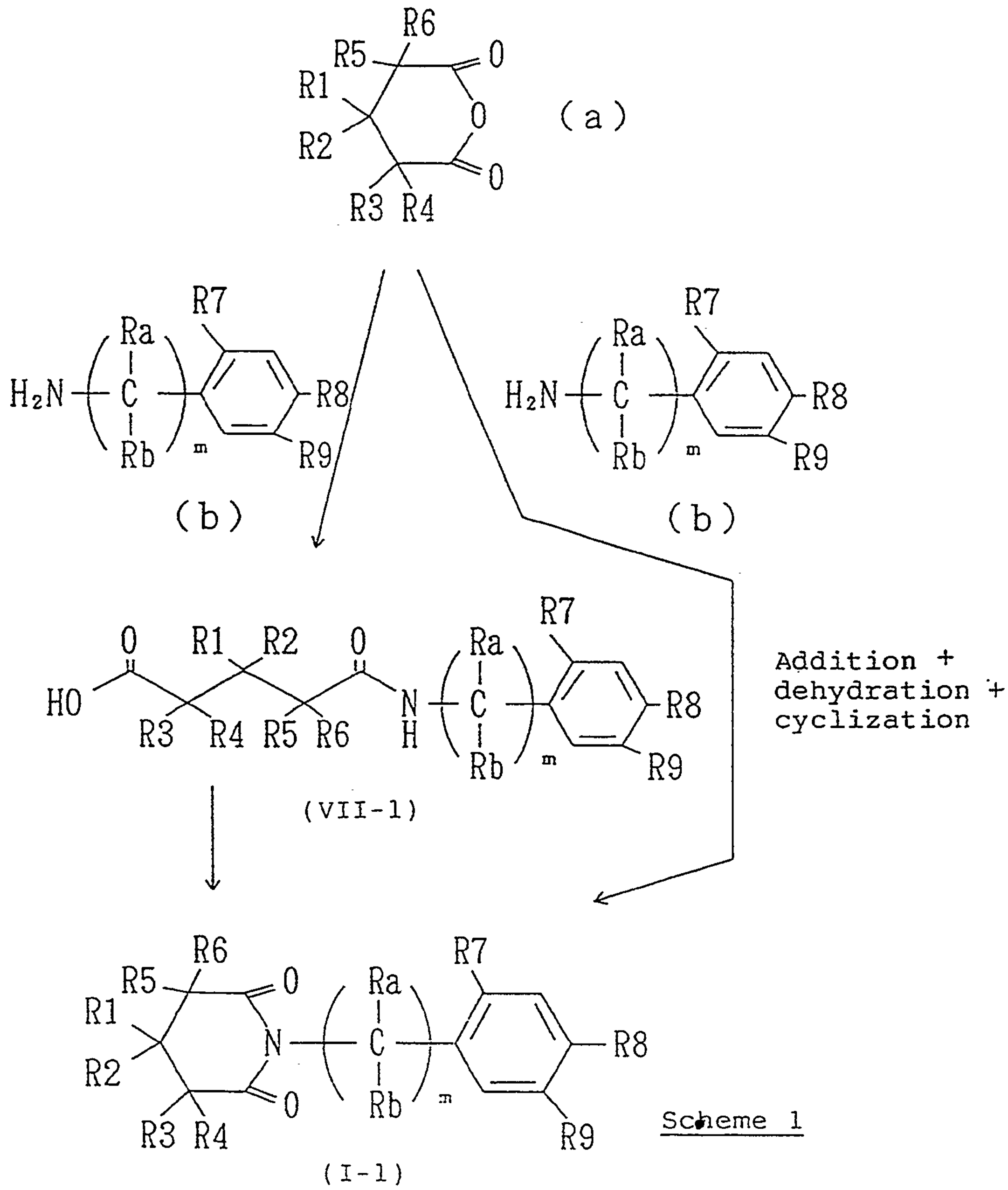


(a)

(b)

(b)

(VII-1)

(I-1)

Scheme 1

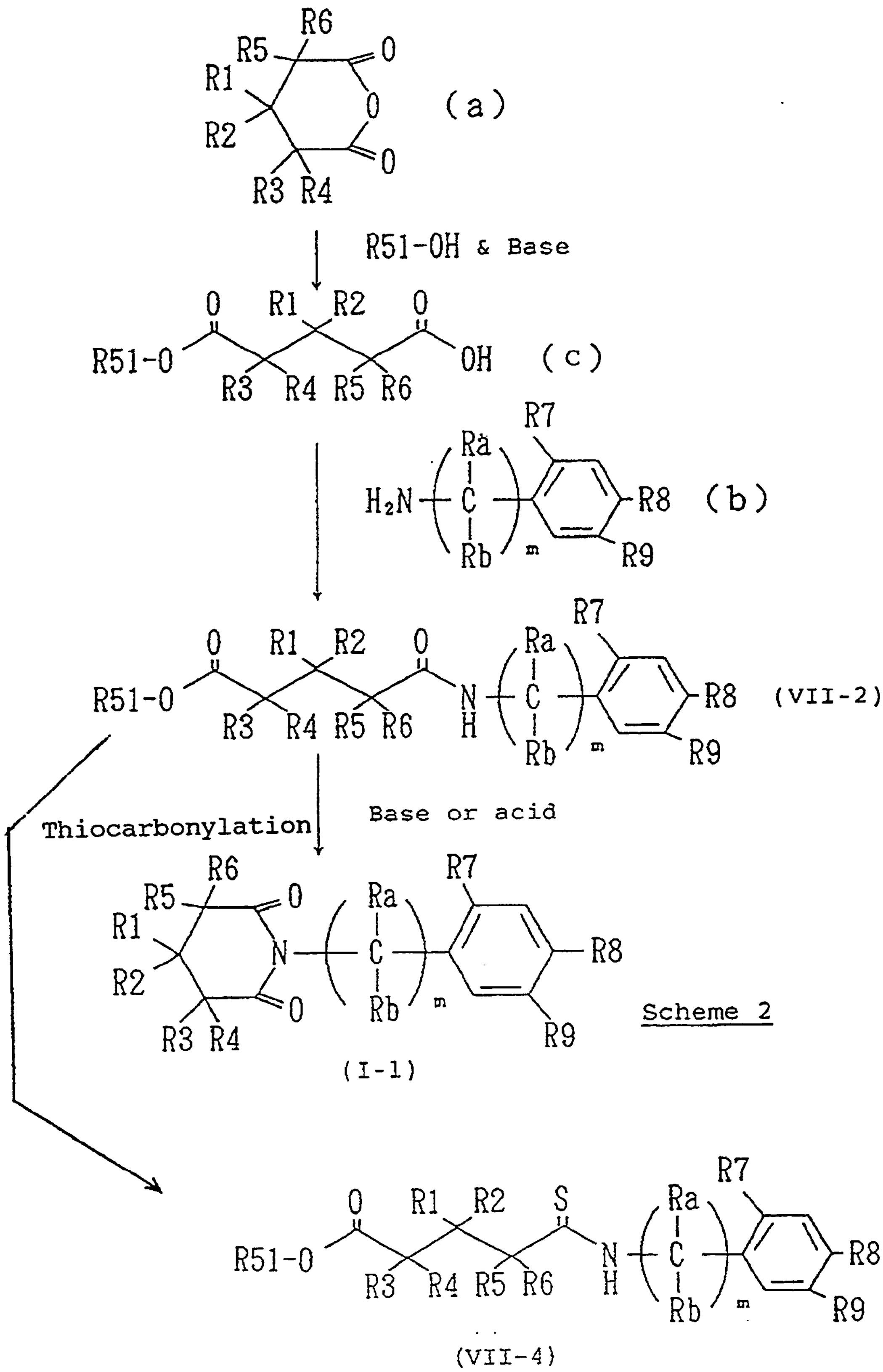


(a) (c) (b) (VII-2) (I-1) (VII-4)

Scheme 2

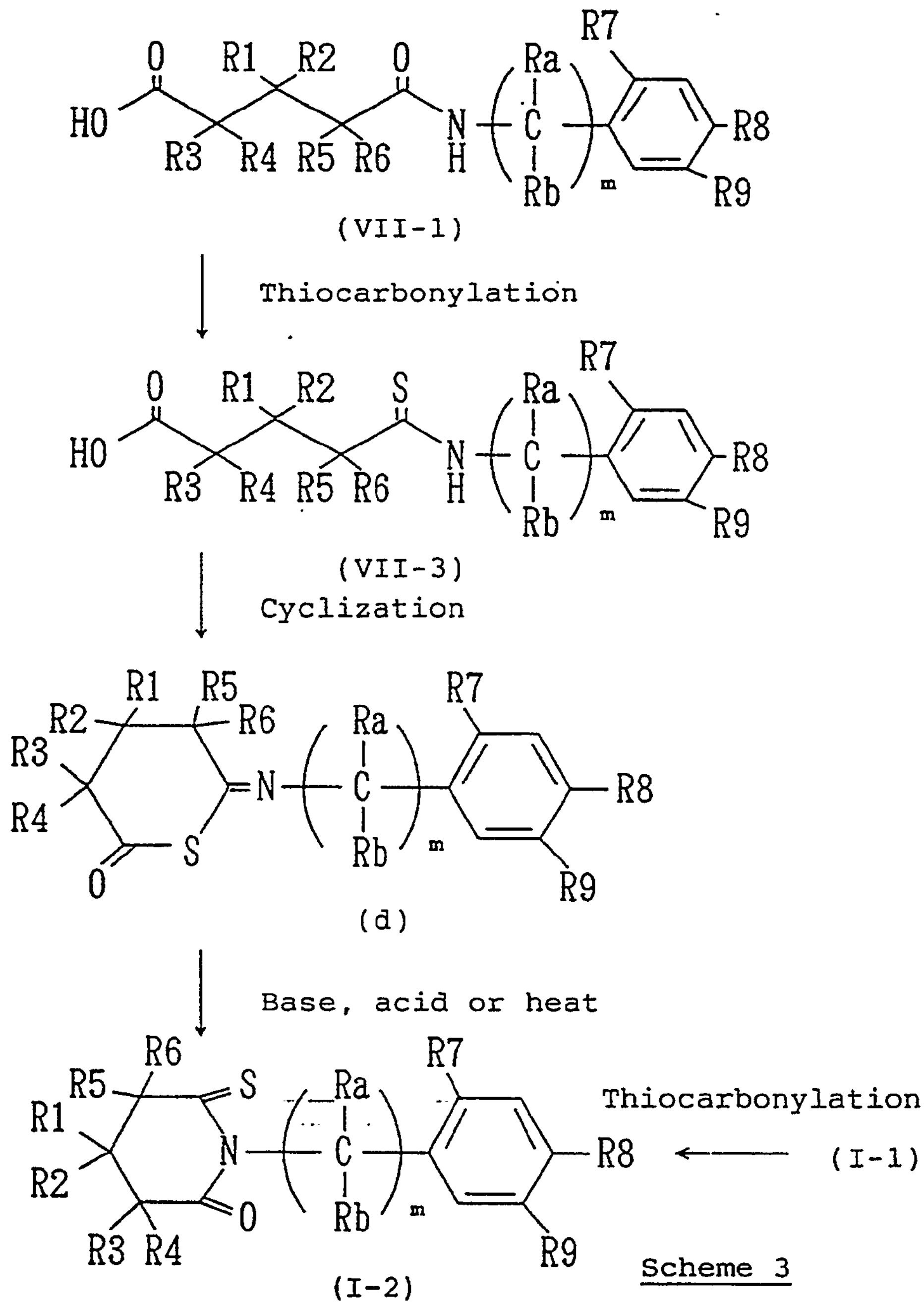


(VII-1)

(VII-3)

(d)

(I-1)

(I-2)

Scheme 3

(1) Scheme 1 shows the preparation method (i) in which a glutaric anhydride derivative (a) is reacted with an amine compound (b) to obtain a glutarmaide derivative (VII-1) and then the resulting compound is subjected to dehydration and cyclization treatment to produce a glutarimide derivative (I-1), or the preparation method (ii) in which an amine compound (b) is added to a glutaric anhydride derivative (a) so as to conduct addition, dehydration and cyclization treatment, thereby obtaining a glutarimide derivative (I-1).

(i) The amine compound (b) is used with an amount of 0.5 to 1.5 equivalent, preferably 0.7 to 1.2 equivalent based on 1 equivalent of the glutaric anhydride derivative (a).

The reaction may proceed even under the absence of a solvent, but the reaction may be accelarated by using a solvent. As the solvent, there may be mentioned aliphatic hydrocarbons such as hexane, heptane, ligroine, petroleum ether, etc.; aromatic hydrocarbons such as benzene, toluene, xylene, chlorobenzene, etc.; halogenated hydrocarbons such as chloroform, methylene chloride, etc.; organic acids such as acetic acid, butyric acid, trifluoroacetic acid, etc.; ethers such as diethyl ether, dioxane, tetrahydrofuran, etc.; ketones such as acetone, methyl ethyl ketone, etc.; nitriles such as acetonitrile, isobutyronitrile, etc.; tertiary amines such as pyridine, N,N-diethylaniline, etc.; acid amides such as formamide, N,N-dimethylformamide, N-methylpyrrolidone, etc.; sulfur-containing compounds such as dimethylsulfoxide, sulforane, etc.; water; and mixtures thereof. Among them, the above aliphatic hydrocarbons, aromatic hydrocarbons, organic acids, acid amides, ethers and

mixtures thereof are preferred.

The reaction temperature may be 20 to 200°C, preferably 80 to 160°C or at the reflux temperature of the solvent and the reaction time is 1 to 48 hours, preferably 3 to 18 hours.

Subsequently, the formed glutaramide derivative (VII-1) is heated in the above solvent by adding, if necessary, a dehydrating agent, at a temperature of 20 to 200°C, preferably 40 to 160°C or at the reflux temperature of the solvent for 30 minutes to 12 hours, preferably 1 to 6 hours to dehydrate and cyclize the glutaramide derivative (VII-1).

As the dehydrating agent, there may be mentioned, for example, thionyl chloride, phosphorus oxychloride, phosphorus pentachloride, polyphosphoric acid, etc.

(ii) After dissolving the glutaric anhydride derivative (a) in the above solvent, the amine compound (b) is added with an amount of 0.5 to 1.5 equivalent, preferably 0.7 to 1.2 equivalent based on 1 equivalent of the glutaric anhydride derivative (a). Then, the mixture is heated to 20 to 200°C, preferably 40 to 160°C for 0.5 to 48 hours, preferably 1 to 36 hours to conduct addition, dehydration and cyclization reaction.

(2) Scheme 2 shows the preparation method in which a glutaric anhydride derivative (a) is subjected to ring-opening treatment in the presence of an alcohol and a base, and the resulting glutarate derivative (c) is reacted with an amine compound (b) to obtain a glutaramide ester derivative (VII-2). Subsequently, a base or an acid is added to conduct cyclization treatment to prepare a glutarimide derivative (I-1) or the derivative is subjected to thiocarbonylation to obtain a derivative (VII-4). The thiocarbonylation reaction may be carried out using the thiocarbonylation agents and reaction conditions mentioned in the following Scheme 3.

The ring-opening treatment is subjected in a solvent and the solvent may include aliphatic hydrocarbons such as hexane, heptane, ligroine, petroleum ether, etc.; aromatic hydrocarbons such as benzene, toluene, xylene, chlorobenzene, etc.; halogenated hydrocarbons such as chloroform, methylene chloride, etc.; ethers such as diethyl ether, dioxane, tetrahydrofuran, etc.; ketones such as acetone, methyl ethyl ketone, etc.; nitriles such as acetonitrile, isobutyronitrile, etc.; tertiary amines such as pyridine, N,N-diethylaniline, etc.; acid amides such as formamide, N,N-dimethylformamide, N-methylpyrrolidone, etc.; sulfur-containing compounds such as dimethylsulfoxide, sulforane, etc.; water; and mixtures thereof. Among them, the above aliphatic hydrocarbons, aromatic hydrocarbons, ethers and mixtures thereof are preferred.

The base is used in an amount of 0.5 to 2.0 equivalent, preferably 0.8 to 1.2 equivalent based on 1 equivalent of the glutaric anhydride derivative (a).

As the base, there may be mentioned nitrogen-containing organic bases such as pyridine, triethylamine, 1,4-diazabicyclo[2.2.2]octane, etc.; inorganic bases such as sodium hydride, potassium hydride, sodium hydroxide, potassium hydroxide, sodium carbonate, potassium carbonate, etc.; metal alcoholates such as sodium methoxide, sodium ethoxide, potassium tert-butoxide, etc. Among them, sodium hydride, sodium hydroxide and potassium hydroxide are preferred.

The reaction temperature is -40 to 130°C, preferably -20 to 80°C or at the reflux temperature of the reaction mixture.

The reaction time requires 5 min to 4 hours, preferably 10 min to 2 hours.

The alcohol is used in an amount of 0.5 to 2.0 equivalent, preferably 0.8 to 1.2 equivalent based on 1 equivalent of the glutaric anhydride derivative (a). In the alcohol (R51-OH), there may be mentioned, $C_{1-8}$ alkyl especially $C_{1-6}$ alkyl, phenyl and benzyl as R51.

Subsequently, the resulting glutarate derivative (c) is reacted with 0.5 to 1.5 equivalent, preferably 0.7 to 1.2 equivalent based on 1 equivalent of an amine compound (b).

As the reaction method, after turning the compound (c) to acid chloride or active ester, the compound (b) is reacted thereto or the glutarate derivative (c) and the amine compound (b) are directly reacted by using a condensing agent.

For turning the glutarate derivative (c) to acid chloride, it is usual to use thionyl chloride, phosphorus oxychloride, phosgene, etc. As the active ester, it can be obtained by adding chloroformate to the glutarate derivative (c) in the presence of a base such as triethylamine and then reacting the amine compound (b) thereto. Also, as the condensing agent, there may be mentioned, for example, various condensing agent for peptide such as DCC, water-soluble DCC, CCBT, etc. The reaction temperature is -40 to 160°C, preferably -20 to 130°C and the reaction time is 1 to 48 hours, preferably 3 to 18 hours.

The resulting glutaramide ester (VII-2) is subjected to cyclization treatment in the presence of the above base or an acid including mineral acids such as sulfuric acid, phosphoric acid, etc., or organic acids such as acetic acid, trifluoroacetic acid, p-toluenesulfonic acid, etc. at 20 to 200°C, preferably 60 to 160°C for 1 to 24 hours.

(3) Scheme 3 shows the preparation method in which (i) the glutaramide derivative (VII-1) of Scheme 1 obtained by reacting the glutaric anhydride derivative (a) and the amine compound (b) is subjected to thiocarbonylation

to obtain a glutarthioamide derivative (VII-3), and the resulting glutarthioamide derivative (VII-3) is subjected to cyclization treatment to obtain a thiopyran derivative, and then the resulting derivative (d) is subjected to heat treatment, or is treated in the presence of a base or an acid to obtain a glutarthioimide derivative (I-2), or (ii) the glutarimide derivative (I-1) obtained in Schemes 1 and 2 is subjected to thiocarbonylation to obtain a glutarthioimide derivative (I-2).

(i) The thiocarbonylation agent is used in an amount of 0.3 to 1.0 equivalent, preferably 0.4 to 0.7 equivalent based on 1 equivalent of the glutaramide derivative (VII-1) to conduct thiocarbonylation treatment at 0 to 200°C, preferably 80 to 160°C for 0.5 to 24 hours in a solvent.

As the solvent, there may be mentioned aliphatic hydrocarbons such as hexane, heptane, ligroin, petroleum ether, etc.; aromatic hydrocarbons such as benzene, toluene, xylene, chlorobenzene, etc.; halogenated hydrocarbons such as chloroform, carbon tetrachloride, methylene chloride, etc.; ethers such as diethyl ether, dioxane, tetrahydrofuran, etc.; tertiary amines such as pyridine, N,N-diethylaniline, etc.; sulfur-containing compounds such as dimethylsulfoxide, sulforane, etc.; water and mixtures thereof. Among them, the above aliphatic hydrocarbons, armatic hydrocarbons, halogenated hydrocarbons, ethers, sulfur-containing compounds and mixtures thereof are preferred.

As the thiocarbonylation agent, there may be mentioned $P_2S_5$, $SiS_2$, $B_2S_3$, Lawesson's reagent, etc.

The resulting glutarthioamide derivative (VII-3) is subjected to cyclization treatment at 0 to 120°C, preferably 20 to 80°C for 1 to 18 hours, then the resulting thiopyran derivative (d) is① treated at 0 to 120°C, preferably 20 to 80°C for 0.5 to 12 hours in the presence of a base or an acid and if necessary a solvent, or② heat treated at 60 to 300°C, preferably 100 to 250°C for 5 min to 4 hours to prepare a glutarthioimide derivative (I-2).

As the base, there may be mentioned secondary amines such as dimethylamine, diethylamine, etc., inorganic bases such as sodium hydride, potassium hydride, sodium hydroxide, potassium hydroxide, sodium carbonate, potassium carbonate, etc.; metal alcoholates such as sodium methoxide, sodium ethoxide, potassium tert-butoxide, etc., metal alkylmercaptides such as sodium methyl mercaptide. Among them, the secondary amines such as dimethylamine and metal alcoholates such as sodium methoxide are preferred.

As the acid, there may be mentioned mineral acids such as hydrochloric acid, phosphoric acid, sulfuric acid, etc., or organic acids such as methane sulfonic, acetic acid, trifluoroacetic acid, trifluoromethane sulfonic acid, p-toluenesulfonic acid, etc.

(ii) The glutarimide derivative (I-1) is subjected to thiocarbonylation treatment by using the above thiocarbonylating agent in an amount of 0.3 to 1.0 equivalent, preferably 0.4 to 0.7 equivalent based on 1 equivalent of the glutarimide derivative (I-1) at 0 to 200°C, preferably 80 to 160°C for 0.5 to 24 hours in the above solvents.

The compound of the present invention has potent weed-killing activity with a low chemical dosage in either of treatments of soil treatment and foliage treatment against broad-leaved weeds, of *Solanaceae* weeds such as *Solanum nigrum, Datura stramonium,* etc., *Malvaceae* weeds such as *Abutilon theophrasti, Side spinosa,* etc., *Convolvulaceae* weeds such as *Ipomoea* spps. including *Ipomoea purpurea,* etc. and *Calystegia* spps., etc., *Amaranthaceae* weeds such as *Amaranthus liividus, Amaranthus retroflexus,* etc., *Compositae* weeds such as *Xanthium pensylvanicum, Ambrosia artemisiaefolia, Helianthus annuu, Galinsoga ciliat, Crisium arvense, Senecio vulgaris, Erigeron annus,* etc., *Cruciferae* weeds such as *Rorippa indica, Sinapis arvensis, Capsella Bursapastris,* etc., *Polygonaceae* weeds such as *Polygonum Blumei, Polygonum convolvulus,* etc. *Portulacaceae* weeds such as *Portulaca oleracea,* etc., *Chenopodiaceae* weeds such as *Chenopodium album, Chenopodium ficifolium, Kochia scoparia,* etc., *Caryophyllaceae* weeds such as *Stellaria media,* etc., *Scrophulariaceae* weeds such as *Veronica persica,* etc., *Cimmekubaceae* weeds such as *Commelina communis,* etc., *Labiatae* weeds such as *Lamium amplexicaule, Lamium purpureum,* etc., *Euphorbiaceae* weeds such as *Euphorbia supina, Euphorbia maculata,* etc., *Rubiaceae* weeds such as *Galium spurium, Galium aparine, Rubia akane,* etc., *Violaceae* weeds such as *Viola arvensis,* etc., and *Leguminosae* weeds such as *Sesbania exaltata, Cassia obtusifolia,* etc.,; and various cropland weeds of *Graminaceous* weeds such as *Sorgham bicolor, Panicum dichotomiflorum, Sorghum halepense, Echinochloa crus-galli, Digitaria adscendens, Avena fatua, Eleusine indica, Setaria viridis, Alopecurus aegualis,* etc., and *Cyperaceous* weeds such as *Cyperus rotundus, Cyperus esculentus,* etc.

Also, as a herbicide for paddy field, the compound of the present invention has potent weed-killing activity with a low chemical dosage in either of treatments of soil treatment under pouring water and foliage treatment against various paddy weeds of *Alismataceae* weeds such as *Alisma canaliculatum, Sagittaria trifolia, Sagittaria pygmaea,* etc., *Cyperaceae* weeds such as *Cyperus difformis, Cyperus serotinus, Scirpus juncoides, Eleocharis kuroguwai,* etc., *Scrothulariaceae* weeds such as *Lindemia pyxidaria, Potenderiaceae* weeds such as *Monochoria vaginalis,* etc., *Potamogetonaceae* weeds such as *Potamogeton distinctus,* etc., *Lythraceae* weeds such as *Rotala indica,* etc., *Gramineae* weeds such as *Echinochloa crus-galli,* etc. The compound shows potent weed-killing power particularly against *Gramineae* weeds with a low chemical dosage.

Also, the compound shows no phytotoxicity to rice, beet, wheat barley, sorghum, peanut, corn, soybean,

cotton, etc, which are important crop plants.

It is the greatest characteristic feature of the compound of the present invention that it has prompt and potent herbicidal activity with extremely low chemical dosage as compared with the conventional herbidices and can be applied by either of the methods of the soil treatment and foliage treatment against various kinds of weeds.

On the other hand, the compound of the present invention shows prompt and potent herbicidal acticity with extremely low chemical dosage as compared with the conventional herbicides so that some of them are available as a herbicide for an orchard, a pasture, a lawn or a non-arable land.

For applying the compound of the present invention as a herbicide, in general, it can be applied by mixing with a suitable carrier, for example, a soild carrier such as clay, talc, bentonite, diatomaceous earth, white carbon, etc,; or a liquid carrier such as water, an alcohol (isopropanol, butanol, benzyl alcohol, furfuryl alcohol, etc.), an aromatic hydrocarbon (toluene, xylene, etc.), an ether (anisol, etc.), a ketone (cyclohexanone, isophorone, etc.), an ester (butyl acetate, etc.), an acid amide (N-methylpyrrolidone, etc.) or a halogenated hydrocarbon (chlorobenzene, etc.). If necessary, a surfactant, an emulsifier, a dispersant, a penetrating agent, a spreader, a thicker, an antifreezing agent, a coagulation preventing agent, a stabilizer, etc. may be added and it can be practically applied in the optional form such as a liquid formulation, an emulsifiable formulation, a wettable power, a flowable, dry flowable, dust, granule, etc.

In a herbicidal composition of the present invention, an amount of an active ingredient of the glutarimide derivatives and glutaric acid amide derivatives of the present invention is in the range of 0.1 to 90 parts by weight and an amount of a herbicidal acceptable carrier or diluent is in the range of 10 to 99.9 parts by weight, based on 100 parts by weight of the herbicidal composition.

More particularly, preferable composition ratios (based on 100 parts by weight of the herbicidal composition) of the glutarimide derivatives and/or glutaric acid amide derivatives of the present invention in each formulation are set forth below.

Wettable Powder

Compound of the present invention -- 5 to 80 parts

Compound of the present invention -- 5 to 80 parts

Solid carrier -------------------- 10 to 85 parts

Surfactant ----------------------- 1 to 10 parts

Others --------------------------- 1 to 5 parts

Emulsifiable concentrate

Compound of the present invention -- 1 to 30 parts

Liquid carrier ------------------- 30 to 95 parts

Surfactant ----------------------- 5 to 15 parts

Flowable

Compound of the present invention -- 5 to 70 parts

Liquid carrier ------------------- 15 to 65 parts

Surfactant ----------------------- 5 to 12 parts

Others --------------------------- 1 to 30 parts

As others, there may be mentioned, for example, antifreezing agents, thicker, etc.

Granule

Compound of the present invention -- 0.1 to 10 parts

Solid carrier --------------------- 90 to 99.99 parts

Others ---------------------------- 1 to 5 parts

Granular Wettable Powder (Dry Flowable)

Compound of the present invention - 20 to 90 parts

Solid carrier --------------------- 10 to 60 parts

Surfactant ------------------------ 1 to 20 parts

Also, if necessary, the compound of the present invention may be applied by mixing with other kinds of herbicides, various insecticides, fungicides, plant growth regulating agents, synergism agents, antidotes, etc. when spreading or formulating.

By mixing with other herbicides and applying, particularly excellent effects such as reduction in cost by decreasing an applied dosage, increase in weed-killing spectrum due to synergistic effect of the mixed chemicals and higher weed-killing effect can be expected. At this time, the compound may be combined with a plural number of known herbicides simultaneously. Preferred chemicals to be mixedly used in combination with the compound of the present invention may include herbicides such as bentazone (3-isopropyl-1H-2,1,3-benzothiadiazin-4(3H)-one 2,2-dioxide), acifluorfen-sodium (sodium 5-[2-chloro-4-(trifluoromethyl)phenoxy]-2-nitrobenzoate), fomesafen (5-[2-chloro-4-(trifluoromethyl)phenoxy]-N-methylsulfonyl-2-nitrobenzamide), lactofen (1′-(carboethoxy)ethyl-5-[2-chloro-4-(trifluoromethyl)phenoxy]-2-nitrobenzoate), metribuzin (4-amino-6-tert-butyl-3-methylthio-1,2,4-triazin-5(4H)one), imazaquin (2-(4-isopropyl-4-methyl-5-oxo-2-imidazolin-2-yl)quinoline-3-carboxylic acid), sethoxydim (2-(1-ethoxy-iminobutyl)-5-[2-(ethylthio)propyl]-3-hydroxycyclohex-2-enone), imazethapyr (5-ethyl-2-(4-isopropyl-4-methyl-5-oxo-2-imidazolin-2-yl)nicotinic acid), cycloxydim (2-[1-(ethoxyimino)butyl]-3-hydroxy-5-thian-3-ylcyclohex-2-enone), linuron (3-(3,4-dichlorophenyl)-1-methoxy-1-methyl urea), quizalofop-ethyl (ethyl-2-[4-(6-chloro-2-quinoxanyloxy)phenoxy]propionate), dichlofop-methyl (methyl-2-[4-(2,4-dichlorophenoxy)phenoxy]propionate), fluazifop-butyl (butyl-2-[4-(5-trifluoromethyl-2-pyridyloxy)phenoxypropionate), fenoxaprop-ethyl (ethyl-2-[4-(6-chloro-2-benzoxazolyloxy)phenoxy]propionate), haloxyfop-methyl (methyl-2-[4-(3-chloro-5-trifluoromethyl-2-pyridyloxy)phenoxy]propionate), toxaphene (a reaction mixture of chlorinated camphene containing 67 to 69% chlorine), alachlor (2-chloro-2′,6′-diethyl-N-methoxymethyl-acetanilide), metolachlor (2-chloro-N-(2-ethyl-6-methyl-phenyl)-N-(2-methoxy-1-methylethyl)acetamide), naptalam (N-1-naphtylphthalamic acid), 2,4-DB (4-(2,4-dichlorophenoxy)butyric acid), MCPB (4-(4-chloro-2-methylphenoxy)butyric acid), chlorimuronethyl (ethyl-2-[3-(4-chloro-6-methoxypyrimidin-2-yl)ureidosulfonyl]benzoate), dimethazone (2-(2-chlorophenyl)methyl-4,4-dimethyl-3-isoxazolidinone), etc. and other kinds of herbicides, there may be mentioned the compounds disclosed in, for example, Farm Chemicals Handbook, 1989.

A dosage to be applied may vary depending on the place to be applied, time to be applied, method of application, cultivated crops, etc., but 0.001 to 10 kg per hectare as an effective component dosage is suitable.

The glutarimide derivatives and glutaric acid amide derivatives of the present invention have an excellent penetrative translocation activity and a very high herbicidal activity at a very low dosage, and show no phytotoxicity against the important crop plants, and can be applied through either soil treatment or soil incorporation treatment against a wide variety of weeds. Especially the glutarimide derivatives and glutaric acid amide derivatives of the present invention have particularly potent selective herbicidal effect against weeds of *Gramineae* with low dosage, without showing phytotoxicity to rice, wheat and barley.

In the following, synthesis examples of the compound of the present invention are described more specifically as examples, but the present invention is not limited by these.

## EXAMPLES

### Example 1-1

Synthesis of N-(4-chloro-2-fluoro-5-isopropoxycarbonylphenyl)-2-methyl-3-trifluoromethylglutarimide

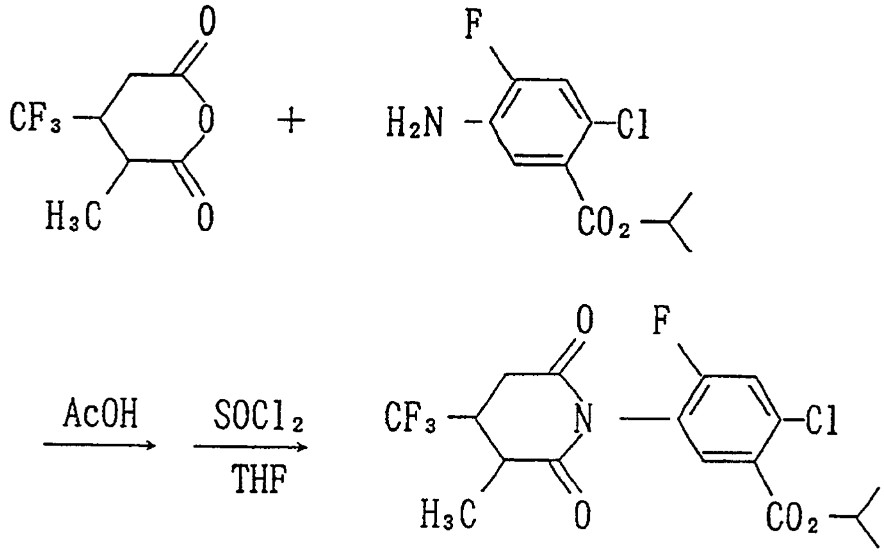


In 10 ml of acetic acid were dissolved 1 g of 2-methyl-3-trifluoromethylglutaric acid anhydride and 1 g of isopropyl 5-amino-2-chloro-4-fluorobenzoate and the mixture was stirred at 100°C for 5 hours. After cooling to room temperature, the mixture was poured into water and extracted twice with ethyl acetate, and the extract was washed with a saturated saline solution and dried over anhydrous sodium sulfate. The solvent was removed and the resulting viscous product was dissolved in 5 ml of tetrahydrofuran and then 0.3 g of thionyl chloride was added dropwise to the solution at room temperature. After stirring for 2 hours under reflux, the reaction mixture was cooled to room temperature and the solvent was removed under reduced pressure. The resulting crude product was purified by preparative thin-layer chromatography (eluent: chloroform) to obtain 0.08 g of the title compound as an oily product.

### Example 1-2

Synthesis of N-(7-fluoro-2H-1,4-benzoxazin-3(4H)-on-6-yl)-3-methylglutarimide

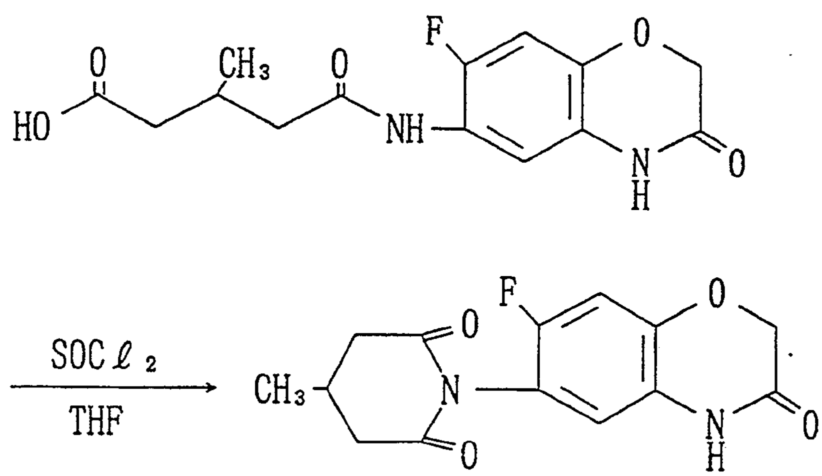


To a solution of 0.5 g of 3-methyl-4-(N-(7-fluoro-2H-1,4-benzoxazin-3(4H)-on-6-yl)carbamoyl) butyric acid and 10 ml of tetrahydrofuran was added dropwise 0.35 g of thionyl chloride at room temperature. After stirring for 2 hours under reflux, the reaction mixture was cooled to room temperature and the solvent was removed

under reduced pressure. The residue was extracted with ethyl acetate and the extract was successively washed with water and a saturated saline solution in this order, dried over anhydrous sodium sulfate, followed by removal of ethyl acetate to obtain 0.44 g of the title compound as crystals.

Example 1-3

Synthesis of N-(7-fluoro-4-propargyl-2H-1,4-benzoxazin-3(4H)-on-6-yl)-3-methylglutarimide

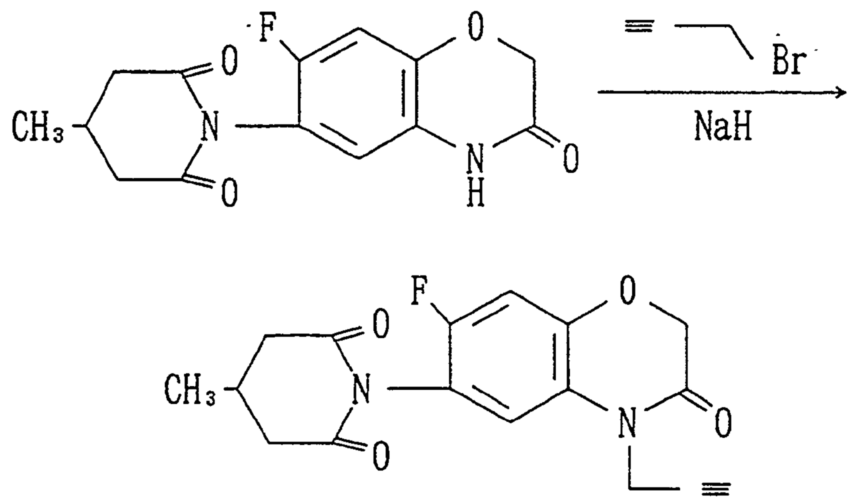


A solution of 0.063 g of a 55% sodium hydride and 5 ml of N,N-dimethylformamide was cooled to 0°C, and to the solution was added dropwise a solution of 0.4 g of N-(7-fluoro-2H-1,4-benzoxazin-3(4H)-on-6-yl)-3-methylglutarimide dissolved in 5 ml of N,N-dimethylformamide and the mixture was stirred at 0°C to 5°C for 30 minutes. To the mixture was added 0.18 g of propargyl bromide and the mixture was stirred at room temperature for one hour. After completion of the reaction, the reaction mixture was poured into water and extracted with ethyl acetate, and the extract was washed with a saturated saline solution and dried over anhydrous sodium sulfate. Then, ethyl acetate was removed and the resulting crude product was washed with diisopropyl ether to obtain 0.2 g of the title compound as crystals.

Example 1-4

Synthesis of N-(4-chloro-2-fluoro-5-isopropoxycarbonylphenyl)-3-isopropyl-2-methylglutarimide

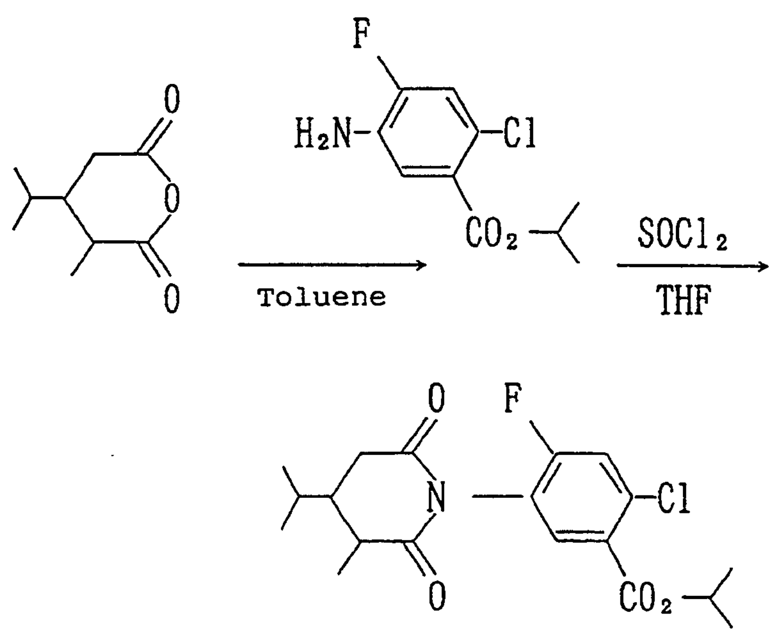

In 7 ml of toluene were dissolved 0.37 g of 3-isopropyl-2-methylglutaric acid anhydride and 0.5 g of isopropyl 5-amino-2-chloro-4-fluorobenzoate and the mixture was stirred at 100°C for 6 hours. After removing the solvent under reduced pressure, 6 ml of tetrahydrofuran was added to the residue and 0.3 g of thionyl chloride was added dropwise at room temperature. After stirring for 4 hours under reflux, the reaction mixture was cooled to room temperature and the solvent was removed under reduced pressure. The resulting crude product was purified by preparative thin layer chromatography (eluent: chloroform : hexane = 1 : 1) to obtain 0.06 g of the title compound as an oily product.

Example 1-5

Synthesis of N-(4-chloro-2-fluorophenyl)-3,3-dimethylglutarimide

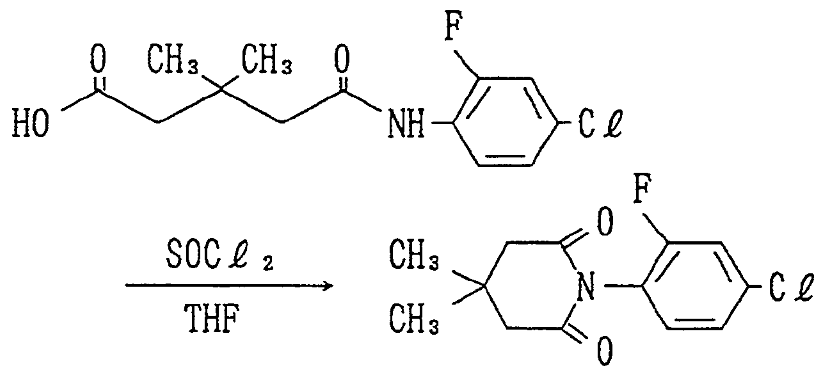


In the same manner as in Example 1-2, from 1 g of 3,3-dimethyl-4-(N-(4-chloro-2-fluorophenyl)carbamoyl)butyric acid, 0.57 g of the title compound was obtained as crystals.

Example 1-6

Synthesis of N-(4-chloro-2-fluorophenyl)-2,2-dimethylglutarimide

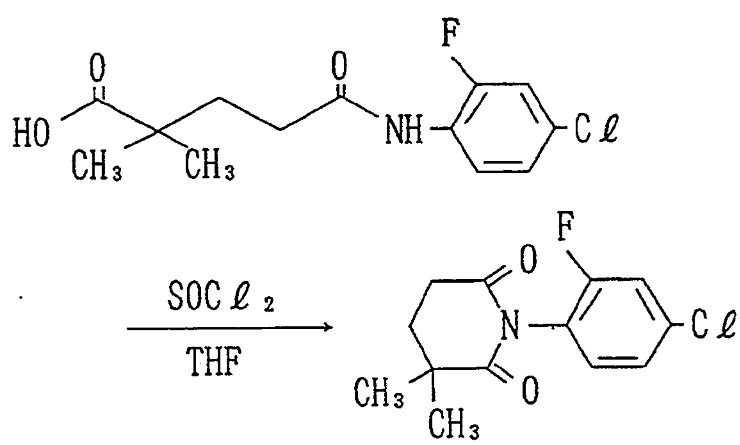


In the same manner as in Example 1-2, from 1 g of 2,2-dimethyl-4-(N-(4-chloro-2-fluorophenyl)carbamoyl)butyric acid, 0.49 g of the title compound was obtained as crystals.

Example 1-7

Synthesis of N-(7-fluoro-4-propargyl-2H-1,4-benzoxazin-3(4H)-on-6-yl)-3-trifluoromethylglutarimide

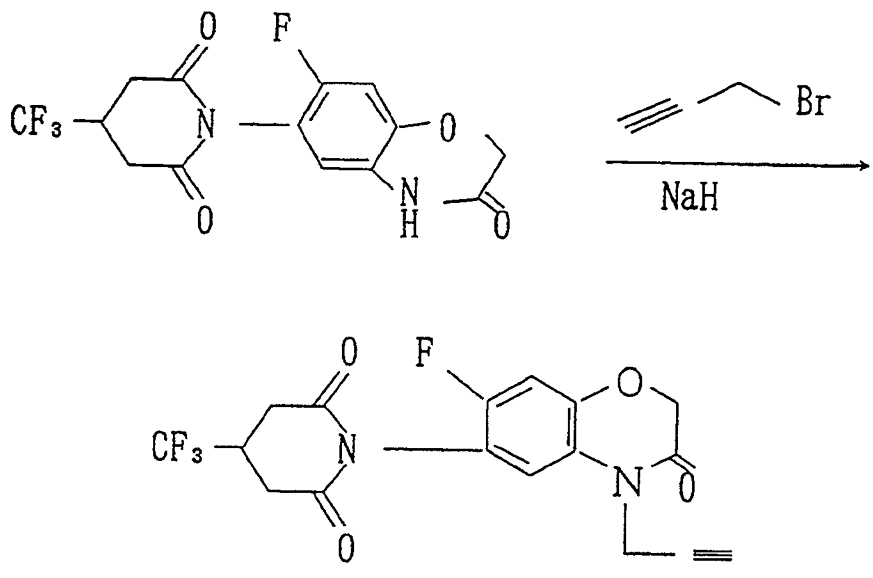


In the same manner as in Example 1-3, from 0.6 g of N-(7-fluoro-2H-1,4-benzoxazin-3(4H)-on-6-yl)-3-trifluoromethylglutarimide, 0.11 g of the title compound was obtained as crystals.

Example 1-8

Synthesis of N-(4-chloro-5-ethoxycarbonyl-2-fluorophenyl)-3-phenylglutarimide

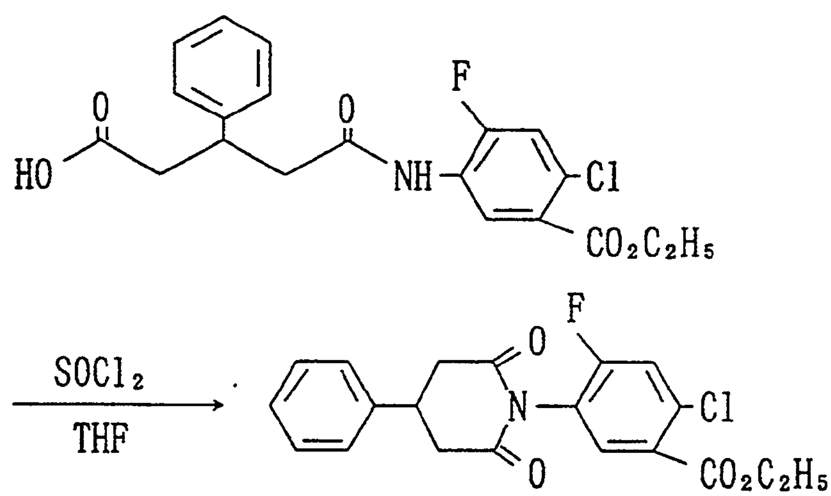


In the same manner as in Example 1-1, from 0.8 g of 3-phenyl-4-(N-(4-chloro-5-ethoxycarbonyl-2-fluorophenyl)carbamoyl)butyric acid, 0.31 g of the title compound was obtained as crystals.

Example 1-9

Synthesis of N-(4-chloro-5-ethoxycarbonyl-2-fluorophenyl)-2-methylglutarimide

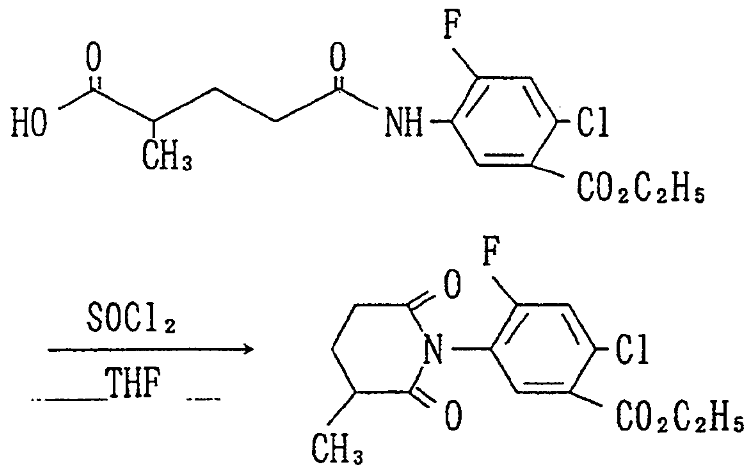


In the same manner as in Example 1-1, from 0.73 g of 2-methyl-4-(N-(4-chloro-5-ethoxycarbonyl-2-fluorophenyl)carbamoyl)butyric acid, 0.43 g of the title compound was obtained as oily product.

Example 1-10

Synthesis of N-(4-chloro-5-ethanesulfonylamino-2-fluorophenyl)-3-trifluoromethylglutarimide

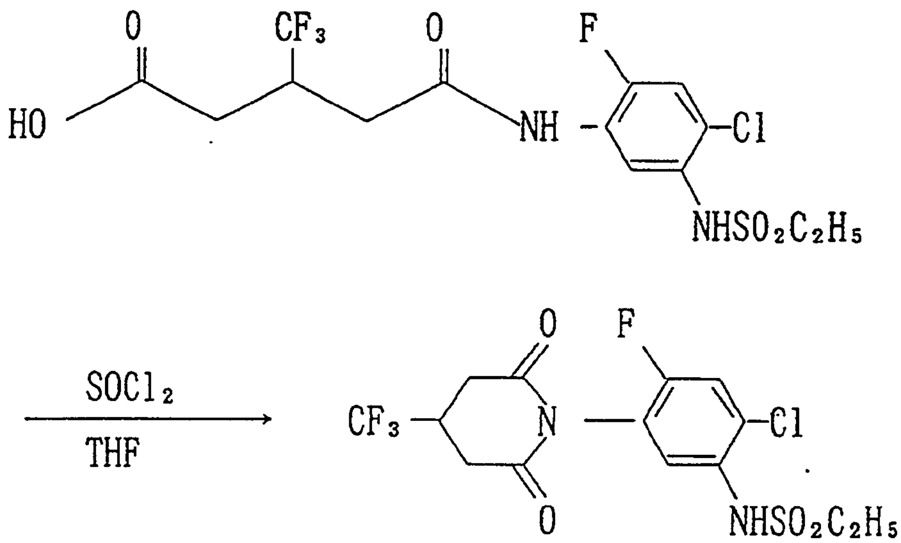


In the same manner as in Example 1-2, from 0.6 g of 3-trifluoromethyl-4-(N-(4-chloro-5-ethanesulfonylamino-2-fluorophenyl)carbamoyl)butyric acid, 0.43 g of the title compound was obtained as crystals.

Example 1-11

Synthesis of N-(4-chloro-5-ethoxycarbonylamino-2-fluorophenyl)-3-trifluoromethylglutarimide

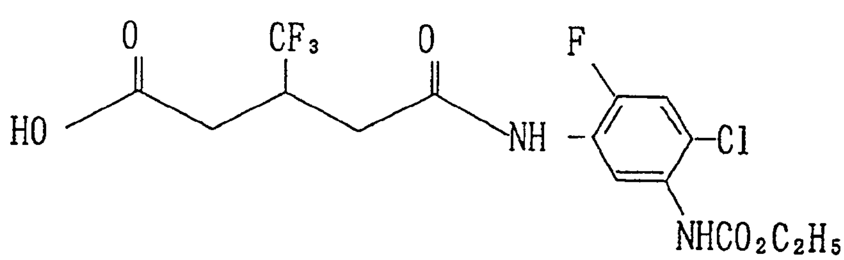

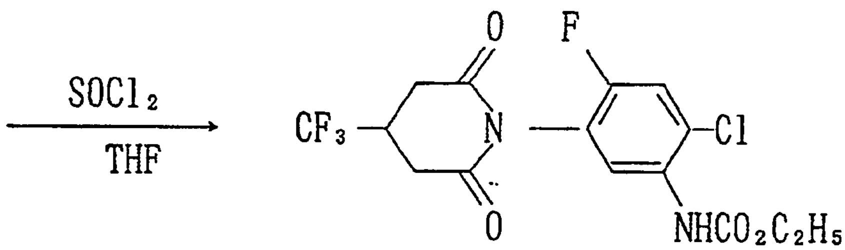


In the same manner as in Example 1-2, from 1.12 g of 3-trifluoromethyl-4-(N-(4-chloro-5-ethoxycarbonylamino-2-fluorophenyl)carbamoyl)butyric acid, 0.71 g of the title compound was obtained as crystals.

Example 1-12

Synthesis of N-(4-chloro-2-fluoro-5-(5-methyl-1,3,4-oxadiazol-2-yl)phenyl)-3-trifluoromethylglutarimide

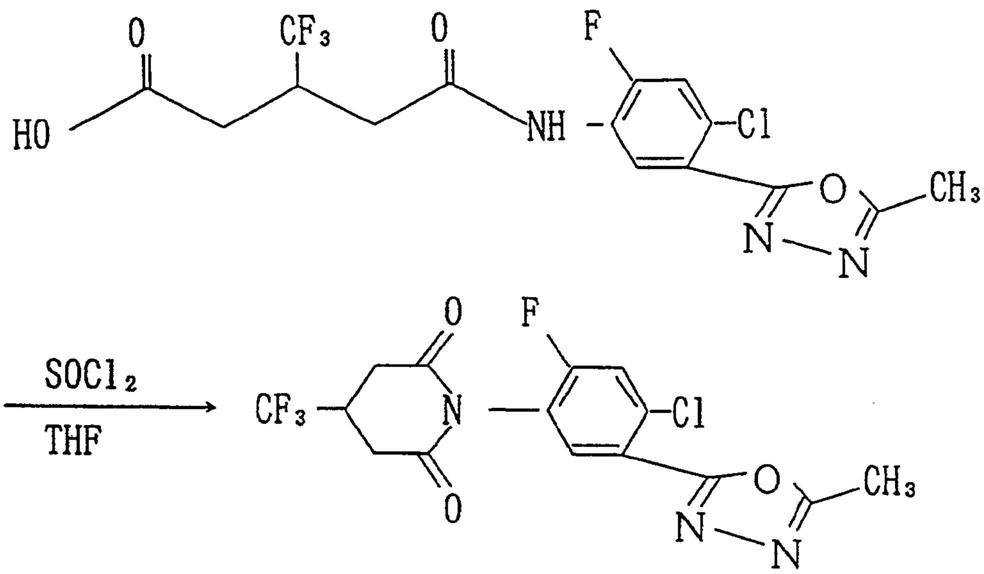


In the same manner as in Example 1-2, from 1.38 g of 3-trifluoromethyl-4-(N-(4-chloro-2-fluoro-5-(5-methyl-1,3,4-oxadiazol-2-yl)phenyl)carbamoyl)butyric acid, 0.42 g of the title compound was obtained as glass state product.

Example 1-13

Synthesis of N-(4-chloro-2-fluoro-5-(1-methyl-6-tri-fluoromethyl-2,4(1H,3H)-pyrimidinedion-3-yl)phenyl)-3-trifluoromethylglutarimide

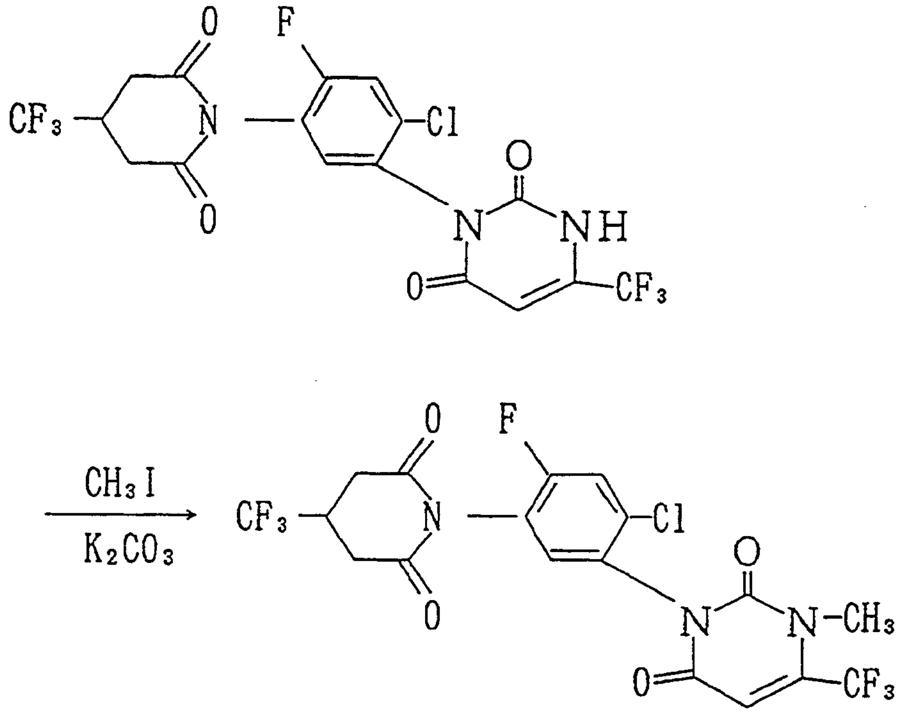


To the mixture of 0.37 g of N-(4-chloro-2-fluoro-5-(6-trifluoromethyl-2,4(1H,3H)-pyrimidinedion-3-yl)phenyl)-3-trifluoromethylglutarimide, 0.11 g of anhydrous potassium carbonate and 5 ml of N,N-dimethylformamide was added dropwise 0.11 g of methyl iodide and the mixture was stirred at room temperature overnight. After completion of the reaction, the reaction mixture was poured into water, extracted twice with ethyl acetate, washed with water and then with a saturated saline solution, and then dried over anhydrous sodium sulfate. Then, ethyl acetate was removed to obtain 0.11 g of the title compound as crystals.

Example 1-14

Synthesis of N-(2,4-difluoro-5-(1-methyl-6-trifluoromethyl-2,4(1H,3H)-pyrimidinedion-3-yl)phenyl)-3-trifluoromethylglutarimide

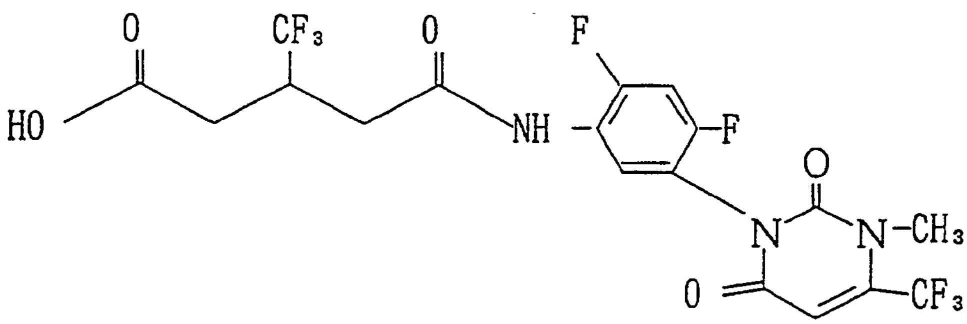

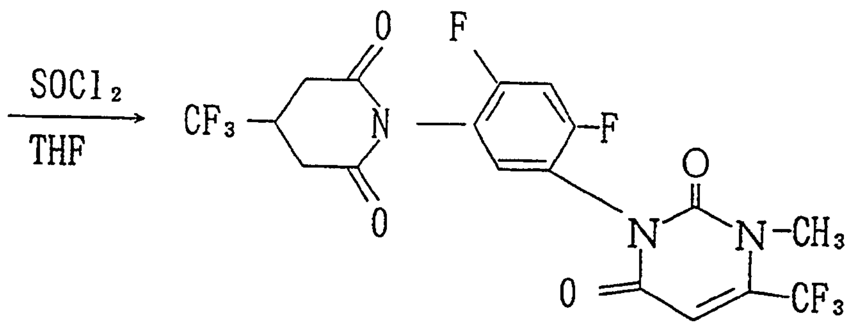


In the same manner as in Example 1-2, from 0.78 g of 3-trifluoromethyl-4-(N-(2,4-difluoro-5-(1-methyl-6-trifluoromethyl-2,4(1H,3H)-pyrimidinedion-3-yl)phenyl)carbamoyl)butyric acid, 0.61 g of the title compound was obtained as crystals.

Example 1-15

Synthesis of N-(4-chloro-2-fluoro-5-(6-trifluoromethyl-2,4(1H,3H)-pyrimidinedion-3-yl)phenyl)-3-trifluoromethylglutarimide

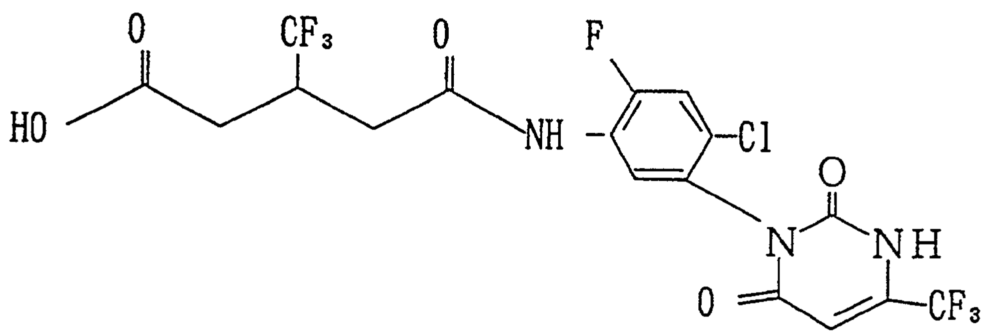

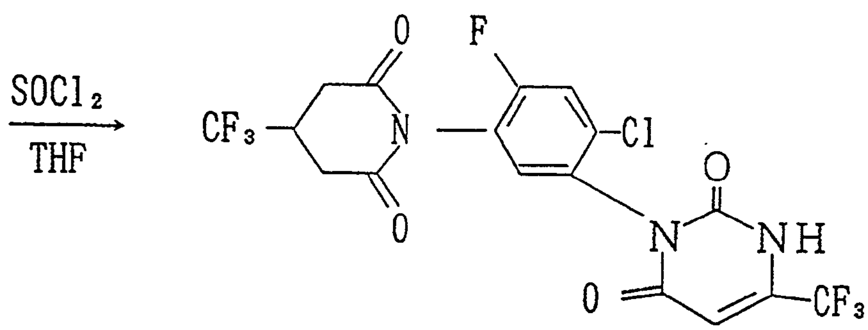


In the same manner as in Example 1-2, from 0.7 g of 3-trifluoromethyl-4-(N-(4-chloro-2-fluoro-5-(6-trifluoromethyl-2,4(1H,3H)-pyrimidinedion-3-yl)phenyl)carbamoyl)butyric acid, 0.41 g of the title compound was obtained as crystals.

Example 1-16

Synthesis of N-(4-chlorophenyl)methyl-3-trifluoromethylglutarimide

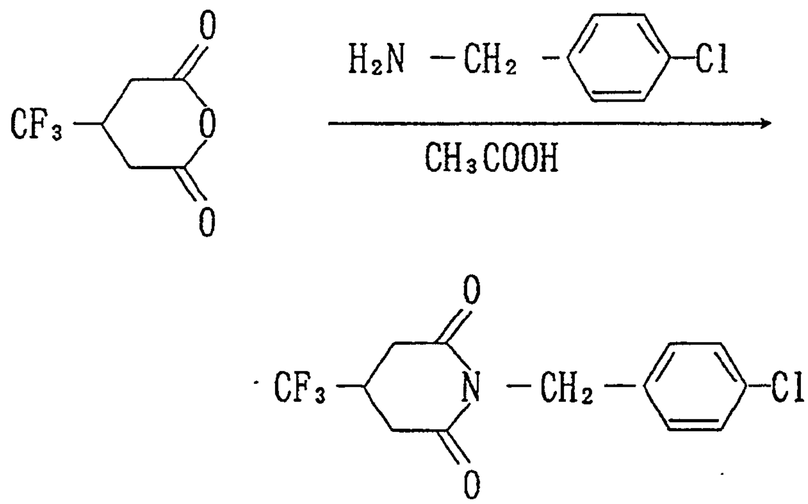


The mixture of 0.5 g of 3-trifluoromethylglutaric acid anhydride, 0.39 g of p-chlorobenzylamine and 5 ml of acetic acid was stirred under reflux for 4 hours. After cooling to room temperature, the reaction mixture was poured into water, and crystals precipitated were collected by filtration and washed to obtain 0.58 g of the title compound as crystals.

Next, Compounds numbers synthesized by the above examples or those in accordance with Schemes 1 to 3 and the structures thereof are shown in Table 1-1 and physical properties of these compounds in Table 1-2.

Table 1-1

| Compound No. | Structure |
|---|---|
| 1-1 | 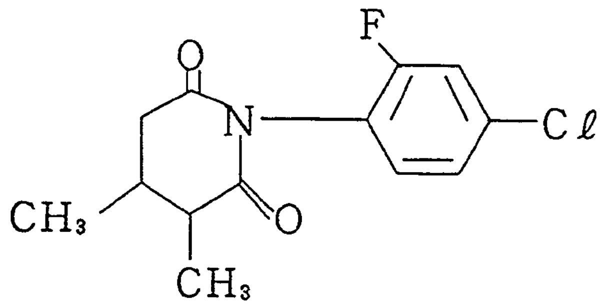 |
| 1-2 | 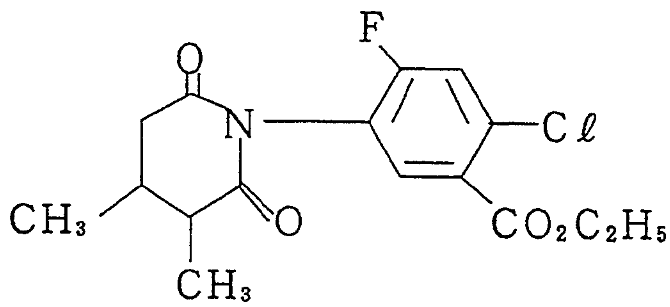 |
| 1-3 | 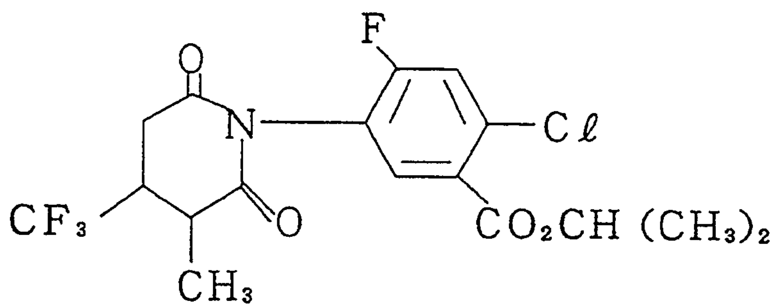 |

Table 1-1 (Cont' d)

| Compound No. | Structure |
|---|---|
| 1-4 | 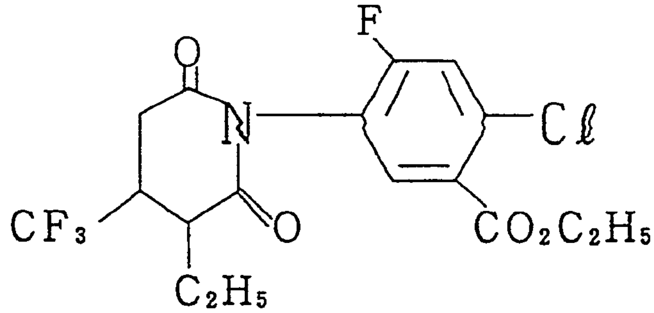 |
| 1-5 | 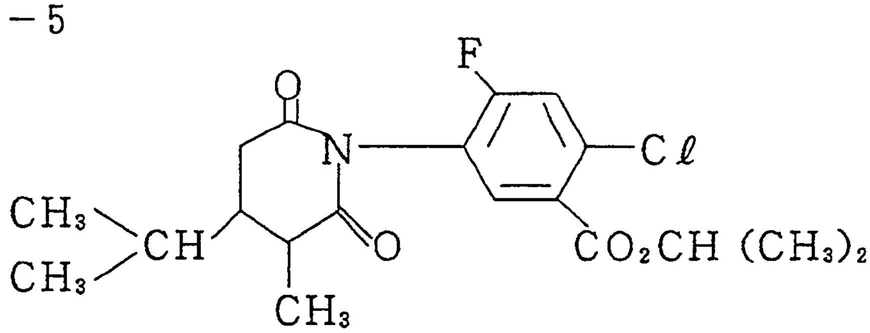 |
| 1-6 | 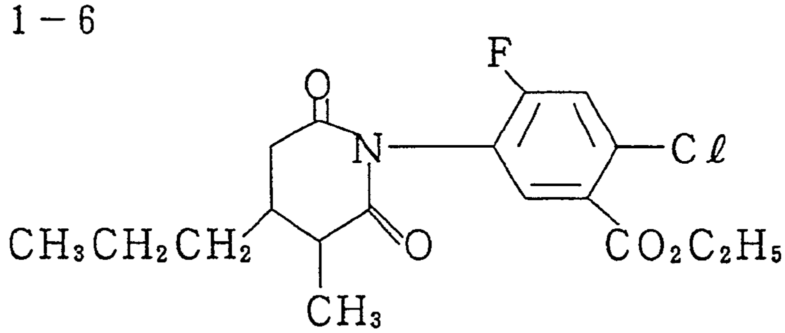 |

Table 1-1 (Cont' d)

| Compound No. | Structure |
|---|---|
| 1-7 | 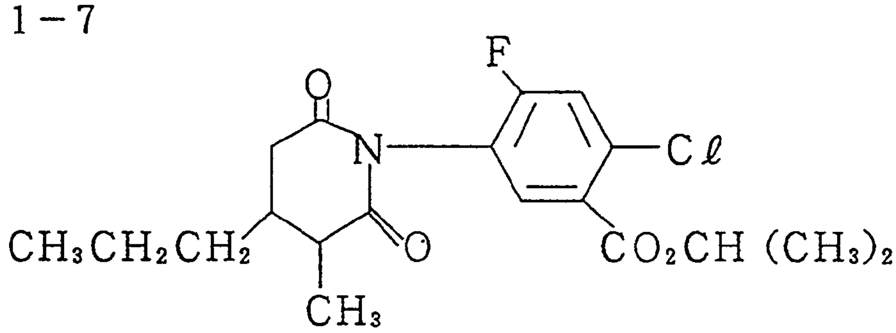 |
| 1-8 | 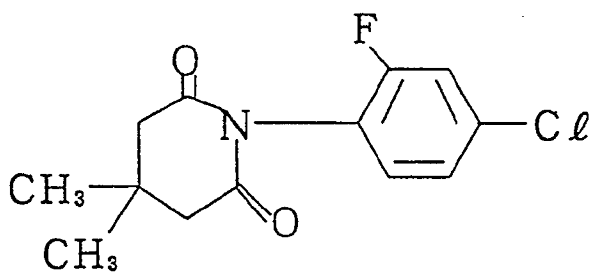 |

Table 1-1 (Cont'd)

| Compound No. | Structure |
|---|---|
| 1-9 | 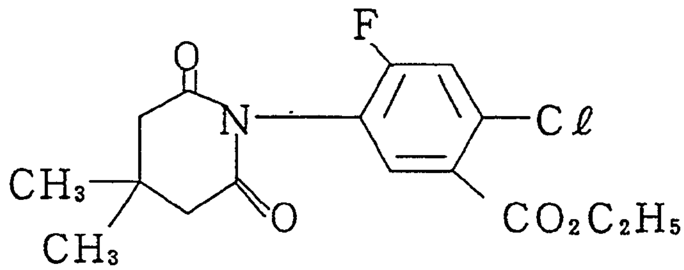 |
| 1-10 | 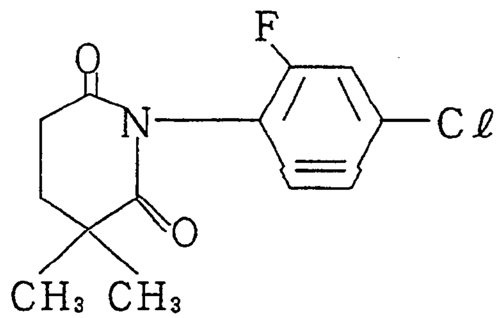 |
| 1-11 | 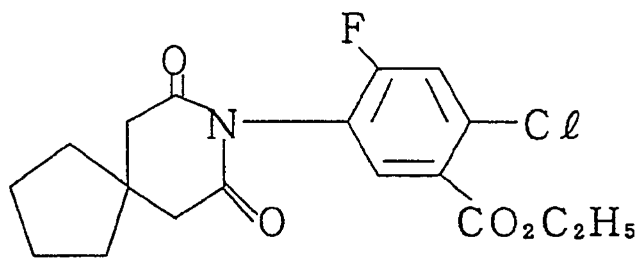 |

Table 1-1 (Cont' d)

| Compound No. | Structure |
|---|---|
| 1-12 | 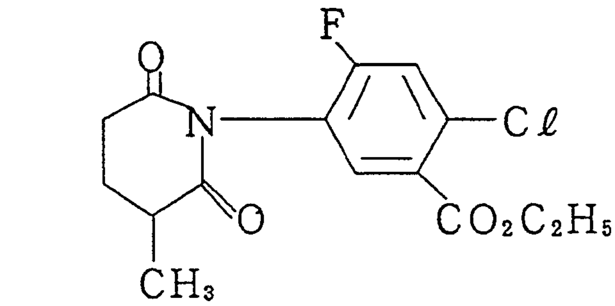 |
| 1-13 | 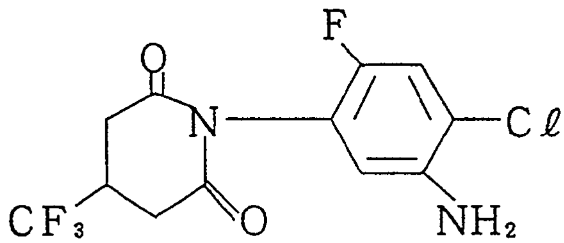 |
| 1-14 | 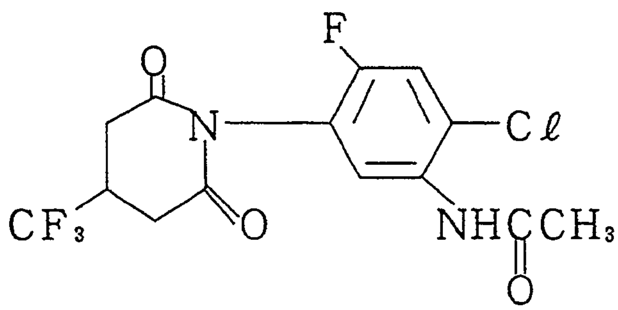 |

Table 1-1 (Cont' d)

| Compound No. | Structure |
|---|---|
| 1-15 | 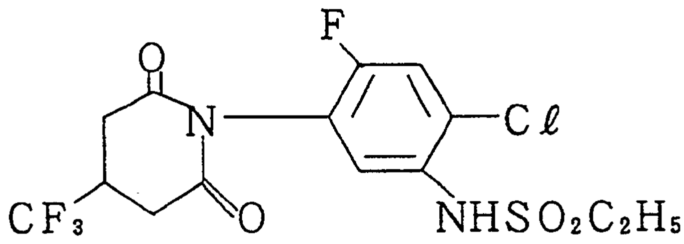 |
| 1-16 | 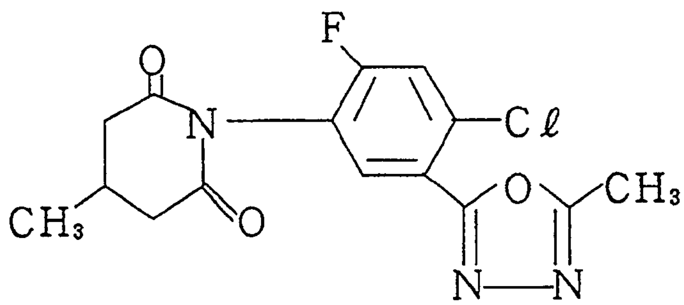 |

Table 1-1 (Cont'd)

| Compound No. | Structure |
|---|---|
| 1-17 | 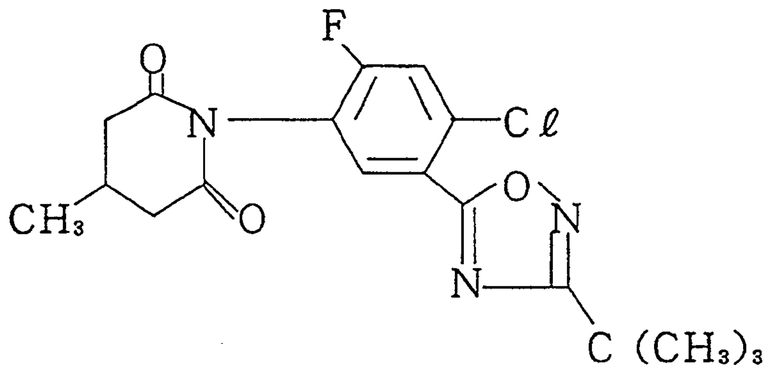 |
| 1-18 | 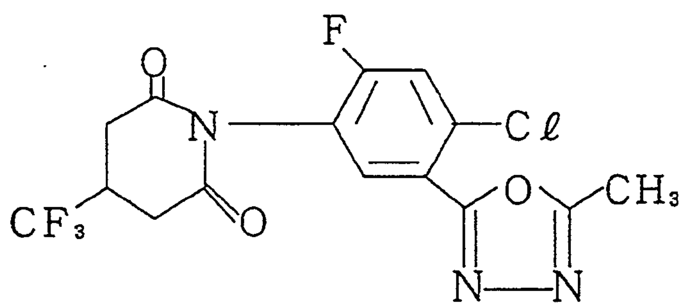 |
| 1-19 | 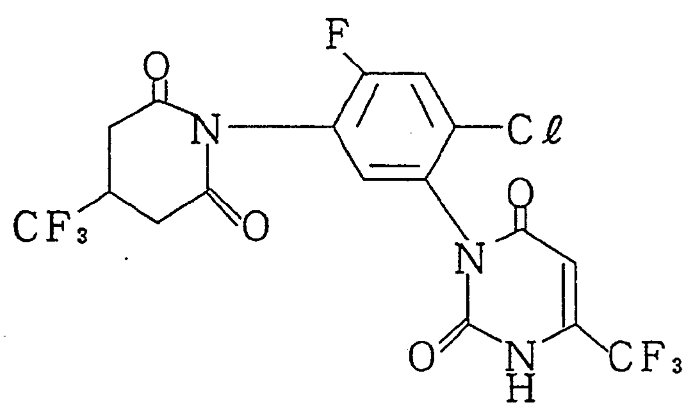 |

Table 1-1 (Cont' d)

| Compound No. | Structure |
| --- | --- |
| 1-20 | 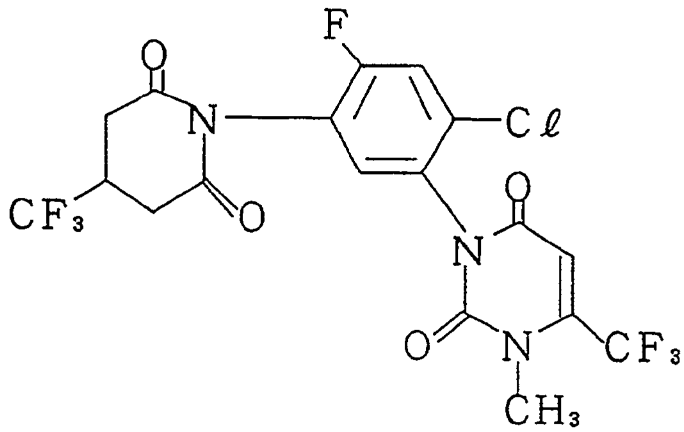 |
| 1-21 | 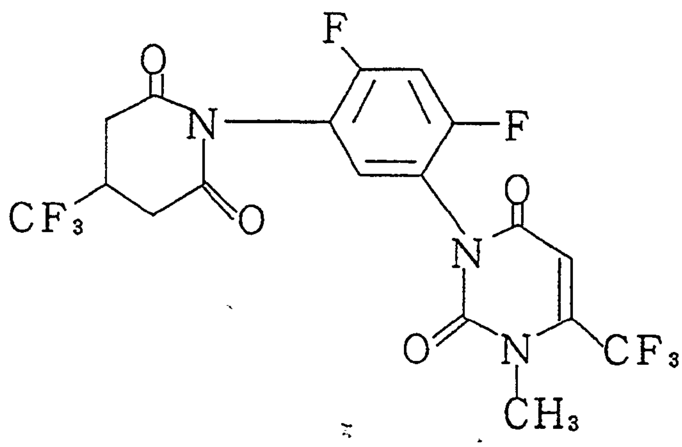 |
| 1-22 | 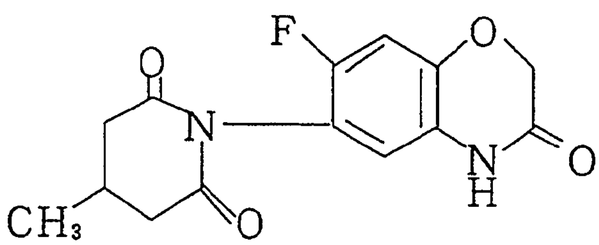 |

Table 1-1 (Cont' d)

| Compound No. | Structure |
|---|---|
| 1-23 | 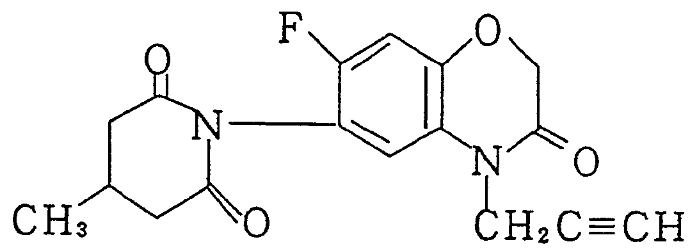 |
| 1-24 | 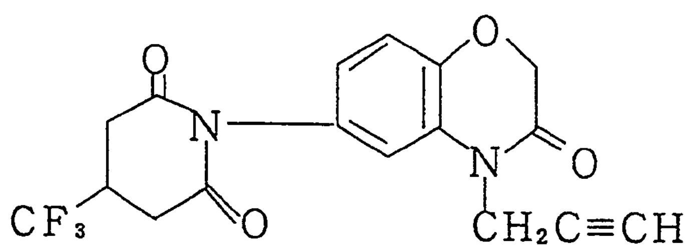 |
| 1-25 | 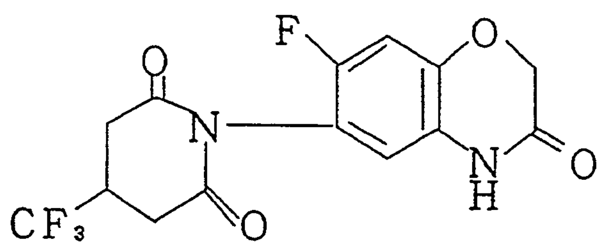 |

Table 1-1 (Cont' d)

| Compound No. | Structure |
| --- | --- |
| 1-26 | 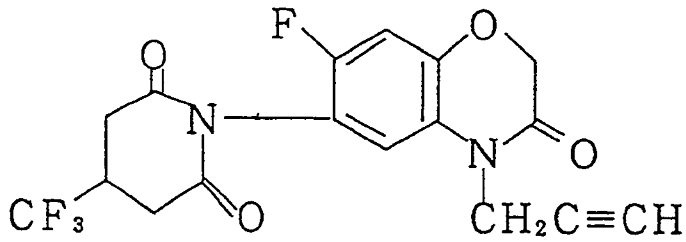 |
| 1-27 | 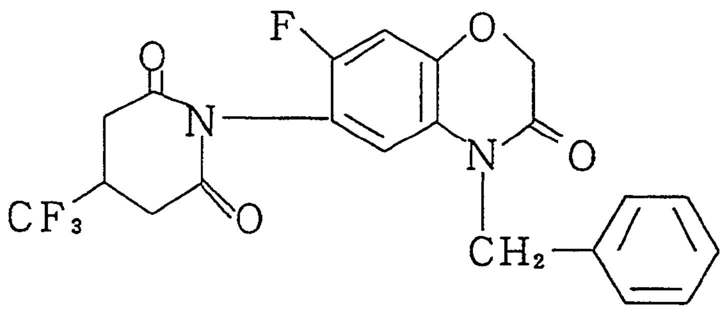 |
| 1-28 | 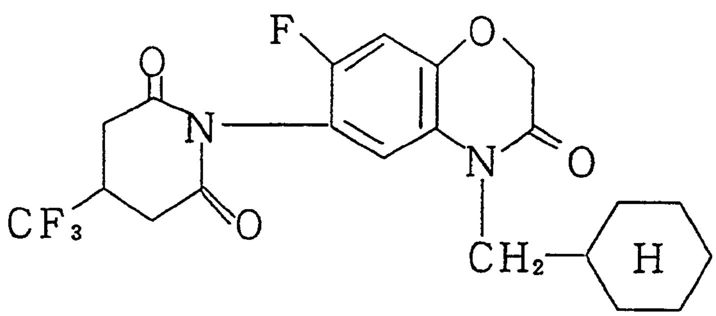 |

Table 1-1 (Cont' d)

| Compound No. | Structure |
|---|---|
| 1-29 | 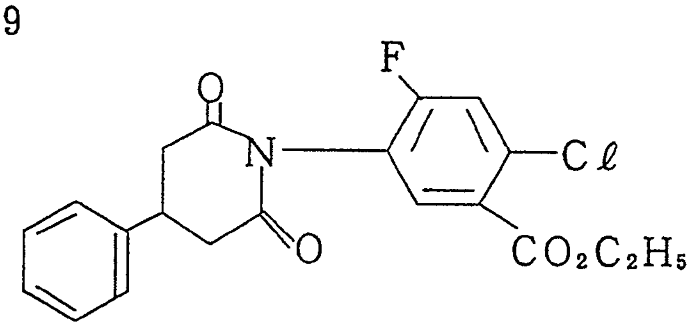 |
| 1-30 | 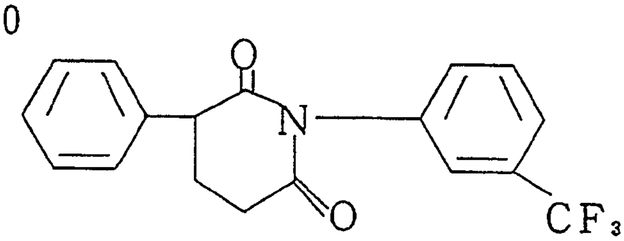 |

Table 1-1 (Cont' d)

| Compound No. | Structure |
|---|---|
| 1-31 | 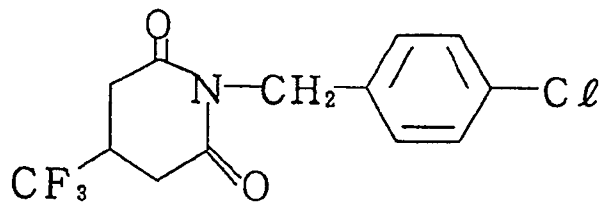 |
| 1-32 | 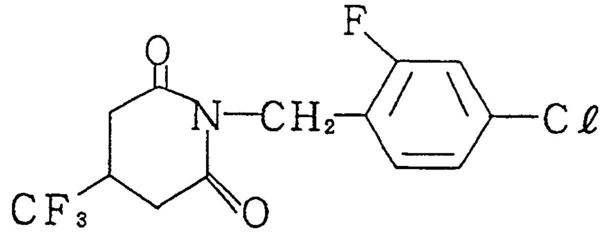 |
| 1-33 | 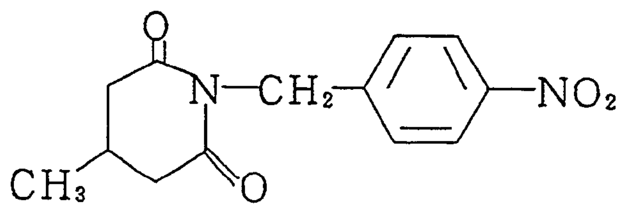 |

Table 1-1 (Cont' d)

| Compound No. | Structure |
|---|---|
| 1-34 | 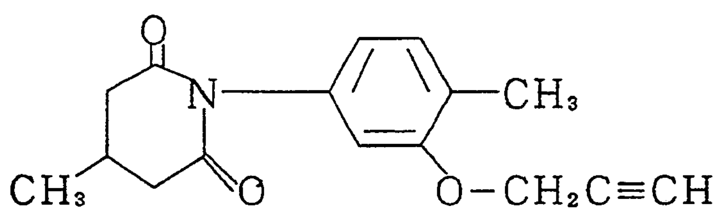 |
| 1-35 | 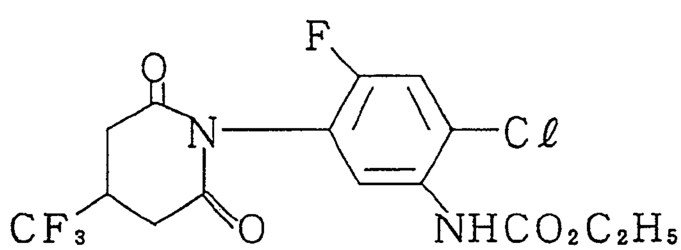 |

Table 1－2

| Compound No. | $^1$H-NMR δ (ppm) [solvent] | physical properties |
|---|---|---|
| 1－1 | 0.81～1.52(6H, m), 1.71～3.15(4H, m), 4.28(2H, q, J=7Hz), 7.24(1H, d, J=9Hz), 7.65(1H, d, J=8Hz) [$CDCl_3$] | oil |
| 1－2 | 1.30(3H, t, J=7Hz), 1.44(3H, d, J=7Hz), 2.49～3.56(4H, m), 4.28(2H, q, J=7Hz), 7.20(1H, d, J=9Hz), 7.65(1H, d, J=7Hz) [$CDCl_3$] | oil |
| 1－3 | 1.33(6H, d, J=6Hz), 1.52(3H, d, J=7Hz), 2.89～3.36(4H, m), 4.93～5.44(1H, m), 7.18(1H, d, J=9Hz), 7.51(1H, d, J=7Hz) [$CDCl_3$] | oil |
| 1－4 | 1.09(3H, t, J=7Hz), 1.37(3H, t, J=7Hz), 1.62～2.31(2H, m), 2.58～3.22(4H, m), 4.38(2H, q, J=7Hz), 7.42(1H, d, J=9Hz), 7.69(1H, d, J=8Hz) [$CDCl_3$] | oil |
| 1－5 | 0.89(6H, d, J=6Hz), 1.28(6H, d, J=6Hz), 1.34(3H, d, J=6Hz), 1.52～3.13(4H, m), 4.88～5.46(1H, m), 7.24(1H, d, J=9Hz), 7.63(1H, d, J=7Hz), [$CDCl_3$] | oil |
| 1－6 | | |
| 1－7 | | |
| 1－8 | 1.20(6H, s), 2.65(4H, s), 7.00～7.39(3H, m) [$CDCl_3$] | mp=134.1～135.6℃ |
| 1－9 | 1.19(6H, s), 1.34(3H, t, J=7Hz), 2.63(4H, s), 4.30(2H, q, J=7Hz), 7.17(1H, d, J=9Hz), 7.56(1H, d, J=7Hz) [$CDCl_3$] | mp=134.4～135.4℃ |
| 1－10 | 1.33(6H, s), 1.88(2H, t, J=7Hz), 2.80(2H, t, J=7Hz), 6.99～7.34(3H, m) [$CDCl_3$] | mp=88.5～89.7℃ |

Table 1-2 (cont'd)

| Compound No. | $^1$H-NMR δ (ppm) [solvent] | physical properties |
|---|---|---|
| 1-11 | 1.36(3H, t, J=7Hz), 1.49~2.00(8H, m), 2.71(4H, s), 4.32(2H, q, J=7Hz), 7.21(1H, d, J=9Hz), 7.62(1H, d, J=8Hz) [$CDCl_3$] | mp=146~150℃ |
| 1-12 | 1.02~1.51(6H, m), 1.65~2.18(2H, m), 2.45~3.01(3H, m), 4.29(2H, q, J=7Hz), 7.27(1H, d, J=9Hz), 7.61(1H, d, J=8Hz) [$CDCl_3$] | oil |
| 1-13 | 2.86~3.15(5H, m), 3.83(2H, br s), 6.37(1H, d, J=6Hz), 7.02(1H, d, J=9Hz) [$CDCl_3$] | mp=154.0 ~155.0 ℃ |
| 1-14 | 2.14(3H, s), 2.78~3.39(5H, m), 7.18(1H, d, J=9Hz), 7.83(1H, d, J=7Hz), 8.68(1H, br s) [$d_6$-DMSO] | mp=197 ~200 ℃ |
| 1-15 | 1.30(3H, t, J=7Hz), 2.72~3.21(5H, m), 3.04(2H, q, J=7Hz), 6.72(1H, br s), 7.13(1H, d, J=8Hz), 7.37(1H, d, J=7Hz) [$CDCl_3$] | mp=70~73℃ |
| 1-16 | 0.87~1.28(3H, m), 2.09~3.10(5H, m), 2.53(3H, s), 7.30(1H, d, J=9Hz), 7.71(1H, d, J=7Hz) [$CDCl_3$] | vitrified |
| 1-17 | 1.03~1.31(3H, m), 1.49(9H, s), 2.20~3.08(5H, m), 7.31(1H, d, J=9Hz), 7.83(1H, d, J=7Hz) [$CDCl_3$] | mp=141~145℃ |
| 1-18 | 2.53(3H, s), 2.76~3.24(5H, m), 7.24(1H, d, J=9Hz), 7.63(1H, d, J=7Hz) [$CDCl_3$] | vitrified |
| 1-19 | 2.69~3.40(5H, m), 5.99(1H, s), 6.64(1H, br s), 7.21(1H, d, J=7Hz), 7.44(1H, d, J=9Hz) [$d_6$-DMSO] | mp=189~193℃ |
| 1-20 | 2.89~3.17(5H, m), 3.50(3H, br s), 6.39(1H, s), 7.46(1H, d, J=7Hz), 7.67(1H, d, J=9Hz) [$d_6$-DMSO] | mp=233~235℃ |

Table 1-2 (cont'd)

| Compound No. | $^1$H-NMR δ (ppm) [solvent] | physical properties |
|---|---|---|
| 1-21 | 2.84～3.24(5H, m), 3.52(3H, br s), 6.36(1H, s), 6.98～7.52(2H, m) [$d_6$-DMSO] | mp=218～220℃ |
| 1-22 | 0.82～1.43(3H, m), 2.16～2.98(5H, m), 4.52(2H, s), 6.62(1H, d, J=7Hz), 6.65(1H, d, J=10Hz) [$d_6$-DMSO] | mp=223～228℃ |
| 1-23 | 0.95～1.50(3H, m), 2.05～3.21(6H, m), 4.67(4H, br s), 6.87(1H, d, J=10Hz), 6.99(1H, d, J=8Hz) [$d_6$-DMSO] | mp=193～196℃ |
| 1-24 | 2.61(1H, t, J=2Hz), 2.78 ～3.49(5H, m), 4.55(2H, d, J=2Hz), 4.59(2H, s), 6.53 ～7.09(3H, m) [$d_6$-DMSO] | mp=204 ～206 ℃ |
| 1-25 | 2.80～3.29(5H, m), 4.55(2H, br s), 6.49(1H, d, J=6Hz), 6.72(1H, d, J=10Hz), 9.42(1H, br s) [$CDCl_3$] | mp=98～103 ℃ |
| 1-26 | 2.33(1H, t, J=2Hz), 2.77～3.36(5H, m), 4.57(2H, d, J=2Hz), 4.61(2H, s), 6.75(1H, d, J=9Hz), 6.85(1H, d, J=6Hz) [$CDCl_3$] | mp=147 ～152 ℃ |
| 1-27 | 2.76～3.24(5H, m), 4.66(2H, s), 5.00(2H, s), 6.48(1H, d, J=7Hz), 6.74(1H, d, J=9Hz), 7.13(5H, br s) [$CDCl_3$] | mp=220 ～223 ℃ |
| 1-28 | 0.77～2.22(11H, m), 2.89～3.30(5H, m), 3.70(2H, br d), 4.58(2H, s), 6.57(1H, d, J=7Hz), 6.76(1H, d, J=9Hz) [$CDCl_3$] | mp=181 ～186 ℃ |
| 1-29 | 1.35(3H, t, J=7Hz), 2.84～3.55(5H, m), 4.32(2H, q, J=7Hz), 7.05～7.62(5H, m), 7.73(1H, d, J=8Hz), 7.79(1H, d, J=8Hz) [$CDCl_3$] | mp=139 ～142 ℃ |
| 1-30 | 2.05～2.53(2H, m), 2.58～2.95(2H, m), 3.92(1H, br t, J=7Hz), 6.85 ～7.64(9H, m) [$CDCl_3$] | mp=136 ～137 ℃ |

Table 1-2 (cont'd)

| Compound No. | $^1$H-NMR δ (ppm) [solvent] | physical properties |
|---|---|---|
| 1-31 | 2.78(5H, br s), 4.31(2H, s), 7.14(4H, s) [$CDCl_3$] | mp=123.0～126.0°C |
| 1-32 | 2.36～3.41(5H, m), 4.99(2H, s), 6.91～7.39(3H, m) [$CDCl_3$] | mp=116.2～117.2°C |
| 1-33 | 0.88～1.29(3H, m), 2.00～3.08(5H, m), 4.94(2H, s), 7.22～8.15(4H, m) [$CDCl_3$] | mp=125～131°C |
| 1-34 | 0.86～1.40(3H, m), 1.91～3.17(9H, m), 4.65(2H, d, J=2Hz), 6.40～7.38(3H, m) [$CDCl_3$] | oil |
| 1-35 | 1.30(3H, t, J=7Hz), 2.49～3.45(5H, m), 4.20(2H, q, J=7Hz), 7.08(1H, br s), 7.24(1H, d, J=9Hz), 8.08(1H, d, J=7Hz) [$CDCl_3$] | mp=149～151°C |

The compound of the present invention synthesized according to the above Examples and synthesized by following the similar procedures to the above Examples or Schemes are shown in Tables 1-3 to 1-9 and Tables 1-3-1 to 1-9-1. The compounds obtainable in accordance with the present invention, however, are not limited to those shown in the following tables.

Table 1-3

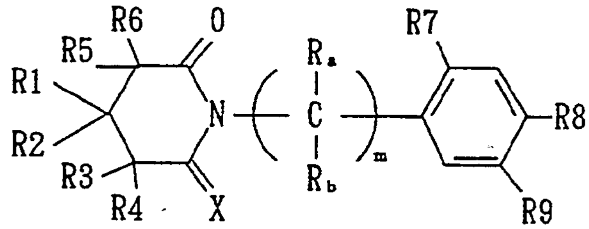

| R1 | R2 | R3 | R4 | R5 | R6 | X | $R_a$ | $R_b$ | m | R7 | R8 | R9 |
|---|---|---|---|---|---|---|---|---|---|---|---|---|
| Me | Me | H | H | H | H | O | - | - | 0 | F | Cl | H |
| Me | Me | H | H | H | H | S | - | - | 0 | F | Cl | H |
| Me | Me | H | H | H | H | O | - | - | 0 | F | Cl | $CO_2Et$ |
| Me | Me | H | H | H | H | S | - | - | 0 | F | Cl | $CO_2Et$ |
| Me | Me | H | H | H | H | O | H | H | 1 | H | Cl | H |
| H | H | Me | Me | H | H | O | - | - | 0 | F | Cl | H |
| H | H | Me | Me | H | H | S | - | - | 0 | F | Cl | H |
| H | H | Me | Me | H | H | O | - | - | 0 | F | Cl | $CO_2Et$ |
| H | H | Me | Me | H | H | S | - | - | 0 | F | Cl | $CO_2Et$ |
| H | H | Me | Me | H | H | O | H | H | 1 | H | Cl | H |
| H | H | H | H | Me | Me | S | - | - | 0 | F | Cl | H |
| H | H | H | H | Me | Me | S | - | - | 0 | F | Cl | $CO_2Et$ |
| H | H | Me | H | H | H | O | - | - | 0 | F | Cl | H |
| H | H | Me | H | H | H | O | - | - | 0 | F | Cl | $CO_2Et$ |
| H | H | Me | H | H | H | S | - | - | 0 | F | Cl | H |
| H | H | H | H | Me | H | S | - | - | 0 | F | Cl | H |
| Ph | H | Me | H | H | H | O | - | - | 0 | F | Cl | H |
| Ph | H | Me | H | H | H | O | - | - | 0 | F | Cl | $CO_2Et$ |
| Me | H | Ph | H | H | H | O | - | - | 0 | F | Cl | H |
| Me | H | Ph | H | H | H | O | - | - | 0 | F | Cl | $CO_2Et$ |

Table 1－3 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | H | H | Ph | H | S | - | - | 0 | F | Cl | H |
| Me | H | H | H | Ph | H | S | - | - | 0 | F | Cl | $CO_2Et$ |
| H | H | H | H | H | H | S | - | - | 0 | F | Cl | H |
| H | H | H | H | H | H | S | - | - | 0 | F | Cl | $CO_2Et$ |
| H | H | H | H | H | H | O | H | H | 1 | H | Cl | H |
| Me | H | Me | H | H | H | O | - | - | 0 | F | Cl | H |
| Me | H | Me | H | H | H | O | - | - | 0 | F | Cl | $CO_2Et$ |
| Me | H | Me | H | H | H | S | - | - | 0 | F | Cl | H |
| Me | H | H | H | Me | H | S | - | - | 0 | F | Cl | H |
| Me | H | H | H | Me | H | S | - | - | 0 | F | Cl | $CO_2Et$ |
| H | H | Me | H | Me | H | O | - | - | 0 | F | Cl | H |
| H | H | Me | H | Me | H | O | - | - | 0 | F | Cl | $CO_2Et$ |
| H | H | Me | H | Me | H | S | - | - | 0 | F | Cl | H |
| F | F | F | F | F | F | O | - | - | 0 | F | Cl | H |
| F | F | F | F | F | F | O | - | - | 0 | F | Cl | $CO_2Et$ |
| F | F | F | F | F | F | O | H | H | 1 | H | Cl | H |
| F | F | F | F | F | F | O | H | H | 1 | H | $NO_2$ | H |
| F | F | F | F | F | F | O | H | H | 1 | H | $NO_2$ | $CO_2H$ |
| $CF_3$ | H | Me | H | H | H | O | - | - | 0 | F | Cℓ | H |
| $CF_3$ | H | Me | H | H | H | O | - | - | 0 | F | Cl | $CO_2Me$ |
| $CF_3$ | H | Me | H | H | H | O | - | - | 0 | F | Cl | $CO_2Et$ |
| $CF_3$ | H | Me | H | H | H | S | - | - | 0 | F | Cl | $CO_2Et$ |
| $CF_3$ | H | H | H | Me | H | S | - | - | 0 | F | Cl | $CO_2Et$ |

Table 1-3-1

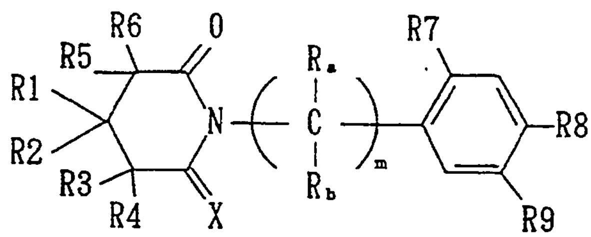

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | H | H | O | - | - | 0 | F | Cl | $CO_2Pr^i$ |
| $CF_3$ | H | Me | H | H | H | O | - | - | 0 | F | Cl | $CO_2Pr^n$ |
| $CF_3$ | H | Me | H | H | H | O | - | - | 0 | F | Cl | $CO_2Bu^n$ |
| $CF_3$ | H | Me | H | H | H | O | - | - | 0 | F | Cl | $CO_2Bu^t$ |
| $CF_3$ | H | Me | H | H | H | O | - | - | 0 | F | Cl | $NO_2$ |
| $CF_3$ | H | Me | H | H | H | O | - | - | 0 | F | Cl | $NH_2$ |
| $CF_3$ | H | Me | H | H | H | O | - | - | 0 | F | Cl | $NHSO_2Me$ |
| $CF_3$ | H | Me | H | H | H | O | - | - | 0 | F | Cl | $NHSO_2Et$ |
| $CF_3$ | H | Me | H | H | H | O | - | - | 0 | F | Cl | $NHSO_2Pr^n$ |
| $CF_3$ | H | Me | H | H | H | O | - | - | 0 | F | Cl | $NHSO_2Pr^i$ |
| $CF_3$ | H | Me | H | H | H | O | - | - | 0 | F | Cl | OH |
| $CF_3$ | H | Me | H | H | H | O | - | - | 0 | F | Cl | OMe |
| $CF_3$ | H | Me | H | H | H | O | - | - | 0 | F | Cl | OEt |
| $CF_3$ | H | Me | H | H | H | O | - | - | 0 | F | Cl | $OPr^i$ |
| $CF_3$ | H | Me | H | H | H | O | - | - | 0 | F | Cl | $OPr^n$ |
| $CF_3$ | H | Me | H | H | H | O | - | - | 0 | F | Cl | $OPen^c$ |
| $CF_3$ | H | Me | H | H | H | O | - | - | 0 | F | Cl | $OCH_2CH{=}CH$ |
| $CF_3$ | H | Me | H | H | H | O | - | - | 0 | F | Cl | $OCH_2C{\equiv}CH$ |
| $CF_3$ | H | Me | H | H | H | O | - | - | 0 | F | Cl | $OCH_2CO_2Me$ |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | H | H | O | - | - | 0 | F | Cl | $OCH_2CO_2Et$ |
| $CF_3$ | H | Me | H | H | H | O | - | - | 0 | F | Cl | $OCH(Me)CO_2Me$ |
| $CF_3$ | H | Me | H | H | H | O | - | - | 0 | F | Cl | $OCH(Me)CO_2Et$ |
| $CF_3$ | H | Me | H | H | H | O | - | - | 0 | F | Cl | $CO_2H$ |
| $CF_3$ | H | Me | H | H | H | O | - | - | 0 | F | Cl | CN |
| $CF_3$ | H | Me | H | H | H | O | - | - | 0 | F | Cl | Me |
| $CF_3$ | H | Me | H | H | H | O | - | - | 0 | F | Cl | Et |
| $CF_3$ | H | Me | H | H | H | O | - | - | 0 | F | Cl | Cl |
| $CF_3$ | H | Me | H | H | H | O | - | - | 0 | F | Cl | Br |
| $CF_3$ | H | Me | H | H | H | O | - | - | 0 | F | Cl | I |
| $CF_3$ | H | Me | H | H | H | O | - | - | 0 | F | Cl | F |
| $CF_3$ | H | Me | H | H | H | O | - | - | 0 | F | Cl | SH |
| $CF_3$ | H | Me | H | H | H | O | - | - | 0 | F | Cl | $C(Me)=NOCH_2CO_2Me$ |
| $CF_3$ | H | Me | H | H | H | O | - | - | 0 | F | Cl | $C(Me)=NOCH_2CO_2Et$ |
| $CF_3$ | H | Me | H | H | H | O | - | - | 0 | F | Cl | $CH=NOCH_2CO_2Me$ |
| $CF_3$ | H | Me | H | H | H | O | - | - | 0 | F | Cl | $CH=NOCH_2CO_2Et$ |
| $CF_3$ | H | Me | H | H | H | O | - | - | 0 | F | Cl | $C(OMe)=NOCH_2CO_2Me$ |
| $CF_3$ | H | Me | H | H | H | O | - | - | 0 | F | Cl | $C(OMe)=NOCH_2CO_2Et$ |
| $CF_3$ | H | Me | H | H | H | O | - | - | 0 | F | Cl | $C(Me)=NN=C(Me)CO_2Me$ |
| $CF_3$ | H | Me | H | H | H | O | - | - | 0 | F | Cl | $C(Me)NN=C(Me)CO_2Et$ |
| $CF_3$ | H | Me | H | H | H | O | - | - | 0 | F | Cl | $CH_2OCH_2CO_2Me$ |
| $CF_3$ | H | Me | H | H | H | O | - | - | 0 | F | Cl | $CH_2OCH_2CO_2Et$ |
| $CF_3$ | H | Me | H | H | H | O | - | - | 0 | F | Cl | $CH_2OCH(Me)CO_2Me$ |
| $CF_3$ | H | Me | H | H | H | O | - | - | 0 | F | Cl | $CH_2OCH(Me)CO_2Et$ |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | H | H | O | - | - | 0 | F | Cl | $CH_2SCH_2CO_2Me$ |
| $CF_3$ | H | Me | H | H | H | O | - | - | 0 | F | Cl | $CH_2SCH_2CO_2Et$ |
| $CF_3$ | H | Me | H | H | H | O | - | - | 0 | F | Cl | $CH_2SCH(Me)CO_2Me$ |
| $CF_3$ | H | Me | H | H | H | O | - | - | 0 | F | Cl | $CH_2SCH(Me)CO_2Et$ |
| $CF_3$ | H | Me | H | H | H | O | - | - | 0 | F | Cl | SMe |
| $CF_3$ | H | Me | H | H | H | O | - | - | 0 | F | Cl | SEt |
| $CF_3$ | H | Me | H | H | H | O | - | - | 0 | F | Cl | $SPr^i$ |
| $CF_3$ | H | Me | H | H | H | O | - | - | 0 | F | Cl | $SPr^n$ |
| $CF_3$ | H | Me | H | H | H | O | - | - | 0 | F | Cl | $SPen^c$ |
| $CF_3$ | H | Me | H | H | H | O | - | - | 0 | F | Cl | $SCH_2CO_2H$ |
| $CF_3$ | H | Me | H | H | H | O | - | - | 0 | F | Cl | $SCH_2CO_2Me$ |
| $CF_3$ | H | Me | H | H | H | O | - | - | 0 | F | Cl | $SCH_2CO_2Et$ |
| $CF_3$ | H | Me | H | H | H | O | - | - | 0 | F | Cl | $SCH(Me)CO_2Me$ |
| $CF_3$ | H | Me | H | H | H | O | - | - | 0 | F | Cl | $SCH(Me)CO_2Et$ |
| $CF_3$ | H | Me | H | H | H | O | - | - | 0 | F | Cl | $SCH_2CN$ |
| $CF_3$ | H | Me | H | H | H | O | - | - | 0 | F | Cl | $SCH_2CH=CH_2$ |
| $CF_3$ | H | Me | H | H | H | O | - | - | 0 | F | Cl | $SCH_2C\equiv CH$ |
| $CF_3$ | H | Me | H | H | H | O | - | - | 0 | F | Cl | $OCH(Me)C\equiv CH$ |
| $CF_3$ | H | Me | H | H | H | O | - | - | 0 | F | Cl | $SCH(Me)C\equiv CH$ |
| $CF_3$ | H | Me | H | H | H | O | - | - | 0 | F | Cl | $N(SO_2Me)_2$ |
| $CF_3$ | H | Me | H | H | H | O | - | - | 0 | F | Cl | $N(SO_2Et)_2$ |
| $CF_3$ | H | Me | H | H | H | O | - | - | 0 | F | Cl | Q1(R29=$Bu^t$) |
| $CF_3$ | H | Me | H | H | H | O | - | - | 0 | F | Cl | Q2(R29=$Bu^t$) |
| $CF_3$ | H | Me | H | H | H | O | - | - | 0 | F | Cl | Q5(R29=$Bu^t$) |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | H | H | O | - | - | 0 | F | Cl | Q7(R34=$Bu^t$ ) |
| $CF_3$ | H | Me | H | H | H | O | - | - | 0 | F | Cl | Q9(R38=R39=H) |
| $CF_3$ | H | Me | H | H | H | O | - | - | 0 | F | Cl | Q10(R38=R39=H) |
| $CF_3$ | H | Me | H | H | H | O | - | - | 0 | F | Cl | Q4(R29=$Bu^t$ ) |
| $CF_3$ | H | Me | H | H | H | O | - | - | 0 | F | Cl | Q8(R35=R36=R37=H) |
| $CF_3$ | H | Me | H | H | H | O | - | - | 0 | F | Cl | Q18(R50=H) |
| $CF_3$ | H | Me | H | H | H | O | - | - | 0 | F | Cl | Q18(R50=Me) |
| $CF_3$ | H | Me | H | H | H | O | - | - | 0 | F | Cl | $NHCO_2Me$ |
| $CF_3$ | H | Me | H | H | H | O | - | - | 0 | F | Cl | $NHCO_2Et$ |
| $CF_3$ | H | Me | H | H | H | O | - | - | 0 | F | Cl | $NHCO_2Pr^n$ |
| $CF_3$ | H | Me | H | H | H | O | - | - | 0 | F | Cl | $NHCO_2Pr^i$ |
| $CF_3$ | H | Me | H | H | H | O | - | - | 0 | F | Cl | $NHCO_2Ph$ |
| $CF_3$ | H | Me | H | H | H | O | - | - | 0 | F | Cl | $NHCO_2Bz$ |
| $CF_3$ | H | Me | H | H | H | O | - | - | 0 | F | Cl | NHCOMe |
| $CF_3$ | H | Me | H | H | H | O | - | - | 0 | F | Cl | $NHCO_2CH_2Ph$ |
| $CF_3$ | H | Me | H | H | H | O | - | - | 0 | F | Cl | $NHCO_2CH_2$(4-Me-Ph) |
| $CF_3$ | H | Me | H | H | H | O | - | - | 0 | F | Cl | $NHCO_2CH_2$(3-Me-Ph) |
| $CF_3$ | H | Me | H | H | H | O | - | - | 0 | F | Cl | $NHCO_2CH_2$(2-Me-Ph) |
| $CF_3$ | H | Me | H | H | H | O | - | - | 0 | F | Cl | $NHCO_2CH_2$(4-CL-Ph) |
| $CF_3$ | H | Me | H | H | H | O | - | - | 0 | F | Cl | $NHCO_2CH_2$(3-CL-Ph) |
| $CF_3$ | H | Me | H | H | H | O | - | - | 0 | F | Cl | $NHCO_2CH_2$(2-CL-Ph) |
| $CF_3$ | H | Me | H | H | H | O | - | - | 0 | F | Cl | Q1(R29=Me) |
| $CF_3$ | H | Me | H | H | H | O | - | - | 0 | F | Cl | Q2(R29=Me) |
| H | H | Ph | H | H | H | O | - | - | 0 | H | H | $CF_3$ |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Ph | H | Me | H | H | H | O | - | - | 0 | F | Cl | $CO_2Pr^i$ |
| Ph | H | Me | H | H | H | O | - | - | 0 | F | Cl | $CO_2Et$ |
| Ph | H | Me | H | H | H | O | - | - | 0 | F | Cl | H |
| Me | H | Me | H | H | H | O | - | - | 0 | F | Cl | $CO_2Pr^i$ |
| Me | H | Me | H | H | H | O | - | - | 0 | F | Cl | $CO_2Pr^n$ |
| Me | H | Me | H | H | H | O | - | - | 0 | F | Cl | $CO_2Bu^n$ |
| Me | H | Me | H | H | H | O | - | - | 0 | F | Cl | $CO_2Bu^t$ |
| Me | H | Me | H | H | H | O | - | - | 0 | F | Cl | $NO_2$ |
| Me | H | Me | H | H | H | O | - | - | 0 | F | Cl | $NH_2$ |
| Me | H | Me | H | H | H | O | - | - | 0 | F | Cl | $NHSO_2Me$ |
| Me | H | Me | H | H | H | O | - | - | 0 | F | Cl | $NHSO_2Et$ |
| Me | H | Me | H | H | H | O | - | - | 0 | F | Cl | $NHSO_2Pr^n$ |
| Me | H | Me | H | H | H | O | - | - | 0 | F | Cl | $NHSO_2Pr^i$ |
| Me | H | Me | H | H | H | O | - | - | 0 | F | Cl | OH |
| Me | H | Me | H | H | H | O | - | - | 0 | F | Cl | OMe |
| Me | H | Me | H | H | H | O | - | - | 0 | F | Cl | OEt |
| Me | H | Me | H | H | H | O | - | - | 0 | F | Cl | $OPr^i$ |
| Me | H | Me | H | H | H | O | - | - | 0 | F | Cl | $OPr^n$ |
| Me | H | Me | H | H | H | O | - | - | 0 | F | Cl | $OPen^c$ |
| Me | H | Me | H | H | H | O | - | - | 0 | F | Cl | $OCH_2CH=CH_2$ |
| Me | H | Me | H | H | H | O | - | - | 0 | F | Cl | $OCH_2C\equiv CH$ |
| Me | H | Me | H | H | H | O | - | - | 0 | F | Cl | $OCH_2CO_2Me$ |
| Me | H | Me | H | H | H | O | - | - | 0 | F | Cl | $OCH_2CO_2Et$ |
| Me | H | Me | H | H | H | O | - | - | 0 | F | Cl | $OCH(Me)CO_2Me$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | H | H | O | - | - | 0 | F | Cl | $OCH(Me)CO_2Et$ |
| Me | H | Me | H | H | H | O | - | - | 0 | F | Cl | $CO_2H$ |
| Me | H | Me | H | H | H | O | - | - | 0 | F | Cl | CN |
| Me | H | Me | H | H | H | O | - | - | 0 | F | Cl | Me |
| Me | H | Me | H | H | H | O | - | - | 0 | F | Cl | Et |
| Me | H | Me | H | H | H | O | - | - | 0 | F | Cl | Cl |
| Me | H | Me | H | H | H | O | - | - | 0 | F | Cl | Br |
| Me | H | Me | H | H | H | O | - | - | 0 | F | Cl | I |
| Me | H | Me | H | H | H | O | - | - | 0 | F | Cl | F |
| Me | H | Me | H | H | H | O | - | - | 0 | F | Cl | SH |
| Me | H | Me | H | H | H | O | - | - | 0 | F | Cl | $C(Me)=NOCH_2CO_2Me$ |
| Me | H | Me | H | H | H | O | - | - | 0 | F | Cl | $C(Me)=NOCH_2CO_2Et$ |
| Me | H | Me | H | H | H | O | - | - | 0 | F | Cl | $CH=NOCH_2CO_2Me$ |
| Me | H | Me | H | H | H | O | - | - | 0 | F | Cl | $CH=NOCH_2CO_2Et$ |
| Me | H | Me | H | H | H | O | - | - | 0 | F | Cl | $C(OMe)=NOCH_2CO_2Me$ |
| Me | H | Me | H | H | H | O | - | - | 0 | F | Cl | $C(OMe)=NOCH_2CO_2Et$ |
| Me | H | Me | H | H | H | O | - | - | 0 | F | Cl | $C(Me)=NN=C(Me)CO_2Me$ |
| Me | H | Me | H | H | H | O | - | - | 0 | F | Cl | $C(Me)=NN=C(Me)CO_2Et$ |
| Me | H | Me | H | H | H | O | - | - | 0 | F | Cl | $CH_2OCH_2CO_2Me$ |
| Me | H | Me | H | H | H | O | - | - | 0 | F | Cl | $CH_2OCH_2CO_2Et$ |
| Me | H | Me | H | H | H | O | - | - | 0 | F | Cl | $CH_2OCH(Me)CO_2Me$ |
| Me | H | Me | H | H | H | O | - | - | 0 | F | Cl | $CH_2OCH(Me)CO_2Et$ |
| Me | H | Me | H | H | H | O | - | - | 0 | F | Cl | $CH_2SCH_2CO_2Me$ |
| Me | H | Me | H | H | H | O | - | - | 0 | F | Cl | $CH_2SCH_2CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | H | H | O | - | - | 0 | F | Cl | $CH_2SCH(Me)CO_2Me$ |
| Me | H | Me | H | H | H | O | - | - | 0 | F | Cl | $CH_2SCH(Me)CO_2Et$ |
| Me | H | Me | H | H | H | O | - | - | 0 | F | Cl | SMe |
| Me | H | Me | H | H | H | O | - | - | 0 | F | Cl | SEt |
| Me | H | Me | H | H | H | O | - | - | 0 | F | Cl | $SPr^i$ |
| Me | H | Me | H | H | H | O | - | - | 0 | F | Cl | $SPr^n$ |
| Me | H | Me | H | H | H | O | - | - | 0 | F | Cl | $SPen^c$ |
| Me | H | Me | H | H | H | O | - | - | 0 | F | Cl | $SCH_2CO_2H$ |
| Me | H | Me | H | H | H | O | - | - | 0 | F | Cl | $SCH_2CO_2Me$ |
| Me | H | Me | H | H | H | O | - | - | 0 | F | Cl | $SCH_2CO_2Et$ |
| Me | H | Me | H | H | H | O | - | - | 0 | F | Cl | $SCH(Me)CO_2Me$ |
| Me | H | Me | H | H | H | O | - | - | 0 | F | Cl | $SCH(Me)CO_2Et$ |
| Me | H | Me | H | H | H | O | - | - | 0 | F | Cl | $SCH_2CN$ |
| Me | H | Me | H | H | H | O | - | - | 0 | F | Cl | $SCH_2CH=CH_2$ |
| Me | H | Me | H | H | H | O | - | - | 0 | F | Cl | $SCH_2C \equiv CH$ |
| Me | H | Me | H | H | H | O | - | - | 0 | F | Cl | $OCH(Me)C \equiv CH$ |
| Me | H | Me | H | H | H | O | - | - | 0 | F | Cl | $SCH(Me)C \equiv CH$ |
| Me | H | Me | H | H | H | O | - | - | 0 | F | Cl | $N(SO_2Me)_2$ |
| Me | H | Me | H | H | H | O | - | - | 0 | F | Cl | $N(SO_2Et)_2$ |
| Me | H | Me | H | H | H | O | - | - | 0 | F | Cl | Q1(R29=$Bu^t$) |
| Me | H | Me | H | H | H | O | - | - | 0 | F | Cl | Q2(R29=$Bu^t$) |
| Me | H | Me | H | H | H | O | - | - | 0 | F | Cl | Q5(R29=$Bu^t$) |
| Me | H | Me | H | H | H | O | - | - | 0 | F | Cl | Q7(R34=$Bu^t$) |
| Me | H | Me | H | H | H | O | - | - | 0 | F | Cl | Q9(R38=R39=H) |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | H | H | O | - | - | 0 | F | Cl | Q10(R38=R39=H) |
| Me | H | Me | H | H | H | O | - | - | 0 | F | Cl | Q4(R29=$Bu^t$ ) |
| Me | H | Me | H | H | H | O | - | - | 0 | F | Cl | Q8(R35=R36=R37=H) |
| Me | H | Me | H | H | H | O | - | - | 0 | F | Cl | Q18(R50=H) |
| Me | H | Me | H | H | H | O | - | - | 0 | F | Cl | Q18(R50=Me) |
| Me | H | Me | H | H | H | O | - | - | 0 | F | Cl | $NHCO_2Me$ |
| Me | H | Me | H | H | H | O | - | - | 0 | F | Cl | $NHCO_2Et$ |
| Me | H | Me | H | H | H | O | - | - | 0 | F | Cl | $NHCO_2Pr^n$ |
| Me | H | Me | H | H | H | O | - | - | 0 | F | Cl | $NHCO_2Pr^i$ |
| Me | H | Me | H | H | H | O | - | - | 0 | F | Cl | $NHCO_2Ph$ |
| Me | H | Me | H | H | H | O | - | - | 0 | F | Cl | $NHCO_2Bz$ |
| Me | H | Me | H | H | H | O | - | - | 0 | F | Cl | NHCOMe |
| Me | H | Me | H | H | H | O | - | - | 0 | F | Cl | $NHCO_2CH_2Ph$ |
| Me | H | Me | H | H | H | O | - | - | 0 | F | Cl | $NHCO_2CH_2$(4-Me-Ph) |
| Me | H | Me | H | H | H | O | - | - | 0 | F | Cl | $NHCO_2CH_2$(3-Me-Ph) |
| Me | H | Me | H | H | H | O | - | - | 0 | F | Cl | $NHCO_2CH_2$(2-Me-Ph) |
| Me | H | Me | H | H | H | O | - | - | 0 | F | Cl | $NHCO_2CH_2$(4-CL -Ph) |
| Me | H | Me | H | H | H | O | - | - | 0 | F | Cl | $NHCO_2CH_2$(3-CL-Ph) |
| Me | H | Me | H | H | H | O | - | - | 0 | F | Cl | $NHCO_2CH_2$(2-CL-Ph) |
| Me | H | Me | H | H | H | O | - | - | 0 | F | Cl | Q1(R29=Me) |
| Me | H | Me | H | H | H | O | - | - | 0 | F | Cl | Q2(R29=Me) |
| Me | H | Me | H | H | H | O | - | - | 0 | F | Cl | $CO_2Me$ |
| Me | H | Me | H | H | H | O | - | - | 0 | F | Cl | $CO_2H$ |
| $CF_3$ | H | Et | H | H | H | O | - | - | 0 | F | Cl | $CO_2Pr^i$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Et | H | H | H | O | - | - | 0 | F | Cl | $CO_2Pr^n$ |
| $CF_3$ | H | Et | H | H | H | O | - | - | 0 | F | Cl | $CO_2Bu^n$ |
| $CF_3$ | H | Et | H | H | H | O | - | - | 0 | F | Cl | $CO_2Bu^t$ |
| $CF_3$ | H | Et | H | H | H | O | - | - | 0 | F | Cl | $NO_2$ |
| $CF_3$ | H | Et | H | H | H | O | - | - | 0 | F | Cl | $NH_2$ |
| $CF_3$ | H | Et | H | H | H | O | - | - | 0 | F | Cl | $NHSO_2Me$ |
| $CF_3$ | H | Et | H | H | H | O | - | - | 0 | F | Cl | $NHSO_2Et$ |
| $CF_3$ | H | Et | H | H | H | O | - | - | 0 | F | Cl | $NHSO_2Pr^n$ |
| $CF_3$ | H | Et | H | H | H | O | - | - | 0 | F | Cl | $NHSO_2Pr^i$ |
| $CF_3$ | H | Et | H | H | H | O | - | - | 0 | F | Cl | OH |
| $CF_3$ | H | Et | H | H | H | O | - | - | 0 | F | Cl | OMe |
| $CF_3$ | H | Et | H | H | H | O | - | - | 0 | F | Cl | OEt |
| $CF_3$ | H | Et | H | H | H | O | - | - | 0 | F | Cl | $OPr^i$ |
| $CF_3$ | H | Et | H | H | H | O | - | - | 0 | F | Cl | $OPr^n$ |
| $CF_3$ | H | Et | H | H | H | O | - | - | 0 | F | Cl | $OPen^c$ |
| $CF_3$ | H | Et | H | H | H | O | - | - | 0 | F | Cl | $OCH_2CH=CH_2$ |
| $CF_3$ | H | Et | H | H | H | O | - | - | 0 | F | Cl | $OCH_2C\equiv CH$ |
| $CF_3$ | H | Et | H | H | H | O | - | - | 0 | F | Cl | $OCH_2CO_2Me$ |
| $CF_3$ | H | Et | H | H | H | O | - | - | 0 | F | Cl | $OCH_2CO_2Et$ |
| $CF_3$ | H | Et | H | H | H | O | - | - | 0 | F | Cl | $OCH(Me)CO_2Me$ |
| $CF_3$ | H | Et | H | H | H | O | - | - | 0 | F | Cl | $OCH(Me)CO_2Et$ |
| $CF_3$ | H | Et | H | H | H | O | - | - | 0 | F | Cl | $CO_2H$ |
| $CF_3$ | H | Et | H | H | H | O | - | - | 0 | F | Cl | CN |
| $CF_3$ | H | Et | H | H | H | O | - | - | 0 | F | Cl | Me |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Et | H | H | H | O | - | - | 0 | F | Cl | Et |
| $CF_3$ | H | Et | H | H | H | O | - | - | 0 | F | Cl | CL |
| $CF_3$ | H | Et | H | H | H | O | - | - | 0 | F | Cl | Br |
| $CF_3$ | H | Et | H | H | H | O | - | - | 0 | F | Cl | I |
| $CF_3$ | H | Et | H | H | H | O | - | - | 0 | F | Cl | F |
| $CF_3$ | H | Et | H | H | H | O | - | - | 0 | F | Cl | SH |
| $CF_3$ | H | Et | H | H | H | O | - | - | 0 | F | Cl | $C(Me)=NOCH_2CO_2Me$ |
| $CF_3$ | H | Et | H | H | H | O | - | - | 0 | F | Cl | $C(Me)=NOCH_2CO_2Et$ |
| $CF_3$ | H | Et | H | H | H | O | - | - | 0 | F | Cl | $CH=NOCH_2CO_2Me$ |
| $CF_3$ | H | Et | H | H | H | O | - | - | 0 | F | Cl | $CH=NOCH_2CO_2Et$ |
| $CF_3$ | H | Et | H | H | H | O | - | - | 0 | F | Cl | $C(OMe)=NOCH_2CO_2Me$ |
| $CF_3$ | H | Et | H | H | H | O | - | - | 0 | F | Cl | $C(OMe)=NOCH_2CO_2Et$ |
| $CF_3$ | H | Et | H | H | H | O | - | - | 0 | F | Cl | $C(Me)=NN=C(Me)CO_2Me$ |
| $CF_3$ | H | Et | H | H | H | O | - | - | 0 | F | Cl | $C(Me)=NN=C(Me)CO_2Et$ |
| $CF_3$ | H | Et | H | H | H | O | - | - | 0 | F | Cl | $CH_2OCH_2CO_2Me$ |
| $CF_3$ | H | Et | H | H | H | O | - | - | 0 | F | Cl | $CH_2OCH_2CO_2Et$ |
| $CF_3$ | H | Et | H | H | H | O | - | - | 0 | F | Cl | $CH_2OCH(Me)CO_2Me$ |
| $CF_3$ | H | Et | H | H | H | O | - | - | 0 | F | Cl | $CH_2OCH(Me)CO_2Et$ |
| $CF_3$ | H | Et | H | H | H | O | - | - | 0 | F | Cl | $CH_2SCH_2CO_2Me$ |
| $CF_3$ | H | Et | H | H | H | O | - | - | 0 | F | Cl | $CH_2SCH_2CO_2Et$ |
| $CF_3$ | H | Et | H | H | H | O | - | - | 0 | F | Cl | $CH_2SCH(Me)CO_2Me$ |
| $CF_3$ | H | Et | H | H | H | O | - | - | 0 | F | Cl | $CH_2SCH(Me)CO_2Et$ |
| $CF_3$ | H | Et | H | H | H | O | - | - | 0 | F | Cl | SMe |
| $CF_3$ | H | Et | H | H | H | O | - | - | 0 | F | Cl | SEt |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Et | H | H | H | O | – | – | 0 | F | Cl | $SPr^i$ |
| $CF_3$ | H | Et | H | H | H | O | – | – | 0 | F | Cl | $SPr^n$ |
| $CF_3$ | H | Et | H | H | H | O | – | – | 0 | F | Cl | $SPen^c$ |
| $CF_3$ | H | Et | H | H | H | O | – | – | 0 | F | Cl | $SCH_2CO_2H$ |
| $CF_3$ | H | Et | H | H | H | O | – | – | 0 | F | Cl | $SCH_2CO_2Me$ |
| $CF_3$ | H | Et | H | H | H | O | – | – | 0 | F | Cl | $SCH_2CO_2Et$ |
| $CF_3$ | H | Et | H | H | H | O | – | – | 0 | F | Cl | $SCH(Me)CO_2Me$ |
| $CF_3$ | H | Et | H | H | H | O | – | – | 0 | F | Cl | $SCH(Me)CO_2Et$ |
| $CF_3$ | H | Et | H | H | H | O | – | – | 0 | F | Cl | $SCH_2CN$ |
| $CF_3$ | H | Et | H | H | H | O | – | – | 0 | F | Cl | $SCH_2CH=CH_2$ |
| $CF_3$ | H | Et | H | H | H | O | – | – | 0 | F | Cl | $SCH_2C\equiv CH$ |
| $CF_3$ | H | Et | H | H | H | O | – | – | 0 | F | Cl | $OCH(Me)C\equiv CH$ |
| $CF_3$ | H | Et | H | H | H | O | – | – | 0 | F | Cl | $SCH(Me)C\equiv CH$ |
| $CF_3$ | H | Et | H | H | H | O | – | – | 0 | F | Cl | $N(SO_2Me)_2$ |
| $CF_3$ | H | Et | H | H | H | O | – | – | 0 | F | Cl | $N(SO_2Et)_2$ |
| $CF_3$ | H | Et | H | H | H | O | – | – | 0 | F | Cl | Q1(R29=$Bu^t$) |
| $CF_3$ | H | Et | H | H | H | O | – | – | 0 | F | Cl | Q2(R29=$Bu^t$) |
| $CF_3$ | H | Et | H | H | H | O | – | – | 0 | F | Cl | Q5(R29=$Bu^t$) |
| $CF_3$ | H | Et | H | H | H | O | – | – | 0 | F | Cl | Q7(R34=$Bu^t$) |
| $CF_3$ | H | Et | H | H | H | O | – | – | 0 | F | Cl | Q9(R38=R39=H) |
| $CF_3$ | H | Et | H | H | H | O | – | – | 0 | F | Cl | Q10(R38=R39=H) |
| $CF_3$ | H | Et | H | H | H | O | – | – | 0 | F | Cl | Q4(R29=$Bu^t$) |
| $CF_3$ | H | Et | H | H | H | O | – | – | 0 | F | Cl | Q8(R35=R36=R37=H) |
| $CF_3$ | H | Et | H | H | H | O | – | – | 0 | F | Cl | Q18(R50=H) |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Et | H | H | H | O | - | - | 0 | F | Cl | Q18(R50=Me) |
| $CF_3$ | H | Et | H | H | H | O | - | - | 0 | F | Cl | $NHCO_2Me$ |
| $CF_3$ | H | Et | H | H | H | O | - | - | 0 | F | Cl | $NHCO_2Et$ |
| $CF_3$ | H | Et | H | H | H | O | - | - | 0 | F | Cl | $NHCO_2Pr^n$ |
| $CF_3$ | H | Et | H | H | H | O | - | - | 0 | F | Cl | $NHCO_2Pr^i$ |
| $CF_3$ | H | Et | H | H | H | O | - | - | 0 | F | Cl | $NHCO_2Ph$ |
| $CF_3$ | H | Et | H | H | H | O | - | - | 0 | F | Cl | $NHCO_2Bz$ |
| $CF_3$ | H | Et | H | H | H | O | - | - | 0 | F | Cl | NHCOMe |
| $CF_3$ | H | Et | H | H | H | O | - | - | 0 | F | Cl | $NHCO_2CH_2Ph$ |
| $CF_3$ | H | Et | H | H | H | O | - | - | 0 | F | Cl | $NHCO_2CH_2$(4-Me-Ph) |
| $CF_3$ | H | Et | H | H | H | O | - | - | 0 | F | Cl | $NHCO_2CH_2$(3-Me-Ph) |
| $CF_3$ | H | Et | H | H | H | O | - | - | 0 | F | Cl | $NHCO_2CH_2$(2-Me-Ph) |
| $CF_3$ | H | Et | H | H | H | O | - | - | 0 | F | Cl | $NHCO_2CH_2$(4-CL-Ph) |
| $CF_3$ | H | Et | H | H | H | O | - | - | 0 | F | Cl | $NHCO_2CH_2$(3-CL-Ph) |
| $CF_3$ | H | Et | H | H | H | O | - | - | 0 | F | Cl | $NHCO_2CH_2$(2-CL-Ph) |
| $CF_3$ | H | Et | H | H | H | O | - | - | 0 | F | Cl | Q1(R29=Me) |
| $CF_3$ | H | Et | H | H | H | O | - | - | 0 | F | Cl | Q2(R29=Me) |
| $CF_3$ | H | Et | H | H | H | O | - | - | 0 | F | Cl | H |
| $CF_3$ | H | Et | H | H | H | O | - | - | 0 | F | Cl | $CO_2H$ |
| $CF_3$ | H | Et | H | H | H | O | - | - | 0 | F | Cl | $CO_2Me$ |
| $CF_3$ | H | Et | H | H | H | O | - | - | 0 | F | Cl | $CO_2Et$ |
| Et | H | Me | H | H | H | O | - | - | 0 | F | Cl | $CO_2Pr^i$ |
| Et | H | Me | H | H | H | O | - | - | 0 | F | Cl | $CO_2Pr^n$ |
| Et | H | Me | H | H | H | O | - | - | 0 | F | Cl | $CO_2Bu^n$ |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | H | H | O | - | - | 0 | F | Cl | $CO_2Bu^t$ |
| Et | H | Me | H | H | H | O | - | - | 0 | F | Cl | $NO_2$ |
| Et | H | Me | H | H | H | O | - | - | 0 | F | Cl | $NH_2$ |
| Et | H | Me | H | H | H | O | - | - | 0 | F | Cl | $NHSO_2Me$ |
| Et | H | Me | H | H | H | O | - | - | 0 | F | Cl | $NHSO_2Et$ |
| Et | H | Me | H | H | H | O | - | - | 0 | F | Cl | $NHSO_2Pr^n$ |
| Et | H | Me | H | H | H | O | - | - | 0 | F | Cl | $NHSO_2Pr^i$ |
| Et | H | Me | H | H | H | O | - | - | 0 | F | Cl | OH |
| Et | H | Me | H | H | H | O | - | - | 0 | F | Cl | OMe |
| Et | H | Me | H | H | H | O | - | - | 0 | F | Cl | OEt |
| Et | H | Me | H | H | H | O | - | - | 0 | F | Cl | $OPr^i$ |
| Et | H | Me | H | H | H | O | - | - | 0 | F | Cl | $OPr^n$ |
| Et | H | Me | H | H | H | O | - | - | 0 | F | Cl | $OPen^c$ |
| Et | H | Me | H | H | H | O | - | - | 0 | F | Cl | $OCH_2CH=CH_2$ |
| Et | H | Me | H | H | H | O | - | - | 0 | F | Cl | $OCH_2C \equiv CH$ |
| Et | H | Me | H | H | H | O | - | - | 0 | F | Cl | $OCH_2CO_2Me$ |
| Et | H | Me | H | H | H | O | - | - | 0 | F | Cl | $OCH_2CO_2Et$ |
| Et | H | Me | H | H | H | O | - | - | 0 | F | Cl | $OCH(Me)CO_2Me$ |
| Et | H | Me | H | H | H | O | - | - | 0 | F | Cl | $OCH(Me)CO_2Et$ |
| Et | H | Me | H | H | H | O | - | - | 0 | F | Cl | $CO_2H$ |
| Et | H | Me | H | H | H | O | - | - | 0 | F | Cl | CN |
| Et | H | Me | H | H | H | O | - | - | 0 | F | Cl | Me |
| Et | H | Me | H | H | H | O | - | - | 0 | F | Cl | Et |
| Et | H | Me | H | H | H | O | - | - | 0 | F | Cl | Cl |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | H | H | O | - | - | 0 | F | Cl | Br |
| Et | H | Me | H | H | H | O | - | - | 0 | F | Cl | I |
| Et | H | Me | H | H | H | O | - | - | 0 | F | Cl | F |
| Et | H | Me | H | H | H | O | - | - | 0 | F | Cl | SH |
| Et | H | Me | H | H | H | O | - | - | 0 | F | Cl | $C(Me)=NOCH_2CO_2Me$ |
| Et | H | Me | H | H | H | O | - | - | 0 | F | Cl | $C(Me)=NOCH_2CO_2Et$ |
| Et | H | Me | H | H | H | O | - | - | 0 | F | Cl | $CH=NOCH_2CO_2Me$ |
| Et | H | Me | H | H | H | O | - | - | 0 | F | Cl | $CH=NOCH_2CO_2Et$ |
| Et | H | Me | H | H | H | O | - | - | 0 | F | Cl | $C(OMe)=NOCH_2CO_2Me$ |
| Et | H | Me | H | H | H | O | - | - | 0 | F | Cl | $C(OMe)=NOCH_2CO_2Et$ |
| Et | H | Me | H | H | H | O | - | - | 0 | F | Cl | $C(Me)=NN=C(Me)CO_2Me$ |
| Et | H | Me | H | H | H | O | - | - | 0 | F | Cl | $C(Me)=NN=C(Me)CO_2Et$ |
| Et | H | Me | H | H | H | O | - | - | 0 | F | Cl | $CH_2OCH_2CO_2Me$ |
| Et | H | Me | H | H | H | O | - | - | 0 | F | Cl | $CH_2OCH_2CO_2Et$ |
| Et | H | Me | H | H | H | O | - | - | 0 | F | Cl | $CH_2OCH(Me)CO_2Me$ |
| Et | H | Me | H | H | H | O | - | - | 0 | F | Cl | $CH_2OCH(Me)CO_2Et$ |
| Et | H | Me | H | H | H | O | - | - | 0 | F | Cl | $CH_2SCH_2CO_2Me$ |
| Et | H | Me | H | H | H | O | - | - | 0 | F | Cl | $CH_2SCH_2CO_2Et$ |
| Et | H | Me | H | H | H | O | - | - | 0 | F | Cl | $CH_2SCH(Me)CO_2Me$ |
| Et | H | Me | H | H | H | O | - | - | 0 | F | Cl | $CH_2SCH(Me)CO_2Et$ |
| Et | H | Me | H | H | H | O | - | - | 0 | F | Cl | SMe |
| Et | H | Me | H | H | H | O | - | - | 0 | F | Cl | SEt |
| Et | H | Me | H | H | H | O | - | - | 0 | F | Cl | $SPr^i$ |
| Et | H | Me | H | H | H | O | - | - | 0 | F | Cl | $SPr^n$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | H | H | O | - | - | 0 | F | Cl | SPen[c] |
| Et | H | Me | H | H | H | O | - | - | 0 | F | Cl | $SCH_2CO_2H$ |
| Et | H | Me | H | H | H | O | - | - | 0 | F | Cl | $SCH_2CO_2Me$ |
| Et | H | Me | H | H | H | O | - | - | 0 | F | Cl | $SCH_2CO_2Et$ |
| Et | H | Me | H | H | H | O | - | - | 0 | F | Cl | $SCH(Me)CO_2Me$ |
| Et | H | Me | H | H | H | O | - | - | 0 | F | Cl | $SCH(Me)CO_2Et$ |
| Et | H | Me | H | H | H | O | - | - | 0 | F | Cl | $SCH_2CN$ |
| Et | H | Me | H | H | H | O | - | - | 0 | F | Cl | $SCH_2CH=CH_2$ |
| Et | H | Me | H | H | H | O | - | - | 0 | F | Cl | $SCH_2C\equiv CH$ |
| Et | H | Me | H | H | H | O | - | - | 0 | F | Cl | $OCH(Me)C\equiv CH$ |
| Et | H | Me | H | H | H | O | - | - | 0 | F | Cl | $SCH(Me)C\equiv CH$ |
| Et | H | Me | H | H | H | O | - | - | 0 | F | Cl | $N(SO_2Me)_2$ |
| Et | H | Me | H | H | H | O | - | - | 0 | F | Cl | $N(SO_2Et)_2$ |
| Et | H | Me | H | H | H | O | - | - | 0 | F | Cl | Q1(R29=$Bu^t$) |
| Et | H | Me | H | H | H | O | - | - | 0 | F | Cl | Q2(R29=$Bu^t$) |
| Et | H | Me | H | H | H | O | - | - | 0 | F | Cl | Q5(R29=$Bu^t$) |
| Et | H | Me | H | H | H | O | - | - | 0 | F | Cl | Q7(R34=$Bu^t$) |
| Et | H | Me | H | H | H | O | - | - | 0 | F | Cl | Q9(R38=R39=H) |
| Et | H | Me | H | H | H | O | - | - | 0 | F | Cl | Q10(R38=R39=H) |
| Et | H | Me | H | H | H | O | - | - | 0 | F | Cl | Q4(R29=$Bu^t$) |
| Et | H | Me | H | H | H | O | - | - | 0 | F | Cl | Q8(R35=R36=R37=H) |
| Et | H | Me | H | H | H | O | - | - | 0 | F | Cl | Q18(R50=H) |
| Et | H | Me | H | H | H | O | - | - | 0 | F | Cl | Q18(R50=Me) |
| Et | H | Me | H | H | H | O | - | - | 0 | F | Cl | $NHCO_2Me$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | H | H | O | - | - | 0 | F | Cl | $NHCO_2Et$ |
| Et | H | Me | H | H | H | O | - | - | 0 | F | Cl | $NHCO_2Pr^n$ |
| Et | H | Me | H | H | H | O | - | - | 0 | F | Cl | $NHCO_2Pr^i$ |
| Et | H | Me | H | H | H | O | - | - | 0 | F | Cl | $NHCO_2Ph$ |
| Et | H | Me | H | H | H | O | - | - | 0 | F | Cl | $NHCO_2Bz$ |
| Et | H | Me | H | H | H | O | - | - | 0 | F | Cl | NHCOMe |
| Et | H | Me | H | H | H | O | - | - | 0 | F | Cl | $NHCO_2CH_2Ph$ |
| Et | H | Me | H | H | H | O | - | - | 0 | F | Cl | $NHCO_2CH_2$(4-Me-Ph) |
| Et | H | Me | H | H | H | O | - | - | 0 | F | Cl | $NHCO_2CH_2$(3-Me-Ph) |
| Et | H | Me | H | H | H | O | - | - | 0 | F | Cl | $NHCO_2CH_2$(2-Me-Ph) |
| Et | H | Me | H | H | H | O | - | - | 0 | F | Cl | $NHCO_2CH_2$(4-CL-Ph) |
| Et | H | Me | H | H | H | O | - | - | 0 | F | Cl | $NHCO_2CH_2$(3-CL-Ph) |
| Et | H | Me | H | H | H | O | - | - | 0 | F | Cl | $NHCO_2CH_2$(2-CL-Ph) |
| Et | H | Me | H | H | H | O | - | - | 0 | F | Cl | Q1(R29=Me) |
| Et | H | Me | H | H | H | O | - | - | 0 | F | Cl | Q2(R29=Me) |
| Et | H | Me | H | H | H | O | - | - | 0 | F | Cl | H |
| Et | H | Me | H | H | H | O | - | - | 0 | F | Cl | $CO_2H$ |
| Et | H | Me | H | H | H | O | - | - | 0 | F | Cl | $CO_2Me$ |
| Et | H | Me | H | H | H | O | - | - | 0 | F | Cl | $CO_2Et$ |
| $Pr^n$ | H | Me | H | H | H | O | - | - | 0 | F | Cl | $CO_2Pr^i$ |
| $Pr^n$ | H | Me | H | H | H | O | - | - | 0 | F | Cl | $CO_2Pr^n$ |
| $Pr^n$ | H | Me | H | H | H | O | - | - | 0 | F | Cl | $CO_2Bu^n$ |
| $Pr^n$ | H | Me | H | H | H | O | - | - | 0 | F | Cl | $CO_2Bu^t$ |
| $Pr^n$ | H | Me | H | H | H | O | - | - | 0 | F | Cl | $NO_2$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | H | H | O | - | - | 0 | F | Cl | $NH_2$ |
| $Pr^n$ | H | Me | H | H | H | O | - | - | 0 | F | Cl | $NHSO_2Me$ |
| $Pr^n$ | H | Me | H | H | H | O | - | - | 0 | F | Cl | $NHSO_2Et$ |
| $Pr^n$ | H | Me | H | H | H | O | - | - | 0 | F | Cl | $NHSO_2Pr^n$ |
| $Pr^n$ | H | Me | H | H | H | O | - | - | 0 | F | Cl | $NHSO_2Pr^i$ |
| $Pr^n$ | H | Me | H | H | H | O | - | - | 0 | F | Cl | OH |
| $Pr^n$ | H | Me | H | H | H | O | - | - | 0 | F | Cl | OMe |
| $Pr^n$ | H | Me | H | H | H | O | - | - | 0 | F | Cl | OEt |
| $Pr^n$ | H | Me | H | H | H | O | - | - | 0 | F | Cl | $OPr^i$ |
| $Pr^n$ | H | Me | H | H | H | O | - | - | 0 | F | Cl | $OPr^n$ |
| $Pr^n$ | H | Me | H | H | H | O | - | - | 0 | F | Cl | $OPen^c$ |
| $Pr^n$ | H | Me | H | H | H | O | - | - | 0 | F | Cl | $OCH_2CH=CH_2$ |
| $Pr^n$ | H | Me | H | H | H | O | - | - | 0 | F | Cl | $OCH_2C\equiv CH$ |
| $Pr^n$ | H | Me | H | H | H | O | - | - | 0 | F | Cl | $OCH_2CO_2Me$ |
| $Pr^n$ | H | Me | H | H | H | O | - | - | 0 | F | Cl | $OCH_2CO_2Et$ |
| $Pr^n$ | H | Me | H | H | H | O | - | - | 0 | F | Cl | $OCH(Me)CO_2Me$ |
| $Pr^n$ | H | Me | H | H | H | O | - | - | 0 | F | Cl | $OCH(Me)CO_2Et$ |
| $Pr^n$ | H | Me | H | H | H | O | - | - | 0 | F | Cl | $CO_2H$ |
| $Pr^n$ | H | Me | H | H | H | O | - | - | 0 | F | Cl | CN |
| $Pr^n$ | H | Me | H | H | H | O | - | - | 0 | F | Cl | Me |
| $Pr^n$ | H | Me | H | H | H | O | - | - | 0 | F | Cl | Et |
| $Pr^n$ | H | Me | H | H | H | O | - | - | 0 | F | Cl | Cl |
| $Pr^n$ | H | Me | H | H | H | O | - | - | 0 | F | Cl | Br |
| $Pr^n$ | H | Me | H | H | H | O | - | - | 0 | F | Cl | I |

Table 1－3－1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | H | H | O | － | － | 0 | F | Cl | F |
| $Pr^n$ | H | Me | H | H | H | O | － | － | 0 | F | Cl | SH |
| $Pr^n$ | H | Me | H | H | H | O | － | － | 0 | F | Cl | $C(Me)=NOCH_2CO_2Me$ |
| $Pr^n$ | H | Me | H | H | H | O | － | － | 0 | F | Cl | $C(Me)=NOCH_2CO_2Et$ |
| $Pr^n$ | H | Me | H | H | H | O | － | － | 0 | F | Cl | $CH=NOCH_2CO_2Me$ |
| $Pr^n$ | H | Me | H | H | H | O | － | － | 0 | F | Cl | $CH=NOCH_2CO_2Et$ |
| $Pr^n$ | H | Me | H | H | H | O | － | － | 0 | F | Cl | $C(OMe)=NOCH_2CO_2Me$ |
| $Pr^n$ | H | Me | H | H | H | O | － | － | 0 | F | Cl | $C(OMe)=NOCH_2CO_2Et$ |
| $Pr^n$ | H | Me | H | H | H | O | － | － | 0 | F | Cl | $C(Me)=NN=C(Me)CO_2Me$ |
| $Pr^n$ | H | Me | H | H | H | O | － | － | 0 | F | Cl | $C(Me)=NN=C(Me)CO_2Et$ |
| $Pr^n$ | H | Me | H | H | H | O | － | － | 0 | F | Cl | $CH_2OCH_2CO_2Me$ |
| $Pr^n$ | H | Me | H | H | H | O | － | － | 0 | F | Cl | $CH_2OCH_2CO_2Et$ |
| $Pr^n$ | H | Me | H | H | H | O | － | － | 0 | F | Cl | $CH_2OCH(Me)CO_2Me$ |
| $Pr^n$ | H | Me | H | H | H | O | － | － | 0 | F | Cl | $CH_2OCH(Me)CO_2Et$ |
| $Pr^n$ | H | Me | H | H | H | O | － | － | 0 | F | Cl | $CH_2SCH_2CO_2Me$ |
| $Pr^n$ | H | Me | H | H | H | O | － | － | 0 | F | Cl | $CH_2SCH_2CO_2Et$ |
| $Pr^n$ | H | Me | H | H | H | O | － | － | 0 | F | Cl | $CH_2SCH(Me)CO_2Me$ |
| $Pr^n$ | H | Me | H | H | H | O | － | － | 0 | F | Cl | $CH_2SCH(Me)CO_2Et$ |
| $Pr^n$ | H | Me | H | H | H | O | － | － | 0 | F | Cl | SMe |
| $Pr^n$ | H | Me | H | H | H | O | － | － | 0 | F | Cl | SEt |
| $Pr^n$ | H | Me | H | H | H | O | － | － | 0 | F | Cl | $SPr^i$ |
| $Pr^n$ | H | Me | H | H | H | O | － | － | 0 | F | Cl | $SPr^n$ |
| $Pr^n$ | H | Me | H | H | H | O | － | － | 0 | F | Cl | $SPen^c$ |
| $Pr^n$ | H | Me | H | H | H | O | － | － | 0 | F | Cl | $SCH_2CO_2H$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | H | H | O | - | - | 0 | F | Cl | $SCH_2CO_2Me$ |
| $Pr^n$ | H | Me | H | H | H | O | - | - | 0 | F | Cl | $SCH_2CO_2Et$ |
| $Pr^n$ | H | Me | H | H | H | O | - | - | 0 | F | Cl | $SCH(Me)CO_2Me$ |
| $Pr^n$ | H | Me | H | H | H | O | - | - | 0 | F | Cl | $SCH(Me)CO_2Et$ |
| $Pr^n$ | H | Me | H | H | H | O | - | - | 0 | F | Cl | $SCH_2CN$ |
| $Pr^n$ | H | Me | H | H | H | O | - | - | 0 | F | Cl | $SCH_2CH{=}CH_2$ |
| $Pr^n$ | H | Me | H | H | H | O | - | - | 0 | F | Cl | $SCH_2C{\equiv}CH$ |
| $Pr^n$ | H | Me | H | H | H | O | - | - | 0 | F | Cl | $OCH(Me)C{\equiv}CH$ |
| $Pr^n$ | H | Me | H | H | H | O | - | - | 0 | F | Cl | $SCH(Me)C{\equiv}CH$ |
| $Pr^n$ | H | Me | H | H | H | O | - | - | 0 | F | Cl | $N(SO_2Me)_2$ |
| $Pr^n$ | H | Me | H | H | H | O | - | - | 0 | F | Cl | $N(SO_2Et)_2$ |
| $Pr^n$ | H | Me | H | H | H | O | - | - | 0 | F | Cl | Q1(R29=$Bu^t$) |
| $Pr^n$ | H | Me | H | H | H | O | - | - | 0 | F | Cl | Q2(R29=$Bu^t$) |
| $Pr^n$ | H | Me | H | H | H | O | - | - | 0 | F | Cl | Q5(R29=$Bu^t$) |
| $Pr^n$ | H | Me | H | H | H | O | - | - | 0 | F | Cl | Q7(R34=$Bu^t$) |
| $Pr^n$ | H | Me | H | H | H | O | - | - | 0 | F | Cl | Q9(R38=R39=H) |
| $Pr^n$ | H | Me | H | H | H | O | - | - | 0 | F | Cl | Q10(R38=R39=H) |
| $Pr^n$ | H | Me | H | H | H | O | - | - | 0 | F | Cl | Q4(R29=$Bu^t$) |
| $Pr^n$ | H | Me | H | H | H | O | - | - | 0 | F | Cl | Q8(R35=R36=R37=H) |
| $Pr^n$ | H | Me | H | H | H | O | - | - | 0 | F | Cl | Q18(R50=H) |
| $Pr^n$ | H | Me | H | H | H | O | - | - | 0 | F | Cl | Q18(R50=Me) |
| $Pr^n$ | H | Me | H | H | H | O | - | - | 0 | F | Cl | $NHCO_2Me$ |
| $Pr^n$ | H | Me | H | H | H | O | - | - | 0 | F | Cl | $NHCO_2Et$ |
| $Pr^n$ | H | Me | H | H | H | O | - | - | 0 | F | Cl | $NHCO_2Pr^n$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | H | H | O | - | - | 0 | F | Cl | $NHCO_2Pr^i$ |
| $Pr^n$ | H | Me | H | H | H | O | - | - | 0 | F | Cl | $NHCO_2Ph$ |
| $Pr^n$ | H | Me | H | H | H | O | - | - | 0 | F | Cl | $NHCO_2Bz$ |
| $Pr^n$ | H | Me | H | H | H | O | - | - | 0 | F | Cl | NHCOMe |
| $Pr^n$ | H | Me | H | H | H | O | - | - | 0 | F | Cl | $NHCO_2CH_2Ph$ |
| $Pr^n$ | H | Me | H | H | H | O | - | - | 0 | F | Cl | $NHCO_2CH_2$(4-Me-Ph) |
| $Pr^n$ | H | Me | H | H | H | O | - | - | 0 | F | Cl | $NHCO_2CH_2$(3-Me-Ph) |
| $Pr^n$ | H | Me | H | H | H | O | - | - | 0 | F | Cl | $NHCO_2CH_2$(2-Me-Ph) |
| $Pr^n$ | H | Me | H | H | H | O | - | - | 0 | F | Cl | $NHCO_2CH_2$(4-Cl-Ph) |
| $Pr^n$ | H | Me | H | H | H | O | - | - | 0 | F | Cl | $NHCO_2CH_2$(3-Cl-Ph) |
| $Pr^n$ | H | Me | H | H | H | O | - | - | 0 | F | Cl | $NHCO_2CH_2$(2-Cl-Ph) |
| $Pr^n$ | H | Me | H | H | H | O | - | - | 0 | F | Cl | Q1(R29=Me) |
| $Pr^n$ | H | Me | H | H | H | O | - | - | 0 | F | Cl | Q2(R29=Me) |
| $Pr^n$ | H | Me | H | H | H | O | - | - | 0 | F | Cl | H |
| $Pr^n$ | H | Me | H | H | H | O | - | - | 0 | F | Cl | $CO_2H$ |
| $Pr^n$ | H | Me | H | H | H | O | - | - | 0 | F | Cl | $CO_2Me$ |
| $Pr^n$ | H | Me | H | H | H | O | - | - | 0 | F | Cl | $CO_2Et$ |
| $Pr^i$ | H | Me | H | H | H | O | - | - | 0 | F | Cl | $CO_2Pr^i$ |
| $Pr^i$ | H | Me | H | H | H | O | - | - | 0 | F | Cl | $CO_2Pr^n$ |
| $Pr^i$ | H | Me | H | H | H | O | - | - | 0 | F | Cl | $CO_2Bu^n$ |
| $Pr^i$ | H | Me | H | H | H | O | - | - | 0 | F | Cl | $CO_2Bu^t$ |
| $Pr^i$ | H | Me | H | H | H | O | - | - | 0 | F | Cl | $NO_2$ |
| $Pr^i$ | H | Me | H | H | H | O | - | - | 0 | F | Cl | $NH_2$ |
| $Pr^i$ | H | Me | H | H | H | O | - | - | 0 | F | Cl | $NHSO_2Me$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | H | H | O | - | - | 0 | F | Cl | $NHSO_2Et$ |
| $Pr^i$ | H | Me | H | H | H | O | - | - | 0 | F | Cl | $NHSO_2Pr^n$ |
| $Pr^i$ | H | Me | H | H | H | O | - | - | 0 | F | Cl | $NHSO_2Pr^i$ |
| $Pr^i$ | H | Me | H | H | H | O | - | - | 0 | F | Cl | OH |
| $Pr^i$ | H | Me | H | H | H | O | - | - | 0 | F | Cl | OMe |
| $Pr^i$ | H | Me | H | H | H | O | - | - | 0 | F | Cl | OEt |
| $Pr^i$ | H | Me | H | H | H | O | - | - | 0 | F | Cl | $OPr^i$ |
| $Pr^i$ | H | Me | H | H | H | O | - | - | 0 | F | Cl | $OPr^n$ |
| $Pr^i$ | H | Me | H | H | H | O | - | - | 0 | F | Cl | $OPen^c$ |
| $Pr^i$ | H | Me | H | H | H | O | - | - | 0 | F | Cl | $OCH_2CH=CH_2$ |
| $Pr^i$ | H | Me | H | H | H | O | - | - | 0 | F | Cl | $OCH_2C{\equiv}CH$ |
| $Pr^i$ | H | Me | H | H | H | O | - | - | 0 | F | Cl | $OCH_2CO_2Me$ |
| $Pr^i$ | H | Me | H | H | H | O | - | - | 0 | F | Cl | $OCH_2CO_2Et$ |
| $Pr^i$ | H | Me | H | H | H | O | - | - | 0 | F | Cl | $OCH(Me)CO_2Me$ |
| $Pr^i$ | H | Me | H | H | H | O | - | - | 0 | F | Cl | $OCH(Me)CO_2Et$ |
| $Pr^i$ | H | Me | H | H | H | O | - | - | 0 | F | Cl | $CO_2H$ |
| $Pr^i$ | H | Me | H | H | H | O | - | - | 0 | F | Cl | CN |
| $Pr^i$ | H | Me | H | H | H | O | - | - | 0 | F | Cl | Me |
| $Pr^i$ | H | Me | H | H | H | O | - | - | 0 | F | Cl | Et |
| $Pr^i$ | H | Me | H | H | H | O | - | - | 0 | F | Cl | Cl |
| $Pr^i$ | H | Me | H | H | H | O | - | - | 0 | F | Cl | Br |
| $Pr^i$ | H | Me | H | H | H | O | - | - | 0 | F | Cl | I |
| $Pr^i$ | H | Me | H | H | H | O | - | - | 0 | F | Cl | F |
| $Pr^i$ | H | Me | H | H | H | O | - | - | 0 | F | Cl | SH |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | H | H | O | - | - | 0 | F | Cl | $C(Me)=NOCH_2CO_2Me$ |
| $Pr^i$ | H | Me | H | H | H | O | - | - | 0 | F | Cl | $C(Me)=NOCH_2CO_2Et$ |
| $Pr^i$ | H | Me | H | H | H | O | - | - | 0 | F | Cl | $CH=NOCH_2CO_2Me$ |
| $Pr^i$ | H | Me | H | H | H | O | - | - | 0 | F | Cl | $CH=NOCH_2CO_2Et$ |
| $Pr^i$ | H | Me | H | H | H | O | - | - | 0 | F | Cl | $C(OMe)=NOCH_2CO_2Me$ |
| $Pr^i$ | H | Me | H | H | H | O | - | - | 0 | F | Cl | $C(OMe)=NOCH_2CO_2Et$ |
| $Pr^i$ | H | Me | H | H | H | O | - | - | 0 | F | Cl | $C(Me)=NN=C(Me)CO_2Me$ |
| $Pr^i$ | H | Me | H | H | H | O | - | - | 0 | F | Cl | $C(Me)=NN=C(Me)CO_2Et$ |
| $Pr^i$ | H | Me | H | H | H | O | - | - | 0 | F | Cl | $CH_2OCH_2CO_2Me$ |
| $Pr^i$ | H | Me | H | H | H | O | - | - | 0 | F | Cl | $CH_2OCH_2CO_2Et$ |
| $Pr^i$ | H | Me | H | H | H | O | - | - | 0 | F | Cl | $CH_2OCH(Me)CO_2Me$ |
| $Pr^i$ | H | Me | H | H | H | O | - | - | 0 | F | Cl | $CH_2OCH(Me)CO_2Et$ |
| $Pr^i$ | H | Me | H | H | H | O | - | - | 0 | F | Cl | $CH_2SCH_2CO_2Me$ |
| $Pr^i$ | H | Me | H | H | H | O | - | - | 0 | F | Cl | $CH_2SCH_2CO_2Et$ |
| $Pr^i$ | H | Me | H | H | H | O | - | - | 0 | F | Cl | $CH_2SCH(Me)CO_2Me$ |
| $Pr^i$ | H | Me | H | H | H | O | - | - | 0 | F | Cl | $CH_2SCH(Me)CO_2Et$ |
| $Pr^i$ | H | Me | H | H | H | O | - | - | 0 | F | Cl | SMe |
| $Pr^i$ | H | Me | H | H | H | O | - | - | 0 | F | Cl | SEt |
| $Pr^i$ | H | Me | H | H | H | O | - | - | 0 | F | Cl | $SPr^i$ |
| $Pr^i$ | H | Me | H | H | H | O | - | - | 0 | F | Cl | $SPr^n$ |
| $Pr^i$ | H | Me | H | H | H | O | - | - | 0 | F | Cl | $SPen^c$ |
| $Pr^i$ | H | Me | H | H | H | O | - | - | 0 | F | Cl | $SCH_2CO_2H$ |
| $Pr^i$ | H | Me | H | H | H | O | - | - | 0 | F | Cl | $SCH_2CO_2Me$ |
| $Pr^i$ | H | Me | H | H | H | O | - | - | 0 | F | Cl | $SCH_2CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | H | H | O | - | - | 0 | F | Cl | $SCH(Me)CO_2Me$ |
| $Pr^i$ | H | Me | H | H | H | O | - | - | 0 | F | Cl | $SCH(Me)CO_2Et$ |
| $Pr^i$ | H | Me | H | H | H | O | - | - | 0 | F | Cl | $SCH_2CN$ |
| $Pr^i$ | H | Me | H | H | H | O | - | - | 0 | F | Cl | $SCH_2CH=CH_2$ |
| $Pr^i$ | H | Me | H | H | H | O | - | - | 0 | F | Cl | $SCH_2C\equiv CH$ |
| $Pr^i$ | H | Me | H | H | H | O | - | - | 0 | F | Cl | $OCH(Me)C\equiv CH$ |
| $Pr^i$ | H | Me | H | H | H | O | - | - | 0 | F | Cl | $SCH(Me)C\equiv CH$ |
| $Pr^i$ | H | Me | H | H | H | O | - | - | 0 | F | Cl | $N(SO_2Me)_2$ |
| $Pr^i$ | H | Me | H | H | H | O | - | - | 0 | F | Cl | $N(SO_2Et)_2$ |
| $Pr^i$ | H | Me | H | H | H | O | - | - | 0 | F | Cl | Q1($R29=Bu^t$) |
| $Pr^i$ | H | Me | H | H | H | O | - | - | 0 | F | Cl | Q2($R29=Bu^t$) |
| $Pr^i$ | H | Me | H | H | H | O | - | - | 0 | F | Cl | Q5($R29=Bu^t$) |
| $Pr^i$ | H | Me | H | H | H | O | - | - | 0 | F | Cl | Q7($R34=Bu^t$) |
| $Pr^i$ | H | Me | H | H | H | O | - | - | 0 | F | Cl | Q9(R38=R39=H) |
| $Pr^i$ | H | Me | H | H | H | O | - | - | 0 | F | Cl | Q10(R38=R39=H) |
| $Pr^i$ | H | Me | H | H | H | O | - | - | 0 | F | Cl | Q4($R29=Bu^t$) |
| $Pr^i$ | H | Me | H | H | H | O | - | - | 0 | F | Cl | Q8(R35=R36=R37=H) |
| $Pr^i$ | H | Me | H | H | H | O | - | - | 0 | F | Cl | Q18(R50=H) |
| $Pr^i$ | H | Me | H | H | H | O | - | - | 0 | F | Cl | Q18(R50=Me) |
| $Pr^i$ | H | Me | H | H | H | O | - | - | 0 | F | Cl | $NHCO_2Me$ |
| $Pr^i$ | H | Me | H | H | H | O | - | - | 0 | F | Cl | $NHCO_2Et$ |
| $Pr^i$ | H | Me | H | H | H | O | - | - | 0 | F | Cl | $NHCO_2Pr^n$ |
| $Pr^i$ | H | Me | H | H | H | O | - | - | 0 | F | Cl | $NHCO_2Pr^i$ |
| $Pr^i$ | H | Me | H | H | H | O | - | - | 0 | F | Cl | $NHCO_2Ph$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | H | H | O | - | - | 0 | F | Cl | $NHCO_2Bz$ |
| $Pr^i$ | H | Me | H | H | H | O | - | - | 0 | F | Cl | NHCOMe |
| $Pr^i$ | H | Me | H | H | H | O | - | - | 0 | F | Cl | $NHCO_2CH_2Ph$ |
| $Pr^i$ | H | Me | H | H | H | O | - | - | 0 | F | Cl | $NHCO_2CH_2$(4-Me-Ph) |
| $Pr^i$ | H | Me | H | H | H | O | - | - | 0 | F | Cl | $NHCO_2CH_2$(3-Me-Ph) |
| $Pr^i$ | H | Me | H | H | H | O | - | - | 0 | F | Cl | $NHCO_2CH_2$(2-Me-Ph) |
| $Pr^i$ | H | Me | H | H | H | O | - | - | 0 | F | Cl | $NHCO_2CH_2$(4-Cl-Ph) |
| $Pr^i$ | H | Me | H | H | H | O | - | - | 0 | F | Cl | $NHCO_2CH_2$(3-Cl-Ph) |
| $Pr^i$ | H | Me | H | H | H | O | - | - | 0 | F | Cl | $NHCO_2CH_2$(2-Cl-Ph) |
| $Pr^i$ | H | Me | H | H | H | O | - | - | 0 | F | Cl | Q1(R29=Me) |
| $Pr^i$ | H | Me | H | H | H | O | - | - | 0 | F | Cl | Q2(R29=Me) |
| $Pr^i$ | H | Me | H | H | H | O | - | - | 0 | F | Cl | H |
| $Pr^i$ | H | Me | H | H | H | O | - | - | 0 | F | Cl | $CO_2H$ |
| $Pr^i$ | H | Me | H | H | H | O | - | - | 0 | F | Cl | $CO_2Me$ |
| $Pr^i$ | H | Me | H | H | H | O | - | - | 0 | F | Cl | $CO_2Et$ |
| Me | H | $CF_3$ | H | H | H | O | - | - | 0 | F | Cl | $CO_2Pr^i$ |
| Me | H | $CF_3$ | H | H | H | O | - | - | 0 | F | Cl | $CO_2Pr^n$ |
| Me | H | $CF_3$ | H | H | H | O | - | - | 0 | F | Cl | $CO_2Bu^n$ |
| Me | H | $CF_3$ | H | H | H | O | - | - | 0 | F | Cl | $CO_2Bu^t$ |
| Me | H | $CF_3$ | H | H | H | O | - | - | 0 | F | Cl | $NO_2$ |
| Me | H | $CF_3$ | H | H | H | O | - | - | 0 | F | Cl | $NH_2$ |
| Me | H | $CF_3$ | H | H | H | O | - | - | 0 | F | Cl | $NHSO_2Me$ |
| Me | H | $CF_3$ | H | H | H | O | - | - | 0 | F | Cl | $NHSO_2Et$ |
| Me | H | $CF_3$ | H | H | H | O | - | - | 0 | F | Cl | $NHSO_2Pr^n$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | $CF_3$ | H | H | H | O | - | - | 0 | F | Cl | $NHSO_2Pr^i$ |
| Me | H | $CF_3$ | H | H | H | O | - | - | 0 | F | Cl | OH |
| Me | H | $CF_3$ | H | H | H | O | - | - | 0 | F | Cl | OMe |
| Me | H | $CF_3$ | H | H | H | O | - | - | 0 | F | Cl | OEt |
| Me | H | $CF_3$ | H | H | H | O | - | - | 0 | F | Cl | $OPr^i$ |
| Me | H | $CF_3$ | H | H | H | O | - | - | 0 | F | Cl | $OPr^n$ |
| Me | H | $CF_3$ | H | H | H | O | - | - | 0 | F | Cl | $OPen^c$ |
| Me | H | $CF_3$ | H | H | H | O | - | - | 0 | F | Cl | $OCH_2CH=CH_2$ |
| Me | H | $CF_3$ | H | H | H | O | - | - | 0 | F | Cl | $OCH_2C\equiv CH$ |
| Me | H | $CF_3$ | H | H | H | O | - | - | 0 | F | Cl | $OCH_2CO_2Me$ |
| Me | H | $CF_3$ | H | H | H | O | - | - | 0 | F | Cl | $OCH_2CO_2Et$ |
| Me | H | $CF_3$ | H | H | H | O | - | - | 0 | F | Cl | $OCH(Me)CO_2Me$ |
| Me | H | $CF_3$ | H | H | H | O | - | - | 0 | F | Cl | $OCH(Me)CO_2Et$ |
| Me | H | $CF_3$ | H | H | H | O | - | - | 0 | F | Cl | $CO_2H$ |
| Me | H | $CF_3$ | H | H | H | O | - | - | 0 | F | Cl | CN |
| Me | H | $CF_3$ | H | H | H | O | - | - | 0 | F | Cl | Me |
| Me | H | $CF_3$ | H | H | H | O | - | - | 0 | F | Cl | Et |
| Me | H | $CF_3$ | H | H | H | O | - | - | 0 | F | Cl | Cl |
| Me | H | $CF_3$ | H | H | H | O | - | - | 0 | F | Cl | Br |
| Me | H | $CF_3$ | H | H | H | O | - | - | 0 | F | Cl | I |
| Me | H | $CF_3$ | H | H | H | O | - | - | 0 | F | Cl | F |
| Me | H | $CF_3$ | H | H | H | O | - | - | 0 | F | Cl | SH |
| Me | H | $CF_3$ | H | H | H | O | - | - | 0 | F | Cl | $C(Me)=NOCH_2CO_2Me$ |
| Me | H | $CF_3$ | H | H | H | O | - | - | 0 | F | Cl | $C(Me)=NOCH_2CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | $CF_3$ | H | H | H | O | - | - | 0 | F | Cl | $CH=NOCH_2CO_2Me$ |
| Me | H | $CF_3$ | H | H | H | O | - | - | 0 | F | Cl | $CH=NOCH_2CO_2Et$ |
| Me | H | $CF_3$ | H | H | H | O | - | - | 0 | F | Cl | $C(OMe)=NOCH_2CO_2Me$ |
| Me | H | $CF_3$ | H | H | H | O | - | - | 0 | F | Cl | $C(OMe)=NOCH_2CO_2Et$ |
| Me | H | $CF_3$ | H | H | H | O | - | - | 0 | F | Cl | $C(Me)=NN=C(Me)CO_2Me$ |
| Me | H | $CF_3$ | H | H | H | O | - | - | 0 | F | Cl | $C(Me)=NN=C(Me)CO_2Et$ |
| Me | H | $CF_3$ | H | H | H | O | - | - | 0 | F | Cl | $CH_2OCH_2CO_2Me$ |
| Me | H | $CF_3$ | H | H | H | O | - | - | 0 | F | Cl | $CH_2OCH_2CO_2Et$ |
| Me | H | $CF_3$ | H | H | H | O | - | - | 0 | F | Cl | $CH_2OCH(Me)CO_2Me$ |
| Me | H | $CF_3$ | H | H | H | O | - | - | 0 | F | Cl | $CH_2OCH(Me)CO_2Et$ |
| Me | H | $CF_3$ | H | H | H | O | - | - | 0 | F | Cl | $CH_2SCH_2CO_2Me$ |
| Me | H | $CF_3$ | H | H | H | O | - | - | 0 | F | Cl | $CH_2SCH_2CO_2Et$ |
| Me | H | $CF_3$ | H | H | H | O | - | - | 0 | F | Cl | $CH_2SCH(Me)CO_2Me$ |
| Me | H | $CF_3$ | H | H | H | O | - | - | 0 | F | Cl | $CH_2SCH(Me)CO_2Et$ |
| Me | H | $CF_3$ | H | H | H | O | - | - | 0 | F | Cl | SMe |
| Me | H | $CF_3$ | H | H | H | O | - | - | 0 | F | Cl | SEt |
| Me | H | $CF_3$ | H | H | H | O | - | - | 0 | F | Cl | $SPr^i$ |
| Me | H | $CF_3$ | H | H | H | O | - | - | 0 | F | Cl | $SPr^n$ |
| Me | H | $CF_3$ | H | H | H | O | - | - | 0 | F | Cl | $SPen^c$ |
| Me | H | $CF_3$ | H | H | H | O | - | - | 0 | F | Cl | $SCH_2CO_2H$ |
| Me | H | $CF_3$ | H | H | H | O | - | - | 0 | F | Cl | $SCH_2CO_2Me$ |
| Me | H | $CF_3$ | H | H | H | O | - | - | 0 | F | Cl | $SCH_2CO_2Et$ |
| Me | H | $CF_3$ | H | H | H | O | - | - | 0 | F | Cl | $SCH(Me)CO_2Me$ |
| Me | H | $CF_3$ | H | H | H | O | - | - | 0 | F | Cl | $SCH(Me)CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | $CF_3$ | H | H | H | O | - | - | 0 | F | Cl | $SCH_2CN$ |
| Me | H | $CF_3$ | H | H | H | O | - | - | 0 | F | Cl | $SCH_2CH=CH_2$ |
| Me | H | $CF_3$ | H | H | H | O | - | - | 0 | F | Cl | $SCH_2C\equiv CH$ |
| Me | H | $CF_3$ | H | H | H | O | - | - | 0 | F | Cl | OCH(Me)C ≡CH |
| Me | H | $CF_3$ | H | H | H | O | - | - | 0 | F | Cl | SCH(Me)C ≡CH |
| Me | H | $CF_3$ | H | H | H | O | - | - | 0 | F | Cl | $N(SO_2Me)_2$ |
| Me | H | $CF_3$ | H | H | H | O | - | - | 0 | F | Cl | $N(SO_2Et)_2$ |
| Me | H | $CF_3$ | H | H | H | O | - | - | 0 | F | Cl | Q1(R29=$Bu^t$ ) |
| Me | H | $CF_3$ | H | H | H | O | - | - | 0 | F | Cl | Q2(R29=$Bu^t$ ) |
| Me | H | $CF_3$ | H | H | H | O | - | - | 0 | F | Cl | Q5(R29=$Bu^t$ ) |
| Me | H | $CF_3$ | H | H | H | O | - | - | 0 | F | Cl | Q7(R34=$Bu^t$ ) |
| Me | H | $CF_3$ | H | H | H | O | - | - | 0 | F | Cl | Q9(R38=R39=H) |
| Me | H | $CF_3$ | H | H | H | O | - | - | 0 | F | Cl | Q10(R38=R39=H) |
| Me | H | $CF_3$ | H | H | H | O | - | - | 0 | F | Cl | Q4(R29=$Bu^t$ ) |
| Me | H | $CF_3$ | H | H | H | O | - | - | 0 | F | Cl | Q8(R35=R36=R37=H) |
| Me | H | $CF_3$ | H | H | H | O | - | - | 0 | F | Cl | Q18(R50=H) |
| Me | H | $CF_3$ | H | H | H | O | - | - | 0 | F | Cl | Q18(R50=Me) |
| Me | H | $CF_3$ | H | H | H | O | - | - | 0 | F | Cl | $NHCO_2Me$ |
| Me | H | $CF_3$ | H | H | H | O | - | - | 0 | F | Cl | $NHCO_2Et$ |
| Me | H | $CF_3$ | H | H | H | O | - | - | 0 | F | Cl | $NHCO_2Pr^n$ |
| Me | H | $CF_3$ | H | H | H | O | - | - | 0 | F | Cl | $NHCO_2Pr^i$ |
| Me | H | $CF_3$ | H | H | H | O | - | - | 0 | F | Cl | $NHCO_2Ph$ |
| Me | H | $CF_3$ | H | H | H | O | - | - | 0 | F | Cl | $NHCO_2Bz$ |
| Me | H | $CF_3$ | H | H | H | O | - | - | 0 | F | Cl | NHCOMe |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | $CF_3$ | H | H | H | O | - | - | 0 | F | Cl | $NHCO_2CH_2Ph$ |
| Me | H | $CF_3$ | H | H | H | O | - | - | 0 | F | Cl | $NHCO_2CH_2$(4-Me-Ph) |
| Me | H | $CF_3$ | H | H | H | O | - | - | 0 | F | Cl | $NHCO_2CH_2$(3-Me-Ph) |
| Me | H | $CF_3$ | H | H | H | O | - | - | 0 | F | Cl | $NHCO_2CH_2$(2-Me-Ph) |
| Me | H | $CF_3$ | H | H | H | O | - | - | 0 | F | Cl | $NHCO_2CH_2$(4-Cl-Ph) |
| Me | H | $CF_3$ | H | H | H | O | - | - | 0 | F | Cl | $NHCO_2CH_2$(3-Cl-Ph) |
| Me | H | $CF_3$ | H | H | H | O | - | - | 0 | F | Cl | $NHCO_2CH_2$(2-Cl-Ph) |
| Me | H | $CF_3$ | H | H | H | O | - | - | 0 | F | Cl | Q1(R29=Me) |
| Me | H | $CF_3$ | H | H | H | O | - | - | 0 | F | Cl | Q2(R29=Me) |
| Me | H | $CF_3$ | H | H | H | O | - | - | 0 | F | Cl | H |
| Me | H | $CF_3$ | H | H | H | O | - | - | 0 | F | Cl | $CO_2H$ |
| Me | H | $CF_3$ | H | H | H | O | - | - | 0 | F | Cl | $CO_2Me$ |
| Me | H | $CF_3$ | H | H | H | O | - | - | 0 | F | Cl | $CO_2Et$ |
| Et | H | $CF_3$ | H | H | H | O | - | - | 0 | F | Cl | $CO_2Pr^i$ |
| Et | H | $CF_3$ | H | H | H | O | - | - | 0 | F | Cl | $CO_2Pr^n$ |
| Et | H | $CF_3$ | H | H | H | O | - | - | 0 | F | Cl | $CO_2Bu^n$ |
| Et | H | $CF_3$ | H | H | H | O | - | - | 0 | F | Cl | $CO_2Bu^t$ |
| Et | H | $CF_3$ | H | H | H | O | - | - | 0 | F | Cl | $NO_2$ |
| Et | H | $CF_3$ | H | H | H | O | - | - | 0 | F | Cl | $NH_2$ |
| Et | H | $CF_3$ | H | H | H | O | - | - | 0 | F | Cl | $NHSO_2Me$ |
| Et | H | $CF_3$ | H | H | H | O | - | - | 0 | F | Cl | $NHSO_2Et$ |
| Et | H | $CF_3$ | H | H | H | O | - | - | 0 | F | Cl | $NHSO_2Pr^n$ |
| Et | H | $CF_3$ | H | H | H | O | - | - | 0 | F | Cl | $NHSO_2Pr^i$ |
| Et | H | $CF_3$ | H | H | H | O | - | - | 0 | F | Cl | OH |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | $CF_3$ | H | H | H | O | - | - | 0 | F | Cl | OMe |
| Et | H | $CF_3$ | H | H | H | O | - | - | 0 | F | Cl | OEt |
| Et | H | $CF_3$ | H | H | H | O | - | - | 0 | F | Cl | $OPr^i$ |
| Et | H | $CF_3$ | H | H | H | O | - | - | 0 | F | Cl | $OPr^n$ |
| Et | H | $CF_3$ | H | H | H | O | - | - | 0 | F | Cl | $OPen^c$ |
| Et | H | $CF_3$ | H | H | H | O | - | - | 0 | F | Cl | $OCH_2CH=CH_2$ |
| Et | H | $CF_3$ | H | H | H | O | - | - | 0 | F | Cl | $OCH_2C\equiv CH$ |
| Et | H | $CF_3$ | H | H | H | O | - | - | 0 | F | Cl | $OCH_2CO_2Me$ |
| Et | H | $CF_3$ | H | H | H | O | - | - | 0 | F | Cl | $OCH_2CO_2Et$ |
| Et | H | $CF_3$ | H | H | H | O | - | - | 0 | F | Cl | $OCH(Me)CO_2Me$ |
| Et | H | $CF_3$ | H | H | H | O | - | - | 0 | F | Cl | $OCH(Me)CO_2Et$ |
| Et | H | $CF_3$ | H | H | H | O | - | - | 0 | F | Cl | $CO_2H$ |
| Et | H | $CF_3$ | H | H | H | O | - | - | 0 | F | Cl | CN |
| Et | H | $CF_3$ | H | H | H | O | - | - | 0 | F | Cl | Me |
| Et | H | $CF_3$ | H | H | H | O | - | - | 0 | F | Cl | Et |
| Et | H | $CF_3$ | H | H | H | O | - | - | 0 | F | Cl | Cl |
| Et | H | $CF_3$ | H | H | H | O | - | - | 0 | F | Cl | Br |
| Et | H | $CF_3$ | H | H | H | O | - | - | 0 | F | Cl | I |
| Et | H | $CF_3$ | H | H | H | O | - | - | 0 | F | Cl | F |
| Et | H | $CF_3$ | H | H | H | O | - | - | 0 | F | Cl | SH |
| Et | H | $CF_3$ | H | H | H | O | - | - | 0 | F | Cl | $C(Me)=NOCH_2CO_2Me$ |
| Et | H | $CF_3$ | H | H | H | O | - | - | 0 | F | Cl | $C(Me)=NOCH_2CO_2Et$ |
| Et | H | $CF_3$ | H | H | H | O | - | - | 0 | F | Cl | $CH=NOCH_2CO_2Me$ |
| Et | H | $CF_3$ | H | H | H | O | - | - | 0 | F | Cl | $CH=NOCH_2CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | $CF_3$ | H | H | H | O | - | - | 0 | F | Cl | $C(OMe)=NOCH_2CO_2Me$ |
| Et | H | $CF_3$ | H | H | H | O | - | - | 0 | F | Cl | $C(OMe)=NOCH_2CO_2Et$ |
| Et | H | $CF_3$ | H | H | H | O | - | - | 0 | F | Cl | $C(Me)=NN=C(Me)CO_2Me$ |
| Et | H | $CF_3$ | H | H | H | O | - | - | 0 | F | Cl | $C(Me)=NN=C(Me)CO_2Et$ |
| Et | H | $CF_3$ | H | H | H | O | - | - | 0 | F | Cl | $CH_2OCH_2CO_2Me$ |
| Et | H | $CF_3$ | H | H | H | O | - | - | 0 | F | Cl | $CH_2OCH_2CO_2Et$ |
| Et | H | $CF_3$ | H | H | H | O | - | - | 0 | F | Cl | $CH_2OCH(Me)CO_2Me$ |
| Et | H | $CF_3$ | H | H | H | O | - | - | 0 | F | Cl | $CH_2OCH(Me)CO_2Et$ |
| Et | H | $CF_3$ | H | H | H | O | - | - | 0 | F | Cl | $CH_2SCH_2CO_2Me$ |
| Et | H | $CF_3$ | H | H | H | O | - | - | 0 | F | Cl | $CH_2SCH_2CO_2Et$ |
| Et | H | $CF_3$ | H | H | H | O | - | - | 0 | F | Cl | $CH_2SCH(Me)CO_2Me$ |
| Et | H | $CF_3$ | H | H | H | O | - | - | 0 | F | Cl | $CH_2SCH(Me)CO_2Et$ |
| Et | H | $CF_3$ | H | H | H | O | - | - | 0 | F | Cl | SMe |
| Et | H | $CF_3$ | H | H | H | O | - | - | 0 | F | Cl | SEt |
| Et | H | $CF_3$ | H | H | H | O | - | - | 0 | F | Cl | $SPr^i$ |
| Et | H | $CF_3$ | H | H | H | O | - | - | 0 | F | Cl | $SPr^n$ |
| Et | H | $CF_3$ | H | H | H | O | - | - | 0 | F | Cl | $SPen^c$ |
| Et | H | $CF_3$ | H | H | H | O | - | - | 0 | F | Cl | $SCH_2CO_2H$ |
| Et | H | $CF_3$ | H | H | H | O | - | - | 0 | F | Cl | $SCH_2CO_2Me$ |
| Et | H | $CF_3$ | H | H | H | O | - | - | 0 | F | Cl | $SCH_2CO_2Et$ |
| Et | H | $CF_3$ | H | H | H | O | - | - | 0 | F | Cl | $SCH(Me)CO_2Me$ |
| Et | H | $CF_3$ | H | H | H | O | - | - | 0 | F | Cl | $SCH(Me)CO_2Et$ |
| Et | H | $CF_3$ | H | H | H | O | - | - | 0 | F | Cl | $SCH_2CN$ |
| Et | H | $CF_3$ | H | H | H | O | - | - | 0 | F | Cl | $SCH_2CH=CH_2$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | $CF_3$ | H | H | H | O | - | - | 0 | F | Cl | $SCH_2C\equiv CH$ |
| Et | H | $CF_3$ | H | H | H | O | - | - | 0 | F | Cl | $OCH(Me)C\equiv CH$ |
| Et | H | $CF_3$ | H | H | H | O | - | - | 0 | F | Cl | $SCH(Me)C\equiv CH$ |
| Et | H | $CF_3$ | H | H | H | O | - | - | 0 | F | Cl | $N(SO_2Me)_2$ |
| Et | H | $CF_3$ | H | H | H | O | - | - | 0 | F | Cl | $N(SO_2Et)_2$ |
| Et | H | $CF_3$ | H | H | H | O | - | - | 0 | F | Cl | Q1(R29=$Bu^t$) |
| Et | H | $CF_3$ | H | H | H | O | - | - | 0 | F | Cl | Q2(R29=$Bu^t$) |
| Et | H | $CF_3$ | H | H | H | O | - | - | 0 | F | Cl | Q5(R29=$Bu^t$) |
| Et | H | $CF_3$ | H | H | H | O | - | - | 0 | F | Cl | Q7(R34=$Bu^t$) |
| Et | H | $CF_3$ | H | H | H | O | - | - | 0 | F | Cl | Q9(R38=R39=H) |
| Et | H | $CF_3$ | H | H | H | O | - | - | 0 | F | Cl | Q10(R38=R39=H) |
| Et | H | $CF_3$ | H | H | H | O | - | - | 0 | F | Cl | Q4(R29=$Bu^t$) |
| Et | H | $CF_3$ | H | H | H | O | - | - | 0 | F | Cl | Q8(R35=R36=R37=H) |
| Et | H | $CF_3$ | H | H | H | O | - | - | 0 | F | Cl | Q18(R50=H) |
| Et | H | $CF_3$ | H | H | H | O | - | - | 0 | F | Cl | Q18(R50=Me) |
| Et | H | $CF_3$ | H | H | H | O | - | - | 0 | F | Cl | $NHCO_2Me$ |
| Et | H | $CF_3$ | H | H | H | O | - | - | 0 | F | Cl | $NHCO_2Et$ |
| Et | H | $CF_3$ | H | H | H | O | - | - | 0 | F | Cl | $NHCO_2Pr^n$ |
| Et | H | $CF_3$ | H | H | H | O | - | - | 0 | F | Cl | $NHCO_2Pr^i$ |
| Et | H | $CF_3$ | H | H | H | O | - | - | 0 | F | Cl | $NHCO_2Ph$ |
| Et | H | $CF_3$ | H | H | H | O | - | - | 0 | F | Cl | $NHCO_2Bz$ |
| Et | H | $CF_3$ | H | H | H | O | - | - | 0 | F | Cl | NHCOMe |
| Et | H | $CF_3$ | H | H | H | O | - | - | 0 | F | Cl | $NHCO_2CH_2Ph$ |
| Et | H | $CF_3$ | H | H | H | O | - | - | 0 | F | Cl | $NHCO_2CH_2$(4-Me-Ph) |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | $CF_3$ | H | H | H | O | - | - | 0 | F | Cl | $NHCO_2CH_2$(3-Me-Ph) |
| Et | H | $CF_3$ | H | H | H | O | - | - | 0 | F | Cl | $NHCO_2CH_2$(2-Me-Ph) |
| Et | H | $CF_3$ | H | H | H | O | - | - | 0 | F | Cl | $NHCO_2CH_2$(4-Cl-Ph) |
| Et | H | $CF_3$ | H | H | H | O | - | - | 0 | F | Cl | $NHCO_2CH_2$(3-Cl-Ph) |
| Et | H | $CF_3$ | H | H | H | O | - | - | 0 | F | Cl | $NHCO_2CH_2$(2-Cl-Ph) |
| Et | H | $CF_3$ | H | H | H | O | - | - | 0 | F | Cl | Q1(R29=Me) |
| Et | H | $CF_3$ | H | H | H | O | - | - | 0 | F | Cl | Q2(R29=Me) |
| Et | H | $CF_3$ | H | H | H | O | - | - | 0 | F | Cl | H |
| Et | H | $CF_3$ | H | H | H | O | - | - | 0 | F | Cl | $CO_2H$ |
| Et | H | $CF_3$ | H | H | H | O | - | - | 0 | F | Cl | $CO_2Me$ |
| Et | H | $CF_3$ | H | H | H | O | - | - | 0 | F | Cl | $CO_2Et$ |
| $Pr^n$ | H | $CF_3$ | H | H | H | O | - | - | 0 | F | Cl | $CO_2Pr^i$ |
| $Pr^n$ | H | $CF_3$ | H | H | H | O | - | - | 0 | F | Cl | $CO_2Pr^n$ |
| $Pr^n$ | H | $CF_3$ | H | H | H | O | - | - | 0 | F | Cl | $CO_2Bu^n$ |
| $Pr^n$ | H | $CF_3$ | H | H | H | O | - | - | 0 | F | Cl | $CO_2Bu^t$ |
| $Pr^n$ | H | $CF_3$ | H | H | H | O | - | - | 0 | F | Cl | $NO_2$ |
| $Pr^n$ | H | $CF_3$ | H | H | H | O | - | - | 0 | F | Cl | $NH_2$ |
| $Pr^n$ | H | $CF_3$ | H | H | H | O | - | - | 0 | F | Cl | $NHSO_2Me$ |
| $Pr^n$ | H | $CF_3$ | H | H | H | O | - | - | 0 | F | Cl | $NHSO_2Et$ |
| $Pr^n$ | H | $CF_3$ | H | H | H | O | - | - | 0 | F | Cl | $NHSO_2Pr^n$ |
| $Pr^n$ | H | $CF_3$ | H | H | H | O | - | - | 0 | F | Cl | $NHSO_2Pr^i$ |
| $Pr^n$ | H | $CF_3$ | H | H | H | O | - | - | 0 | F | Cl | OH |
| $Pr^n$ | H | $CF_3$ | H | H | H | O | - | - | 0 | F | Cl | OMe |
| $Pr^n$ | H | $CF_3$ | H | H | H | O | - | - | 0 | F | Cl | OEt |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | $CF_3$ | H | H | H | O | - | - | 0 | F | Cl | $OPr^i$ |
| $Pr^n$ | H | $CF_3$ | H | H | H | O | - | - | 0 | F | Cl | $OPr^n$ |
| $Pr^n$ | H | $CF_3$ | H | H | H | O | - | - | 0 | F | Cl | $OPen^c$ |
| $Pr^n$ | H | $CF_3$ | H | H | H | O | - | - | 0 | F | Cl | $OCH_2CH=CH_2$ |
| $Pr^n$ | H | $CF_3$ | H | H | H | O | - | - | 0 | F | Cl | $OCH_2C\equiv CH$ |
| $Pr^n$ | H | $CF_3$ | H | H | H | O | - | - | 0 | F | Cl | $OCH_2CO_2Me$ |
| $Pr^n$ | H | $CF_3$ | H | H | H | O | - | - | 0 | F | Cl | $OCH_2CO_2Et$ |
| $Pr^n$ | H | $CF_3$ | H | H | H | O | - | - | 0 | F | Cl | $OCH(Me)CO_2Me$ |
| $Pr^n$ | H | $CF_3$ | H | H | H | O | - | - | 0 | F | Cl | $OCH(Me)CO_2Et$ |
| $Pr^n$ | H | $CF_3$ | H | H | H | O | - | - | 0 | F | Cl | $CO_2H$ |
| $Pr^n$ | H | $CF_3$ | H | H | H | O | - | - | 0 | F | Cl | CN |
| $Pr^n$ | H | $CF_3$ | H | H | H | O | - | - | 0 | F | Cl | Me |
| $Pr^n$ | H | $CF_3$ | H | H | H | O | - | - | 0 | F | Cl | Et |
| $Pr^n$ | H | $CF_3$ | H | H | H | O | - | - | 0 | F | Cl | CL |
| $Pr^n$ | H | $CF_3$ | H | H | H | O | - | - | 0 | F | Cl | Br |
| $Pr^n$ | H | $CF_3$ | H | H | H | O | - | - | 0 | F | Cl | I |
| $Pr^n$ | H | $CF_3$ | H | H | H | O | - | - | 0 | F | Cl | F |
| $Pr^n$ | H | $CF_3$ | H | H | H | O | - | - | 0 | F | Cl | SH |
| $Pr^n$ | H | $CF_3$ | H | H | H | O | - | - | 0 | F | Cl | $C(Me)=NOCH_2CO_2Me$ |
| $Pr^n$ | H | $CF_3$ | H | H | H | O | - | - | 0 | F | Cl | $C(Me)=NOCH_2CO_2Et$ |
| $Pr^n$ | H | $CF_3$ | H | H | H | O | - | - | 0 | F | Cl | $CH=NOCH_2CO_2Me$ |
| $Pr^n$ | H | $CF_3$ | H | H | H | O | - | - | 0 | F | Cl | $CH=NOCH_2CO_2Et$ |
| $Pr^n$ | H | $CF_3$ | H | H | H | O | - | - | 0 | F | Cl | $C(OMe)=NOCH_2CO_2Me$ |
| $Pr^n$ | H | $CF_3$ | H | H | H | O | - | - | 0 | F | Cl | $C(OMe)=NOCH_2CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | $CF_3$ | H | H | H | O | - | - | 0 | F | Cl | C(Me)=NN=C(Me)$CO_2$Me |
| $Pr^n$ | H | $CF_3$ | H | H | H | O | - | - | 0 | F | Cl | C(Me)=NN=C(Me)$CO_2$Et |
| $Pr^n$ | H | $CF_3$ | H | H | H | O | - | - | 0 | F | Cl | $CH_2OCH_2CO_2Me$ |
| $Pr^n$ | H | $CF_3$ | H | H | H | O | - | - | 0 | F | Cl | $CH_2OCH_2CO_2Et$ |
| $Pr^n$ | H | $CF_3$ | H | H | H | O | - | - | 0 | F | Cl | $CH_2OCH(Me)CO_2Me$ |
| $Pr^n$ | H | $CF_3$ | H | H | H | O | - | - | 0 | F | Cl | $CH_2OCH(Me)CO_2Et$ |
| $Pr^n$ | H | $CF_3$ | H | H | H | O | - | - | 0 | F | Cl | $CH_2SCH_2CO_2Me$ |
| $Pr^n$ | H | $CF_3$ | H | H | H | O | - | - | 0 | F | Cl | $CH_2SCH_2CO_2Et$ |
| $Pr^n$ | H | $CF_3$ | H | H | H | O | - | - | 0 | F | Cl | $CH_2SCH(Me)CO_2Me$ |
| $Pr^n$ | H | $CF_3$ | H | H | H | O | - | - | 0 | F | Cl | $CH_2SCH(Me)CO_2Et$ |
| $Pr^n$ | H | $CF_3$ | H | H | H | O | - | - | 0 | F | Cl | SMe |
| $Pr^n$ | H | $CF_3$ | H | H | H | O | - | - | 0 | F | Cl | SEt |
| $Pr^n$ | H | $CF_3$ | H | H | H | O | - | - | 0 | F | Cl | $SPr^i$ |
| $Pr^n$ | H | $CF_3$ | H | H | H | O | - | - | 0 | F | Cl | $SPr^n$ |
| $Pr^n$ | H | $CF_3$ | H | H | H | O | - | - | 0 | F | Cl | $SPen^c$ |
| $Pr^n$ | H | $CF_3$ | H | H | H | O | - | - | 0 | F | Cl | $SCH_2CO_2H$ |
| $Pr^n$ | H | $CF_3$ | H | H | H | O | - | - | 0 | F | Cl | $SCH_2CO_2Me$ |
| $Pr^n$ | H | $CF_3$ | H | H | H | O | - | - | 0 | F | Cl | $SCH_2CO_2Et$ |
| $Pr^n$ | H | $CF_3$ | H | H | H | O | - | - | 0 | F | Cl | $SCH(Me)CO_2Me$ |
| $Pr^n$ | H | $CF_3$ | H | H | H | O | - | - | 0 | F | Cl | $SCH(Me)CO_2Et$ |
| $Pr^n$ | H | $CF_3$ | H | H | H | O | - | - | 0 | F | Cl | $SCH_2CN$ |
| $Pr^n$ | H | $CF_3$ | H | H | H | O | - | - | 0 | F | Cl | $SCH_2CH=CH_2$ |
| $Pr^n$ | H | $CF_3$ | H | H | H | O | - | - | 0 | F | Cl | $SCH_2C\equiv CH$ |
| $Pr^n$ | H | $CF_3$ | H | H | H | O | - | - | 0 | F | Cl | $OCH(Me)C\equiv CH$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | $CF_3$ | H | H | H | O | - | - | 0 | F | Cl | SCH(Me)C ≡CH |
| $Pr^n$ | H | $CF_3$ | H | H | H | O | - | - | 0 | F | Cl | $N(SO_2Me)_2$ |
| $Pr^n$ | H | $CF_3$ | H | H | H | O | - | - | 0 | F | Cl | $N(SO_2Et)_2$ |
| $Pr^n$ | H | $CF_3$ | H | H | H | O | - | - | 0 | F | Cl | Q1(R29=$Bu^t$) |
| $Pr^n$ | H | $CF_3$ | H | H | H | O | - | - | 0 | F | Cl | Q2(R29=$Bu^t$) |
| $Pr^n$ | H | $CF_3$ | H | H | H | O | - | - | 0 | F | Cl | Q5(R29=$Bu^t$) |
| $Pr^n$ | H | $CF_3$ | H | H | H | O | - | - | 0 | F | Cl | Q7(R34=$Bu^t$) |
| $Pr^n$ | H | $CF_3$ | H | H | H | O | - | - | 0 | F | Cl | Q9(R38=R39=H) |
| $Pr^n$ | H | $CF_3$ | H | H | H | O | - | - | 0 | F | Cl | Q10(R38=R39=H) |
| $Pr^n$ | H | $CF_3$ | H | H | H | O | - | - | 0 | F | Cl | Q4(R29=$Bu^t$) |
| $Pr^n$ | H | $CF_3$ | H | H | H | O | - | - | 0 | F | Cl | Q8(R35=R36=R37=H) |
| $Pr^n$ | H | $CF_3$ | H | H | H | O | - | - | 0 | F | Cl | Q18(R50=H) |
| $Pr^n$ | H | $CF_3$ | H | H | H | O | - | - | 0 | F | Cl | Q18(R50=Me) |
| $Pr^n$ | H | $CF_3$ | H | H | H | O | - | - | 0 | F | Cl | $NHCO_2Me$ |
| $Pr^n$ | H | $CF_3$ | H | H | H | O | - | - | 0 | F | Cl | $NHCO_2Et$ |
| $Pr^n$ | H | $CF_3$ | H | H | H | O | - | - | 0 | F | Cl | $NHCO_2Pr^n$ |
| $Pr^n$ | H | $CF_3$ | H | H | H | O | - | - | 0 | F | Cl | $NHCO_2Pr^i$ |
| $Pr^n$ | H | $CF_3$ | H | H | H | O | - | - | 0 | F | Cl | $NHCO_2Ph$ |
| $Pr^n$ | H | $CF_3$ | H | H | H | O | - | - | 0 | F | Cl | $NHCO_2Bz$ |
| $Pr^n$ | H | $CF_3$ | H | H | H | O | - | - | 0 | F | Cl | NHCOMe |
| $Pr^n$ | H | $CF_3$ | H | H | H | O | - | - | 0 | F | Cl | $NHCO_2CH_2Ph$ |
| $Pr^n$ | H | $CF_3$ | H | H | H | O | - | - | 0 | F | Cl | $NHCO_2CH_2$(4-Me-Ph) |
| $Pr^n$ | H | $CF_3$ | H | H | H | O | - | - | 0 | F | Cl | $NHCO_2CH_2$(3-Me-Ph) |
| $Pr^n$ | H | $CF_3$ | H | H | H | O | - | - | 0 | F | Cl | $NHCO_2CH_2$(2-Me-Ph) |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | $CF_3$ | H | H | H | O | - | - | 0 | F | Cl | $NHCO_2CH_2$(4-CL-Ph) |
| $Pr^n$ | H | $CF_3$ | H | H | H | O | - | - | 0 | F | Cl | $NHCO_2CH_2$(3-CL-Ph) |
| $Pr^n$ | H | $CF_3$ | H | H | H | O | - | - | 0 | F | Cl | $NHCO_2CH_2$(2-CL-Ph) |
| $Pr^n$ | H | $CF_3$ | H | H | H | O | - | - | 0 | F | Cl | Q1(R29=Me) |
| $Pr^n$ | H | $CF_3$ | H | H | H | O | - | - | 0 | F | Cl | Q2(R29=Me) |
| $Pr^n$ | H | $CF_3$ | H | H | H | O | - | - | 0 | F | Cl | H |
| $Pr^n$ | H | $CF_3$ | H | H | H | O | - | - | 0 | F | Cl | $CO_2H$ |
| $Pr^n$ | H | $CF_3$ | H | H | H | O | - | - | 0 | F | Cl | $CO_2Me$ |
| $Pr^n$ | H | $CF_3$ | H | H | H | O | - | - | 0 | F | Cl | $CO_2Et$ |
| $Pr^i$ | H | $CF_3$ | H | H | H | O | - | - | 0 | F | Cl | $CO_2Pr^i$ |
| $Pr^i$ | H | $CF_3$ | H | H | H | O | - | - | 0 | F | Cl | $CO_2Pr^n$ |
| $Pr^i$ | H | $CF_3$ | H | H | H | O | - | - | 0 | F | Cl | $CO_2Bu^n$ |
| $Pr^i$ | H | $CF_3$ | H | H | H | O | - | - | 0 | F | Cl | $CO_2Bu^t$ |
| $Pr^i$ | H | $CF_3$ | H | H | H | O | - | - | 0 | F | Cl | $NO_2$ |
| $Pr^i$ | H | $CF_3$ | H | H | H | O | - | - | 0 | F | Cl | $NH_2$ |
| $Pr^i$ | H | $CF_3$ | H | H | H | O | - | - | 0 | F | Cl | $NHSO_2Me$ |
| $Pr^i$ | H | $CF_3$ | H | H | H | O | - | - | 0 | F | Cl | $NHSO_2Et$ |
| $Pr^i$ | H | $CF_3$ | H | H | H | O | - | - | 0 | F | Cl | $NHSO_2Pr^n$ |
| $Pr^i$ | H | $CF_3$ | H | H | H | O | - | - | 0 | F | Cl | $NHSO_2Pr^i$ |
| $Pr^i$ | H | $CF_3$ | H | H | H | O | - | - | 0 | F | Cl | OH |
| $Pr^i$ | H | $CF_3$ | H | H | H | O | - | - | 0 | F | Cl | OMe |
| $Pr^i$ | H | $CF_3$ | H | H | H | O | - | - | 0 | F | Cl | OEt |
| $Pr^i$ | H | $CF_3$ | H | H | H | O | - | - | 0 | F | Cl | $OPr^i$ |
| $Pr^i$ | H | $CF_3$ | H | H | H | O | - | - | 0 | F | Cl | $OPr^n$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | $CF_3$ | H | H | H | O | - | - | 0 | F | Cl | $OPen^c$ |
| $Pr^i$ | H | $CF_3$ | H | H | H | O | - | - | 0 | F | Cl | $OCH_2CH=CH_2$ |
| $Pr^i$ | H | $CF_3$ | H | H | H | O | - | - | 0 | F | Cl | $OCH_2C\equiv CH$ |
| $Pr^i$ | H | $CF_3$ | H | H | H | O | - | - | 0 | F | Cl | $OCH_2CO_2Me$ |
| $Pr^i$ | H | $CF_3$ | H | H | H | O | - | - | 0 | F | Cl | $OCH_2CO_2Et$ |
| $Pr^i$ | H | $CF_3$ | H | H | H | O | - | - | 0 | F | Cl | $OCH(Me)CO_2Me$ |
| $Pr^i$ | H | $CF_3$ | H | H | H | O | - | - | 0 | F | Cl | $OCH(Me)CO_2Et$ |
| $Pr^i$ | H | $CF_3$ | H | H | H | O | - | - | 0 | F | Cl | $CO_2H$ |
| $Pr^i$ | H | $CF_3$ | H | H | H | O | - | - | 0 | F | Cl | CN |
| $Pr^i$ | H | $CF_3$ | H | H | H | O | - | - | 0 | F | Cl | Me |
| $Pr^i$ | H | $CF_3$ | H | H | H | O | - | - | 0 | F | Cl | Et |
| $Pr^i$ | H | $CF_3$ | H | H | H | O | - | - | 0 | F | Cl | CL |
| $Pr^i$ | H | $CF_3$ | H | H | H | O | - | - | 0 | F | Cl | Br |
| $Pr^i$ | H | $CF_3$ | H | H | H | O | - | - | 0 | F | Cl | I |
| $Pr^i$ | H | $CF_3$ | H | H | H | O | - | - | 0 | F | Cl | F |
| $Pr^i$ | H | $CF_3$ | H | H | H | O | - | - | 0 | F | Cl | SH |
| $Pr^i$ | H | $CF_3$ | H | H | H | O | - | - | 0 | F | Cl | $C(Me)=NOCH_2CO_2Me$ |
| $Pr^i$ | H | $CF_3$ | H | H | H | O | - | - | 0 | F | Cl | $C(Me)=NOCH_2CO_2Et$ |
| $Pr^i$ | H | $CF_3$ | H | H | H | O | - | - | 0 | F | Cl | $CH=NOCH_2CO_2Me$ |
| $Pr^i$ | H | $CF_3$ | H | H | H | O | - | - | 0 | F | Cl | $CH=NOCH_2CO_2Et$ |
| $Pr^i$ | H | $CF_3$ | H | H | H | O | - | - | 0 | F | Cl | $C(OMe)=NOCH_2CO_2Me$ |
| $Pr^i$ | H | $CF_3$ | H | H | H | O | - | - | 0 | F | Cl | $C(OMe)=NOCH_2CO_2Et$ |
| $Pr^i$ | H | $CF_3$ | H | H | H | O | - | - | 0 | F | Cl | $C(Me)=NN=C(Me)CO_2Me$ |
| $Pr^i$ | H | $CF_3$ | H | H | H | O | - | - | 0 | F | Cl | $C(Me)=NN=C(Me)CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | $CF_3$ | H | H | H | O | - | - | 0 | F | Cl | $CH_2OCH_2CO_2Me$ |
| $Pr^i$ | H | $CF_3$ | H | H | H | O | - | - | 0 | F | Cl | $CH_2OCH_2CO_2Et$ |
| $Pr^i$ | H | $CF_3$ | H | H | H | O | - | - | 0 | F | Cl | $CH_2OCH(Me)CO_2Me$ |
| $Pr^i$ | H | $CF_3$ | H | H | H | O | - | - | 0 | F | Cl | $CH_2OCH(Me)CO_2Et$ |
| $Pr^i$ | H | $CF_3$ | H | H | H | O | - | - | 0 | F | Cl | $CH_2SCH_2CO_2Me$ |
| $Pr^i$ | H | $CF_3$ | H | H | H | O | - | - | 0 | F | Cl | $CH_2SCH_2CO_2Et$ |
| $Pr^i$ | H | $CF_3$ | H | H | H | O | - | - | 0 | F | Cl | $CH_2SCH(Me)CO_2Me$ |
| $Pr^i$ | H | $CF_3$ | H | H | H | O | - | - | 0 | F | Cl | $CH_2SCH(Me)CO_2Et$ |
| $Pr^i$ | H | $CF_3$ | H | H | H | O | - | - | 0 | F | Cl | SMe |
| $Pr^i$ | H | $CF_3$ | H | H | H | O | - | - | 0 | F | Cl | SEt |
| $Pr^i$ | H | $CF_3$ | H | H | H | O | - | - | 0 | F | Cl | $SPr^i$ |
| $Pr^i$ | H | $CF_3$ | H | H | H | O | - | - | 0 | F | Cl | $SPr^n$ |
| $Pr^i$ | H | $CF_3$ | H | H | H | O | - | - | 0 | F | Cl | $SPen^c$ |
| $Pr^i$ | H | $CF_3$ | H | H | H | O | - | - | 0 | F | Cl | $SCH_2CO_2H$ |
| $Pr^i$ | H | $CF_3$ | H | H | H | O | - | - | 0 | F | Cl | $SCH_2CO_2Me$ |
| $Pr^i$ | H | $CF_3$ | H | H | H | O | - | - | 0 | F | Cl | $SCH_2CO_2Et$ |
| $Pr^i$ | H | $CF_3$ | H | H | H | O | - | - | 0 | F | Cl | $SCH(Me)CO_2Me$ |
| $Pr^i$ | H | $CF_3$ | H | H | H | O | - | - | 0 | F | Cl | $SCH(Me)CO_2Et$ |
| $Pr^i$ | H | $CF_3$ | H | H | H | O | - | - | 0 | F | Cl | $SCH_2CN$ |
| $Pr^i$ | H | $CF_3$ | H | H | H | O | - | - | 0 | F | Cl | $SCH_2CH=CH_2$ |
| $Pr^i$ | H | $CF_3$ | H | H | H | O | - | - | 0 | F | Cl | $SCH_2C\equiv CH$ |
| $Pr^i$ | H | $CF_3$ | H | H | H | O | - | - | 0 | F | Cl | $OCH(Me)C\equiv CH$ |
| $Pr^i$ | H | $CF_3$ | H | H | H | O | - | - | 0 | F | Cl | $SCH(Me)C\equiv CH$ |
| $Pr^i$ | H | $CF_3$ | H | H | H | O | - | - | 0 | F | Cl | $N(SO_2Me)_2$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | $CF_3$ | H | H | H | O | - | - | 0 | F | Cl | $N(SO_2Et)_2$ |
| $Pr^i$ | H | $CF_3$ | H | H | H | O | - | - | 0 | F | Cl | Q1(R29=$Bu^t$) |
| $Pr^i$ | H | $CF_3$ | H | H | H | O | - | - | 0 | F | Cl | Q2(R29=$Bu^t$) |
| $Pr^i$ | H | $CF_3$ | H | H | H | O | - | - | 0 | F | Cl | Q5(R29=$Bu^t$) |
| $Pr^i$ | H | $CF_3$ | H | H | H | O | - | - | 0 | F | Cl | Q7(R34=$Bu^t$) |
| $Pr^i$ | H | $CF_3$ | H | H | H | O | - | - | 0 | F | Cl | Q9(R38=R39=H) |
| $Pr^i$ | H | $CF_3$ | H | H | H | O | - | - | 0 | F | Cl | Q10(R38=R39=H) |
| $Pr^i$ | H | $CF_3$ | H | H | H | O | - | - | 0 | F | Cl | Q4(R29=$Bu^t$) |
| $Pr^i$ | H | $CF_3$ | H | H | H | O | - | - | 0 | F | Cl | Q8(R35=R36=R37=H) |
| $Pr^i$ | H | $CF_3$ | H | H | H | O | - | - | 0 | F | Cl | Q18(R50=H) |
| $Pr^i$ | H | $CF_3$ | H | H | H | O | - | - | 0 | F | Cl | Q18(R50=Me) |
| $Pr^i$ | H | $CF_3$ | H | H | H | O | - | - | 0 | F | Cl | $NHCO_2Me$ |
| $Pr^i$ | H | $CF_3$ | H | H | H | O | - | - | 0 | F | Cl | $NHCO_2Et$ |
| $Pr^i$ | H | $CF_3$ | H | H | H | O | - | - | 0 | F | Cl | $NHCO_2Pr^n$ |
| $Pr^i$ | H | $CF_3$ | H | H | H | O | - | - | 0 | F | Cl | $NHCO_2Pr^i$ |
| $Pr^i$ | H | $CF_3$ | H | H | H | O | - | - | 0 | F | Cl | $NHCO_2Ph$ |
| $Pr^i$ | H | $CF_3$ | H | H | H | O | - | - | 0 | F | Cl | $NHCO_2Bz$ |
| $Pr^i$ | H | $CF_3$ | H | H | H | O | - | - | 0 | F | Cl | NHCOMe |
| $Pr^i$ | H | $CF_3$ | H | H | H | O | - | - | 0 | F | Cl | $NHCO_2CH_2Ph$ |
| $Pr^i$ | H | $CF_3$ | H | H | H | O | - | - | 0 | F | Cl | $NHCO_2CH_2$(4-Me-Ph) |
| $Pr^i$ | H | $CF_3$ | H | H | H | O | - | - | 0 | F | Cl | $NHCO_2CH_2$(3-Me-Ph) |
| $Pr^i$ | H | $CF_3$ | H | H | H | O | - | - | 0 | F | Cl | $NHCO_2CH_2$(2-Me-Ph) |
| $Pr^i$ | H | $CF_3$ | H | H | H | O | - | - | 0 | F | Cl | $NHCO_2CH_2$(4-CL-Ph) |
| $Pr^i$ | H | $CF_3$ | H | H | H | O | - | - | 0 | F | Cl | $NHCO_2CH_2$(3-CL-Ph) |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | $CF_3$ | H | H | H | O | - | - | 0 | F | Cl | $NHCO_2CH_2$(2-CL-Ph) |
| $Pr^i$ | H | $CF_3$ | H | H | H | O | - | - | 0 | F | Cl | Q1(R29=Me) |
| $Pr^i$ | H | $CF_3$ | H | H | H | O | - | - | 0 | F | Cl | Q2(R29=Me) |
| $Pr^i$ | H | $CF_3$ | H | H | H | O | - | - | 0 | F | Cl | H |
| $Pr^i$ | H | $CF_3$ | H | H | H | O | - | - | 0 | F | Cl | $CO_2H$ |
| $Pr^i$ | H | $CF_3$ | H | H | H | O | - | - | 0 | F | Cl | $CO_2Me$ |
| $Pr^i$ | H | $CF_3$ | H | H | H | O | - | - | 0 | F | Cl | $CO_2Et$ |
| $CF_3$ | H | $CF_3$ | H | H | H | O | - | - | 0 | F | Cl | $CO_2Pr^i$ |
| $CF_3$ | H | $CF_3$ | H | H | H | O | - | - | 0 | F | Cl | $CO_2Pr^n$ |
| $CF_3$ | H | $CF_3$ | H | H | H | O | - | - | 0 | F | Cl | $CO_2Bu^n$ |
| $CF_3$ | H | $CF_3$ | H | H | H | O | - | - | 0 | F | Cl | $CO_2Bu^t$ |
| $CF_3$ | H | $CF_3$ | H | H | H | O | - | - | 0 | F | Cl | $NO_2$ |
| $CF_3$ | H | $CF_3$ | H | H | H | O | - | - | 0 | F | Cl | $NH_2$ |
| $CF_3$ | H | $CF_3$ | H | H | H | O | - | - | 0 | F | Cl | $NHSO_2Me$ |
| $CF_3$ | H | $CF_3$ | H | H | H | O | - | - | 0 | F | Cl | $NHSO_2Et$ |
| $CF_3$ | H | $CF_3$ | H | H | H | O | - | - | 0 | F | Cl | $NHSO_2Pr^n$ |
| $CF_3$ | H | $CF_3$ | H | H | H | O | - | - | 0 | F | Cl | $NHSO_2Pr^i$ |
| $CF_3$ | H | $CF_3$ | H | H | H | O | - | - | 0 | F | Cl | OH |
| $CF_3$ | H | $CF_3$ | H | H | H | O | - | - | 0 | F | Cl | OMe |
| $CF_3$ | H | $CF_3$ | H | H | H | O | - | - | 0 | F | Cl | OEt |
| $CF_3$ | H | $CF_3$ | H | H | H | O | - | - | 0 | F | Cl | $OPr^i$ |
| $CF_3$ | H | $CF_3$ | H | H | H | O | - | - | 0 | F | Cl | $OPr^n$ |
| $CF_3$ | H | $CF_3$ | H | H | H | O | - | - | 0 | F | Cl | $OPen^c$ |
| $CF_3$ | H | $CF_3$ | H | H | H | O | - | - | 0 | F | Cl | $OCH_2CH=CH_2$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | $CF_3$ | H | H | H | O | - | - | 0 | F | Cl | $OCH_2C\equiv CH$ |
| $CF_3$ | H | $CF_3$ | H | H | H | O | - | - | 0 | F | Cl | $OCH_2CO_2Me$ |
| $CF_3$ | H | $CF_3$ | H | H | H | O | - | - | 0 | F | Cl | $OCH_2CO_2Et$ |
| $CF_3$ | H | $CF_3$ | H | H | H | O | - | - | 0 | F | Cl | $OCH(Me)CO_2Me$ |
| $CF_3$ | H | $CF_3$ | H | H | H | O | - | - | 0 | F | Cl | $OCH(Me)CO_2Et$ |
| $CF_3$ | H | $CF_3$ | H | H | H | O | - | - | 0 | F | Cl | $CO_2H$ |
| $CF_3$ | H | $CF_3$ | H | H | H | O | - | - | 0 | F | Cl | CN |
| $CF_3$ | H | $CF_3$ | H | H | H | O | - | - | 0 | F | Cl | Me |
| $CF_3$ | H | $CF_3$ | H | H | H | O | - | - | 0 | F | Cl | Et |
| $CF_3$ | H | $CF_3$ | H | H | H | O | - | - | 0 | F | Cl | CL |
| $CF_3$ | H | $CF_3$ | H | H | H | O | - | - | 0 | F | Cl | Br |
| $CF_3$ | H | $CF_3$ | H | H | H | O | - | - | 0 | F | Cl | I |
| $CF_3$ | H | $CF_3$ | H | H | H | O | - | - | 0 | F | Cl | F |
| $CF_3$ | H | $CF_3$ | H | H | H | O | - | - | 0 | F | Cl | SH |
| $CF_3$ | H | $CF_3$ | H | H | H | O | - | - | 0 | F | Cl | $C(Me)=NOCH_2CO_2Me$ |
| $CF_3$ | H | $CF_3$ | H | H | H | O | - | - | 0 | F | Cl | $C(Me)=NOCH_2CO_2Et$ |
| $CF_3$ | H | $CF_3$ | H | H | H | O | - | - | 0 | F | Cl | $CH=NOCH_2CO_2Me$ |
| $CF_3$ | H | $CF_3$ | H | H | H | O | - | - | 0 | F | Cl | $CH=NOCH_2CO_2Et$ |
| $CF_3$ | H | $CF_3$ | H | H | H | O | - | - | 0 | F | Cl | $C(OMe)=NOCH_2CO_2Me$ |
| $CF_3$ | H | $CF_3$ | H | H | H | O | - | - | 0 | F | Cl | $C(OMe)=NOCH_2CO_2Et$ |
| $CF_3$ | H | $CF_3$ | H | H | H | O | - | - | 0 | F | Cl | $C(Me)=NN=C(Me)CO_2Me$ |
| $CF_3$ | H | $CF_3$ | H | H | H | O | - | - | 0 | F | Cl | $C(Me)=NN=C(Me)CO_2Et$ |
| $CF_3$ | H | $CF_3$ | H | H | H | O | - | - | 0 | F | Cl | $CH_2OCH_2CO_2Me$ |
| $CF_3$ | H | $CF_3$ | H | H | H | O | - | - | 0 | F | Cl | $CH_2OCH_2CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | $CF_3$ | H | H | H | O | - | - | 0 | F | Cl | $CH_2OCH(Me)CO_2Me$ |
| $CF_3$ | H | $CF_3$ | H | H | H | O | - | - | 0 | F | Cl | $CH_2OCH(Me)CO_2Et$ |
| $CF_3$ | H | $CF_3$ | H | H | H | O | - | - | 0 | F | Cl | $CH_2SCH_2CO_2Me$ |
| $CF_3$ | H | $CF_3$ | H | H | H | O | - | - | 0 | F | Cl | $CH_2SCH_2CO_2Et$ |
| $CF_3$ | H | $CF_3$ | H | H | H | O | - | - | 0 | F | Cl | $CH_2SCH(Me)CO_2Me$ |
| $CF_3$ | H | $CF_3$ | H | H | H | O | - | - | 0 | F | Cl | $CH_2SCH(Me)CO_2Et$ |
| $CF_3$ | H | $CF_3$ | H | H | H | O | - | - | 0 | F | Cl | SMe |
| $CF_3$ | H | $CF_3$ | H | H | H | O | - | - | 0 | F | Cl | SEt |
| $CF_3$ | H | $CF_3$ | H | H | H | O | - | - | 0 | F | Cl | $SPr^i$ |
| $CF_3$ | H | $CF_3$ | H | H | H | O | - | - | 0 | F | Cl | $SPr^n$ |
| $CF_3$ | H | $CF_3$ | H | H | H | O | - | - | 0 | F | Cl | $SPen^c$ |
| $CF_3$ | H | $CF_3$ | H | H | H | O | - | - | 0 | F | Cl | $SCH_2CO_2H$ |
| $CF_3$ | H | $CF_3$ | H | H | H | O | - | - | 0 | F | Cl | $SCH_2CO_2Me$ |
| $CF_3$ | H | $CF_3$ | H | H | H | O | - | - | 0 | F | Cl | $SCH_2CO_2Et$ |
| $CF_3$ | H | $CF_3$ | H | H | H | O | - | - | 0 | F | Cl | $SCH(Me)CO_2Me$ |
| $CF_3$ | H | $CF_3$ | H | H | H | O | - | - | 0 | F | Cl | $SCH(Me)CO_2Et$ |
| $CF_3$ | H | $CF_3$ | H | H | H | O | - | - | 0 | F | Cl | $SCH_2CN$ |
| $CF_3$ | H | $CF_3$ | H | H | H | O | - | - | 0 | F | Cl | $SCH_2CH=CH_2$ |
| $CF_3$ | H | $CF_3$ | H | H | H | O | - | - | 0 | F | Cl | $SCH_2C\equiv CH$ |
| $CF_3$ | H | $CF_3$ | H | H | H | O | - | - | 0 | F | Cl | $OCH(Me)C\equiv CH$ |
| $CF_3$ | H | $CF_3$ | H | H | H | O | - | - | 0 | F | Cl | $SCH(Me)C\equiv CH$ |
| $CF_3$ | H | $CF_3$ | H | H | H | O | - | - | 0 | F | Cl | $N(SO_2Me)_2$ |
| $CF_3$ | H | $CF_3$ | H | H | H | O | - | - | 0 | F | Cl | $N(SO_2Et)_2$ |
| $CF_3$ | H | $CF_3$ | H | H | H | O | - | - | 0 | F | Cl | Q1($R29=Bu^t$) |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | $CF_3$ | H | H | H | O | - | - | 0 | F | Cl | Q2(R29=$Bu^t$) |
| $CF_3$ | H | $CF_3$ | H | H | H | O | - | - | 0 | F | Cl | Q5(R29=$Bu^t$) |
| $CF_3$ | H | $CF_3$ | H | H | H | O | - | - | 0 | F | Cl | Q7(R34=$Bu^t$) |
| $CF_3$ | H | $CF_3$ | H | H | H | O | - | - | 0 | F | Cl | Q9(R38=R39=H) |
| $CF_3$ | H | $CF_3$ | H | H | H | O | - | - | 0 | F | Cl | Q10(R38=R39=H) |
| $CF_3$ | H | $CF_3$ | H | H | H | O | - | - | 0 | F | Cl | Q4(R29=$Bu^t$) |
| $CF_3$ | H | $CF_3$ | H | H | H | O | - | - | 0 | F | Cl | Q8(R35=R36=R37=H) |
| $CF_3$ | H | $CF_3$ | H | H | H | O | - | - | 0 | F | Cl | Q18(R50=H) |
| $CF_3$ | H | $CF_3$ | H | H | H | O | - | - | 0 | F | Cl | Q18(R50=Me) |
| $CF_3$ | H | $CF_3$ | H | H | H | O | - | - | 0 | F | Cl | $NHCO_2Me$ |
| $CF_3$ | H | $CF_3$ | H | H | H | O | - | - | 0 | F | Cl | $NHCO_2Et$ |
| $CF_3$ | H | $CF_3$ | H | H | H | O | - | - | 0 | F | Cl | $NHCO_2Pr^n$ |
| $CF_3$ | H | $CF_3$ | H | H | H | O | - | - | 0 | F | Cl | $NHCO_2Pr^i$ |
| $CF_3$ | H | $CF_3$ | H | H | H | O | - | - | 0 | F | Cl | $NHCO_2Ph$ |
| $CF_3$ | H | $CF_3$ | H | H | H | O | - | - | 0 | F | Cl | $NHCO_2Bz$ |
| $CF_3$ | H | $CF_3$ | H | H | H | O | - | - | 0 | F | Cl | NHCOMe |
| $CF_3$ | H | $CF_3$ | H | H | H | O | - | - | 0 | F | Cl | $NHCO_2CH_2Ph$ |
| $CF_3$ | H | $CF_3$ | H | H | H | O | - | - | 0 | F | Cl | $NHCO_2CH_2$(4-Me-Ph) |
| $CF_3$ | H | $CF_3$ | H | H | H | O | - | - | 0 | F | Cl | $NHCO_2CH_2$(3-Me-Ph) |
| $CF_3$ | H | $CF_3$ | H | H | H | O | - | - | 0 | F | Cl | $NHCO_2CH_2$(2-Me-Ph) |
| $CF_3$ | H | $CF_3$ | H | H | H | O | - | - | 0 | F | Cl | $NHCO_2CH_2$(4-Cl-Ph) |
| $CF_3$ | H | $CF_3$ | H | H | H | O | - | - | 0 | F | Cl | $NHCO_2CH_2$(3-Cl-Ph) |
| $CF_3$ | H | $CF_3$ | H | H | H | O | - | - | 0 | F | Cl | $NHCO_2CH_2$(2-Cl-Ph) |
| $CF_3$ | H | $CF_3$ | H | H | H | O | - | - | 0 | F | Cl | Q1(R29=Me) |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | $CF_3$ | H | H | H | O | - | - | 0 | F | Cl | Q2(R29=Me) |
| $CF_3$ | H | $CF_3$ | H | H | H | O | - | - | 0 | F | Cl | H |
| $CF_3$ | H | $CF_3$ | H | H | H | O | - | - | 0 | F | Cl | $CO_2H$ |
| $CF_3$ | H | $CF_3$ | H | H | H | O | - | - | 0 | F | Cl | $CO_2Me$ |
| $CF_3$ | H | $CF_3$ | H | H | H | O | - | - | 0 | F | Cl | $CO_2Et$ |
| H | $-CH_2CH_2CH_2-$ | | H | H | H | O | - | - | 0 | F | Cl | $CO_2Pr^i$ |
| H | $-CH_2CH_2CH_2-$ | | H | H | H | O | - | - | 0 | F | Cl | $CO_2Pr^n$ |
| H | $-CH_2CH_2CH_2-$ | | H | H | H | O | - | - | 0 | F | Cl | $CO_2Bu^n$ |
| H | $-CH_2CH_2CH_2-$ | | H | H | H | O | - | - | 0 | F | Cl | $CO_2Bu^t$ |
| H | $-CH_2CH_2CH_2-$ | | H | H | H | O | - | - | 0 | F | Cl | $NO_2$ |
| H | $-CH_2CH_2CH_2-$ | | H | H | H | O | - | - | 0 | F | Cl | $NH_2$ |
| H | $-CH_2CH_2CH_2-$ | | H | H | H | O | - | - | 0 | F | Cl | $NHSO_2Me$ |
| H | $-CH_2CH_2CH_2-$ | | H | H | H | O | - | - | 0 | F | Cl | $NHSO_2Et$ |
| H | $-CH_2CH_2CH_2-$ | | H | H | H | O | - | - | 0 | F | Cl | $NHSO_2Pr^n$ |
| H | $-CH_2CH_2CH_2-$ | | H | H | H | O | - | - | 0 | F | Cl | $NHSO_2Pr^i$ |
| H | $-CH_2CH_2CH_2-$ | | H | H | H | O | - | - | 0 | F | Cl | OH |
| H | $-CH_2CH_2CH_2-$ | | H | H | H | O | - | - | 0 | F | Cl | OMe |
| H | $-CH_2CH_2CH_2-$ | | H | H | H | O | - | - | 0 | F | Cl | OEt |
| H | $-CH_2CH_2CH_2-$ | | H | H | H | O | - | - | 0 | F | Cl | $OPr^i$ |
| H | $-CH_2CH_2CH_2-$ | | H | H | H | O | - | - | 0 | F | Cl | $OPr^n$ |
| H | $-CH_2CH_2CH_2-$ | | H | H | H | O | - | - | 0 | F | Cl | $OPen^c$ |
| H | $-CH_2CH_2CH_2-$ | | H | H | H | O | - | - | 0 | F | Cl | $OCH_2CH=CH_2$ |
| H | $-CH_2CH_2CH_2-$ | | H | H | H | O | - | - | 0 | F | Cl | $OCH_2C\equiv CH$ |
| H | $-CH_2CH_2CH_2-$ | | H | H | H | O | - | - | 0 | F | Cl | $OCH_2CO_2Me$ |

Table 1-3-1 (Cont' d)

| R1 | R2 R3 | R4 | R5 | R6 | X | $R_a$ | $R_b$ | m | R7 | R8 | R9 |
|---|---|---|---|---|---|---|---|---|---|---|---|
| H | $-CH_2CH_2CH_2-$ | H | H | H | O | - | - | 0 | F | Cl | $OCH_2CO_2Et$ |
| H | $-CH_2CH_2CH_2-$ | H | H | H | O | - | - | 0 | F | Cl | $OCH(Me)CO_2Me$ |
| H | $-CH_2CH_2CH_2-$ | H | H | H | O | - | - | 0 | F | Cl | $OCH(Me)CO_2Et$ |
| H | $-CH_2CH_2CH_2-$ | H | H | H | O | - | - | 0 | F | Cl | $CO_2H$ |
| H | $-CH_2CH_2CH_2-$ | H | H | H | O | - | - | 0 | F | Cl | CN |
| H | $-CH_2CH_2CH_2-$ | H | H | H | O | - | - | 0 | F | Cl | Me |
| H | $-CH_2CH_2CH_2-$ | H | H | H | O | - | - | 0 | F | Cl | Et |
| H | $-CH_2CH_2CH_2-$ | H | H | H | O | - | - | 0 | F | Cl | Cl |
| H | $-CH_2CH_2CH_2-$ | H | H | H | O | - | - | 0 | F | Cl | Br |
| H | $-CH_2CH_2CH_2-$ | H | H | H | O | - | - | 0 | F | Cl | I |
| H | $-CH_2CH_2CH_2-$ | H | H | H | O | - | - | 0 | F | Cl | F |
| H | $-CH_2CH_2CH_2-$ | H | H | H | O | - | - | 0 | F | Cl | SH |
| H | $-CH_2CH_2CH_2-$ | H | H | H | O | - | - | 0 | F | Cl | $C(Me)=NOCH_2CO_2Me$ |
| H | $-CH_2CH_2CH_2-$ | H | H | H | O | - | - | 0 | F | Cl | $C(Me)=NOCH_2CO_2Et$ |
| H | $-CH_2CH_2CH_2-$ | H | H | H | O | - | - | 0 | F | Cl | $CH=NOCH_2CO_2Me$ |
| H | $-CH_2CH_2CH_2-$ | H | H | H | O | - | - | 0 | F | Cl | $CH=NOCH_2CO_2Et$ |
| H | $-CH_2CH_2CH_2-$ | H | H | H | O | - | - | 0 | F | Cl | $C(OMe)=NOCH_2CO_2Me$ |
| H | $-CH_2CH_2CH_2-$ | H | H | H | O | - | - | 0 | F | Cl | $C(OMe)=NOCH_2CO_2Et$ |
| H | $-CH_2CH_2CH_2-$ | H | H | H | O | - | - | 0 | F | Cl | $C(Me)=NN=C(Me)CO_2Me$ |
| H | $-CH_2CH_2CH_2-$ | H | H | H | O | - | - | 0 | F | Cl | $C(Me)=NN=C(Me)CO_2Et$ |
| H | $-CH_2CH_2CH_2-$ | H | H | H | O | - | - | 0 | F | Cl | $CH_2OCH_2CO_2Me$ |
| H | $-CH_2CH_2CH_2-$ | H | H | H | O | - | - | 0 | F | Cl | $CH_2OCH_2CO_2Et$ |
| H | $-CH_2CH_2CH_2-$ | H | H | H | O | - | - | 0 | F | Cl | $CH_2OCH(Me)CO_2Me$ |
| H | $-CH_2CH_2CH_2-$ | H | H | H | O | - | - | 0 | F | Cl | $CH_2OCH(Me)CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| H | $-CH_2CH_2CH_2-$ | | H | H | H | O | - | - | 0 | F | Cl | $CH_2SCH_2CO_2Me$ |
| H | $-CH_2CH_2CH_2-$ | | H | H | H | O | - | - | 0 | F | Cl | $CH_2SCH_2CO_2Et$ |
| H | $-CH_2CH_2CH_2-$ | | H | H | H | O | - | - | 0 | F | Cl | $CH_2SCH(Me)CO_2Me$ |
| H | $-CH_2CH_2CH_2-$ | | H | H | H | O | - | - | 0 | F | Cl | $CH_2SCH(Me)CO_2Et$ |
| H | $-CH_2CH_2CH_2-$ | | H | H | H | O | - | - | 0 | F | Cl | SMe |
| H | $-CH_2CH_2CH_2-$ | | H | H | H | O | - | - | 0 | F | Cl | SEt |
| H | $-CH_2CH_2CH_2-$ | | H | H | H | O | - | - | 0 | F | Cl | $SPr^i$ |
| H | $-CH_2CH_2CH_2-$ | | H | H | H | O | - | - | 0 | F | Cl | $SPr^n$ |
| H | $-CH_2CH_2CH_2-$ | | H | H | H | O | - | - | 0 | F | Cl | $SPen^c$ |
| H | $-CH_2CH_2CH_2-$ | | H | H | H | O | - | - | 0 | F | Cl | $SCH_2CO_2H$ |
| H | $-CH_2CH_2CH_2-$ | | H | H | H | O | - | - | 0 | F | Cl | $SCH_2CO_2Me$ |
| H | $-CH_2CH_2CH_2-$ | | H | H | H | O | - | - | 0 | F | Cl | $SCH_2CO_2Et$ |
| H | $-CH_2CH_2CH_2-$ | | H | H | H | O | - | - | 0 | F | Cl | $SCH(Me)CO_2Me$ |
| H | $-CH_2CH_2CH_2-$ | | H | H | H | O | - | - | 0 | F | Cl | $SCH(Me)CO_2Et$ |
| H | $-CH_2CH_2CH_2-$ | | H | H | H | O | - | - | 0 | F | Cl | $SCH_2CN$ |
| H | $-CH_2CH_2CH_2-$ | | H | H | H | O | - | - | 0 | F | Cl | $SCH_2CH=CH_2$ |
| H | $-CH_2CH_2CH_2-$ | | H | H | H | O | - | - | 0 | F | Cl | $SCH_2C\equiv CH$ |
| H | $-CH_2CH_2CH_2-$ | | H | H | H | O | - | - | 0 | F | Cl | $OCH(Me)C\equiv CH$ |
| H | $-CH_2CH_2CH_2-$ | | H | H | H | O | - | - | 0 | F | Cl | $SCH(Me)C\equiv CH$ |
| H | $-CH_2CH_2CH_2-$ | | H | H | H | O | - | - | 0 | F | Cl | $N(SO_2Me)_2$ |
| H | $-CH_2CH_2CH_2-$ | | H | H | H | O | - | - | 0 | F | Cl | $N(SO_2Et)_2$ |
| H | $-CH_2CH_2CH_2-$ | | H | H | H | O | - | - | 0 | F | Cl | Q1(R29=$Bu^t$) |
| H | $-CH_2CH_2CH_2-$ | | H | H | H | O | - | - | 0 | F | Cl | Q2(R29=$Bu^t$) |
| H | $-CH_2CH_2CH_2-$ | | H | H | H | O | - | - | 0 | F | Cl | Q5(R29=$Bu^t$) |

Table 1-3-1 (Cont'd)

| R1 | R2 R3 | R4 | R5 | R6 | X | $R_a$ | $R_b$ | m | R7 | R8 | R9 |
|---|---|---|---|---|---|---|---|---|---|---|---|
| H | $-CH_2CH_2CH_2-$ | H | H | H | O | - | - | 0 | F | Cl | Q7(R34=$Bu^t$) |
| H | $-CH_2CH_2CH_2-$ | H | H | H | O | - | - | 0 | F | Cl | Q9(R38=R39=H) |
| H | $-CH_2CH_2CH_2-$ | H | H | H | O | - | - | 0 | F | Cl | Q10(R38=R39=H) |
| H | $-CH_2CH_2CH_2-$ | H | H | H | O | - | - | 0 | F | Cl | Q4(R29=$Bu^t$) |
| H | $-CH_2CH_2CH_2-$ | H | H | H | O | - | - | 0 | F | Cl | Q8(R35=R36=R37=H) |
| H | $-CH_2CH_2CH_2-$ | H | H | H | O | - | - | 0 | F | Cl | Q18(R50=H) |
| H | $-CH_2CH_2CH_2-$ | H | H | H | O | - | - | 0 | F | Cl | Q18(R50=Me) |
| H | $-CH_2CH_2CH_2-$ | H | H | H | O | - | - | 0 | F | Cl | $NHCO_2Me$ |
| H | $-CH_2CH_2CH_2-$ | H | H | H | O | - | - | 0 | F | Cl | $NHCO_2Et$ |
| H | $-CH_2CH_2CH_2-$ | H | H | H | O | - | - | 0 | F | Cl | $NHCO_2Pr^n$ |
| H | $-CH_2CH_2CH_2-$ | H | H | H | O | - | - | 0 | F | Cl | $NHCO_2Pr^i$ |
| H | $-CH_2CH_2CH_2-$ | H | H | H | O | - | - | 0 | F | Cl | $NHCO_2Ph$ |
| H | $-CH_2CH_2CH_2-$ | H | H | H | O | - | - | 0 | F | Cl | $NHCO_2Bz$ |
| H | $-CH_2CH_2CH_2-$ | H | H | H | O | - | - | 0 | F | Cl | NHCOMe |
| H | $-CH_2CH_2CH_2-$ | H | H | H | O | - | - | 0 | F | Cl | $NHCO_2CH_2Ph$ |
| H | $-CH_2CH_2CH_2-$ | H | H | H | O | - | - | 0 | F | Cl | $NHCO_2CH_2$(4-Me-Ph) |
| H | $-CH_2CH_2CH_2-$ | H | H | H | O | - | - | 0 | F | Cl | $NHCO_2CH_2$(3-Me-Ph) |
| H | $-CH_2CH_2CH_2-$ | H | H | H | O | - | - | 0 | F | Cl | $NHCO_2CH_2$(2-Me-Ph) |
| H | $-CH_2CH_2CH_2-$ | H | H | H | O | - | - | 0 | F | Cl | $NHCO_2CH_2$(4-Cl-Ph) |
| H | $-CH_2CH_2CH_2-$ | H | H | H | O | - | - | 0 | F | Cl | $NHCO_2CH_2$(3-Cl-Ph) |
| H | $-CH_2CH_2CH_2-$ | H | H | H | O | - | - | 0 | F | Cl | $NHCO_2CH_2$(2-Cl-Ph) |
| H | $-CH_2CH_2CH_2-$ | H | H | H | O | - | - | 0 | F | Cl | Q1(R29=Me) |
| H | $-CH_2CH_2CH_2-$ | H | H | H | O | - | - | 0 | F | Cl | Q2(R29=Me) |
| H | $-CH_2CH_2CH_2-$ | H | H | H | O | - | - | 0 | F | Cl | H |

Table 1-3-1 (Cont'd)

| R1 | R2 R3 | R4 | R5 | R6 | X | $R_a$ | $R_b$ | m | R7 | R8 | R9 |
|---|---|---|---|---|---|---|---|---|---|---|---|
| H | $-CH_2CH_2CH_2-$ | H | H | H | O | - | - | 0 | F | Cl | $CO_2H$ |
| H | $-CH_2CH_2CH_2-$ | H | H | H | O | - | - | 0 | F | Cl | $CO_2Me$ |
| H | $-CH_2CH_2CH_2-$ | H | H | H | O | - | - | 0 | F | Cl | $CO_2Et$ |
| H | $-(CH_2)_4-$ | H | H | H | O | - | - | 0 | F | Cl | $CO_2Pr^i$ |
| H | $-(CH_2)_4-$ | H | H | H | O | - | - | 0 | F | Cl | $CO_2Pr^n$ |
| H | $-(CH_2)_4-$ | H | H | H | O | - | - | 0 | F | Cl | $CO_2Bu^n$ |
| H | $-(CH_2)_4-$ | H | H | H | O | - | - | 0 | F | Cl | $CO_2Bu^t$ |
| H | $-(CH_2)_4-$ | H | H | H | O | - | - | 0 | F | Cl | $NO_2$ |
| H | $-(CH_2)_4-$ | H | H | H | O | - | - | 0 | F | Cl | $NH_2$ |
| H | $-(CH_2)_4-$ | H | H | H | O | - | - | 0 | F | Cl | $NHSO_2Me$ |
| H | $-(CH_2)_4-$ | H | H | H | O | - | - | 0 | F | Cl | $NHSO_2Et$ |
| H | $-(CH_2)_4-$ | H | H | H | O | - | - | 0 | F | Cl | $NHSO_2Pr^n$ |
| H | $-(CH_2)_4-$ | H | H | H | O | - | - | 0 | F | Cl | $NHSO_2Pr^i$ |
| H | $-(CH_2)_4-$ | H | H | H | O | - | - | 0 | F | Cl | OH |
| H | $-(CH_2)_4-$ | H | H | H | O | - | - | 0 | F | Cl | OMe |
| H | $-(CH_2)_4-$ | H | H | H | O | - | - | 0 | F | Cl | OEt |
| H | $-(CH_2)_4-$ | H | H | H | O | - | - | 0 | F | Cl | $OPr^i$ |
| H | $-(CH_2)_4-$ | H | H | H | O | - | - | 0 | F | Cl | $OPr^n$ |
| H | $-(CH_2)_4-$ | H | H | H | O | - | - | 0 | F | Cl | $OPen^c$ |
| H | $-(CH_2)_4-$ | H | H | H | O | - | - | 0 | F | Cl | $OCH_2CH=CH_2$ |
| H | $-(CH_2)_4-$ | H | H | H | O | - | - | 0 | F | Cl | $OCH_2C\equiv CH$ |
| H | $-(CH_2)_4-$ | H | H | H | O | - | - | 0 | F | Cl | $OCH_2CO_2Me$ |
| H | $-(CH_2)_4-$ | H | H | H | O | - | - | 0 | F | Cl | $OCH_2CO_2Et$ |
| H | $-(CH_2)_4-$ | H | H | H | O | - | - | 0 | F | Cl | $OCH(Me)CO_2Me$ |

Table 1-3-1 (Cont' d)

| R1 | R2 R3 | R4 | R5 | R6 | X | $R_a$ | $R_b$ | m | R7 | R8 | R9 |
|---|---|---|---|---|---|---|---|---|---|---|---|
| H | $-(CH_2)_4-$ | H | H | H | 0 | - | - | 0 | F | Cl | $OCH(Me)CO_2Et$ |
| H | $-(CH_2)_4-$ | H | H | H | 0 | - | - | 0 | F | Cl | $CO_2H$ |
| H | $-(CH_2)_4-$ | H | H | H | 0 | - | - | 0 | F | Cl | CN |
| H | $-(CH_2)_4-$ | H | H | H | 0 | - | - | 0 | F | Cl | Me |
| H | $-(CH_2)_4-$ | H | H | H | 0 | - | - | 0 | F | Cl | Et |
| H | $-(CH_2)_4-$ | H | H | H | 0 | - | - | 0 | F | Cl | Cl |
| H | $-(CH_2)_4-$ | H | H | H | 0 | - | - | 0 | F | Cl | Br |
| H | $-(CH_2)_4-$ | H | H | H | 0 | - | - | 0 | F | Cl | I |
| H | $-(CH_2)_4-$ | H | H | H | 0 | - | - | 0 | F | Cl | F |
| H | $-(CH_2)_4-$ | H | H | H | 0 | - | - | 0 | F | Cl | SH |
| H | $-(CH_2)_4-$ | H | H | H | 0 | - | - | 0 | F | Cl | $C(Me)=NOCH_2CO_2Me$ |
| H | $-(CH_2)_4-$ | H | H | H | 0 | - | - | 0 | F | Cl | $C(Me)=NOCH_2CO_2Et$ |
| H | $-(CH_2)_4-$ | H | H | H | 0 | - | - | 0 | F | Cl | $CH=NOCH_2CO_2Me$ |
| H | $-(CH_2)_4-$ | H | H | H | 0 | - | - | 0 | F | Cl | $CH=NOCH_2CO_2Et$ |
| H | $-(CH_2)_4-$ | H | H | H | 0 | - | - | 0 | F | Cl | $C(OMe)=NOCH_2CO_2Me$ |
| H | $-(CH_2)_4-$ | H | H | H | 0 | - | - | 0 | F | Cl | $C(OMe)=NOCH_2CO_2Et$ |
| H | $-(CH_2)_4-$ | H | H | H | 0 | - | - | 0 | F | Cl | $C(Me)=NN=C(Me)CO_2Me$ |
| H | $-(CH_2)_4-$ | H | H | H | 0 | - | - | 0 | F | Cl | $C(Me)=NN=C(Me)CO_2Et$ |
| H | $-(CH_2)_4-$ | H | H | H | 0 | - | - | 0 | F | Cl | $CH_2OCH_2CO_2Me$ |
| H | $-(CH_2)_4-$ | H | H | H | 0 | - | - | 0 | F | Cl | $CH_2OCH_2CO_2Et$ |
| H | $-(CH_2)_4-$ | H | H | H | 0 | - | - | 0 | F | Cl | $CH_2OCH(Me)CO_2Me$ |
| H | $-(CH_2)_4-$ | H | H | H | 0 | - | - | 0 | F | Cl | $CH_2OCH(Me)CO_2Et$ |
| H | $-(CH_2)_4-$ | H | H | H | 0 | - | - | 0 | F | Cl | $CH_2SCH_2CO_2Me$ |
| H | $-(CH_2)_4-$ | H | H | H | 0 | - | - | 0 | F | Cl | $CH_2SCH_2CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| H | $-(CH_2)_4-$ | | H | H | H | O | - | - | 0 | F | Cl | $CH_2SCH(Me)CO_2Me$ |
| H | $-(CH_2)_4-$ | | H | H | H | O | - | - | 0 | F | Cl | $CH_2SCH(Me)CO_2Et$ |
| H | $-(CH_2)_4-$ | | H | H | H | O | - | - | 0 | F | Cl | SMe |
| H | $-(CH_2)_4-$ | | H | H | H | O | - | - | 0 | F | Cl | SEt |
| H | $-(CH_2)_4-$ | | H | H | H | O | - | - | 0 | F | Cl | $SPr^i$ |
| H | $-(CH_2)_4-$ | | H | H | H | O | - | - | 0 | F | Cl | $SPr^n$ |
| H | $-(CH_2)_4-$ | | H | H | H | O | - | - | 0 | F | Cl | $SPen^c$ |
| H | $-(CH_2)_4-$ | | H | H | H | O | - | - | 0 | F | Cl | $SCH_2CO_2H$ |
| H | $-(CH_2)_4-$ | | H | H | H | O | - | - | 0 | F | Cl | $SCH_2CO_2Me$ |
| H | $-(CH_2)_4-$ | | H | H | H | O | - | - | 0 | F | Cl | $SCH_2CO_2Et$ |
| H | $-(CH_2)_4-$ | | H | H | H | O | - | - | 0 | F | Cl | $SCH(Me)CO_2Me$ |
| H | $-(CH_2)_4-$ | | H | H | H | O | - | - | 0 | F | Cl | $SCH(Me)CO_2Et$ |
| H | $-(CH_2)_4-$ | | H | H | H | O | - | - | 0 | F | Cl | $SCH_2CN$ |
| H | $-(CH_2)_4-$ | | H | H | H | O | - | - | 0 | F | Cl | $SCH_2CH=CH_2$ |
| H | $-(CH_2)_4-$ | | H | H | H | O | - | - | 0 | F | Cl | $SCH_2C\equiv CH$ |
| H | $-(CH_2)_4-$ | | H | H | H | O | - | - | 0 | F | Cl | $OCH(Me)C\equiv CH$ |
| H | $-(CH_2)_4-$ | | H | H | H | O | - | - | 0 | F | Cl | $SCH(Me)C\equiv CH$ |
| H | $-(CH_2)_4-$ | | H | H | H | O | - | - | 0 | F | Cl | $N(SO_2Me)_2$ |
| H | $-(CH_2)_4-$ | | H | H | H | O | - | - | 0 | F | Cl | $N(SO_2Et)_2$ |
| H | $-(CH_2)_4-$ | | H | H | H | O | - | - | 0 | F | Cl | Q1(R29=$Bu^t$) |
| H | $-(CH_2)_4-$ | | H | H | H | O | - | - | 0 | F | Cl | Q2(R29=$Bu^t$) |
| H | $-(CH_2)_4-$ | | H | H | H | O | - | - | 0 | F | Cl | Q5(R29=$Bu^t$) |
| H | $-(CH_2)_4-$ | | H | H | H | O | - | - | 0 | F | Cl | Q7(R34=$Bu^t$) |
| H | $-(CH_2)_4-$ | | H | H | H | O | - | - | 0 | F | Cl | Q9(R38=R39=H) |

Table 1-3-1 (Cont'd)

| R1 | R2 R3 | R4 | R5 | R6 | X | $R_a$ | $R_b$ | m | R7 | R8 | R9 |
|---|---|---|---|---|---|---|---|---|---|---|---|
| H | $-(CH_2)_4-$ | H | H | H | O | - | - | 0 | F | Cl | Q10(R38=R39=H) |
| H | $-(CH_2)_4-$ | H | H | H | O | - | - | 0 | F | Cl | Q4(R29=$Bu^t$) |
| H | $-(CH_2)_4-$ | H | H | H | O | - | - | 0 | F | Cl | Q8(R35=R36=R37=H) |
| H | $-(CH_2)_4-$ | H | H | H | O | - | - | 0 | F | Cl | Q18(R50=H) |
| H | $-(CH_2)_4-$ | H | H | H | O | - | - | 0 | F | Cl | Q18(R50=Me) |
| H | $-(CH_2)_4-$ | H | H | H | O | - | - | 0 | F | Cl | $NHCO_2Me$ |
| H | $-(CH_2)_4-$ | H | H | H | O | - | - | 0 | F | Cl | $NHCO_2Et$ |
| H | $-(CH_2)_4-$ | H | H | H | O | - | - | 0 | F | Cl | $NHCO_2Pr^n$ |
| H | $-(CH_2)_4-$ | H | H | H | O | - | - | 0 | F | Cl | $NHCO_2Pr^i$ |
| H | $-(CH_2)_4-$ | H | H | H | O | - | - | 0 | F | Cl | $NHCO_2Ph$ |
| H | $-(CH_2)_4-$ | H | H | H | O | - | - | 0 | F | Cl | $NHCO_2Bz$ |
| H | $-(CH_2)_4-$ | H | H | H | O | - | - | 0 | F | Cl | NHCOMe |
| H | $-(CH_2)_4-$ | H | H | H | O | - | - | 0 | F | Cl | $NHCO_2CH_2Ph$ |
| H | $-(CH_2)_4-$ | H | H | H | O | - | - | 0 | F | Cl | $NHCO_2CH_2$(4-Me-Ph) |
| H | $-(CH_2)_4-$ | H | H | H | O | - | - | 0 | F | Cl | $NHCO_2CH_2$(3-Me-Ph) |
| H | $-(CH_2)_4-$ | H | H | H | O | - | - | 0 | F | Cl | $NHCO_2CH_2$(2-Me-Ph) |
| H | $-(CH_2)_4-$ | H | H | H | O | - | - | 0 | F | Cl | $NHCO_2CH_2$(4-Cl-Ph) |
| H | $-(CH_2)_4-$ | H | H | H | O | - | - | 0 | F | Cl | $NHCO_2CH_2$(3-Cl-Ph) |
| H | $-(CH_2)_4-$ | H | H | H | O | - | - | 0 | F | Cl | $NHCO_2CH_2$(2-Cl-Ph) |
| H | $-(CH_2)_4-$ | H | H | H | O | - | - | 0 | F | Cl | Q1(R29=Me) |
| H | $-(CH_2)_4-$ | H | H | H | O | - | - | 0 | F | Cl | Q2(R29=Me) |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| H | $-(CH_2)_4-$ | | H | H | H | O | - | - | 0 | F | Cl | H |
| H | $-(CH_2)_4-$ | | H | H | H | O | - | - | 0 | F | Cl | $CO_2H$ |
| H | $-(CH_2)_4-$ | | H | H | H | O | - | - | 0 | F | Cl | $CO_2Me$ |
| H | $-(CH_2)_4-$ | | H | H | H | O | - | - | 0 | F | Cl | $CO_2Et$ |
| Me | H | Me | H | Me | H | O | - | - | 0 | F | Cl | $CO_2Pr^i$ |
| Me | H | Me | H | Me | H | O | - | - | 0 | F | Cl | $CO_2Pr^n$ |
| Me | H | Me | H | Me | H | O | - | - | 0 | F | Cl | $CO_2Bu^n$ |
| Me | H | Me | H | Me | H | O | - | - | 0 | F | Cl | $CO_2Bu^t$ |
| Me | H | Me | H | Me | H | O | - | - | 0 | F | Cl | $NO_2$ |
| Me | H | Me | H | Me | H | O | - | - | 0 | F | Cl | $NH_2$ |
| Me | H | Me | H | Me | H | O | - | - | 0 | F | Cl | $NHSO_2Me$ |
| Me | H | Me | H | Me | H | O | - | - | 0 | F | Cl | $NHSO_2Et$ |
| Me | H | Me | H | Me | H | O | - | - | 0 | F | Cl | $NHSO_2Pr^n$ |
| Me | H | Me | H | Me | H | O | - | - | 0 | F | Cl | $NHSO_2Pr^i$ |
| Me | H | Me | H | Me | H | O | - | - | 0 | F | Cl | OH |
| Me | H | Me | H | Me | H | O | - | - | 0 | F | Cl | OMe |
| Me | H | Me | H | Me | H | O | - | - | 0 | F | Cl | OEt |
| Me | H | Me | H | Me | H | O | - | - | 0 | F | Cl | $OPr^i$ |
| Me | H | Me | H | Me | H | O | - | - | 0 | F | Cl | $OPr^n$ |
| Me | H | Me | H | Me | H | O | - | - | 0 | F | Cl | $OPen^c$ |
| Me | H | Me | H | Me | H | O | - | - | 0 | F | Cl | $OCH_2CH=CH_2$ |
| Me | H | Me | H | Me | H | O | - | - | 0 | F | Cl | $OCH_2C\equiv CH$ |
| Me | H | Me | H | Me | H | O | - | - | 0 | F | Cl | $OCH_2CO_2Me$ |
| Me | H | Me | H | Me | H | O | - | - | 0 | F | Cl | $OCH_2CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | Me | H | O | - | - | 0 | F | Cl | $OCH(Me)CO_2Me$ |
| Me | H | Me | H | Me | H | O | - | - | 0 | F | Cl | $OCH(Me)CO_2Et$ |
| Me | H | Me | H | Me | H | O | - | - | 0 | F | Cl | $CO_2H$ |
| Me | H | Me | H | Me | H | O | - | - | 0 | F | Cl | CN |
| Me | H | Me | H | Me | H | O | - | - | 0 | F | Cl | Me |
| Me | H | Me | H | Me | H | O | - | - | 0 | F | Cl | Et |
| Me | H | Me | H | Me | H | O | - | - | 0 | F | Cl | CL |
| Me | H | Me | H | Me | H | O | - | - | 0 | F | Cl | Br |
| Me | H | Me | H | Me | H | O | - | - | 0 | F | Cl | I |
| Me | H | Me | H | Me | H | O | - | - | 0 | F | Cl | F |
| Me | H | Me | H | Me | H | O | - | - | 0 | F | Cl | SH |
| Me | H | Me | H | Me | H | O | - | - | 0 | F | Cl | $C(Me)=NOCH_2CO_2Me$ |
| Me | H | Me | H | Me | H | O | - | - | 0 | F | Cl | $C(Me)=NOCH_2CO_2Et$ |
| Me | H | Me | H | Me | H | O | - | - | 0 | F | Cl | $CH=NOCH_2CO_2Me$ |
| Me | H | Me | H | Me | H | O | - | - | 0 | F | Cl | $CH=NOCH_2CO_2Et$ |
| Me | H | Me | H | Me | H | O | - | - | 0 | F | Cl | $C(OMe)=NOCH_2CO_2Me$ |
| Me | H | Me | H | Me | H | O | - | - | 0 | F | Cl | $C(OMe)=NOCH_2CO_2Et$ |
| Me | H | Me | H | Me | H | O | - | - | 0 | F | Cl | $C(Me)=NN=C(Me)CO_2Me$ |
| Me | H | Me | H | Me | H | O | - | - | 0 | F | Cl | $C(Me)=NN=C(Me)CO_2Et$ |
| Me | H | Me | H | Me | H | O | - | - | 0 | F | Cl | $CH_2OCH_2CO_2Me$ |
| Me | H | Me | H | Me | H | O | - | - | 0 | F | Cl | $CH_2OCH_2CO_2Et$ |
| Me | H | Me | H | Me | H | O | - | - | 0 | F | Cl | $CH_2OCH(Me)CO_2Me$ |
| Me | H | Me | H | Me | H | O | - | - | 0 | F | Cl | $CH_2OCH(Me)CO_2Et$ |
| Me | H | Me | H | Me | H | O | - | - | 0 | F | Cl | $CH_2SCH_2CO_2Me$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | Me | H | O | - | - | 0 | F | Cl | $CH_2SCH_2CO_2Et$ |
| Me | H | Me | H | Me | H | O | - | - | 0 | F | Cl | $CH_2SCH(Me)CO_2Me$ |
| Me | H | Me | H | Me | H | O | - | - | 0 | F | Cl | $CH_2SCH(Me)CO_2Et$ |
| Me | H | Me | H | Me | H | O | - | - | 0 | F | Cl | SMe |
| Me | H | Me | H | Me | H | O | - | - | 0 | F | Cl | SEt |
| Me | H | Me | H | Me | H | O | - | - | 0 | F | Cl | $SPr^i$ |
| Me | H | Me | H | Me | H | O | - | - | 0 | F | Cl | $SPr^n$ |
| Me | H | Me | H | Me | H | O | - | - | 0 | F | Cl | $SPen^c$ |
| Me | H | Me | H | Me | H | O | - | - | 0 | F | Cl | $SCH_2CO_2H$ |
| Me | H | Me | H | Me | H | O | - | - | 0 | F | Cl | $SCH_2CO_2Me$ |
| Me | H | Me | H | Me | H | O | - | - | 0 | F | Cl | $SCH_2CO_2Et$ |
| Me | H | Me | H | Me | H | O | - | - | 0 | F | Cl | $SCH(Me)CO_2Me$ |
| Me | H | Me | H | Me | H | O | - | - | 0 | F | Cl | $SCH(Me)CO_2Et$ |
| Me | H | Me | H | Me | H | O | - | - | 0 | F | Cl | $SCH_2CN$ |
| Me | H | Me | H | Me | H | O | - | - | 0 | F | Cl | $SCH_2CH=CH_2$ |
| Me | H | Me | H | Me | H | O | - | - | 0 | F | Cl | $SCH_2C\equiv CH$ |
| Me | H | Me | H | Me | H | O | - | - | 0 | F | Cl | $OCH(Me)C\equiv CH$ |
| Me | H | Me | H | Me | H | O | - | - | 0 | F | Cl | $SCH(Me)C\equiv CH$ |
| Me | H | Me | H | Me | H | O | - | - | 0 | F | Cl | $N(SO_2Me)_2$ |
| Me | H | Me | H | Me | H | O | - | - | 0 | F | Cl | $N(SO_2Et)_2$ |
| Me | H | Me | H | Me | H | O | - | - | 0 | F | Cl | $Q1(R29=Bu^t)$ |
| Me | H | Me | H | Me | H | O | - | - | 0 | F | Cl | $Q2(R29=Bu^t)$ |
| Me | H | Me | H | Me | H | O | - | - | 0 | F | Cl | $Q5(R29=Bu^t)$ |
| Me | H | Me | H | Me | H | O | - | - | 0 | F | Cl | $Q7(R34=Bu^t)$ |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | Me | H | O | - | - | 0 | F | Cl | Q9(R38=R39=H) |
| Me | H | Me | H | Me | H | O | - | - | 0 | F | Cl | Q10(R38=R39=H) |
| Me | H | Me | H | Me | H | O | - | - | 0 | F | Cl | Q4(R29=$Bu^t$ ) |
| Me | H | Me | H | Me | H | O | - | - | 0 | F | Cl | Q8(R35=R36=R37=H) |
| Me | H | Me | H | Me | H | O | - | - | 0 | F | Cl | Q18(R50=H) |
| Me | H | Me | H | Me | H | O | - | - | 0 | F | Cl | Q18(R50=Me) |
| Me | H | Me | H | Me | H | O | - | - | 0 | F | Cl | $NHCO_2Me$ |
| Me | H | Me | H | Me | H | O | - | - | 0 | F | Cl | $NHCO_2Et$ |
| Me | H | Me | H | Me | H | O | - | - | 0 | F | Cl | $NHCO_2Pr^n$ |
| Me | H | Me | H | Me | H | O | - | - | 0 | F | Cl | $NHCO_2Pr^i$ |
| Me | H | Me | H | Me | H | O | - | - | 0 | F | Cl | $NHCO_2Ph$ |
| Me | H | Me | H | Me | H | O | - | - | 0 | F | Cl | $NHCO_2Bz$ |
| Me | H | Me | H | Me | H | O | - | - | 0 | F | Cl | NHCOMe |
| Me | H | Me | H | Me | H | O | - | - | 0 | F | Cl | $NHCO_2CH_2Ph$ |
| Me | H | Me | H | Me | H | O | - | - | 0 | F | Cl | $NHCO_2CH_2$(4-Me-Ph) |
| Me | H | Me | H | Me | H | O | - | - | 0 | F | Cl | $NHCO_2CH_2$(3-Me-Ph) |
| Me | H | Me | H | Me | H | O | - | - | 0 | F | Cl | $NHCO_2CH_2$(2-Me-Ph) |
| Me | H | Me | H | Me | H | O | - | - | 0 | F | Cl | $NHCO_2CH_2$(4-CL-Ph) |
| Me | H | Me | H | Me | H | O | - | - | 0 | F | Cl | $NHCO_2CH_2$(3-CL-Ph) |
| Me | H | Me | H | Me | H | O | - | - | 0 | F | Cl | $NHCO_2CH_2$(2-CL-Ph) |
| Me | H | Me | H | Me | H | O | - | - | 0 | F | Cl | Q1(R29=Me) |
| Me | H | Me | H | Me | H | O | - | - | 0 | F | Cl | Q2(R29=Me) |
| Me | H | Me | H | Me | H | O | - | - | 0 | F | Cl | H |
| Me | H | Me | H | Me | H | O | - | - | 0 | F | Cl | $CO_2H$ |

Table 1－3－1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | Me | H | O | - | - | 0 | F | Cl | $CO_2Me$ |
| Me | H | Me | H | Me | H | O | - | - | 0 | F | Cl | $CO_2Et$ |
| $CF_3$ | H | Me | H | Me | H | O | - | - | 0 | F | Cl | $CO_2Pr^i$ |
| $CF_3$ | H | Me | H | Me | H | O | - | - | 0 | F | Cl | $CO_2Pr^n$ |
| $CF_3$ | H | Me | H | Me | H | O | - | - | 0 | F | Cl | $CO_2Bu^n$ |
| $CF_3$ | H | Me | H | Me | H | O | - | - | 0 | F | Cl | $CO_2Bu^t$ |
| $CF_3$ | H | Me | H | Me | H | O | - | - | 0 | F | Cl | $NO_2$ |
| $CF_3$ | H | Me | H | Me | H | O | - | - | 0 | F | Cl | $NH_2$ |
| $CF_3$ | H | Me | H | Me | H | O | - | - | 0 | F | Cl | $NHSO_2Me$ |
| $CF_3$ | H | Me | H | Me | H | O | - | - | 0 | F | Cl | $NHSO_2Et$ |
| $CF_3$ | H | Me | H | Me | H | O | - | - | 0 | F | Cl | $NHSO_2Pr^n$ |
| $CF_3$ | H | Me | H | Me | H | O | - | - | 0 | F | Cl | $NHSO_2Pr^i$ |
| $CF_3$ | H | Me | H | Me | H | O | - | - | 0 | F | Cl | OH |
| $CF_3$ | H | Me | H | Me | H | O | - | - | 0 | F | Cl | OMe |
| $CF_3$ | H | Me | H | Me | H | O | - | - | 0 | F | Cl | OEt |
| $CF_3$ | H | Me | H | Me | H | O | - | - | 0 | F | Cl | $OPr^i$ |
| $CF_3$ | H | Me | H | Me | H | O | - | - | 0 | F | Cl | $OPr^n$ |
| $CF_3$ | H | Me | H | Me | H | O | - | - | 0 | F | Cl | $OPen^c$ |
| $CF_3$ | H | Me | H | Me | H | O | - | - | 0 | F | Cl | $OCH_2CH=CH_2$ |
| $CF_3$ | H | Me | H | Me | H | O | - | - | 0 | F | Cl | $OCH_2C\equiv CH$ |
| $CF_3$ | H | Me | H | Me | H | O | - | - | 0 | F | Cl | $OCH_2CO_2Me$ |
| $CF_3$ | H | Me | H | Me | H | O | - | - | 0 | F | Cl | $OCH_2CO_2Et$ |
| $CF_3$ | H | Me | H | Me | H | O | - | - | 0 | F | Cl | $OCH(Me)CO_2Me$ |
| $CF_3$ | H | Me | H | Me | H | O | - | - | 0 | F | Cl | $OCH(Me)CO_2Et$ |

Table 1-3-1 (Cont'd)

| R1 | R2 | R3 | R4 | R5 | R6 | X | Ra | Rb | m | R7 | R8 | R9 |
|---|---|---|---|---|---|---|---|---|---|---|---|---|
| $CF_3$ | H | Me | H | Me | H | O | - | - | 0 | F | Cl | $CO_2H$ |
| $CF_3$ | H | Me | H | Me | H | O | - | - | 0 | F | Cl | CN |
| $CF_3$ | H | Me | H | Me | H | O | - | - | 0 | F | Cl | Me |
| $CF_3$ | H | Me | H | Me | H | O | - | - | 0 | F | Cl | Et |
| $CF_3$ | H | Me | H | Me | H | O | - | - | 0 | F | Cl | CL |
| $CF_3$ | H | Me | H | Me | H | O | - | - | 0 | F | Cl | Br |
| $CF_3$ | H | Me | H | Me | H | O | - | - | 0 | F | Cl | I |
| $CF_3$ | H | Me | H | Me | H | O | - | - | 0 | F | Cl | F |
| $CF_3$ | H | Me | H | Me | H | O | - | - | 0 | F | Cl | SH |
| $CF_3$ | H | Me | H | Me | H | O | - | - | 0 | F | Cl | $C(Me)=NOCH_2CO_2Me$ |
| $CF_3$ | H | Me | H | Me | H | O | - | - | 0 | F | Cl | $C(Me)=NOCH_2CO_2Et$ |
| $CF_3$ | H | Me | H | Me | H | O | - | - | 0 | F | Cl | $CH=NOCH_2CO_2Me$ |
| $CF_3$ | H | Me | H | Me | H | O | - | - | 0 | F | Cl | $CH=NOCH_2CO_2Et$ |
| $CF_3$ | H | Me | H | Me | H | O | - | - | 0 | F | Cl | $C(OMe)=NOCH_2CO_2Me$ |
| $CF_3$ | H | Me | H | Me | H | O | - | - | 0 | F | Cl | $C(OMe)=NOCH_2CO_2Et$ |
| $CF_3$ | H | Me | H | Me | H | O | - | - | 0 | F | Cl | $C(Me)=NN=C(Me)CO_2Me$ |
| $CF_3$ | H | Me | H | Me | H | O | - | - | 0 | F | Cl | $C(Me)=NN=C(Me)CO_2Et$ |
| $CF_3$ | H | Me | H | Me | H | O | - | - | 0 | F | Cl | $CH_2OCH_2CO_2Me$ |
| $CF_3$ | H | Me | H | Me | H | O | - | - | 0 | F | Cl | $CH_2OCH_2CO_2Et$ |
| $CF_3$ | H | Me | H | Me | H | O | - | - | 0 | F | Cl | $CH_2OCH(Me)CO_2Me$ |
| $CF_3$ | H | Me | H | Me | H | O | - | - | 0 | F | Cl | $CH_2OCH(Me)CO_2Et$ |
| $CF_3$ | H | Me | H | Me | H | O | - | - | 0 | F | Cl | $CH_2SCH_2CO_2Me$ |
| $CF_3$ | H | Me | H | Me | H | O | - | - | 0 | F | Cl | $CH_2SCH_2CO_2Et$ |
| $CF_3$ | H | Me | H | Me | H | O | - | - | 0 | F | Cl | $CH_2SCH(Me)CO_2Me$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | Me | H | O | - | - | 0 | F | Cl | $CH_2SCH(Me)CO_2Et$ |
| $CF_3$ | H | Me | H | Me | H | O | - | - | 0 | F | Cl | SMe |
| $CF_3$ | H | Me | H | Me | H | O | - | - | 0 | F | Cl | SEt |
| $CF_3$ | H | Me | H | Me | H | O | - | - | 0 | F | Cl | $SPr^i$ |
| $CF_3$ | H | Me | H | Me | H | O | - | - | 0 | F | Cl | $SPr^n$ |
| $CF_3$ | H | Me | H | Me | H | O | - | - | 0 | F | Cl | $SPen^c$ |
| $CF_3$ | H | Me | H | Me | H | O | - | - | 0 | F | Cl | $SCH_2CO_2H$ |
| $CF_3$ | H | Me | H | Me | H | O | - | - | 0 | F | Cl | $SCH_2CO_2Me$ |
| $CF_3$ | H | Me | H | Me | H | O | - | - | 0 | F | Cl | $SCH_2CO_2Et$ |
| $CF_3$ | H | Me | H | Me | H | O | - | - | 0 | F | Cl | $SCH(Me)CO_2Me$ |
| $CF_3$ | H | Me | H | Me | H | O | - | - | 0 | F | Cl | $SCH(Me)CO_2Et$ |
| $CF_3$ | H | Me | H | Me | H | O | - | - | 0 | F | Cl | $SCH_2CN$ |
| $CF_3$ | H | Me | H | Me | H | O | - | - | 0 | F | Cl | $SCH_2CH=CH_2$ |
| $CF_3$ | H | Me | H | Me | H | O | - | - | 0 | F | Cl | $SCH_2C\equiv CH$ |
| $CF_3$ | H | Me | H | Me | H | O | - | - | 0 | F | Cl | $OCH(Me)C\equiv CH$ |
| $CF_3$ | H | Me | H | Me | H | O | - | - | 0 | F | Cl | $SCH(Me)C\equiv CH$ |
| $CF_3$ | H | Me | H | Me | H | O | - | - | 0 | F | Cl | $N(SO_2Me)_2$ |
| $CF_3$ | H | Me | H | Me | H | O | - | - | 0 | F | Cl | $N(SO_2Et)_2$ |
| $CF_3$ | H | Me | H | Me | H | O | - | - | 0 | F | Cl | Q1(R29=$Bu^t$) |
| $CF_3$ | H | Me | H | Me | H | O | - | - | 0 | F | Cl | Q2(R29=$Bu^t$) |
| $CF_3$ | H | Me | H | Me | H | O | - | - | 0 | F | Cl | Q5(R29=$Bu^t$) |
| $CF_3$ | H | Me | H | Me | H | O | - | - | 0 | F | Cl | Q7(R34=$Bu^t$) |
| $CF_3$ | H | Me | H | Me | H | O | - | - | 0 | F | Cl | Q9(R38=R39=H) |
| $CF_3$ | H | Me | H | Me | H | O | - | - | 0 | F | Cl | Q10(R38=R39=H) |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | Me | H | O | - | - | 0 | F | Cl | Q4(R29=$Bu^t$ ) |
| $CF_3$ | H | Me | H | Me | H | O | - | - | 0 | F | Cl | Q8(R35=R36=R37=H) |
| $CF_3$ | H | Me | H | Me | H | O | - | - | 0 | F | Cl | Q18(R50=H) |
| $CF_3$ | H | Me | H | Me | H | O | - | - | 0 | F | Cl | Q18(R50=Me) |
| $CF_3$ | H | Me | H | Me | H | O | - | - | 0 | F | Cl | $NHCO_2Me$ |
| $CF_3$ | H | Me | H | Me | H | O | - | - | 0 | F | Cl | $NHCO_2Et$ |
| $CF_3$ | H | Me | H | Me | H | O | - | - | 0 | F | Cl | $NHCO_2Pr^n$ |
| $CF_3$ | H | Me | H | Me | H | O | - | - | 0 | F | Cl | $NHCO_2Pr^i$ |
| $CF_3$ | H | Me | H | Me | H | O | - | - | 0 | F | Cl | $NHCO_2Ph$ |
| $CF_3$ | H | Me | H | Me | H | O | - | - | 0 | F | Cl | $NHCO_2Bz$ |
| $CF_3$ | H | Me | H | Me | H | O | - | - | 0 | F | Cl | NHCOMe |
| $CF_3$ | H | Me | H | Me | H | O | - | - | 0 | F | Cl | $NHCO_2CH_2Ph$ |
| $CF_3$ | H | Me | H | Me | H | O | - | - | 0 | F | Cl | $NHCO_2CH_2$(4-Me-Ph) |
| $CF_3$ | H | Me | H | Me | H | O | - | - | 0 | F | Cl | $NHCO_2CH_2$(3-Me-Ph) |
| $CF_3$ | H | Me | H | Me | H | O | - | - | 0 | F | Cl | $NHCO_2CH_2$(2-Me-Ph) |
| $CF_3$ | H | Me | H | Me | H | O | - | - | 0 | F | Cl | $NHCO_2CH_2$(4-CL-Ph) |
| $CF_3$ | H | Me | H | Me | H | O | - | - | 0 | F | Cl | $NHCO_2CH_2$(3-CL-Ph) |
| $CF_3$ | H | Me | H | Me | H | O | - | - | 0 | F | Cl | $NHCO_2CH_2$(2-CL-Ph) |
| $CF_3$ | H | Me | H | Me | H | O | - | - | 0 | F | Cl | Q1(R29=Me) |
| $CF_3$ | H | Me | H | Me | H | O | - | - | 0 | F | Cl | Q2(R29=Me) |
| $CF_3$ | H | Me | H | Me | H | O | - | - | 0 | F | Cl | H |
| $CF_3$ | H | Me | H | Me | H | O | - | - | 0 | F | Cl | $CO_2H$ |
| $CF_3$ | H | Me | H | Me | H | O | - | - | 0 | F | Cl | $CO_2Me$ |
| $CF_3$ | H | Me | H | Me | H | O | - | - | 0 | F | Cl | $CO_2Et$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | Me | H | 0 | - | - | 0 | F | Cl | $CO_2Pr^i$ |
| Et | H | Me | H | Me | H | 0 | - | - | 0 | F | Cl | $CO_2Pr^n$ |
| Et | H | Me | H | Me | H | 0 | - | - | 0 | F | Cl | $CO_2Bu^n$ |
| Et | H | Me | H | Me | H | 0 | - | - | 0 | F | Cl | $CO_2Bu^t$ |
| Et | H | Me | H | Me | H | 0 | - | - | 0 | F | Cl | $NO_2$ |
| Et | H | Me | H | Me | H | 0 | - | - | 0 | F | Cl | $NH_2$ |
| Et | H | Me | H | Me | H | 0 | - | - | 0 | F | Cl | $NHSO_2Me$ |
| Et | H | Me | H | Me | H | 0 | - | - | 0 | F | Cl | $NHSO_2Et$ |
| Et | H | Me | H | Me | H | 0 | - | - | 0 | F | Cl | $NHSO_2Pr^n$ |
| Et | H | Me | H | Me | H | 0 | - | - | 0 | F | Cl | $NHSO_2Pr^i$ |
| Et | H | Me | H | Me | H | 0 | - | - | 0 | F | Cl | OH |
| Et | H | Me | H | Me | H | 0 | - | - | 0 | F | Cl | OMe |
| Et | H | Me | H | Me | H | 0 | - | - | 0 | F | Cl | OEt |
| Et | H | Me | H | Me | H | 0 | - | - | 0 | F | Cl | $OPr^i$ |
| Et | H | Me | H | Me | H | 0 | - | - | 0 | F | Cl | $OPr^n$ |
| Et | H | Me | H | Me | H | 0 | - | - | 0 | F | Cl | $OPen^c$ |
| Et | H | Me | H | Me | H | 0 | - | - | 0 | F | Cl | $OCH_2CH=CH_2$ |
| Et | H | Me | H | Me | H | 0 | - | - | 0 | F | Cl | $OCH_2C\equiv CH$ |
| Et | H | Me | H | Me | H | 0 | - | - | 0 | F | Cl | $OCH_2CO_2Me$ |
| Et | H | Me | H | Me | H | 0 | - | - | 0 | F | Cl | $OCH_2CO_2Et$ |
| Et | H | Me | H | Me | H | 0 | - | - | 0 | F | Cl | $OCH(Me)CO_2Me$ |
| Et | H | Me | H | Me | H | 0 | - | - | 0 | F | Cl | $OCH(Me)CO_2Et$ |
| Et | H | Me | H | Me | H | 0 | - | - | 0 | F | Cl | $CO_2H$ |
| Et | H | Me | H | Me | H | 0 | - | - | 0 | F | Cl | CN |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | Me | H | O | - | - | 0 | F | Cl | Me |
| Et | H | Me | H | Me | H | O | - | - | 0 | F | Cl | Et |
| Et | H | Me | H | Me | H | O | - | - | 0 | F | Cl | CL |
| Et | H | Me | H | Me | H | O | - | - | 0 | F | Cl | Br |
| Et | H | Me | H | Me | H | O | - | - | 0 | F | Cl | I |
| Et | H | Me | H | Me | H | O | - | - | 0 | F | Cl | F |
| Et | H | Me | H | Me | H | O | - | - | 0 | F | Cl | SH |
| Et | H | Me | H | Me | H | O | - | - | 0 | F | Cl | $C(Me)=NOCH_2CO_2Me$ |
| Et | H | Me | H | Me | H | O | - | - | 0 | F | Cl | $C(Me)=NOCH_2CO_2Et$ |
| Et | H | Me | H | Me | H | O | - | - | 0 | F | Cl | $CH=NOCH_2CO_2Me$ |
| Et | H | Me | H | Me | H | O | - | - | 0 | F | Cl | $CH=NOCH_2CO_2Et$ |
| Et | H | Me | H | Me | H | O | - | - | 0 | F | Cl | $C(OMe)=NOCH_2CO_2Me$ |
| Et | H | Me | H | Me | H | O | - | - | 0 | F | Cl | $C(OMe)=NOCH_2CO_2Et$ |
| Et | H | Me | H | Me | H | O | - | - | 0 | F | Cl | $C(Me)=NN=C(Me)CO_2Me$ |
| Et | H | Me | H | Me | H | O | - | - | 0 | F | Cl | $C(Me)=NN=C(Me)CO_2Et$ |
| Et | H | Me | H | Me | H | O | - | - | 0 | F | Cl | $CH_2OCH_2CO_2Me$ |
| Et | H | Me | H | Me | H | O | - | - | 0 | F | Cl | $CH_2OCH_2CO_2Et$ |
| Et | H | Me | H | Me | H | O | - | - | 0 | F | Cl | $CH_2OCH(Me)CO_2Me$ |
| Et | H | Me | H | Me | H | O | - | - | 0 | F | Cl | $CH_2OCH(Me)CO_2Et$ |
| Et | H | Me | H | Me | H | O | - | - | 0 | F | Cl | $CH_2SCH_2CO_2Me$ |
| Et | H | Me | H | Me | H | O | - | - | 0 | F | Cl | $CH_2SCH_2CO_2Et$ |
| Et | H | Me | H | Me | H | O | - | - | 0 | F | Cl | $CH_2SCH(Me)CO_2Me$ |
| Et | H | Me | H | Me | H | O | - | - | 0 | F | Cl | $CH_2SCH(Me)CO_2Et$ |
| Et | H | Me | H | Me | H | O | - | - | 0 | F | Cl | SMe |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | Me | H | O | - | - | 0 | F | Cl | SEt |
| Et | H | Me | H | Me | H | O | - | - | 0 | F | Cl | $SPr^i$ |
| Et | H | Me | H | Me | H | O | - | - | 0 | F | Cl | $SPr^n$ |
| Et | H | Me | H | Me | H | O | - | - | 0 | F | Cl | $SPen^c$ |
| Et | H | Me | H | Me | H | O | - | - | 0 | F | Cl | $SCH_2CO_2H$ |
| Et | H | Me | H | Me | H | O | - | - | 0 | F | Cl | $SCH_2CO_2Me$ |
| Et | H | Me | H | Me | H | O | - | - | 0 | F | Cl | $SCH_2CO_2Et$ |
| Et | H | Me | H | Me | H | O | - | - | 0 | F | Cl | $SCH(Me)CO_2Me$ |
| Et | H | Me | H | Me | H | O | - | - | 0 | F | Cl | $SCH(Me)CO_2Et$ |
| Et | H | Me | H | Me | H | O | - | - | 0 | F | Cl | $SCH_2CN$ |
| Et | H | Me | H | Me | H | O | - | - | 0 | F | Cl | $SCH_2CH=CH_2$ |
| Et | H | Me | H | Me | H | O | - | - | 0 | F | Cl | $SCH_2C\equiv CH$ |
| Et | H | Me | H | Me | H | O | - | - | 0 | F | Cl | $OCH(Me)C\equiv CH$ |
| Et | H | Me | H | Me | H | O | - | - | 0 | F | Cl | $SCH(Me)C\equiv CH$ |
| Et | H | Me | H | Me | H | O | - | - | 0 | F | Cl | $N(SO_2Me)_2$ |
| Et | H | Me | H | Me | H | O | - | - | 0 | F | Cl | $N(SO_2Et)_2$ |
| Et | H | Me | H | Me | H | O | - | - | 0 | F | Cl | $Q1(R29=Bu^t)$ |
| Et | H | Me | H | Me | H | O | - | - | 0 | F | Cl | $Q2(R29=Bu^t)$ |
| Et | H | Me | H | Me | H | O | - | - | 0 | F | Cl | $Q5(R29=Bu^t)$ |
| Et | H | Me | H | Me | H | O | - | - | 0 | F | Cl | $Q7(R34=Bu^t)$ |
| Et | H | Me | H | Me | H | O | - | - | 0 | F | Cl | Q9(R38=R39=H) |
| Et | H | Me | H | Me | H | O | - | - | 0 | F | Cl | Q10(R38=R39=H) |
| Et | H | Me | H | Me | H | O | - | - | 0 | F | Cl | $Q4(R29=Bu^t)$ |
| Et | H | Me | H | Me | H | O | - | - | 0 | F | Cl | Q8(R35=R36=R37=H) |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | Me | H | O | - | - | 0 | F | Cl | Q18(R50=H) |
| Et | H | Me | H | Me | H | O | - | - | 0 | F | Cl | Q18(R50=Me) |
| Et | H | Me | H | Me | H | O | - | - | 0 | F | Cl | $NHCO_2Me$ |
| Et | H | Me | H | Me | H | O | - | - | 0 | F | Cl | $NHCO_2Et$ |
| Et | H | Me | H | Me | H | O | - | - | 0 | F | Cl | $NHCO_2Pr^n$ |
| Et | H | Me | H | Me | H | O | - | - | 0 | F | Cl | $NHCO_2Pr^i$ |
| Et | H | Me | H | Me | H | O | - | - | 0 | F | Cl | $NHCO_2Ph$ |
| Et | H | Me | H | Me | H | O | - | - | 0 | F | Cl | $NHCO_2Bz$ |
| Et | H | Me | H | Me | H | O | - | - | 0 | F | Cl | NHCOMe |
| Et | H | Me | H | Me | H | O | - | - | 0 | F | Cl | $NHCO_2CH_2Ph$ |
| Et | H | Me | H | Me | H | O | - | - | 0 | F | Cl | $NHCO_2CH_2$(4-Me-Ph) |
| Et | H | Me | H | Me | H | O | - | - | 0 | F | Cl | $NHCO_2CH_2$(3-Me-Ph) |
| Et | H | Me | H | Me | H | O | - | - | 0 | F | Cl | $NHCO_2CH_2$(2-Me-Ph) |
| Et | H | Me | H | Me | H | O | - | - | 0 | F | Cl | $NHCO_2CH_2$(4-Cl-Ph) |
| Et | H | Me | H | Me | H | O | - | - | 0 | F | Cl | $NHCO_2CH_2$(3-Cl-Ph) |
| Et | H | Me | H | Me | H | O | - | - | 0 | F | Cl | $NHCO_2CH_2$(2-Cl-Ph) |
| Et | H | Me | H | Me | H | O | - | - | 0 | F | Cl | Q1(R29=Me) |
| Et | H | Me | H | Me | H | O | - | - | 0 | F | Cl | Q2(R29=Me) |
| Et | H | Me | H | Me | H | O | - | - | 0 | F | Cl | H |
| Et | H | Me | H | Me | H | O | - | - | 0 | F | Cl | $CO_2H$ |
| Et | H | Me | H | Me | H | O | - | - | 0 | F | Cl | $CO_2Me$ |
| Et | H | Me | H | Me | H | O | - | - | 0 | F | Cl | $CO_2Et$ |
| $Pr^n$ | H | Me | H | Me | H | O | - | - | 0 | F | Cl | $CO_2Pr^i$ |
| $Pr^n$ | H | Me | H | Me | H | O | - | - | 0 | F | Cl | $CO_2Pr^n$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | Me | H | O | - | - | 0 | F | Cl | $CO_2Bu^n$ |
| $Pr^n$ | H | Me | H | Me | H | O | - | - | 0 | F | Cl | $CO_2Bu^t$ |
| $Pr^n$ | H | Me | H | Me | H | O | - | - | 0 | F | Cl | $NO_2$ |
| $Pr^n$ | H | Me | H | Me | H | O | - | - | 0 | F | Cl | $NH_2$ |
| $Pr^n$ | H | Me | H | Me | H | O | - | - | 0 | F | Cl | $NHSO_2Me$ |
| $Pr^n$ | H | Me | H | Me | H | O | - | - | 0 | F | Cl | $NHSO_2Et$ |
| $Pr^n$ | H | Me | H | Me | H | O | - | - | 0 | F | Cl | $NHSO_2Pr^n$ |
| $Pr^n$ | H | Me | H | Me | H | O | - | - | 0 | F | Cl | $NHSO_2Pr^i$ |
| $Pr^n$ | H | Me | H | Me | H | O | - | - | 0 | F | Cl | OH |
| $Pr^n$ | H | Me | H | Me | H | O | - | - | 0 | F | Cl | OMe |
| $Pr^n$ | H | Me | H | Me | H | O | - | - | 0 | F | Cl | OEt |
| $Pr^n$ | H | Me | H | Me | H | O | - | - | 0 | F | Cl | $OPr^i$ |
| $Pr^n$ | H | Me | H | Me | H | O | - | - | 0 | F | Cl | $OPr^n$ |
| $Pr^n$ | H | Me | H | Me | H | O | - | - | 0 | F | Cl | $OPen^c$ |
| $Pr^n$ | H | Me | H | Me | H | O | - | - | 0 | F | Cl | $OCH_2CH=CH_2$ |
| $Pr^n$ | H | Me | H | Me | H | O | - | - | 0 | F | Cl | $OCH_2C\equiv CH$ |
| $Pr^n$ | H | Me | H | Me | H | O | - | - | 0 | F | Cl | $OCH_2CO_2Me$ |
| $Pr^n$ | H | Me | H | Me | H | O | - | - | 0 | F | Cl | $OCH_2CO_2Et$ |
| $Pr^n$ | H | Me | H | Me | H | O | - | - | 0 | F | Cl | $OCH(Me)CO_2Me$ |
| $Pr^n$ | H | Me | H | Me | H | O | - | - | 0 | F | Cl | $OCH(Me)CO_2Et$ |
| $Pr^n$ | H | Me | H | Me | H | O | - | - | 0 | F | Cl | $CO_2H$ |
| $Pr^n$ | H | Me | H | Me | H | O | - | - | 0 | F | Cl | CN |
| $Pr^n$ | H | Me | H | Me | H | O | - | - | 0 | F | Cl | Me |
| $Pr^n$ | H | Me | H | Me | H | O | - | - | 0 | F | Cl | Et |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | Me | H | O | - | - | 0 | F | Cl | Cl |
| $Pr^n$ | H | Me | H | Me | H | O | - | - | 0 | F | Cl | Br |
| $Pr^n$ | H | Me | H | Me | H | O | - | - | 0 | F | Cl | I |
| $Pr^n$ | H | Me | H | Me | H | O | - | - | 0 | F | Cl | F |
| $Pr^n$ | H | Me | H | Me | H | O | - | - | 0 | F | Cl | SH |
| $Pr^n$ | H | Me | H | Me | H | O | - | - | 0 | F | Cl | $C(Me)=NOCH_2CO_2Me$ |
| $Pr^n$ | H | Me | H | Me | H | O | - | - | 0 | F | Cl | $C(Me)=NOCH_2CO_2Et$ |
| $Pr^n$ | H | Me | H | Me | H | O | - | - | 0 | F | Cl | $CH=NOCH_2CO_2Me$ |
| $Pr^n$ | H | Me | H | Me | H | O | - | - | 0 | F | Cl | $CH=NOCH_2CO_2Et$ |
| $Pr^n$ | H | Me | H | Me | H | O | - | - | 0 | F | Cl | $C(OMe)=NOCH_2CO_2Me$ |
| $Pr^n$ | H | Me | H | Me | H | O | - | - | 0 | F | Cl | $C(OMe)=NOCH_2CO_2Et$ |
| $Pr^n$ | H | Me | H | Me | H | O | - | - | 0 | F | Cl | $C(Me)=NN=C(Me)CO_2Me$ |
| $Pr^n$ | H | Me | H | Me | H | O | - | - | 0 | F | Cl | $C(Me)=NN=C(Me)CO_2Et$ |
| $Pr^n$ | H | Me | H | Me | H | O | - | - | 0 | F | Cl | $CH_2OCH_2CO_2Me$ |
| $Pr^n$ | H | Me | H | Me | H | O | - | - | 0 | F | Cl | $CH_2OCH_2CO_2Et$ |
| $Pr^n$ | H | Me | H | Me | H | O | - | - | 0 | F | Cl | $CH_2OCH(Me)CO_2Me$ |
| $Pr^n$ | H | Me | H | Me | H | O | - | - | 0 | F | Cl | $CH_2OCH(Me)CO_2Et$ |
| $Pr^n$ | H | Me | H | Me | H | O | - | - | 0 | F | Cl | $CH_2SCH_2CO_2Me$ |
| $Pr^n$ | H | Me | H | Me | H | O | - | - | 0 | F | Cl | $CH_2SCH_2CO_2Et$ |
| $Pr^n$ | H | Me | H | Me | H | O | - | - | 0 | F | Cl | $CH_2SCH(Me)CO_2Me$ |
| $Pr^n$ | H | Me | H | Me | H | O | - | - | 0 | F | Cl | $CH_2SCH(Me)CO_2Et$ |
| $Pr^n$ | H | Me | H | Me | H | O | - | - | 0 | F | Cl | SMe |
| $Pr^n$ | H | Me | H | Me | H | O | - | - | 0 | F | Cl | SEt |
| $Pr^n$ | H | Me | H | Me | H | O | - | - | 0 | F | Cl | $SPr^i$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | Me | H | O | - | - | 0 | F | Cl | $SPr^n$ |
| $Pr^n$ | H | Me | H | Me | H | O | - | - | 0 | F | Cl | $SPen^c$ |
| $Pr^n$ | H | Me | H | Me | H | O | - | - | 0 | F | Cl | $SCH_2CO_2H$ |
| $Pr^n$ | H | Me | H | Me | H | O | - | - | 0 | F | Cl | $SCH_2CO_2Me$ |
| $Pr^n$ | H | Me | H | Me | H | O | - | - | 0 | F | Cl | $SCH_2CO_2Et$ |
| $Pr^n$ | H | Me | H | Me | H | O | - | - | 0 | F | Cl | $SCH(Me)CO_2Me$ |
| $Pr^n$ | H | Me | H | Me | H | O | - | - | 0 | F | Cl | $SCH(Me)CO_2Et$ |
| $Pr^n$ | H | Me | H | Me | H | O | - | - | 0 | F | Cl | $SCH_2CN$ |
| $Pr^n$ | H | Me | H | Me | H | O | - | - | 0 | F | Cl | $SCH_2CH=CH_2$ |
| $Pr^n$ | H | Me | H | Me | H | O | - | - | 0 | F | Cl | $SCH_2C\equiv CH$ |
| $Pr^n$ | H | Me | H | Me | H | O | - | - | 0 | F | Cl | $OCH(Me)C\equiv CH$ |
| $Pr^n$ | H | Me | H | Me | H | O | - | - | 0 | F | Cl | $SCH(Me)C\equiv CH$ |
| $Pr^n$ | H | Me | H | Me | H | O | - | - | 0 | F | Cl | $N(SO_2Me)_2$ |
| $Pr^n$ | H | Me | H | Me | H | O | - | - | 0 | F | Cl | $N(SO_2Et)_2$ |
| $Pr^n$ | H | Me | H | Me | H | O | - | - | 0 | F | Cl | Q1(R29=$Bu^t$) |
| $Pr^n$ | H | Me | H | Me | H | O | - | - | 0 | F | Cl | Q2(R29=$Bu^t$) |
| $Pr^n$ | H | Me | H | Me | H | O | - | - | 0 | F | Cl | Q5(R29=$Bu^t$) |
| $Pr^n$ | H | Me | H | Me | H | O | - | - | 0 | F | Cl | Q7(R34=$Bu^t$) |
| $Pr^n$ | H | Me | H | Me | H | O | - | - | 0 | F | Cl | Q9(R38=R39=H) |
| $Pr^n$ | H | Me | H | Me | H | O | - | - | 0 | F | Cl | Q10(R38=R39=H) |
| $Pr^n$ | H | Me | H | Me | H | O | - | - | 0 | F | Cl | Q4(R29=$Bu^t$) |
| $Pr^n$ | H | Me | H | Me | H | O | - | - | 0 | F | Cl | Q8(R35=R36=R37=H) |
| $Pr^n$ | H | Me | H | Me | H | O | - | - | 0 | F | Cl | Q18(R50=H) |
| $Pr^n$ | H | Me | H | Me | H | O | - | - | 0 | F | Cl | Q18(R50=Me) |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | Me | H | O | - | - | 0 | F | Cl | $NHCO_2Me$ |
| $Pr^n$ | H | Me | H | Me | H | O | - | - | 0 | F | Cl | $NHCO_2Et$ |
| $Pr^n$ | H | Me | H | Me | H | O | - | - | 0 | F | Cl | $NHCO_2Pr^n$ |
| $Pr^n$ | H | Me | H | Me | H | O | - | - | 0 | F | Cl | $NHCO_2Pr^i$ |
| $Pr^n$ | H | Me | H | Me | H | O | - | - | 0 | F | Cl | $NHCO_2Ph$ |
| $Pr^n$ | H | Me | H | Me | H | O | - | - | 0 | F | Cl | $NHCO_2Bz$ |
| $Pr^n$ | H | Me | H | Me | H | O | - | - | 0 | F | Cl | NHCOMe |
| $Pr^n$ | H | Me | H | Me | H | O | - | - | 0 | F | Cl | $NHCO_2CH_2Ph$ |
| $Pr^n$ | H | Me | H | Me | H | O | - | - | 0 | F | Cl | $NHCO_2CH_2$(4-Me-Ph) |
| $Pr^n$ | H | Me | H | Me | H | O | - | - | 0 | F | Cl | $NHCO_2CH_2$(3-Me-Ph) |
| $Pr^n$ | H | Me | H | Me | H | O | - | - | 0 | F | Cl | $NHCO_2CH_2$(2-Me-Ph) |
| $Pr^n$ | H | Me | H | Me | H | O | - | - | 0 | F | Cl | $NHCO_2CH_2$(4-Cl-Ph) |
| $Pr^n$ | H | Me | H | Me | H | O | - | - | 0 | F | Cl | $NHCO_2CH_2$(3-Cl-Ph) |
| $Pr^n$ | H | Me | H | Me | H | O | - | - | 0 | F | Cl | $NHCO_2CH_2$(2-Cl-Ph) |
| $Pr^n$ | H | Me | H | Me | H | O | - | - | 0 | F | Cl | Q1(R29=Me) |
| $Pr^n$ | H | Me | H | Me | H | O | - | - | 0 | F | Cl | Q2(R29=Me) |
| $Pr^n$ | H | Me | H | Me | H | O | - | - | 0 | F | Cl | H |
| $Pr^n$ | H | Me | H | Me | H | O | - | - | 0 | F | Cl | $CO_2H$ |
| $Pr^n$ | H | Me | H | Me | H | O | - | - | 0 | F | Cl | $CO_2Me$ |
| $Pr^n$ | H | Me | H | Me | H | O | - | - | 0 | F | Cl | $CO_2Et$ |
| $Pr^i$ | H | Me | H | Me | H | O | - | - | 0 | F | Cl | $CO_2Pr^i$ |
| $Pr^i$ | H | Me | H | Me | H | O | - | - | 0 | F | Cl | $CO_2Pr^n$ |
| $Pr^i$ | H | Me | H | Me | H | O | - | - | 0 | F | Cl | $CO_2Bu^n$ |
| $Pr^i$ | H | Me | H | Me | H | O | - | - | 0 | F | Cl | $CO_2Bu^t$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | Me | H | O | - | - | 0 | F | Cl | $NO_2$ |
| $Pr^i$ | H | Me | H | Me | H | O | - | - | 0 | F | Cl | $NH_2$ |
| $Pr^i$ | H | Me | H | Me | H | O | - | - | 0 | F | Cl | $NHSO_2Me$ |
| $Pr^i$ | H | Me | H | Me | H | O | - | - | 0 | F | Cl | $NHSO_2Et$ |
| $Pr^i$ | H | Me | H | Me | H | O | - | - | 0 | F | Cl | $NHSO_2Pr^n$ |
| $Pr^i$ | H | Me | H | Me | H | O | - | - | 0 | F | Cl | $NHSO_2Pr^i$ |
| $Pr^i$ | H | Me | H | Me | H | O | - | - | 0 | F | Cl | OH |
| $Pr^i$ | H | Me | H | Me | H | O | - | - | 0 | F | Cl | OMe |
| $Pr^i$ | H | Me | H | Me | H | O | - | - | 0 | F | Cl | OEt |
| $Pr^i$ | H | Me | H | Me | H | O | - | - | 0 | F | Cl | $OPr^i$ |
| $Pr^i$ | H | Me | H | Me | H | O | - | - | 0 | F | Cl | $OPr^n$ |
| $Pr^i$ | H | Me | H | Me | H | O | - | - | 0 | F | Cl | $OPen^c$ |
| $Pr^i$ | H | Me | H | Me | H | O | - | - | 0 | F | Cl | $OCH_2CH=CH_2$ |
| $Pr^i$ | H | Me | H | Me | H | O | - | - | 0 | F | Cl | $OCH_2C\equiv CH$ |
| $Pr^i$ | H | Me | H | Me | H | O | - | - | 0 | F | Cl | $OCH_2CO_2Me$ |
| $Pr^i$ | H | Me | H | Me | H | O | - | - | 0 | F | Cl | $OCH_2CO_2Et$ |
| $Pr^i$ | H | Me | H | Me | H | O | - | - | 0 | F | Cl | $OCH(Me)CO_2Me$ |
| $Pr^i$ | H | Me | H | Me | H | O | - | - | 0 | F | Cl | $OCH(Me)CO_2Et$ |
| $Pr^i$ | H | Me | H | Me | H | O | - | - | 0 | F | Cl | $CO_2H$ |
| $Pr^i$ | H | Me | H | Me | H | O | - | - | 0 | F | Cl | CN |
| $Pr^i$ | H | Me | H | Me | H | O | - | - | 0 | F | Cl | Me |
| $Pr^i$ | H | Me | H | Me | H | O | - | - | 0 | F | Cl | Et |
| $Pr^i$ | H | Me | H | Me | H | O | - | - | 0 | F | Cl | Cl |
| $Pr^i$ | H | Me | H | Me | H | O | - | - | 0 | F | Cl | Br |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | Me | H | O | - | - | 0 | F | Cl | I |
| $Pr^i$ | H | Me | H | Me | H | O | - | - | 0 | F | Cl | F |
| $Pr^i$ | H | Me | H | Me | H | O | - | - | 0 | F | Cl | SH |
| $Pr^i$ | H | Me | H | Me | H | O | - | - | 0 | F | Cl | $C(Me)=NOCH_2CO_2Me$ |
| $Pr^i$ | H | Me | H | Me | H | O | - | - | 0 | F | Cl | $C(Me)=NOCH_2CO_2Et$ |
| $Pr^i$ | H | Me | H | Me | H | O | - | - | 0 | F | Cl | $CH=NOCH_2CO_2Me$ |
| $Pr^i$ | H | Me | H | Me | H | O | - | - | 0 | F | Cl | $CH=NOCH_2CO_2Et$ |
| $Pr^i$ | H | Me | H | Me | H | O | - | - | 0 | F | Cl | $C(OMe)=NOCH_2CO_2Me$ |
| $Pr^i$ | H | Me | H | Me | H | O | - | - | 0 | F | Cl | $C(OMe)=NOCH_2CO_2Et$ |
| $Pr^i$ | H | Me | H | Me | H | O | - | - | 0 | F | Cl | $C(Me)=NN=C(Me)CO_2Me$ |
| $Pr^i$ | H | Me | H | Me | H | O | - | - | 0 | F | Cl | $C(Me)=NN=C(Me)CO_2Et$ |
| $Pr^i$ | H | Me | H | Me | H | O | - | - | 0 | F | Cl | $CH_2OCH_2CO_2Me$ |
| $Pr^i$ | H | Me | H | Me | H | O | - | - | 0 | F | Cl | $CH_2OCH_2CO_2Et$ |
| $Pr^i$ | H | Me | H | Me | H | O | - | - | 0 | F | Cl | $CH_2OCH(Me)CO_2Me$ |
| $Pr^i$ | H | Me | H | Me | H | O | - | - | 0 | F | Cl | $CH_2OCH(Me)CO_2Et$ |
| $Pr^i$ | H | Me | H | Me | H | O | - | - | 0 | F | Cl | $CH_2SCH_2CO_2Me$ |
| $Pr^i$ | H | Me | H | Me | H | O | - | - | 0 | F | Cl | $CH_2SCH_2CO_2Et$ |
| $Pr^i$ | H | Me | H | Me | H | O | - | - | 0 | F | Cl | $CH_2SCH(Me)CO_2Me$ |
| $Pr^i$ | H | Me | H | Me | H | O | - | - | 0 | F | Cl | $CH_2SCH(Me)CO_2Et$ |
| $Pr^i$ | H | Me | H | Me | H | O | - | - | 0 | F | Cl | SMe |
| $Pr^i$ | H | Me | H | Me | H | O | - | - | 0 | F | Cl | SEt |
| $Pr^i$ | H | Me | H | Me | H | O | - | - | 0 | F | Cl | $SPr^i$ |
| $Pr^i$ | H | Me | H | Me | H | O | - | - | 0 | F | Cl | $SPr^n$ |
| $Pr^i$ | H | Me | H | Me | H | O | - | - | 0 | F | Cl | $SPen^c$ |

Table 1-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | Me | H | O | - | - | 0 | F | Cl | $SCH_2CO_2H$ |
| $Pr^i$ | H | Me | H | Me | H | O | - | - | 0 | F | Cl | $SCH_2CO_2Me$ |
| $Pr^i$ | H | Me | H | Me | H | O | - | - | 0 | F | Cl | $SCH_2CO_2Et$ |
| $Pr^i$ | H | Me | H | Me | H | O | - | - | 0 | F | Cl | $SCH(Me)CO_2Me$ |
| $Pr^i$ | H | Me | H | Me | H | O | - | - | 0 | F | Cl | $SCH(Me)CO_2Et$ |
| $Pr^i$ | H | Me | H | Me | H | O | - | - | 0 | F | Cl | $SCH_2CN$ |
| $Pr^i$ | H | Me | H | Me | H | O | - | - | 0 | F | Cl | $SCH_2CH=CH_2$ |
| $Pr^i$ | H | Me | H | Me | H | O | - | - | 0 | F | Cl | $SCH_2C\equiv CH$ |
| $Pr^i$ | H | Me | H | Me | H | O | - | - | 0 | F | Cl | $OCH(Me)C\equiv CH$ |
| $Pr^i$ | H | Me | H | Me | H | O | - | - | 0 | F | Cl | $SCH(Me)C\equiv CH$ |
| $Pr^i$ | H | Me | H | Me | H | O | - | - | 0 | F | Cl | $N(SO_2Me)_2$ |
| $Pr^i$ | H | Me | H | Me | H | O | - | - | 0 | F | Cl | $N(SO_2Et)_2$ |
| $Pr^i$ | H | Me | H | Me | H | O | - | - | 0 | F | Cl | Q1(R29=$Bu^t$) |
| $Pr^i$ | H | Me | H | Me | H | O | - | - | 0 | F | Cl | Q2(R29=$Bu^t$) |
| $Pr^i$ | H | Me | H | Me | H | O | - | - | 0 | F | Cl | Q5(R29=$Bu^t$) |
| $Pr^i$ | H | Me | H | Me | H | O | - | - | 0 | F | Cl | Q7(R34=$Bu^t$) |
| $Pr^i$ | H | Me | H | Me | H | O | - | - | 0 | F | Cl | Q9(R38=R39=H) |
| $Pr^i$ | H | Me | H | Me | H | O | - | - | 0 | F | Cl | Q10(R38=R39=H) |
| $Pr^i$ | H | Me | H | Me | H | O | - | - | 0 | F | Cl | Q4(R29=$Bu^t$) |
| $Pr^i$ | H | Me | H | Me | H | O | - | - | 0 | F | Cl | Q8(R35=R36=R37=H) |
| $Pr^i$ | H | Me | H | Me | H | O | - | - | 0 | F | Cl | Q18(R50=H) |
| $Pr^i$ | H | Me | H | Me | H | O | - | - | 0 | F | Cl | Q18(R50=Me) |
| $Pr^i$ | H | Me | H | Me | H | O | - | - | 0 | F | Cl | $NHCO_2Me$ |
| $Pr^i$ | H | Me | H | Me | H | O | - | - | 0 | F | Cl | $NHCO_2Et$ |

Table 1-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | Me | H | O | - | - | 0 | F | Cl | $NHCO_2Pr^n$ |
| $Pr^i$ | H | Me | H | Me | H | O | - | - | 0 | F | Cl | $NHCO_2Pr^i$ |
| $Pr^i$ | H | Me | H | Me | H | O | - | - | 0 | F | Cl | $NHCO_2Ph$ |
| $Pr^i$ | H | Me | H | Me | H | O | - | - | 0 | F | Cl | $NHCO_2Bz$ |
| $Pr^i$ | H | Me | H | Me | H | O | - | - | 0 | F | Cl | NHCOMe |
| $Pr^i$ | H | Me | H | Me | H | O | - | - | 0 | F | Cl | $NHCO_2CH_2Ph$ |
| $Pr^i$ | H | Me | H | Me | H | O | - | - | 0 | F | Cl | $NHCO_2CH_2$(4-Me-Ph) |
| $Pr^i$ | H | Me | H | Me | H | O | - | - | 0 | F | Cl | $NHCO_2CH_2$(3-Me-Ph) |
| $Pr^i$ | H | Me | H | Me | H | O | - | - | 0 | F | Cl | $NHCO_2CH_2$(2-Me-Ph) |
| $Pr^i$ | H | Me | H | Me | H | O | - | - | 0 | F | Cl | $NHCO_2CH_2$(4-Cl-Ph) |
| $Pr^i$ | H | Me | H | Me | H | O | - | - | 0 | F | Cl | $NHCO_2CH_2$(3-Cl-Ph) |
| $Pr^i$ | H | Me | H | Me | H | O | - | - | 0 | F | Cl | $NHCO_2CH_2$(2-Cl-Ph) |
| $Pr^i$ | H | Me | H | Me | H | O | - | - | 0 | F | Cl | Q1(R29=Me) |
| $Pr^i$ | H | Me | H | Me | H | O | - | - | 0 | F | Cl | Q2(R29=Me) |
| $Pr^i$ | H | Me | H | Me | H | O | - | - | 0 | F | Cl | H |
| $Pr^i$ | H | Me | H | Me | H | O | - | - | 0 | F | Cl | $CO_2H$ |
| $Pr^i$ | H | Me | H | Me | H | O | - | - | 0 | F | Cl | $CO_2Me$ |
| $Pr^i$ | H | Me | H | Me | H | O | - | - | 0 | F | Cl | $CO_2Et$ |

Table 1-4

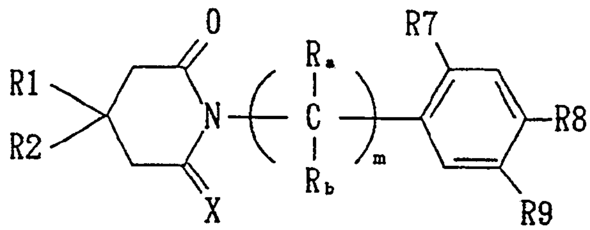

| R1 | R2 | X | $R_a$ | $R_b$ | m | R7 | R8 | R9 |
|---|---|---|---|---|---|---|---|---|
| Me | H | S | - | - | 0 | F | Cl | H |
| Me | H | O | H | H | 1 | H | Cl | H |
| Me | H | O | Me | H | 1 | H | Cl | H |
| Me | H | O | Me | Me | 1 | H | Cl | H |
| Me | H | O | H | H | 2 | H | Cl | H |
| Me | H | O | - | - | 0 | F | Cl | Ph |
| Et | Me | O | - | - | 0 | F | Cl | H |
| Me | H | O | - | - | 0 | F | Cl | $NH_2$ |
| Me | H | O | H | H | 1 | H | Cl | $CO_2Et$ |
| Me | H | O | H | H | 1 | H | $NO_2$ | $CO_2H$ |
| Me | H | O | H | H | 1 | H | $NO_2$ | $CO_2Et$ |
| Me | H | S | - | - | 0 | F | Cl | $CO_2Et$ |
| Me | H | S | H | H | 1 | H | $NO_2$ | $CO_2Et$ |
| Me | H | O | - | - | 0 | F | Cl | $NHSO_2Me$ |
| Me | H | O | - | - | 0 | F | Cl | $N(SO_2Me)_2$ |
| Me | H | O | - | - | 0 | F | Cl | $NHSO_2Et$ |
| Me | H | O | - | - | 0 | F | Cl | Q1(R29=$Bu^t$ ) |

Table 1-4 (Cont'd)

| R1 | R2 | X | $R_a$ | $R_b$ | m | R7 | R8 | R9 |
|---|---|---|---|---|---|---|---|---|
| Me | H | O | - | - | 0 | F | Cl | Q2(R29=$Bu^t$) |
| Me | H | O | - | - | 0 | F | Cl | Q4(R29=$Bu^t$) |
| Me | H | O | - | - | 0 | F | Cl | Q4(R29=$CF_3$) |
| Me | H | O | - | - | 0 | F | Cl | Q5(R29=Cl) |
| Me | H | O | - | - | 0 | F | Cl | Q1(R29=$N(Me)_2$) |
| Me | H | S | - | - | 0 | F | Cl | Q1(R29=$Bu^t$) |
| Me | H | O | - | - | 0 | F | Cl | Q7(R34=$Bu^t$) |
| Me | H | O | - | - | 0 | F | Cl | Q9(R38=R39=H) |
| Me | H | O | - | - | 0 | F | Cl | Q15(R48=Me, R49=H) |
| $CF_3$ | H | S | - | - | 0 | F | Cl | H |
| $CF_3$ | H | O | H | H | 1 | H | Cl | H |
| $CF_3$ | H | O | H | H | 1 | H | $NO_2$ | H |
| $CF_3$ | H | O | H | H | 1 | H | $NO_2$ | $CO_2H$ |
| $CF_3$ | H | O | H | H | 1 | H | $NO_2$ | $CO_2Et$ |
| $CF_3$ | H | O | H | H | 2 | H | $NO_2$ | $CO_2H$ |
| $CF_3$ | H | O | - | - | 0 | F | Cl | Ph |
| $CF_3$ | H | O | - | - | 0 | F | Cl | $NH_2$ |
| $CF_3$ | H | S | - | - | 0 | F | Cl | $OPr^i$ |
| $CF_3$ | H | S | - | - | 0 | F | Cl | $OPen^c$ |
| $CF_3$ | H | S | - | - | 0 | F | Cl | $OCH_2C\equiv CH$ |
| $CF_3$ | H | S | - | - | 0 | F | Cl | $OCH(Me)C\equiv CH$ |
| $CF_3$ | H | S | - | - | 0 | F | Cl | $OCH_2CO_2Me$ |
| $CF_3$ | H | S | - | - | 0 | F | Cl | $OCH(Me)CO_2Et$ |
| $CF_3$ | H | S | - | - | 0 | F | Cl | $OCH(CH_2OMe)CO_2Et$ |

Table 1-4 (Cont'd)

| R1 | R2 | X | $R_a$ | $R_b$ | m | R7 | R8 | R9 |
|---|---|---|---|---|---|---|---|---|
| $CF_3$ | H | S | - | - | 0 | H | Cl | $CO_2H$ |
| $CF_3$ | H | S | - | - | 0 | H | Cl | $CO_2Me$ |
| $CF_3$ | H | S | - | - | 0 | H | Cl | $CO_2Et$ |
| $CF_3$ | H | S | - | - | 0 | H | Cl | $CO_2Pr^i$ |
| $CF_3$ | H | S | - | - | 0 | F | Cl | $CO_2Me$ |
| $CF_3$ | H | S | - | - | 0 | F | Cl | $CO_2Et$ |
| $CF_3$ | H | S | - | - | 0 | F | Cl | $CO_2Pr^n$ |
| $CF_3$ | H | S | - | - | 0 | F | Cl | $CO_2Pr^i$ |
| $CF_3$ | H | S | - | - | 0 | F | Cl | $CO_2Bu^n$ |
| $CF_3$ | H | S | - | - | 0 | F | Cl | $CO_2Bu^t$ |
| $CF_3$ | H | S | - | - | 0 | F | Cl | $CO_2Pen^c$ |
| $CF_3$ | H | S | - | - | 0 | F | Cl | $CO_2Bz$ |
| $CF_3$ | H | S | - | - | 0 | F | Cl | $CO_2Na$ |
| $CF_3$ | H | S | - | - | 0 | F | Cl | $C(OMe)=NOCH_2CO_2Me$ |
| $CF_3$ | H | S | - | - | 0 | F | Cl | $C(Me)=NN=C(Me)CO_2Et$ |
| $CF_3$ | H | S | - | - | 0 | F | Cl | $SPr^i$ |
| $CF_3$ | H | S | - | - | 0 | H | Cl | $SCH_2CO_2Me$ |
| $CF_3$ | H | S | - | - | 0 | F | Cl | $SCH_2CO_2Me$ |
| $CF_3$ | H | O | - | - | 0 | F | Cl | $SCH_2OCH_2CO_2Me$ |
| $CF_3$ | H | O | - | - | 0 | F | Cl | $CH_2OCH_2CO_2Et$ |
| $CF_3$ | H | O | - | - | 0 | F | Cl | $CH_2SCH_2CO_2Et$ |
| $CF_3$ | H | O | H | H | 1 | F | Cl | $SCH_2CO_2Me$ |
| $CF_3$ | H | O | - | - | 0 | F | Cl | $NHSO_2Me$ |
| $CF_3$ | H | O | - | - | 0 | F | Cl | $NHSO_2Et$ |

Table 1-4 (Cont' d)

| R1 | R2 | X | $R_a$ | $R_b$ | m | R7 | R8 | R9 |
|---|---|---|---|---|---|---|---|---|
| $CF_3$ | H | O | - | - | 0 | F | Cl | $N(SO_2Me)_2$ |
| $CF_3$ | H | O | - | - | 0 | F | Cl | $N(SO_2Me)Me$ |
| $CF_3$ | H | O | - | - | 0 | F | Cl | $N(SO_2Me)Et$ |
| $CF_3$ | H | S | - | - | 0 | F | Cl | $NHSO_2Me$ |
| $CF_3$ | H | S | - | - | 0 | F | Cl | $NHSO_2Et$ |
| $CF_3$ | H | S | - | - | 0 | F | Cl | $N(SO_2Me)_2$ |
| $CF_3$ | H | S | - | - | 0 | F | Cl | $N(SO_2Me)Et$ |
| $CF_3$ | H | O | - | - | 0 | F | Cl | Q1(R29=$Bu^t$) |
| $CF_3$ | H | O | - | - | 0 | F | Cl | Q1(R29=$Pen^n$) |
| $CF_3$ | H | O | - | - | 0 | F | Cl | Q1(R29=NHMe) |
| $CF_3$ | H | O | - | - | 0 | F | Cl | Q1(R29=$N(Me)_2$) |
| $CF_3$ | H | O | - | - | 0 | F | Cl | Q1(R29=$CON(Me)_2$) |
| $CF_3$ | H | O | - | - | 0 | F | Cl | Q2(R29=$Bu^t$) |
| $CF_3$ | H | O | - | - | 0 | F | Cl | Q2(R29=$Pen^n$) |
| $CF_3$ | H | O | - | - | 0 | F | Cl | Q3(R29=Ph) |
| $CF_3$ | H | O | - | - | 0 | F | Cl | Q4(R29=$Bu^t$) |
| $CF_3$ | H | O | - | - | 0 | F | Cl | Q4(R29=$Pen^n$) |
| $CF_3$ | H | O | - | - | 0 | F | Cl | Q4(R29=$CON(Me)_2$) |
| $CF_3$ | H | O | - | - | 0 | F | Cl | Q4(R29=$CF_3$) |
| $CF_3$ | H | O | - | - | 0 | F | Cl | Q4(R29=$N(Me)_2$) |
| $CF_3$ | H | O | H | H | 1 | F | Cl | Q2(R29=$Bu^t$) |
| $CF_3$ | H | O | - | - | 0 | F | Cl | Q5(R29=$Bu^t$) |
| $CF_3$ | H | O | - | - | 0 | F | Cl1 | Q5(R29=Cl) |
| $CF_3$ | H | O | - | - | 0 | F | Cl | Q5(R29=$CON(Me)_2$) |

Table 1-4 (Cont' d)

| R1 | R2 | X | $R_a$ | $R_b$ | m | R7 | R8 | R9 |
|---|---|---|---|---|---|---|---|---|
| $CF_3$ | H | O | - | - | 0 | F | Cl | Q7(R34=$Bu^t$ ) |
| $CF_3$ | H | S | - | - | 0 | F | Cl | Q7(R34=$Bu^t$ ) |
| $CF_3$ | H | O | - | - | 0 | F | Cl | Q8(R35=R36=H, R37=Me) |
| $CF_3$ | H | O | - | - | 0 | F | Cl | Q9(R38=R39=H) |
| $CF_3$ | H | O | - | - | 0 | F | Cl | Q9(R38=Cl, R39=H) |
| $CF_3$ | H | O | - | - | 0 | F | Cl | Q10(R38=R39=H) |
| $CF_3$ | H | O | - | - | 0 | F | Cl | Q12(R41=Me, R42=R43=H |
| $CF_3$ | H | O | - | - | 0 | F | Cl | Q13(R44=$Bu^t$ , R45=H) |
| $CF_3$ | H | O | - | - | 0 | F | Cl | Q15(R48=$Bu^t$ , R49=H) |
| $CF_3$ | H | S | - | - | 0 | F | Cl | Q1(R29=$Bu^t$ ) |
| $CF_3$ | H | S | - | - | 0 | F | Cl | Q2(R29=$Bu^t$ ) |
| $CF_3$ | H | S | - | - | 0 | F | Cl | Q4(R29=$Bu^t$ ) |
| $CF_3$ | H | S | - | - | 0 | F | Cl | Q5(R29=$Bu^t$ ) |
| $CF_3$ | H | S | - | - | 0 | F | Cl | Q9(R38=R39=H) |
| $CF_3$ | H | S | - | - | 0 | F | Cl | Q13(R44=$Bu^t$ , R45=H) |
| $CF_3$ | H | S | - | - | 0 | F | Cl | Q15(R48=$Bu^t$ , R49=H) |
| Et | H | S | - | - | 0 | F | Cl | $CO_2Et$ |
| $Pr^n$ | H | S | - | - | 0 | F | Cl | $CO_2Et$ |
| $Pr^i$ | H | S | - | - | 0 | F | Cl | $CO_2Et$ |
| Et | H | O | H | H | 1 | F | Cl | $CO_2Et$ |
| $CF_3O$ | H | O | - | - | 0 | F | Cl | $CO_2Me$ |
| $CF_3O$ | H | O | - | - | 0 | F | Cl | $CO_2Et$ |
| $CF_3S$ | H | O | - | - | 0 | F | Cl | $CO_2Me$ |
| $CF_3S$ | H | O | - | - | 0 | F | Cl | $CO_2Et$ |

Table 1-4 (Cont'd)

| R1 | R2 | X | Ra | Rb | m | R7 | R8 | R9 |
|---|---|---|---|---|---|---|---|---|
| $-CH_2CH_2CH_2-$ | | O | - | - | 0 | F | Cl | $CO_2Et$ |
| $CF_3CF_2$ | H | O | H | H | 1 | F | Cl | H |
| $CF_3CF_2$ | H | O | - | - | 0 | F | Cl | Q1(R29=$Bu^t$) |
| $-CF_2CF_2CF_2-$ | | O | - | - | 0 | F | Cl | $CO_2Et$ |
| $HF_2C$ | H | S | - | - | 0 | F | Cl | $CO_2Et$ |
| $CF_3O$ | H | S | - | - | 0 | F | Cl | $CO_2Et$ |
| $CF_3S$ | H | S | - | - | 0 | F | Cl | $CO_2Et$ |
| $CF_3CF_2$ | H | S | - | - | 0 | F | Cl | $CO_2Et$ |
| Ph | H | O | - | - | 0 | F | Cl | H |
| Ph | H | O | - | - | 0 | F | Cl | $CO_2Me$ |
| Ph | H | O | - | - | 0 | F | Cl | $CO_2Et$ |
| 2-F-Ph | H | O | - | - | 0 | F | Cl | $CO_2Et$ |
| 3-F-Ph | H | O | - | - | 0 | F | Cl | $CO_2Et$ |
| 4-F-Ph | H | O | - | - | 0 | F | Cl | $CO_2Et$ |
| Penta-F-Ph | H | O | - | - | 0 | F | Cl | $CO_2Et$ |
| 2-Cl-Ph | H | O | - | - | 0 | F | Cl | $CO_2Et$ |
| 3-Cl-Ph | H | O | - | - | 0 | F | Cl | $CO_2Et$ |
| 4-Cl-Ph | H | O | - | - | 0 | F | Cl | $CO_2Et$ |
| 2,6-$F_2$-Ph | H | O | - | - | 0 | F | Cl | $CO_2Et$ |
| 2,6-$Cl_2$-Ph | H | O | - | - | 0 | F | Cl | $CO_2Et$ |
| 2,3-$F_2$-Ph | H | O | - | - | 0 | F | Cl | $CO_2Et$ |
| 2-F-Ph | H | O | - | - | 0 | F | Cl | H |
| 3-F-Ph | H | O | - | - | 0 | F | Cl | H |
| 4-F-Ph | H | O | - | - | 0 | F | Cl | H |

Table 1-4 (Cont' d)

| R1 | R2 | X | $R_a$ | $R_b$ | m | R7 | R8 | R9 |
|---|---|---|---|---|---|---|---|---|
| 2-Cl-Ph | H | O | - | - | 0 | F | Cl | H |
| 3-Cl-Ph | H | O | - | - | 0 | F | Cl | H |
| 4-Cl-Ph | H | O | - | - | 0 | F | Cl | H |
| 2-$CF_3$-Ph | H | O | - | - | 0 | F | Cl | H |
| 3-$CF_3$-Ph | H | O | - | - | 0 | F | Cl | H |
| 4-$CF_3$-Ph | H | O | - | - | 0 | F | Cl | H |
| 2-$CF_3$-Ph | H | O | - | - | 0 | F | Cl | $CO_2Et$ |
| 3-$CF_3$-Ph | H | O | - | - | 0 | F | Cl | $CO_2Et$ |
| 4-$CF_3$-Ph | H | O | - | - | 0 | F | Cl | $CO_2Et$ |
| Bz | H | O | - | - | 0 | F | Cl | H |
| Ph | H | S | - | - | 0 | F | Cl | H |
| 2-F-Ph | H | S | - | - | 0 | F | Cl | H |
| 3-F-Ph | H | S | - | - | 0 | F | Cl | H |
| 4-F-Ph | H | S | - | - | 0 | F | Cl | H |
| 2-$CF_3$-Ph | H | S | - | - | 0 | F | Cl | H |
| 3-$CF_3$-Ph | H | S | - | - | 0 | F | Cl | H |
| 4-$CF_3$-Ph | H | S | - | - | 0 | F | Cl | H |
| $CF_3CH_2$ | H | S | - | - | 0 | F | Cl | H |
| $CF_3CH_2$ | H | O | H | H | 1 | H | Cl | H |
| $CF_3CH_2$ | H | S | - | - | 0 | F | Cl | $OPr^i$ |
| $CF_3CH_2$ | H | S | - | - | 0 | F | Cl | OCH(Me)C≡CH |
| $CF_3CH_2$ | H | S | - | - | 0 | F | Cl | $SCH_2CO_2Me$ |
| $CF_3CH_2$ | H | S | - | - | 0 | F | Cl | OCH(Me)$CO_2Me$ |
| F | F | O | - | - | 0 | F | Cl | H |

Table 1-4 (Cont'd)

| R1 | R2 | X | $R_a$ | $R_b$ | m | R7 | R8 | R9 |
|---|---|---|---|---|---|---|---|---|
| F | F | O | - | - | 0 | F | Cl | $CO_2Et$ |
| $CF_3CH_2$ | H | O | H | H | 1 | H | $NO_2$ | $CO_2H$ |
| $CF_3CH_2$ | H | O | H | H | 1 | H | $NO_2$ | $CO_2Et$ |

Table 1-4-1

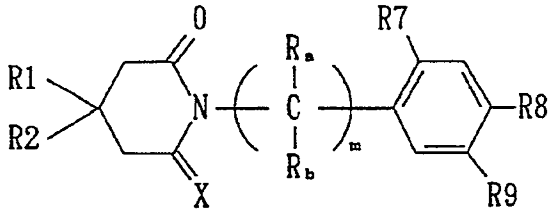

| R1 | R2 | X | Ra | Rb | m | R7 | R8 | R9 |
|---|---|---|---|---|---|---|---|---|
| $-(CH_2)_4-$ | | O | - | - | 0 | F | Cl | H |
| $-(CH_2)_4-$ | | O | - | - | 0 | F | Cl | $CO_2Me$ |
| $-(CH_2)_4-$ | | O | - | - | 0 | F | Cl | $CO_2Et$ |
| Me | H | O | H | H | 1 | H | $NO_2$ | H |
| Me | H | O | - | - | 0 | H | Me | $OCH_2C\equiv CH$ |
| $CF_3$ | H | O | H | H | 1 | F | Cl | H |
| $CF_3$ | H | O | H | H | 1 | F | Cl | $CO_2Me$ |
| $CF_3$ | H | O | H | H | 1 | F | Cl | $CO_2Et$ |
| $CF_3$ | H | O | H | H | 1 | H | $NO_2$ | H |
| Me | H | O | H | H | 1 | F | Cl | $CO_2Pr^i$ |
| Me | H | O | - | - | 0 | F | Cl | Q1(R29=Me) |
| Me | H | O | - | - | 0 | F | Cl | Q1(R29=Et) |
| Me | H | O | - | - | 0 | F | Cl | Q1(R29=$Pr^n$) |
| Me | H | O | - | - | 0 | F | Cl | Q1(R29=$Pr^i$) |
| $CF_3$ | H | O | - | - | 0 | F | Cl | Q1(R29=Me) |
| $CF_3$ | H | O | - | - | 0 | F | Cl | Q1(R29=Et) |
| $CF_3$ | H | O | - | - | 0 | F | Cl | Q1(R29=$Pr^n$ ) |
| $CF_3$ | H | O | - | - | 0 | F | Cl | Q1(R29=$Pr^i$ ) |
| $CF_3$ | H | O | - | - | 0 | F | Cl | Q18(R50=H) |

Table 1-4-1 (Cont'd)

| R1 | R2 | X | $R_a$ | $R_b$ | m | R7 | R8 | R9 |
|---|---|---|---|---|---|---|---|---|
| $CF_3$ | H | O | - | - | 0 | F | Cl | Q18(R50=Me) |
| $CF_3$ | H | O | - | - | 0 | F | F | Q18(R50=Me) |
| Me | H | O | - | - | 0 | F | Cl | $NHCOCH_3$ |
| Me | H | O | - | - | 0 | F | Cl | $NHSO_2Pr^n$ |
| Me | H | O | - | - | 0 | F | Cl | $NHSO_2Pr^i$ |
| Me | H | O | - | - | 0 | F | Cl | $NHCH_2CO_2Me$ |
| Me | H | O | - | - | 0 | F | Cl | $NHCH_2CO_2Et$ |
| Me | H | O | - | - | 0 | F | Cl | $NHCH(Me)CO_2Me$ |
| Me | H | O | - | - | 0 | F | Cl | $NHCH(Me)CO_2Et$ |
| Me | H | O | - | - | 0 | F | Cl | $NHCO_2Me$ |
| Me | H | O | - | - | 0 | F | Cl | $NHCO_2Et$ |
| Me | H | O | - | - | 0 | F | Cl | $NHCO_2Bz$ |
| Me | H | O | - | - | 0 | F | Cl | $NHCO_2CH_2(4\text{-}Me\text{-}Ph)$ |
| Me | H | O | - | - | 0 | F | Cl | $NHCO_2CH_2(3\text{-}Me\text{-}Ph)$ |
| Me | H | O | - | - | 0 | F | Cl | $NHCO_2CH_2(2\text{-}Me\text{-}Ph)$ |
| Me | H | O | - | - | 0 | F | Cl | $NHCO_2CH_2(4\text{-}Cl\text{-}Ph)$ |
| $CF_3$ | H | O | - | - | 0 | F | Cl | $NHCOCH_3$ |
| $CF_3$ | H | O | - | - | 0 | F | Cl | $NHSO_2Pr^n$ |
| $CF_3$ | H | O | - | - | 0 | F | Cl | $NHSO_2Pr^i$ |
| $CF_3$ | H | O | - | - | 0 | F | Cl | $NHCH_2CO_2Me$ |
| $CF_3$ | H | O | - | - | 0 | F | Cl | $NHCH_2CO_2Et$ |
| $CF_3$ | H | O | - | - | 0 | F | Cl | $NHCH(Me)CO_2Me$ |
| $CF_3$ | H | O | - | - | 0 | F | Cl | $NHCH(Me)CO_2Et$ |
| $CF_3$ | H | O | - | - | 0 | F | Cl | $NHCO_2Me$ |

Table 1-4-1 (Cont' d)

| R1 | R2 | X | $R_a$ | $R_b$ | m | R7 | R8 | R9 |
|---|---|---|---|---|---|---|---|---|
| $CF_3$ | H | 0 | - | - | 0 | F | Cl | $NHCO_2Et$ |
| $CF_3$ | H | 0 | - | - | 0 | F | Cl | $NHCO_2Bz$ |
| $CF_3$ | H | 0 | - | - | 0 | F | Cl | $NHCO_2CH_2$(4-Me-Ph) |
| $CF_3$ | H | 0 | - | - | 0 | F | Cl | $NHCO_2CH_2$(3-Me-Ph) |
| $CF_3$ | H | 0 | - | - | 0 | F | Cl | $NHCO_2CH_2$(2-Me-Ph) |
| $CF_3$ | H | 0 | - | - | 0 | F | Cl | $NHCO_2CH_2$(4-Cl-Ph) |
| Ph | H | 0 | - | - | 0 | F | Cl | $NHCOCH_3$ |
| Ph | H | 0 | - | - | 0 | F | Cl | $NHSO_2Pr^n$ |
| Ph | H | 0 | - | - | 0 | F | Cl | $NHSO_2Pr^i$ |
| Ph | H | 0 | - | - | 0 | F | Cl | $NHCH_2CO_2Me$ |
| Ph | H | 0 | - | - | 0 | F | Cl | $NHCH_2CO_2Et$ |
| Ph | H | 0 | - | - | 0 | F | Cl | $NHCH(Me)CO_2Me$ |
| Ph | H | 0 | - | - | 0 | F | Cl | $NHCH(Me)CO_2Et$ |
| Ph | H | 0 | - | - | 0 | F | Cl | $NHCO_2Me$ |
| Ph | H | 0 | - | - | 0 | F | Cl | $NHCO_2Et$ |
| Ph | H | 0 | - | - | 0 | F | Cl | $NHCO_2Bz$ |
| Ph | H | 0 | - | - | 0 | F | Cl | $NHCO_2CH_2$(4-Me-Ph) |
| Ph | H | 0 | - | - | 0 | F | Cl | $NHCO_2CH_2$(3-Me-Ph) |
| Ph | H | 0 | - | - | 0 | F | Cl | $NHCO_2CH_2$(2-Me-Ph) |
| Ph | H | 0 | - | - | 0 | F | Cl | $NHCO_2CH_2$(4-Cl-Ph) |
| Ph | H | 0 | - | - | 0 | F | Cl | $NO_2$ |
| Ph | H | 0 | - | - | 0 | F | Cl | $NH_2$ |
| Ph | H | 0 | - | - | 0 | F | Cl | $NHSO_2Me$ |
| Ph | H | 0 | - | - | 0 | F | Cl | $NHSO_2Et$ |

Table 1-4-1 (Cont'd)

| R1 | R2 | X | $R_a$ | $R_b$ | m | R7 | R8 | R9 |
|---|---|---|---|---|---|---|---|---|
| Ph | H | O | - | - | 0 | F | Cl | $N(SO_2Me)_2$ |
| $Pr^n$ | H | O | - | - | 0 | F | Cl | $NH_2$ |
| $Pr^n$ | H | O | - | - | 0 | F | Cl | $NHSO_2Me$ |
| $Pr^n$ | H | O | - | - | 0 | F | Cl | $NHSO_2Et$ |
| $Pr^n$ | H | O | - | - | 0 | F | Cl | $N(SO_2Me)_2$ |
| $Pr^i$ | H | O | - | - | 0 | F | Cl | $NH_2$ |
| $Pr^i$ | H | O | - | - | 0 | F | Cl | $NHSO_2Me$ |
| $Pr^i$ | H | O | - | - | 0 | F | Cl | $NHSO_2Et$ |
| $Pr^i$ | H | O | - | - | 0 | F | Cl | $N(SO_2Me)_2$ |
| Et | H | O | - | - | 0 | F | Cl | $NH_2$ |
| Et | H | O | - | - | 0 | F | Cl | $NHSO_2Me$ |
| Et | H | O | - | - | 0 | F | Cl | $NHSO_2Et$ |
| Et | H | O | - | - | 0 | F | Cl | $N(SO_2Me)_2$ |
| Et | H | O | - | - | 0 | F | Cl | $NHCOCH_3$ |
| Et | H | O | - | - | 0 | F | Cl | $NHSO_2Pr^n$ |
| Et | H | O | - | - | 0 | F | Cl | $NHSO_2Pr^i$ |
| Et | H | O | - | - | 0 | F | Cl | $NHCH_2CO_2Me$ |
| Et | H | O | - | - | 0 | F | Cl | $NHCH_2CO_2Et$ |
| Et | H | O | - | - | 0 | F | Cl | $NHCH(Me)CO_2Me$ |
| Et | H | O | - | - | 0 | F | Cl | $NHCH(Me)CO_2Et$ |
| Et | H | O | - | - | 0 | F | Cl | $NHCO_2Me$ |
| Et | H | O | - | - | 0 | F | Cl | $NHCO_2Et$ |
| Et | H | O | - | - | 0 | F | Cl | $NHCO_2Bz$ |
| Et | H | O | - | - | 0 | F | Cl | $NHCO_2CH_2(4\text{-}Me\text{-}Ph)$ |

Table 1-4-1 (Cont'd)

| R1 | R2 | X | $R_a$ | $R_b$ | m | R7 | R8 | R9 |
|---|---|---|---|---|---|---|---|---|
| Et | H | O | - | - | 0 | F | Cl | $NHCO_2CH_2$(3-Me-Ph) |
| Et | H | O | - | - | 0 | F | Cl | $NHCO_2CH_2$(2-Me-Ph) |
| Et | H | O | - | - | 0 | F | Cl | $NHCO_2CH_2$(4-Cl-Ph) |
| $Pr^n$ | H | O | - | - | 0 | F | Cl | $NHCOCH_3$ |
| $Pr^n$ | H | O | - | - | 0 | F | Cl | $NHSO_2Pr^n$ |
| $Pr^n$ | H | O | - | - | 0 | F | Cl | $NHSO_2Pr^i$ |
| $Pr^n$ | H | O | - | - | 0 | F | Cl | $NHCH_2CO_2Me$ |
| $Pr^n$ | H | O | - | - | 0 | F | Cl | $NHCH_2CO_2Et$ |
| $Pr^n$ | H | O | - | - | 0 | F | Cl | NHCH(Me)$CO_2Me$ |
| $Pr^n$ | H | O | - | - | 0 | F | Cl | NHCH(Me)$CO_2Et$ |
| $Pr^n$ | H | O | - | - | 0 | F | Cl | $NHCO_2Me$ |
| $Pr^n$ | H | O | - | - | 0 | F | Cl | $NHCO_2Et$ |
| $Pr^n$ | H | O | - | - | 0 | F | Cl | $NHCO_2Bz$ |
| $Pr^n$ | H | O | - | - | 0 | F | Cl | $NHCO_2CH_2$(4-Me-Ph) |
| $Pr^n$ | H | O | - | - | 0 | F | Cl | $NHCO_2CH_2$(3-Me-Ph) |
| $Pr^n$ | H | O | - | - | 0 | F | Cl | $NHCO_2CH_2$(2-Me-Ph) |
| $Pr^n$ | H | O | - | - | 0 | F | Cl | $NHCO_2CH_2$(4-Cl-Ph) |
| $Pr^i$ | H | O | - | - | 0 | F | Cl | $NHCOCH_3$ |
| $Pr^i$ | H | O | - | - | 0 | F | Cl | $NHSO_2Pr^n$ |
| $Pr^i$ | H | O | - | - | 0 | F | Cl | $NHSO_2Pr^i$ |
| $Pr^i$ | H | O | - | - | 0 | F | Cl | $NHCH_2CO_2Me$ |
| $Pr^i$ | H | O | - | - | 0 | F | Cl | $NHCH_2CO_2Et$ |
| $Pr^i$ | H | O | - | - | 0 | F | Cl | NHCH(Me)$CO_2Me$ |
| $Pr^i$ | H | O | - | - | 0 | F | Cl | NHCH(Me)$CO_2Et$ |

Table 1-4-1 (Cont'd)

| R1 | R2 | X | $R_a$ | $R_b$ | m | R7 | R8 | R9 |
|---|---|---|---|---|---|---|---|---|
| $Pr^i$ | H | O | - | - | 0 | F | Cl | $NHCO_2Me$ |
| $Pr^i$ | H | O | - | - | 0 | F | Cl | $NHCO_2Et$ |
| $Pr^i$ | H | O | - | - | 0 | F | Cl | $NHCO_2Bz$ |
| $Pr^i$ | H | O | - | - | 0 | F | Cl | $NHCO_2CH_2$(4-Me-Ph) |
| $Pr^i$ | H | O | - | - | 0 | F | Cl | $NHCO_2CH_2$(3-Me-Ph) |
| $Pr^i$ | H | O | - | - | 0 | F | Cl | $NHCO_2CH_2$(2-Me-Ph) |
| $Pr^i$ | H | O | - | - | 0 | F | Cl | $NHCO_2CH_2$(4-Cl-Ph) |

Table 1-5

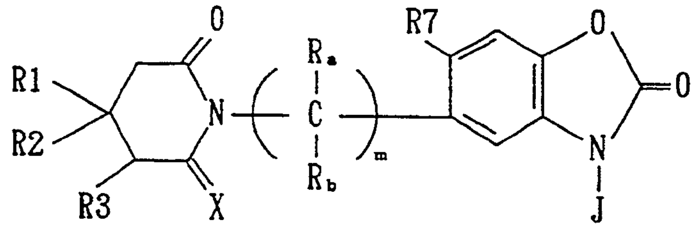

| R1 | R2 | R3 | X | $R_a$ | $R_b$ | m | R7 | J |
|---|---|---|---|---|---|---|---|---|
| $CF_3$ | H | H | O | - | - | 0 | F | Et |
| $CF_3$ | H | H | O | - | - | 0 | F | $CH_2C\equiv CH$ |
| $CF_3$ | H | H | O | - | - | 0 | F | Bz |
| $CF_3$ | H | H | O | - | - | 0 | H | $CH_2C\equiv CH$ |
| $CF_3$ | H | H | S | - | - | 0 | F | $CH_2C\equiv CH$ |
| $CF_3$ | H | Me | O | - | - | 0 | F | $CH_2C\equiv CH$ |
| $CH_3$ | H | H | O | - | - | 0 | F | $CH_2C\equiv CH$ |
| $CH_3$ | H | H | S | - | - | 0 | F | $CH_2C\equiv CH$ |

Table 1-5-1

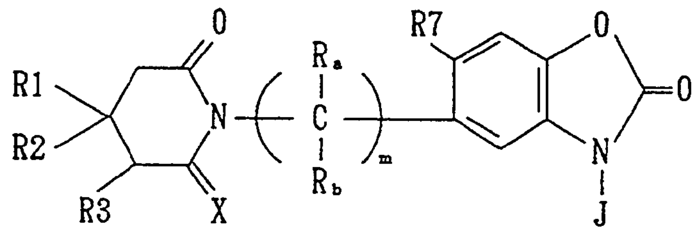

| R1 | R2 | R3 | X | $R_a$ | $R_b$ | m | R7 | J |
|---|---|---|---|---|---|---|---|---|
| Me | H | Me | O | - | - | 0 | F | $CH_2C\equiv CH$ |
| Ft | H | Me | O | - | - | 0 | F | $CH_2C\equiv CH$ |
| $Pr^n$ | H | Me | O | - | - | 0 | F | $CH_2C\equiv CH$ |
| $Pr^i$ | H | Me | O | - | - | 0 | F | $\cdot CH_2C\equiv CH$ |
| H | $-CH_2CH_2CH_2-$ | | O | - | - | 0 | F | $CH_2C\equiv CH$ |

Table 1－6

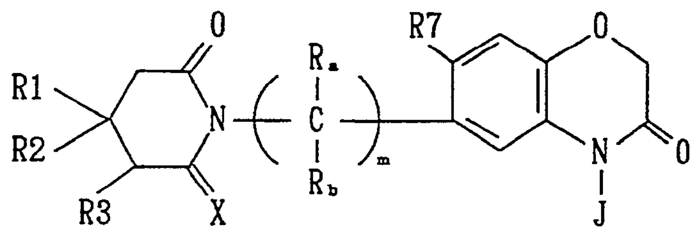

| R1 | R2 | R3 | X | $R_a$ | $R_b$ | m | R7 | J |
|---|---|---|---|---|---|---|---|---|
| $CH_3$ | H | H | O | - | - | 0 | F | $CH_2C\equiv CH$ |
| $CH_3$ | H | H | S | - | - | 0 | F | $CH_2C\equiv CH$ |
| $CH_3$ | H | H | O | - | - | 0 | F | Et |
| $CH_3$ | H | H | S | - | - | 0 | F | Et |
| $CH_3$ | H | H | O | - | - | 0 | H | $CH_2C\equiv CH$ |
| Et | H | H | O | - | - | 0 | F | $CH_2C\equiv CH$ |
| $Pr^n$ | H | H | O | - | - | 0 | F | $CH_2C\equiv CH$ |
| $Pr^i$ | H | H | O | - | - | 0 | F | $CH_2C\equiv CH$ |
| $-CH_2CH_2CH_2-$ | | H | O | - | - | 0 | F | $CH_2C\equiv CH$ |
| $Bu^t$ | H | H | O | - | - | 0 | F | $CH_2C\equiv CH$ |
| $CH_3$ | H | H | O | - | - | 0 | F | $CH_2CH=CH_2$ |
| $CH_3$ | H | H | O | - | - | 0 | F | Q16 |
| $CF_3$ | H | H | O | - | - | 0 | F | $CH_2C\equiv CH$ |
| $CF_3$ | H | H | S | - | - | 0 | F | $CH_2C\equiv CH$ |
| $CF_3$ | H | H | O | - | - | 0 | F | $CH_2CH=CH_2$ |
| $CF_3$ | H | H | S | - | - | 0 | F | $CH_2CH=CH_2$ |
| $CF_3$ | H | H | O | - | - | 0 | F | Q16 |
| $CF_3$ | H | H | O | - | - | 0 | F | Q17 |

Table 1-6 (Cont'd)

| R1 | R2 | R3 | X | $R_a$ | $R_b$ | m | R7 | J |
|---|---|---|---|---|---|---|---|---|
| $CF_3$ | H | H | S | - | - | 0 | F | Q16 |
| $CF_3$ | H | H | S | - | - | 0 | F | Q17 |
| $CF_3$ | H | H | O | - | - | 0 | F | Et |
| $CF_3$ | H | H | S | - | - | 0 | F | Et |
| $CF_3$ | H | H | O | - | - | 0 | F | $CH_2OH$ |
| $CF_3$ | H | H | O | - | - | 0 | F | $CH_2Cl$ |
| $CF_3$ | H | H | O | H | H | 1 | F | $CH_2C\equiv CH$ |
| $CF_3$ | H | H | O | - | - | 0 | F | Bz |
| $CF_3$ | H | H | S | - | - | 0 | F | Bz |
| $CF_3CF_2$ | H | H | O | - | - | 0 | F | $CH_2C\equiv CH$ |
| $CF_3CF_2$ | H | H | O | - | - | 0 | F | $CH_2CH=CH_2$ |
| $CF_3CF_2$ | H | H | O | - | - | 0 | F | $CH_2C\equiv N$ |
| $CF_3CF_2$ | H | H | O | - | - | 0 | F | Bz |
| $CF_3CF_2$ | H | H | O | - | - | 0 | F | Q16 |
| $CF_3CF_2$ | H | H | S | - | - | 0 | F | $CH_2C\equiv CH$ |
| $CF_3CH_2$ | H | H | O | - | - | 0 | F | $CH_2C\equiv CH$ |
| $CF_3CH_2$ | H | H | S | - | - | 0 | F | $CH_2C\equiv CH$ |
| $CF_3CH_2$ | H | H | O | - | - | 0 | H | $CH_2C\equiv CH$ |
| $CF_3CH_2$ | H | H | S | - | - | 0 | H | $CH_2C\equiv CH$ |
| $CF_3CF_2$ | H | H | O | - | - | 0 | H | $CH_2C\equiv CH$ |
| $CF_3CF_2$ | H | H | S | - | - | 0 | H | $CH_2C\equiv CH$ |
| $-CF_2CF_2CF_2-$ | | H | O | - | - | 0 | F | $CH_2C\equiv CH$ |
| $CF_3$ | H | Me | O | - | - | 0 | F | $CH_2C\equiv CH$ |
| $CF_3CF_2$ | H | Me | O | - | - | 0 | F | $CH_2C\equiv CH$ |

Table 1-6 (Cont'd)

| R1 | R2 | R3 | X | $R_a$ | $R_b$ | m | R7 | J |
|---|---|---|---|---|---|---|---|---|
| $CF_3CF_2$ | H | Me | O | - | - | 0 | F | $CH_2C\equiv CH$ |
| $CF_3$ | H | H | O | - | - | 0 | F | H |
| $CF_3CF_2$ | H | H | O | - | - | 0 | F | H |
| $CF_3CH_2$ | H | H | O | - | - | 0 | F | H |
| $CH_3$ | H | H | O | - | - | 0 | F | H |
| $CF_3$ | H | H | O | - | - | 0 | H | H |

Table 1-6-1

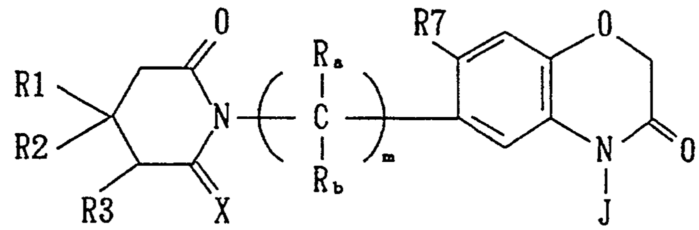

| R1 | R2 | R3 | X | $R_a$ | $R_b$ | m | R7 | J |
|---|---|---|---|---|---|---|---|---|
| Me | H | Me | O | - | - | 0 | F | $CH_2C{\equiv}CH$ |
| $CF_3$ | H | Me | O | - | - | 0 | H | $CH_2C{\equiv}CH$ |
| $CF_3$ | H | H | O | - | - | 0 | H | H |
| $CF_3$ | H | H | O | - | - | 0 | H | $CH_2C{\equiv}CH$ |
| $CF_3$ | H | H | O | - | - | 0 | F | $CH_2$-Q19 |
| Et | H | Me | O | - | - | 0 | F | $CH_2C{\equiv}CH$ |
| Et | H | Me | O | - | - | 0 | F | H |
| $Pr^n$ | H | Me | O | - | - | 0 | F | H |
| $Pr^n$ | H | Me | O | - | - | 0 | F | $CH_2C{\equiv}CH$ |
| $Pr^i$ | H | Me | O | - | - | 0 | F | H |
| $Pr^i$ | H | Me | O | - | - | 0 | F | $CH_2C{\equiv}CH$ |
| $Pr^i$ | H | Me | O | - | - | 0 | F | Et |
| $Pr^n$ | H | Me | O | - | - | 0 | F | Et |
| Ph | H | H | O | - | - | 0 | F | $CH_2C{\equiv}CH$ |
| Ph | H | Me | O | - | - | 0 | F | $CH_2C{\equiv}CH$ |
| Me | H | $CF_3$ | O | - | - | 0 | F | $CH_2C{\equiv}CH$ |
| H | $-CH_2CH_2CH_2-$ | | O | - | - | 0 | F | $CH_2C{\equiv}CH$ |
| Ph | H | H | O | - | - | 0 | F | H |

Table 1-6-1 (Cont'd)

| R1 | R2 | R3 | X | $R_a$ | $R_b$ | m | R7 | J |
|---|---|---|---|---|---|---|---|---|
| Ph | H | Me | O | - | - | 0 | F | H |
| H | $-CH_2CH_2CH_2-$ | | O | - | - | 0 | F | H |

Table 1-7

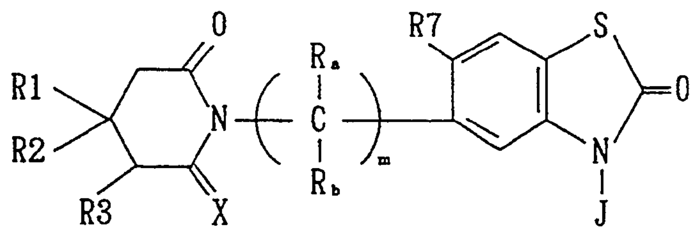

| R1 | R2 | R3 | X | $R_a$ | $R_b$ | m | R7 | J |
|---|---|---|---|---|---|---|---|---|
| $CH_3$ | H | H | O | - | - | 0 | F | $CH_2C\equiv CH$ |
| $CH_3$ | H | H | S | - | - | 0 | F | $CH_2C\equiv CH$ |
| $CH_3$ | H | H | O | - | - | 0 | F | Et |
| $CH_3$ | H | H | S | - | - | 0 | F | Et |
| $CH_3$ | H | H | O | - | - | 0 | H | $CH_2C\equiv CH$ |
| Et | H | H | O | - | - | 0 | F | $CH_2C\equiv CH$ |
| $Pr^n$ | H | H | O | - | - | 0 | F | $CH_2C\equiv CH$ |
| $Pr^i$ | H | H | O | - | - | 0 | F | $CH_2C\equiv CH$ |
| $-CH_2CH_2CH_2-$ | | H | O | - | - | 0 | F | $CH_2C\equiv CH$ |
| $Bu^t$ | H | H | O | - | - | 0 | F | $CH_2C\equiv CH$ |
| $CH_3$ | H | H | O | - | - | 0 | F | $CH_2CH=CH_2$ |
| $CH_3$ | H | H | O | - | - | 0 | F | Q16 |
| $CF_3$ | H | H | O | - | - | 0 | F | $CH_2C\equiv CH$ |
| $CF_3$ | H | H | S | - | - | 0 | F | $CH_2C\equiv CH$ |
| $CF_3$ | H | H | O | - | - | 0 | F | $CH_2CH=CH_2$ |

Table 1-7 (Cont'd)

| R1 | R2 | R3 | X | $R_a$ | $R_b$ | m | R7 | J |
|---|---|---|---|---|---|---|---|---|
| $CF_3$ | H | H | S | - | - | 0 | F | $CH_2CH=CH_2$ |
| $CF_3$ | H | H | O | - | - | 0 | F | Q16 |
| $CF_3$ | H | H | O | - | - | 0 | F | Q17 |
| $CF_3$ | H | H | S | - | - | 0 | F | Q16 |
| $CF_3$ | H | H | S | - | - | 0 | F | Q17 |
| $CF_3$ | H | H | O | - | - | 0 | F | Et |
| $CF_3$ | H | H | S | - | - | 0 | F | Et |
| $CF_3$ | H | H | O | - | - | 0 | F | $CH_2OH$ |
| $CF_3$ | H | H | O | - | - | 0 | F | $CH_2Cl$ |
| $CF_3$ | H | H | O | H | H | 1 | F | $CH_2C\equiv CH$ |
| $CF_3$ | H | H | O | - | - | 0 | F | Bz |
| $CF_3$ | H | H | S | - | - | 0 | F | Bz |
| $CF_3CF_2$ | H | H | O | - | - | 0 | F | $CH_2C\equiv CH$ |
| $CF_3CF_2$ | H | H | O | - | - | 0 | F | $CH_2CH=CH_2$ |
| $CF_3CF_2$ | H | H | O | - | - | 0 | F | $CH_2C\equiv N$ |
| $CF_3CF_2$ | H | H | O | - | - | 0 | F | Bz |
| $CF_3CF_2$ | H | H | O | - | - | 0 | F | Q16 |
| $CF_3CF_2$ | H | H | S | - | - | 0 | F | $CH_2C\equiv CH$ |
| $CF_3CH_2$ | H | H | O | - | - | 0 | F | $CH_2C\equiv CH$ |
| $CF_3CH_2$ | H | H | S | - | - | 0 | F | $CH_2C\equiv CH$ |
| $CF_3CH_2$ | H | H | O | - | - | 0 | H | $CH_2C\equiv CH$ |
| $CF_3CH_2$ | H | H | S | - | - | 0 | H | $CH_2C\equiv CH$ |
| $CF_3CF_2$ | H | H | O | - | - | 0 | H | $CH_2C\equiv CH$ |
| $CF_3CF_2$ | H | H | S | - | - | 0 | H | $CH_2C\equiv CH$ |

Table 1-7 (Cont'd)

| R1 | R2 | R3 | X | $R_a$ | $R_b$ | m | R7 | J |
|---|---|---|---|---|---|---|---|---|
| $-CF_2CF_2CF_2-$ | | H | O | - | - | 0 | F | $CH_2C\equiv CH$ |
| $CF_3$ | H | Me | O | - | - | 0 | F | $CH_2C\equiv CH$ |
| $CF_3CF_2$ | H | Me | O | - | - | 0 | F | $CH_2C\equiv CH$ |
| $CF_3CF_2$ | H | Me | O | - | - | 0 | F | $CH_2C\equiv CH$ |
| $CF_3$ | H | H | O | - | - | 0 | F | H |
| $CF_3CF_2$ | H | H | O | - | - | 0 | F | H |
| $CF_3CH_2$ | H | H | O | - | - | 0 | F | H |
| $CH_3$ | H | H | O | - | - | 0 | F | H |
| $CF_3$ | H | H | O | - | - | 0 | H | H |

Table 1-7-1

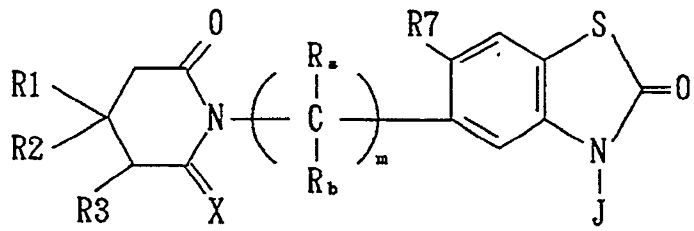

| R1 | R2 | R3 | X | $R_a$ | $R_b$ | m | R7 | J |
|---|---|---|---|---|---|---|---|---|
| Me | H | Me | O | - | - | 0 | F | $CH_2C\equiv CH$ |
| $CF_3$ | H | Me | O | - | - | 0 | H | $CH_2C\equiv CH$ |
| $CF_3$ | H | H | O | - | - | 0 | H | H |
| $CF_3$ | H | H | O | - | - | 0 | H | $CH_2C\equiv CH$ |
| $CF_3$ | H | H | O | - | - | 0 | F | $CH_2$-Q19 |
| Et | H | Me | O | - | - | 0 | F | $CH_2C\equiv CH$ |
| Et | H | Me | O | - | - | 0 | F | H |
| $Pr^n$ | H | Me | O | - | - | 0 | F | H |
| $Pr^n$ | H | Me | O | - | - | 0 | F | $CH_2C\equiv CH$ |
| $Pr^i$ | H | Me | O | - | - | 0 | F | H |
| $Pr^i$ | H | Me | O | - | - | 0 | F | $CH_2C\equiv CH$ |
| $Pr^i$ | H | Me | O | - | - | 0 | F | Et |
| $Pr^n$ | H | Me | O | - | - | 0 | F | Et |
| Ph | H | H | O | - | - | 0 | F | $CH_2C\equiv CH$ |
| Ph | H | Me | O | - | - | 0 | F | $CH_2C\equiv CH$ |
| Me | H | $CF_3$ | O | - | - | 0 | F | $CH_2C\equiv CH$ |
| H | $-CH_2CH_2CH_2-$ | | O | - | - | 0 | F | $CH_2C\equiv CH$ |

Table 1-7-1 (Cont'd)

| R1 | R2 | R3 | X | $R_a$ | $R_b$ | m | R7 | J |
|---|---|---|---|---|---|---|---|---|
| Ph | H | H | O | - | - | 0 | F | H |
| Ph | H | Me | O | - | - | 0 | F | H |
| H | $-CH_2CH_2CH_2-$ | | O | - | - | 0 | F | H |

Table 1-8

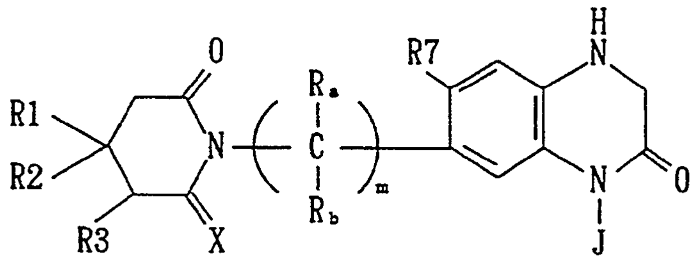

| R1 | R2 | R3 | X | $R_a$ | $R_b$ | m | R7 | J |
|---|---|---|---|---|---|---|---|---|
| $CF_3$ | H | H | O | - | - | 0 | F | Et |
| $CF_3$ | H | H | O | - | - | 0 | F | $CH_2C\equiv CH$ |
| $CF_3$ | H | H | O | - | - | 0 | F | Bz |
| $CF_3$ | H | H | O | - | - | 0 | H | $CH_2C\equiv CH$ |
| $CF_3$ | H | H | S | - | - | 0 | F | $CH_2C\equiv CH$ |
| $CF_3$ | H | Me | O | - | - | 0 | F | $CH_2C\equiv CH$ |
| $CH_3$ | H | H | O | - | - | 0 | F | $CH_2C\equiv CH$ |
| $CH_3$ | H | H | S | - | - | 0 | F | $CH_2C\equiv CH$ |

Table 1-8-1

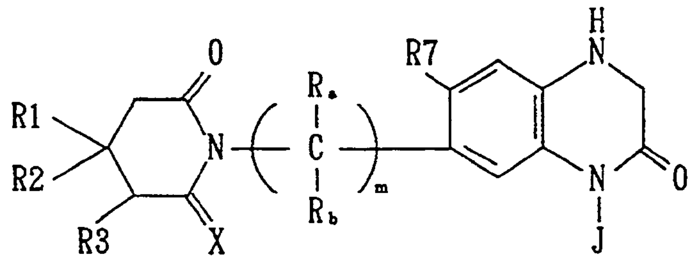

| R1 | R2 | R3 | X | $R_a$ | $R_b$ | m | R7 | J |
|---|---|---|---|---|---|---|---|---|
| Me | H | Me | O | - | - | 0 | F | $CH_2C\equiv CH$ |
| Et | H | Me | O | - | - | 0 | F | $CH_2C\equiv CH$ |
| $Pr^n$ | H | Me | O | - | - | 0 | F | $CH_2C\equiv CH$ |
| $Pr^i$ | H | Me | O | - | - | 0 | F | $CH_2C\equiv CH$ |
| H | $-CH_2CH_2CH_2-$ | | O | - | - | 0 | F | $CH_2C\equiv CH$ |
| $Pr^n$ | H | Me | O | - | - | 0 | F | H |
| $Pr^i$ | H | Me | O | - | - | 0 | F | H |
| Et | H | Me | O | - | - | 0 | F | H |
| Me | H | Me | O | - | - | 0 | F | H |
| $CF_3$ | H | Me | O | - | - | 0 | F | H |

Table 1-9

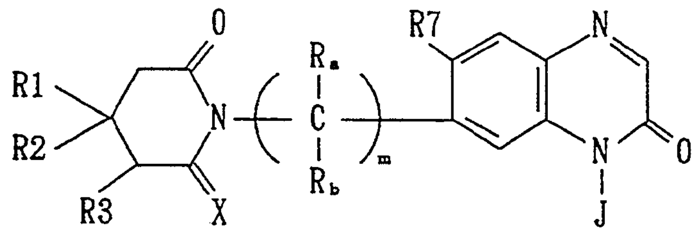

| R1 | R2 | R3 | X | $R_a$ | $R_b$ | m | R7 | J |
|---|---|---|---|---|---|---|---|---|
| $CF_3$ | H | H | O | - | - | 0 | F | Et |
| $CF_3$ | H | H | O | - | - | 0 | F | $CH_2C\equiv CH$ |
| $CF_3$ | H | H | O | - | - | 0 | F | Bz |
| $CF_3$ | H | H | O | - | - | 0 | H | $CH_2C\equiv CH$ |
| $CF_3$ | H | H | S | - | - | 0 | F | $CH_2C\equiv CH$ |
| $CF_3$ | H | Me | O | - | - | 0 | F | $CH_2C\equiv CH$ |
| $CH_3$ | H | H | O | - | - | 0 | F | $CH_2C\equiv CH$ |
| $CH_3$ | H | H | S | - | - | 0 | F | $CH_2C\equiv CH$ |

Table 1-9-1

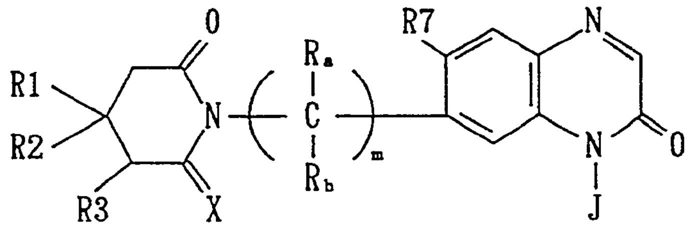

| R1 | R2 | R3 | X | $R_a$ | $R_b$ | m | R7 | J |
|---|---|---|---|---|---|---|---|---|
| Me | H | Me | O | - | - | 0 | F | $CH_2C{\equiv}CH$ |
| Et | H | Me | O | - | - | 0 | F | $CH_2C{\equiv}CH$ |
| $Pr^n$ | H | Me | O | - | - | 0 | F | $CH_2C{\equiv}CH$ |
| $Pr^i$ | H | Me | O | - | - | 0 | F | $CH_2C{\equiv}CH$ |
| H | $-CH_2CH_2CH_2-$ | | O | - | - | 0 | F | $CH_2C{\equiv}CH$ |
| $Pr^n$ | H | Me | O | - | - | 0 | F | H |
| $Pr^i$ | H | Me | O | - | - | 0 | F | H |
| Et | H | Me | O | - | - | 0 | F | H |
| Me | H | Me | O | - | - | 0 | F | H |
| $CF_3$ | H | Me | O | - | - | 0 | F | H |

Symbols are groups represented by the following chemical structures.

Me ; $CH_3$

$Pen^n$ ; $CH_2C(CH_3)_3$

Et ; $CH_2CH_3$

Penta-F-Ph ; 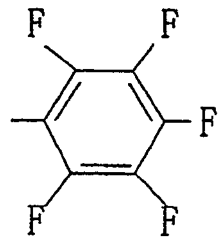

Ph ; 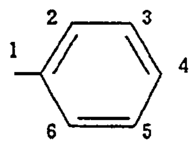

2-F-Ph ; 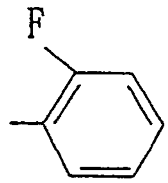

$Pr^i$ ; $CH(CH_3)_2$

$Bu^n$ ; $CH_2CH_2CH_2CH_3$

$Pr^n$ ; $CH_2CH_2CH_3$

$Bu^t$ ; $C(CH_3)_3$

$Pen^c$ ; 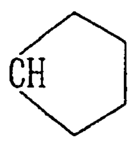

Bz ; $CH_2C_6H_5$

Q1 ;

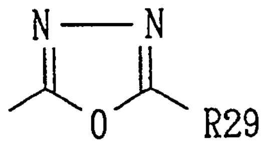

Q2 ;

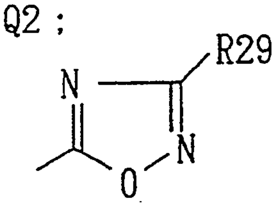

Q3;

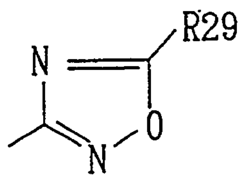

Q4;

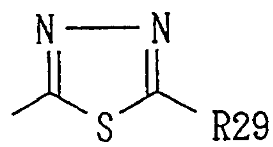

Q5;

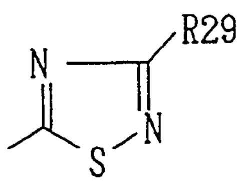

Q6;

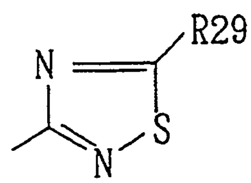

Q7;

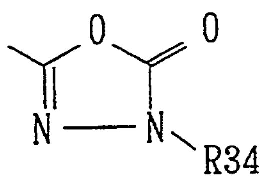

Q8;

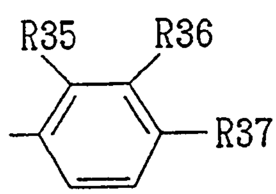

Q9;

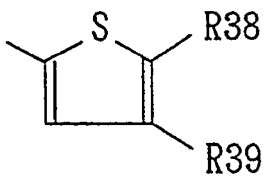

Q10;

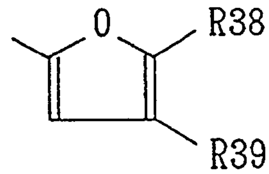

Q11;

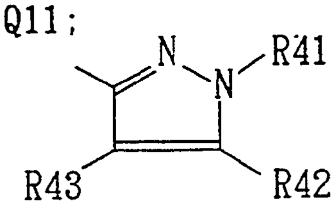

Q12;

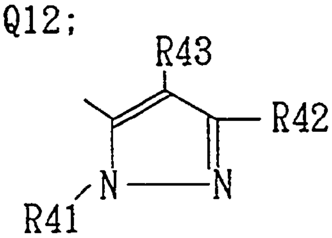

Q13;

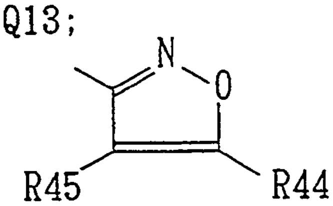

Q14;

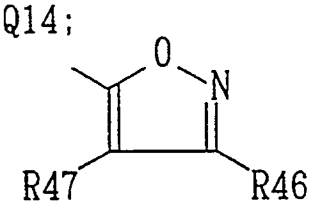

Q15;

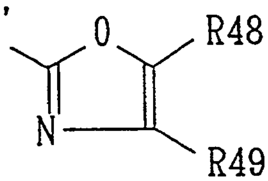

Q16;

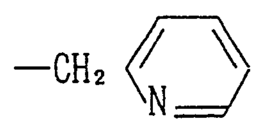

Q17;

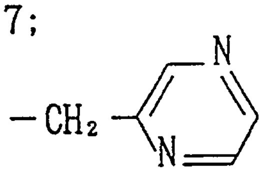

Q18;

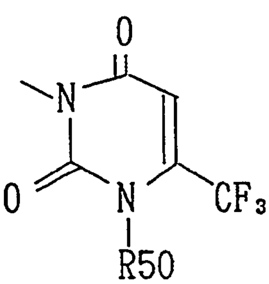

Q19;

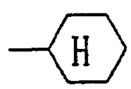

Example 2-1

Synthesis of 3-methyl-4-(N-7-fluoro-2H-1,4-benzoxazin-3(4H)-on-6-yl)carbamoyl)butyric acid

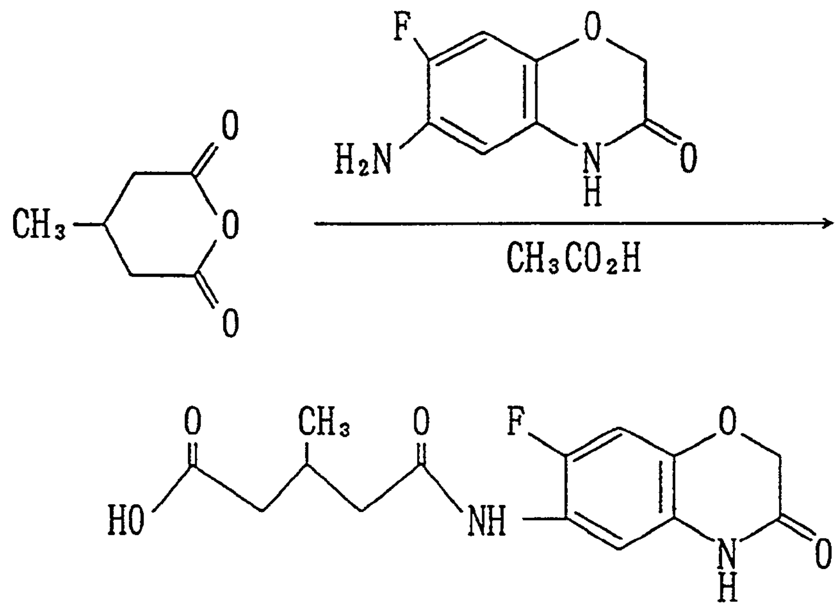


In 10 ml of acetic acid were dissolved 0.35 g of 3-methylglutaric anhydride and 0.5 g of 7-fluoro-6-amino-2H-1,4-benzoxazin-3(4H)-on, and the mixture was stirred for 2 hours under reflux. After completion of the reaction, the reaction mixture was poured into water and extracted with ethyl acetate, and the extract was washed with saturated saline solution and dried over anhydrous sodium sulfate. Then, ethyl acetate was removed to obtain 0.53 g of the title compound as crystals.

Example 2-2

Synthesis of 3,3-dimethyl-4-(N-(4-chloro-2-fluorophenyl)carbamoyl)butyric acid

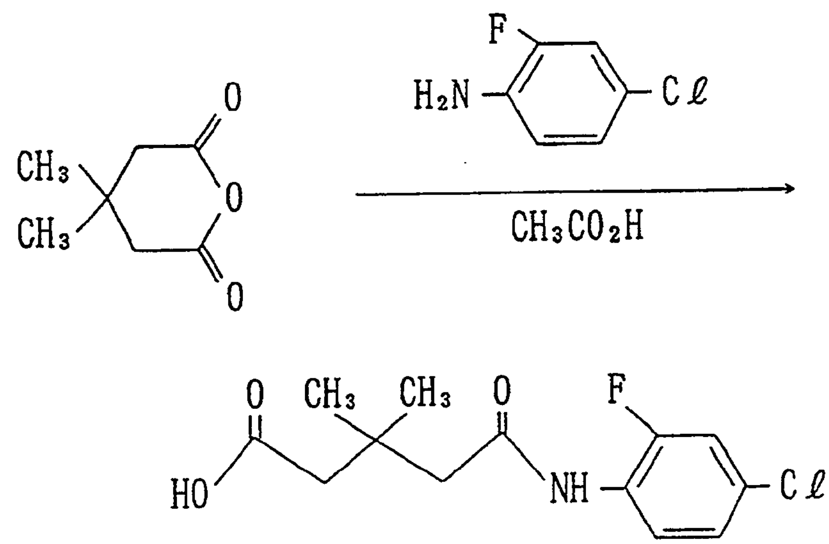


In the same manner as in Example 2-1, from 1 g of 3,3-dimethylglutaric anhydride and 1 g of 4-chloro-2-fluoro aniline, 1.54 g of the title compound was obtained as crystals.

Example 2-3

Synthesis of 2,2-dimethyl-4-(N-(4-chloro-2-fluorophenyl)carbamoyl)butyric acid

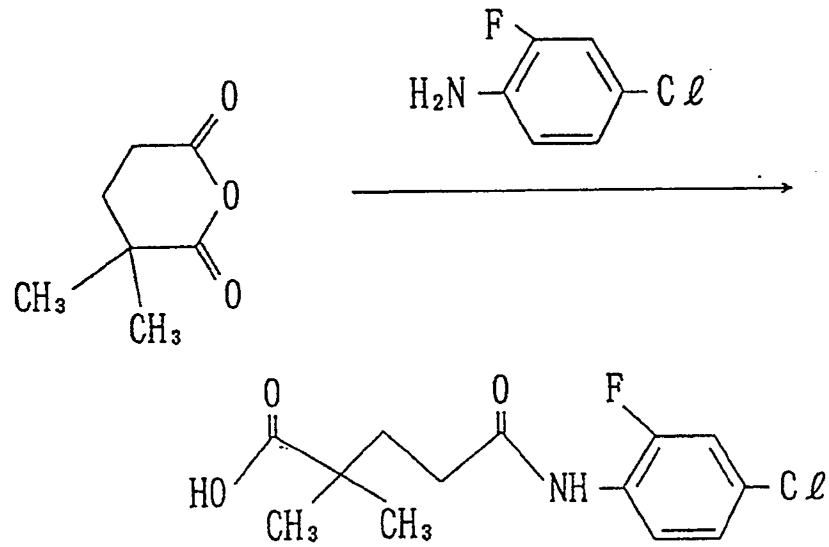

In the same manner as in Example 2-1, from 1 g of 2,2-dimethylglutalic anhydride and 1 g of 4-chloro-2-fluoroaniline, 1.18 g of the title compound was obtained as crystals.

Example 2-4

Synthesis of 3-phenyl-4-(N-(4-chloro-5-ethoxycarbonyl-2-fluorophenyl)carbamoyl) butyric acid

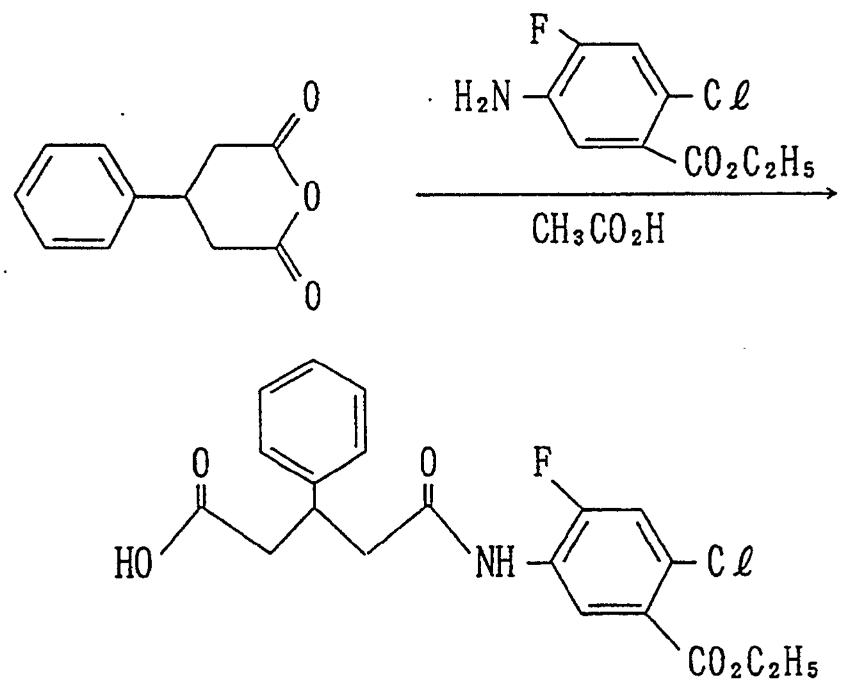


In the same manner as in Example 2-1, from 0.52 g of 3-phenylglutaric anhydride and 0.6 g of ethyl 5-amino-2-chloro-4-fluorobenzoate, 0.83 g of the title compound was obtained as crystals.

Example 2-5

Synthesis of 2-methyl-4-(N-(4-chloro-5-ethoxycarbonyl-2-fluorophenyl)carbamoyl)butyric acid

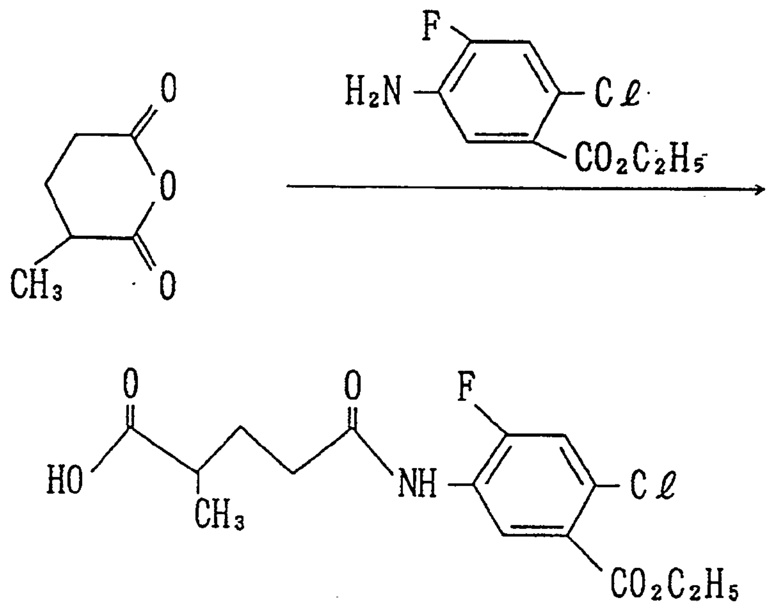

In the same manner as in Example 2-1, from 0.35 g of 2-methylglutaric anhydride and 0.6 g of ethyl 5-amino-2-chloro-4-fluorobenzoate, 0.86 g of the title compound was obtained as an oily product.

## Example 2-6

Synthesis of 2,2,3,3,4,4-hexafluoro-4-(N-(4-chloro-5-ethoxycarbonyl-2-fluorophenyl)carmoyl)butyric acid

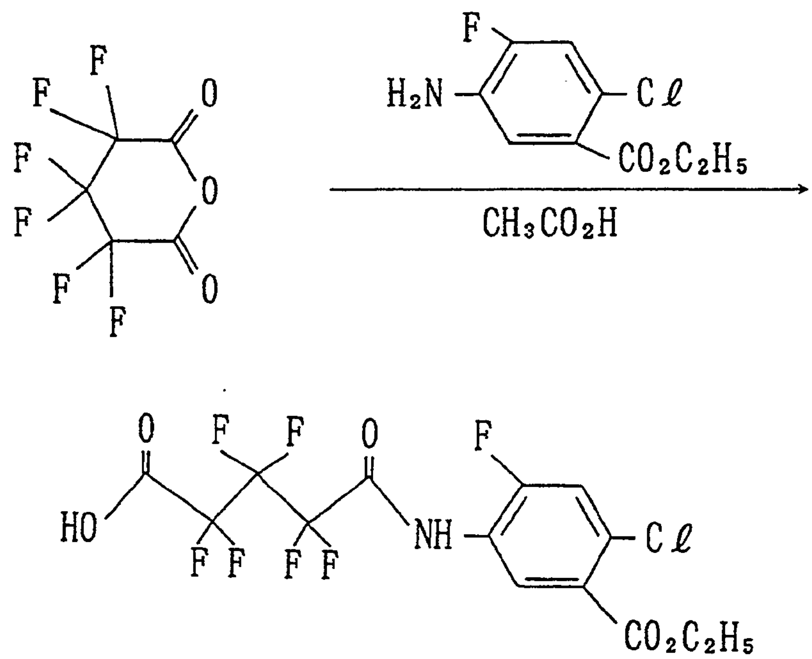


In the same manner as in Example 2-1, from 0.71 g of 2,2,3,3,4,4-hexafluorogutaric anhydride and 0.7 g of ethyl 5-amino-2-chloro-4-fluorobenzoate, 0.2 g of the title compound was obtained as crystals.

## Example 2-7

Synthesis of ethyl 3-methyl-4-(N-(4-chloro-5-ethoxycarbonyl-2-fluorophenyl)carbamoyl)butyrate

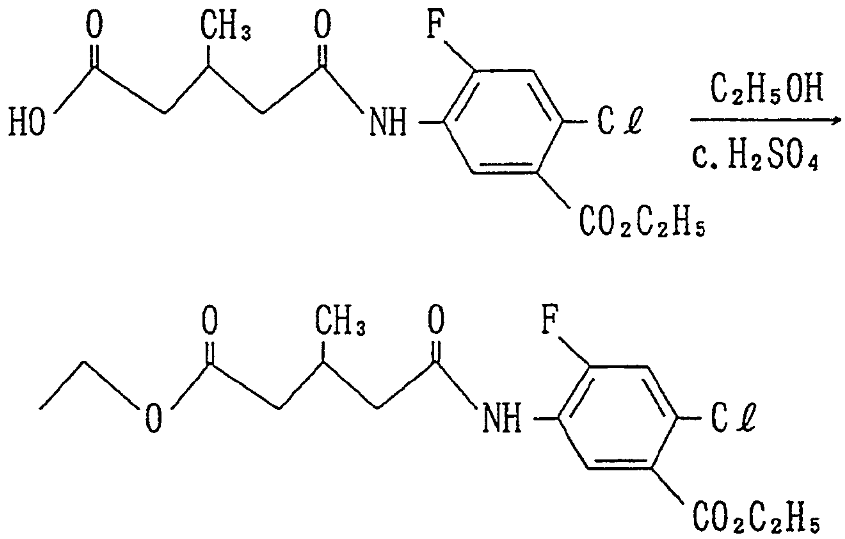


In 10 ml of ethanol was dissolved 0.5 g of 3-methyl-4-(N-(4-chloro-5-ethoxycarbonyl-2-fluorophenyl)carbamoyl)butyric acid, and a catalytic amount of conc. sulfuric acid was added to the solution and the mixture was stirred for 1 hour under reflux. After completion of the reaction, the reaction mixture was cooled to room temperature, ethanol was removed therefrom and the reaction mixture was extracted with ethyl acetate. The extract was washed with water and dried over anhydrous sodium sulfate. Ethyl acetate was removed therefrom to obtain 0.47 g of the title compound as an oily product.

Reference Example 2-1

Synthesis of 3-phenylglutaric anhydride

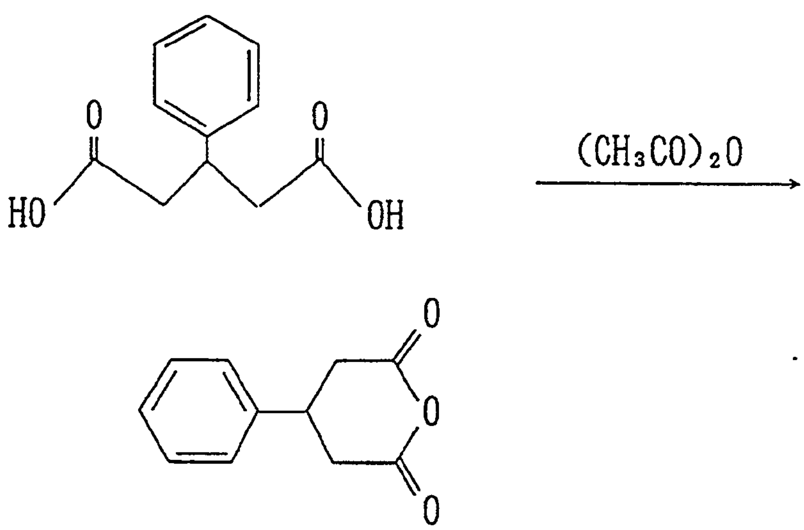


In 10 ml of acetic anhydride were dissolved 3 g of 3-phenylglutaric acid, and the mixture was stirred for 4 hours under reflux. After completion of the reaction, the obtained crude product was washed with diisopropylether to obtain of 2.5 g of the title compound as crystals.

Reference Example 2-2

Synthesis of 3-trifluoromethylglutaric anhydride

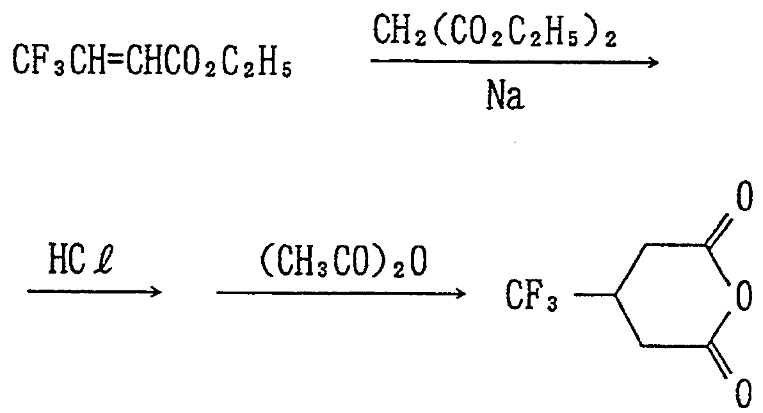


In 15 ml of absolute ethanol was dissolved 0.74 g of sodium, and to the resultant solution was added dropwise a mixture of 5 g of ethyl 4,4,4-trifluorocrotonate and 4.76 g of diethyl malonate at a temperature of 5 to 10°C. After completion of the reaction, the solvent was removed under reduced pressure. 10 ml of acetic anhydride was added thereto and the mixture was stirred for 2 hours under reflux. After completion of the reaction, the produced salts were separated by filtration and washed with acetic acid, and the filtrate was concentrated. The resulting crude product was washed with diisopropylether to obtained 4.11 g of the title compound as white crystals.

Reference Example 2-3

Synthesis of 2-methyl-3-trifluoromethylglutaric anhydride

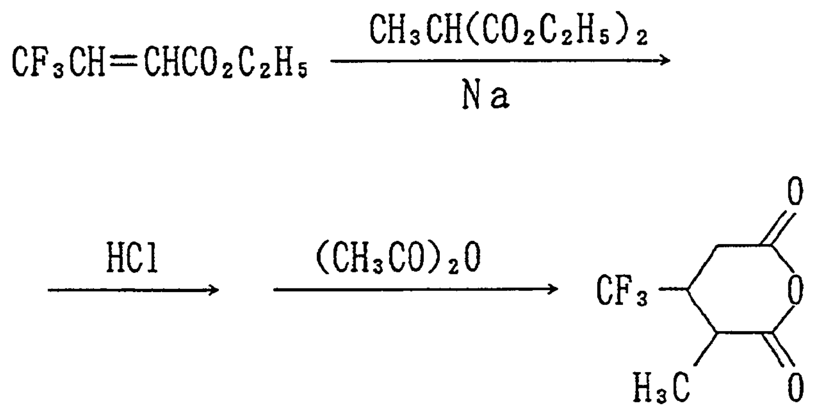


In the same manner as in Reference Example 2-2, from 3 g of ethyl 4,4,4-trifluorocrotonate and 3.11 g of diethyl methylmalonate, 3.1 g of the title compound was obtained as an oily product.

Reference Example 2-4

Synthesis of 2-ethyl-3-trifluoromethylglutaric anhydride

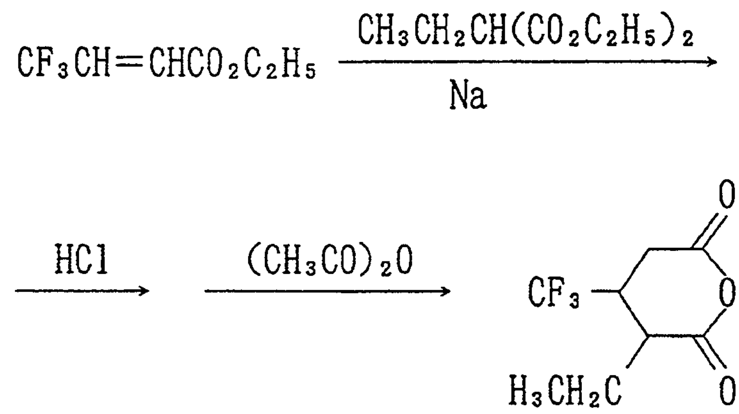


In the same manner as in Reference Example 2-2, from 5 g of ethyl 4,4,4-trifluorocrotonate and 5.6 g of diethyl ethylmalonate, 4.91 g of the title compound was obtained as an oily product.

Reference Example 2-5

Synthesis of 3-isopropyl-2-methylglutaric anhydride

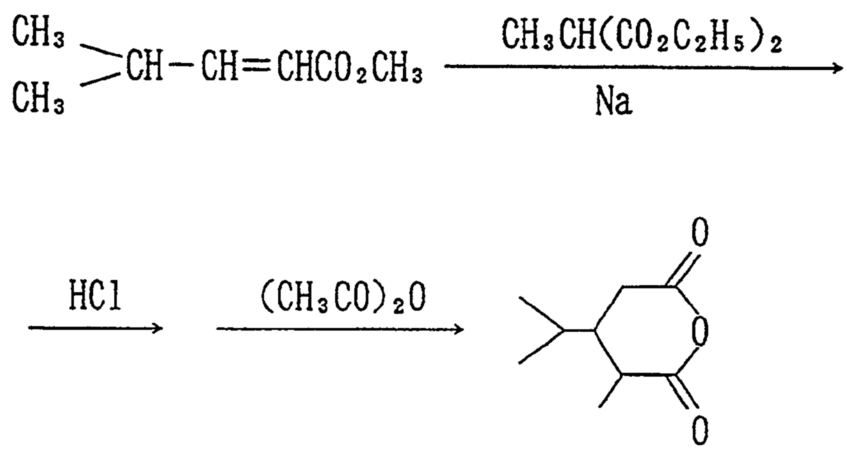


In the same manner as in Reference Example 2-2, from 5 g of methyl 3-methyl-1-pentenate and 6.8 g of diethyl methylmalonate, 1.57 g of the title compound was obtained as an oily product.

Reference Example 2-6

Synthesis of 2-methylglutaric anhydride

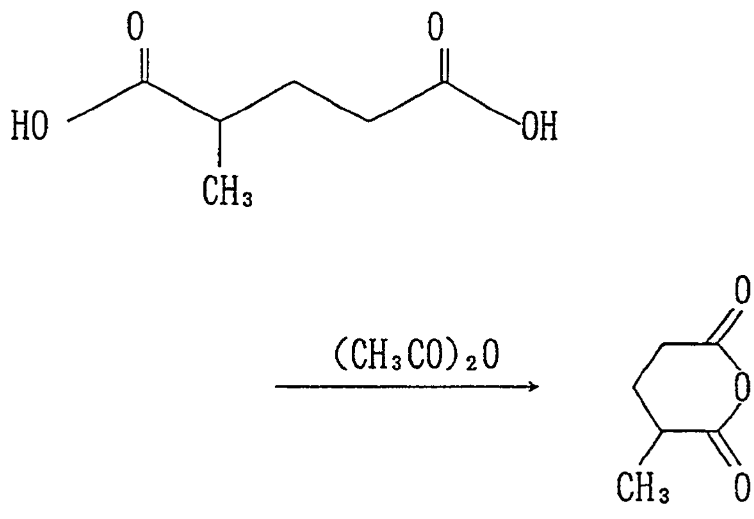


In the same manner as in Reference Example 2-1, from 5 g of 2-methylglutaric acid, 3.47 g of the title compound was obtained as crystals.

Reference Example 2-7

Synthesis of 3-isopropylglutaric anhydride

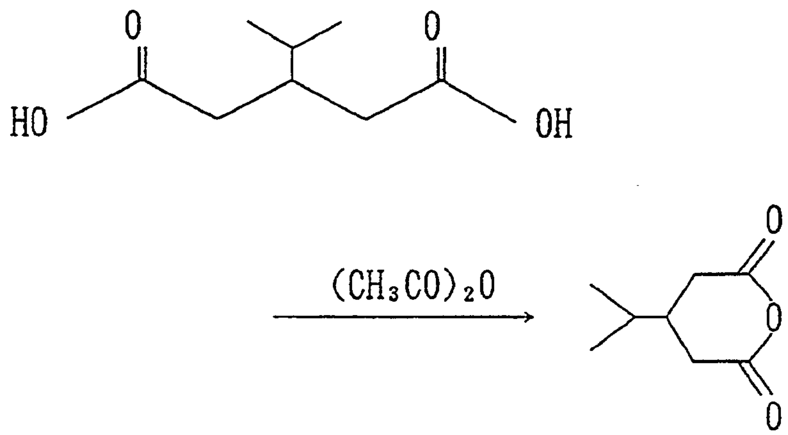


In the same manner as in Reference Example 2-1, from 1.2 g of 3-isopropylglutaric acid, 0.94 g of the title compound was obtained as an oily product.

Reference Example 2-8

Synthesis of 3-n-propylglutaric anhydride

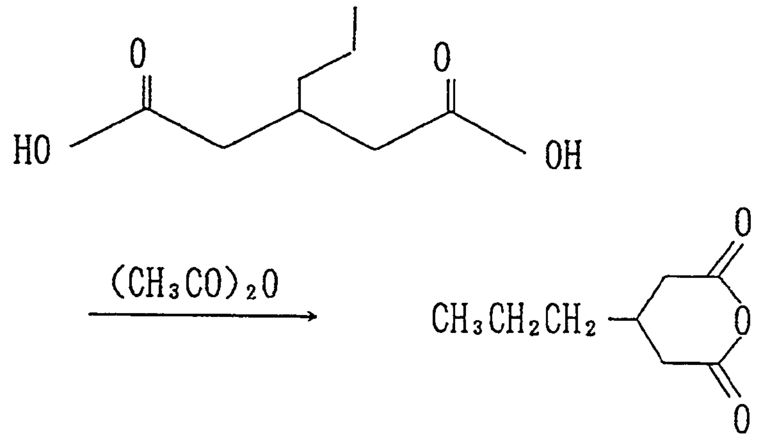


In the same manner as in Reference Example 2-1, from 5 g of 3-n-propylglutaric acid, 3.97 g of the title compound was obtained as an oily product.

Reference Example 2-9

Synthesis of 2,3-dimethylglutaric anhydride

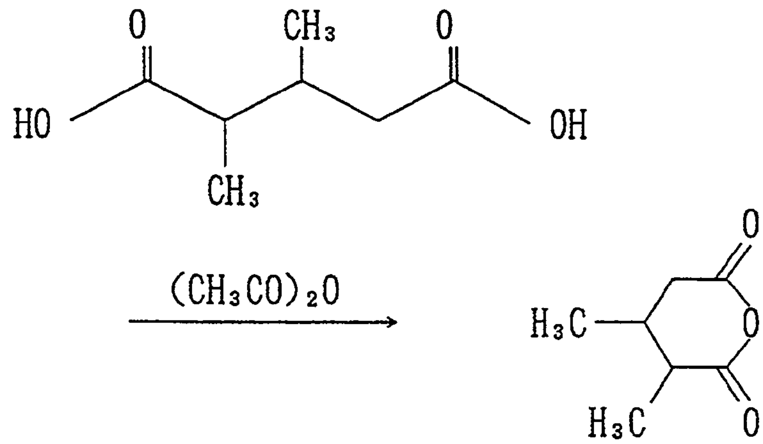


In the same manner as in Reference Example 2-1, from 5 g of 2,3-dimethylglutaric acid, 4.19 g of the title compound was obtained as an oily product.

The compound of the present invention synthesized according to the above Examples and synthesized by following the similar procedures to the above Examples or Schemes are shown in Tables 2-1 and 2-2. The compounds obtainable in accordance with the present invention, however, are not limited to those shown in the following tables.

Table 2-1

| Compound No. | Structure |
|---|---|
| 2-1 | 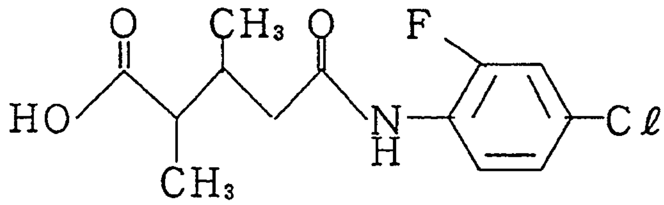 |
| 2-2 | 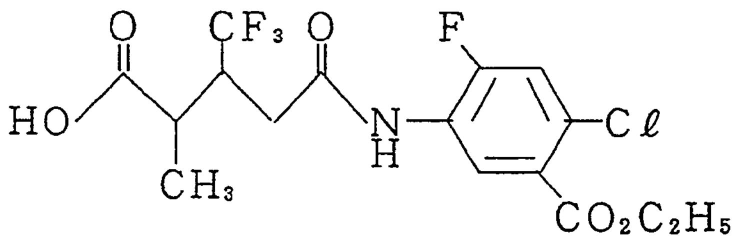 |
| 2-3 | 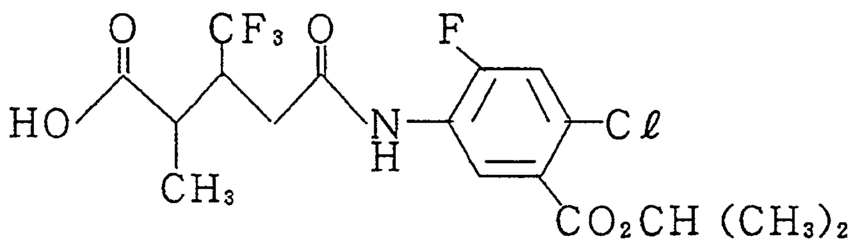 |

Table 2-1 (Cont' d)

| Compound No. | Structure |
|---|---|
| 2-4 | 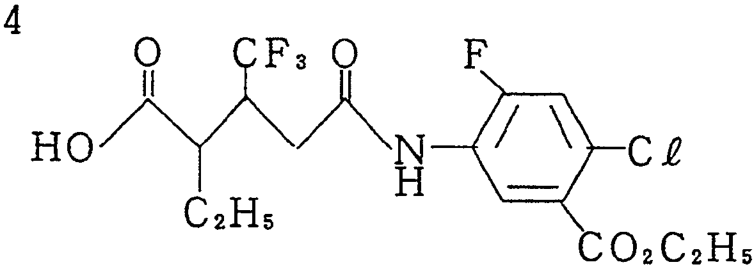 |
| 2-5 | 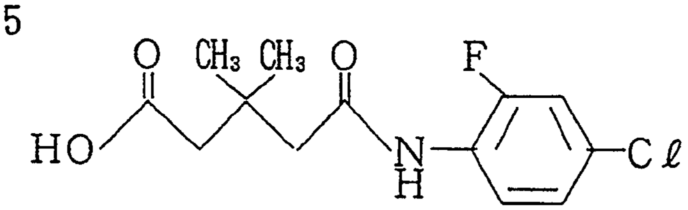 |
| 2-6 | 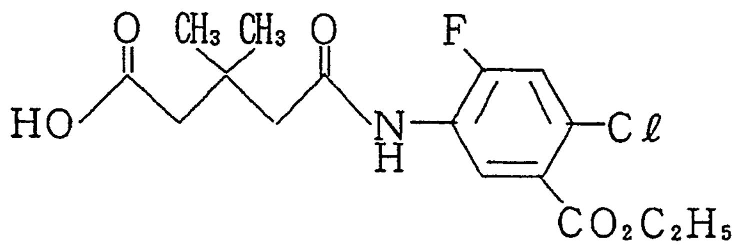 |

Table 2-1 (Cont' d)

| Compound No. | Structure |
|---|---|
| 2-7 | 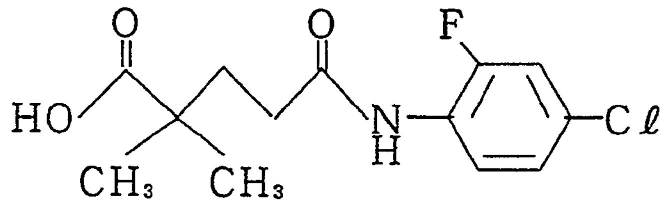 |
| 2-8 | 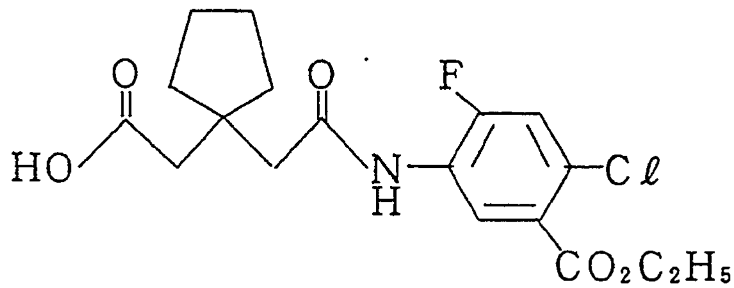 |
| 2-9 | 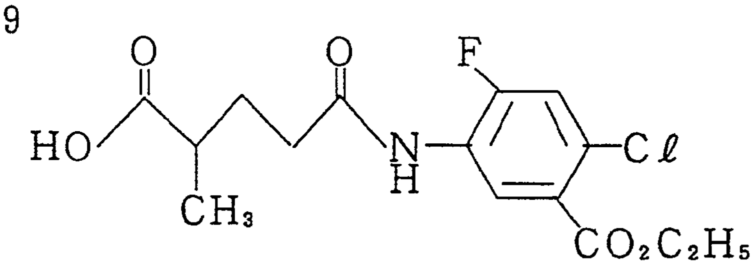 |

Table 2-1 (Cont' d)

| Compound No. | Structure |
| --- | --- |
| 2-10 | 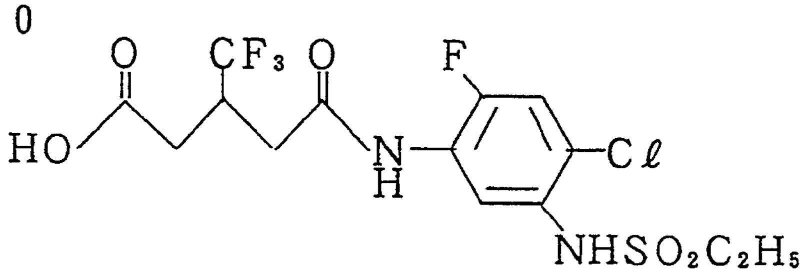 |
| 2-11 | 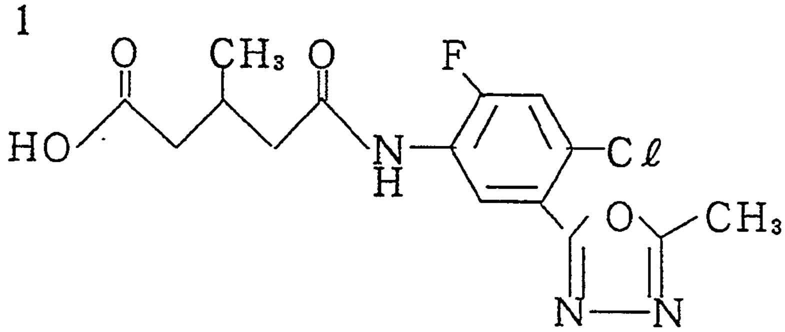 |
| 2-12 | 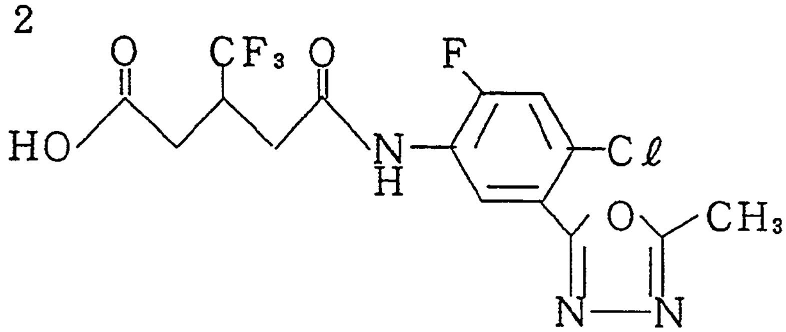 |

Table 2-1 (Cont' d)

| Compound No. | Structure |
|---|---|
| 2-13 | 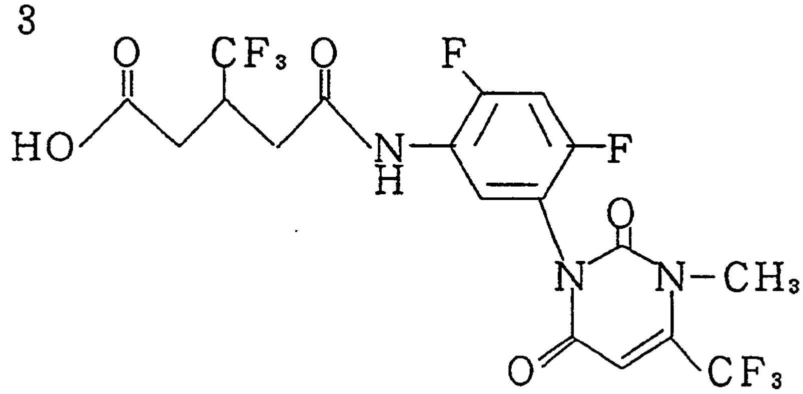 |
| 2-14 | 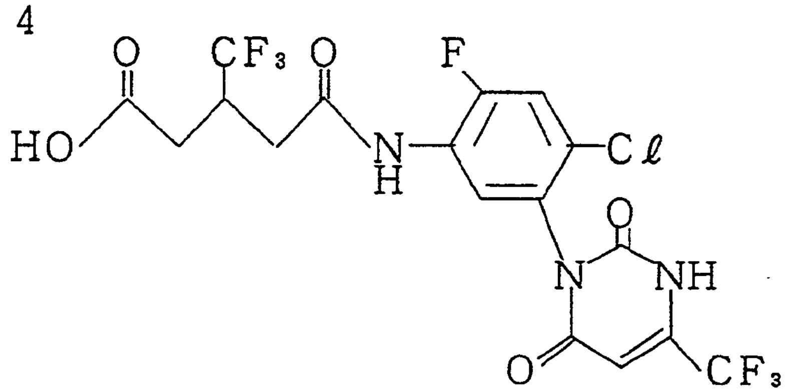 |

Table 2-1 (Cont' d)

| Compound No. | Structure |
| --- | --- |
| 2-15 | 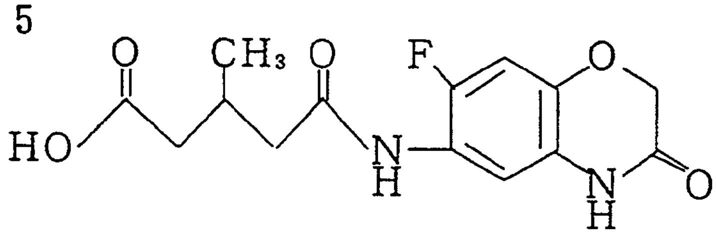 |
| 2-16 | 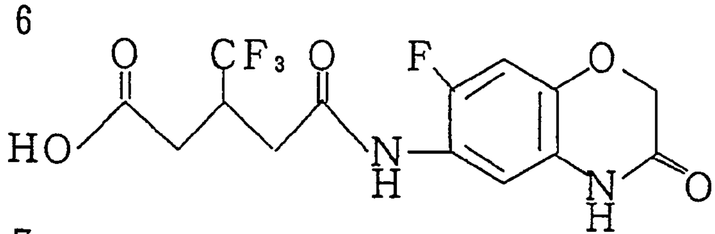 |
| 2-17 | 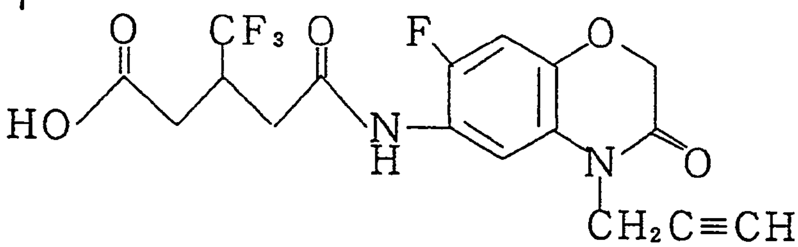 |

Table 2-1 (Cont' d)

| Compound No. | Structure |
| --- | --- |
| 2-18 | 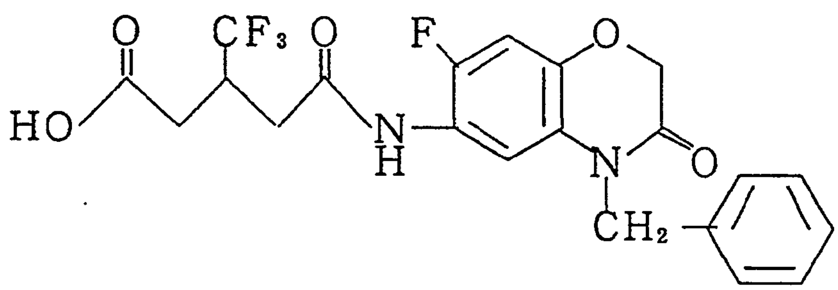 |
| 2-19 | 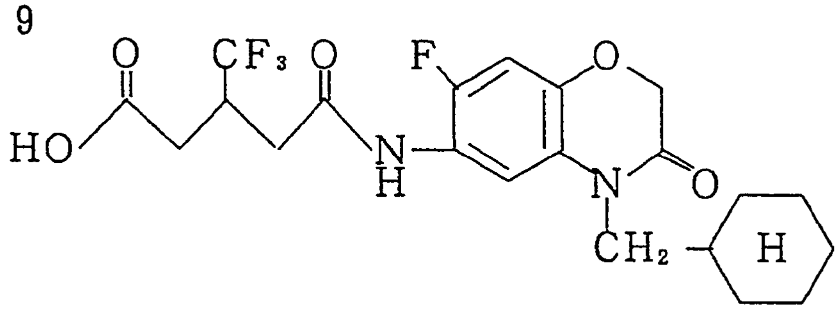 |

Table 2-1 (Cont' d)

| Compound No. | Structure |
|---|---|
| 2-20 | 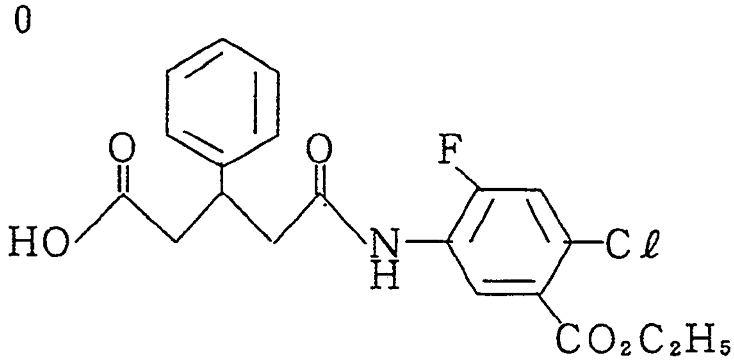 |
| 2-21 | 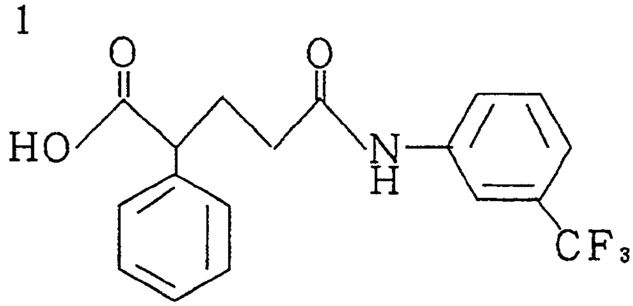 |
| 2-22 | 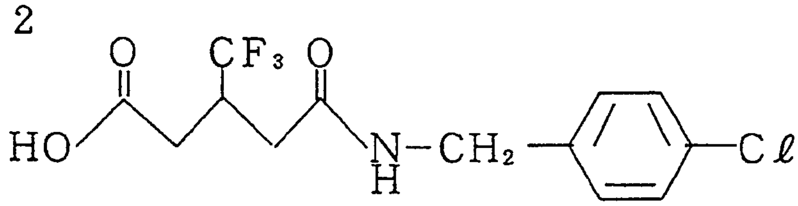 |

Table 2-1 (Cont'd)

| Compound No. | Structure |
|---|---|
| 2-23 | 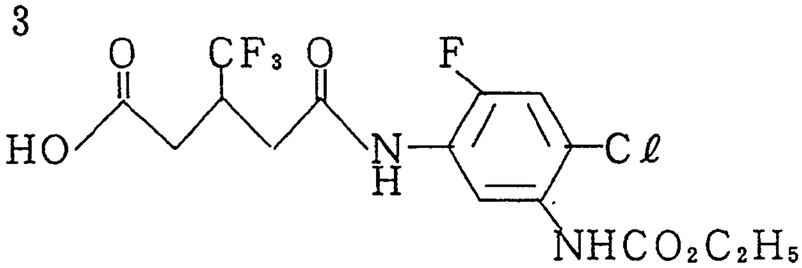 |
| 2-24 | 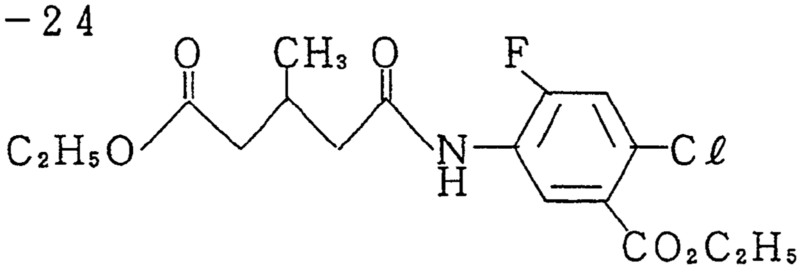 |
| 2-25 | 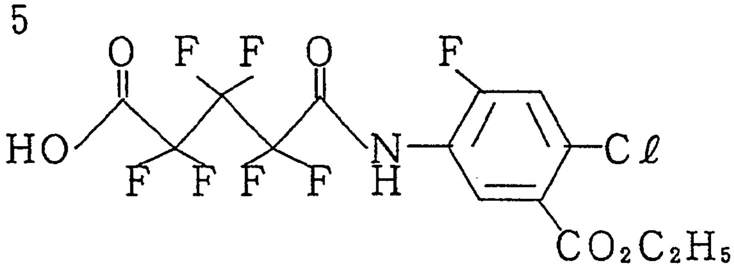 |

Table 2-2

| Compound No. | $^1$H-NMR δ (ppm) [solvent] | Physical Properties |
|---|---|---|
| 2-1 | 0.92~1.47(6H, m), 1.37(3H, t, J=7Hz), 2.15~3.00(4H, m), 4.33(2H, q, J=7Hz), 7.08(1H, d, J=10Hz), 7.91(1H, br s), 8.62(1H, d, J=8Hz), 9.15~10.32(1H, m) [$CDCl_3$] | oil |
| 2-2 | 0.94~1.41(3H, m), 1.36(3H, t, J=7Hz), 2.48~3.79(4H, m), 4.32(2H, q, J=7Hz), 7.04(1H, d, J=10Hz), 8.44(1H, d, J=8Hz), 8.65(1H, br s), 9.98(1H, br s) [$CDCl_3$] | oil |
| 2-3 | 1.10~1.52(9H, m), 2.53~3.45(4H, m), 5.01~5.49(1H, m), 7.05(1H, d, J=10Hz), 7.68(1H, br s), 8.48(1H, d, J=8Hz), 10.13(1H, br s) [$CDCl_3$] | oil |
| 2-4 | 0.67~2.03(5H, m), 1.40(3H, t, J=7Hz), 2.39~3.63(4H, m), 4.40(2H, q, J=7Hz), 7.17(1H, d, J=10Hz), 8.53(1H, br s), 8.61(1H, d, J=8Hz), 10.05(1H, br s) [$CDCl_3$] | oil |
| 2-5 | 1.12(6H, s), 2.40(4H, d, J=8Hz), 6.93~7.33 (2H, m), 7.83~8.21(1H, m), 9.53(1H, br s), 11.10(1H, br s) [$d_6$-DMSO] | mp=132.1~133.2℃ |
| 2-6 | 1.14(6H, s), 1.39(3H, t, J=7Hz), 2.38(2H, s), 2.46(2H, s), 4.36(2H, q, J=7Hz), 6.09~7.12(1H, m), 7.28(1H, d, J=10Hz), 8.69(1H, d, J=8Hz), 9.80(1H, br s) [$d_6$-DMSO] | mp=110.8~111.8℃ |
| 2-7 | 1.14(6H, s), 1.60~2.05(2H, m), 2.15~2.65 (2H, m), 7.00~7.43(2H, m), 7.81~8.19(1H, m), 9.71(1H, br s), 11.50(1H, br s) [$d_6$-DMSO] | mp=111.7~113.8℃ |
| 2-8 | 1.37(3H, t, J=7Hz), 2.68(8H, br s), 2.52(2H, br s), 2.58(2H, br s), 4.33(2H, q, 7Hz), 7.08(1H, d, J=10Hz), 8.63(1H, d, J=8Hz), 8.65(1H, br s), 10.30(1H, br s) [$CDCl_3$] | mp=117~119 ℃ |
| 2-9 | 0.99~1.54(3H, m), 1.34(3H, t, J=7Hz), 1.68~2.86(5H, m), 4.29(2H, q, J=7Hz), 6.99(1H, d, J=10Hz), 8.34(1H, br s), 8.43(1H, d, J=8Hz), 10.58(1H, br s) [$CDCl_3$] | oil |

Table 2-2 (Cont' d)

| Compound No. | $^{1}$H-NMR δ (ppm) [solvent] | Physical Properties |
|---|---|---|
| 2-10 | 1.33(3H, t, J=7Hz), 2.33~2.86(5H, m), 3.09(2H, q, J=7Hz), 7.37(1H, d, J=10Hz), 8.21(1H, d, J=8Hz), 9.25(1H, br s) 10.01(1H, s) [$d_6$-DMSO] | mp=138~139 ℃ |
| 2-11 | 0.92~1.35(3H, m), 2.23~2.68(5H, m), 2.60(3H, s), 7.28(1H, d, J=10Hz), 8.15(1H, br s), 8.69(1H, d, J=8Hz), 9.21(1H, br s) [$CDCl_3$] | oil |
| 2-12 | 2.39~2.92(5H, m), 2.59(3H, s), 7.25(1H, d, J=10Hz), 8.59(1H, br s), 8.64(1H, d, J=8Hz), 9.73(1H, br s) [$d_6$-DMSO] | oil |
| 2-13 | 2.30~2.87(5H, m), 3.42(3H, br s), 6.36(1H, s), 6.95 ~7.36(1H, m), 7.68~8.12(2H, m), 9.77(1H, br s) [$d_6$-DMSO] | mp=195~196 ℃ |
| 2-14 | 2.33~3.28(5H, m), 6.09(1H, s), 7.35(1H, d, J=10Hz), 8.02(1H, d, J=8Hz), 9.90(1H, br s) [$d_6$-DMSO] | mp=205~207 ℃ |
| 2-15 | 0.99(3H, br d), 1.84~2.86(5H, m), 4.39(2H, s), 6.58(1H, d, J=10Hz), 7.37(1H, d, J=8Hz), 9.10(1H, br s), 10.30(1H, br s), 11.69(1H, br s) [$d_6$-DMSO] | mp=222~226 ℃ |
| 2-16 | 2.31~2.83(4H, m), 2.93~3.61(1H, m), 4.43(2H, s), 6.63(1H, d, 11Hz), 7.47(1H, d, J=8Hz), 9.47(1H, br s), 10.40(1H, br s) [$d_6$-DMSO] | mp=226~229 ℃ |
| 2-17 | 2.26~3.76(5H, m), 2.96(1H, t, J=2Hz), 4.57(4H, br s), 6.78(1H, d, J=11Hz), 7.67(1H, d, J=8Hz), 9.64(1H, br s) [$d_6$-DMSO] | mp=170~174 ℃ |
| 2-18 | 2.21~2.78(4H, m), 2.80~3.71(1H, m), 4.61(2H, s), 5.00(2H, br s), 6.69(1H, d, J=10Hz), 7.16(5H, br s), 7.53(1H, d, J=8Hz), 9.45(1H, br s) [$d_6$-DMSO] | mp=216~221 ℃ |

Table 2-2 (Cont'd)

| Compound No. | $^1$H-NMR δ (ppm) [solvent] | Physical Properties |
|---|---|---|
| 2-19 | 0.74~2.05(10H, m), 2.36 ~2.87(5H, m), 3.68(2H, d, J=6Hz), 4.49(2H, s) 6.70(1H, d, J=10Hz), 7.54(1H, d, J=7Hz), 7.57(1H, br s), 9.52(1H, br s) [$d_6$-DMSO] | mp=192~199 ℃ |
| 2-20 | 1.34(3H, t, J=7Hz), 2.51~2.92(5H, m), 4.30(2H, q, J=7Hz), 6.99(1H, d, J=10Hz), 7.13(5H, s), 7.53(1H, br s), 8.49(1H, d, J=8Hz), 11.01(1H, br s) [$CDCl_3$] | mp=117~120 ℃ |
| 2-21 | 1.99~2.76(4H, m), 3.44~3.91(1H, m), 7.11~7.99(9H, m), 8.51~9.12(1H, m) [$CDCl_3$] | vitrified |
| 2-22 | 2.31~2.63(5H, m), 4.29(2H, d, J=6Hz), 7.27(4H, s), 8.46(1H, br s), 9.87(1H, br s) [$d_6$-DMSO] | mp=171~175 ℃ |
| 2-23 | 1.32(3H, t, J=7Hz), 2.40~2.94(4H, m), 3.02~3.61(1H, m), 4.23(2H, q, J=7Hz), 7.19(1H, d, J=10Hz), 7.72(1H, br s), 8.64(1H, d, J=8Hz), 9.64(1H, br s) [$d_6$-DMSO] | mp=159~161 ℃ |
| 2-24 | 0.82~1.42(3H, m), 1.23(3H, t, J=7Hz), 1.37(3H, t, J=7Hz), 2.04~2.91(5H, m), 4.16(2H, q, J=7Hz), 4.39(2H, q, J=7Hz), 7.04(1H, d, J=10Hz), 8.43(1H, br s), 8.55(1H, d, J=9Hz) [$CDCl_3$] | oil |
| 2-25 | 1.38(3H, t, J=7Hz), 4.34(2H, q, J=7Hz), 7.15(2H, d, J=10Hz), 7.65(1H, br s), 8.71(1H, d, J=9Hz), 10.95(1H, br s) [$CDCl_3$] | mp=83~86℃ |

The compound of the present invention synthesized according to the above Examples and synthesized by following the similar procedures to the above Examples or Schemes are shown in Tables 2-3 to 2-9 and Tables 2-3-1 to 2-9-1. The compounds obtainable in accordance with the present invention, however, are not limited to those shown in the following tables.

Table 2-3

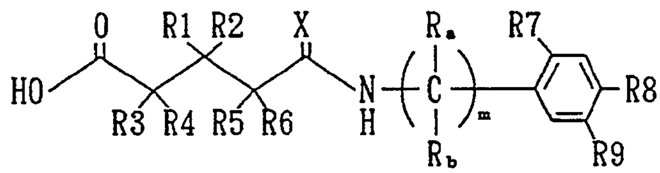

| R1 | R2 | R3 | R4 | R5 | R6 | X | $R_a$ | $R_b$ | m | R7 | R8 | R9 |
|---|---|---|---|---|---|---|---|---|---|---|---|---|
| Me | Me | H | H | H | H | O | – | – | 0 | F | Cl | H |
| Me | Me | H | H | H | H | S | – | – | 0 | F | Cl | H |
| Me | Me | H | H | H | H | O | – | – | 0 | F | Cl | $CO_2Et$ |
| Me | Me | H | H | H | H | S | – | – | 0 | F | Cl | $CO_2Et$ |
| Me | Me | H | H | H | H | O | H | H | 1 | H | Cl | H |
| H | H | Me | Me | H | H | O | – | – | 0 | F | Cl | H |
| H | H | Me | Me | H | H | S | – | – | 0 | F | Cl | H |
| H | H | Me | Me | H | H | O | – | – | 0 | F | Cl | $CO_2Et$ |
| H | H | Me | Me | H | H | S | – | – | 0 | F | Cl | $CO_2Et$ |
| H | H | Me | Me | H | H | O | H | H | 1 | H | Cl | H |
| H | H | H | H | Me | Me | S | – | – | 0 | F | Cl | H |
| H | H | H | H | Me | Me | S | – | – | 0 | F | Cl | $CO_2Et$ |
| H | H | Me | H | H | H | O | – | – | 0 | F | Cl | H |
| H | H | Me | H | H | H | O | – | – | 0 | F | Cl | $CO_2Et$ |
| H | H | Me | H | H | H | S | – | – | 0 | F | Cl | H |
| H | H | H | H | Me | H | S | – | – | 0 | F | Cl | H |
| Ph | H | Me | H | H | H | O | – | – | 0 | F | Cl | H |
| Ph | H | Me | H | H | H | O | – | – | 0 | F | Cl | $CO_2Et$ |
| Me | H | Ph | H | H | H | O | – | – | 0 | F | Cl | H |

Table 2-3 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Ph | H | H | H | O | – | – | 0 | F | Cl | $CO_2Et$ |
| Me | H | H | H | Ph | H | S | – | – | 0 | F | Cl | H |
| Me | H | H | H | Ph | H | S | – | – | 0 | F | Cl | $CO_2Et$ |
| H | H | H | H | H | H | S | – | – | 0 | F | Cl | H |
| H | H | H | H | H | H | S | – | – | 0 | F | Cl | $CO_2Et$ |
| H | H | H | H | H | H | O | H | H | 1 | H | Cl | H |
| Me | H | Me | H | H | H | O | – | – | 0 | F | Cl | H |
| Me | H | Me | H | H | H | O | – | – | 0 | F | Cl | $CO_2Et$ |
| Me | H | Me | H | H | H | S | – | – | 0 | F | Cl | H |
| Me | H | H | H | Me | H | S | – | – | 0 | F | Cl | H |
| Me | H | H | H | Me | H | S | – | – | 0 | F | Cl | $CO_2Et$ |
| H | H | Me | H | Me | H | O | – | – | 0 | F | Cl | H |
| H | H | Me | H | Me | H | O | – | – | 0 | F | Cl | $CO_2Et$ |
| H | H | Me | H | Me | H | S | – | – | 0 | F | Cl | H |
| F | F | F | F | F | F | O | – | – | 0 | F | Cl | H |
| F | F | F | F | F | F | O | – | – | 0 | F | Cl | $CO_2Et$ |
| F | F | F | F | F | F | O | H | H | 1 | H | Cl | H |
| F | F | F | F | F | F | O | H | H | 1 | H | $NO_2$ | H |
| F | F | F | F | F | F | O | H | H | 1 | H | $NO_2$ | $CO_2H$ |
| $CF_3$ | H | Me | H | H | H | O | – | – | 0 | F | Cl | H |
| $CF_3$ | H | Me | H | H | H | O | – | – | 0 | F | Cl | $CO_2Me$ |
| $CF_3$ | H | Me | H | H | H | O | – | – | 0 | F | Cl | $CO_2Et$ |
| $CF_3$ | H | Me | H | H | H | S | – | – | 0 | F | Cl | $CO_2Et$ |
| $CF_3$ | H | H | H | Me | H | S | – | – | 0 | F | Cl | $CO_2Et$ |

Table 2-3-1

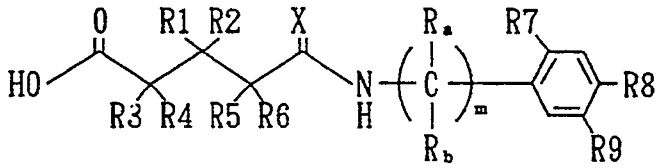

または

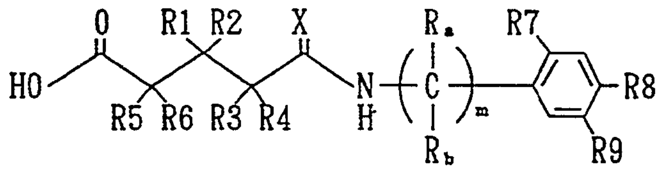

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | H | H | O | - | - | 0 | F | Cl | $CO_2Pr^i$ |
| $CF_3$ | H | Me | H | H | H | O | - | - | 0 | F | Cl | $CO_2Pr^n$ |
| $CF_3$ | H | Me | H | H | H | O | - | - | 0 | F | Cl | $CO_2Bu^n$ |
| $CF_3$ | H | Me | H | H | H | O | - | - | 0 | F | Cl | $CO_2Bu^t$ |
| $CF_3$ | H | Me | H | H | H | O | - | - | 0 | F | Cl | $NO_2$ |
| $CF_3$ | H | Me | H | H | H | O | - | - | 0 | F | Cl | $NH_2$ |
| $CF_3$ | H | Me | H | H | H | O | - | - | 0 | F | Cl | $NHSO_2Me$ |
| $CF_3$ | H | Me | H | H | H | O | - | - | 0 | F | Cl | $NHSO_2Et$ |
| $CF_3$ | H | Me | H | H | H | O | - | - | 0 | F | Cl | $NHSO_2Pr^n$ |
| $CF_3$ | H | Me | H | H | H | O | - | - | 0 | F | Cl | $NHSO_2Pr^i$ |
| $CF_3$ | H | Me | H | H | H | O | - | - | 0 | F | Cl | OH |
| $CF_3$ | H | Me | H | H | H | O | - | - | 0 | F | Cl | OMe |
| $CF_3$ | H | Me | H | H | H | O | - | - | 0 | F | Cl | OEt |
| $CF_3$ | H | Me | H | H | H | O | - | - | 0 | F | Cl | $OPr^i$ |
| $CF_3$ | H | Me | H | H | H | O | - | - | 0 | F | Cl | $OPr^n$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | H | H | O | - | - | 0 | F | Cl | OPen c |
| $CF_3$ | H | Me | H | H | H | O | - | - | 0 | F | Cl | $OCH_2CH=CH$ |
| $CF_3$ | H | Me | H | H | H | O | - | - | 0 | F | Cl | $OCH_2C\equiv CH$ |
| $CF_3$ | H | Me | H | H | H | O | - | - | 0 | F | Cl | $OCH_2CO_2Me$ |
| $CF_3$ | H | Me | H | H | H | O | - | - | 0 | F | Cl | $OCH_2CO_2Et$ |
| $CF_3$ | H | Me | H | H | H | O | - | - | 0 | F | Cl | $OCH(Me)CO_2Me$ |
| $CF_3$ | H | Me | H | H | H | O | - | - | 0 | F | Cl | $OCH(Me)CO_2Et$ |
| $CF_3$ | H | Me | H | H | H | O | - | - | 0 | F | Cl | $CO_2H$ |
| $CF_3$ | H | Me | H | H | H | O | - | - | 0 | F | Cl | CN |
| $CF_3$ | H | Me | H | H | H | O | - | - | 0 | F | Cl | Me |
| $CF_3$ | H | Me | H | H | H | O | - | - | 0 | F | Cl | Et |
| $CF_3$ | H | Me | H | H | H | O | - | - | 0 | F | Cl | Cl |
| $CF_3$ | H | Me | H | H | H | O | - | - | 0 | F | Cl | Br |
| $CF_3$ | H | Me | H | H | H | O | - | - | 0 | F | Cl | I |
| $CF_3$ | H | Me | H | H | H | O | - | - | 0 | F | Cl | F |
| $CF_3$ | H | Me | H | H | H | O | - | - | 0 | F | Cl | SH |
| $CF_3$ | H | Me | H | H | H | O | - | - | 0 | F | Cl | $C(Me)=NOCH_2CO_2Me$ |
| $CF_3$ | H | Me | H | H | H | O | - | - | 0 | F | Cl | $C(Me)=NOCH_2CO_2Et$ |
| $CF_3$ | H | Me | H | H | H | O | - | - | 0 | F | Cl | $CH=NOCH_2CO_2Me$ |
| $CF_3$ | H | Me | H | H | H | O | - | - | 0 | F | Cl | $CH=NOCH_2CO_2Et$ |
| $CF_3$ | H | Me | H | H | H | O | - | - | 0 | F | Cl | $C(OMe)=NOCH_2CO_2Me$ |
| $CF_3$ | H | Me | H | H | H | O | - | - | 0 | F | Cl | $C(OMe)=NOCH_2CO_2Et$ |
| $CF_3$ | H | Me | H | H | H | O | - | - | 0 | F | Cl | $C(Me)=NN=C(Me)CO_2Me$ |
| $CF_3$ | H | Me | H | H | H | O | - | - | 0 | F | Cl | $C(Me)NN=C(Me)CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | H | H | O | - | - | 0 | F | Cl | $CH_2OCH_2CO_2Me$ |
| $CF_3$ | H | Me | H | H | H | O | - | - | 0 | F | Cl | $CH_2OCH_2CO_2Et$ |
| $CF_3$ | H | Me | H | H | H | O | - | - | 0 | F | Cl | $CH_2OCH(Me)CO_2Me$ |
| $CF_3$ | H | Me | H | H | H | O | - | - | 0 | F | Cl | $CH_2OCH(Me)CO_2Et$ |
| $CF_3$ | H | Me | H | H | H | O | - | - | 0 | F | Cl | $CH_2SCH_2CO_2Me$ |
| $CF_3$ | H | Me | H | H | H | O | - | - | 0 | F | Cl | $CH_2SCH_2CO_2Et$ |
| $CF_3$ | H | Me | H | H | H | O | - | - | 0 | F | Cl | $CH_2SCH(Me)CO_2Me$ |
| $CF_3$ | H | Me | H | H | H | O | - | - | 0 | F | Cl | $CH_2SCH(Me)CO_2Et$ |
| $CF_3$ | H | Me | H | H | H | O | - | - | 0 | F | Cl | SMe |
| $CF_3$ | H | Me | H | H | H | O | - | - | 0 | F | Cl | SEt |
| $CF_3$ | H | Me | H | H | H | O | - | - | 0 | F | Cl | $SPr^i$ |
| $CF_3$ | H | Me | H | H | H | O | - | - | 0 | F | Cl | $SPr^n$ |
| $CF_3$ | H | Me | H | H | H | O | - | - | 0 | F | Cl | $SPen^c$ |
| $CF_3$ | H | Me | H | H | H | O | - | - | 0 | F | Cl | $SCH_2CO_2H$ |
| $CF_3$ | H | Me | H | H | H | O | - | - | 0 | F | Cl | $SCH_2CO_2Me$ |
| $CF_3$ | H | Me | H | H | H | O | - | - | 0 | F | Cl | $SCH_2CO_2Et$ |
| $CF_3$ | H | Me | H | H | H | O | - | - | 0 | F | Cl | $SCH(Me)CO_2Me$ |
| $CF_3$ | H | Me | H | H | H | O | - | - | 0 | F | Cl | $SCH(Me)CO_2Et$ |
| $CF_3$ | H | Me | H | H | H | O | - | - | 0 | F | Cl | $SCH_2CN$ |
| $CF_3$ | H | Me | H | H | H | O | - | - | 0 | F | Cl | $SCH_2CH=CH_2$ |
| $CF_3$ | H | Me | H | H | H | O | - | - | 0 | F | Cl | $SCH_2C\equiv CH$ |
| $CF_3$ | H | Me | H | H | H | O | - | - | 0 | F | Cl | $OCH(Me)C\equiv CH$ |
| $CF_3$ | H | Me | H | H | H | O | - | - | 0 | F | Cl | $SCH(Me)C\equiv CH$ |
| $CF_3$ | H | Me | H | H | H | O | - | - | 0 | F | Cl | $N(SO_2Me)_2$ |

Table 2-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | H | H | 0 | - | - | 0 | F | Cl | $N(SO_2Et)_2$ |
| $CF_3$ | H | Me | H | H | H | 0 | - | - | 0 | F | Cl | Q1(R29=$Bu^t$ ) |
| $CF_3$ | H | Me | H | H | H | 0 | - | - | 0 | F | Cl | Q2(R29=$Bu^t$ ) |
| $CF_3$ | H | Me | H | H | H | 0 | - | - | 0 | F | Cl | Q5(R29=$Bu^t$ ) |
| $CF_3$ | H | Me | H | H | H | 0 | - | - | 0 | F | Cl | Q7(R34=$Bu^t$ ) |
| $CF_3$ | H | Me | H | H | H | 0 | - | - | 0 | F | Cl | Q9(R38=R39=H) |
| $CF_3$ | H | Me | H | H | H | 0 | - | - | 0 | F | Cl | Q10(R38=R39=H) |
| $CF_3$ | H | Me | H | H | H | 0 | - | - | 0 | F | Cl | Q4(R29=$Bu^t$ ) |
| $CF_3$ | H | Me | H | H | H | 0 | - | - | 0 | F | Cl | Q8(R35=R36=R37=H) |
| $CF_3$ | H | Me | H | H | H | 0 | - | - | 0 | F | Cl | Q18(R50=H) |
| $CF_3$ | H | Me | H | H | H | 0 | - | - | 0 | F | Cl | Q18(R50=Me) |
| $CF_3$ | H | Me | H | H | H | 0 | - | - | 0 | F | Cl | $NHCO_2Me$ |
| $CF_3$ | H | Me | H | H | H | 0 | - | - | 0 | F | Cl | $NHCO_2Et$ |
| $CF_3$ | H | Me | H | H | H | 0 | - | - | 0 | F | Cl | $NHCO_2Pr^n$ |
| $CF_3$ | H | Me | H | H | H | 0 | - | - | 0 | F | Cl | $NHCO_2Pr^i$ |
| $CF_3$ | H | Me | H | H | H | 0 | - | - | 0 | F | Cl | $NHCO_2Ph$ |
| $CF_3$ | H | Me | H | H | H | 0 | - | - | 0 | F | Cl | $NHCO_2Bz$ |
| $CF_3$ | H | Me | H | H | H | 0 | - | - | 0 | F | Cl | NHCOMe |
| $CF_3$ | H | Me | H | H | H | 0 | - | - | 0 | F | Cl | $NHCO_2CH_2Ph$ |
| $CF_3$ | H | Me | H | H | H | 0 | - | - | 0 | F | Cl | $NHCO_2CH_2$(4-Me-Ph) |
| $CF_3$ | H | Me | H | H | H | 0 | - | - | 0 | F | Cl | $NHCO_2CH_2$(3-Me-Ph) |
| $CF_3$ | H | Me | H | H | H | 0 | - | - | 0 | F | Cl | $NHCO_2CH_2$(2-Me-Ph) |
| $CF_3$ | H | Me | H | H | H | 0 | - | - | 0 | F | Cl | $NHCO_2CH_2$(4-CL-Ph) |
| $CF_3$ | H | Me | H | H | H | 0 | - | - | 0 | F | Cl | $NHCO_2CH_2$(3-CL-Ph) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | H | H | O | - | - | 0 | F | Cl | $NHCO_2CH_2$(2-CL-Ph) |
| $CF_3$ | H | Me | H | H | H | O | - | - | 0 | F | Cl | Q1(R29=Me) |
| $CF_3$ | H | Me | H | H | H | O | - | - | 0 | F | Cl | Q2(R29=Me) |
| H | H | Ph | H | H | H | O | - | - | 0 | H | H | $CF_3$ |
| Ph | H | Me | H | H | H | O | - | - | 0 | F | Cl | $CO_2Pr^i$ |
| Ph | H | Me | H | H | H | O | - | - | 0 | F | Cl | $CO_2Et$ |
| Ph | H | Me | H | H | H | O | - | - | 0 | F | Cl | H |
| Me | H | Me | H | H | H | O | - | - | 0 | F | Cl | $CO_2Pr^i$ |
| Me | H | Me | H | H | H | O | - | - | 0 | F | Cl | $CO_2Pr^n$ |
| Me | H | Me | H | H | H | O | - | - | 0 | F | Cl | $CO_2Bu^n$ |
| Me | H | Me | H | H | H | O | - | - | 0 | F | Cl | $CO_2Bu^t$ |
| Me | H | Me | H | H | H | O | - | - | 0 | F | Cl | $NO_2$ |
| Me | H | Me | H | H | H | O | - | - | 0 | F | Cl | $NH_2$ |
| Me | H | Me | H | H | H | O | - | - | 0 | F | Cl | $NHSO_2Me$ |
| Me | H | Me | H | H | H | O | - | - | 0 | F | Cl | $NHSO_2Et$ |
| Me | H | Me | H | H | H | O | - | - | 0 | F | Cl | $NHSO_2Pr^n$ |
| Me | H | Me | H | H | H | O | - | - | 0 | F | Cl | $NHSO_2Pr^i$ |
| Me | H | Me | H | H | H | O | - | - | 0 | F | Cl | OH |
| Me | H | Me | H | H | H | O | - | - | 0 | F | Cl | OMe |
| Me | H | Me | H | H | H | O | - | - | 0 | F | Cl | OEt |
| Me | H | Me | H | H | H | O | - | - | 0 | F | Cl | $OPr^i$ |
| Me | H | Me | H | H | H | O | - | - | 0 | F | Cl | $OPr^n$ |
| Me | H | Me | H | H | H | O | - | - | 0 | F | Cl | $OPen^c$ |
| Me | H | Me | H | H | H | O | - | - | 0 | F | Cl | $OCH_2CH=CH_2$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | H | H | O | - | - | 0 | F | Cl | $OCH_2C{\equiv}CH$ |
| Me | H | Me | H | H | H | O | - | - | 0 | F | Cl | $OCH_2CO_2Me$ |
| Me | H | Me | H | H | H | O | - | - | 0 | F | Cl | $OCH_2CO_2Et$ |
| Me | H | Me | H | H | H | O | - | - | 0 | F | Cl | $OCH(Me)CO_2Me$ |
| Me | H | Me | H | H | H | O | - | - | 0 | F | Cl | $OCH(Me)CO_2Et$ |
| Me | H | Me | H | H | H | O | - | - | 0 | F | Cl | $CO_2H$ |
| Me | H | Me | H | H | H | O | - | - | 0 | F | Cl | CN |
| Me | H | Me | H | H | H | O | - | - | 0 | F | Cl | Me |
| Me | H | Me | H | H | H | O | - | - | 0 | F | Cl | Et |
| Me | H | Me | H | H | H | O | - | - | 0 | F | Cl | Cl |
| Me | H | Me | H | H | H | O | - | - | 0 | F | Cl | Br |
| Me | H | Me | H | H | H | O | - | - | 0 | F | Cl | I |
| Me | H | Me | H | H | H | O | - | - | 0 | F | Cl | F |
| Me | H | Me | H | H | H | O | - | - | 0 | F | Cl | SH |
| Me | H | Me | H | H | H | O | - | - | 0 | F | Cl | $C(Me)=NOCH_2CO_2Me$ |
| Me | H | Me | H | H | H | O | - | - | 0 | F | Cl | $C(Me)=NOCH_2CO_2Et$ |
| Me | H | Me | H | H | H | O | - | - | 0 | F | Cl | $CH=NOCH_2CO_2Me$ |
| Me | H | Me | H | H | H | O | - | - | 0 | F | Cl | $CH=NOCH_2CO_2Et$ |
| Me | H | Me | H | H | H | O | - | - | 0 | F | Cl | $C(OMe)=NOCH_2CO_2Me$ |
| Me | H | Me | H | H | H | O | - | - | 0 | F | Cl | $C(OMe)=NOCH_2CO_2Et$ |
| Me | H | Me | H | H | H | O | - | - | 0 | F | Cl | $C(Me)=NN=C(Me)CO_2Me$ |
| Me | H | Me | H | H | H | O | - | - | 0 | F | Cl | $C(Me)=NN=C(Me)CO_2Et$ |
| Me | H | Me | H | H | H | O | - | - | 0 | F | Cl | $CH_2OCH_2CO_2Me$ |
| Me | H | Me | H | H | H | O | - | - | 0 | F | Cl | $CH_2OCH_2CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | H | H | O | - | - | 0 | F | Cl | $CH_2OCH(Me)CO_2Me$ |
| Me | H | Me | H | H | H | O | - | - | 0 | F | Cl | $CH_2OCH(Me)CO_2Et$ |
| Me | H | Me | H | H | H | O | - | - | 0 | F | Cl | $CH_2SCH_2CO_2Me$ |
| Me | H | Me | H | H | H | O | - | - | 0 | F | Cl | $CH_2SCH_2CO_2Et$ |
| Me | H | Me | H | H | H | O | - | - | 0 | F | Cl | $CH_2SCH(Me)CO_2Me$ |
| Me | H | Me | H | H | H | O | - | - | 0 | F | Cl | $CH_2SCH(Me)CO_2Et$ |
| Me | H | Me | H | H | H | O | - | - | 0 | F | Cl | SMe |
| Me | H | Me | H | H | H | O | - | - | 0 | F | Cl | SEt |
| Me | H | Me | H | H | H | O | - | - | 0 | F | Cl | $SPr^i$ |
| Me | H | Me | H | H | H | O | - | - | 0 | F | Cl | $SPr^n$ |
| Me | H | Me | H | H | H | O | - | - | 0 | F | Cl | $SPen^c$ |
| Me | H | Me | H | H | H | O | - | - | 0 | F | Cl | $SCH_2CO_2H$ |
| Me | H | Me | H | H | H | O | - | - | 0 | F | Cl | $SCH_2CO_2Me$ |
| Me | H | Me | H | H | H | O | - | - | 0 | F | Cl | $SCH_2CO_2Et$ |
| Me | H | Me | H | H | H | O | - | - | 0 | F | Cl | $SCH(Me)CO_2Me$ |
| Me | H | Me | H | H | H | O | - | - | 0 | F | Cl | $SCH(Me)CO_2Et$ |
| Me | H | Me | H | H | H | O | - | - | 0 | F | Cl | $SCH_2CN$ |
| Me | H | Me | H | H | H | O | - | - | 0 | F | Cl | $SCH_2CH=CH_2$ |
| Me | H | Me | H | H | H | O | - | - | 0 | F | Cl | $SCH_2C\equiv CH$ |
| Me | H | Me | H | H | H | O | - | - | 0 | F | Cl | $OCH(Me)C\equiv CH$ |
| Me | H | Me | H | H | H | O | - | - | 0 | F | Cl | $SCH(Me)C\equiv CH$ |
| Me | H | Me | H | H | H | O | - | - | 0 | F | Cl | $N(SO_2Me)_2$ |
| Me | H | Me | H | H | H | O | - | - | 0 | F | Cl | $N(SO_2Et)_2$ |
| Me | H | Me | H | H | H | O | - | - | 0 | F | Cl | $Q1(R29=Bu^t)$ |

Table 2-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | H | H | O | - | - | 0 | F | Cl | Q2(R29=$Bu^t$) |
| Me | H | Me | H | H | H | O | - | - | 0 | F | Cl | Q5(R29=$Bu^t$) |
| Me | H | Me | H | H | H | O | - | - | 0 | F | Cl | Q7(R34=$Bu^t$) |
| Me | H | Me | H | H | H | O | - | - | 0 | F | Cl | Q9(R38=R39=H) |
| Me | H | Me | H | H | H | O | - | - | 0 | F | Cl | Q10(R38=R39=H) |
| Me | H | Me | H | H | H | O | - | - | 0 | F | Cl | Q4(R29=$Bu^t$) |
| Me | H | Me | H | H | H | O | - | - | 0 | F | Cl | Q8(R35=R36=R37=H) |
| Me | H | Me | H | H | H | O | - | - | 0 | F | Cl | Q18(R50=H) |
| Me | H | Me | H | H | H | O | - | - | 0 | F | Cl | Q18(R50=Me) |
| Me | H | Me | H | H | H | O | - | - | 0 | F | Cl | $NHCO_2Me$ |
| Me | H | Me | H | H | H | O | - | - | 0 | F | Cl | $NHCO_2Et$ |
| Me | H | Me | H | H | H | O | - | - | 0 | F | Cl | $NHCO_2Pr^n$ |
| Me | H | Me | H | H | H | O | - | - | 0 | F | Cl | $NHCO_2Pr^i$ |
| Me | H | Me | H | H | H | O | - | - | 0 | F | Cl | $NHCO_2Ph$ |
| Me | H | Me | H | H | H | O | - | - | 0 | F | Cl | $NHCO_2Bz$ |
| Me | H | Me | H | H | H | O | - | - | 0 | F | Cl | NHCOMe |
| Me | H | Me | H | H | H | O | - | - | 0 | F | Cl | $NHCO_2CH_2Ph$ |
| Me | H | Me | H | H | H | O | - | - | 0 | F | Cl | $NHCO_2CH_2$(4-Me-Ph) |
| Me | H | Me | H | H | H | O | - | - | 0 | F | Cl | $NHCO_2CH_2$(3-Me-Ph) |
| Me | H | Me | H | H | H | O | - | - | 0 | F | Cl | $NHCO_2CH_2$(2-Me-Ph) |
| Me | H | Me | H | H | H | O | - | - | 0 | F | Cl | $NHCO_2CH_2$(4-CL -Ph) |
| Me | H | Me | H | H | H | O | - | - | 0 | F | Cl | $NHCO_2CH_2$(3-CL-Ph) |
| Me | H | Me | H | H | H | O | - | - | 0 | F | Cl | $NHCO_2CH_2$(2-CL-Ph) |
| Me | H | Me | H | H | H | O | - | - | 0 | F | Cl | Q1(R29=Me) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | H | H | O | - | - | 0 | F | Cl | Q2(R29=Me) |
| Me | H | Me | H | H | H | O | - | - | 0 | F | Cl | $CO_2Me$ |
| Me | H | Me | H | H | H | O | - | - | 0 | F | Cl | $CO_2H$ |
| $CF_3$ | H | Et | H | H | H | O | - | - | 0 | F | Cl | $CO_2Pr^i$ |
| $CF_3$ | H | Et | H | H | H | O | - | - | 0 | F | Cl | $CO_2Pr^n$ |
| $CF_3$ | H | Et | H | H | H | O | - | - | 0 | F | Cl | $CO_2Bu^n$ |
| $CF_3$ | H | Et | H | H | H | O | - | - | 0 | F | Cl | $CO_2Bu^t$ |
| $CF_3$ | H | Et | H | H | H | O | - | - | 0 | F | Cl | $NO_2$ |
| $CF_3$ | H | Et | H | H | H | O | - | - | 0 | F | Cl | $NH_2$ |
| $CF_3$ | H | Et | H | H | H | O | - | - | 0 | F | Cl | $NHSO_2Me$ |
| $CF_3$ | H | Et | H | H | H | O | - | - | 0 | F | Cl | $NHSO_2Et$ |
| $CF_3$ | H | Et | H | H | H | O | - | - | 0 | F | Cl | $NHSO_2Pr^n$ |
| $CF_3$ | H | Et | H | H | H | O | - | - | 0 | F | Cl | $NHSO_2Pr^i$ |
| $CF_3$ | H | Et | H | H | H | O | - | - | 0 | F | Cl | OH |
| $CF_3$ | H | Et | H | H | H | O | - | - | 0 | F | Cl | OMe |
| $CF_3$ | H | Et | H | H | H | O | - | - | 0 | F | Cl | OEt |
| $CF_3$ | H | Et | H | H | H | O | - | - | 0 | F | Cl | $OPr^i$ |
| $CF_3$ | H | Et | H | H | H | O | - | - | 0 | F | Cl | $OPr^n$ |
| $CF_3$ | H | Et | H | H | H | O | - | - | 0 | F | Cl | $OPen^c$ |
| $CF_3$ | H | Et | H | H | H | O | - | - | 0 | F | Cl | $OCH_2CH=CH_2$ |
| $CF_3$ | H | Et | H | H | H | O | - | - | 0 | F | Cl | $OCH_2C\equiv CH$ |
| $CF_3$ | H | Et | H | H | H | O | - | - | 0 | F | Cl | $OCH_2CO_2Me$ |
| $CF_3$ | H | Et | H | H | H | O | - | - | 0 | F | Cl | $OCH_2CO_2Et$ |
| $CF_3$ | H | Et | H | H | H | O | - | - | 0 | F | Cl | $OCH(Me)CO_2Me$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Et | H | H | H | 0 | - | - | 0 | F | Cl | $OCH(Me)CO_2Et$ |
| $CF_3$ | H | Et | H | H | H | 0 | - | - | 0 | F | Cl | $CO_2H$ |
| $CF_3$ | H | Et | H | H | H | 0 | - | - | 0 | F | Cl | CN |
| $CF_3$ | H | Et | H | H | H | 0 | - | - | 0 | F | Cl | Me |
| $CF_3$ | H | Et | H | H | H | 0 | - | - | 0 | F | Cl | Et |
| $CF_3$ | H | Et | H | H | H | 0 | - | - | 0 | F | Cl | CL |
| $CF_3$ | H | Et | H | H | H | 0 | - | - | 0 | F | Cl | Br |
| $CF_3$ | H | Et | H | H | H | 0 | - | - | 0 | F | Cl | I |
| $CF_3$ | H | Et | H | H | H | 0 | - | - | 0 | F | Cl | F |
| $CF_3$ | H | Et | H | H | H | 0 | - | - | 0 | F | Cl | SH |
| $CF_3$ | H | Et | H | H | H | 0 | - | - | 0 | F | Cl | $C(Me)=NOCH_2CO_2Me$ |
| $CF_3$ | H | Et | H | H | H | 0 | - | - | 0 | F | Cl | $C(Me)=NOCH_2CO_2Et$ |
| $CF_3$ | H | Et | H | H | H | 0 | - | - | 0 | F | Cl | $CH=NOCH_2CO_2Me$ |
| $CF_3$ | H | Et | H | H | H | 0 | - | - | 0 | F | Cl | $CH=NOCH_2CO_2Et$ |
| $CF_3$ | H | Et | H | H | H | 0 | - | - | 0 | F | Cl | $C(OMe)=NOCH_2CO_2Me$ |
| $CF_3$ | H | Et | H | H | H | 0 | - | - | 0 | F | Cl | $C(OMe)=NOCH_2CO_2Et$ |
| $CF_3$ | H | Et | H | H | H | 0 | - | - | 0 | F | Cl | $C(Me)=NN=C(Me)CO_2Me$ |
| $CF_3$ | H | Et | H | H | H | 0 | - | - | 0 | F | Cl | $C(Me)=NN=C(Me)CO_2Et$ |
| $CF_3$ | H | Et | H | H | H | 0 | - | - | 0 | F | Cl | $CH_2OCH_2CO_2Me$ |
| $CF_3$ | H | Et | H | H | H | 0 | - | - | 0 | F | Cl | $CH_2OCH_2CO_2Et$ |
| $CF_3$ | H | Et | H | H | H | 0 | - | - | 0 | F | Cl | $CH_2OCH(Me)CO_2Me$ |
| $CF_3$ | H | Et | H | H | H | 0 | - | - | 0 | F | Cl | $CH_2OCH(Me)CO_2Et$ |
| $CF_3$ | H | Et | H | H | H | 0 | - | - | 0 | F | Cl | $CH_2SCH_2CO_2Me$ |
| $CF_3$ | H | Et | H | H | H | 0 | - | - | 0 | F | Cl | $CH_2SCH_2CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Et | H | H | H | O | - | - | 0 | F | Cl | $CH_2SCH(Me)CO_2Me$ |
| $CF_3$ | H | Et | H | H | H | O | - | - | 0 | F | Cl | $CH_2SCH(Me)CO_2Et$ |
| $CF_3$ | H | Et | H | H | H | O | - | - | 0 | F | Cl | SMe |
| $CF_3$ | H | Et | H | H | H | O | - | - | 0 | F | Cl | SEt |
| $CF_3$ | H | Et | H | H | H | O | - | - | 0 | F | Cl | $SPr^i$ |
| $CF_3$ | H | Et | H | H | H | O | - | - | 0 | F | Cl | $SPr^n$ |
| $CF_3$ | H | Et | H | H | H | O | - | - | 0 | F | Cl | $SPen^c$ |
| $CF_3$ | H | Et | H | H | H | O | - | - | 0 | F | Cl | $SCH_2CO_2H$ |
| $CF_3$ | H | Et | H | H | H | O | - | - | 0 | F | Cl | $SCH_2CO_2Me$ |
| $CF_3$ | H | Et | H | H | H | O | - | - | 0 | F | Cl | $SCH_2CO_2Et$ |
| $CF_3$ | H | Et | H | H | H | O | - | - | 0 | F | Cl | $SCH(Me)CO_2Me$ |
| $CF_3$ | H | Et | H | H | H | O | - | - | 0 | F | Cl | $SCH(Me)CO_2Et$ |
| $CF_3$ | H | Et | H | H | H | O | - | - | 0 | F | Cl | $SCH_2CN$ |
| $CF_3$ | H | Et | H | H | H | O | - | - | 0 | F | Cl | $SCH_2CH=CH_2$ |
| $CF_3$ | H | Et | H | H | H | O | - | - | 0 | F | Cl | $SCH_2C\equiv CH$ |
| $CF_3$ | H | Et | H | H | H | O | - | - | 0 | F | Cl | $OCH(Me)C\equiv CH$ |
| $CF_3$ | H | Et | H | H | H | O | - | - | 0 | F | Cl | $SCH(Me)C\equiv CH$ |
| $CF_3$ | H | Et | H | H | H | O | - | - | 0 | F | Cl | $N(SO_2Me)_2$ |
| $CF_3$ | H | Et | H | H | H | O | - | - | 0 | F | Cl | $N(SO_2Et)_2$ |
| $CF_3$ | H | Et | H | H | H | O | - | - | 0 | F | Cl | Q1(R29=$Bu^t$) |
| $CF_3$ | H | Et | H | H | H | O | - | - | 0 | F | Cl | Q2(R29=$Bu^t$) |
| $CF_3$ | H | Et | H | H | H | O | - | - | 0 | F | Cl | Q5(R29=$Bu^t$) |
| $CF_3$ | H | Et | H | H | H | O | - | - | 0 | F | Cl | Q7(R34=$Bu^t$) |
| $CF_3$ | H | Et | H | H | H | O | - | - | 0 | F | Cl | Q9(R38=R39=H) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Et | H | H | H | O | - | - | 0 | F | Cl | Q10(R38=R39=H) |
| $CF_3$ | H | Et | H | H | H | O | - | - | 0 | F | Cl | Q4(R29=$Bu^t$) |
| $CF_3$ | H | Et | H | H | H | O | - | - | 0 | F | Cl | Q8(R35=R36=R37=H) |
| $CF_3$ | H | Et | H | H | H | O | - | - | 0 | F | Cl | Q18(R50=H) |
| $CF_3$ | H | Et | H | H | H | O | - | - | 0 | F | Cl | Q18(R50=Me) |
| $CF_3$ | H | Et | H | H | H | O | - | - | 0 | F | Cl | $NHCO_2Me$ |
| $CF_3$ | H | Et | H | H | H | O | - | - | 0 | F | Cl | $NHCO_2Et$ |
| $CF_3$ | H | Et | H | H | H | O | - | - | 0 | F | Cl | $NHCO_2Pr^n$ |
| $CF_3$ | H | Et | H | H | H | O | - | - | 0 | F | Cl | $NHCO_2Pr^i$ |
| $CF_3$ | H | Et | H | H | H | O | - | - | 0 | F | Cl | $NHCO_2Ph$ |
| $CF_3$ | H | Et | H | H | H | O | - | - | 0 | F | Cl | $NHCO_2Bz$ |
| $CF_3$ | H | Et | H | H | H | O | - | - | 0 | F | Cl | NHCOMe |
| $CF_3$ | H | Et | H | H | H | O | - | - | 0 | F | Cl | $NHCO_2CH_2Ph$ |
| $CF_3$ | H | Et | H | H | H | O | - | - | 0 | F | Cl | $NHCO_2CH_2$(4-Me-Ph) |
| $CF_3$ | H | Et | H | H | H | O | - | - | 0 | F | Cl | $NHCO_2CH_2$(3-Me-Ph) |
| $CF_3$ | H | Et | H | H | H | O | - | - | 0 | F | Cl | $NHCO_2CH_2$(2-Me-Ph) |
| $CF_3$ | H | Et | H | H | H | O | - | - | 0 | F | Cl | $NHCO_2CH_2$(4-CL-Ph) |
| $CF_3$ | H | Et | H | H | H | O | - | - | 0 | F | Cl | $NHCO_2CH_2$(3-CL-Ph) |
| $CF_3$ | H | Et | H | H | H | O | - | - | 0 | F | Cl | $NHCO_2CH_2$(2-CL-Ph) |
| $CF_3$ | H | Et | H | H | H | O | - | - | 0 | F | Cl | Q1(R29=Me) |
| $CF_3$ | H | Et | H | H | H | O | - | - | 0 | F | Cl | Q2(R29=Me) |
| $CF_3$ | H | Et | H | H | H | O | - | - | 0 | F | Cl | H |
| $CF_3$ | H | Et | H | H | H | O | - | - | 0 | F | Cl | $CO_2H$ |
| $CF_3$ | H | Et | H | H | H | O | - | - | 0 | F | Cl | $CO_2Me$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Et | H | H | H | O | - | - | 0 | F | Cl | $CO_2Et$ |
| Et | H | Me | H | H | H | O | - | - | 0 | F | Cl | $CO_2Pr^i$ |
| Et | H | Me | H | H | H | O | - | - | 0 | F | Cl | $CO_2Pr^n$ |
| Et | H | Me | H | H | H | O | - | - | 0 | F | Cl | $CO_2Bu^n$ |
| Et | H | Me | H | H | H | O | - | - | 0 | F | Cl | $CO_2Bu^t$ |
| Et | H | Me | H | H | H | O | - | - | 0 | F | Cl | $NO_2$ |
| Et | H | Me | H | H | H | O | - | - | 0 | F | Cl | $NH_2$ |
| Et | H | Me | H | H | H | O | - | - | 0 | F | Cl | $NHSO_2Me$ |
| Et | H | Me | H | H | H | O | - | - | 0 | F | Cl | $NHSO_2Et$ |
| Et | H | Me | H | H | H | O | - | - | 0 | F | Cl | $NHSO_2Pr^n$ |
| Et | H | Me | H | H | H | O | - | - | 0 | F | Cl | $NHSO_2Pr^i$ |
| Et | H | Me | H | H | H | O | - | - | 0 | F | Cl | OH |
| Et | H | Me | H | H | H | O | - | - | 0 | F | Cl | OMe |
| Et | H | Me | H | H | H | O | - | - | 0 | F | Cl | OEt |
| Et | H | Me | H | H | H | O | - | - | 0 | F | Cl | $OPr^i$ |
| Et | H | Me | H | H | H | O | - | - | 0 | F | Cl | $OPr^n$ |
| Et | H | Me | H | H | H | O | - | - | 0 | F | Cl | $OPen^c$ |
| Et | H | Me | H | H | H | O | - | - | 0 | F | Cl | $OCH_2CH=CH_2$ |
| Et | H | Me | H | H | H | O | - | - | 0 | F | Cl | $OCH_2C\equiv CH$ |
| Et | H | Me | H | H | H | O | - | - | 0 | F | Cl | $OCH_2CO_2Me$ |
| Et | H | Me | H | H | H | O | - | - | 0 | F | Cl | $OCH_2CO_2Et$ |
| Et | H | Me | H | H | H | O | - | - | 0 | F | Cl | $OCH(Me)CO_2Me$ |
| Et | H | Me | H | H | H | O | - | - | 0 | F | Cl | $OCH(Me)CO_2Et$ |
| Et | H | Me | H | H | H | O | - | - | 0 | F | Cl | $CO_2H$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | H | H | O | - | - | 0 | F | Cl | CN |
| Et | H | Me | H | H | H | O | - | - | 0 | F | Cl | Me |
| Et | H | Me | H | H | H | O | - | - | 0 | F | Cl | Et |
| Et | H | Me | H | H | H | O | - | - | 0 | F | Cl | Cl |
| Et | H | Me | H | H | H | O | - | - | 0 | F | Cl | Br |
| Et | H | Me | H | H | H | O | - | - | 0 | F | Cl | I |
| Et | H | Me | H | H | H | O | - | - | 0 | F | Cl | F |
| Et | H | Me | H | H | H | O | - | - | 0 | F | Cl | SH |
| Et | H | Me | H | H | H | O | - | - | 0 | F | Cl | $C(Me)=NOCH_2CO_2Me$ |
| Et | H | Me | H | H | H | O | - | - | 0 | F | Cl | $C(Me)=NOCH_2CO_2Et$ |
| Et | H | Me | H | H | H | O | - | - | 0 | F | Cl | $CH=NOCH_2CO_2Me$ |
| Et | H | Me | H | H | H | O | - | - | 0 | F | Cl | $CH=NOCH_2CO_2Et$ |
| Et | H | Me | H | H | H | O | - | - | 0 | F | Cl | $C(OMe)=NOCH_2CO_2Me$ |
| Et | H | Me | H | H | H | O | - | - | 0 | F | Cl | $C(OMe)=NOCH_2CO_2Et$ |
| Et | H | Me | H | H | H | O | - | - | 0 | F | Cl | $C(Me)=NN=C(Me)CO_2Me$ |
| Et | H | Me | H | H | H | O | - | - | 0 | F | Cl | $C(Me)=NN=C(Me)CO_2Et$ |
| Et | H | Me | H | H | H | O | - | - | 0 | F | Cl | $CH_2OCH_2CO_2Me$ |
| Et | H | Me | H | H | H | O | - | - | 0 | F | Cl | $CH_2OCH_2CO_2Et$ |
| Et | H | Me | H | H | H | O | - | - | 0 | F | Cl | $CH_2OCH(Me)CO_2Me$ |
| Et | H | Me | H | H | H | O | - | - | 0 | F | Cl | $CH_2OCH(Me)CO_2Et$ |
| Et | H | Me | H | H | H | O | - | - | 0 | F | Cl | $CH_2SCH_2CO_2Me$ |
| Et | H | Me | H | H | H | O | - | - | 0 | F | Cl | $CH_2SCH_2CO_2Et$ |
| Et | H | Me | H | H | H | O | - | - | 0 | F | Cl | $CH_2SCH(Me)CO_2Me$ |
| Et | H | Me | H | H | H | O | - | - | 0 | F | Cl | $CH_2SCH(Me)CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | H | H | O | - | - | 0 | F | Cl | SMe |
| Et | H | Me | H | H | H | O | - | - | 0 | F | Cl | SEt |
| Et | H | Me | H | H | H | O | - | - | 0 | F | Cl | $SPr^i$ |
| Et | H | Me | H | H | H | O | - | - | 0 | F | Cl | $SPr^n$ |
| Et | H | Me | H | H | H | O | - | - | 0 | F | Cl | $SPen^c$ |
| Et | H | Me | H | H | H | O | - | - | 0 | F | Cl | $SCH_2CO_2H$ |
| Et | H | Me | H | H | H | O | - | - | 0 | F | Cl | $SCH_2CO_2Me$ |
| Et | H | Me | H | H | H | O | - | - | 0 | F | Cl | $SCH_2CO_2Et$ |
| Et | H | Me | H | H | H | O | - | - | 0 | F | Cl | $SCH(Me)CO_2Me$ |
| Et | H | Me | H | H | H | O | - | - | 0 | F | Cl | $SCH(Me)CO_2Et$ |
| Et | H | Me | H | H | H | O | - | - | 0 | F | Cl | $SCH_2CN$ |
| Et | H | Me | H | H | H | O | - | - | 0 | F | Cl | $SCH_2CH=CH_2$ |
| Et | H | Me | H | H | H | O | - | - | 0 | F | Cl | $SCH_2C\equiv CH$ |
| Et | H | Me | H | H | H | O | - | - | 0 | F | Cl | $OCH(Me)C\equiv CH$ |
| Et | H | Me | H | H | H | O | - | - | 0 | F | Cl | $SCH(Me)C\equiv CH$ |
| Et | H | Me | H | H | H | O | - | - | 0 | F | Cl | $N(SO_2Me)_2$ |
| Et | H | Me | H | H | H | O | - | - | 0 | F | Cl | $N(SO_2Et)_2$ |
| Et | H | Me | H | H | H | O | - | - | 0 | F | Cl | Q1(R29=$Bu^t$) |
| Et | H | Me | H | H | H | O | - | - | 0 | F | Cl | Q2(R29=$Bu^t$) |
| Et | H | Me | H | H | H | O | - | - | 0 | F | Cl | Q5(R29=$Bu^t$) |
| Et | H | Me | H | H | H | O | - | - | 0 | F | Cl | Q7(R34=$Bu^t$) |
| Et | H | Me | H | H | H | O | - | - | 0 | F | Cl | Q9(R38=R39=H) |
| Et | H | Me | H | H | H | O | - | - | 0 | F | Cl | Q10(R38=R39=H) |
| Et | H | Me | H | H | H | O | - | - | 0 | F | Cl | Q4(R29=$Bu^t$) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | H | H | O | - | - | 0 | F | Cl | Q8(R35=R36=R37=H) |
| Et | H | Me | H | H | H | O | - | - | 0 | F | Cl | Q18(R50=H) |
| Et | H | Me | H | H | H | O | - | - | 0 | F | Cl | Q18(R50=Me) |
| Et | H | Me | H | H | H | O | - | - | 0 | F | Cl | $NHCO_2Me$ |
| Et | H | Me | H | H | H | O | - | - | 0 | F | Cl | $NHCO_2Et$ |
| Et | H | Me | H | H | H | O | - | - | 0 | F | Cl | $NHCO_2Pr^n$ |
| Et | H | Me | H | H | H | O | - | - | 0 | F | Cl | $NHCO_2Pr^i$ |
| Et | H | Me | H | H | H | O | - | - | 0 | F | Cl | $NHCO_2Ph$ |
| Et | H | Me | H | H | H | O | - | - | 0 | F | Cl | $NHCO_2Bz$ |
| Et | H | Me | H | H | H | O | - | - | 0 | F | Cl | NHCOMe |
| Et | H | Me | H | H | H | O | - | - | 0 | F | Cl | $NHCO_2CH_2Ph$ |
| Et | H | Me | H | H | H | O | - | - | 0 | F | Cl | $NHCO_2CH_2$(4-Me-Ph) |
| Et | H | Me | H | H | H | O | - | - | 0 | F | Cl | $NHCO_2CH_2$(3-Me-Ph) |
| Et | H | Me | H | H | H | O | - | - | 0 | F | Cl | $NHCO_2CH_2$(2-Me-Ph) |
| Et | H | Me | H | H | H | O | - | - | 0 | F | Cl | $NHCO_2CH_2$(4-CL-Ph) |
| Et | H | Me | H | H | H | O | - | - | 0 | F | Cl | $NHCO_2CH_2$(3-CL-Ph) |
| Et | H | Me | H | H | H | O | - | - | 0 | F | Cl | $NHCO_2CH_2$(2-CL-Ph) |
| Et | H | Me | H | H | H | O | - | - | 0 | F | Cl | Q1(R29=Me) |
| Et | H | Me | H | H | H | O | - | - | 0 | F | Cl | Q2(R29=Me) |
| Et | H | Me | H | H | H | O | - | - | 0 | F | Cl | H |
| Et | H | Me | H | H | H | O | - | - | 0 | F | Cl | $CO_2H$ |
| Et | H | Me | H | H | H | O | - | - | 0 | F | Cl | $CO_2Me$ |
| Et | H | Me | H | H | H | O | - | - | 0 | F | Cl | $CO_2Et$ |
| $Pr^n$ | H | Me | H | H | H | O | - | - | 0 | F | Cl | $CO_2Pr^i$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | H | H | O | - | - | 0 | F | Cl | $CO_2Pr^n$ |
| $Pr^n$ | H | Me | H | H | H | O | - | - | 0 | F | Cl | $CO_2Bu^n$ |
| $Pr^n$ | H | Me | H | H | H | O | - | - | 0 | F | Cl | $CO_2Bu^t$ |
| $Pr^n$ | H | Me | H | H | H | O | - | - | 0 | F | Cl | $NO_2$ |
| $Pr^n$ | H | Me | H | H | H | O | - | - | 0 | F | Cl | $NH_2$ |
| $Pr^n$ | H | Me | H | H | H | O | - | - | 0 | F | Cl | $NHSO_2Me$ |
| $Pr^n$ | H | Me | H | H | H | O | - | - | 0 | F | Cl | $NHSO_2Et$ |
| $Pr^n$ | H | Me | H | H | H | O | - | - | 0 | F | Cl | $NHSO_2Pr^n$ |
| $Pr^n$ | H | Me | H | H | H | O | - | - | 0 | F | Cl | $NHSO_2Pr^i$ |
| $Pr^n$ | H | Me | H | H | H | O | - | - | 0 | F | Cl | OH |
| $Pr^n$ | H | Me | H | H | H | O | - | - | 0 | F | Cl | OMe |
| $Pr^n$ | H | Me | H | H | H | O | - | - | 0 | F | Cl | OEt |
| $Pr^n$ | H | Me | H | H | H | O | - | - | 0 | F | Cl | $OPr^i$ |
| $Pr^n$ | H | Me | H | H | H | O | - | - | 0 | F | Cl | $OPr^n$ |
| $Pr^n$ | H | Me | H | H | H | O | - | - | 0 | F | Cl | $OPen^c$ |
| $Pr^n$ | H | Me | H | H | H | O | - | - | 0 | F | Cl | $OCH_2CH=CH_2$ |
| $Pr^n$ | H | Me | H | H | H | O | - | - | 0 | F | Cl | $OCH_2C\equiv CH$ |
| $Pr^n$ | H | Me | H | H | H | O | - | - | 0 | F | Cl | $OCH_2CO_2Me$ |
| $Pr^n$ | H | Me | H | H | H | O | - | - | 0 | F | Cl | $OCH_2CO_2Et$ |
| $Pr^n$ | H | Me | H | H | H | O | - | - | 0 | F | Cl | $OCH(Me)CO_2Me$ |
| $Pr^n$ | H | Me | H | H | H | O | - | - | 0 | F | Cl | $OCH(Me)CO_2Et$ |
| $Pr^n$ | H | Me | H | H | H | O | - | - | 0 | F | Cl | $CO_2H$ |
| $Pr^n$ | H | Me | H | H | H | O | - | - | 0 | F | Cl | CN |
| $Pr^n$ | H | Me | H | H | H | O | - | - | 0 | F | Cl | Me |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | H | H | O | - | - | 0 | F | Cl | Et |
| $Pr^n$ | H | Me | H | H | H | O | - | - | 0 | F | Cl | Cl |
| $Pr^n$ | H | Me | H | H | H | O | - | - | 0 | F | Cl | Br |
| $Pr^n$ | H | Me | H | H | H | O | - | - | 0 | F | Cl | I |
| $Pr^n$ | H | Me | H | H | H | O | - | - | 0 | F | Cl | F |
| $Pr^n$ | H | Me | H | H | H | O | - | - | 0 | F | Cl | SH |
| $Pr^n$ | H | Me | H | H | H | O | - | - | 0 | F | Cl | $C(Me)=NOCH_2CO_2Me$ |
| $Pr^n$ | H | Me | H | H | H | O | - | - | 0 | F | Cl | $C(Me)=NOCH_2CO_2Et$ |
| $Pr^n$ | H | Me | H | H | H | O | - | - | 0 | F | Cl | $CH=NOCH_2CO_2Me$ |
| $Pr^n$ | H | Me | H | H | H | O | - | - | 0 | F | Cl | $CH=NOCH_2CO_2Et$ |
| $Pr^n$ | H | Me | H | H | H | O | - | - | 0 | F | Cl | $C(OMe)=NOCH_2CO_2Me$ |
| $Pr^n$ | H | Me | H | H | H | O | - | - | 0 | F | Cl | $C(OMe)=NOCH_2CO_2Et$ |
| $Pr^n$ | H | Me | H | H | H | O | - | - | 0 | F | Cl | $C(Me)=NN=C(Me)CO_2Me$ |
| $Pr^n$ | H | Me | H | H | H | O | - | - | 0 | F | Cl | $C(Me)=NN=C(Me)CO_2Et$ |
| $Pr^n$ | H | Me | H | H | H | O | - | - | 0 | F | Cl | $CH_2OCH_2CO_2Me$ |
| $Pr^n$ | H | Me | H | H | H | O | - | - | 0 | F | Cl | $CH_2OCH_2CO_2Et$ |
| $Pr^n$ | H | Me | H | H | H | O | - | - | 0 | F | Cl | $CH_2OCH(Me)CO_2Me$ |
| $Pr^n$ | H | Me | H | H | H | O | - | - | 0 | F | Cl | $CH_2OCH(Me)CO_2Et$ |
| $Pr^n$ | H | Me | H | H | H | O | - | - | 0 | F | Cl | $CH_2SCH_2CO_2Me$ |
| $Pr^n$ | H | Me | H | H | H | O | - | - | 0 | F | Cl | $CH_2SCH_2CO_2Et$ |
| $Pr^n$ | H | Me | H | H | H | O | - | - | 0 | F | Cl | $CH_2SCH(Me)CO_2Me$ |
| $Pr^n$ | H | Me | H | H | H | O | - | - | 0 | F | Cl | $CH_2SCH(Me)CO_2Et$ |
| $Pr^n$ | H | Me | H | H | H | O | - | - | 0 | F | Cl | SMe |
| $Pr^n$ | H | Me | H | H | H | O | - | - | 0 | F | Cl | SEt |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | H | H | O | - | - | 0 | F | Cl | $SPr^i$ |
| $Pr^n$ | H | Me | H | H | H | O | - | - | 0 | F | Cl | $SPr^n$ |
| $Pr^n$ | H | Me | H | H | H | O | - | - | 0 | F | Cl | $SPen^c$ |
| $Pr^n$ | H | Me | H | H | H | O | - | - | 0 | F | Cl | $SCH_2CO_2H$ |
| $Pr^n$ | H | Me | H | H | H | O | - | - | 0 | F | Cl | $SCH_2CO_2Me$ |
| $Pr^n$ | H | Me | H | H | H | O | - | - | 0 | F | Cl | $SCH_2CO_2Et$ |
| $Pr^n$ | H | Me | H | H | H | O | - | - | 0 | F | Cl | $SCH(Me)CO_2Me$ |
| $Pr^n$ | H | Me | H | H | H | O | - | - | 0 | F | Cl | $SCH(Me)CO_2Et$ |
| $Pr^n$ | H | Me | H | H | H | O | - | - | 0 | F | Cl | $SCH_2CN$ |
| $Pr^n$ | H | Me | H | H | H | O | - | - | 0 | F | Cl | $SCH_2CH=CH_2$ |
| $Pr^n$ | H | Me | H | H | H | O | - | - | 0 | F | Cl | $SCH_2C\equiv CH$ |
| $Pr^n$ | H | Me | H | H | H | O | - | - | 0 | F | Cl | $OCH(Me)C\equiv CH$ |
| $Pr^n$ | H | Me | H | H | H | O | - | - | 0 | F | Cl | $SCH(Me)C\equiv CH$ |
| $Pr^n$ | H | Me | H | H | H | O | - | - | 0 | F | Cl | $N(SO_2Me)_2$ |
| $Pr^n$ | H | Me | H | H | H | O | - | - | 0 | F | Cl | $N(SO_2Et)_2$ |
| $Pr^n$ | H | Me | H | H | H | O | - | - | 0 | F | Cl | Q1(R29=$Bu^t$) |
| $Pr^n$ | H | Me | H | H | H | O | - | - | 0 | F | Cl | Q2(R29=$Bu^t$) |
| $Pr^n$ | H | Me | H | H | H | O | - | - | 0 | F | Cl | Q5(R29=$Bu^t$) |
| $Pr^n$ | H | Me | H | H | H | O | - | - | 0 | F | Cl | Q7(R34=$Bu^t$) |
| $Pr^n$ | H | Me | H | H | H | O | - | - | 0 | F | Cl | Q9(R38=R39=H) |
| $Pr^n$ | H | Me | H | H | H | O | - | - | 0 | F | Cl | Q10(R38=R39=H) |
| $Pr^n$ | H | Me | H | H | H | O | - | - | 0 | F | Cl | Q4(R29=$Bu^t$) |
| $Pr^n$ | H | Me | H | H | H | O | - | - | 0 | F | Cl | Q8(R35=R36=R37=H) |
| $Pr^n$ | H | Me | H | H | H | O | - | - | 0 | F | Cl | Q18(R50=H) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | H | H | O | - | - | 0 | F | Cl | Q18(R50=Me) |
| $Pr^n$ | H | Me | H | H | H | O | - | - | 0 | F | Cl | $NHCO_2Me$ |
| $Pr^n$ | H | Me | H | H | H | O | - | - | 0 | F | Cl | $NHCO_2Et$ |
| $Pr^n$ | H | Me | H | H | H | O | - | - | 0 | F | Cl | $NHCO_2Pr^n$ |
| $Pr^n$ | H | Me | H | H | H | O | - | - | 0 | F | Cl | $NHCO_2Pr^i$ |
| $Pr^n$ | H | Me | H | H | H | O | - | - | 0 | F | Cl | $NHCO_2Ph$ |
| $Pr^n$ | H | Me | H | H | H | O | - | - | 0 | F | Cl | $NHCO_2Bz$ |
| $Pr^n$ | H | Me | H | H | H | O | - | - | 0 | F | Cl | NHCOMe |
| $Pr^n$ | H | Me | H | H | H | O | - | - | 0 | F | Cl | $NHCO_2CH_2Ph$ |
| $Pr^n$ | H | Me | H | H | H | O | - | - | 0 | F | Cl | $NHCO_2CH_2$(4-Me-Ph) |
| $Pr^n$ | H | Me | H | H | H | O | - | - | 0 | F | Cl | $NHCO_2CH_2$(3-Me-Ph) |
| $Pr^n$ | H | Me | H | H | H | O | - | - | 0 | F | Cl | $NHCO_2CH_2$(2-Me-Ph) |
| $Pr^n$ | H | Me | H | H | H | O | - | - | 0 | F | Cl | $NHCO_2CH_2$(4-Cl-Ph) |
| $Pr^n$ | H | Me | H | H | H | O | - | - | 0 | F | Cl | $NHCO_2CH_2$(3-Cl-Ph) |
| $Pr^n$ | H | Me | H | H | H | O | - | - | 0 | F | Cl | $NHCO_2CH_2$(2-Cl-Ph) |
| $Pr^n$ | H | Me | H | H | H | O | - | - | 0 | F | Cl | Q1(R29=Me) |
| $Pr^n$ | H | Me | H | H | H | O | - | - | 0 | F | Cl | Q2(R29=Me) |
| $Pr^n$ | H | Me | H | H | H | O | - | - | 0 | F | Cl | H |
| $Pr^n$ | H | Me | H | H | H | O | - | - | 0 | F | Cl | $CO_2H$ |
| $Pr^n$ | H | Me | H | H | H | O | - | - | 0 | F | Cl | $CO_2Me$ |
| $Pr^n$ | H | Me | H | H | H | O | - | - | 0 | F | Cl | $CO_2Et$ |
| $Pr^i$ | H | Me | H | H | H | O | - | - | 0 | F | Cl | $CO_2Pr^i$ |
| $Pr^i$ | H | Me | H | H | H | O | - | - | 0 | F | Cl | $CO_2Pr^n$ |
| $Pr^i$ | H | Me | H | H | H | O | - | - | 0 | F | Cl | $CO_2Bu^n$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | H | H | O | - | - | 0 | F | Cl | $CO_2Bu^t$ |
| $Pr^i$ | H | Me | H | H | H | O | - | - | 0 | F | Cl | $NO_2$ |
| $Pr^i$ | H | Me | H | H | H | O | - | - | 0 | F | Cl | $NH_2$ |
| $Pr^i$ | H | Me | H | H | H | O | - | - | 0 | F | Cl | $NHSO_2Me$ |
| $Pr^i$ | H | Me | H | H | H | O | - | - | 0 | F | Cl | $NHSO_2Et$ |
| $Pr^i$ | H | Me | H | H | H | O | - | - | 0 | F | Cl | $NHSO_2Pr^n$ |
| $Pr^i$ | H | Me | H | H | H | O | - | - | 0 | F | Cl | $NHSO_2Pr^i$ |
| $Pr^i$ | H | Me | H | H | H | O | - | - | 0 | F | Cl | OH |
| $Pr^i$ | H | Me | H | H | H | O | - | - | 0 | F | Cl | OMe |
| $Pr^i$ | H | Me | H | H | H | O | - | - | 0 | F | Cl | OEt |
| $Pr^i$ | H | Me | H | H | H | O | - | - | 0 | F | Cl | $OPr^i$ |
| $Pr^i$ | H | Me | H | H | H | O | - | - | 0 | F | Cl | $OPr^n$ |
| $Pr^i$ | H | Me | H | H | H | O | - | - | 0 | F | Cl | $OPen^c$ |
| $Pr^i$ | H | Me | H | H | H | O | - | - | 0 | F | Cl | $OCH_2CH=CH_2$ |
| $Pr^i$ | H | Me | H | H | H | O | - | - | 0 | F | Cl | $OCH_2C{\equiv}CH$ |
| $Pr^i$ | H | Me | H | H | H | O | - | - | 0 | F | Cl | $OCH_2CO_2Me$ |
| $Pr^i$ | H | Me | H | H | H | O | - | - | 0 | F | Cl | $OCH_2CO_2Et$ |
| $Pr^i$ | H | Me | H | H | H | O | - | - | 0 | F | Cl | $OCH(Me)CO_2Me$ |
| $Pr^i$ | H | Me | H | H | H | O | - | - | 0 | F | Cl | $OCH(Me)CO_2Et$ |
| $Pr^i$ | H | Me | H | H | H | O | - | - | 0 | F | Cl | $CO_2H$ |
| $Pr^i$ | H | Me | H | H | H | O | - | - | 0 | F | Cl | CN |
| $Pr^i$ | H | Me | H | H | H | O | - | - | 0 | F | Cl | Me |
| $Pr^i$ | H | Me | H | H | H | O | - | - | 0 | F | Cl | Et |
| $Pr^i$ | H | Me | H | H | H | O | - | - | 0 | F | Cl | Cl |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | H | H | O | - | - | 0 | F | Cl | Br |
| $Pr^i$ | H | Me | H | H | H | O | - | - | 0 | F | Cl | I |
| $Pr^i$ | H | Me | H | H | H | O | - | - | 0 | F | Cl | F |
| $Pr^i$ | H | Me | H | H | H | O | - | - | 0 | F | Cl | SH |
| $Pr^i$ | H | Me | H | H | H | O | - | - | 0 | F | Cl | $C(Me)=NOCH_2CO_2Me$ |
| $Pr^i$ | H | Me | H | H | H | O | - | - | 0 | F | Cl | $C(Me)=NOCH_2CO_2Et$ |
| $Pr^i$ | H | Me | H | H | H | O | - | - | 0 | F | Cl | $CH=NOCH_2CO_2Me$ |
| $Pr^i$ | H | Me | H | H | H | O | - | - | 0 | F | Cl | $CH=NOCH_2CO_2Et$ |
| $Pr^i$ | H | Me | H | H | H | O | - | - | 0 | F | Cl | $C(OMe)=NOCH_2CO_2Me$ |
| $Pr^i$ | H | Me | H | H | H | O | - | - | 0 | F | Cl | $C(OMe)=NOCH_2CO_2Et$ |
| $Pr^i$ | H | Me | H | H | H | O | - | - | 0 | F | Cl | $C(Me)=NN=C(Me)CO_2Me$ |
| $Pr^i$ | H | Me | H | H | H | O | - | - | 0 | F | Cl | $C(Me)=NN=C(Me)CO_2Et$ |
| $Pr^i$ | H | Me | H | H | H | O | - | - | 0 | F | Cl | $CH_2OCH_2CO_2Me$ |
| $Pr^i$ | H | Me | H | H | H | O | - | - | 0 | F | Cl | $CH_2OCH_2CO_2Et$ |
| $Pr^i$ | H | Me | H | H | H | O | - | - | 0 | F | Cl | $CH_2OCH(Me)CO_2Me$ |
| $Pr^i$ | H | Me | H | H | H | O | - | - | 0 | F | Cl | $CH_2OCH(Me)CO_2Et$ |
| $Pr^i$ | H | Me | H | H | H | O | - | - | 0 | F | Cl | $CH_2SCH_2CO_2Me$ |
| $Pr^i$ | H | Me | H | H | H | O | - | - | 0 | F | Cl | $CH_2SCH_2CO_2Et$ |
| $Pr^i$ | H | Me | H | H | H | O | - | - | 0 | F | Cl | $CH_2SCH(Me)CO_2Me$ |
| $Pr^i$ | H | Me | H | H | H | O | - | - | 0 | F | Cl | $CH_2SCH(Me)CO_2Et$ |
| $Pr^i$ | H | Me | H | H | H | O | - | - | 0 | F | Cl | SMe |
| $Pr^i$ | H | Me | H | H | H | O | - | - | 0 | F | Cl | SEt |
| $Pr^i$ | H | Me | H | H | H | O | - | - | 0 | F | Cl | $SPr^i$ |
| $Pr^i$ | H | Me | H | H | H | O | - | - | 0 | F | Cl | $SPr^n$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | H | H | O | - | - | 0 | F | Cl | $SPen^c$ |
| $Pr^i$ | H | Me | H | H | H | O | - | - | 0 | F | Cl | $SCH_2CO_2H$ |
| $Pr^i$ | H | Me | H | H | H | O | - | - | 0 | F | Cl | $SCH_2CO_2Me$ |
| $Pr^i$ | H | Me | H | H | H | O | - | - | 0 | F | Cl | $SCH_2CO_2Et$ |
| $Pr^i$ | H | Me | H | H | H | O | - | - | 0 | F | Cl | $SCH(Me)CO_2Me$ |
| $Pr^i$ | H | Me | H | H | H | O | - | - | 0 | F | Cl | $SCH(Me)CO_2Et$ |
| $Pr^i$ | H | Me | H | H | H | O | - | - | 0 | F | Cl | $SCH_2CN$ |
| $Pr^i$ | H | Me | H | H | H | O | - | - | 0 | F | Cl | $SCH_2CH=CH_2$ |
| $Pr^i$ | H | Me | H | H | H | O | - | - | 0 | F | Cl | $SCH_2C\equiv CH$ |
| $Pr^i$ | H | Me | H | H | H | O | - | - | 0 | F | Cl | $OCH(Me)C\equiv CH$ |
| $Pr^i$ | H | Me | H | H | H | O | - | - | 0 | F | Cl | $SCH(Me)C\equiv CH$ |
| $Pr^i$ | H | Me | H | H | H | O | - | - | 0 | F | Cl | $N(SO_2Me)_2$ |
| $Pr^i$ | H | Me | H | H | H | O | - | - | 0 | F | Cl | $N(SO_2Et)_2$ |
| $Pr^i$ | H | Me | H | H | H | O | - | - | 0 | F | Cl | $Q1(R29=Bu^t)$ |
| $Pr^i$ | H | Me | H | H | H | O | - | - | 0 | F | Cl | $Q2(R29=Bu^t)$ |
| $Pr^i$ | H | Me | H | H | H | O | - | - | 0 | F | Cl | $Q5(R29=Bu^t)$ |
| $Pr^i$ | H | Me | H | H | H | O | - | - | 0 | F | Cl | $Q7(R34=Bu^t)$ |
| $Pr^i$ | H | Me | H | H | H | O | - | - | 0 | F | Cl | Q9(R38=R39=H) |
| $Pr^i$ | H | Me | H | H | H | O | - | - | 0 | F | Cl | Q10(R38=R39=H) |
| $Pr^i$ | H | Me | H | H | H | O | - | - | 0 | F | Cl | $Q4(R29=Bu^t)$ |
| $Pr^i$ | H | Me | H | H | H | O | - | - | 0 | F | Cl | Q8(R35=R36=R37=H) |
| $Pr^i$ | H | Me | H | H | H | O | - | - | 0 | F | Cl | Q18(R50=H) |
| $Pr^i$ | H | Me | H | H | H | O | - | - | 0 | F | Cl | Q18(R50=Me) |
| $Pr^i$ | H | Me | H | H | H | O | - | - | 0 | F | Cl | $NHCO_2Me$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | H | H | O | - | - | 0 | F | Cl | $NHCO_2Et$ |
| $Pr^i$ | H | Me | H | H | H | O | - | - | 0 | F | Cl | $NHCO_2Pr^n$ |
| $Pr^i$ | H | Me | H | H | H | O | - | - | 0 | F | Cl | $NHCO_2Pr^i$ |
| $Pr^i$ | H | Me | H | H | H | O | - | - | 0 | F | Cl | $NHCO_2Ph$ |
| $Pr^i$ | H | Me | H | H | H | O | - | - | 0 | F | Cl | $NHCO_2Bz$ |
| $Pr^i$ | H | Me | H | H | H | O | - | - | 0 | F | Cl | NHCOMe |
| $Pr^i$ | H | Me | H | H | H | O | - | - | 0 | F | Cl | $NHCO_2CH_2Ph$ |
| $Pr^i$ | H | Me | H | H | H | O | - | - | 0 | F | Cl | $NHCO_2CH_2$(4-Me-Ph) |
| $Pr^i$ | H | Me | H | H | H | O | - | - | 0 | F | Cl | $NHCO_2CH_2$(3-Me-Ph) |
| $Pr^i$ | H | Me | H | H | H | O | - | - | 0 | F | Cl | $NHCO_2CH_2$(2-Me-Ph) |
| $Pr^i$ | H | Me | H | H | H | O | - | - | 0 | F | Cl | $NHCO_2CH_2$(4-Cl-Ph) |
| $Pr^i$ | H | Me | H | H | H | O | - | - | 0 | F | Cl | $NHCO_2CH_2$(3-Cl-Ph) |
| $Pr^i$ | H | Me | H | H | H | O | - | - | 0 | F | Cl | $NHCO_2CH_2$(2-Cl-Ph) |
| $Pr^i$ | H | Me | H | H | H | O | - | - | 0 | F | Cl | Q1(R29=Me) |
| $Pr^i$ | H | Me | H | H | H | O | - | - | 0 | F | Cl | Q2(R29=Me) |
| $Pr^i$ | H | Me | H | H | H | O | - | - | 0 | F | Cl | H |
| $Pr^i$ | H | Me | H | H | H | O | - | - | 0 | F | Cl | $CO_2H$ |
| $Pr^i$ | H | Me | H | H | H | O | - | - | 0 | F | Cl | $CO_2Me$ |
| $Pr^i$ | H | Me | H | H | H | O | - | - | 0 | F | Cl | $CO_2Et$ |
| Me | H | $CF_3$ | H | H | H | O | - | - | 0 | F | Cl | $CO_2Pr^i$ |
| Me | H | $CF_3$ | H | H | H | O | - | - | 0 | F | Cl | $CO_2Pr^n$ |
| Me | H | $CF_3$ | H | H | H | O | - | - | 0 | F | Cl | $CO_2Bu^n$ |
| Me | H | $CF_3$ | H | H | H | O | - | - | 0 | F | Cl | $CO_2Bu^t$ |
| Me | H | $CF_3$ | H | H | H | O | - | - | 0 | F | Cl | $NO_2$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | $CF_3$ | H | H | H | O | - | - | 0 | F | Cl | $NH_2$ |
| Me | H | $CF_3$ | H | H | H | O | - | - | 0 | F | Cl | $NHSO_2Me$ |
| Me | H | $CF_3$ | H | H | H | O | - | - | 0 | F | Cl | $NHSO_2Et$ |
| Me | H | $CF_3$ | H | H | H | O | - | - | 0 | F | Cl | $NHSO_2Pr^n$ |
| Me | H | $CF_3$ | H | H | H | O | - | - | 0 | F | Cl | $NHSO_2Pr^i$ |
| Me | H | $CF_3$ | H | H | H | O | - | - | 0 | F | Cl | OH |
| Me | H | $CF_3$ | H | H | H | O | - | - | 0 | F | Cl | OMe |
| Me | H | $CF_3$ | H | H | H | O | - | - | 0 | F | Cl | OEt |
| Me | H | $CF_3$ | H | H | H | O | - | - | 0 | F | Cl | $OPr^i$ |
| Me | H | $CF_3$ | H | H | H | O | - | - | 0 | F | Cl | $OPr^n$ |
| Me | H | $CF_3$ | H | H | H | O | - | - | 0 | F | Cl | $OPen^c$ |
| Me | H | $CF_3$ | H | H | H | O | - | - | 0 | F | Cl | $OCH_2CH=CH_2$ |
| Me | H | $CF_3$ | H | H | H | O | - | - | 0 | F | Cl | $OCH_2C\equiv CH$ |
| Me | H | $CF_3$ | H | H | H | O | - | - | 0 | F | Cl | $OCH_2CO_2Me$ |
| Me | H | $CF_3$ | H | H | H | O | - | - | 0 | F | Cl | $OCH_2CO_2Et$ |
| Me | H | $CF_3$ | H | H | H | O | - | - | 0 | F | Cl | $OCH(Me)CO_2Me$ |
| Me | H | $CF_3$ | H | H | H | O | - | - | 0 | F | Cl | $OCH(Me)CO_2Et$ |
| Me | H | $CF_3$ | H | H | H | O | - | - | 0 | F | Cl | $CO_2H$ |
| Me | H | $CF_3$ | H | H | H | O | - | - | 0 | F | Cl | CN |
| Me | H | $CF_3$ | H | H | H | O | - | - | 0 | F | Cl | Me |
| Me | H | $CF_3$ | H | H | H | O | - | - | 0 | F | Cl | Et |
| Me | H | $CF_3$ | H | H | H | O | - | - | 0 | F | Cl | Cl |
| Me | H | $CF_3$ | H | H | H | O | - | - | 0 | F | Cl | Br |
| Me | H | $CF_3$ | H | H | H | O | - | - | 0 | F | Cl | I |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | $CF_3$ | H | H | H | O | - | - | 0 | F | Cl | F |
| Me | H | $CF_3$ | H | H | H | O | - | - | 0 | F | Cl | SH |
| Me | H | $CF_3$ | H | H | H | O | - | - | 0 | F | Cl | $C(Me)=NOCH_2CO_2Me$ |
| Me | H | $CF_3$ | H | H | H | O | - | - | 0 | F | Cl | $C(Me)=NOCH_2CO_2Et$ |
| Me | H | $CF_3$ | H | H | H | O | - | - | 0 | F | Cl | $CH=NOCH_2CO_2Me$ |
| Me | H | $CF_3$ | H | H | H | O | - | - | 0 | F | Cl | $CH=NOCH_2CO_2Et$ |
| Me | H | $CF_3$ | H | H | H | O | - | - | 0 | F | Cl | $C(OMe)=NOCH_2CO_2Me$ |
| Me | H | $CF_3$ | H | H | H | O | - | - | 0 | F | Cl | $C(OMe)=NOCH_2CO_2Et$ |
| Me | H | $CF_3$ | H | H | H | O | - | - | 0 | F | Cl | $C(Me)=NN=C(Me)CO_2Me$ |
| Me | H | $CF_3$ | H | H | H | O | - | - | 0 | F | Cl | $C(Me)=NN=C(Me)CO_2Et$ |
| Me | H | $CF_3$ | H | H | H | O | - | - | 0 | F | Cl | $CH_2OCH_2CO_2Me$ |
| Me | H | $CF_3$ | H | H | H | O | - | - | 0 | F | Cl | $CH_2OCH_2CO_2Et$ |
| Me | H | $CF_3$ | H | H | H | O | - | - | 0 | F | Cl | $CH_2OCH(Me)CO_2Me$ |
| Me | H | $CF_3$ | H | H | H | O | - | - | 0 | F | Cl | $CH_2OCH(Me)CO_2Et$ |
| Me | H | $CF_3$ | H | H | H | O | - | - | 0 | F | Cl | $CH_2SCH_2CO_2Me$ |
| Me | H | $CF_3$ | H | H | H | O | - | - | 0 | F | Cl | $CH_2SCH_2CO_2Et$ |
| Me | H | $CF_3$ | H | H | H | O | - | - | 0 | F | Cl | $CH_2SCH(Me)CO_2Me$ |
| Me | H | $CF_3$ | H | H | H | O | - | - | 0 | F | Cl | $CH_2SCH(Me)CO_2Et$ |
| Me | H | $CF_3$ | H | H | H | O | - | - | 0 | F | Cl | SMe |
| Me | H | $CF_3$ | H | H | H | O | - | - | 0 | F | Cl | SEt |
| Me | H | $CF_3$ | H | H | H | O | - | - | 0 | F | Cl | $SPr^i$ |
| Me | H | $CF_3$ | H | H | H | O | - | - | 0 | F | Cl | $SPr^n$ |
| Me | H | $CF_3$ | H | H | H | O | - | - | 0 | F | Cl | $SPen^c$ |
| Me | H | $CF_3$ | H | H | H | O | - | - | 0 | F | Cl | $SCH_2CO_2H$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | $CF_3$ | H | H | H | O | - | - | 0 | F | Cl | $SCH_2CO_2Me$ |
| Me | H | $CF_3$ | H | H | H | O | - | - | 0 | F | Cl | $SCH_2CO_2Et$ |
| Me | H | $CF_3$ | H | H | H | O | - | - | 0 | F | Cl | $SCH(Me)CO_2Me$ |
| Me | H | $CF_3$ | H | H | H | O | - | - | 0 | F | Cl | $SCH(Me)CO_2Et$ |
| Me | H | $CF_3$ | H | H | H | O | - | - | 0 | F | Cl | $SCH_2CN$ |
| Me | H | $CF_3$ | H | H | H | O | - | - | 0 | F | Cl | $SCH_2CH=CH_2$ |
| Me | H | $CF_3$ | H | H | H | O | - | - | 0 | F | Cl | $SCH_2C\equiv CH$ |
| Me | H | $CF_3$ | H | H | H | O | - | - | 0 | F | Cl | $OCH(Me)C\equiv CH$ |
| Me | H | $CF_3$ | H | H | H | O | - | - | 0 | F | Cl | $SCH(Me)C\equiv CH$ |
| Me | H | $CF_3$ | H | H | H | O | - | - | 0 | F | Cl | $N(SO_2Me)_2$ |
| Me | H | $CF_3$ | H | H | H | O | - | - | 0 | F | Cl | $N(SO_2Et)_2$ |
| Me | H | $CF_3$ | H | H | H | O | - | - | 0 | F | Cl | Q1(R29=$Bu^t$) |
| Me | H | $CF_3$ | H | H | H | O | - | - | 0 | F | Cl | Q2(R29=$Bu^t$) |
| Me | H | $CF_3$ | H | H | H | O | - | - | 0 | F | Cl | Q5(R29=$Bu^t$) |
| Me | H | $CF_3$ | H | H | H | O | - | - | 0 | F | Cl | Q7(R34=$Bu^t$) |
| Me | H | $CF_3$ | H | H | H | O | - | - | 0 | F | Cl | Q9(R38=R39=H) |
| Me | H | $CF_3$ | H | H | H | O | - | - | 0 | F | Cl | Q10(R38=R39=H) |
| Me | H | $CF_3$ | H | H | H | O | - | - | 0 | F | Cl | Q4(R29=$Bu^t$) |
| Me | H | $CF_3$ | H | H | H | O | - | - | 0 | F | Cl | Q8(R35=R36=R37=H) |
| Me | H | $CF_3$ | H | H | H | O | - | - | 0 | F | Cl | Q18(R50=H) |
| Me | H | $CF_3$ | H | H | H | O | - | - | 0 | F | Cl | Q18(R50=Me) |
| Me | H | $CF_3$ | H | H | H | O | - | - | 0 | F | Cl | $NHCO_2Me$ |
| Me | H | $CF_3$ | H | H | H | O | - | - | 0 | F | Cl | $NHCO_2Et$ |
| Me | H | $CF_3$ | H | H | H | O | - | - | 0 | F | Cl | $NHCO_2Pr^n$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | $CF_3$ | H | H | H | O | - | - | 0 | F | Cl | $NHCO_2Pr^i$ |
| Me | H | $CF_3$ | H | H | H | O | - | - | 0 | F | Cl | $NHCO_2Ph$ |
| Me | H | $CF_3$ | H | H | H | O | - | - | 0 | F | Cl | $NHCO_2Bz$ |
| Me | H | $CF_3$ | H | H | H | O | - | - | 0 | F | Cl | NHCOMe |
| Me | H | $CF_3$ | H | H | H | O | - | - | 0 | F | Cl | $NHCO_2CH_2Ph$ |
| Me | H | $CF_3$ | H | H | H | O | - | - | 0 | F | Cl | $NHCO_2CH_2$(4-Me-Ph) |
| Me | H | $CF_3$ | H | H | H | O | - | - | 0 | F | Cl | $NHCO_2CH_2$(3-Me-Ph) |
| Me | H | $CF_3$ | H | H | H | O | - | - | 0 | F | Cl | $NHCO_2CH_2$(2-Me-Ph) |
| Me | H | $CF_3$ | H | H | H | O | - | - | 0 | F | Cl | $NHCO_2CH_2$(4-Cl-Ph) |
| Me | H | $CF_3$ | H | H | H | O | - | - | 0 | F | Cl | $NHCO_2CH_2$(3-Cl-Ph) |
| Me | H | $CF_3$ | H | H | H | O | - | - | 0 | F | Cl | $NHCO_2CH_2$(2-Cl-Ph) |
| Me | H | $CF_3$ | H | H | H | O | - | - | 0 | F | Cl | Q1(R29=Me) |
| Me | H | $CF_3$ | H | H | H | O | - | - | 0 | F | Cl | Q2(R29=Me) |
| Me | H | $CF_3$ | H | H | H | O | - | - | 0 | F | Cl | H |
| Me | H | $CF_3$ | H | H | H | O | - | - | 0 | F | Cl | $CO_2H$ |
| Me | H | $CF_3$ | H | H | H | O | - | - | 0 | F | Cl | $CO_2Me$ |
| Me | H | $CF_3$ | H | H | H | O | - | - | 0 | F | Cl | $CO_2Et$ |
| Et | H | $CF_3$ | H | H | H | O | - | - | 0 | F | Cl | $CO_2Pr^i$ |
| Et | H | $CF_3$ | H | H | H | O | - | - | 0 | F | Cl | $CO_2Pr^n$ |
| Et | H | $CF_3$ | H | H | H | O | - | - | 0 | F | Cl | $CO_2Bu^n$ |
| Et | H | $CF_3$ | H | H | H | O | - | - | 0 | F | Cl | $CO_2Bu^t$ |
| Et | H | $CF_3$ | H | H | H | O | - | - | 0 | F | Cl | $NO_2$ |
| Et | H | $CF_3$ | H | H | H | O | - | - | 0 | F | Cl | $NH_2$ |
| Et | H | $CF_3$ | H | H | H | O | - | - | 0 | F | Cl | $NHSO_2Me$ |

Table 2-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | $CF_3$ | H | H | H | O | - | - | 0 | F | Cl | $NHSO_2Et$ |
| Et | H | $CF_3$ | H | H | H | O | - | - | 0 | F | Cl | $NHSO_2Pr^n$ |
| Et | H | $CF_3$ | H | H | H | O | - | - | 0 | F | Cl | $NHSO_2Pr^i$ |
| Et | H | $CF_3$ | H | H | H | O | - | - | 0 | F | Cl | OH |
| Et | H | $CF_3$ | H | H | H | O | - | - | 0 | F | Cl | OMe |
| Et | H | $CF_3$ | H | H | H | O | - | - | 0 | F | Cl | OEt |
| Et | H | $CF_3$ | H | H | H | O | - | - | 0 | F | Cl | $OPr^i$ |
| Et | H | $CF_3$ | H | H | H | O | - | - | 0 | F | Cl | $OPr^n$ |
| Et | H | $CF_3$ | H | H | H | O | - | - | 0 | F | Cl | $OPen^c$ |
| Et | H | $CF_3$ | H | H | H | O | - | - | 0 | F | Cl | $OCH_2CH=CH_2$ |
| Et | H | $CF_3$ | H | H | H | O | - | - | 0 | F | Cl | $OCH_2C\equiv CH$ |
| Et | H | $CF_3$ | H | H | H | O | - | - | 0 | F | Cl | $OCH_2CO_2Me$ |
| Et | H | $CF_3$ | H | H | H | O | - | - | 0 | F | Cl | $OCH_2CO_2Et$ |
| Et | H | $CF_3$ | H | H | H | O | - | - | 0 | F | Cl | $OCH(Me)CO_2Me$ |
| Et | H | $CF_3$ | H | H | H | O | - | - | 0 | F | Cl | $OCH(Me)CO_2Et$ |
| Et | H | $CF_3$ | H | H | H | O | - | - | 0 | F | Cl | $CO_2H$ |
| Et | H | $CF_3$ | H | H | H | O | - | - | 0 | F | Cl | CN |
| Et | H | $CF_3$ | H | H | H | O | - | - | 0 | F | Cl | Me |
| Et | H | $CF_3$ | H | H | H | O | - | - | 0 | F | Cl | Et |
| Et | H | $CF_3$ | H | H | H | O | - | - | 0 | F | Cl | Cl |
| Et | H | $CF_3$ | H | H | H | O | - | - | 0 | F | Cl | Br |
| Et | H | $CF_3$ | H | H | H | O | - | - | 0 | F | Cl | I |
| Et | H | $CF_3$ | H | H | H | O | - | - | 0 | F | Cl | F |
| Et | H | $CF_3$ | H | H | H | O | - | - | 0 | F | Cl | SH |

Table 2-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | $CF_3$ | H | H | H | O | - | - | 0 | F | Cl | $C(Me)=NOCH_2CO_2Me$ |
| Et | H | $CF_3$ | H | H | H | O | - | - | 0 | F | Cl | $C(Me)=NOCH_2CO_2Et$ |
| Et | H | $CF_3$ | H | H | H | O | - | - | 0 | F | Cl | $CH=NOCH_2CO_2Me$ |
| Et | H | $CF_3$ | H | H | H | O | - | - | 0 | F | Cl | $CH=NOCH_2CO_2Et$ |
| Et | H | $CF_3$ | H | H | H | O | - | - | 0 | F | Cl | $C(OMe)=NOCH_2CO_2Me$ |
| Et | H | $CF_3$ | H | H | H | O | - | - | 0 | F | Cl | $C(OMe)=NOCH_2CO_2Et$ |
| Et | H | $CF_3$ | H | H | H | O | - | - | 0 | F | Cl | $C(Me)=NN=C(Me)CO_2Me$ |
| Et | H | $CF_3$ | H | H | H | O | - | - | 0 | F | Cl | $C(Me)=NN=C(Me)CO_2Et$ |
| Et | H | $CF_3$ | H | H | H | O | - | - | 0 | F | Cl | $CH_2OCH_2CO_2Me$ |
| Et | H | $CF_3$ | H | H | H | O | - | - | 0 | F | Cl | $CH_2OCH_2CO_2Et$ |
| Et | H | $CF_3$ | H | H | H | O | - | - | 0 | F | Cl | $CH_2OCH(Me)CO_2Me$ |
| Et | H | $CF_3$ | H | H | H | O | - | - | 0 | F | Cl | $CH_2OCH(Me)CO_2Et$ |
| Et | H | $CF_3$ | H | H | H | O | - | - | 0 | F | Cl | $CH_2SCH_2CO_2Me$ |
| Et | H | $CF_3$ | H | H | H | O | - | - | 0 | F | Cl | $CH_2SCH_2CO_2Et$ |
| Et | H | $CF_3$ | H | H | H | O | - | - | 0 | F | Cl | $CH_2SCH(Me)CO_2Me$ |
| Et | H | $CF_3$ | H | H | H | O | - | - | 0 | F | Cl | $CH_2SCH(Me)CO_2Et$ |
| Et | H | $CF_3$ | H | H | H | O | - | - | 0 | F | Cl | SMe |
| Et | H | $CF_3$ | H | H | H | O | - | - | 0 | F | Cl | SEt |
| Et | H | $CF_3$ | H | H | H | O | - | - | 0 | F | Cl | $SPr^i$ |
| Et | H | $CF_3$ | H | H | H | O | - | - | 0 | F | Cl | $SPr^n$ |
| Et | H | $CF_3$ | H | H | H | O | - | - | 0 | F | Cl | $SPen^c$ |
| Et | H | $CF_3$ | H | H | H | O | - | - | 0 | F | Cl | $SCH_2CO_2H$ |
| Et | H | $CF_3$ | H | H | H | O | - | - | 0 | F | Cl | $SCH_2CO_2Me$ |
| Et | H | $CF_3$ | H | H | H | O | - | - | 0 | F | Cl | $SCH_2CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | $CF_3$ | H | H | H | 0 | - | - | 0 | F | Cl | $SCH(Me)CO_2Me$ |
| Et | H | $CF_3$ | H | H | H | 0 | - | - | 0 | F | Cl | $SCH(Me)CO_2Et$ |
| Et | H | $CF_3$ | H | H | H | 0 | - | - | 0 | F | Cl | $SCH_2CN$ |
| Et | H | $CF_3$ | H | H | H | 0 | - | - | 0 | F | Cl | $SCH_2CH=CH_2$ |
| Et | H | $CF_3$ | H | H | H | 0 | - | - | 0 | F | Cl | $SCH_2C\equiv CH$ |
| Et | H | $CF_3$ | H | H | H | 0 | - | - | 0 | F | Cl | $OCH(Me)C\equiv CH$ |
| Et | H | $CF_3$ | H | H | H | 0 | - | - | 0 | F | Cl | $SCH(Me)C\equiv CH$ |
| Et | H | $CF_3$ | H | H | H | 0 | - | - | 0 | F | Cl | $N(SO_2Me)_2$ |
| Et | H | $CF_3$ | H | H | H | 0 | - | - | 0 | F | Cl | $N(SO_2Et)_2$ |
| Et | H | $CF_3$ | H | H | H | 0 | - | - | 0 | F | Cl | Q1(R29=$Bu^t$) |
| Et | H | $CF_3$ | H | H | H | 0 | - | - | 0 | F | Cl | Q2(R29=$Bu^t$) |
| Et | H | $CF_3$ | H | H | H | 0 | - | - | 0 | F | Cl | Q5(R29=$Bu^t$) |
| Et | H | $CF_3$ | H | H | H | 0 | - | - | 0 | F | Cl | Q7(R34=$Bu^t$) |
| Et | H | $CF_3$ | H | H | H | 0 | - | - | 0 | F | Cl | Q9(R38=R39=H) |
| Et | H | $CF_3$ | H | H | H | 0 | - | - | 0 | F | Cl | Q10(R38=R39=H) |
| Et | H | $CF_3$ | H | H | H | 0 | - | - | 0 | F | Cl | Q4(R29=$Bu^t$) |
| Et | H | $CF_3$ | H | H | H | 0 | - | - | 0 | F | Cl | Q8(R35=R36=R37=H) |
| Et | H | $CF_3$ | H | H | H | 0 | - | - | 0 | F | Cl | Q18(R50=H) |
| Et | H | $CF_3$ | H | H | H | 0 | - | - | 0 | F | Cl | Q18(R50=Me) |
| Et | H | $CF_3$ | H | H | H | 0 | - | - | 0 | F | Cl | $NHCO_2Me$ |
| Et | H | $CF_3$ | H | H | H | 0 | - | - | 0 | F | Cl | $NHCO_2Et$ |
| Et | H | $CF_3$ | H | H | H | 0 | - | - | 0 | F | Cl | $NHCO_2Pr^n$ |
| Et | H | $CF_3$ | H | H | H | 0 | - | - | 0 | F | Cl | $NHCO_2Pr^i$ |
| Et | H | $CF_3$ | H | H | H | 0 | - | - | 0 | F | Cl | $NHCO_2Ph$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | $CF_3$ | H | H | H | O | - | - | 0 | F | Cl | $NHCO_2Bz$ |
| Et | H | $CF_3$ | H | H | H | O | - | - | 0 | F | Cl | NHCOMe |
| Et | H | $CF_3$ | H | H | H | O | - | - | 0 | F | Cl | $NHCO_2CH_2Ph$ |
| Et | H | $CF_3$ | H | H | H | O | - | - | 0 | F | Cl | $NHCO_2CH_2$(4-Me-Ph) |
| Et | H | $CF_3$ | H | H | H | O | - | - | 0 | F | Cl | $NHCO_2CH_2$(3-Me-Ph) |
| Et | H | $CF_3$ | H | H | H | O | - | - | 0 | F | Cl | $NHCO_2CH_2$(2-Me-Ph) |
| Et | H | $CF_3$ | H | H | H | O | - | - | 0 | F | Cl | $NHCO_2CH_2$(4-Cl-Ph) |
| Et | H | $CF_3$ | H | H | H | O | - | - | 0 | F | Cl | $NHCO_2CH_2$(3-Cl-Ph) |
| Et | H | $CF_3$ | H | H | H | O | - | - | 0 | F | Cl | $NHCO_2CH_2$(2-Cl-Ph) |
| Et | H | $CF_3$ | H | H | H | O | - | - | 0 | F | Cl | Q1(R29=Me) |
| Et | H | $CF_3$ | H | H | H | O | - | - | 0 | F | Cl | Q2(R29=Me) |
| Et | H | $CF_3$ | H | H | H | O | - | - | 0 | F | Cl | H |
| Et | H | $CF_3$ | H | H | H | O | - | - | 0 | F | Cl | $CO_2H$ |
| Et | H | $CF_3$ | H | H | H | O | - | - | 0 | F | Cl | $CO_2Me$ |
| Et | H | $CF_3$ | H | H | H | O | - | - | 0 | F | Cl | $CO_2Et$ |
| $Pr^n$ | H | $CF_3$ | H | H | H | O | - | - | 0 | F | Cl | $CO_2Pr^i$ |
| $Pr^n$ | H | $CF_3$ | H | H | H | O | - | - | 0 | F | Cl | $CO_2Pr^n$ |
| $Pr^n$ | H | $CF_3$ | H | H | H | O | - | - | 0 | F | Cl | $CO_2Bu^n$ |
| $Pr^n$ | H | $CF_3$ | H | H | H | O | - | - | 0 | F | Cl | $CO_2Bu^t$ |
| $Pr^n$ | H | $CF_3$ | H | H | H | O | - | - | 0 | F | Cl | $NO_2$ |
| $Pr^n$ | H | $CF_3$ | H | H | H | O | - | - | 0 | F | Cl | $NH_2$ |
| $Pr^n$ | H | $CF_3$ | H | H | H | O | - | - | 0 | F | Cl | $NHSO_2Me$ |
| $Pr^n$ | H | $CF_3$ | H | H | H | O | - | - | 0 | F | Cl | $NHSO_2Et$ |
| $Pr^n$ | H | $CF_3$ | H | H | H | O | - | - | 0 | F | Cl | $NHSO_2Pr^n$ |

Table 2-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | $CF_3$ | H | H | H | O | - | - | 0 | F | Cl | $NHSO_2Pr^i$ |
| $Pr^n$ | H | $CF_3$ | H | H | H | O | - | - | 0 | F | Cl | OH |
| $Pr^n$ | H | $CF_3$ | H | H | H | O | - | - | 0 | F | Cl | OMe |
| $Pr^n$ | H | $CF_3$ | H | H | H | O | - | - | 0 | F | Cl | OEt |
| $Pr^n$ | H | $CF_3$ | H | H | H | O | - | - | 0 | F | Cl | $OPr^i$ |
| $Pr^n$ | H | $CF_3$ | H | H | H | O | - | - | 0 | F | Cl | $OPr^n$ |
| $Pr^n$ | H | $CF_3$ | H | H | H | O | - | - | 0 | F | Cl | $OPen^c$ |
| $Pr^n$ | H | $CF_3$ | H | H | H | O | - | - | 0 | F | Cl | $OCH_2CH=CH_2$ |
| $Pr^n$ | H | $CF_3$ | H | H | H | O | - | - | 0 | F | Cl | $OCH_2C\equiv CH$ |
| $Pr^n$ | H | $CF_3$ | H | H | H | O | - | - | 0 | F | Cl | $OCH_2CO_2Me$ |
| $Pr^n$ | H | $CF_3$ | H | H | H | O | - | - | 0 | F | Cl | $OCH_2CO_2Et$ |
| $Pr^n$ | H | $CF_3$ | H | H | H | O | - | - | 0 | F | Cl | $OCH(Me)CO_2Me$ |
| $Pr^n$ | H | $CF_3$ | H | H | H | O | - | - | 0 | F | Cl | $OCH(Me)CO_2Et$ |
| $Pr^n$ | H | $CF_3$ | H | H | H | O | - | - | 0 | F | Cl | $CO_2H$ |
| $Pr^n$ | H | $CF_3$ | H | H | H | O | - | - | 0 | F | Cl | CN |
| $Pr^n$ | H | $CF_3$ | H | H | H | O | - | - | 0 | F | Cl | Me |
| $Pr^n$ | H | $CF_3$ | H | H | H | O | - | - | 0 | F | Cl | Et |
| $Pr^n$ | H | $CF_3$ | H | H | H | O | - | - | 0 | F | Cl | CL |
| $Pr^n$ | H | $CF_3$ | H | H | H | O | - | - | 0 | F | Cl | Br |
| $Pr^n$ | H | $CF_3$ | H | H | H | O | - | - | 0 | F | Cl | I |
| $Pr^n$ | H | $CF_3$ | H | H | H | O | - | - | 0 | F | Cl | F |
| $Pr^n$ | H | $CF_3$ | H | H | H | O | - | - | 0 | F | Cl | SH |
| $Pr^n$ | H | $CF_3$ | H | H | H | O | - | - | 0 | F | Cl | $C(Me)=NOCH_2CO_2Me$ |
| $Pr^n$ | H | $CF_3$ | H | H | H | O | - | - | 0 | F | Cl | $C(Me)=NOCH_2CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | $CF_3$ | H | H | H | O | - | - | 0 | F | Cl | $CH=NOCH_2CO_2Me$ |
| $Pr^n$ | H | $CF_3$ | H | H | H | O | - | - | 0 | F | Cl | $CH=NOCH_2CO_2Et$ |
| $Pr^n$ | H | $CF_3$ | H | H | H | O | - | - | 0 | F | Cl | $C(OMe)=NOCH_2CO_2Me$ |
| $Pr^n$ | H | $CF_3$ | H | H | H | O | - | - | 0 | F | Cl | $C(OMe)=NOCH_2CO_2Et$ |
| $Pr^n$ | H | $CF_3$ | H | H | H | O | - | - | 0 | F | Cl | $C(Me)=NN=C(Me)CO_2Me$ |
| $Pr^n$ | H | $CF_3$ | H | H | H | O | - | - | 0 | F | Cl | $C(Me)=NN=C(Me)CO_2Et$ |
| $Pr^n$ | H | $CF_3$ | H | H | H | O | - | - | 0 | F | Cl | $CH_2OCH_2CO_2Me$ |
| $Pr^n$ | H | $CF_3$ | H | H | H | O | - | - | 0 | F | Cl | $CH_2OCH_2CO_2Et$ |
| $Pr^n$ | H | $CF_3$ | H | H | H | O | - | - | 0 | F | Cl | $CH_2OCH(Me)CO_2Me$ |
| $Pr^n$ | H | $CF_3$ | H | H | H | O | - | - | 0 | F | Cl | $CH_2OCH(Me)CO_2Et$ |
| $Pr^n$ | H | $CF_3$ | H | H | H | O | - | - | 0 | F | Cl | $CH_2SCH_2CO_2Me$ |
| $Pr^n$ | H | $CF_3$ | H | H | H | O | - | - | 0 | F | Cl | $CH_2SCH_2CO_2Et$ |
| $Pr^n$ | H | $CF_3$ | H | H | H | O | - | - | 0 | F | Cl | $CH_2SCH(Me)CO_2Me$ |
| $Pr^n$ | H | $CF_3$ | H | H | H | O | - | - | 0 | F | Cl | $CH_2SCH(Me)CO_2Et$ |
| $Pr^n$ | H | $CF_3$ | H | H | H | O | - | - | 0 | F | Cl | SMe |
| $Pr^n$ | H | $CF_3$ | H | H | H | O | - | - | 0 | F | Cl | SEt |
| $Pr^n$ | H | $CF_3$ | H | H | H | O | - | - | 0 | F | Cl | $SPr^i$ |
| $Pr^n$ | H | $CF_3$ | H | H | H | O | - | - | 0 | F | Cl | $SPr^n$ |
| $Pr^n$ | H | $CF_3$ | H | H | H | O | - | - | 0 | F | Cl | $SPen^c$ |
| $Pr^n$ | H | $CF_3$ | H | H | H | O | - | - | 0 | F | Cl | $SCH_2CO_2H$ |
| $Pr^n$ | H | $CF_3$ | H | H | H | O | - | - | 0 | F | Cl | $SCH_2CO_2Me$ |
| $Pr^n$ | H | $CF_3$ | H | H | H | O | - | - | 0 | F | Cl | $SCH_2CO_2Et$ |
| $Pr^n$ | H | $CF_3$ | H | H | H | O | - | - | 0 | F | Cl | $SCH(Me)CO_2Me$ |
| $Pr^n$ | H | $CF_3$ | H | H | H | O | - | - | 0 | F | Cl | $SCH(Me)CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | $CF_3$ | H | H | H | O | - | - | 0 | F | Cl | $SCH_2CN$ |
| $Pr^n$ | H | $CF_3$ | H | H | H | O | - | - | 0 | F | Cl | $SCH_2CH=CH_2$ |
| $Pr^n$ | H | $CF_3$ | H | H | H | O | - | - | 0 | F | Cl | $SCH_2C\equiv CH$ |
| $Pr^n$ | H | $CF_3$ | H | H | H | O | - | - | 0 | F | Cl | $OCH(Me)C\equiv CH$ |
| $Pr^n$ | H | $CF_3$ | H | H | H | O | - | - | 0 | F | Cl | $SCH(Me)C\equiv CH$ |
| $Pr^n$ | H | $CF_3$ | H | H | H | O | - | - | 0 | F | Cl | $N(SO_2Me)_2$ |
| $Pr^n$ | H | $CF_3$ | H | H | H | O | - | - | 0 | F | Cl | $N(SO_2Et)_2$ |
| $Pr^n$ | H | $CF_3$ | H | H | H | O | - | - | 0 | F | Cl | Q1(R29=$Bu^t$) |
| $Pr^n$ | H | $CF_3$ | H | H | H | O | - | - | 0 | F | Cl | Q2(R29=$Bu^t$) |
| $Pr^n$ | H | $CF_3$ | H | H | H | O | - | - | 0 | F | Cl | Q5(R29=$Bu^t$) |
| $Pr^n$ | H | $CF_3$ | H | H | H | O | - | - | 0 | F | Cl | Q7(R34=$Bu^t$) |
| $Pr^n$ | H | $CF_3$ | H | H | H | O | - | - | 0 | F | Cl | Q9(R38=R39=H) |
| $Pr^n$ | H | $CF_3$ | H | H | H | O | - | - | 0 | F | Cl | Q10(R38=R39=H) |
| $Pr^n$ | H | $CF_3$ | H | H | H | O | - | - | 0 | F | Cl | Q4(R29=$Bu^t$) |
| $Pr^n$ | H | $CF_3$ | H | H | H | O | - | - | 0 | F | Cl | Q8(R35=R36=R37=H) |
| $Pr^n$ | H | $CF_3$ | H | H | H | O | - | - | 0 | F | Cl | Q18(R50=H) |
| $Pr^n$ | H | $CF_3$ | H | H | H | O | - | - | 0 | F | Cl | Q18(R50=Me) |
| $Pr^n$ | H | $CF_3$ | H | H | H | O | - | - | 0 | F | Cl | $NHCO_2Me$ |
| $Pr^n$ | H | $CF_3$ | H | H | H | O | - | - | 0 | F | Cl | $NHCO_2Et$ |
| $Pr^n$ | H | $CF_3$ | H | H | H | O | - | - | 0 | F | Cl | $NHCO_2Pr^n$ |
| $Pr^n$ | H | $CF_3$ | H | H | H | O | - | - | 0 | F | Cl | $NHCO_2Pr^i$ |
| $Pr^n$ | H | $CF_3$ | H | H | H | O | - | - | 0 | F | Cl | $NHCO_2Ph$ |
| $Pr^n$ | H | $CF_3$ | H | H | H | O | - | - | 0 | F | Cl | $NHCO_2Bz$ |
| $Pr^n$ | H | $CF_3$ | H | H | H | O | - | - | 0 | F | Cl | NHCOMe |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | $CF_3$ | H | H | H | O | - | - | 0 | F | Cl | $NHCO_2CH_2Ph$ |
| $Pr^n$ | H | $CF_3$ | H | H | H | O | - | - | 0 | F | Cl | $NHCO_2CH_2$(4-Me-Ph) |
| $Pr^n$ | H | $CF_3$ | H | H | H | O | - | - | 0 | F | Cl | $NHCO_2CH_2$(3-Me-Ph) |
| $Pr^n$ | H | $CF_3$ | H | H | H | O | - | - | 0 | F | Cl | $NHCO_2CH_2$(2-Me-Ph) |
| $Pr^n$ | H | $CF_3$ | H | H | H | O | - | - | 0 | F | Cl | $NHCO_2CH_2$(4-CL-Ph) |
| $Pr^n$ | H | $CF_3$ | H | H | H | O | - | - | 0 | F | Cl | $NHCO_2CH_2$(3-CL-Ph) |
| $Pr^n$ | H | $CF_3$ | H | H | H | O | - | - | 0 | F | Cl | $NHCO_2CH_2$(2-CL-Ph) |
| $Pr^n$ | H | $CF_3$ | H | H | H | O | - | - | 0 | F | Cl | Q1(R29=Me) |
| $Pr^n$ | H | $CF_3$ | H | H | H | O | - | - | 0 | F | Cl | Q2(R29=Me) |
| $Pr^n$ | H | $CF_3$ | H | H | H | O | - | - | 0 | F | Cl | H |
| $Pr^n$ | H | $CF_3$ | H | H | H | O | - | - | 0 | F | Cl | $CO_2H$ |
| $Pr^n$ | H | $CF_3$ | H | H | H | O | - | - | 0 | F | Cl | $CO_2Me$ |
| $Pr^n$ | H | $CF_3$ | H | H | H | O | - | - | 0 | F | Cl | $CO_2Et$ |
| $Pr^i$ | H | $CF_3$ | H | H | H | O | - | - | 0 | F | Cl | $CO_2Pr^i$ |
| $Pr^i$ | H | $CF_3$ | H | H | H | O | - | - | 0 | F | Cl | $CO_2Pr^n$ |
| $Pr^i$ | H | $CF_3$ | H | H | H | O | - | - | 0 | F | Cl | $CO_2Bu^n$ |
| $Pr^i$ | H | $CF_3$ | H | H | H | O | - | - | 0 | F | Cl | $CO_2Bu^t$ |
| $Pr^i$ | H | $CF_3$ | H | H | H | O | - | - | 0 | F | Cl | $NO_2$ |
| $Pr^i$ | H | $CF_3$ | H | H | H | O | - | - | 0 | F | Cl | $NH_2$ |
| $Pr^i$ | H | $CF_3$ | H | H | H | O | - | - | 0 | F | Cl | $NHSO_2Me$ |
| $Pr^i$ | H | $CF_3$ | H | H | H | O | - | - | 0 | F | Cl | $NHSO_2Et$ |
| $Pr^i$ | H | $CF_3$ | H | H | H | O | - | - | 0 | F | Cl | $NHSO_2Pr^n$ |
| $Pr^i$ | H | $CF_3$ | H | H | H | O | - | - | 0 | F | Cl | $NHSO_2Pr^i$ |
| $Pr^i$ | H | $CF_3$ | H | H | H | O | - | - | 0 | F | Cl | OH |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | $CF_3$ | H | H | H | O | - | - | 0 | F | Cl | OMe |
| $Pr^i$ | H | $CF_3$ | H | H | H | O | - | - | 0 | F | Cl | OEt |
| $Pr^i$ | H | $CF_3$ | H | H | H | O | - | - | 0 | F | Cl | $OPr^i$ |
| $Pr^i$ | H | $CF_3$ | H | H | H | O | - | - | 0 | F | Cl | $OPr^n$ |
| $Pr^i$ | H | $CF_3$ | H | H | H | O | - | - | 0 | F | Cl | $OPen^c$ |
| $Pr^i$ | H | $CF_3$ | H | H | H | O | - | - | 0 | F | Cl | $OCH_2CH=CH_2$ |
| $Pr^i$ | H | $CF_3$ | H | H | H | O | - | - | 0 | F | Cl | $OCH_2C\equiv CH$ |
| $Pr^i$ | H | $CF_3$ | H | H | H | O | - | - | 0 | F | Cl | $OCH_2CO_2Me$ |
| $Pr^i$ | H | $CF_3$ | H | H | H | O | - | - | 0 | F | Cl | $OCH_2CO_2Et$ |
| $Pr^i$ | H | $CF_3$ | H | H | H | O | - | - | 0 | F | Cl | $OCH(Me)CO_2Me$ |
| $Pr^i$ | H | $CF_3$ | H | H | H | O | - | - | 0 | F | Cl | $OCH(Me)CO_2Et$ |
| $Pr^i$ | H | $CF_3$ | H | H | H | O | - | - | 0 | F | Cl | $CO_2H$ |
| $Pr^i$ | H | $CF_3$ | H | H | H | O | - | - | 0 | F | Cl | CN |
| $Pr^i$ | H | $CF_3$ | H | H | H | O | - | - | 0 | F | Cl | Me |
| $Pr^i$ | H | $CF_3$ | H | H | H | O | - | - | 0 | F | Cl | Et |
| $Pr^i$ | H | $CF_3$ | H | H | H | O | - | - | 0 | F | Cl | CL |
| $Pr^i$ | H | $CF_3$ | H | H | H | O | - | - | 0 | F | Cl | Br |
| $Pr^i$ | H | $CF_3$ | H | H | H | O | - | - | 0 | F | Cl | I |
| $Pr^i$ | H | $CF_3$ | H | H | H | O | - | - | 0 | F | Cl | F |
| $Pr^i$ | H | $CF_3$ | H | H | H | O | - | - | 0 | F | Cl | SH |
| $Pr^i$ | H | $CF_3$ | H | H | H | O | - | - | 0 | F | Cl | $C(Me)=NOCH_2CO_2Me$ |
| $Pr^i$ | H | $CF_3$ | H | H | H | O | - | - | 0 | F | Cl | $C(Me)=NOCH_2CO_2Et$ |
| $Pr^i$ | H | $CF_3$ | H | H | H | O | - | - | 0 | F | Cl | $CH=NOCH_2CO_2Me$ |
| $Pr^i$ | H | $CF_3$ | H | H | H | O | - | - | 0 | F | Cl | $CH=NOCH_2CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | $CF_3$ | H | H | H | O | - | - | 0 | F | Cl | $C(OMe)=NOCH_2CO_2Me$ |
| $Pr^i$ | H | $CF_3$ | H | H | H | O | - | - | 0 | F | Cl | $C(OMe)=NOCH_2CO_2Et$ |
| $Pr^i$ | H | $CF_3$ | H | H | H | O | - | - | 0 | F | Cl | $C(Me)=NN=C(Me)CO_2Me$ |
| $Pr^i$ | H | $CF_3$ | H | H | H | O | - | - | 0 | F | Cl | $C(Me)=NN=C(Me)CO_2Et$ |
| $Pr^i$ | H | $CF_3$ | H | H | H | O | - | - | 0 | F | Cl | $CH_2OCH_2CO_2Me$ |
| $Pr^i$ | H | $CF_3$ | H | H | H | O | - | - | 0 | F | Cl | $CH_2OCH_2CO_2Et$ |
| $Pr^i$ | H | $CF_3$ | H | H | H | O | - | - | 0 | F | Cl | $CH_2OCH(Me)CO_2Me$ |
| $Pr^i$ | H | $CF_3$ | H | H | H | O | - | - | 0 | F | Cl | $CH_2OCH(Me)CO_2Et$ |
| $Pr^i$ | H | $CF_3$ | H | H | H | O | - | - | 0 | F | Cl | $CH_2SCH_2CO_2Me$ |
| $Pr^i$ | H | $CF_3$ | H | H | H | O | - | - | 0 | F | Cl | $CH_2SCH_2CO_2Et$ |
| $Pr^i$ | H | $CF_3$ | H | H | H | O | - | - | 0 | F | Cl | $CH_2SCH(Me)CO_2Me$ |
| $Pr^i$ | H | $CF_3$ | H | H | H | O | - | - | 0 | F | Cl | $CH_2SCH(Me)CO_2Et$ |
| $Pr^i$ | H | $CF_3$ | H | H | H | O | - | - | 0 | F | Cl | SMe |
| $Pr^i$ | H | $CF_3$ | H | H | H | O | - | - | 0 | F | Cl | SEt |
| $Pr^i$ | H | $CF_3$ | H | H | H | O | - | - | 0 | F | Cl | $SPr^i$ |
| $Pr^i$ | H | $CF_3$ | H | H | H | O | - | - | 0 | F | Cl | $SPr^n$ |
| $Pr^i$ | H | $CF_3$ | H | H | H | O | - | - | 0 | F | Cl | $SPen^c$ |
| $Pr^i$ | H | $CF_3$ | H | H | H | O | - | - | 0 | F | Cl | $SCH_2CO_2H$ |
| $Pr^i$ | H | $CF_3$ | H | H | H | O | - | - | 0 | F | Cl | $SCH_2CO_2Me$ |
| $Pr^i$ | H | $CF_3$ | H | H | H | O | - | - | 0 | F | Cl | $SCH_2CO_2Et$ |
| $Pr^i$ | H | $CF_3$ | H | H | H | O | - | - | 0 | F | Cl | $SCH(Me)CO_2Me$ |
| $Pr^i$ | H | $CF_3$ | H | H | H | O | - | - | 0 | F | Cl | $SCH(Me)CO_2Et$ |
| $Pr^i$ | H | $CF_3$ | H | H | H | O | - | - | 0 | F | Cl | $SCH_2CN$ |
| $Pr^i$ | H | $CF_3$ | H | H | H | O | - | - | 0 | F | Cl | $SCH_2CH=CH_2$ |

Table 2-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | $CF_3$ | H | H | H | O | - | - | 0 | F | Cl | $SCH_2C{\equiv}CH$ |
| $Pr^i$ | H | $CF_3$ | H | H | H | O | - | - | 0 | F | Cl | $OCH(Me)C{\equiv}CH$ |
| $Pr^i$ | H | $CF_3$ | H | H | H | O | - | - | 0 | F | Cl | $SCH(Me)C{\equiv}CH$ |
| $Pr^i$ | H | $CF_3$ | H | H | H | O | - | - | 0 | F | Cl | $N(SO_2Me)_2$ |
| $Pr^i$ | H | $CF_3$ | H | H | H | O | - | - | 0 | F | Cl | $N(SO_2Et)_2$ |
| $Pr^i$ | H | $CF_3$ | H | H | H | O | - | - | 0 | F | Cl | Q1(R29=$Bu^t$) |
| $Pr^i$ | H | $CF_3$ | H | H | H | O | - | - | 0 | F | Cl | Q2(R29=$Bu^t$) |
| $Pr^i$ | H | $CF_3$ | H | H | H | O | - | - | 0 | F | Cl | Q5(R29=$Bu^t$) |
| $Pr^i$ | H | $CF_3$ | H | H | H | O | - | - | 0 | F | Cl | Q7(R34=$Bu^t$) |
| $Pr^i$ | H | $CF_3$ | H | H | H | O | - | - | 0 | F | Cl | Q9(R38=R39=H) |
| $Pr^i$ | H | $CF_3$ | H | H | H | O | - | - | 0 | F | Cl | Q10(R38=R39=H) |
| $Pr^i$ | H | $CF_3$ | H | H | H | O | - | - | 0 | F | Cl | Q4(R29=$Bu^t$) |
| $Pr^i$ | H | $CF_3$ | H | H | H | O | - | - | 0 | F | Cl | Q8(R35=R36=R37=H) |
| $Pr^i$ | H | $CF_3$ | H | H | H | O | - | - | 0 | F | Cl | Q18(R50=H) |
| $Pr^i$ | H | $CF_3$ | H | H | H | O | - | - | 0 | F | Cl | Q18(R50=Me) |
| $Pr^i$ | H | $CF_3$ | H | H | H | O | - | - | 0 | F | Cl | $NHCO_2Me$ |
| $Pr^i$ | H | $CF_3$ | H | H | H | O | - | - | 0 | F | Cl | $NHCO_2Et$ |
| $Pr^i$ | H | $CF_3$ | H | H | H | O | - | - | 0 | F | Cl | $NHCO_2Pr^n$ |
| $Pr^i$ | H | $CF_3$ | H | H | H | O | - | - | 0 | F | Cl | $NHCO_2Pr^i$ |
| $Pr^i$ | H | $CF_3$ | H | H | H | O | - | - | 0 | F | Cl | $NHCO_2Ph$ |
| $Pr^i$ | H | $CF_3$ | H | H | H | O | - | - | 0 | F | Cl | $NHCO_2Bz$ |
| $Pr^i$ | H | $CF_3$ | H | H | H | O | - | - | 0 | F | Cl | NHCOMe |
| $Pr^i$ | H | $CF_3$ | H | H | H | O | - | - | 0 | F | Cl | $NHCO_2CH_2Ph$ |
| $Pr^i$ | H | $CF_3$ | H | H | H | O | - | - | 0 | F | Cl | $NHCO_2CH_2$(4-Me-Ph) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | $CF_3$ | H | H | H | O | - | - | 0 | F | Cl | $NHCO_2CH_2$(3-Me-Ph) |
| $Pr^i$ | H | $CF_3$ | H | H | H | O | - | - | 0 | F | Cl | $NHCO_2CH_2$(2-Me-Ph) |
| $Pr^i$ | H | $CF_3$ | H | H | H | O | - | - | 0 | F | Cl | $NHCO_2CH_2$(4-Cl-Ph) |
| $Pr^i$ | H | $CF_3$ | H | H | H | O | - | - | 0 | F | Cl | $NHCO_2CH_2$(3-Cl-Ph) |
| $Pr^i$ | H | $CF_3$ | H | H | H | O | - | - | 0 | F | Cl | $NHCO_2CH_2$(2-Cl-Ph) |
| $Pr^i$ | H | $CF_3$ | H | H | H | O | - | - | 0 | F | Cl | Q1(R29=Me) |
| $Pr^i$ | H | $CF_3$ | H | H | H | O | - | - | 0 | F | Cl | Q2(R29=Me) |
| $Pr^i$ | H | $CF_3$ | H | H | H | O | - | - | 0 | F | Cl | H |
| $Pr^i$ | H | $CF_3$ | H | H | H | O | - | - | 0 | F | Cl | $CO_2H$ |
| $Pr^i$ | H | $CF_3$ | H | H | H | O | - | - | 0 | F | Cl | $CO_2Me$ |
| $Pr^i$ | H | $CF_3$ | H | H | H | O | - | - | 0 | F | Cl | $CO_2Et$ |
| $CF_3$ | H | $CF_3$ | H | H | H | O | - | - | 0 | F | Cl | $CO_2Pr^i$ |
| $CF_3$ | H | $CF_3$ | H | H | H | O | - | - | 0 | F | Cl | $CO_2Pr^n$ |
| $CF_3$ | H | $CF_3$ | H | H | H | O | - | - | 0 | F | Cl | $CO_2Bu^n$ |
| $CF_3$ | H | $CF_3$ | H | H | H | O | - | - | 0 | F | Cl | $CO_2Bu^t$ |
| $CF_3$ | H | $CF_3$ | H | H | H | O | - | - | 0 | F | Cl | $NO_2$ |
| $CF_3$ | H | $CF_3$ | H | H | H | O | - | - | 0 | F | Cl | $NH_2$ |
| $CF_3$ | H | $CF_3$ | H | H | H | O | - | - | 0 | F | Cl | $NHSO_2Me$ |
| $CF_3$ | H | $CF_3$ | H | H | H | O | - | - | 0 | F | Cl | $NHSO_2Et$ |
| $CF_3$ | H | $CF_3$ | H | H | H | O | - | - | 0 | F | Cl | $NHSO_2Pr^n$ |
| $CF_3$ | H | $CF_3$ | H | H | H | O | - | - | 0 | F | Cl | $NHSO_2Pr^i$ |
| $CF_3$ | H | $CF_3$ | H | H | H | O | - | - | 0 | F | Cl | OH |
| $CF_3$ | H | $CF_3$ | H | H | H | O | - | - | 0 | F | Cl | OMe |
| $CF_3$ | H | $CF_3$ | H | H | H | O | - | - | 0 | F | Cl | OEt |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | $CF_3$ | H | H | H | O | - | - | 0 | F | Cl | $OPr^i$ |
| $CF_3$ | H | $CF_3$ | H | H | H | O | - | - | 0 | F | Cl | $OPr^n$ |
| $CF_3$ | H | $CF_3$ | H | H | H | O | - | - | 0 | F | Cl | $OPen^c$ |
| $CF_3$ | H | $CF_3$ | H | H | H | O | - | - | 0 | F | Cl | $OCH_2CH=CH_2$ |
| $CF_3$ | H | $CF_3$ | H | H | H | O | - | - | 0 | F | Cl | $OCH_2C\equiv CH$ |
| $CF_3$ | H | $CF_3$ | H | H | H | O | - | - | 0 | F | Cl | $OCH_2CO_2Me$ |
| $CF_3$ | H | $CF_3$ | H | H | H | O | - | - | 0 | F | Cl | $OCH_2CO_2Et$ |
| $CF_3$ | H | $CF_3$ | H | H | H | O | - | - | 0 | F | Cl | $OCH(Me)CO_2Me$ |
| $CF_3$ | H | $CF_3$ | H | H | H | O | - | - | 0 | F | Cl | $OCH(Me)CO_2Et$ |
| $CF_3$ | H | $CF_3$ | H | H | H | O | - | - | 0 | F | Cl | $CO_2H$ |
| $CF_3$ | H | $CF_3$ | H | H | H | O | - | - | 0 | F | Cl | CN |
| $CF_3$ | H | $CF_3$ | H | H | H | O | - | - | 0 | F | Cl | Me |
| $CF_3$ | H | $CF_3$ | H | H | H | O | - | - | 0 | F | Cl | Et |
| $CF_3$ | H | $CF_3$ | H | H | H | O | - | - | 0 | F | Cl | CL |
| $CF_3$ | H | $CF_3$ | H | H | H | O | - | - | 0 | F | Cl | Br |
| $CF_3$ | H | $CF_3$ | H | H | H | O | - | - | 0 | F | Cl | I |
| $CF_3$ | H | $CF_3$ | H | H | H | O | - | - | 0 | F | Cl | F |
| $CF_3$ | H | $CF_3$ | H | H | H | O | - | - | 0 | F | Cl | SH |
| $CF_3$ | H | $CF_3$ | H | H | H | O | - | - | 0 | F | Cl | $C(Me)=NOCH_2CO_2Me$ |
| $CF_3$ | H | $CF_3$ | H | H | H | O | - | - | 0 | F | Cl | $C(Me)=NOCH_2CO_2Et$ |
| $CF_3$ | H | $CF_3$ | H | H | H | O | - | - | 0 | F | Cl | $CH=NOCH_2CO_2Me$ |
| $CF_3$ | H | $CF_3$ | H | H | H | O | - | - | 0 | F | Cl | $CH=NOCH_2CO_2Et$ |
| $CF_3$ | H | $CF_3$ | H | H | H | O | - | - | 0 | F | Cl | $C(OMe)=NOCH_2CO_2Me$ |
| $CF_3$ | H | $CF_3$ | H | H | H | O | - | - | 0 | F | Cl | $C(OMe)=NOCH_2CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | $CF_3$ | H | H | H | O | - | - | 0 | F | Cl | $C(Me)=NN=C(Me)CO_2Me$ |
| $CF_3$ | H | $CF_3$ | H | H | H | O | - | - | 0 | F | Cl | $C(Me)=NN=C(Me)CO_2Et$ |
| $CF_3$ | H | $CF_3$ | H | H | H | O | - | - | 0 | F | Cl | $CH_2OCH_2CO_2Me$ |
| $CF_3$ | H | $CF_3$ | H | H | H | O | - | - | 0 | F | Cl | $CH_2OCH_2CO_2Et$ |
| $CF_3$ | H | $CF_3$ | H | H | H | O | - | - | 0 | F | Cl | $CH_2OCH(Me)CO_2Me$ |
| $CF_3$ | H | $CF_3$ | H | H | H | O | - | - | 0 | F | Cl | $CH_2OCH(Me)CO_2Et$ |
| $CF_3$ | H | $CF_3$ | H | H | H | O | - | - | 0 | F | Cl | $CH_2SCH_2CO_2Me$ |
| $CF_3$ | H | $CF_3$ | H | H | H | O | - | - | 0 | F | Cl | $CH_2SCH_2CO_2Et$ |
| $CF_3$ | H | $CF_3$ | H | H | H | O | - | - | 0 | F | Cl | $CH_2SCH(Me)CO_2Me$ |
| $CF_3$ | H | $CF_3$ | H | H | H | O | - | - | 0 | F | Cl | $CH_2SCH(Me)CO_2Et$ |
| $CF_3$ | H | $CF_3$ | H | H | H | O | - | - | 0 | F | Cl | SMe |
| $CF_3$ | H | $CF_3$ | H | H | H | O | - | - | 0 | F | Cl | SEt |
| $CF_3$ | H | $CF_3$ | H | H | H | O | - | - | 0 | F | Cl | $SPr^i$ |
| $CF_3$ | H | $CF_3$ | H | H | H | O | - | - | 0 | F | Cl | $SPr^n$ |
| $CF_3$ | H | $CF_3$ | H | H | H | O | - | - | 0 | F | Cl | $SPen^c$ |
| $CF_3$ | H | $CF_3$ | H | H | H | O | - | - | 0 | F | Cl | $SCH_2CO_2H$ |
| $CF_3$ | H | $CF_3$ | H | H | H | O | - | - | 0 | F | Cl | $SCH_2CO_2Me$ |
| $CF_3$ | H | $CF_3$ | H | H | H | O | - | - | 0 | F | Cl | $SCH_2CO_2Et$ |
| $CF_3$ | H | $CF_3$ | H | H | H | O | - | - | 0 | F | Cl | $SCH(Me)CO_2Me$ |
| $CF_3$ | H | $CF_3$ | H | H | H | O | - | - | 0 | F | Cl | $SCH(Me)CO_2Et$ |
| $CF_3$ | H | $CF_3$ | H | H | H | O | - | - | 0 | F | Cl | $SCH_2CN$ |
| $CF_3$ | H | $CF_3$ | H | H | H | O | - | - | 0 | F | Cl | $SCH_2CH=CH_2$ |
| $CF_3$ | H | $CF_3$ | H | H | H | O | - | - | 0 | F | Cl | $SCH_2C\equiv CH$ |
| $CF_3$ | H | $CF_3$ | H | H | H | O | - | - | 0 | F | Cl | $OCH(Me)C\equiv CH$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | $CF_3$ | H | H | H | O | - | - | 0 | F | Cl | SCH(Me)C ≡CH |
| $CF_3$ | H | $CF_3$ | H | H | H | O | - | - | 0 | F | Cl | $N(SO_2Me)_2$ |
| $CF_3$ | H | $CF_3$ | H | H | H | O | - | - | 0 | F | Cl | $N(SO_2Et)_2$ |
| $CF_3$ | H | $CF_3$ | H | H | H | O | - | - | 0 | F | Cl | Q1(R29=$Bu^t$) |
| $CF_3$ | H | $CF_3$ | H | H | H | O | - | - | 0 | F | Cl | Q2(R29=$Bu^t$) |
| $CF_3$ | H | $CF_3$ | H | H | H | O | - | - | 0 | F | Cl | Q5(R29=$Bu^t$) |
| $CF_3$ | H | $CF_3$ | H | H | H | O | - | - | 0 | F | Cl | Q7(R34=$Bu^t$) |
| $CF_3$ | H | $CF_3$ | H | H | H | O | - | - | 0 | F | Cl | Q9(R38=R39=H) |
| $CF_3$ | H | $CF_3$ | H | H | H | O | - | - | 0 | F | Cl | Q10(R38=R39=H) |
| $CF_3$ | H | $CF_3$ | H | H | H | O | - | - | 0 | F | Cl | Q4(R29=$Bu^t$) |
| $CF_3$ | H | $CF_3$ | H | H | H | O | - | - | 0 | F | Cl | Q8(R35=R36=R37=H) |
| $CF_3$ | H | $CF_3$ | H | H | H | O | - | - | 0 | F | Cl | Q18(R50=H) |
| $CF_3$ | H | $CF_3$ | H | H | H | O | - | - | 0 | F | Cl | Q18(R50=Me) |
| $CF_3$ | H | $CF_3$ | H | H | H | O | - | - | 0 | F | Cl | $NHCO_2Me$ |
| $CF_3$ | H | $CF_3$ | H | H | H | O | - | - | 0 | F | Cl | $NHCO_2Et$ |
| $CF_3$ | H | $CF_3$ | H | H | H | O | - | - | 0 | F | Cl | $NHCO_2Pr^n$ |
| $CF_3$ | H | $CF_3$ | H | H | H | O | - | - | 0 | F | Cl | $NHCO_2Pr^i$ |
| $CF_3$ | H | $CF_3$ | H | H | H | O | - | - | 0 | F | Cl | $NHCO_2Ph$ |
| $CF_3$ | H | $CF_3$ | H | H | H | O | - | - | 0 | F | Cl | $NHCO_2Bz$ |
| $CF_3$ | H | $CF_3$ | H | H | H | O | - | - | 0 | F | Cl | NHCOMe |
| $CF_3$ | H | $CF_3$ | H | H | H | O | - | - | 0 | F | Cl | $NHCO_2CH_2Ph$ |
| $CF_3$ | H | $CF_3$ | H | H | H | O | - | - | 0 | F | Cl | $NHCO_2CH_2$(4-Me-Ph) |
| $CF_3$ | H | $CF_3$ | H | H | H | O | - | - | 0 | F | Cl | $NHCO_2CH_2$(3-Me-Ph) |
| $CF_3$ | H | $CF_3$ | H | H | H | O | - | - | 0 | F | Cl | $NHCO_2CH_2$(2-Me-Ph) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | $CF_3$ | H | H | H | O | - | - | 0 | F | Cl | $NHCO_2CH_2$(4-Cl-Ph) |
| $CF_3$ | H | $CF_3$ | H | H | H | O | - | - | 0 | F | Cl | $NHCO_2CH_2$(3-Cl-Ph) |
| $CF_3$ | H | $CF_3$ | H | H | H | O | - | - | 0 | F | Cl | $NHCO_2CH_2$(2-Cl-Ph) |
| $CF_3$ | H | $CF_3$ | H | H | H | O | - | - | 0 | F | Cl | Q1(R29=Me) |
| $CF_3$ | H | $CF_3$ | H | H | H | O | - | - | 0 | F | Cl | Q2(R29=Me) |
| $CF_3$ | H | $CF_3$ | H | H | H | O | - | - | 0 | F | Cl | H |
| $CF_3$ | H | $CF_3$ | H | H | H | O | - | - | 0 | F | Cl | $CO_2H$ |
| $CF_3$ | H | $CF_3$ | H | H | H | O | - | - | 0 | F | Cl | $CO_2Me$ |
| $CF_3$ | H | $CF_3$ | H | H | H | O | - | - | 0 | F | Cl | $CO_2Et$ |
| H | $-CH_2CH_2CH_2-$ | | H | H | H | O | - | - | 0 | F | Cl | $CO_2Pr^i$ |
| H | $-CH_2CH_2CH_2-$ | | H | H | H | O | - | - | 0 | F | Cl | $CO_2Pr^n$ |
| H | $-CH_2CH_2CH_2-$ | | H | H | H | O | - | - | 0 | F | Cl | $CO_2Bu^n$ |
| H | $-CH_2CH_2CH_2-$ | | H | H | H | O | - | - | 0 | F | Cl | $CO_2Bu^t$ |
| H | $-CH_2CH_2CH_2-$ | | H | H | H | O | - | - | 0 | F | Cl | $NO_2$ |
| H | $-CH_2CH_2CH_2-$ | | H | H | H | O | - | - | 0 | F | Cl | $NH_2$ |
| H | $-CH_2CH_2CH_2-$ | | H | H | H | O | - | - | 0 | F | Cl | $NHSO_2Me$ |
| H | $-CH_2CH_2CH_2-$ | | H | H | H | O | - | - | 0 | F | Cl | $NHSO_2Et$ |
| H | $-CH_2CH_2CH_2-$ | | H | H | H | O | - | - | 0 | F | Cl | $NHSO_2Pr^n$ |
| H | $-CH_2CH_2CH_2-$ | | H | H | H | O | - | - | 0 | F | Cl | $NHSO_2Pr^i$ |
| H | $-CH_2CH_2CH_2-$ | | H | H | H | O | - | - | 0 | F | Cl | OH |
| H | $-CH_2CH_2CH_2-$ | | H | H | H | O | - | - | 0 | F | Cl | OMe |
| H | $-CH_2CH_2CH_2-$ | | H | H | H | O | - | - | 0 | F | Cl | OEt |
| H | $-CH_2CH_2CH_2-$ | | H | H | H | O | - | - | 0 | F | Cl | $OPr^i$ |
| H | $-CH_2CH_2CH_2-$ | | H | H | H | O | - | - | 0 | F | Cl | $OPr^n$ |

Table 2-3-1 (Cont'd)

| R1 | R2 R3 | R4 | R5 | R6 | X | $R_a$ | $R_b$ | m | R7 | R8 | R9 |
|---|---|---|---|---|---|---|---|---|---|---|---|
| H | $-CH_2CH_2CH_2-$ | H | H | H | O | - | - | 0 | F | Cl | OPen [c] |
| H | $-CH_2CH_2CH_2-$ | H | H | H | O | - | - | 0 | F | Cl | $OCH_2CH=CH_2$ |
| H | $-CH_2CH_2CH_2-$ | H | H | H | O | - | - | 0 | F | Cl | $OCH_2C\equiv CH$ |
| H | $-CH_2CH_2CH_2-$ | H | H | H | O | - | - | 0 | F | Cl | $OCH_2CO_2Me$ |
| H | $-CH_2CH_2CH_2-$ | H | H | H | O | - | - | 0 | F | Cl | $OCH_2CO_2Et$ |
| H | $-CH_2CH_2CH_2-$ | H | H | H | O | - | - | 0 | F | Cl | $OCH(Me)CO_2Me$ |
| H | $-CH_2CH_2CH_2-$ | H | H | H | O | - | - | 0 | F | Cl | $OCH(Me)CO_2Et$ |
| H | $-CH_2CH_2CH_2-$ | H | H | H | O | - | - | 0 | F | Cl | $CO_2H$ |
| H | $-CH_2CH_2CH_2-$ | H | H | H | O | - | - | 0 | F | Cl | CN |
| H | $-CH_2CH_2CH_2-$ | H | H | H | O | - | - | 0 | F | Cl | Me |
| H | $-CH_2CH_2CH_2-$ | H | H | H | O | - | - | 0 | F | Cl | Et |
| H | $-CH_2CH_2CH_2-$ | H | H | H | O | - | - | 0 | F | Cl | Cl |
| H | $-CH_2CH_2CH_2-$ | H | H | H | O | - | - | 0 | F | Cl | Br |
| H | $-CH_2CH_2CH_2-$ | H | H | H | O | - | - | 0 | F | Cl | I |
| H | $-CH_2CH_2CH_2-$ | H | H | H | O | - | - | 0 | F | Cl | F |
| H | $-CH_2CH_2CH_2-$ | H | H | H | O | - | - | 0 | F | Cl | SH |
| H | $-CH_2CH_2CH_2-$ | H | H | H | O | - | - | 0 | F | Cl | $C(Me)=NOCH_2CO_2Me$ |
| H | $-CH_2CH_2CH_2-$ | H | H | H | O | - | - | 0 | F | Cl | $C(Me)=NOCH_2CO_2Et$ |
| H | $-CH_2CH_2CH_2-$ | H | H | H | O | - | - | 0 | F | Cl | $CH=NOCH_2CO_2Me$ |
| H | $-CH_2CH_2CH_2-$ | H | H | H | O | - | - | 0 | F | Cl | $CH=NOCH_2CO_2Et$ |
| H | $-CH_2CH_2CH_2-$ | H | H | H | O | - | - | 0 | F | Cl | $C(OMe)=NOCH_2CO_2Me$ |
| H | $-CH_2CH_2CH_2-$ | H | H | H | O | - | - | 0 | F | Cl | $C(OMe)=NOCH_2CO_2Et$ |
| H | $-CH_2CH_2CH_2-$ | H | H | H | O | - | - | 0 | F | Cl | $C(Me)=NN=C(Me)CO_2Me$ |
| H | $-CH_2CH_2CH_2-$ | H | H | H | O | - | - | 0 | F | Cl | $C(Me)=NN=C(Me)CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| H | $-CH_2CH_2CH_2-$ | | H | H | H | O | - | - | 0 | F | Cl | $CH_2OCH_2CO_2Me$ |
| H | $-CH_2CH_2CH_2-$ | | H | H | H | O | - | - | 0 | F | Cl | $CH_2OCH_2CO_2Et$ |
| H | $-CH_2CH_2CH_2-$ | | H | H | H | O | - | - | 0 | F | Cl | $CH_2OCH(Me)CO_2Me$ |
| H | $-CH_2CH_2CH_2-$ | | H | H | H | O | - | - | 0 | F | Cl | $CH_2OCH(Me)CO_2Et$ |
| H | $-CH_2CH_2CH_2-$ | | H | H | H | O | - | - | 0 | F | Cl | $CH_2SCH_2CO_2Me$ |
| H | $-CH_2CH_2CH_2-$ | | H | H | H | O | - | - | 0 | F | Cl | $CH_2SCH_2CO_2Et$ |
| H | $-CH_2CH_2CH_2-$ | | H | H | H | O | - | - | 0 | F | Cl | $CH_2SCH(Me)CO_2Me$ |
| H | $-CH_2CH_2CH_2-$ | | H | H | H | O | - | - | 0 | F | Cl | $CH_2SCH(Me)CO_2Et$ |
| H | $-CH_2CH_2CH_2-$ | | H | H | H | O | - | - | 0 | F | Cl | SMe |
| H | $-CH_2CH_2CH_2-$ | | H | H | H | O | - | - | 0 | F | Cl | SEt |
| H | $-CH_2CH_2CH_2-$ | | H | H | H | O | - | - | 0 | F | Cl | $SPr^i$ |
| H | $-CH_2CH_2CH_2-$ | | H | H | H | O | - | - | 0 | F | Cl | $SPr^n$ |
| H | $-CH_2CH_2CH_2-$ | | H | H | H | O | - | - | 0 | F | Cl | $SPen^c$ |
| H | $-CH_2CH_2CH_2-$ | | H | H | H | O | - | - | 0 | F | Cl | $SCH_2CO_2H$ |
| H | $-CH_2CH_2CH_2-$ | | H | H | H | O | - | - | 0 | F | Cl | $SCH_2CO_2Me$ |
| H | $-CH_2CH_2CH_2-$ | | H | H | H | O | - | - | 0 | F | Cl | $SCH_2CO_2Et$ |
| H | $-CH_2CH_2CH_2-$ | | H | H | H | O | - | - | 0 | F | Cl | $SCH(Me)CO_2Me$ |
| H | $-CH_2CH_2CH_2-$ | | H | H | H | O | - | - | 0 | F | Cl | $SCH(Me)CO_2Et$ |
| H | $-CH_2CH_2CH_2-$ | | H | H | H | O | - | - | 0 | F | Cl | $SCH_2CN$ |
| H | $-CH_2CH_2CH_2-$ | | H | H | H | O | - | - | 0 | F | Cl | $SCH_2CH=CH_2$ |
| H | $-CH_2CH_2CH_2-$ | | H | H | H | O | - | - | 0 | F | Cl | $SCH_2C\equiv CH$ |
| H | $-CH_2CH_2CH_2-$ | | H | H | H | O | - | - | 0 | F | Cl | $OCH(Me)C\equiv CH$ |
| H | $-CH_2CH_2CH_2-$ | | H | H | H | O | - | - | 0 | F | Cl | $SCH(Me)C\equiv CH$ |
| H | $-CH_2CH_2CH_2-$ | | H | H | H | O | - | - | 0 | F | Cl | $N(SO_2Me)_2$ |

Table 2-3-1 (Cont'd)

| R1 | R2 R3 | R4 | R5 | R6 | X | $R_a$ | $R_b$ | m | R7 | R8 | R9 |
|---|---|---|---|---|---|---|---|---|---|---|---|
| H | $-CH_2CH_2CH_2-$ | H | H | H | 0 | - | - | 0 | F | Cl | $N(SO_2Et)_2$ |
| H | $-CH_2CH_2CH_2-$ | H | H | H | 0 | - | - | 0 | F | Cl | Q1(R29=$Bu^t$ ) |
| H | $-CH_2CH_2CH_2-$ | H | H | H | 0 | - | - | 0 | F | Cl | Q2(R29=$Bu^t$ ) |
| H | $-CH_2CH_2CH_2-$ | H | H | H | 0 | - | - | 0 | F | Cl | Q5(R29=$Bu^t$ ) |
| H | $-CH_2CH_2CH_2-$ | H | H | H | 0 | - | - | 0 | F | Cl | Q7(R34=$Bu^t$ ) |
| H | $-CH_2CH_2CH_2-$ | H | H | H | 0 | - | - | 0 | F | Cl | Q9(R38=R39=H) |
| H | $-CH_2CH_2CH_2-$ | H | H | H | 0 | - | - | 0 | F | Cl | Q10(R38=R39=H) |
| H | $-CH_2CH_2CH_2-$ | H | H | H | 0 | - | - | 0 | F | Cl | Q4(R29=$Bu^t$ ) |
| H | $-CH_2CH_2CH_2-$ | H | H | H | 0 | - | - | 0 | F | Cl | Q8(R35=R36=R37=H) |
| H | $-CH_2CH_2CH_2-$ | H | H | H | 0 | - | - | 0 | F | Cl | Q18(R50=H) |
| H | $-CH_2CH_2CH_2-$ | H | H | H | 0 | - | - | 0 | F | Cl | Q18(R50=Me) |
| H | $-CH_2CH_2CH_2-$ | H | H | H | 0 | - | - | 0 | F | Cl | $NHCO_2Me$ |
| H | $-CH_2CH_2CH_2-$ | H | H | H | 0 | - | - | 0 | F | Cl | $NHCO_2Et$ |
| H | $-CH_2CH_2CH_2-$ | H | H | H | 0 | - | - | 0 | F | Cl | $NHCO_2Pr^n$ |
| H | $-CH_2CH_2CH_2-$ | H | H | H | 0 | - | - | 0 | F | Cl | $NHCO_2Pr^i$ |
| H | $-CH_2CH_2CH_2-$ | H | H | H | 0 | - | - | 0 | F | Cl | $NHCO_2Ph$ |
| H | $-CH_2CH_2CH_2-$ | H | H | H | 0 | - | - | 0 | F | Cl | $NHCO_2Bz$ |
| H | $-CH_2CH_2CH_2-$ | H | H | H | 0 | - | - | 0 | F | Cl | NHCOMe |
| H | $-CH_2CH_2CH_2-$ | H | H | H | 0 | - | - | 0 | F | Cl | $NHCO_2CH_2Ph$ |
| H | $-CH_2CH_2CH_2-$ | H | H | H | 0 | - | - | 0 | F | Cl | $NHCO_2CH_2$(4-Me-Ph) |
| H | $-CH_2CH_2CH_2-$ | H | H | H | 0 | - | - | 0 | F | Cl | $NHCO_2CH_2$(3-Me-Ph) |
| H | $-CH_2CH_2CH_2-$ | H | H | H | 0 | - | - | 0 | F | Cl | $NHCO_2CH_2$(2-Me-Ph) |
| H | $-CH_2CH_2CH_2-$ | H | H | H | 0 | - | - | 0 | F | Cl | $NHCO_2CH_2$(4-Cl-Ph) |
| H | $-CH_2CH_2CH_2-$ | H | H | H | 0 | - | - | 0 | F | Cl | $NHCO_2CH_2$(3-Cl-Ph) |

Table 2-3-1 (Cont'd)

| R1 | R2 R3 | R4 | R5 | R6 | X | $R_a$ | $R_b$ | m | R7 | R8 | R9 |
|---|---|---|---|---|---|---|---|---|---|---|---|
| H | $-CH_2CH_2CH_2-$ | H | H | H | O | - | - | 0 | F | Cl | $NHCO_2CH_2$(2-Cl-Ph) |
| H | $-CH_2CH_2CH_2-$ | H | H | H | O | - | - | 0 | F | Cl | Q1(R29=Me) |
| H | $-CH_2CH_2CH_2-$ | H | H | H | O | - | - | 0 | F | Cl | Q2(R29=Me) |
| H | $-CH_2CH_2CH_2-$ | H | H | H | O | - | - | 0 | F | Cl | H |
| H | $-CH_2CH_2CH_2-$ | H | H | H | O | - | - | 0 | F | Cl | $CO_2H$ |
| H | $-CH_2CH_2CH_2-$ | H | H | H | O | - | - | 0 | F | Cl | $CO_2Me$ |
| H | $-CH_2CH_2CH_2-$ | H | H | H | O | - | - | 0 | F | Cl | $CO_2Et$ |
| H | $-(CH_2)_4-$ | H | H | H | O | - | - | 0 | F | Cl | $CO_2Pr^i$ |
| H | $-(CH_2)_4-$ | H | H | H | O | - | - | 0 | F | Cl | $CO_2Pr^n$ |
| H | $-(CH_2)_4-$ | H | H | H | O | - | - | 0 | F | Cl | $CO_2Bu^n$ |
| H | $-(CH_2)_4-$ | H | H | H | O | - | - | 0 | F | Cl | $CO_2Bu^t$ |
| H | $-(CH_2)_4-$ | H | H | H | O | - | - | 0 | F | Cl | $NO_2$ |
| H | $-(CH_2)_4-$ | H | H | H | O | - | - | 0 | F | Cl | $NH_2$ |
| H | $-(CH_2)_4-$ | H | H | H | O | - | - | 0 | F | Cl | $NHSO_2Me$ |
| H | $-(CH_2)_4-$ | H | H | H | O | - | - | 0 | F | Cl | $NHSO_2Et$ |
| H | $-(CH_2)_4-$ | H | H | H | O | - | - | 0 | F | Cl | $NHSO_2Pr^n$ |
| H | $-(CH_2)_4-$ | H | H | H | O | - | - | 0 | F | Cl | $NHSO_2Pr^i$ |
| H | $-(CH_2)_4-$ | H | H | H | O | - | - | 0 | F | Cl | OH |
| H | $-(CH_2)_4-$ | H | H | H | O | - | - | 0 | F | Cl | OMe |
| H | $-(CH_2)_4-$ | H | H | H | O | - | - | 0 | F | Cl | OEt |
| H | $-(CH_2)_4-$ | H | H | H | O | - | - | 0 | F | Cl | $OPr^i$ |
| H | $-(CH_2)_4-$ | H | H | H | O | - | - | 0 | F | Cl | $OPr^n$ |
| H | $-(CH_2)_4-$ | H | H | H | O | - | - | 0 | F | Cl | $OPen^c$ |
| H | $-(CH_2)_4-$ | H | H | H | O | - | - | 0 | F | Cl | $OCH_2CH=CH_2$ |

Table 2-3-1 (Cont'd)

| R1 | R2 R3 | R4 | R5 | R6 | X | $R_a$ | $R_b$ | m | R7 | R8 | R9 |
|---|---|---|---|---|---|---|---|---|---|---|---|
| H | $-(CH_2)_4-$ | H | H | H | O | - | - | 0 | F | Cl | $OCH_2C{\equiv}CH$ |
| H | $-(CH_2)_4-$ | H | H | H | O | - | - | 0 | F | Cl | $OCH_2CO_2Me$ |
| H | $-(CH_2)_4-$ | H | H | H | O | - | - | 0 | F | Cl | $OCH_2CO_2Et$ |
| H | $-(CH_2)_4-$ | H | H | H | O | - | - | 0 | F | Cl | $OCH(Me)CO_2Me$ |
| H | $-(CH_2)_4-$ | H | H | H | O | - | - | 0 | F | Cl | $OCH(Me)CO_2Et$ |
| H | $-(CH_2)_4-$ | H | H | H | O | - | - | 0 | F | Cl | $CO_2H$ |
| H | $-(CH_2)_4-$ | H | H | H | O | - | - | 0 | F | Cl | CN |
| H | $-(CH_2)_4-$ | H | H | H | O | - | - | 0 | F | Cl | Me |
| H | $-(CH_2)_4-$ | H | H | H | O | - | - | 0 | F | Cl | Et |
| H | $-(CH_2)_4-$ | H | H | H | O | - | - | 0 | F | Cl | Cl |
| H | $-(CH_2)_4-$ | H | H | H | O | - | - | 0 | F | Cl | Br |
| H | $-(CH_2)_4-$ | H | H | H | O | - | - | 0 | F | Cl | I |
| H | $-(CH_2)_4-$ | H | H | H | O | - | - | 0 | F | Cl | F |
| H | $-(CH_2)_4-$ | H | H | H | O | - | - | 0 | F | Cl | SH |
| H | $-(CH_2)_4-$ | H | H | H | O | - | - | 0 | F | Cl | $C(Me)=NOCH_2CO_2Me$ |
| H | $-(CH_2)_4-$ | H | H | H | O | - | - | 0 | F | Cl | $C(Me)=NOCH_2CO_2Et$ |
| H | $-(CH_2)_4-$ | H | H | H | O | - | - | 0 | F | Cl | $CH=NOCH_2CO_2Me$ |
| H | $-(CH_2)_4-$ | H | H | H | O | - | - | 0 | F | Cl | $CH=NOCH_2CO_2Et$ |
| H | $-(CH_2)_4-$ | H | H | H | O | - | - | 0 | F | Cl | $C(OMe)=NOCH_2CO_2Me$ |
| H | $-(CH_2)_4-$ | H | H | H | O | - | - | 0 | F | Cl | $C(OMe)=NOCH_2CO_2Et$ |
| H | $-(CH_2)_4-$ | H | H | H | O | - | - | 0 | F | Cl | $C(Me)=NN=C(Me)CO_2Me$ |
| H | $-(CH_2)_4-$ | H | H | H | O | - | - | 0 | F | Cl | $C(Me)=NN=C(Me)CO_2Et$ |
| H | $-(CH_2)_4-$ | H | H | H | O | - | - | 0 | F | Cl | $CH_2OCH_2CO_2Me$ |
| H | $-(CH_2)_4-$ | H | H | H | O | - | - | 0 | F | Cl | $CH_2OCH_2CO_2Et$ |

Table 2-3-1 (Cont'd)

| R1 | R2 R3 | R4 | R5 | R6 | X | $R_a$ | $R_b$ | m | R7 | R8 | R9 |
|---|---|---|---|---|---|---|---|---|---|---|---|
| H | $-(CH_2)_4-$ | H | H | H | O | - | - | 0 | F | Cl | $CH_2OCH(Me)CO_2Me$ |
| H | $-(CH_2)_4-$ | H | H | H | O | - | - | 0 | F | Cl | $CH_2OCH(Me)CO_2Et$ |
| H | $-(CH_2)_4-$ | H | H | H | O | - | - | 0 | F | Cl | $CH_2SCH_2CO_2Me$ |
| H | $-(CH_2)_4-$ | H | H | H | O | - | - | 0 | F | Cl | $CH_2SCH_2CO_2Et$ |
| H | $-(CH_2)_4-$ | H | H | H | O | - | - | 0 | F | Cl | $CH_2SCH(Me)CO_2Me$ |
| H | $-(CH_2)_4-$ | H | H | H | O | - | - | 0 | F | Cl | $CH_2SCH(Me)CO_2Et$ |
| H | $-(CH_2)_4-$ | H | H | H | O | - | - | 0 | F | Cl | SMe |
| H | $-(CH_2)_4-$ | H | H | H | O | - | - | 0 | F | Cl | SEt |
| H | $-(CH_2)_4-$ | H | H | H | O | - | - | 0 | F | Cl | $SPr^i$ |
| H | $-(CH_2)_4-$ | H | H | H | O | - | - | 0 | F | Cl | $SPr^n$ |
| H | $-(CH_2)_4-$ | H | H | H | O | - | - | 0 | F | Cl | $SPen^c$ |
| H | $-(CH_2)_4-$ | H | H | H | O | - | - | 0 | F | Cl | $SCH_2CO_2H$ |
| H | $-(CH_2)_4-$ | H | H | H | O | - | - | 0 | F | Cl | $SCH_2CO_2Me$ |
| H | $-(CH_2)_4-$ | H | H | H | O | - | - | 0 | F | Cl | $SCH_2CO_2Et$ |
| H | $-(CH_2)_4-$ | H | H | H | O | - | - | 0 | F | Cl | $SCH(Me)CO_2Me$ |
| H | $-(CH_2)_4-$ | H | H | H | O | - | - | 0 | F | Cl | $SCH(Me)CO_2Et$ |
| H | $-(CH_2)_4-$ | H | H | H | O | - | - | 0 | F | Cl | $SCH_2CN$ |
| H | $-(CH_2)_4-$ | H | H | H | O | - | - | 0 | F | Cl | $SCH_2CH=CH_2$ |
| H | $-(CH_2)_4-$ | H | H | H | O | - | - | 0 | F | Cl | $SCH_2C\equiv CH$ |
| H | $-(CH_2)_4-$ | H | H | H | O | - | - | 0 | F | Cl | $OCH(Me)C\equiv CH$ |
| H | $-(CH_2)_4-$ | H | H | H | O | - | - | 0 | F | Cl | $SCH(Me)C\equiv CH$ |
| H | $-(CH_2)_4-$ | H | H | H | O | - | - | 0 | F | Cl | $N(SO_2Me)_2$ |
| H | $-(CH_2)_4-$ | H | H | H | O | - | - | 0 | F | Cl | $N(SO_2Et)_2$ |
| H | $-(CH_2)_4-$ | H | H | H | O | - | - | 0 | F | Cl | Q1(R29=$Bu^t$) |

Table 2-3-1 (Cont'd)

| R1 | R2 R3 | R4 | R5 | R6 | X | $R_a$ | $R_b$ | m | R7 | R8 | R9 |
|---|---|---|---|---|---|---|---|---|---|---|---|
| H | $-(CH_2)_4-$ | H | H | H | O | - | - | 0 | F | Cl | Q2(R29=$Bu^t$) |
| H | $-(CH_2)_4-$ | H | H | H | O | - | - | 0 | F | Cl | Q5(R29=$Bu^t$) |
| H | $-(CH_2)_4-$ | H | H | H | O | - | - | 0 | F | Cl | Q7(R34=$Bu^t$) |
| H | $-(CH_2)_4-$ | H | H | H | O | - | - | 0 | F | Cl | Q9(R38=R39=H) |
| H | $-(CH_2)_4-$ | H | H | H | O | - | - | 0 | F | Cl | Q10(R38=R39=H) |
| H | $-(CH_2)_4-$ | H | H | H | O | - | - | 0 | F | Cl | Q4(R29=$Bu^t$ ) |
| H | $-(CH_2)_4-$ | H | H | H | O | - | - | 0 | F | Cl | Q8(R35=R36=R37=H) |
| H | $-(CH_2)_4-$ | H | H | H | O | - | - | 0 | F | Cl | Q18(R50=H) |
| H | $-(CH_2)_4-$ | H | H | H | O | - | - | 0 | F | Cl | Q18(R50=Me) |
| H | $-(CH_2)_4-$ | H | H | H | O | - | - | 0 | F | Cl | $NHCO_2Me$ |
| H | $-(CH_2)_4-$ | H | H | H | O | - | - | 0 | F | Cl | $NHCO_2Et$ |
| H | $-(CH_2)_4-$ | H | H | H | O | - | - | 0 | F | Cl | $NHCO_2Pr^n$ |
| H | $-(CH_2)_4-$ | H | H | H | O | - | - | 0 | F | Cl | $NHCO_2Pr^i$ |
| H | $-(CH_2)_4-$ | H | H | H | O | - | - | 0 | F | Cl | $NHCO_2Ph$ |
| H | $-(CH_2)_4-$ | H | H | H | O | - | - | 0 | F | Cl | $NHCO_2Bz$ |
| H | $-(CH_2)_4-$ | H | H | H | O | - | - | 0 | F | Cl | NHCOMe |
| H | $-(CH_2)_4-$ | H | H | H | O | - | - | 0 | F | Cl | $NHCO_2CH_2Ph$ |
| H | $-(CH_2)_4-$ | H | H | H | O | - | - | 0 | F | Cl | $NHCO_2CH_2$(4-Me-Ph) |
| H | $-(CH_2)_4-$ | H | H | H | O | - | - | 0 | F | Cl | $NHCO_2CH_2$(3-Me-Ph) |
| H | $-(CH_2)_4-$ | H | H | H | O | - | - | 0 | F | Cl | $NHCO_2CH_2$(2-Me-Ph) |
| H | $-(CH_2)_4-$ | H | H | H | O | - | - | 0 | F | Cl | $NHCO_2CH_2$(4-Cl-Ph) |
| H | $-(CH_2)_4-$ | H | H | H | O | - | - | 0 | F | Cl | $NHCO_2CH_2$(3-Cl-Ph) |
| H | $-(CH_2)_4-$ | H | H | H | O | - | - | 0 | F | Cl | $NHCO_2CH_2$(2-Cl-Ph) |
| H | $-(CH_2)_4-$ | H | H | H | O | - | - | 0 | F | Cl | Q1(R29=Me) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| H | $-(CH_2)_4-$ | | H | H | H | O | - | - | 0 | F | Cl | Q2(R29=Me) |
| H | $-(CH_2)_4-$ | | H | H | H | O | - | - | 0 | F | Cl | H |
| H | $-(CH_2)_4-$ | | H | H | H | O | - | - | 0 | F | Cl | $CO_2H$ |
| H | $-(CH_2)_4-$ | | H | H | H | O | - | - | 0 | F | Cl | $CO_2Me$ |
| H | $-(CH_2)_4-$ | | H | H | H | O | - | - | 0 | F | Cl | $CO_2Et$ |
| Me | H | Me | H | Me | H | O | - | - | 0 | F | Cl | $CO_2Pr^i$ |
| Me | H | Me | H | Me | H | O | - | - | 0 | F | Cl | $CO_2Pr^n$ |
| Me | H | Me | H | Me | H | O | - | - | 0 | F | Cl | $CO_2Bu^n$ |
| Me | H | Me | H | Me | H | O | - | - | 0 | F | Cl | $CO_2Bu^t$ |
| Me | H | Me | H | Me | H | O | - | - | 0 | F | Cl | $NO_2$ |
| Me | H | Me | H | Me | H | O | - | - | 0 | F | Cl | $NH_2$ |
| Me | H | Me | H | Me | H | O | - | - | 0 | F | Cl | $NHSO_2Me$ |
| Me | H | Me | H | Me | H | O | - | - | 0 | F | Cl | $NHSO_2Et$ |
| Me | H | Me | H | Me | H | O | - | - | 0 | F | Cl | $NHSO_2Pr^n$ |
| Me | H | Me | H | Me | H | O | - | - | 0 | F | Cl | $NHSO_2Pr^i$ |
| Me | H | Me | H | Me | H | O | - | - | 0 | F | Cl | OH |
| Me | H | Me | H | Me | H | O | - | - | 0 | F | Cl | OMe |
| Me | H | Me | H | Me | H | O | - | - | 0 | F | Cl | OEt |
| Me | H | Me | H | Me | H | O | - | - | 0 | F | Cl | $OPr^i$ |
| Me | H | Me | H | Me | H | O | - | - | 0 | F | Cl | $OPr^n$ |
| Me | H | Me | H | Me | H | O | - | - | 0 | F | Cl | $OPen^c$ |
| Me | H | Me | H | Me | H | O | - | - | 0 | F | Cl | $OCH_2CH=CH_2$ |
| Me | H | Me | H | Me | H | O | - | - | 0 | F | Cl | $OCH_2C\equiv CH$ |
| Me | H | Me | H | Me | H | O | - | - | 0 | F | Cl | $OCH_2CO_2Me$ |

Table 2-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | Me | H | O | - | - | 0 | F | Cl | $OCH_2CO_2Et$ |
| Me | H | Me | H | Me | H | O | - | - | 0 | F | Cl | $OCH(Me)CO_2Me$ |
| Me | H | Me | H | Me | H | O | - | - | 0 | F | Cl | $OCH(Me)CO_2Et$ |
| Me | H | Me | H | Me | H | O | - | - | 0 | F | Cl | $CO_2H$ |
| Me | H | Me | H | Me | H | O | - | - | 0 | F | Cl | CN |
| Me | H | Me | H | Me | H | O | - | - | 0 | F | Cl | Me |
| Me | H | Me | H | Me | H | O | - | - | 0 | F | Cl | Et |
| Me | H | Me | H | Me | H | O | - | - | 0 | F | Cl | CL |
| Me | H | Me | H | Me | H | O | - | - | 0 | F | Cl | Br |
| Me | H | Me | H | Me | H | O | - | - | 0 | F | Cl | I |
| Me | H | Me | H | Me | H | O | - | - | 0 | F | Cl | F |
| Me | H | Me | H | Me | H | O | - | - | 0 | F | Cl | SH |
| Me | H | Me | H | Me | H | O | - | - | 0 | F | Cl | $C(Me)=NOCH_2CO_2Me$ |
| Me | H | Me | H | Me | H | O | - | - | 0 | F | Cl | $C(Me)=NOCH_2CO_2Et$ |
| Me | H | Me | H | Me | H | O | - | - | 0 | F | Cl | $CH=NOCH_2CO_2Me$ |
| Me | H | Me | H | Me | H | O | - | - | 0 | F | Cl | $CH=NOCH_2CO_2Et$ |
| Me | H | Me | H | Me | H | O | - | - | 0 | F | Cl | $C(OMe)=NOCH_2CO_2Me$ |
| Me | H | Me | H | Me | H | O | - | - | 0 | F | Cl | $C(OMe)=NOCH_2CO_2Et$ |
| Me | H | Me | H | Me | H | O | - | - | 0 | F | Cl | $C(Me)=NN=C(Me)CO_2Me$ |
| Me | H | Me | H | Me | H | O | - | - | 0 | F | Cl | $C(Me)=NN=C(Me)CO_2Et$ |
| Me | H | Me | H | Me | H | O | - | - | 0 | F | Cl | $CH_2OCH_2CO_2Me$ |
| Me | H | Me | H | Me | H | O | - | - | 0 | F | Cl | $CH_2OCH_2CO_2Et$ |
| Me | H | Me | H | Me | H | O | - | - | 0 | F | Cl | $CH_2OCH(Me)CO_2Me$ |
| Me | H | Me | H | Me | H | O | - | - | 0 | F | Cl | $CH_2OCH(Me)CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | Me | H | O | - | - | 0 | F | Cl | $CH_2SCH_2CO_2Me$ |
| Me | H | Me | H | Me | H | O | - | - | 0 | F | Cl | $CH_2SCH_2CO_2Et$ |
| Me | H | Me | H | Me | H | O | - | - | 0 | F | Cl | $CH_2SCH(Me)CO_2Me$ |
| Me | H | Me | H | Me | H | O | - | - | 0 | F | Cl | $CH_2SCH(Me)CO_2Et$ |
| Me | H | Me | H | Me | H | O | - | - | 0 | F | Cl | SMe |
| Me | H | Me | H | Me | H | O | - | - | 0 | F | Cl | SEt |
| Me | H | Me | H | Me | H | O | - | - | 0 | F | Cl | $SPr^i$ |
| Me | H | Me | H | Me | H | O | - | - | 0 | F | Cl | $SPr^n$ |
| Me | H | Me | H | Me | H | O | - | - | 0 | F | Cl | $SPen^c$ |
| Me | H | Me | H | Me | H | O | - | - | 0 | F | Cl | $SCH_2CO_2H$ |
| Me | H | Me | H | Me | H | O | - | - | 0 | F | Cl | $SCH_2CO_2Me$ |
| Me | H | Me | H | Me | H | O | - | - | 0 | F | Cl | $SCH_2CO_2Et$ |
| Me | H | Me | H | Me | H | O | - | - | 0 | F | Cl | $SCH(Me)CO_2Me$ |
| Me | H | Me | H | Me | H | O | - | - | 0 | F | Cl | $SCH(Me)CO_2Et$ |
| Me | H | Me | H | Me | H | O | - | - | 0 | F | Cl | $SCH_2CN$ |
| Me | H | Me | H | Me | H | O | - | - | 0 | F | Cl | $SCH_2CH=CH_2$ |
| Me | H | Me | H | Me | H | O | - | - | 0 | F | Cl | $SCH_2C\equiv CH$ |
| Me | H | Me | H | Me | H | O | - | - | 0 | F | Cl | $OCH(Me)C\equiv CH$ |
| Me | H | Me | H | Me | H | O | - | - | 0 | F | Cl | $SCH(Me)C\equiv CH$ |
| Me | H | Me | H | Me | H | O | - | - | 0 | F | Cl | $N(SO_2Me)_2$ |
| Me | H | Me | H | Me | H | O | - | - | 0 | F | Cl | $N(SO_2Et)_2$ |
| Me | H | Me | H | Me | H | O | - | - | 0 | F | Cl | Q1($R29=Bu^t$) |
| Me | H | Me | H | Me | H | O | - | - | 0 | F | Cl | Q2($R29=Bu^t$) |
| Me | H | Me | H | Me | H | O | - | - | 0 | F | Cl | Q5($R29=Bu^t$) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | Me | H | O | - | - | 0 | F | Cl | Q7(R34=$Bu^t$) |
| Me | H | Me | H | Me | H | O | - | - | 0 | F | Cl | Q9(R38=R39=H) |
| Me | H | Me | H | Me | H | O | - | - | 0 | F | Cl | Q10(R38=R39=H) |
| Me | H | Me | H | Me | H | O | - | - | 0 | F | Cl | Q4(R29=$Bu^t$) |
| Me | H | Me | H | Me | H | O | - | - | 0 | F | Cl | Q8(R35=R36=R37=H) |
| Me | H | Me | H | Me | H | O | - | - | 0 | F | Cl | Q18(R50=H) |
| Me | H | Me | H | Me | H | O | - | - | 0 | F | Cl | Q18(R50=Me) |
| Me | H | Me | H | Me | H | O | - | - | 0 | F | Cl | $NHCO_2Me$ |
| Me | H | Me | H | Me | H | O | - | - | 0 | F | Cl | $NHCO_2Et$ |
| Me | H | Me | H | Me | H | O | - | - | 0 | F | Cl | $NHCO_2Pr^n$ |
| Me | H | Me | H | Me | H | O | - | - | 0 | F | Cl | $NHCO_2Pr^i$ |
| Me | H | Me | H | Me | H | O | - | - | 0 | F | Cl | $NHCO_2Ph$ |
| Me | H | Me | H | Me | H | O | - | - | 0 | F | Cl | $NHCO_2Bz$ |
| Me | H | Me | H | Me | H | O | - | - | 0 | F | Cl | NHCOMe |
| Me | H | Me | H | Me | H | O | - | - | 0 | F | Cl | $NHCO_2CH_2Ph$ |
| Me | H | Me | H | Me | H | O | - | - | 0 | F | Cl | $NHCO_2CH_2$(4-Me-Ph) |
| Me | H | Me | H | Me | H | O | - | - | 0 | F | Cl | $NHCO_2CH_2$(3-Me-Ph) |
| Me | H | Me | H | Me | H | O | - | - | 0 | F | Cl | $NHCO_2CH_2$(2-Me-Ph) |
| Me | H | Me | H | Me | H | O | - | - | 0 | F | Cl | $NHCO_2CH_2$(4-CL-Ph) |
| Me | H | Me | H | Me | H | O | - | - | 0 | F | Cl | $NHCO_2CH_2$(3-CL-Ph) |
| Me | H | Me | H | Me | H | O | - | - | 0 | F | Cl | $NHCO_2CH_2$(2-CL-Ph) |
| Me | H | Me | H | Me | H | O | - | - | 0 | F | Cl | Q1(R29=Me) |
| Me | H | Me | H | Me | H | O | - | - | 0 | F | Cl | Q2(R29=Me) |
| Me | H | Me | H | Me | H | O | - | - | 0 | F | Cl | H |

Table 2-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| Me | H | Me | H | Me | H | O | - | - | 0 | F | Cl | $CO_2H$ |
| Me | H | Me | H | Me | H | O | - | - | 0 | F | Cl | $CO_2Me$ |
| Me | H | Me | H | Me | H | O | - | - | 0 | F | Cl | $CO_2Et$ |
| $CF_3$ | H | Me | H | Me | H | O | - | - | 0 | F | Cl | $CO_2Pr^i$ |
| $CF_3$ | H | Me | H | Me | H | O | - | - | 0 | F | Cl | $CO_2Pr^n$ |
| $CF_3$ | H | Me | H | Me | H | O | - | - | 0 | F | Cl | $CO_2Bu^n$ |
| $CF_3$ | H | Me | H | Me | H | O | - | - | 0 | F | Cl | $CO_2Bu^t$ |
| $CF_3$ | H | Me | H | Me | H | O | - | - | 0 | F | Cl | $NO_2$ |
| $CF_3$ | H | Me | H | Me | H | O | - | - | 0 | F | Cl | $NH_2$ |
| $CF_3$ | H | Me | H | Me | H | O | - | - | 0 | F | Cl | $NHSO_2Me$ |
| $CF_3$ | H | Me | H | Me | H | O | - | - | 0 | F | Cl | $NHSO_2Et$ |
| $CF_3$ | H | Me | H | Me | H | O | - | - | 0 | F | Cl | $NHSO_2Pr^n$ |
| $CF_3$ | H | Me | H | Me | H | O | - | - | 0 | F | Cl | $NHSO_2Pr^i$ |
| $CF_3$ | H | Me | H | Me | H | O | - | - | 0 | F | Cl | OH |
| $CF_3$ | H | Me | H | Me | H | O | - | - | 0 | F | Cl | OMe |
| $CF_3$ | H | Me | H | Me | H | O | - | - | 0 | F | Cl | OEt |
| $CF_3$ | H | Me | H | Me | H | O | - | - | 0 | F | Cl | $OPr^i$ |
| $CF_3$ | H | Me | H | Me | H | O | - | - | 0 | F | Cl | $OPr^n$ |
| $CF_3$ | H | Me | H | Me | H | O | - | - | 0 | F | Cl | $OPen^c$ |
| $CF_3$ | H | Me | H | Me | H | O | - | - | 0 | F | Cl | $OCH_2CH=CH_2$ |
| $CF_3$ | H | Me | H | Me | H | O | - | - | 0 | F | Cl | $OCH_2C\equiv CH$ |
| $CF_3$ | H | Me | H | Me | H | O | - | - | 0 | F | Cl | $OCH_2CO_2Me$ |
| $CF_3$ | H | Me | H | Me | H | O | - | - | 0 | F | Cl | $OCH_2CO_2Et$ |
| $CF_3$ | H | Me | H | Me | H | O | - | - | 0 | F | Cl | $OCH(Me)CO_2Me$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | Me | H | O | - | - | 0 | F | Cl | $OCH(Me)CO_2Et$ |
| $CF_3$ | H | Me | H | Me | H | O | - | - | 0 | F | Cl | $CO_2H$ |
| $CF_3$ | H | Me | H | Me | H | O | - | - | 0 | F | Cl | CN |
| $CF_3$ | H | Me | H | Me | H | O | - | - | 0 | F | Cl | Me |
| $CF_3$ | H | Me | H | Me | H | O | - | - | 0 | F | Cl | Et |
| $CF_3$ | H | Me | H | Me | H | O | - | - | 0 | F | Cl | CL |
| $CF_3$ | H | Me | H | Me | H | O | - | - | 0 | F | Cl | Br |
| $CF_3$ | H | Me | H | Me | H | O | - | - | 0 | F | Cl | I |
| $CF_3$ | H | Me | H | Me | H | O | - | - | 0 | F | Cl | F |
| $CF_3$ | H | Me | H | Me | H | O | - | - | 0 | F | Cl | SH |
| $CF_3$ | H | Me | H | Me | H | O | - | - | 0 | F | Cl | $C(Me)=NOCH_2CO_2Me$ |
| $CF_3$ | H | Me | H | Me | H | O | - | - | 0 | F | Cl | $C(Me)=NOCH_2CO_2Et$ |
| $CF_3$ | H | Me | H | Me | H | O | - | - | 0 | F | Cl | $CH=NOCH_2CO_2Me$ |
| $CF_3$ | H | Me | H | Me | H | O | - | - | 0 | F | Cl | $CH=NOCH_2CO_2Et$ |
| $CF_3$ | H | Me | H | Me | H | O | - | - | 0 | F | Cl | $C(OMe)=NOCH_2CO_2Me$ |
| $CF_3$ | H | Me | H | Me | H | O | - | - | 0 | F | Cl | $C(OMe)=NOCH_2CO_2Et$ |
| $CF_3$ | H | Me | H | Me | H | O | - | - | 0 | F | Cl | $C(Me)=NN=C(Me)CO_2Me$ |
| $CF_3$ | H | Me | H | Me | H | O | - | - | 0 | F | Cl | $C(Me)=NN=C(Me)CO_2Et$ |
| $CF_3$ | H | Me | H | Me | H | O | - | - | 0 | F | Cl | $CH_2OCH_2CO_2Me$ |
| $CF_3$ | H | Me | H | Me | H | O | - | - | 0 | F | Cl | $CH_2OCH_2CO_2Et$ |
| $CF_3$ | H | Me | H | Me | H | O | - | - | 0 | F | Cl | $CH_2OCH(Me)CO_2Me$ |
| $CF_3$ | H | Me | H | Me | H | O | - | - | 0 | F | Cl | $CH_2OCH(Me)CO_2Et$ |
| $CF_3$ | H | Me | H | Me | H | O | - | - | 0 | F | Cl | $CH_2SCH_2CO_2Me$ |
| $CF_3$ | H | Me | H | Me | H | O | - | - | 0 | F | Cl | $CH_2SCH_2CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | Me | H | O | - | - | 0 | F | Cl | $CH_2SCH(Me)CO_2Me$ |
| $CF_3$ | H | Me | H | Me | H | O | - | - | 0 | F | Cl | $CH_2SCH(Me)CO_2Et$ |
| $CF_3$ | H | Me | H | Me | H | O | - | - | 0 | F | Cl | SMe |
| $CF_3$ | H | Me | H | Me | H | O | - | - | 0 | F | Cl | SEt |
| $CF_3$ | H | Me | H | Me | H | O | - | - | 0 | F | Cl | $SPr^i$ |
| $CF_3$ | H | Me | H | Me | H | O | - | - | 0 | F | Cl | $SPr^n$ |
| $CF_3$ | H | Me | H | Me | H | O | - | - | 0 | F | Cl | $SPen^c$ |
| $CF_3$ | H | Me | H | Me | H | O | - | - | 0 | F | Cl | $SCH_2CO_2H$ |
| $CF_3$ | H | Me | H | Me | H | O | - | - | 0 | F | Cl | $SCH_2CO_2Me$ |
| $CF_3$ | H | Me | H | Me | H | O | - | - | 0 | F | Cl | $SCH_2CO_2Et$ |
| $CF_3$ | H | Me | H | Me | H | O | - | - | 0 | F | Cl | $SCH(Me)CO_2Me$ |
| $CF_3$ | H | Me | H | Me | H | O | - | - | 0 | F | Cl | $SCH(Me)CO_2Et$ |
| $CF_3$ | H | Me | H | Me | H | O | - | - | 0 | F | Cl | $SCH_2CN$ |
| $CF_3$ | H | Me | H | Me | H | O | - | - | 0 | F | Cl | $SCH_2CH=CH_2$ |
| $CF_3$ | H | Me | H | Me | H | O | - | - | 0 | F | Cl | $SCH_2C\equiv CH$ |
| $CF_3$ | H | Me | H | Me | H | O | - | - | 0 | F | Cl | $OCH(Me)C\equiv CH$ |
| $CF_3$ | H | Me | H | Me | H | O | - | - | 0 | F | Cl | $SCH(Me)C\equiv CH$ |
| $CF_3$ | H | Me | H | Me | H | O | - | - | 0 | F | Cl | $N(SO_2Me)_2$ |
| $CF_3$ | H | Me | H | Me | H | O | - | - | 0 | F | Cl | $N(SO_2Et)_2$ |
| $CF_3$ | H | Me | H | Me | H | O | - | - | 0 | F | Cl | Q1(R29=$Bu^t$) |
| $CF_3$ | H | Me | H | Me | H | O | - | - | 0 | F | Cl | Q2(R29=$Bu^t$) |
| $CF_3$ | H | Me | H | Me | H | O | - | - | 0 | F | Cl | Q5(R29=$Bu^t$) |
| $CF_3$ | H | Me | H | Me | H | O | - | - | 0 | F | Cl | Q7(R34=$Bu^t$) |
| $CF_3$ | H | Me | H | Me | H | O | - | - | 0 | F | Cl | Q9(R38=R39=H) |

Table 2-3-1 (Cont' d)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | Me | H | O | - | - | 0 | F | Cl | Q10(R38=R39=H) |
| $CF_3$ | H | Me | H | Me | H | O | - | - | 0 | F | Cl | Q4(R29=$Bu^t$ ) |
| $CF_3$ | H | Me | H | Me | H | O | - | - | 0 | F | Cl | Q8(R35=R36=R37=H) |
| $CF_3$ | H | Me | H | Me | H | O | - | - | 0 | F | Cl | Q18(R50=H) |
| $CF_3$ | H | Me | H | Me | H | O | - | - | 0 | F | Cl | Q18(R50=Me) |
| $CF_3$ | H | Me | H | Me | H | O | - | - | 0 | F | Cl | $NHCO_2Me$ |
| $CF_3$ | H | Me | H | Me | H | O | - | - | 0 | F | Cl | $NHCO_2Et$ |
| $CF_3$ | H | Me | H | Me | H | O | - | - | 0 | F | Cl | $NHCO_2Pr^n$ |
| $CF_3$ | H | Me | H | Me | H | O | - | - | 0 | F | Cl | $NHCO_2Pr^i$ |
| $CF_3$ | H | Me | H | Me | H | O | - | - | 0 | F | Cl | $NHCO_2Ph$ |
| $CF_3$ | H | Me | H | Me | H | O | - | - | 0 | F | Cl | $NHCO_2Bz$ |
| $CF_3$ | H | Me | H | Me | H | O | - | - | 0 | F | Cl | NHCOMe |
| $CF_3$ | H | Me | H | Me | H | O | - | - | 0 | F | Cl | $NHCO_2CH_2Ph$ |
| $CF_3$ | H | Me | H | Me | H | O | - | - | 0 | F | Cl | $NHCO_2CH_2$(4-Me-Ph) |
| $CF_3$ | H | Me | H | Me | H | O | - | - | 0 | F | Cl | $NHCO_2CH_2$(3-Me-Ph) |
| $CF_3$ | H | Me | H | Me | H | O | - | - | 0 | F | Cl | $NHCO_2CH_2$(2-Me-Ph) |
| $CF_3$ | H | Me | H | Me | H | O | - | - | 0 | F | Cl | $NHCO_2CH_2$(4-CL-Ph) |
| $CF_3$ | H | Me | H | Me | H | O | - | - | 0 | F | Cl | $NHCO_2CH_2$(3-CL-Ph) |
| $CF_3$ | H | Me | H | Me | H | O | - | - | 0 | F | Cl | $NHCO_2CH_2$(2-CL-Ph) |
| $CF_3$ | H | Me | H | Me | H | O | - | - | 0 | F | Cl | Q1(R29=Me) |
| $CF_3$ | H | Me | H | Me | H | O | - | - | 0 | F | Cl | Q2(R29=Me) |
| $CF_3$ | H | Me | H | Me | H | O | - | - | 0 | F | Cl | H |
| $CF_3$ | H | Me | H | Me | H | O | - | - | 0 | F | Cl | $CO_2H$ |
| $CF_3$ | H | Me | H | Me | H | O | - | - | 0 | F | Cl | $CO_2Me$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $CF_3$ | H | Me | H | Me | H | O | - | - | 0 | F | Cl | $CO_2Et$ |
| Et | H | Me | H | Me | H | O | - | - | 0 | F | Cl | $CO_2Pr^i$ |
| Et | H | Me | H | Me | H | O | - | - | 0 | F | Cl | $CO_2Pr^n$ |
| Et | H | Me | H | Me | H | O | - | - | 0 | F | Cl | $CO_2Bu^n$ |
| Et | H | Me | H | Me | H | O | - | - | 0 | F | Cl | $CO_2Bu^t$ |
| Et | H | Me | H | Me | H | O | - | - | 0 | F | Cl | $NO_2$ |
| Et | H | Me | H | Me | H | O | - | - | 0 | F | Cl | $NH_2$ |
| Et | H | Me | H | Me | H | O | - | - | 0 | F | Cl | $NHSO_2Me$ |
| Et | H | Me | H | Me | H | O | - | - | 0 | F | Cl | $NHSO_2Et$ |
| Et | H | Me | H | Me | H | O | - | - | 0 | F | Cl | $NHSO_2Pr^n$ |
| Et | H | Me | H | Me | H | O | - | - | 0 | F | Cl | $NHSO_2Pr^i$ |
| Et | H | Me | H | Me | H | O | - | - | 0 | F | Cl | OH |
| Et | H | Me | H | Me | H | O | - | - | 0 | F | Cl | OMe |
| Et | H | Me | H | Me | H | O | - | - | 0 | F | Cl | OEt |
| Et | H | Me | H | Me | H | O | - | - | 0 | F | Cl | $OPr^i$ |
| Et | H | Me | H | Me | H | O | - | - | 0 | F | Cl | $OPr^n$ |
| Et | H | Me | H | Me | H | O | - | - | 0 | F | Cl | $OPen^c$ |
| Et | H | Me | H | Me | H | O | - | - | 0 | F | Cl | $OCH_2CH=CH_2$ |
| Et | H | Me | H | Me | H | O | - | - | 0 | F | Cl | $OCH_2C\equiv CH$ |
| Et | H | Me | H | Me | H | O | - | - | 0 | F | Cl | $OCH_2CO_2Me$ |
| Et | H | Me | H | Me | H | O | - | - | 0 | F | Cl | $OCH_2CO_2Et$ |
| Et | H | Me | H | Me | H | O | - | - | 0 | F | Cl | $OCH(Me)CO_2Me$ |
| Et | H | Me | H | Me | H | O | - | - | 0 | F | Cl | $OCH(Me)CO_2Et$ |
| Et | H | Me | H | Me | H | O | - | - | 0 | F | Cl | $CO_2H$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | Me | H | O | - | - | 0 | F | Cl | CN |
| Et | H | Me | H | Me | H | O | - | - | 0 | F | Cl | Me |
| Et | H | Me | H | Me | H | O | - | - | 0 | F | Cl | Et |
| Et | H | Me | H | Me | H | O | - | - | 0 | F | Cl | CL |
| Et | H | Me | H | Me | H | O | - | - | 0 | F | Cl | Br |
| Et | H | Me | H | Me | H | O | - | - | 0 | F | Cl | I |
| Et | H | Me | H | Me | H | O | - | - | 0 | F | Cl | F |
| Et | H | Me | H | Me | H | O | - | - | 0 | F | Cl | SH |
| Et | H | Me | H | Me | H | O | - | - | 0 | F | Cl | $C(Me)=NOCH_2CO_2Me$ |
| Et | H | Me | H | Me | H | O | - | - | 0 | F | Cl | $C(Me)=NOCH_2CO_2Et$ |
| Et | H | Me | H | Me | H | O | - | - | 0 | F | Cl | $CH=NOCH_2CO_2Me$ |
| Et | H | Me | H | Me | H | O | - | - | 0 | F | Cl | $CH=NOCH_2CO_2Et$ |
| Et | H | Me | H | Me | H | O | - | - | 0 | F | Cl | $C(OMe)=NOCH_2CO_2Me$ |
| Et | H | Me | H | Me | H | O | - | - | 0 | F | Cl | $C(OMe)=NOCH_2CO_2Et$ |
| Et | H | Me | H | Me | H | O | - | - | 0 | F | Cl | $C(Me)=NN=C(Me)CO_2Me$ |
| Et | H | Me | H | Me | H | O | - | - | 0 | F | Cl | $C(Me)=NN=C(Me)CO_2Et$ |
| Et | H | Me | H | Me | H | O | - | - | 0 | F | Cl | $CH_2OCH_2CO_2Me$ |
| Et | H | Me | H | Me | H | O | - | - | 0 | F | Cl | $CH_2OCH_2CO_2Et$ |
| Et | H | Me | H | Me | H | O | - | - | 0 | F | Cl | $CH_2OCH(Me)CO_2Me$ |
| Et | H | Me | H | Me | H | O | - | - | 0 | F | Cl | $CH_2OCH(Me)CO_2Et$ |
| Et | H | Me | H | Me | H | O | - | - | 0 | F | Cl | $CH_2SCH_2CO_2Me$ |
| Et | H | Me | H | Me | H | O | - | - | 0 | F | Cl | $CH_2SCH_2CO_2Et$ |
| Et | H | Me | H | Me | H | O | - | - | 0 | F | Cl | $CH_2SCH(Me)CO_2Me$ |
| Et | H | Me | H | Me | H | O | - | - | 0 | F | Cl | $CH_2SCH(Me)CO_2Et$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | Me | H | O | - | - | 0 | F | Cl | SMe |
| Et | H | Me | H | Me | H | O | - | - | 0 | F | Cl | SEt |
| Et | H | Me | H | Me | H | O | - | - | 0 | F | Cl | $SPr^i$ |
| Et | H | Me | H | Me | H | O | - | - | 0 | F | Cl | $SPr^n$ |
| Et | H | Me | H | Me | H | O | - | - | 0 | F | Cl | $SPen^c$ |
| Et | H | Me | H | Me | H | O | - | - | 0 | F | Cl | $SCH_2CO_2H$ |
| Et | H | Me | H | Me | H | O | - | - | 0 | F | Cl | $SCH_2CO_2Me$ |
| Et | H | Me | H | Me | H | O | - | - | 0 | F | Cl | $SCH_2CO_2Et$ |
| Et | H | Me | H | Me | H | O | - | - | 0 | F | Cl | $SCH(Me)CO_2Me$ |
| Et | H | Me | H | Me | H | O | - | - | 0 | F | Cl | $SCH(Me)CO_2Et$ |
| Et | H | Me | H | Me | H | O | - | - | 0 | F | Cl | $SCH_2CN$ |
| Et | H | Me | H | Me | H | O | - | - | 0 | F | Cl | $SCH_2CH=CH_2$ |
| Et | H | Me | H | Me | H | O | - | - | 0 | F | Cl | $SCH_2C\equiv CH$ |
| Et | H | Me | H | Me | H | O | - | - | 0 | F | Cl | $OCH(Me)C\equiv CH$ |
| Et | H | Me | H | Me | H | O | - | - | 0 | F | Cl | $SCH(Me)C\equiv CH$ |
| Et | H | Me | H | Me | H | O | - | - | 0 | F | Cl | $N(SO_2Me)_2$ |
| Et | H | Me | H | Me | H | O | - | - | 0 | F | Cl | $N(SO_2Et)_2$ |
| Et | H | Me | H | Me | H | O | - | - | 0 | F | Cl | Q1(R29=$Bu^t$) |
| Et | H | Me | H | Me | H | O | - | - | 0 | F | Cl | Q2(R29=$Bu^t$) |
| Et | H | Me | H | Me | H | O | - | - | 0 | F | Cl | Q5(R29=$Bu^t$) |
| Et | H | Me | H | Me | H | O | - | - | 0 | F | Cl | Q7(R34=$Bu^t$) |
| Et | H | Me | H | Me | H | O | - | - | 0 | F | Cl | Q9(R38=R39=H) |
| Et | H | Me | H | Me | H | O | - | - | 0 | F | Cl | Q10(R38=R39=H) |
| Et | H | Me | H | Me | H | O | - | - | 0 | F | Cl | Q4(R29=$Bu^t$) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| Et | H | Me | H | Me | H | O | - | - | 0 | F | Cl | Q8(R35=R36=R37=H) |
| Et | H | Me | H | Me | H | O | - | - | 0 | F | Cl | Q18(R50=H) |
| Et | H | Me | H | Me | H | O | - | - | 0 | F | Cl | Q18(R50=Me) |
| Et | H | Me | H | Me | H | O | - | - | 0 | F | Cl | $NHCO_2Me$ |
| Et | H | Me | H | Me | H | O | - | - | 0 | F | Cl | $NHCO_2Et$ |
| Et | H | Me | H | Me | H | O | - | - | 0 | F | Cl | $NHCO_2Pr^n$ |
| Et | H | Me | H | Me | H | O | - | - | 0 | F | Cl | $NHCO_2Pr^i$ |
| Et | H | Me | H | Me | H | O | - | - | 0 | F | Cl | $NHCO_2Ph$ |
| Et | H | Me | H | Me | H | O | - | - | 0 | F | Cl | $NHCO_2Bz$ |
| Et | H | Me | H | Me | H | O | - | - | 0 | F | Cl | NHCOMe |
| Et | H | Me | H | Me | H | O | - | - | 0 | F | Cl | $NHCO_2CH_2Ph$ |
| Et | H | Me | H | Me | H | O | - | - | 0 | F | Cl | $NHCO_2CH_2$(4-Me-Ph) |
| Et | H | Me | H | Me | H | O | - | - | 0 | F | Cl | $NHCO_2CH_2$(3-Me-Ph) |
| Et | H | Me | H | Me | H | O | - | - | 0 | F | Cl | $NHCO_2CH_2$(2-Me-Ph) |
| Et | H | Me | H | Me | H | O | - | - | 0 | F | Cl | $NHCO_2CH_2$(4-Cl-Ph) |
| Et | H | Me | H | Me | H | O | - | - | 0 | F | Cl | $NHCO_2CH_2$(3-Cl-Ph) |
| Et | H | Me | H | Me | H | O | - | - | 0 | F | Cl | $NHCO_2CH_2$(2-Cl-Ph) |
| Et | H | Me | H | Me | H | O | - | - | 0 | F | Cl | Q1(R29=Me) |
| Et | H | Me | H | Me | H | O | - | - | 0 | F | Cl | Q2(R29=Me) |
| Et | H | Me | H | Me | H | O | - | - | 0 | F | Cl | H |
| Et | H | Me | H | Me | H | O | - | - | 0 | F | Cl | $CO_2H$ |
| Et | H | Me | H | Me | H | O | - | - | 0 | F | Cl | $CO_2Me$ |
| Et | H | Me | H | Me | H | O | - | - | 0 | F | Cl | $CO_2Et$ |
| $Pr^n$ | H | Me | H | Me | H | O | - | - | 0 | F | Cl | $CO_2Pr^i$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | Me | H | O | - | - | 0 | F | Cl | $CO_2Pr^n$ |
| $Pr^n$ | H | Me | H | Me | H | O | - | - | 0 | F | Cl | $CO_2Bu^n$ |
| $Pr^n$ | H | Me | H | Me | H | O | - | - | 0 | F | Cl | $CO_2Bu^t$ |
| $Pr^n$ | H | Me | H | Me | H | O | - | - | 0 | F | Cl | $NO_2$ |
| $Pr^n$ | H | Me | H | Me | H | O | - | - | 0 | F | Cl | $NH_2$ |
| $Pr^n$ | H | Me | H | Me | H | O | - | - | 0 | F | Cl | $NHSO_2Me$ |
| $Pr^n$ | H | Me | H | Me | H | O | - | - | 0 | F | Cl | $NHSO_2Et$ |
| $Pr^n$ | H | Me | H | Me | H | O | - | - | 0 | F | Cl | $NHSO_2Pr^n$ |
| $Pr^n$ | H | Me | H | Me | H | O | - | - | 0 | F | Cl | $NHSO_2Pr^i$ |
| $Pr^n$ | H | Me | H | Me | H | O | - | - | 0 | F | Cl | OH |
| $Pr^n$ | H | Me | H | Me | H | O | - | - | 0 | F | Cl | OMe |
| $Pr^n$ | H | Me | H | Me | H | O | - | - | 0 | F | Cl | OEt |
| $Pr^n$ | H | Me | H | Me | H | O | - | - | 0 | F | Cl | $OPr^i$ |
| $Pr^n$ | H | Me | H | Me | H | O | - | - | 0 | F | Cl | $OPr^n$ |
| $Pr^n$ | H | Me | H | Me | H | O | - | - | 0 | F | Cl | $OPen^c$ |
| $Pr^n$ | H | Me | H | Me | H | O | - | - | 0 | F | Cl | $OCH_2CH=CH_2$ |
| $Pr^n$ | H | Me | H | Me | H | O | - | - | 0 | F | Cl | $OCH_2C\equiv CH$ |
| $Pr^n$ | H | Me | H | Me | H | O | - | - | 0 | F | Cl | $OCH_2CO_2Me$ |
| $Pr^n$ | H | Me | H | Me | H | O | - | - | 0 | F | Cl | $OCH_2CO_2Et$ |
| $Pr^n$ | H | Me | H | Me | H | O | - | - | 0 | F | Cl | $OCH(Me)CO_2Me$ |
| $Pr^n$ | H | Me | H | Me | H | O | - | - | 0 | F | Cl | $OCH(Me)CO_2Et$ |
| $Pr^n$ | H | Me | H | Me | H | O | - | - | 0 | F | Cl | $CO_2H$ |
| $Pr^n$ | H | Me | H | Me | H | O | - | - | 0 | F | Cl | CN |
| $Pr^n$ | H | Me | H | Me | H | O | - | - | 0 | F | Cl | Me |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | Me | H | O | - | - | 0 | F | Cl | Et |
| $Pr^n$ | H | Me | H | Me | H | O | - | - | 0 | F | Cl | Cl |
| $Pr^n$ | H | Me | H | Me | H | O | - | - | 0 | F | Cl | Br |
| $Pr^n$ | H | Me | H | Me | H | O | - | - | 0 | F | Cl | I |
| $Pr^n$ | H | Me | H | Me | H | O | - | - | 0 | F | Cl | F |
| $Pr^n$ | H | Me | H | Me | H | O | - | - | 0 | F | Cl | SH |
| $Pr^n$ | H | Me | H | Me | H | O | - | - | 0 | F | Cl | $C(Me)=NOCH_2CO_2Me$ |
| $Pr^n$ | H | Me | H | Me | H | O | - | - | 0 | F | Cl | $C(Me)=NOCH_2CO_2Et$ |
| $Pr^n$ | H | Me | H | Me | H | O | - | - | 0 | F | Cl | $CH=NOCH_2CO_2Me$ |
| $Pr^n$ | H | Me | H | Me | H | O | - | - | 0 | F | Cl | $CH=NOCH_2CO_2Et$ |
| $Pr^n$ | H | Me | H | Me | H | O | - | - | 0 | F | Cl | $C(OMe)=NOCH_2CO_2Me$ |
| $Pr^n$ | H | Me | H | Me | H | O | - | - | 0 | F | Cl | $C(OMe)=NOCH_2CO_2Et$ |
| $Pr^n$ | H | Me | H | Me | H | O | - | - | 0 | F | Cl | $C(Me)=NN=C(Me)CO_2Me$ |
| $Pr^n$ | H | Me | H | Me | H | O | - | - | 0 | F | Cl | $C(Me)=NN=C(Me)CO_2Et$ |
| $Pr^n$ | H | Me | H | Me | H | O | - | - | 0 | F | Cl | $CH_2OCH_2CO_2Me$ |
| $Pr^n$ | H | Me | H | Me | H | O | - | - | 0 | F | Cl | $CH_2OCH_2CO_2Et$ |
| $Pr^n$ | H | Me | H | Me | H | O | - | - | 0 | F | Cl | $CH_2OCH(Me)CO_2Me$ |
| $Pr^n$ | H | Me | H | Me | H | O | - | - | 0 | F | Cl | $CH_2OCH(Me)CO_2Et$ |
| $Pr^n$ | H | Me | H | Me | H | O | - | - | 0 | F | Cl | $CH_2SCH_2CO_2Me$ |
| $Pr^n$ | H | Me | H | Me | H | O | - | - | 0 | F | Cl | $CH_2SCH_2CO_2Et$ |
| $Pr^n$ | H | Me | H | Me | H | O | - | - | 0 | F | Cl | $CH_2SCH(Me)CO_2Me$ |
| $Pr^n$ | H | Me | H | Me | H | O | - | - | 0 | F | Cl | $CH_2SCH(Me)CO_2Et$ |
| $Pr^n$ | H | Me | H | Me | H | O | - | - | 0 | F | Cl | SMe |
| $Pr^n$ | H | Me | H | Me | H | O | - | - | 0 | F | Cl | SEt |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | Me | H | O | - | - | 0 | F | Cl | $SPr^i$ |
| $Pr^n$ | H | Me | H | Me | H | O | - | - | 0 | F | Cl | $SPr^n$ |
| $Pr^n$ | H | Me | H | Me | H | O | - | - | 0 | F | Cl | $SPen^c$ |
| $Pr^n$ | H | Me | H | Me | H | O | - | - | 0 | F | Cl | $SCH_2CO_2H$ |
| $Pr^n$ | H | Me | H | Me | H | O | - | - | 0 | F | Cl | $SCH_2CO_2Me$ |
| $Pr^n$ | H | Me | H | Me | H | O | - | - | 0 | F | Cl | $SCH_2CO_2Et$ |
| $Pr^n$ | H | Me | H | Me | H | O | - | - | 0 | F | Cl | $SCH(Me)CO_2Me$ |
| $Pr^n$ | H | Me | H | Me | H | O | - | - | 0 | F | Cl | $SCH(Me)CO_2Et$ |
| $Pr^n$ | H | Me | H | Me | H | O | - | - | 0 | F | Cl | $SCH_2CN$ |
| $Pr^n$ | H | Me | H | Me | H | O | - | - | 0 | F | Cl | $SCH_2CH=CH_2$ |
| $Pr^n$ | H | Me | H | Me | H | O | - | - | 0 | F | Cl | $SCH_2C\equiv CH$ |
| $Pr^n$ | H | Me | H | Me | H | O | - | - | 0 | F | Cl | $OCH(Me)C\equiv CH$ |
| $Pr^n$ | H | Me | H | Me | H | O | - | - | 0 | F | Cl | $SCH(Me)C\equiv CH$ |
| $Pr^n$ | H | Me | H | Me | H | O | - | - | 0 | F | Cl | $N(SO_2Me)_2$ |
| $Pr^n$ | H | Me | H | Me | H | O | - | - | 0 | F | Cl | $N(SO_2Et)_2$ |
| $Pr^n$ | H | Me | H | Me | H | O | - | - | 0 | F | Cl | Q1($R29=Bu^t$) |
| $Pr^n$ | H | Me | H | Me | H | O | - | - | 0 | F | Cl | Q2($R29=Bu^t$) |
| $Pr^n$ | H | Me | H | Me | H | O | - | - | 0 | F | Cl | Q5($R29=Bu^t$) |
| $Pr^n$ | H | Me | H | Me | H | O | - | - | 0 | F | Cl | Q7($R34=Bu^t$) |
| $Pr^n$ | H | Me | H | Me | H | O | - | - | 0 | F | Cl | Q9(R38=R39=H) |
| $Pr^n$ | H | Me | H | Me | H | O | - | - | 0 | F | Cl | Q10(R38=R39=H) |
| $Pr^n$ | H | Me | H | Me | H | O | - | - | 0 | F | Cl | Q4($R29=Bu^t$) |
| $Pr^n$ | H | Me | H | Me | H | O | - | - | 0 | F | Cl | Q8(R35=R36=R37=H) |
| $Pr^n$ | H | Me | H | Me | H | O | - | - | 0 | F | Cl | Q18(R50=H) |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^n$ | H | Me | H | Me | H | O | - | - | 0 | F | Cl | Q18(R50=Me) |
| $Pr^n$ | H | Me | H | Me | H | O | - | - | 0 | F | Cl | $NHCO_2Me$ |
| $Pr^n$ | H | Me | H | Me | H | O | - | - | 0 | F | Cl | $NHCO_2Et$ |
| $Pr^n$ | H | Me | H | Me | H | O | - | - | 0 | F | Cl | $NHCO_2Pr^n$ |
| $Pr^n$ | H | Me | H | Me | H | O | - | - | 0 | F | Cl | $NHCO_2Pr^i$ |
| $Pr^n$ | H | Me | H | Me | H | O | - | - | 0 | F | Cl | $NHCO_2Ph$ |
| $Pr^n$ | H | Me | H | Me | H | O | - | - | 0 | F | Cl | $NHCO_2Bz$ |
| $Pr^n$ | H | Me | H | Me | H | O | - | - | 0 | F | Cl | NHCOMe |
| $Pr^n$ | H | Me | H | Me | H | O | - | - | 0 | F | Cl | $NHCO_2CH_2Ph$ |
| $Pr^n$ | H | Me | H | Me | H | O | - | - | 0 | F | Cl | $NHCO_2CH_2$(4-Me-Ph) |
| $Pr^n$ | H | Me | H | Me | H | O | - | - | 0 | F | Cl | $NHCO_2CH_2$(3-Me-Ph) |
| $Pr^n$ | H | Me | H | Me | H | O | - | - | 0 | F | Cl | $NHCO_2CH_2$(2-Me-Ph) |
| $Pr^n$ | H | Me | H | Me | H | O | - | - | 0 | F | Cl | $NHCO_2CH_2$(4-Cl-Ph) |
| $Pr^n$ | H | Me | H | Me | H | O | - | - | 0 | F | Cl | $NHCO_2CH_2$(3-Cl-Ph) |
| $Pr^n$ | H | Me | H | Me | H | O | - | - | 0 | F | Cl | $NHCO_2CH_2$(2-Cl-Ph) |
| $Pr^n$ | H | Me | H | Me | H | O | - | - | 0 | F | Cl | Q1(R29=Me) |
| $Pr^n$ | H | Me | H | Me | H | O | - | - | 0 | F | Cl | Q2(R29=Me) |
| $Pr^n$ | H | Me | H | Me | H | O | - | - | 0 | F | Cl | H |
| $Pr^n$ | H | Me | H | Me | H | O | - | - | 0 | F | Cl | $CO_2H$ |
| $Pr^n$ | H | Me | H | Me | H | O | - | - | 0 | F | Cl | $CO_2Me$ |
| $Pr^n$ | H | Me | H | Me | H | O | - | - | 0 | F | Cl | $CO_2Et$ |
| $Pr^i$ | H | Me | H | Me | H | O | - | - | 0 | F | Cl | $CO_2Pr^i$ |
| $Pr^i$ | H | Me | H | Me | H | O | - | - | 0 | F | Cl | $CO_2Pr^n$ |
| $Pr^i$ | H | Me | H | Me | H | O | - | - | 0 | F | Cl | $CO_2Bu^n$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | Me | H | O | - | - | 0 | F | Cl | $CO_2Bu^t$ |
| $Pr^i$ | H | Me | H | Me | H | O | - | - | 0 | F | Cl | $NO_2$ |
| $Pr^i$ | H | Me | H | Me | H | O | - | - | 0 | F | Cl | $NH_2$ |
| $Pr^i$ | H | Me | H | Me | H | O | - | - | 0 | F | Cl | $NHSO_2Me$ |
| $Pr^i$ | H | Me | H | Me | H | O | - | - | 0 | F | Cl | $NHSO_2Et$ |
| $Pr^i$ | H | Me | H | Me | H | O | - | - | 0 | F | Cl | $NHSO_2Pr^n$ |
| $Pr^i$ | H | Me | H | Me | H | O | - | - | 0 | F | Cl | $NHSO_2Pr^i$ |
| $Pr^i$ | H | Me | H | Me | H | O | - | - | 0 | F | Cl | OH |
| $Pr^i$ | H | Me | H | Me | H | O | - | - | 0 | F | Cl | OMe |
| $Pr^i$ | H | Me | H | Me | H | O | - | - | 0 | F | Cl | OEt |
| $Pr^i$ | H | Me | H | Me | H | O | - | - | 0 | F | Cl | $OPr^i$ |
| $Pr^i$ | H | Me | H | Me | H | O | - | - | 0 | F | Cl | $OPr^n$ |
| $Pr^i$ | H | Me | H | Me | H | O | - | - | 0 | F | Cl | $OPen^c$ |
| $Pr^i$ | H | Me | H | Me | H | O | - | - | 0 | F | Cl | $OCH_2CH=CH_2$ |
| $Pr^i$ | H | Me | H | Me | H | O | - | - | 0 | F | Cl | $OCH_2C\equiv CH$ |
| $Pr^i$ | H | Me | H | Me | H | O | - | - | 0 | F | Cl | $OCH_2CO_2Me$ |
| $Pr^i$ | H | Me | H | Me | H | O | - | - | 0 | F | Cl | $OCH_2CO_2Et$ |
| $Pr^i$ | H | Me | H | Me | H | O | - | - | 0 | F | Cl | $OCH(Me)CO_2Me$ |
| $Pr^i$ | H | Me | H | Me | H | O | - | - | 0 | F | Cl | $OCH(Me)CO_2Et$ |
| $Pr^i$ | H | Me | H | Me | H | O | - | - | 0 | F | Cl | $CO_2H$ |
| $Pr^i$ | H | Me | H | Me | H | O | - | - | 0 | F | Cl | CN |
| $Pr^i$ | H | Me | H | Me | H | O | - | - | 0 | F | Cl | Me |
| $Pr^i$ | H | Me | H | Me | H | O | - | - | 0 | F | Cl | Et |
| $Pr^i$ | H | Me | H | Me | H | O | - | - | 0 | F | Cl | Cl |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | Me | H | O | - | - | 0 | F | Cl | Br |
| $Pr^i$ | H | Me | H | Me | H | O | - | - | 0 | F | Cl | I |
| $Pr^i$ | H | Me | H | Me | H | O | - | - | 0 | F | Cl | F |
| $Pr^i$ | H | Me | H | Me | H | O | - | - | 0 | F | Cl | SH |
| $Pr^i$ | H | Me | H | Me | H | O | - | - | 0 | F | Cl | $C(Me)=NOCH_2CO_2Me$ |
| $Pr^i$ | H | Me | H | Me | H | O | - | - | 0 | F | Cl | $C(Me)=NOCH_2CO_2Et$ |
| $Pr^i$ | H | Me | H | Me | H | O | - | - | 0 | F | Cl | $CH=NOCH_2CO_2Me$ |
| $Pr^i$ | H | Me | H | Me | H | O | - | - | 0 | F | Cl | $CH=NOCH_2CO_2Et$ |
| $Pr^i$ | H | Me | H | Me | H | O | - | - | 0 | F | Cl | $C(OMe)=NOCH_2CO_2Me$ |
| $Pr^i$ | H | Me | H | Me | H | O | - | - | 0 | F | Cl | $C(OMe)=NOCH_2CO_2Et$ |
| $Pr^i$ | H | Me | H | Me | H | O | - | - | 0 | F | Cl | $C(Me)=NN=C(Me)CO_2Me$ |
| $Pr^i$ | H | Me | H | Me | H | O | - | - | 0 | F | Cl | $C(Me)=NN=C(Me)CO_2Et$ |
| $Pr^i$ | H | Me | H | Me | H | O | - | - | 0 | F | Cl | $CH_2OCH_2CO_2Me$ |
| $Pr^i$ | H | Me | H | Me | H | O | - | - | 0 | F | Cl | $CH_2OCH_2CO_2Et$ |
| $Pr^i$ | H | Me | H | Me | H | O | - | - | 0 | F | Cl | $CH_2OCH(Me)CO_2Me$ |
| $Pr^i$ | H | Me | H | Me | H | O | - | - | 0 | F | Cl | $CH_2OCH(Me)CO_2Et$ |
| $Pr^i$ | H | Me | H | Me | H | O | - | - | 0 | F | Cl | $CH_2SCH_2CO_2Me$ |
| $Pr^i$ | H | Me | H | Me | H | O | - | - | 0 | F | Cl | $CH_2SCH_2CO_2Et$ |
| $Pr^i$ | H | Me | H | Me | H | O | - | - | 0 | F | Cl | $CH_2SCH(Me)CO_2Me$ |
| $Pr^i$ | H | Me | H | Me | H | O | - | - | 0 | F | Cl | $CH_2SCH(Me)CO_2Et$ |
| $Pr^i$ | H | Me | H | Me | H | O | - | - | 0 | F | Cl | SMe |
| $Pr^i$ | H | Me | H | Me | H | O | - | - | 0 | F | Cl | SEt |
| $Pr^i$ | H | Me | H | Me | H | O | - | - | 0 | F | Cl | $SPr^i$ |
| $Pr^i$ | H | Me | H | Me | H | O | - | - | 0 | F | Cl | $SPr^n$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | Me | H | O | - | - | 0 | F | Cl | $SPen^c$ |
| $Pr^i$ | H | Me | H | Me | H | O | - | - | 0 | F | Cl | $SCH_2CO_2H$ |
| $Pr^i$ | H | Me | H | Me | H | O | - | - | 0 | F | Cl | $SCH_2CO_2Me$ |
| $Pr^i$ | H | Me | H | Me | H | O | - | - | 0 | F | Cl | $SCH_2CO_2Et$ |
| $Pr^i$ | H | Me | H | Me | H | O | - | - | 0 | F | Cl | $SCH(Me)CO_2Me$ |
| $Pr^i$ | H | Me | H | Me | H | O | - | - | 0 | F | Cl | $SCH(Me)CO_2Et$ |
| $Pr^i$ | H | Me | H | Me | H | O | - | - | 0 | F | Cl | $SCH_2CN$ |
| $Pr^i$ | H | Me | H | Me | H | O | - | - | 0 | F | Cl | $SCH_2CH=CH_2$ |
| $Pr^i$ | H | Me | H | Me | H | O | - | - | 0 | F | Cl | $SCH_2C\equiv CH$ |
| $Pr^i$ | H | Me | H | Me | H | O | - | - | 0 | F | Cl | $OCH(Me)C\equiv CH$ |
| $Pr^i$ | H | Me | H | Me | H | O | - | - | 0 | F | Cl | $SCH(Me)C\equiv CH$ |
| $Pr^i$ | H | Me | H | Me | H | O | - | - | 0 | F | Cl | $N(SO_2Me)_2$ |
| $Pr^i$ | H | Me | H | Me | H | O | - | - | 0 | F | Cl | $N(SO_2Et)_2$ |
| $Pr^i$ | H | Me | H | Me | H | O | - | - | 0 | F | Cl | Q1(R29=$Bu^t$) |
| $Pr^i$ | H | Me | H | Me | H | O | - | - | 0 | F | Cl | Q2(R29=$Bu^t$) |
| $Pr^i$ | H | Me | H | Me | H | O | - | - | 0 | F | Cl | Q5(R29=$Bu^t$) |
| $Pr^i$ | H | Me | H | Me | H | O | - | - | 0 | F | Cl | Q7(R34=$Bu^t$) |
| $Pr^i$ | H | Me | H | Me | H | O | - | - | 0 | F | Cl | Q9(R38=R39=H) |
| $Pr^i$ | H | Me | H | Me | H | O | - | - | 0 | F | Cl | Q10(R38=R39=H) |
| $Pr^i$ | H | Me | H | Me | H | O | - | - | 0 | F | Cl | Q4(R29=$Bu^t$) |
| $Pr^i$ | H | Me | H | Me | H | O | - | - | 0 | F | Cl | Q8(R35=R36=R37=H) |
| $Pr^i$ | H | Me | H | Me | H | O | - | - | 0 | F | Cl | Q18(R50=H) |
| $Pr^i$ | H | Me | H | Me | H | O | - | - | 0 | F | Cl | Q18(R50=Me) |
| $Pr^i$ | H | Me | H | Me | H | O | - | - | 0 | F | Cl | $NHCO_2Me$ |

Table 2-3-1 (Cont'd)

| R1 | R2 | R3 | R4 | R5 | R6 | X | $R_a$ | $R_b$ | m | R7 | R8 | R9 |
|---|---|---|---|---|---|---|---|---|---|---|---|---|
| $Pr^i$ | H | Me | H | Me | H | O | - | - | 0 | F | Cl | $NHCO_2Et$ |
| $Pr^i$ | H | Me | H | Me | H | O | - | - | 0 | F | Cl | $NHCO_2Pr^n$ |
| $Pr^i$ | H | Me | H | Me | H | O | - | - | 0 | F | Cl | $NHCO_2Pr^i$ |
| $Pr^i$ | H | Me | H | Me | H | O | - | - | 0 | F | Cl | $NHCO_2Ph$ |
| $Pr^i$ | H | Me | H | Me | H | O | - | - | 0 | F | Cl | $NHCO_2Bz$ |
| $Pr^i$ | H | Me | H | Me | H | O | - | - | 0 | F | Cl | NHCOMe |
| $Pr^i$ | H | Me | H | Me | H | O | - | - | 0 | F | Cl | $NHCO_2CH_2Ph$ |
| $Pr^i$ | H | Me | H | Me | H | O | - | - | 0 | F | Cl | $NHCO_2CH_2$(4-Me-Ph) |
| $Pr^i$ | H | Me | H | Me | H | O | - | - | 0 | F | Cl | $NHCO_2CH_2$(3-Me-Ph) |
| $Pr^i$ | H | Me | H | Me | H | O | - | - | 0 | F | Cl | $NHCO_2CH_2$(2-Me-Ph) |
| $Pr^i$ | H | Me | H | Me | H | O | - | - | 0 | F | Cl | $NHCO_2CH_2$(4-Cl-Ph) |
| $Pr^i$ | H | Me | H | Me | H | O | - | - | 0 | F | Cl | $NHCO_2CH_2$(3-Cl-Ph) |
| $Pr^i$ | H | Me | H | Me | H | O | - | - | 0 | F | Cl | $NHCO_2CH_2$(2-Cl-Ph) |
| $Pr^i$ | H | Me | H | Me | H | O | - | - | 0 | F | Cl | Q1(R29=Me) |
| $Pr^i$ | H | Me | H | Me | H | O | - | - | 0 | F | Cl | Q2(R29=Me) |
| $Pr^i$ | H | Me | H | Me | H | O | - | - | 0 | F | Cl | H |
| $Pr^i$ | H | Me | H | Me | H | O | - | - | 0 | F | Cl | $CO_2H$ |
| $Pr^i$ | H | Me | H | Me | H | O | - | - | 0 | F | Cl | $CO_2Me$ |
| $Pr^i$ | H | Me | H | Me | H | O | - | - | 0 | F | Cl | $CO_2Et$ |

Table 2-4

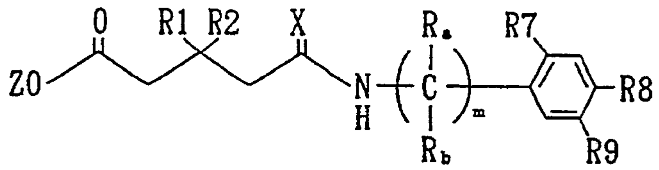

| Z | R1 | R2 | X | $R_a$ | $R_b$ | m | R7 | R8 | R9 |
|---|---|---|---|---|---|---|---|---|---|
| H | Me | H | S | – | – | 0 | F | Cl | H |
| H | Me | H | O | H | H | 1 | H | Cl | H |
| H | Me | H | O | Me | H | 1 | H | Cl | H |
| H | Me | H | O | Me | Me | 1 | H | Cl | H |
| H | Me | H | O | H | H | 2 | H | Cl | H |
| H | Me | H | O | – | – | 0 | F | Cl | Ph |
| H | Et | Me | O | – | – | 0 | F | Cl | H |
| H | Me | H | O | – | – | 0 | F | Cl | $NH_2$ |
| H | Me | H | O | H | H | 1 | H | Cl | $CO_2Et$ |
| H | Me | H | O | H | H | 1 | H | $NO_2$ | $CO_2H$ |
| H | Me | H | O | H | H | 1 | H | $NO_2$ | $CO_2Et$ |
| H | Me | H | S | - | - | 0 | F | Cl | $CO_2Et$ |
| H | Me | H | S | H | H | 1 | H | $NO_2$ | $CO_2Et$ |
| H | Me | H | O | - | - | 0 | F | Cl | $NHSO_2Me$ |

Table 2−4 (Cont' d)

| Z | R1 | R2 | X | $R_a$ | $R_b$ | m | R7 | R8 | R9 |
|---|---|---|---|---|---|---|---|---|---|
| H | Me | H | O | – | – | 0 | F | Cl | $N(SO_2Me)_2$ |
| H | Me | H | O | – | – | 0 | F | Cl | $NHSO_2Et$ |
| H | Me | H | O | – | – | 0 | F | Cl | Q1(R29=$Bu^t$ ) |
| H | Me | H | O | – | – | 0 | F | Cl | Q2(R29=$Bu^t$ ) |
| H | Me | H | O | – | – | 0 | F | Cl | Q4(R29=$Bu^t$ ) |
| H | Me | H | O | – | – | 0 | F | Cl | Q4(R29=$CF_3$ ) |
| H | Me | H | O | – | – | 0 | F | Cl | Q5(R29=Cl) |
| H | Me | H | O | – | – | 0 | F | Cl | Q1(R29=$N(Me)_2$) |
| H | Me | H | S | – | – | 0 | F | Cl | Q1(R29=$Bu^t$ ) |
| H | Me | H | O | – | – | 0 | F | Cl | Q7(R34=$Bu^t$ ) |
| H | Me | H | O | – | – | 0 | F | Cl | Q9(R38=R39=H) |
| H | Me | H | O | – | – | 0 | F | Cl | Q15(R48=Me, R49=H) |
| H | $CF_3$ | H | S | – | – | 0 | F | Cl | H |
| H | $CF_3$ | H | O | H | H | 1 | H | Cl | H |
| H | $CF_3$ | H | O | H | H | 1 | H | $NO_2$ | H |
| H | $CF_3$ | H | O | H | H | 1 | H | $NO_2$ | $CO_2H$ |
| H | $CF_3$ | H | O | H | H | 1 | H | $NO_2$ | $CO_2Et$ |
| H | $CF_3$ | H | O | H | H | 2 | H | $NO_2$ | $CO_2H$ |
| H | $CF_3$ | H | O | – | – | 0 | F | Cl | Ph |
| H | $CF_3$ | H | O | – | – | 0 | F | Cl | $NH_2$ |
| H | $CF_3$ | H | S | – | – | 0 | F | Cl | $OPr^i$ |
| H | $CF_3$ | H | S | – | – | 0 | F | Cl | $OPen^c$ |
| H | $CF_3$ | H | S | – | – | 0 | F | Cl | $OCH_2C\equiv CH$ |
| H | $CF_3$ | H | S | – | – | 0 | F | Cl | $OCH(Me)C\equiv CH$ |

Table 2-4 (Cont'd)

| Z | R1 | R2 | X | $R_a$ | $R_b$ | m | R7 | R8 | R9 |
|---|---|---|---|---|---|---|---|---|---|
| H | $CF_3$ | H | S | – | – | 0 | F | Cl | $OCH_2CO_2Me$ |
| H | $CF_3$ | H | S | – | – | 0 | F | Cl | $OCH(Me)CO_2Et$ |
| H | $CF_3$ | H | S | – | – | 0 | F | Cl | $OCH(CH_2OMe)CO_2Et$ |
| H | $CF_3$ | H | S | – | – | 0 | H | Cl | $CO_2H$ |
| H | $CF_3$ | H | S | – | – | 0 | H | Cl | $CO_2Me$ |
| H | $CF_3$ | H | S | – | – | 0 | H | Cl | $CO_2Et$ |
| H | $CF_3$ | H | S | – | – | 0 | H | Cl | $CO_2Pr^i$ |
| H | $CF_3$ | H | S | – | – | 0 | F | Cl | $CO_2Me$ |
| H | $CF_3$ | H | S | – | – | 0 | F | Cl | $CO_2Et$ |
| H | $CF_3$ | H | S | – | – | 0 | F | Cl | $CO_2Pr^n$ |
| H | $CF_3$ | H | S | – | – | 0 | F | Cl | $CO_2Pr^i$ |
| H | $CF_3$ | H | S | – | – | 0 | F | Cl | $CO_2Bu^n$ |
| H | $CF_3$ | H | S | – | – | 0 | F | Cl | $CO_2Bu^t$ |
| H | $CF_3$ | H | S | – | – | 0 | F | Cl | $CO_2Pen^c$ |
| H | $CF_3$ | H | S | – | – | 0 | F | Cl | $CO_2Bz$ |
| H | $CF_3$ | H | S | – | – | 0 | F | Cl | $CO_2Na$ |
| H | $CF_3$ | H | S | – | – | 0 | F | Cl | $C(OMe)=NOCH_2CO_2Me$ |
| H | $CF_3$ | H | S | – | – | 0 | F | Cl | $C(Me)=NN=C(Me)CO_2Et$ |
| H | $CF_3$ | H | S | – | – | 0 | F | Cl | $SPr^i$ |
| H | $CF_3$ | H | S | – | – | 0 | H | Cl | $SCH_2CO_2Me$ |
| H | $CF_3$ | H | S | – | – | 0 | F | Cl | $SCH_2CO_2Me$ |
| H | $CF_3$ | H | O | – | – | 0 | F | Cl | $SCH_2OCH_2CO_2Me$ |
| H | $CF_3$ | H | O | – | – | 0 | F | Cl | $CH_2OCH_2CO_2Et$ |
| H | $CF_3$ | H | O | – | – | 0 | F | Cl | $CH_2SCH_2CO_2Et$ |

Table 2-4 (Cont'd)

| Z | R1 | R2 | X | $R_a$ | $R_b$ | m | R7 | R8 | R9 |
|---|---|---|---|---|---|---|---|---|---|
| H | $CF_3$ | H | O | H | H | 1 | F | Cl | $SCH_2CO_2Me$ |
| H | $CF_3$ | H | O | - | - | 0 | F | Cl | $NHSO_2Me$ |
| H | $CF_3$ | H | O | - | - | 0 | F | Cl | $NHSO_2Et$ |
| H | $CF_3$ | H | O | - | - | 0 | F | Cl | $N(SO_2Me)_2$ |
| H | $CF_3$ | H | O | - | - | 0 | F | Cl | $N(SO_2Me)Me$ |
| H | $CF_3$ | H | O | - | - | 0 | F | Cl | N(SOMe)Et |
| H | $CF_3$ | H | S | - | - | 0 | F | Cl | $NHSO_2Me$ |
| H | $CF_3$ | H | S | - | - | 0 | F | Cl | $NHSO_2Et$ |
| H | $CF_3$ | H | S | - | - | 0 | F | Cl | $N(SO_2Me)_2$ |
| H | $CF_3$ | H | S | - | - | 0 | F | Cl | $N(SO_2Me)Et$ |
| H | $CF_3$ | H | O | - | - | 0 | F | Cl | Q1(R29=$Bu^t$) |
| H | $CF_3$ | H | O | - | - | 0 | F | Cl | Q1(R29=$Pen^n$) |
| H | $CF_3$ | H | O | - | - | 0 | F | Cl | Q1(R29=NHMe) |
| H | $CF_3$ | H | O | - | - | 0 | F | Cl | Q1(R29=$N(Me)_2$) |
| H | $CF_3$ | H | O | - | - | 0 | F | Cl | Q1(R29=$CON(Me)_2$) |
| H | $CF_3$ | H | O | - | - | 0 | F | Cl | Q2(R29=$Bu^t$) |
| H | $CF_3$ | H | O | - | - | 0 | F | Cl | Q2(R29=$Pen^n$) |
| H | $CF_3$ | H | O | - | - | 0 | F | Cl | Q3(R29=Ph) |
| H | $CF_3$ | H | O | - | - | 0 | F | Cl | Q4(R29=$Bu^t$) |
| H | $CF_3$ | H | O | - | - | 0 | F | Cl | Q4(R29=$Pen^n$) |
| H | $CF_3$ | H | O | - | - | 0 | F | Cl | Q4(R29=$CON(Me)_2$) |
| H | $CF_3$ | H | O | - | - | 0 | F | Cl | Q4(R29=$CF_3$) |
| H | $CF_3$ | H | O | - | - | 0 | F | Cl | Q4(R29=$N(Me)_2$) |
| H | $CF_3$ | H | O | H | H | 1 | F | Cl | Q2(R29=$Bu^t$) |

Table 2-4 (Cont'd)

| Z | R1 | R2 | X | $R_a$ | $R_b$ | m | R7 | R8 | R9 |
|---|---|---|---|---|---|---|---|---|---|
| H | $CF_3$ | H | O | – | – | 0 | F | Cl | Q5(R29=$Bu^t$ ) |
| H | $CF_3$ | H | O | – | – | 0 | F | Cl | Q5(R29=Cl) |
| H | $CF_3$ | H | O | – | – | 0 | F | Cl | Q5(R29=CON$(Me)_2$) |
| H | $CF_3$ | H | O | – | – | 0 | F | Cl | Q7(R34=$Bu^t$ ) |
| H | $CF_3$ | H | S | – | – | 0 | F | Cl | Q7(R34=$Bu^t$ ) |
| H | $CF_3$ | H | O | – | – | 0 | F | Cl | Q8(R35=R36=H, R37=Me) |
| H | $CF_3$ | H | O | – | – | 0 | F | Cl | Q9(R38=R39=H) |
| H | $CF_3$ | H | O | – | – | 0 | F | Cl | Q9(R38=Cl, R39=H) |
| H | $CF_3$ | H | O | – | – | 0 | F | Cl | Q10(R38=R39=H) |
| H | $CF_3$ | H | O | – | – | 0 | F | Cl | Q12(R41=Me, R42=R43=H) |
| H | $CF_3$ | H | O | – | – | 0 | F | Cl | Q13(R44=$Bu^t$, R45=H) |
| H | $CF_3$ | H | O | – | – | 0 | F | Cl | Q15(R48=$Bu^t$, R49=H) |
| H | $CF_3$ | H | S | – | – | 0 | F | Cl | Q1(R29=$Bu^t$ ) |
| H | $CF_3$ | H | S | – | – | 0 | F | Cl | Q2(R29=$Bu^t$ ) |
| H | $CF_3$ | H | S | – | – | 0 | F | Cl | Q4(R29=$Bu^t$ ) |
| H | $CF_3$ | H | S | – | – | 0 | F | Cl | Q5(R29=$Bu^t$ ) |
| H | $CF_3$ | H | S | – | – | 0 | F | Cl | Q9(R38=R39=H) |
| H | $CF_3$ | H | S | – | – | 0 | F | Cl | Q13(R44=$Bu^t$, R45=H) |
| H | $CF_3$ | H | S | – | – | 0 | F | Cl | Q15(R48=$Bu^t$, R49=H) |
| H | Et | H | S | – | – | 0 | F | Cl | $CO_2Et$ |
| H | $Pr^n$ | H | S | – | – | 0 | F | Cl | $CO_2Et$ |
| H | $Pr^i$ | H | S | – | – | 0 | F | Cl | $CO_2Et$ |

Table 2-4 (Cont'd)

| Z | R1 | R2 | X | $R_a$ | $R_b$ | m | R7 | R8 | R9 |
|---|---|---|---|---|---|---|---|---|---|
| H | Et | H | O | H | H | 1 | F | Cl | $CO_2Et$ |
| H | $CF_3O$ | H | O | − | − | 0 | F | Cl | $CO_2Me$ |
| H | $CF_3O$ | H | O | − | − | 0 | F | Cl | $CO_2Et$ |
| H | $CF_3S$ | H | O | − | − | 0 | F | Cl | $CO_2Me$ |
| H | $CF_3S$ | H | O | − | − | 0 | F | Cl | $CO_2Et$ |
| H | $-CH_2CH_2CH_2-$ | | O | − | − | 0 | F | Cl | $CO_2Et$ |
| H | $CF_3CF_2$ | H | O | H | H | 1 | F | Cl | H |
| H | $CF_3CF_2$ | H | O | − | − | 0 | F | Cl | Q1(R29=Bu$^t$) |
| H | $-CF_2CF_2CF_2-$ | | O | − | − | 0 | F | Cl | $CO_2Et$ |
| H | $HF_2C$ | H | S | − | − | 0 | F | Cl | $CO_2Et$ |
| H | $CF_3O$ | H | S | − | − | 0 | F | Cl | $CO_2Et$ |
| H | $CF_3S$ | H | S | − | − | 0 | F | Cl | $CO_2Et$ |
| H | $CF_3CF_2$ | H | S | − | − | 0 | F | Cl | $CO_2Et$ |
| H | Ph | H | O | − | − | 0 | F | Cl | H |
| H | Ph | H | O | − | − | 0 | F | Cl | $CO_2Me$ |
| H | Ph | H | O | − | − | 0 | F | Cl | $CO_2Et$ |
| H | 2-F-Ph | H | O | − | − | 0 | F | Cl | $CO_2Et$ |
| H | 3-F-Ph | H | O | − | − | 0 | F | Cl | $CO_2Et$ |

Table 2-4 (Cont'd)

| Z | R1 | R2 | X | $R_a$ | $R_b$ | m | R7 | R8 | R9 |
|---|---|---|---|---|---|---|---|---|---|
| H | 4-F-Ph | H | O | – | – | 0 | F | Cl | $CO_2Et$ |
| H | Penta-F-Ph | H | O | – | – | 0 | F | Cl | $CO_2Et$ |
| H | 2-Cl-Ph | H | O | – | – | 0 | F | Cl | $CO_2Et$ |
| H | 3-Cl-Ph | H | O | – | – | 0 | F | Cl | $CO_2Et$ |
| H | 4-Cl-Ph | H | O | – | – | 0 | F | Cl | $CO_2Et$ |
| H | 2,6-F-Ph | H | O | – | – | 0 | F | Cl | $CO_2Et$ |
| H | 2,6-Cl-Ph | H | O | – | – | 0 | F | Cl | $CO_2Et$ |
| H | 2,3-F-Ph | H | O | – | – | 0 | F | Cl | $CO_2Et$ |
| H | 2-F-Ph | H | O | – | – | 0 | F | Cl | H |
| H | 3-F-Ph | H | O | – | – | 0 | F | Cl | H |
| H | 4-F-Ph | H | O | – | – | 0 | F | Cl | H |
| H | 2-Cl-Ph | H | O | – | – | 0 | F | Cl | H |
| H | 3-Cl-Ph | H | O | – | – | 0 | F | Cl | H |
| H | 4-Cl-Ph | H | O | – | – | 0 | F | Cl | H |
| H | 2-$CF_3$-Ph | H | O | – | – | 0 | F | Cl | H |
| H | 3-$CF_3$-Ph | H | O | – | – | 0 | F | Cl | H |

Table 2-4 (Cont' d)

| Z | R1 | R2 | X | $R_a$ | $R_b$ | m | R7 | R8 | R9 |
|---|---|---|---|---|---|---|---|---|---|
| H | 4-$CF_3$-Ph | H | O | – | – | 0 | F | Cl | H |
| H | 2-$CF_3$-Ph | H | O | – | – | 0 | F | Cl | $CO_2Et$ |
| H | 3-$CF_3$-Ph | H | O | – | – | 0 | F | Cl | $CO_2Et$ |
| H | 4-$CF_3$-Ph | H | O | – | – | 0 | F | Cl | $CO_2Et$ |
| H | Bz | H | O | – | – | 0 | F | Cl | H |
| H | Ph | H | S | – | – | 0 | F | Cl | H |
| H | 2-F-Ph | H | S | – | – | 0 | F | Cl | H |
| H | 3-F-Ph | H | S | – | – | 0 | F | Cl | H |
| H | 4-F-Ph | H | S | – | – | 0 | F | Cl | H |
| H | 2-$CF_3$-Ph | H | S | – | – | 0 | F | Cl | H |
| H | 3-$CF_3$-Ph | H | S | – | – | 0 | F | Cl | H |
| H | 4-$CF_3$-Ph | H | S | – | – | 0 | F | Cl | H |
| H | $CF_3CH_2$ | H | S | – | – | 0 | F | Cl | H |
| H | $CF_3CH_2$ | H | O | H | H | 1 | H | Cl | H |
| H | $CF_3CH_2$ | H | S | – | – | 0 | F | Cl | $OPr^i$ |
| H | $CF_3CH_2$ | H | S | – | – | 0 | F | Cl | OCH(Me)C ≡CH |
| H | $CF_3CH_2$ | H | S | – | – | 0 | F | Cl | $SCH_2CO_2Me$ |
| H | $CF_3CH_2$ | H | S | – | – | 0 | F | Cl | $OCH(Me)CO_2Me$ |
| H | F | F | O | – | – | 0 | F | Cl | H |
| H | F | F | O | – | – | 0 | F | Cl | $CO_2Et$ |
| H | $CF_3CH_2$ | H | O | H | H | 1 | H | $NO_2$ | $CO_2H$ |
| H | $CF_3CH_2$ | H | O | H | H | 1 | H | $NO_2$ | $CO_2Et$ |

Table 2-4 (Cont' d)

| Z | R1 | R2 | X | $R_a$ | $R_b$ | m | R7 | R8 | R9 |
|---|---|---|---|---|---|---|---|---|---|
| Et | Me | H | O | – | – | 0 | F | Cl | $CO_2Et$ |
| Me | Me | H | O | – | – | 0 | F | Cl | $CO_2Et$ |
| Et | Me | H | S | – | – | 0 | F | Cl | $CO_2Et$ |
| Me | Me | H | S | – | – | 0 | F | Cl | $CO_2Et$ |
| Et | $CF_3$ | H | O | – | – | 0 | F | Cl | $CO_2Et$ |
| Me | $CF_3$ | H | O | – | – | 0 | F | Cl | $CO_2Et$ |
| Et | $CF_3$ | H | S | – | – | 0 | F | Cl | $CO_2Et$ |
| Me | $CF_3$ | H | S | – | – | 0 | F | Cl | $CO_2Et$ |
| Me | $CF_3CF_2$ | H | O | – | – | 0 | F | Cl | $CO_2Et$ |
| Me | $CF_3CH_2$ | H | O | – | – | 0 | F | Cl | $CO_2Et$ |
| Me | $-CF_2CF_2CF_2-$ | | O | – | – | 0 | F | Cl | $CO_2Et$ |

Table 2-4-1

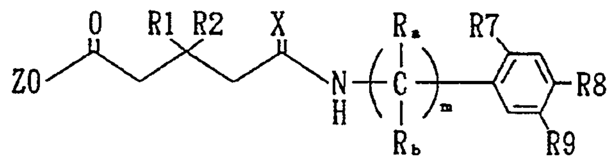

| Z | R1 | R2 | X | $R_a$ | $R_b$ | m | R7 | R8 | R9 |
|---|---|---|---|---|---|---|---|---|---|
| H | $-(CH_2)_4-$ | | O | - | - | 0 | F | Cl | H |
| H | $-(CH_2)_4-$ | | O | - | - | 0 | F | Cl | $CO_2Me$ |
| H | $-(CH_2)_4-$ | | O | - | - | 0 | F | Cl | $CO_2Et$ |
| H | Me | H | O | H | H | 1 | H | $NO_2$ | H |
| H | Me | H | O | - | - | 0 | H | Me | $OCH_2C\equiv CH$ |
| H | $CF_3$ | H | O | H | H | 1 | F | Cl | H |
| H | $CF_3$ | H | O | H | H | 1 | F | Cl | $CO_2Me$ |
| H | $CF_3$ | H | O | H | H | 1 | F | Cl | $CO_2Et$ |
| H | $CF_3$ | H | O | H | H | 1 | H | $NO_2$ | H |
| H | Me | H | O | H | H | 1 | F | Cl | $CO_2Pr^i$ |
| H | Me | H | O | - | - | 0 | F | Cl | Q1(R29=Me) |
| H | Me | H | O | - | - | 0 | F | Cl | Q1(R29=Et) |
| H | Me | H | O | - | - | 0 | F | Cl | Q1(R29=$Pr^n$) |
| H | Me | H | O | - | - | 0 | F | Cl | Q1(R29=$Pr^i$) |
| H | $CF_3$ | H | O | - | - | 0 | F | Cl | Q1(R29=Me) |
| H | $CF_3$ | H | O | - | - | 0 | F | Cl | Q1(R29=Et) |
| H | $CF_3$ | H | O | - | - | 0 | F | Cl | Q1(R29=$Pr^n$) |
| H | $CF_3$ | H | O | - | - | 0 | F | Cl | Q1(R29=$Pr^i$) |
| H | $CF_3$ | H | O | - | - | 0 | F | Cl | Q18(R50=H) |

Table 2-4-1 (Cont'd)

| Z | R1 | R2 | X | $R_a$ | $R_b$ | m | R7 | R8 | R9 |
|---|---|---|---|---|---|---|---|---|---|
| H | $CF_3$ | H | O | - | - | 0 | F | Cl | Q18(R50=Me) |
| H | $CF_3$ | H | O | - | - | 0 | F | F | Q18(R50=Me) |
| H | Me | H | O | - | - | 0 | F | Cl | $NHCOCH_3$ |
| H | Me | H | O | - | - | 0 | F | Cl | $NHSO_2Pr^n$ |
| H | Me | H | O | - | - | 0 | F | Cl | $NHSO_2Pr^i$ |
| H | Me | H | O | - | - | 0 | F | Cl | $NHCH_2CO_2Me$ |
| H | Me | H | O | - | - | 0 | F | Cl | $NHCH_2CO_2Et$ |
| H | Me | H | O | - | - | 0 | F | Cl | $NHCH(Me)CO_2Me$ |
| H | Me | H | O | - | - | 0 | F | Cl | $NHCH(Me)CO_2Et$ |
| H | Me | H | O | - | - | 0 | F | Cl | $NHCO_2Me$ |
| H | Me | H | O | - | - | 0 | F | Cl | $NHCO_2Et$ |
| H | Me | H | O | - | - | 0 | F | Cl | $NHCO_2Bz$ |
| H | Me | H | O | - | - | 0 | F | Cl | $NHCO_2CH_2$(4-Me-Ph) |
| H | Me | H | O | - | - | 0 | F | Cl | $NHCO_2CH_2$(3-Me-Ph) |
| H | Me | H | O | - | - | 0 | F | Cl | $NHCO_2CH_2$(2-Me-Ph) |
| H | Me | H | O | - | - | 0 | F | Cl | $NHCO_2CH_2$(4-Cl-Ph) |
| H | $CF_3$ | H | O | - | - | 0 | F | Cl | $NHCOCH_3$ |
| H | $CF_3$ | H | O | - | - | 0 | F | Cl | $NHSO_2Pr^n$ |
| H | $CF_3$ | H | O | - | - | 0 | F | Cl | $NHSO_2Pr^i$ |
| H | $CF_3$ | H | O | - | - | 0 | F | Cl | $NHCH_2CO_2Me$ |
| H | $CF_3$ | H | O | - | - | 0 | F | Cl | $NHCH_2CO_2Et$ |
| H | $CF_3$ | H | O | - | - | 0 | F | Cl | $NHCH(Me)CO_2Me$ |
| H | $CF_3$ | H | O | - | - | 0 | F | Cl | $NHCH(Me)CO_2Et$ |
| H | $CF_3$ | H | O | - | - | 0 | F | Cl | $NHCO_2Me$ |

Table 2-4-1 (Cont'd)

| Z | R1 | R2 | X | $R_a$ | $R_b$ | m | R7 | R8 | R9 |
|---|---|---|---|---|---|---|---|---|---|
| H | $CF_3$ | H | O | - | - | 0 | F | Cl | $NHCO_2Et$ |
| H | $CF_3$ | H | O | - | - | 0 | F | Cl | $NHCO_2Bz$ |
| H | $CF_3$ | H | O | - | - | 0 | F | Cl | $NHCO_2CH_2$(4-Me-Ph) |
| H | $CF_3$ | H | O | - | - | 0 | F | Cl | $NHCO_2CH_2$(3-Me-Ph) |
| H | $CF_3$ | H | O | - | - | 0 | F | Cl | $NHCO_2CH_2$(2-Me-Ph) |
| H | $CF_3$ | H | O | - | - | 0 | F | Cl | $NHCO_2CH_2$(4-Cl-Ph) |
| H | Ph | H | O | - | - | 0 | F | Cl | $NHCOCH_3$ |
| H | Ph | H | O | - | - | 0 | F | Cl | $NHSO_2Pr^n$ |
| H | Ph | H | O | - | - | 0 | F | Cl | $NHSO_2Pr^i$ |
| H | Ph | H | O | - | - | 0 | F | Cl | $NHCH_2CO_2Me$ |
| H | Ph | H | O | - | - | 0 | F | Cl | $NHCH_2CO_2Et$ |
| H | Ph | H | O | - | - | 0 | F | Cl | NHCH(Me)$CO_2Me$ |
| H | Ph | H | O | - | - | 0 | F | Cl | NHCH(Me)$CO_2Et$ |
| H | Ph | H | O | - | - | 0 | F | Cl | $NHCO_2Me$ |
| H | Ph | H | O | - | - | 0 | F | Cl | $NHCO_2Et$ |
| H | Ph | H | O | - | - | 0 | F | Cl | $NHCO_2Bz$ |
| H | Ph | H | O | - | - | 0 | F | Cl | $NHCO_2CH_2$(4-Me-Ph) |
| H | Ph | H | O | - | - | 0 | F | Cl | $NHCO_2CH_2$(3-Me-Ph) |
| H | Ph | H | O | - | - | 0 | F | Cl | $NHCO_2CH_2$(2-Me-Ph) |
| H | Ph | H | O | - | - | 0 | F | Cl | $NHCO_2CH_2$(4-Cl-Ph) |
| H | Ph | H | O | - | - | 0 | F | Cl | $NO_2$ |
| H | Ph | H | O | - | - | 0 | F | Cl | $NH_2$ |
| H | Ph | H | O | - | - | 0 | F | Cl | $NHSO_2Me$ |
| H | Ph | H | O | - | - | 0 | F | Cl | $NHSO_2Et$ |

Table 2-4-1 (Cont'd)

| Z | R1 | R2 | X | $R_a$ | $R_b$ | m | R7 | R8 | R9 |
|---|---|---|---|---|---|---|---|---|---|
| H | Ph | H | O | - | - | 0 | F | Cl | $N(SO_2Me)_2$ |
| H | $Pr^n$ | H | O | - | - | 0 | F | Cl | $NH_2$ |
| H | $Pr^n$ | H | O | - | - | 0 | F | Cl | $NHSO_2Me$ |
| H | $Pr^n$ | H | O | - | - | 0 | F | Cl | $NHSO_2Et$ |
| H | $Pr^n$ | H | O | - | - | 0 | F | Cl | $N(SO_2Me)_2$ |
| H | $Pr^i$ | H | O | - | - | 0 | F | Cl | $NH_2$ |
| H | $Pr^i$ | H | O | - | - | 0 | F | Cl | $NHSO_2Me$ |
| H | $Pr^i$ | H | O | - | - | 0 | F | Cl | $NHSO_2Et$ |
| H | $Pr^i$ | H | O | - | - | 0 | F | Cl | $N(SO_2Me)_2$ |
| H | Et | H | O | - | - | 0 | F | Cl | $NH_2$ |
| H | Et | H | O | - | - | 0 | F | Cl | $NHSO_2Me$ |
| H | Et | H | O | - | - | 0 | F | Cl | $NHSO_2Et$ |
| H | Et | H | O | - | - | 0 | F | Cl | $N(SO_2Me)_2$ |
| H | Et | H | O | - | - | 0 | F | Cl | $NHCOCH_3$ |
| H | Et | H | O | - | - | 0 | F | Cl | $NHSO_2Pr^n$ |
| H | Et | H | O | - | - | 0 | F | Cl | $NHSO_2Pr^i$ |
| H | Et | H | O | - | - | 0 | F | Cl | $NHCH_2CO_2Me$ |
| H | Et | H | O | - | - | 0 | F | Cl | $NHCH_2CO_2Et$ |
| H | Et | H | O | - | - | 0 | F | Cl | $NHCH(Me)CO_2Me$ |
| H | Et | H | O | - | - | 0 | F | Cl | $NHCH(Me)CO_2Et$ |
| H | Et | H | O | - | - | 0 | F | Cl | $NHCO_2Me$ |
| H | Et | H | O | - | - | 0 | F | Cl | $NHCO_2Et$ |
| H | Et | H | O | - | - | 0 | F | Cl | $NHCO_2Bz$ |
| H | Et | H | O | - | - | 0 | F | Cl | $NHCO_2CH_2$(4-Me-Ph) |

Table 2-4-1 (Cont'd)

| Z | R1 | R2 | X | $R_a$ | $R_b$ | m | R7 | R8 | R9 |
|---|---|---|---|---|---|---|---|---|---|
| H | Et | H | O | - | - | 0 | F | Cl | $NHCO_2CH_2$(3-Me-Ph) |
| H | Et | H | O | - | - | 0 | F | Cl | $NHCO_2CH_2$(2-Me-Ph) |
| H | Et | H | O | - | - | 0 | F | Cl | $NHCO_2CH_2$(4-Cl-Ph) |
| H | $Pr^n$ | H | O | - | - | 0 | F | Cl | $NHCOCH_3$ |
| H | $Pr^n$ | H | O | - | - | 0 | F | Cl | $NHSO_2Pr^n$ |
| H | $Pr^n$ | H | O | - | - | 0 | F | Cl | $NHSO_2Pr^i$ |
| H | $Pr^n$ | H | O | - | - | 0 | F | Cl | $NHCH_2CO_2Me$ |
| H | $Pr^n$ | H | O | - | - | 0 | F | Cl | $NHCH_2CO_2Et$ |
| H | $Pr^n$ | H | O | - | - | 0 | F | Cl | NHCH(Me)$CO_2Me$ |
| H | $Pr^n$ | H | O | - | - | 0 | F | Cl | NHCH(Me)$CO_2Et$ |
| H | $Pr^n$ | H | O | - | - | 0 | F | Cl | $NHCO_2Me$ |
| H | $Pr^n$ | H | O | - | - | 0 | F | Cl | $NHCO_2Et$ |
| H | $Pr^n$ | H | O | - | - | 0 | F | Cl | $NHCO_2Bz$ |
| H | $Pr^n$ | H | O | - | - | 0 | F | Cl | $NHCO_2CH_2$(4-Me-Ph) |
| H | $Pr^n$ | H | O | - | - | 0 | F | Cl | $NHCO_2CH_2$(3-Me-Ph) |
| H | $Pr^n$ | H | O | - | - | 0 | F | Cl | $NHCO_2CH_2$(2-Me-Ph) |
| H | $Pr^n$ | H | O | - | - | 0 | F | Cl | $NHCO_2CH_2$(4-Cl-Ph) |
| H | $Pr^i$ | H | O | - | - | 0 | F | Cl | $NHCOCH_3$ |
| H | $Pr^i$ | H | O | - | - | 0 | F | Cl | $NHSO_2Pr^n$ |
| H | $Pr^i$ | H | O | - | - | 0 | F | Cl | $NHSO_2Pr^i$ |
| H | $Pr^i$ | H | O | - | - | 0 | F | Cl | $NHCH_2CO_2Me$ |
| H | $Pr^i$ | H | O | - | - | 0 | F | Cl | $NHCH_2CO_2Et$ |
| H | $Pr^i$ | H | O | - | - | 0 | F | Cl | NHCH(Me)$CO_2Me$ |
| H | $Pr^i$ | H | O | - | - | 0 | F | Cl | NHCH(Me)$CO_2Et$ |

Table 2-4-1 (Cont'd)

| Z | R1 | R2 | X | $R_a$ | $R_b$ | m | R7 | R8 | R9 |
|---|---|---|---|---|---|---|---|---|---|
| H | $Pr^i$ | H | O | - | - | 0 | F | Cl | $NHCO_2Me$ |
| H | $Pr^i$ | H | O | - | - | 0 | F | Cl | $NHCO_2Et$ |
| H | $Pr^i$ | H | O | - | - | 0 | F | Cl | $NHCO_2Bz$ |
| H | $Pr^i$ | H | O | - | - | 0 | F | Cl | $NHCO_2CH_2$(4-Me-Ph) |
| H | $Pr^i$ | H | O | - | - | 0 | F | Cl | $NHCO_2CH_2$(3-Me-Ph) |
| H | $Pr^i$ | H | O | - | - | 0 | F | Cl | $NHCO_2CH_2$(2-Me-Ph) |
| H | $Pr^i$ | H | O | - | - | 0 | F | Cl | $NHCO_2CH_2$(4-Cl-Ph) |

Table 2-5

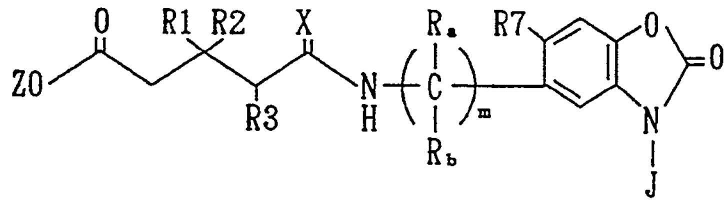

| Z | R1 | R2 | R3 | X | Ra | Rb | m | R7 | J |
|---|---|---|---|---|---|---|---|---|---|
| H | $CF_3$ | H | H | O | – | – | 0 | F | Et |
| H | $CF_3$ | H | H | O | – | – | 0 | F | $CH_2C\equiv CH$ |
| H | $CF_3$ | H | H | O | – | – | 0 | F | Bz |
| H | $CF_3$ | H | H | O | – | – | 0 | H | $CH_2C\equiv CH$ |
| H | $CF_3$ | H | H | S | – | – | 0 | F | $CH_2C\equiv CH$ |
| H | $CF_3$ | H | Me | O | – | – | 0 | F | $CH_2C\equiv CH$ |
| H | $CH_3$ | H | H | O | – | – | 0 | F | $CH_2C\equiv CH$ |
| H | $CH_3$ | H | H | S | – | – | 0 | F | $CH_2C\equiv CH$ |

Table 2-5-1

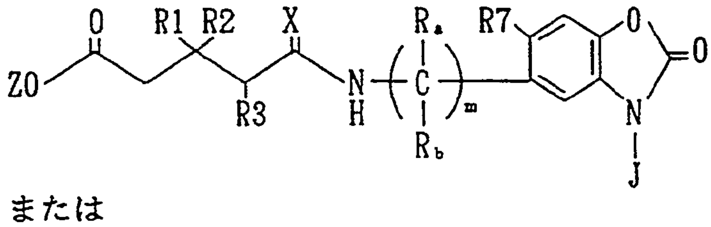

または

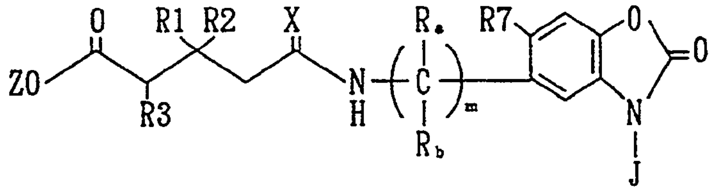

| Z | R1 | R2 | R3 | X | Ra | Rb | m | R7 | J |
|---|---|---|---|---|---|---|---|---|---|
| H | Me | H | Me | O | - | - | 0 | F | $CH_2C\equiv CH$ |
| H | Et | H | Me | O | - | - | 0 | F | $CH_2C\equiv CH$ |
| H | $Pr^n$ | H | Me | O | - | - | 0 | F | $CH_2C\equiv CH$ |
| H | $Pr^i$ | H | Me | O | - | - | 0 | F | $CH_2C\equiv CH$ |
| H | H | $-CH_2CH_2CH_2-$ | | O | - | - | 0 | F | $CH_2C\equiv CH$ |

Table 2-6

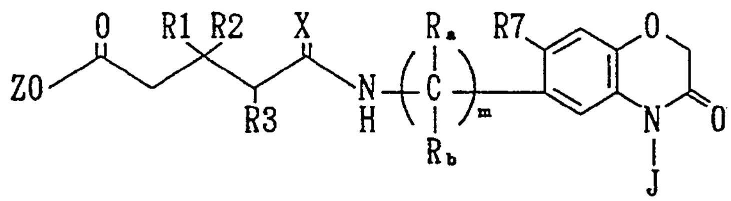

| Z | R1 | R2 | R3 | X | $R_a$ | $R_b$ | m | R7 | J |
|---|---|---|---|---|---|---|---|---|---|
| H | $CH_3$ | H | H | O | – | – | 0 | F | $CH_2C\equiv CH$ |
| H | $CH_3$ | H | H | S | – | – | 0 | F | $CH_2C\equiv CH$ |
| H | $CH_3$ | H | H | O | – | – | 0 | F | Et |
| H | $CH_3$ | H | H | S | – | – | 0 | F | Et |
| H | $CH_3$ | H | H | O | – | – | 0 | H | $CH_2C\equiv CH$ |
| H | Et | H | H | O | – | – | 0 | F | $CH_2C\equiv CH$ |
| H | $Pr^n$ | H | H | O | – | – | 0 | F | $CH_2C\equiv CH$ |
| H | $Pr^i$ | H | H | O | – | – | 0 | F | $CH_2C\equiv CH$ |
| H | $-CH_2CH_2CH_2-$ | | H | O | – | – | 0 | F | $CH_2C\equiv CH$ |
| H | $Bu^t$ | H | H | O | – | – | 0 | F | $CH_2C\equiv CH$ |
| H | $CH_3$ | H | H | O | – | – | 0 | F | $CH_2CH\equiv CH_2$ |
| H | $CH_3$ | H | H | O | – | – | 0 | F | Q16 |

Table 2-6 (Cont'd)

| Z | R1 | R2 | R3 | X | $R_a$ | $R_b$ | m | R7 | J |
|---|---|---|---|---|---|---|---|---|---|
| H | $CF_3$ | H | H | O | – | – | 0 | F | $CH_2C{\equiv}CH$ |
| H | $CF_3$ | H | H | S | – | – | 0 | F | $CH_2C{\equiv}CH$ |
| H | $CF_3$ | H | H | O | – | – | 0 | F | $CH_2CH{=}CH_2$ |
| H | $CF_3$ | H | H | S | – | – | 0 | F | $CH_2CH{=}CH_2$ |
| H | $CF_3$ | H | H | O | – | – | 0 | F | Q16 |
| H | $CF_3$ | H | H | O | – | – | 0 | F | Q17 |
| H | $CF_3$ | H | H | S | – | – | 0 | F | Q16 |
| H | $CF_3$ | H | H | S | – | – | 0 | F | Q17 |
| H | $CF_3$ | H | H | O | – | – | 0 | F | Et |
| H | $CF_3$ | H | H | S | – | – | 0 | F | Et |
| H | $CF_3$ | H | H | O | – | – | 0 | F | $CH_2OH$ |
| H | $CF_3$ | H | H | O | – | – | 0 | F | $CH_2Cl$ |
| H | $CF_3$ | H | H | O | H | H | 1 | F | $CH_2C{\equiv}CH$ |
| H | $CF_3$ | H | H | O | – | – | 0 | F | Bz |
| H | $CF_3$ | H | H | S | – | – | 0 | F | Bz |
| H | $CF_3CF_2$ | H | H | O | – | – | 0 | F | $CH_2C{\equiv}CH$ |
| H | $CF_3CF_2$ | H | H | O | – | – | 0 | F | $CH_2CH{=}CH_2$ |
| H | $CF_3CF_2$ | H | H | O | – | – | 0 | F | $CH_2CN$ |
| H | $CF_3CF_2$ | H | H | O | – | – | 0 | F | Bz |
| H | $CF_3CF_2$ | H | H | O | – | – | 0 | F | Q16 |
| H | $CF_3CF_2$ | H | H | S | – | – | 0 | F | $CH_2C{\equiv}CH$ |
| H | $CF_3CH_2$ | H | H | O | – | – | 0 | F | $CH_2C{\equiv}CH$ |
| H | $CF_3CH_2$ | H | H | S | – | – | 0 | F | $CH_2C{\equiv}CH$ |
| H | $CF_3CH_2$ | H | H | O | – | – | 0 | H | $CH_2C{\equiv}CH$ |

Table 2-6 (Cont' d)

| Z | R1 | R2 | R3 | X | $R_a$ | $R_b$ | m | R7 | J |
|---|---|---|---|---|---|---|---|---|---|
| H | $CF_3CH_2$ | H | H | S | – | – | 0 | H | $CH_2C{\equiv}CH$ |
| H | $CF_3CF_2$ | H | H | O | – | – | 0 | H | $CH_2C{\equiv}CH$ |
| H | $CF_3CF_2$ | H | H | S | – | – | 0 | H | $CH_2C{\equiv}CH$ |
| H | $-CF_2CF_2CF_2-$ | | H | O | – | – | 0 | F | $CH_2C{\equiv}CH$ |
| H | $CF_3$ | H | Me | O | – | – | 0 | F | $CH_2C{\equiv}CH$ |
| H | $CF_3CF_2$ | H | Me | O | – | – | 0 | F | $CH_2C{\equiv}CH$ |
| H | $CF_3CF_2$ | H | Me | O | – | – | 0 | F | $CH_2C{\equiv}CH$ |
| H | $CF_3$ | H | H | O | – | – | 0 | F | H |
| H | $CF_3CF_2$ | H | H | O | – | – | 0 | F | H |
| H | $CF_3CH_2$ | H | H | O | – | – | 0 | F | H |
| H | $CH_3$ | H | H | O | – | – | 0 | F | H |
| H | $CF_3$ | H | H | O | – | – | 0 | H | H |

Table 2-6-1

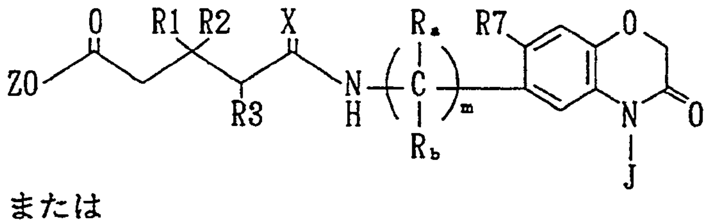

または

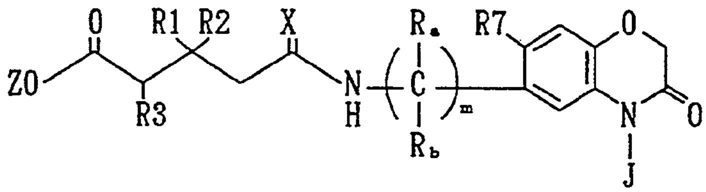

| Z | R1 | R2 | R3 | X | $R_a$ | $R_b$ | m | R7 | J |
|---|---|---|---|---|---|---|---|---|---|
| H | Me | H | Me | O | - | - | 0 | F | $CH_2C\equiv CH$ |
| H | $CF_3$ | H | Me | O | - | - | 0 | H | $CH_2C\equiv CH$ |
| H | $CF_3$ | H | H | O | - | - | 0 | H | H |
| H | $CF_3$ | H | H | O | - | - | 0 | H | $CH_2C\equiv CH$ |
| H | $CF_3$ | H | H | O | - | - | 0 | F | $CH_2$-Q19 |
| H | Et | H | Me | O | - | - | 0 | F | $CH_2C\equiv CH$ |
| H | Et | H | Me | O | - | - | 0 | F | H |
| H | $Pr^n$ | H | Me | O | - | - | 0 | F | H |
| H | $Pr^n$ | H | Me | O | - | - | 0 | F | $CH_2C\equiv CH$ |
| H | $Pr^i$ | H | Me | O | - | - | 0 | F | H |
| H | $Pr^i$ | H | Me | O | - | - | 0 | F | $CH_2C\equiv CH$ |
| H | $Pr^i$ | H | Me | O | - | - | 0 | F | Et |
| H | $Pr^n$ | H | Me | O | - | - | 0 | F | Et |
| H | Ph | H | H | O | - | - | 0 | F | $CH_2C\equiv CH$ |
| H | Ph | H | Me | O | - | - | 0 | F | $CH_2C\equiv CH$ |
| H | Me | H | $CF_3$ | O | - | - | 0 | F | $CH_2C\equiv CH$ |

Table 2-6-1 (Cont'd)

| Z | R1 | R2 | R3 | X | $R_a$ | $R_b$ | m | R7 | J |
|---|---|---|---|---|---|---|---|---|---|
| H | H | $-CH_2CH_2CH_2-$ | | O | - | - | 0 | F | $CH_2C\equiv CH$ |
| H | Ph | H | H | O | - | - | 0 | F | H |
| H | Ph | H | Me | O | - | - | 0 | F | H |
| H | H | $-CH_2CH_2CH_2-$ | | O | - | - | 0 | F | H |

Table 2-7

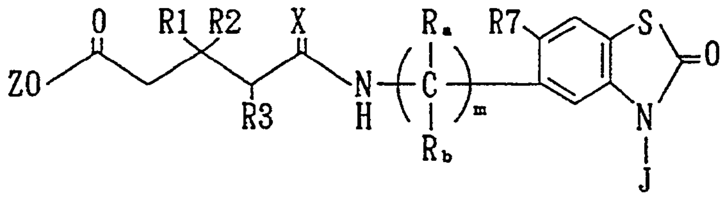

| Z | R1 | R2 | R3 | X | $R_a$ | $R_b$ | m | R7 | J |
|---|---|---|---|---|---|---|---|---|---|
| H | $CH_3$ | H | H | O | – | – | 0 | F | $CH_2C\equiv CH$ |
| H | $CH_3$ | H | H | S | – | – | 0 | F | $CH_2C\equiv CH$ |
| H | $CH_3$ | H | H | O | – | – | 0 | F | Et |
| H | $CH_3$ | H | H | S | – | – | 0 | F | Et |
| H | $CH_3$ | H | H | O | – | – | 0 | H | $CH_2C\equiv CH$ |
| H | Et | H | H | O | – | – | 0 | F | $CH_2C\equiv CH$ |
| H | $Pr^n$ | H | H | O | – | – | 0 | F | $CH_2C\equiv CH$ |
| H | $Pr^i$ | H | H | O | – | – | 0 | F | $CH_2C\equiv CH$ |
| H | $-CH_2CH_2CH_2-$ | | H | O | – | – | 0 | F | $CH_2C\equiv CH$ |
| H | $Bu^t$ | H | H | O | – | – | 0 | F | $CH_2C\equiv CH$ |
| H | $CH_3$ | H | H | O | – | – | 0 | F | $CH_2CH=CH_2$ |
| H | $CH_3$ | H | H | O | – | – | 0 | F | Q16 |
| H | $CF_3$ | H | H | O | – | – | 0 | F | $CH_2C\equiv CH$ |

Table 2-7 (Cont'd)

| Z | R1 | R2 | R3 | X | $R_a$ | $R_b$ | m | R7 | J |
|---|---|---|---|---|---|---|---|---|---|
| H | $CF_3$ | H | H | S | – | – | 0 | F | $CH_2C\equiv CH$ |
| H | $CF_3$ | H | H | O | – | – | 0 | F | $CH_2CH=CH_2$ |
| H | $CF_3$ | H | H | S | – | – | 0 | F | $CH_2CH=CH_2$ |
| H | $CF_3$ | H | H | O | – | – | 0 | F | Q16 |
| H | $CF_3$ | H | H | O | – | – | 0 | F | Q17 |
| H | $CF_3$ | H | H | S | – | – | 0 | F | Q16 |
| H | $CF_3$ | H | H | S | – | – | 0 | F | Q17 |
| H | $CF_3$ | H | H | O | – | – | 0 | F | Et |
| H | $CF_3$ | H | H | S | – | – | 0 | F | Et |
| H | $CF_3$ | H | H | O | – | – | 0 | F | $CH_2OH$ |
| H | $CF_3$ | H | H | O | – | – | 0 | F | $CH_2Cl$ |
| H | $CF_3$ | H | H | O | H | H | 1 | F | $CH_2C\equiv CH$ |
| H | $CF_3$ | H | H | O | – | – | 0 | F | Bz |
| H | $CF_3$ | H | H | S | – | – | 0 | F | Bz |
| H | $CF_3CF_2$ | H | H | O | – | – | 0 | F | $CH_2C\equiv CH$ |
| H | $CF_3CF_2$ | H | H | O | – | – | 0 | F | $CH_2CH=CH_2$ |
| H | $CF_3CF_2$ | H | H | O | – | – | 0 | F | $CH_2CN$ |
| H | $CF_3CF_2$ | H | H | O | – | – | 0 | F | Bz |
| H | $CF_3CF_2$ | H | H | O | – | – | 0 | F | Q16 |
| H | $CF_3CF_2$ | H | H | S | – | – | 0 | F | $CH_2C\equiv CH$ |
| H | $CF_3CH_2$ | H | H | O | – | – | 0 | F | $CH_2C\equiv CH$ |
| H | $CF_3CH_2$ | H | H | S | – | – | 0 | F | $CH_2C\equiv CH$ |
| H | $CF_3CH_2$ | H | H | O | – | – | 0 | H | $CH_2C\equiv CH$ |
| H | $CF_3CH_2$ | H | H | S | – | – | 0 | H | $CH_2C\equiv CH$ |

Table 2-7 (Cont'd)

| Z | R1 | R2 | R3 | X | $R_a$ | $R_b$ | m | R7 | J |
|---|---|---|---|---|---|---|---|---|---|
| H | $CF_3CF_2$ | H | H | O | – | – | 0 | H | $CH_2C{\equiv}CH$ |
| H | $CF_3CF_2$ | H | H | S | – | – | 0 | H | $CH_2C{\equiv}CH$ |
| H | $-CF_2CF_2CF_2-$ | | H | O | – | – | 0 | F | $CH_2C{\equiv}CH$ |
| H | $CF_3$ | H | Me | O | – | – | 0 | F | $CH_2C{\equiv}CH$ |
| H | $CF_3CF_2$ | H | Me | O | – | – | 0 | F | $CH_2C{\equiv}CH$ |
| H | $CF_3CF_2$ | H | Me | O | – | – | 0 | F | $CH_2C{\equiv}CH$ |
| H | $CF_3$ | H | H | O | – | – | 0 | F | H |
| H | $CF_3CF_2$ | H | H | O | – | – | 0 | F | H |
| H | $CF_3CH_2$ | H | H | O | – | – | 0 | F | H |
| H | $CH_3$ | H | H | O | – | – | 0 | F | H |
| H | $CF_3$ | H | H | O | – | – | 0 | H | H |

Table 2-7-1

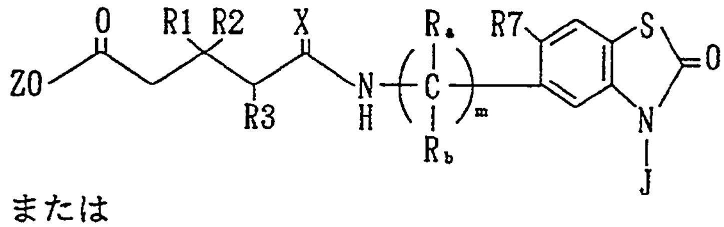

または

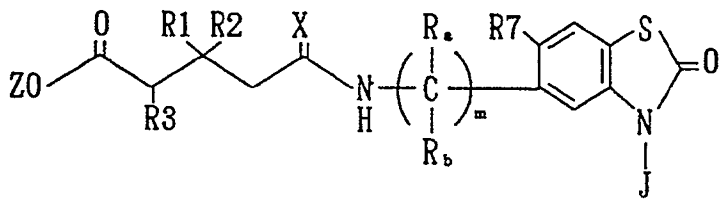

| Z | R1 | R2 | R3 | X | $R_a$ | $R_b$ | m | R7 | J |
|---|---|---|---|---|---|---|---|---|---|
| H | Me | H | Me | O | - | - | 0 | F | $CH_2C \equiv CH$ |
| H | $CF_3$ | H | Me | O | - | - | 0 | H | $CH_2C \equiv CH$ |
| H | $CF_3$ | H | H | O | - | - | 0 | H | H |
| H | $CF_3$ | H | H | O | - | - | 0 | H | $CH_2C \equiv CH$ |
| H | $CF_3$ | H | H | O | - | - | 0 | F | $CH_2$-Q19 |
| H | Et | H | Me | O | - | - | 0 | F | $CH_2C \equiv CH$ |
| H | Et | H | Me | O | - | - | 0 | F | H |
| H | $Pr^n$ | H | Me | O | - | - | 0 | F | H |
| H | $Pr^n$ | H | Me | O | - | - | 0 | F | $CH_2C \equiv CH$ |
| H | $Pr^i$ | H | Me | O | - | - | 0 | F | H |
| H | $Pr^i$ | H | Me | O | - | - | 0 | F | $CH_2C \equiv CH$ |
| H | $Pr^i$ | H | Me | O | - | - | 0 | F | Et |
| H | $Pr^n$ | H | Me | O | - | - | 0 | F | Et |
| H | Ph | H | H | O | - | - | 0 | F | $CH_2C \equiv CH$ |
| H | Ph | H | Me | O | - | - | 0 | F | $CH_2C \equiv CH$ |

Table 2-7-1 (Cont' d)

| Z | R1 | R2 | R3 | X | $R_a$ | $R_b$ | m | R7 | J |
|---|---|---|---|---|---|---|---|---|---|
| H | Me | H | $CF_3$ | O | - | - | 0 | F | $CH_2C\equiv CH$ |
| H | H | $-CH_2CH_2CH_2-$ | | O | - | - | 0 | F | $CH_2C\equiv CH$ |
| H | Ph | H | H | O | - | - | 0 | F | H |
| H | Ph | H | Me | O | - | - | 0 | F | H |
| H | H | $-CH_2CH_2CH_2-$ | | O | - | - | 0 | F | H |

Table 2-8

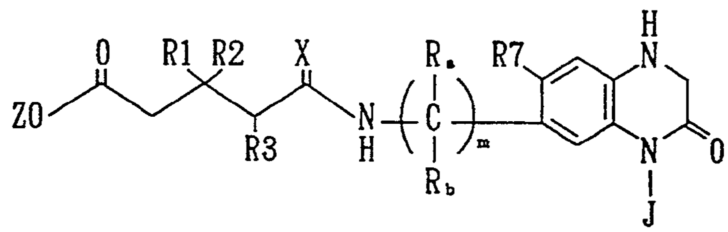

| Z | R1 | R2 | R3 | X | $R_a$ | $R_b$ | m | R7 | J |
|---|---|---|---|---|---|---|---|---|---|
| H | $CF_3$ | H | H | O | – | – | 0 | F | Et |
| H | $CF_3$ | H | H | O | – | – | 0 | F | $CH_2C\equiv CH$ |
| H | $CF_3$ | H | H | O | – | – | 0 | F | Bz |
| H | $CF_3$ | H | H | O | – | – | 0 | H | $CH_2C\equiv CH$ |
| H | $CF_3$ | H | H | S | – | – | 0 | F | $CH_2C\equiv CH$ |
| H | $CF_3$ | H | Me | O | – | – | 0 | F | $CH_2C\equiv CH$ |
| H | $CH_3$ | H | H | O | – | – | 0 | F | $CH_2C\equiv CH$ |
| H | $CH_3$ | H | H | S | – | – | 0 | F | $CH_2C\equiv CH$ |

Table 2-8-1

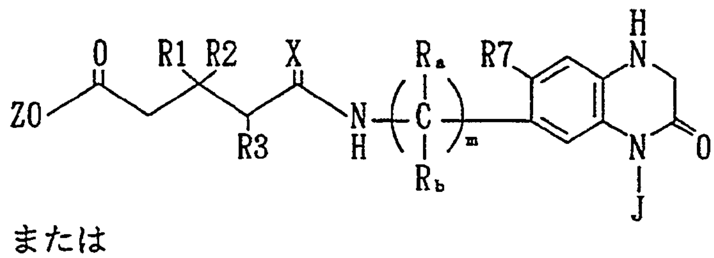

または

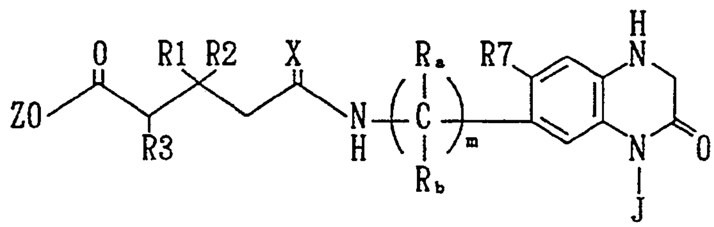

| Z | R1 | R2 | R3 | X | $R_a$ | $R_b$ | m | R7 | J |
|---|---|---|---|---|---|---|---|---|---|
| H | Me | H | Me | O | - | - | 0 | F | $CH_2C\equiv CH$ |
| H | Et | H | Me | O | - | - | 0 | F | $CH_2C\equiv CH$ |
| H | $Pr^n$ | H | Me | O | - | - | 0 | F | $CH_2C\equiv CH$ |
| H | $Pr^i$ | H | Me | O | - | - | 0 | F | $CH_2C\equiv CH$ |
| H | H | $-CH_2CH_2CH_2-$ | | O | - | - | 0 | F | $CH_2C\equiv CH$ |
| H | $Pr^n$ | H | Me | O | - | - | 0 | F | H |
| H | $Pr^i$ | H | Me | O | - | - | 0 | F | H |
| H | Et | H | Me | O | - | - | 0 | F | H |
| H | Me | H | Me | O | - | - | 0 | F | H |
| H | $CF_3$ | H | Me | O | - | - | 0 | F | H |

Table 2-9

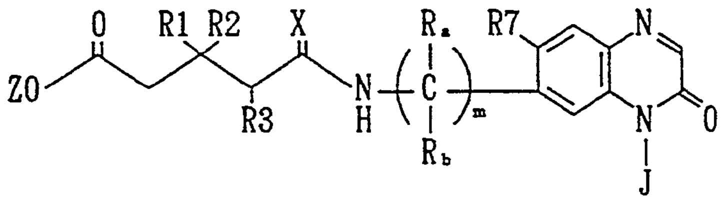

| Z | R1 | R2 | R3 | X | $R_a$ | $R_b$ | m | R7 | J |
|---|---|---|---|---|---|---|---|---|---|
| H | $CF_3$ | H | H | O | – | – | 0 | F | Et |
| H | $CF_3$ | H | H | O | – | – | 0 | F | $CH_2C\equiv CH$ |
| H | $CF_3$ | H | H | O | – | – | 0 | F | Bz |
| H | $CF_3$ | H | H | O | – | – | 0 | H | $CH_2C\equiv CH$ |
| H | $CF_3$ | H | H | S | – | – | 0 | F | $CH_2C\equiv CH$ |
| H | $CF_3$ | H | Me | O | – | – | 0 | F | $CH_2C\equiv CH$ |
| H | $CH_3$ | H | H | O | – | – | 0 | F | $CH_2C\equiv CH$ |
| H | $CH_3$ | H | H | S | – | – | 0 | F | $CH_2C\equiv CH$ |

# Table 2－9－1

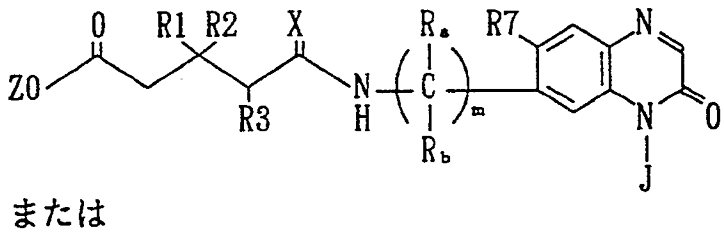

または

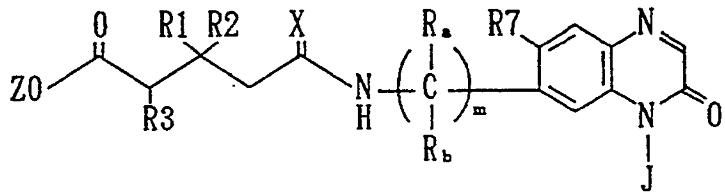

| Z | R1 | R2 | R3 | X | $R_a$ | $R_b$ | m | R7 | J |
|---|---|---|---|---|---|---|---|---|---|
| H | Me | H | Me | O | - | - | 0 | F | $CH_2C\equiv CH$ |
| H | Et | H | Me | O | - | - | 0 | F | $CH_2C\equiv CH$ |
| H | $Pr^n$ | H | Me | O | - | - | 0 | F | $CH_2C\equiv CH$ |
| H | $Pr^i$ | H | Me | O | - | - | 0 | F | $CH_2C\equiv CH$ |
| H | H | $-CH_2CH_2CH_2-$ | | O | - | - | 0 | F | $CH_2C\equiv CH$ |
| H | $Pr^n$ | H | Me | O | - | - | 0 | F | H |
| H | $Pr^i$ | H | Me | O | - | - | 0 | F | H |
| H | Et | H | Me | O | - | - | 0 | F | H |
| H | Me | H | Me | O | - | - | 0 | F | H |
| H | $CF_3$ | H | Me | O | - | - | 0 | F | H |

Symbols are groups represented by the following chemical structures.

Me ; $CH_3$

Et ; $CH_2CH_3$

Ph ; 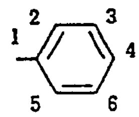

$Pr^i$ ; $CH(CH_3)_2$

$Pr^n$ ; $CH_2CH_2CH_3$

$Pen^c$ ; 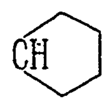

$Bu^n$ ; $CH_2CH_2CH_2CH_3$

$Bu^t$ ; $C(CH_3)_3$

Bz ; $CH_2C_6H_5$

$Pen^n$ ; $CH_2C(CH_3)_3$

Penta-F-Ph; 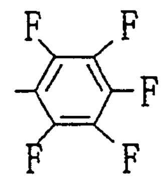

2-F-Ph ; 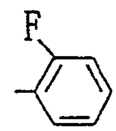

Q 1 ; 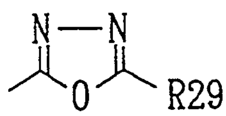

Q 2 ; 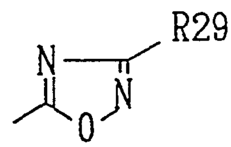

Q 3 ; 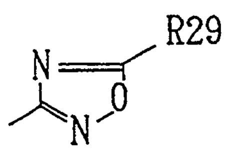

Q 4 ; 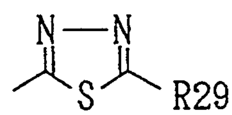

Q 5 ; 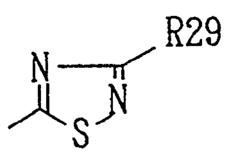

Q 6 ; 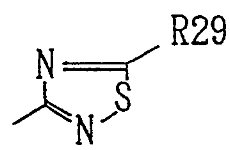

Q 7 ; 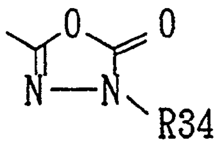

Q 8 ; 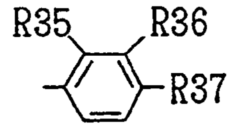

Q 9 ; 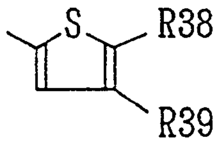

Q10 ; 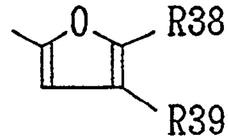

Q11 ; 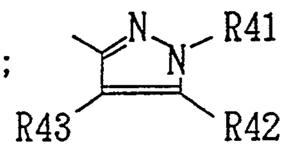

Q12 ; 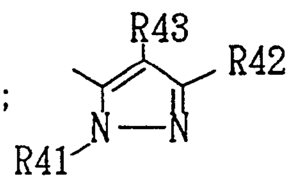

Q13 ; 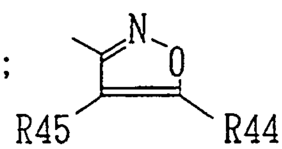

Q14 ; 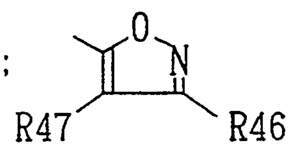

Q15 ; 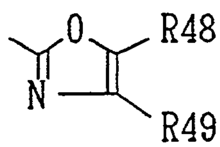

Q16 ; 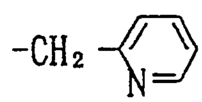

Q17 ; 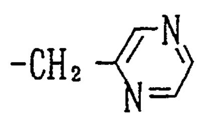

Q18 ; 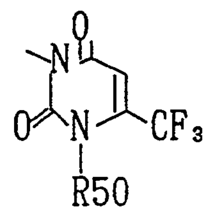

Q19 ; 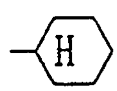

Shown below are the examples of formulations using the compounds of the present invention. It should be understood, however, that the formulations coming within the concept of the present invention are not limited to those shown below. In the following descriptions of Formulation Examples, all "parts" are by weight unless otherwise noted.

Formulation example 1 (Wettable Powder)

Compound No. 1-2 of this invention ----- 50 parts

Zieklite PFP -------------------------- 43 parts

(Kaolin type clay: Zieklite Kogyo K.K., trade name)

Sorpole 5050 -------------------------- 2 parts

(Anionic surfactant: produced by Toho Chemical Industry K.K., trade name)

Lunox 1000C ---------------------------- 3 parts

(Anionic surfactant: produced by Toho Chemical Industry K.K., trade name)

Carplex #80 (Antisolidifying agent) ---- 2 parts

(White carbon: produced by Shionogi & Co., Ltd., trade name)

The above components were uniformly mixed and crushed to prepare a wettable powder.

Formulation example 2 (Wettable Powder)

Compound No. 1-3 of this invention ----- 50 parts

Zieklite PFP -------------------------- 43 parts

(Kaolin type clay: produced by Zieklite Kogyo K.K., trade name)

Sorpole 5050 -------------------------- 2 parts

(Anionic surfactant: produced by Toho Chemical Industry K.K., trade name)

Lunox 1000C ---------------------------- 3 parts

(Anionic surfactant: produced by Toho Chemical Industry K.K., trade name)

Carplex #80 (Antisolidifying agent) ---- 2 parts

(White carbon: produced by Shionogi & Co., Ltd., trade name)

The above components were uniformly mixed and crushed to prepare a wettable powder.

Formulation example 3 (Wettable Powder)

Compound No. 1-23 of this invention ---- 50 parts

Zieklite PFP --------------------------- 43 parts

(Kaolin type clay: produced by Zieklite Kogyo K.K., trade name)

Sorpole 5050 --------------------------- 2 parts

(Anionic surfactant: produced by Toho Chemical Industry K.K., trade name)

Lunox 1000C ---------------------------- 3 parts

(Anionic surfactant: produced by Toho Chemical Industry K.K., trade name)

Carplex #80 (Antisolidifying agent) ---- 2 parts

(White carbon: produced by Shionogi & Co., Ltd., trade name)

The above components were uniformly mixed and crushed to prepare a wettable powder.

Formulation example 4 (Wettable Powder)

Compound No. 1-26 of this invention ---- 50 parts

Zieklite PFP -------------------------- 43 parts

(Kaolin type clay: produced by Zieklite Kogyo K.K., trade name)

Sorpole 5050 -------------------------- 2 parts

(Anionic surfactant: produced by Toho Chemical Industry K.K., trade name)

Lunox 1000C --------------------------- 3 parts

(Anionic surfactant: produced by Toho Chemical Industry K.K., trade name)

Carplex #80 (Antisolidifying agent) ---- 2 parts

(White carbon: produced by Shionogi & Co., Ltd., trade name)

The above components were uniformly mixed and crushed to prepare a wettable powder.

Formulation example 5 (Wettable Powder)

Compound No. 1-35 of this invention ---- 40 parts

Zieklite PFP -------------------------- 53 parts

(Kaolin type clay: produced by Zieklite Kogyo K.K., trade name)

Sorpole 5050 --------------------------- 2 parts
(Anionic surfactant: produced by Toho Chemical Industry K.K., trade name)
Lunox 1000C ---------------------------- 3 parts
(Anionic surfactant: produced by Toho Chemical Industry K.K., trade name)
Carplex #80 (Antisolidifying agent) ---- 2 parts
(White carbon: produced by Shionogi & Co., Ltd., trade name)

The above components were uniformly mixed and crushed to prepare a wettable powder.

Formulation example 6 (Emulsifiable Concentrate)

Compound No. 1-2 of this invention ----- 3 parts
Xylene --------------------------------- 76 parts
Isophorone ----------------------------- 15 parts
Sorpole 3005X -------------------------- 6 parts
(Mixture of nonionic surfactant and anionic surfactant: produced by Toho Chemical Industry K.K., trade name)

The above components were uniformly mixed to prepare an emulsifiable concentrate.

Formulation example 7 (Emulsifiable Concentrate)

Compound No. 1-3 of this invention ----- 3 parts
Xylene --------------------------------- 76 parts
Isophorone ----------------------------- 15 parts

Sorpole 3005X -------------------------- 6 parts
(Mixture of nonionic surfactant and anionic surfactant: produced by Toho Chemical Industry K.K., trade name)

The above components were uniformly mixed to prepare an emulsifiable concentrate.

Formulation example 8 (Emulsifiable Concentrate)

Compound No. 1-23 of this invention ---- 3 parts

Xylene --------------------------------- 76 parts

Isophorone ---------------------------- 15 parts

Sorpole 3005X ------------------------- 6 parts

(Mixture of nonionic surfactant and anionic surfactant: produced by Toho Chemical Industry K.K., trade name)

The above components were uniformly mixed to prepare an emulsifiable concentrate.

Formulation example 9 (Flowable)

Compound No. 1-2 of this invention ----- 35 parts

Agrizol S-711 ------------------------- 8 parts

(Nonionic surfactant: produced by Kao Corp., trade name)

Lunox 1000C ---------------------------- 0.5 part

(Anionic surfactant: produced by Toho Chemical Industry K.K., trade name)

1 % aqueous Rhodopole solution --------- 20 parts

(Thickening agent: produced by Rhone Poulenc Co., trade name)

Ethylene glycol (Antifreezing agent) --- 8 parts

Water ---------------------------------- 28.5 parts

The above components were uniformly mixed to prepare a flowable.

Formulation example 10 (Flowable)

Compound No. 1-3 of this invention ----- 35 parts

Agrizol S-711 -------------------------- 8 parts

(Nonionic surfactant: produced by Kao Corp., trade name)

Lunox 1000C ---------------------------- 0.5 part

(Anionic surfactant: produced by Toho Chemical Industry K.K., trade name)

1 % aqueous Rhodopole solution --------- 20 parts

(Thickening agent: produced by Rhone Poulenc Co., trade name)

Ethylene glycol (Antifreezing agent) --- 8 parts

Water ---------------------------------- 28.5 parts

The above components were uniformly mixed to prepare a flowable.

Formulation example 11 (Flowable)

Compound No. 1-26 of this invention ---- 35 parts

Agrizol S-711 -------------------------- 8 parts

(Nonionic surfactant: produced by Kao Corp., trade name)

Lunox 1000C ---------------------------- 0.5 part

(Anionic surfactant: produced by Toho Chemical Industry K.K., trade name)

1 % aqueous Rhodopole solution --------- 20 parts

(Thickening agent: produced by Rhone Poulenc Co., trade name)

Ethylene glycol (Antifreezing agent) --- 8 parts

Water ---------------------------------- 28.5 parts

The above components were uniformly mixed to prepare a flowable.

Formulation example 12 (Granule)

Compound No. 1-2 of this invention ----- 0.1 part

Bentonite ------------------------------ 55.0 parts

Talc ----------------------------------- 44.9 parts

The above components were uniformly mixed and crushed, mixed and kneaded under stirring by adding a small amount of water, and then granulated by using an extrusion type granulating machine and dried to prepare a granule.

Formulation example 13 (Granule)

Compound No. 1-3 of this invention ----- 0.1 part

Bentonite ------------------------------ 55.0 parts

Talc ----------------------------------- 44.9 parts

The above components were uniformly mixed and crushed, mixed and kneaded under stirring by adding a small amount of water, and then granulated by using an extrusion type granulating machine and dried to prepare a granule.

Formulation example 14 Granular Wettable Powder (Dry flowable)

Compound No. 1-2 of this invention ----- 75 parts

Isoban No. 1 --------------------------- 10 parts

(Anionic sufactant: produced by Kuraray Isopren Chemical Co., Ltd., trade name)

Vanilex N ------------------------------ 5 parts

(Anionic surfactant: produced by Sanyo Kokusaku Pulp K.K., trade name)

Carplex #80 (Antisolidifying agent) ---- 10 parts

(White carbon: produced by Shionogi Seiyaku K.K., trade name)

The above components were uniformly mixed and finely pulverized to prepare a dry flowable.

Formulation example 15 Granular Wettable Power (Dry flowable)

Compound No. 1-3 of this invention ----- 75 parts

Isoban No. 1 --------------------------- 10 parts

(Anionic sufactant: produced by Kuraray Isopren Chemical Co., Ltd., trade name)

Vanilex N ------------------------------ 5 parts

(Anionic surfactant: produced by Sanyo Kokusaku Pulp K.K., trade name)

Carplex #80 (Antisolidifying agent) ---- 10 parts

(White carbon: produced by Shionogi & Co., Ltd., trade name)

The above components were uniformly mixed and finely pulverized to prepare a dry flowable.

Formulation example 16 Granular Wettable Powder (Dry flowable)

Compound No. 1-23 of this invention ---- 75 parts

Isoban No. 1 --------------------------- 10 parts

(Anionic sufactant: produced by Kuraray Isopren Chemical Co., Ltd., trade name)

Vanilex N ------------------------------ 5 parts

(Anionic surfactant: produced by Sanyo-Kokusaku Pulp Co., Ltd., trade name)

Carplex #80 (Antisolidifying agent) ---- 10 parts

(White carbon: produced by Shionogi & Co., Ltd., trade name)

The above components were uniformly mixed and finely pulverized to prepare a dry flowable

Formulation example 17 Granular Wettable Powder (Dry flowable)

Compound No. 1-26 of this invention ---- 75 parts

Isoban No. 1 --------------------------- 10 parts
(Anionic sufactant: produced by Kuraray Isopren Chemical Co., Ltd.., trade name)

Vanilex N ------------------------------ 5 parts
(Anionic surfactant: produced by Sanyo-Kokusaku Pulp Co., Ltd., trade name)

Carplex #80 (Antisolidifying agent) ---- 10 parts
(White carbon: produced by Shionogi & Co., Ltd., trade name)

The above components were uniformly mixed and finely pulverized to prepare a dry flowable.

Formulation example 18 Granular Wettable Power (Dry flowable)

Compound No. 1-35 of this invention ---- 75 parts

Isoban No. 1 --------------------------- 10 parts
(Anionic sufactant: produced by Kuraray Isopren Chemical Co., Ltd., trade name)

Vanilex N ------------------------------ 5 parts
(Anionic surfactant: produced by Sanyo-Kokusaku Pulp Co., Ltd., trade name)

Carplex #80 (Antisolidifying agent) ---- 10 parts
(White carbon: produced by Shionogi & Co., Ltd., trade name)

The above components were uniformly mixed and finely pulverized to prepare a dry flowable.

For using the material, in the case of the above wettable powder, emulsifiable concentrate, dry flowable or flowable, they can be used by diluting with water 50 to 1000-fold and spread with an effective ingredient of 0.001 to 2 kg/ha (hectare).

The compound of the present invention can be also applied to prevention and removal of various kinds of

weeds at, other than agricultural and horticultural grounds such as farmland, paddy field, orchard, etc., non-arable land such as railroad line, factory site, road, bank, river basin, park, ground, graveyard, school and hospital grounds, shrine and temple grounds, surroundings of buildings, transformer substation, tank yard, air port, patrol road of power line, lawn yard, golf course, etc., or pasture and lawn yard. An applied chemical dosage thereof may vary depending on the place to be applied, time to be applied, kinds of subjective weeds, cultivated crop plants, etc., but generally it is suitable with a ratio of 0.01 to 10 kg per hectare.

Next, availability of the compound of the present invention as a herbicide is explained more specifically in the following test examples.

Test Example-1 Herbicidal effect test by soil treatment

In a plastic box of 15 cm length, 22 cm width and 6 cm depth, there was placed a sterilized dilvial soil, and seeds of barnyardgrass (*Echinochloa crusgalli*), large crabgrass (*Digitaria adscendens*), annual sedge (*Cyperus microiria*), black nightshade (*Solanum nigrum* L.), hairly galinosoga (*Galinsoga ciliat*), rorippa ssp. (*Rorippa atrovirens*), rice (*Oryza sativa*), corn (*Zea mays*), wheat (*Triticum aestivum*), soybean (*Glysine max*) and cotton (*Gossypium herbaceum*) were sown mixedly. After covering soil to about 1 cm over the seeds, herbicides were sprayed evenly on the soil surface to predetermined proportions of the active ingredient. In spraying, the wettable powder, emulsifiable concentrate, dry flowable or flowable in the above formulation examples were diluted with water and sprayed over the entire surface by means of a small sprayer. Three weeks after spraying, the herbicidal effect on crop plants including rice, corn, wheat, soybean, cotton and various kinds of weeds were examined according to the judgement criteria shown below. The results are shown in Table 3-1-1.

Judgement criteria

5 ... Growth control,rates of more than 90% (almost completely withered)
4 ... Growth control rates of 70 to 90%
3 ... Growth control rates of 40 to 70%
2 ... Growth control rates of 20 to 40%
1 ... Growth control rates of 5 to 20%
0 ... Growth control rates of less than 5% (substantially no effect)

The above growth control rates are determined by measuring the top fresh weights of the treated plants and those of the non-treated plants, and culculated from the following formula:

$$\text{Growth control rate (\%)} = \left(1 - \frac{\text{Top fresh weight of the treated plants}}{\text{Top fresh weight of the non-treated plants}}\right) \times 100$$

Test Example-2 Herbicidal effect test by foliage treatment

In a plastic box of 15 cm length, 22 cm width and 6 cm depth, there was placed a sterilized dilvial soil, and seeds of barnyardgrass (*Echinochloa crusgalli*), large crabgrass (*Digitaria adscendens*), annual sedge (*Cyperus microiria*), black nightshade (*Solanum nigrum* L.), hairly galinosoga (*Galinsoga ciliat*), rorippa ssp. (*Rorippa indica*), rice (*Oryza sativa*), corn (*Zea mays*), wheat (*Triticum vulgare*), soybean (*Glysine max*), cotton (*Gossypium herbaceum*) and beet (*Beta vulgaris*) were sown in shapes of spots, respectively, followed by covering of soil to about 1 cm over the seeds. After respective plants have grown to the second and the third leaf stage, herbicides were sprayed evenly onto the foliage portion at predetermined proportions of the active ingredient.

In spraying, the wettable powder, emulsifiable concentrate, dry flowable or flowable in the above formulation examples were diluted with water and sprayed over the entire surface of the foliage portions of various weeds and crop plants by means of a small sprayer. Four weeks after spraying, the herbicidal effect on crop plants including rice, corn, wheat, soybean, cotton and various kinds of weeds were examined according to the judgement criteria shown in Test Example-1. The results are shown in Table 3-2-1.

Test Exmaple-3 Herbicidal effect test under in an irrigation condition.

Wagner pots, each having an area of 1/5000 are, were packed with alluvial soil and then water is charged to mix them to make filling water condition with a water depth of 2 cm. Each seed of barnyardgrass (*Echinochloa crusgalli*), monochoria (*Monochoria vaginalis*), toothcup (*Rotala indica*) and bulrush (*Scirpus juncoides*) was

sown in the above pots and each tuber of arrowhead (*Sagittaria pygmaea*) and flatstage (*Cyperus serotinus*) was placed, and then rice plant seedlings at 2.5 leaf stage were also transplanted. Then, the pots were placed in a green house at 25 to 30°C to grown plants, and 3 days later, a predetermined amount of dilute solution of chemical of each test compound was added dropwise to the water surface with mess pipet. Three weeks after addition of the chemicals, the herbicidal effects to rice and various kinds of weeds were investigated according to judgement criteria in Test Example-1. The results are shown in Table 3-3-1.

Table 3-1-1

| Compound No. | Application amount (g/a) | N | M | K | H | D | I | R | T | W | S | C |
|---|---|---|---|---|---|---|---|---|---|---|---|---|
| 1-1 | 0.4 | 4 | 5 | 5 | 5 | 5 | 5 | 2 | 0 | 3 | 0 | 0 |
| | 0.8 | 5 | 5 | 5 | 5 | 5 | 5 | 3 | 0 | 4 | 0 | 0 |
| | 1.6 | 5 | 5 | 5 | 5 | 5 | 5 | 4 | 1 | 4 | 0 | 0 |
| 1-2 | 0.4 | 4 | 5 | 5 | 5 | 4 | 5 | 0 | 0 | 0 | 0 | 0 |
| | 0.8 | 5 | 5 | 5 | 5 | 5 | 5 | 1 | 0 | 1 | 0 | 0 |
| | 1.6 | 5 | 5 | 5 | 5 | 5 | 5 | 3 | 1 | 3 | 0 | 0 |
| 1-3 | 0.4 | 5 | 5 | 5 | 5 | 5 | 5 | 4 | 2 | 2 | 0 | 0 |
| | 0.8 | 5 | 5 | 5 | 5 | 5 | 5 | 5 | 3 | 3 | 0 | 1 |
| | 1.6 | 5 | 5 | 5 | 5 | 5 | 5 | 5 | 4 | 5 | 2 | 3 |
| 1-5 | 1.6 | 4 | 5 | 5 | 5 | 5 | 5 | 1 | 0 | 1 | 0 | 0 |
| 1-15 | 1.6 | 3 | 5 | 5 | 5 | 5 | 5 | 0 | 0 | 0 | 0 | 0 |
| 1-22 | 1.6 | 5 | 5 | 5 | 5 | 5 | 5 | 0 | 0 | 0 | 0 | 0 |
| 1-23 | 0.4 | 5 | 5 | 5 | 5 | 5 | 5 | 2 | 4 | 4 | 0 | 2 |
| | 0.8 | 5 | 5 | 5 | 5 | 5 | 5 | 3 | 5 | 5 | 1 | 3 |
| | 1.6 | 5 | 5 | 5 | 5 | 5 | 5 | 5 | 5 | 5 | 2 | 4 |

Table 3-1-1 (Cont'd)

| Compound No. | Application amount (g/a) | N | M | K | H | D | I | R | T | W | S | C |
|---|---|---|---|---|---|---|---|---|---|---|---|---|
| 1-24 | 0.4 | 3 | 5 | 5 | 5 | 5 | 5 | 1 | 0 | 0 | 0 | 0 |
| | 0.8 | 4 | 5 | 5 | 5 | 5 | 5 | 4 | 2 | 1 | 0 | 1 |
| | 1.6 | 5 | 5 | 5 | 5 | 5 | 5 | 5 | 4 | 4 | 1 | 3 |
| 1-25 | 1.6 | 5 | 5 | 5 | 5 | 5 | 5 | 1 | 0 | 0 | 0 | 1 |
| 1-26 | 0.4 | 5 | 5 | 5 | 5 | 5 | 5 | 5 | 4 | 4 | 0 | 5 |
| | 0.8 | 5 | 5 | 5 | 5 | 5 | 5 | 5 | 5 | 5 | 2 | 5 |
| | 1.6 | 5 | 5 | 5 | 5 | 5 | 5 | 5 | 5 | 5 | 4 | 5 |
| 1-27 | 0.4 | 5 | 5 | 5 | 5 | 5 | 5 | 2 | 0 | 2 | 0 | 0 |
| | 0.8 | 5 | 5 | 5 | 5 | 5 | 5 | 3 | 1 | 4 | 0 | 1 |
| | 1.6 | 5 | 5 | 5 | 5 | 5 | 5 | 4 | 4 | 5 | 1 | 2 |
| 1-28 | 1.6 | 5 | 5 | 5 | 5 | 5 | 5 | 0 | 0 | 2 | 0 | 0 |
| 1-35 | 1.6 | 4 | 5 | 5 | 5 | 5 | 5 | 0 | 0 | 1 | 0 | 0 |
| 1-12 | 6.3 | 2 | 5 | 5 | 2 | 4 | 5 | 2 | 0 | 1 | 0 | 0 |

Table 3-1-1 (Cont'd)

| Compound No. | Application amount (g/a) | N | M | K | H | D | I | R | T | W | S | C |
|---|---|---|---|---|---|---|---|---|---|---|---|---|
| | 1.6 | 1 | 1 | 5 | 5 | 5 | 5 | 0 | 0 | 0 | 0 | 0 |
| 1-29 | 3.2 | 2 | 2 | 5 | 5 | 5 | 5 | 0 | 0 | 0 | 0 | 0 |
| | 6.3 | 3 | 3 | 5 | 5 | 5 | 5 | 0 | 0 | 0 | 0 | 0 |
| 1-31 | 6.3 | 4 | 4 | 5 | 5 | 5 | 5 | 0 | 0 | 0 | 0 | 0 |
| 2-32 | 6.3 | 3 | 4 | 5 | 3 | 5 | 5 | 2 | 0 | 0 | 0 | 0 |

Table 3-2-1

| Compound No. | Application amount (g/a) | N | M | K | H | D | I | R | T | W | S | C | B |
|---|---|---|---|---|---|---|---|---|---|---|---|---|---|
| 1-2 | 1.6 | 5 | 5 | 5 | 5 | 5 | 5 | | | | | | |
| 1-3 | 0.4 | 5 | 5 | 5 | 5 | 5 | 5 | | | | | | |
| | 0.8 | 5 | 5 | 5 | 5 | 5 | 5 | | | | | | |
| | 1.6 | 5 | 5 | 5 | 5 | 5 | 5 | | | | | | |
| 1-5 | 1.6 | 5 | 5 | 5 | 5 | 5 | 5 | | | | | | |
| 1-15 | 1.6 | 4 | 5 | 5 | 5 | 5 | 5 | | | | | | |
| 1-23 | 0.4 | 5 | 5 | 5 | 5 | 5 | 5 | 2 | 4 | 2 | 5 | 5 | 5 |
| | 0.8 | 5 | 5 | 5 | 5 | 5 | 5 | 3 | 5 | 3 | 5 | 5 | 5 |
| | 1.6 | 5 | 5 | 5 | 5 | 5 | 5 | 5 | 5 | 5 | 5 | 5 | 5 |
| 1-24 | 1.6 | 5 | 4 | 5 | 5 | 5 | 5 | | | | | | |
| 1-25 | 1.6 | 5 | 3 | 5 | 5 | 5 | 5 | | | | | | |
| 1-26 | 0.4 | 5 | 5 | 5 | 5 | 5 | 5 | | | | | | |
| | 0.8 | 5 | 5 | 5 | 5 | 5 | 5 | | | | | | |
| | 1.6 | 5 | 5 | 5 | 5 | 5 | 5 | | | | | | |

Table 3-2-1 (Cont'd)

| Compound No. | Application amount (g/a) | N | M | K | H | D | I | R | T | W | S | C | B |
|---|---|---|---|---|---|---|---|---|---|---|---|---|---|
| 1-27 | 1.6 | 5 | 3 | 5 | 5 | 5 | 3 | | | | | | |
| 1-35 | 1.6 | 3 | 4 | 5 | 5 | 5 | 5 | | | | | | |
| 1-22 | 1.6 | 4 | 2 | 5 | 5 | 5 | 5 | 2 | 1 | 1 | 3 | 5 | 2 |
| 1-12 | 6.3 | 2 | 2 | 5 | 5 | 4 | 2 | 3 | 2 | 0 | 2 | 2 | 5 |
| 1-29 | 1.6 | 1 | 1 | 4 | 5 | 5 | 3 | 0 | 0 | 0 | 0 | 4 | 4 |
| | 3.2 | 2 | 2 | 5 | 5 | 5 | 4 | 0 | 0 | 0 | 1 | 5 | 5 |
| | 6.3 | 3 | 3 | 5 | 5 | 5 | 5 | 1 | 1 | 0 | 2 | 5 | 5 |
| 1-31 | 6.3 | 2 | 2 | 5 | 5 | 5 | 2 | 2 | 1 | 1 | 3 | 5 | 5 |
| 2-32 | 6.3 | 4 | 2 | 5 | 3 | 5 | 5 | 3 | 1 | 1 | 2 | 4 | 5 |

Table 3-3-1

| Compound No. | Application amount (g/a) | N | b | c | d | e | f | g |
|---|---|---|---|---|---|---|---|---|
| 1-1 | 0.4 | 5 | 5 | 5 | 5 | 5 | 4 | 0 |
| 1-5 | 0.4 | 5 | 5 | 5 | 5 | 5 | 5 | 0 |

In the table, symbols represent the following meanings. N (*Echinochloa crusgalli*), M (*Digitaria adscendens*), K (*Cyperus microiria*), H (*Solanum nigrum L.*), D (*Galinsoga ciliat*), I (*Rorippa indica*), R (*Oryza sativa*), T (*Zea mays*), W (*Triticum vulgare*), S (*Glysine max*), C (*Gossypium herbaceum*), B (*Beta vulgaris*), b (*Scirpus zuncoides*), c (*Monochoria vaginalis*), d (*Rotala indica*), e (*Sagittaria pygmaea*), f (*Cyperus serotinus*), g (transplanted *Oryza sativa*).

## Claims

**1.** Glutarimide derivatives represented by the formula (I):

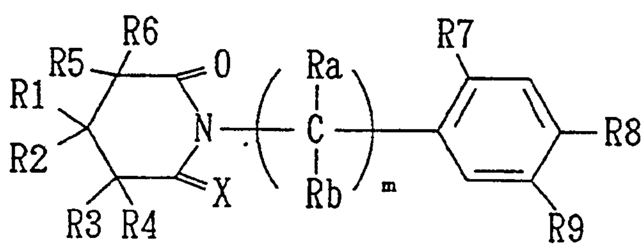

(I)

wherein X represents oxygen atom or sulfur atom;

R1, R2, R3, R4, R5 and R6 each independently represent hydrogen atom, a halogen atom, cyano group, a($C_1$ to $C_6$)alkyl group, a($C_3$ to $C_6$)cycloalkyl group, a($C_3$ to $C_6$)alkenyl group, a($C_3$ to $C_6$)alkynyl group, a($C_1$ to $C_6$)haloalkyl group, a($C_1$ to $C_6$)alkoxy group, a($C_1$ to $C_6$)haloalkoxy group, a($C_1$ to $C_6$)alkylthio group, a($C_1$ to $C_6$)haloalkylthio group, a($C_3$ to $C_6$)halocycloalkyl group, a phenyl group which may be substituted one or more substituent (the substituent is at least one selected from a halogen atom, a($C_1$ to $C_4$)alkyl group, a($C_1$ to $C_4$)haloalkyl group, a($C_1$ to $C_4$)alkoxy group and a($C_1$ to $C_4$)haloalkoxy group) or a benzyl group which may be substituted one or more substituent (the substituent is at least one selected from a halogen atom, a($C_1$ to $C_4$)alkyl group, a($C_1$ to $C_4$)haloalkyl group, a($C_1$ to $C_4$)alkoxy group and a($C_1$ to $C_4$)haloalkoxy group), (two substituent groups on the same carbon atom may form a spiro ring by 2 to 5 methylene chains which may be optionally susbtituted by a halogen atom or two substituent groups on the adjacent carbon atoms may form a 5- to 6-membered ring by 1 to 4 methylene chains which may be optionally susbtituted by a halogen atom);

m is an integer of 0 to 3;

Ra and Rb each independently represent hydrogen atom or a($C_1$ to $C_4$)alkyl group;

R7 represents hydrogen atom or a halogen atom;

(i) when R8 and R9 are taken separately,

R8 represents a halogen atom, a($C_1$ to $C_4$)alkyl group, hydrogen atom, cyano group or nitro group;

R9 represents hydrogen atom, a halogen atom, a($C_1$ to $C_6$)alkyl group, cyano group, a($C_1$ to $C_4$)haloalkyl group, nitro group, amino group, hydroxyl group, mercapto group, carboxyl group, OR11 [wherein R11 represents a($C_1$ to $C_6$)alkyl group, a($C_3$ to $C_6$)cycloalkyl group, a($C_3$ to $C_6$)cycloalkyl($C_1$ to $C_2$)alkyl group, a($C_2$ to $C_6$)alkenyl group, a($C_3$ to $C_6$)alkynyl group, a($C_1$ to $C_4$)haloalkyl group, phenyl group or benzyl group],

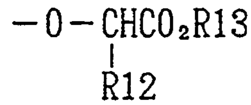

[wherein R12 represents hydrogen atom, a($C_1$ to $C_3$)alkyl group, methylthio group, phenylthio group or methoxymethyl group, R13 represents hydrogen atom, sodium atom, potassium atom, ammonium group, a($C_1$ to $C_6$)alkyl group, a($C_2$ to $C_6$)alkenyl group, a($C_3$ to $C_6$)alkynyl group, a($C_3$ to $C_6$)cycloalkyl group, a($C_3$ to $C_6$)cycolalkyl($C_1$ to $C_2$)alkyl group, a($C_2$ to $C_4$)haloalkyl group, phenyl group, phenethyl group or benzyl group], $-CO_2R14$ [wherein R14 represents a($C_1$ to $C_6$)alkyl group, a($C_2$ to $C_6$)alkenyl group, a($C_3$ to $C_6$)alkynyl group, a($C_3$ to $C_6$)cycloalkyl group, a($C_2$ to $C_4$)haloalkyl group, phenyl group or benzyl group], $-CO_2CH_2CO_2R15$ [wherein R15 represents hydrogen atom or a($C_1$ to $C_4$)alkyl group],

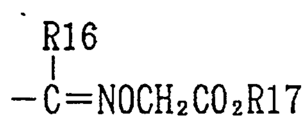

[wherein R16 represents hydrogen atom, a($C_1$ to $C_3$)alkyl group, a($C_1$ to $C_3$)alkoxy group or a($C_1$ to $C_3$)alkylthio group, R17 represents hydrogen atom, a($C_1$ to $C_6$)alkyl group, a($C_3$ to $C_6$)cycloalkyl group, a($C_2$ to $C_3$)haloalkyl group, phenyl group or benzyl group],

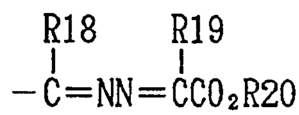

[wherein R18 represents hydrogen atom, a($C_1$ to $C_3$)alkyl group, a($C_1$ to $C_3$)alkoxy group or a($C_1$ to $C_3$)alkylthio group, R19 represents hydrogen atom or methyl group, R20 represents hydrogen atom, a($C_1$ to $C_4$)alkyl group, a($C_3$ to $C_6$)cycloalkyl group, a($C_2$ to $C_4$)haloalkyl group, phenyl group or benzyl group],

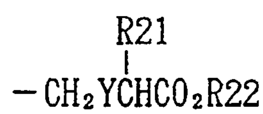

[wherein Y represents oxygen atom or sulfur atom, R21 represents hydrogen or a($C_1$ to $C_3$)alkyl group, R22 represents hydrogen atom, a($C_1$ to $C_6$)alkyl group, a($C_3$ to $C_6$)cycloalkyl group, a($C_2$ to $C_4$)haloalkyl group, phenyl group or benzyl group], -SR23 [wherein R23 represents a($C_1$ to $C_6$)alkyl group, a($C_3$ to $C_6$)cycloalkyl group, a($C_2$ to $C_6$)alkenyl group, a($C_3$ to $C_6$)alkynyl group, cyanomethyl group, hydroxymethyl group or chloromethyl group],

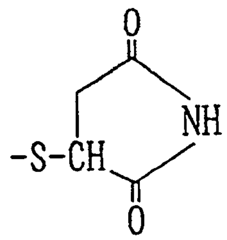 , 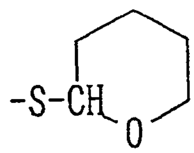 ,

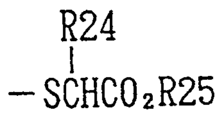

[wherein R24 represents hydrogen atom, a($C_1$ to $C_3$)alkyl group, methylthio group, phenylthio group or methoxymethyl group, R25 represents hydrogen atom, sodium atom, potassium atom, ammonium group, diisopropylammonium group, a($C_1$ to $C_6$)alkyl group, a($C_2$ to $C_6$)alkenyl group, a($C_3$ to $C_6$)alkynyl group, a($C_3$ to $C_6$)cycloalkyl group, a($C_3$ to $C_6$)cycloalkyl($C_1$ to $C_2$)alkyl group, a($C_2$ to $C_4$)haloalkyl group, phenyl group, benzyl group, α-methylbenzyl group, $-CH_2CH_2CH_2N(CH_3)_2$, a phenyl group which may be substituted one or more substituent (the substituent is at least one selected from a halogen atom, a($C_1$ to $C_4$)alkyl group, a($C_1$ to $C_4$)alkoxy group, a($C_1$ to $C_2$)alkylamino group, dimethylamino group, a($C_1$ to $C_2$)alkoxycarbonyl group, a($C_1$ to $C_2$)alkylcarbonyl group, a($C_1$ to $C_4$)haloalkyl group and a($C_1$ to $C_4$)haloalkyloxy group), or a benzyl group which may be substituted one or more substituent (the substituent is at least one selected from a halogen atom, a($C_1$ to $C_4$)alkyl group, a($C_1$ to $C_3$)alkoxy group, a($C_1$ to $C_2$)alkylamino group, dimethylamino group, a($C_1$ to $C_2$)alkylcarbonyl group, $-NHCOCF_3$, a($C_1$ to $C_2$)alkoxycarbonyl group, methylsulfonyl group, trifluoromethylsulfonyl group, a($C_1$ to $C_2$)haloalkyl group and a($C_1$ to $C_2$)haloalkyloxy group)], $-SCH_2(CH_2)_nCO_2R25$ [wherein n is an integer of 1 to 4, R25 is the same meaning as defined above], $-SCH_2$-Y-R26 [wherein Y is the same meaning as defined above, R26 represents hydrogen atom, a($C_1$ to $C_5$)alkyl group, cyanomethyl group, a($C_1$ to $C_4$)alkylcarbonyl group, $-COCH_2Cl$ or $CON(CH_3)_2$], $-SCH_2$-Y-CH(R24)$CO_2$R25 [wherein Y, R24 and R25 are the same meanings as defined above], $-SCH_2$-Y-$CH_2$-$(CH_2)_n$-$CO_2$R25 [wherein Y, n and R25 are the same meanings as defined above],

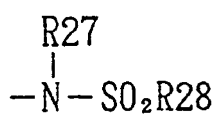

[wherein R27 represents hydrogen atom, sodium atom, a($C_1$ to $C_4$)alkyl group, a($C_1$ to $C_4$)alkylsulfonyl group or a($C_1$ to $C_3$)alkoxy($C_1$ to $C_2$)alkyl group, and R28 represents a($C_1$ to $C_4$)alkyl group or a($C_1$ to $C_3$)haloalkyl group], -N(R27)$CO_2$R25 [wherein R27 and R25 are the same meanings as defined above], -N(R26)CH(R12)$CO_2$R25 [wherein R12, R25 and R26 are the same meanings as defined above], -N(R27)COR11 [wherein R11 and R27 are the same meanings as defined above],

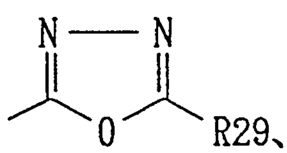 , 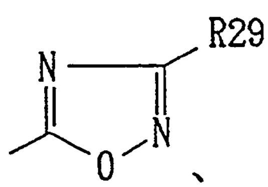 , 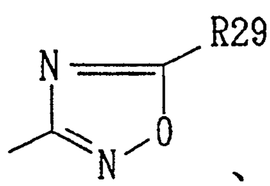 ,

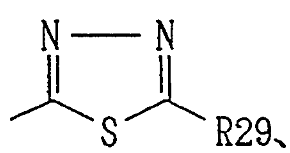 , 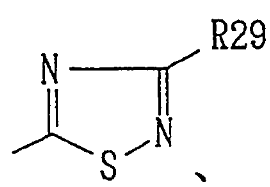 , 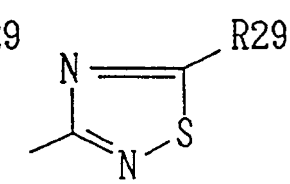 ,

[wherein R29 represents hydrogen atom, a($C_1$ to $C_6$)alkyl group, a($C_3$ to $C_6$)cycloalkyl group, a($C_1$ to

$C_3$)haloalkyl group, a($C_1$ to $C_5$)alkoxy group, a($C_1$ to $C_6$)alkylthio group, a($C_1$ to $C_4$)alkoxycarbonyl group, mercapto group, hydroxyl group, amino group, a($C_1$ to $C_3$)alkoxy($C_1$ to $C_2$)alkyl group, a($C_1$ to $C_6$)alkylamino group, NR30(R31) {wherein R30 and R31 each independently represent a($C_1$ to $C_6$)alkyl group}, -CON($CH_3$)$_2$, -CONHSO$_2$CH$_3$, a($C_1$ to $C_6$)alkylsulfonyl group, a halogen atom, phenyl group, benzyl group or -N(R32)SO$_2$R33 {wherein R32 represents hydrogen atom, sodium atom, a($C_1$ to $C_6$)alkyl group, a($C_1$ to $C_4$)alkylsulfonyl group, a($C_1$ to $C_3$)alkoxy($C_1$ to $C_2$)alkyl group, and R33 represents a($C_1$ to $C_6$)alkyl group or a($C_1$ to $C_4$)haloalkyl group}]

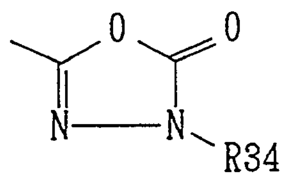

[wherein R34 represents a($C_1$ to $C_6$)alkyl group or a($C_3$ to $C_6$)cycloalkyl group],

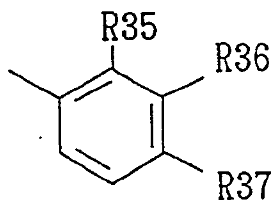

[wherein R35, R36 and R37 each independently represent hydrogen atom or a($C_1$ to $C_6$)alkyl group],

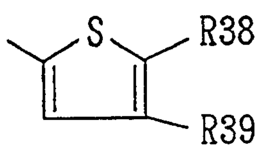
、
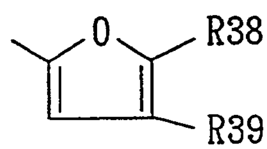

[wherein R38 and R39 each independently represent a halogen atom, hydrogen atom, a($C_1$ to $C_6$)alkyl group or dimethylamino group],

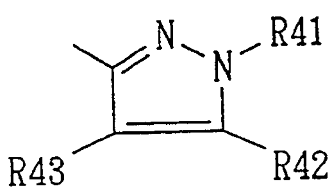
、
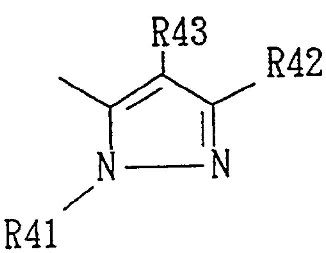

[wherein R41 represents hydrogen atom, a($C_1$ to $C_6$)alkyl group, a($C_3$ to $C_6$)cycloalkyl group, phenyl group, benzyl group or 2-pyridyl group, R42 represents hydrogen atom, a halogen atom, hydroxyl group, amino group, mercapto group, a($C_1$ to $C_6$)alkyl group, a($C_1$ to $C_4$)alkylamino group, -NR30(R31) {wherein R30 and R31 are the same meanings as defined above}, a($C_1$ to $C_4$)alkylthio group, -CO$_2$R30 {wherein R30 is the same meaning as defined above}, a($C_1$ to $C_4$)alkylsulfonyl group, a($C_1$ to $C_4$)alkoxy group or N(R27)SO$_2$R28 {wherein R27 and R28 are the same meanings as defined above}, R43 represents hydrogen atom, a halogen atom, nitro group, amino group, cyano group, a($C_1$ to $C_4$)alkylamino group, -NR30(R31) {wherein R30 and R31 are the same meanings as defined above}, a($C_1$ to $C_4$)alkylcarbonyl group or a($C_1$ to $C_4$)alkoxycarbonyl group],

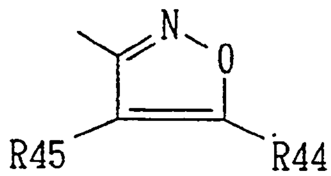
,
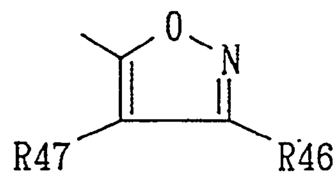

[wherein R44 represents hydrogen atom, hydroxyl group, methoxy group, amino group, benzoyl group or a($C_1$ to $C_6$)alkyl group, R45 represents hydrogen atom, cyano group, acetyl group, a($C_1$ to $C_6$)alkyl group, carboxyl group or a($C_1$ to $C_4$)alkoxycarbonyl group, R46 and R47 each independently represent hydrogen atom, phenyl group, hydroxyl group, methoxy group or a($C_1$ to $C_6$)alkyl group],

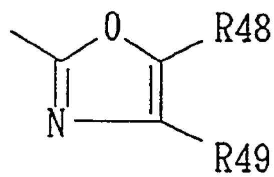

[wherein R48 and R49 each independently represent hydrogen atom or a($C_1$ to $C_6$)alkyl group], or

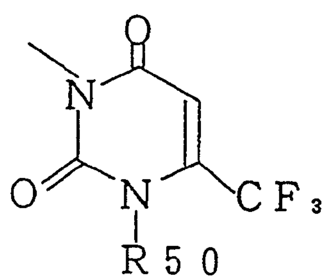

[wherein R50 represents hydrogen atom, sodium atom or a($C_1$ to $C_4$)alkyl group];
(ii) when R8 and R9 form a ring,

the glutarimide derivatives are represented by the formula (II), formula (III), formula (IV), formula (V) or formula (VI):

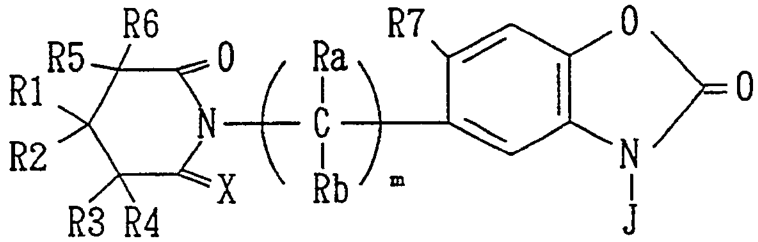

(II)

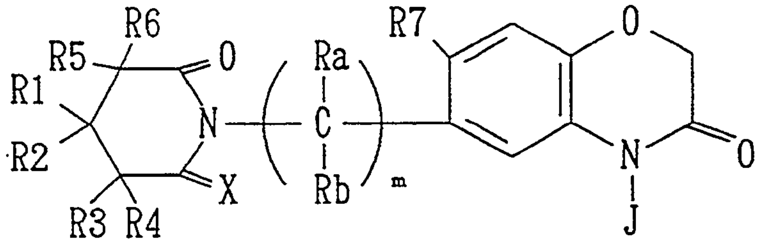

(III)

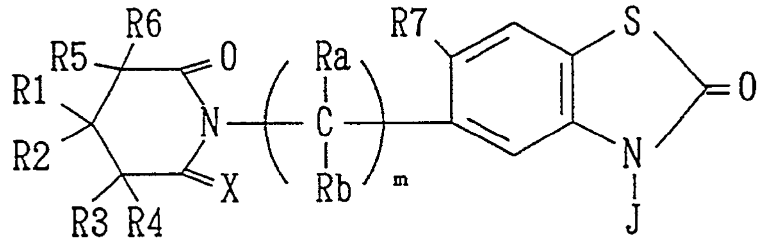

(IV)

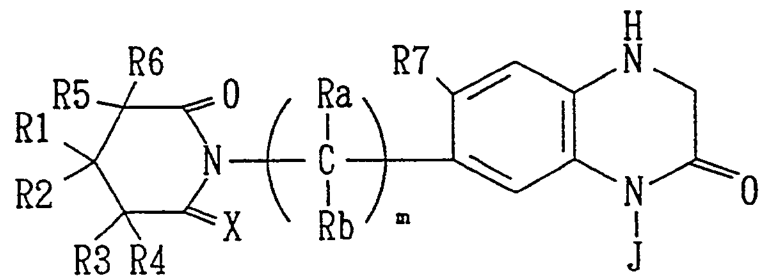

(V)

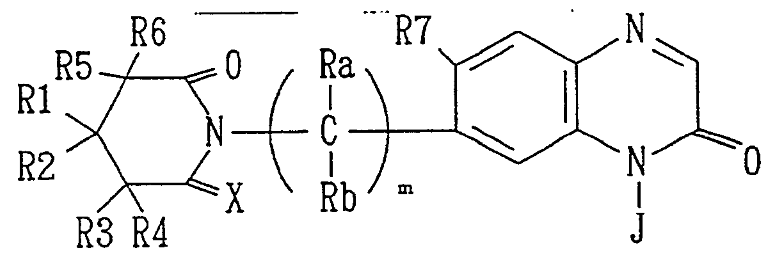

(VI)

wherein Ra, Rb, m, R1, R2, R3, R4, R5, R6, R7 and X are the same meanings as defined above;

J represents hydrogen atom, a($C_1$ to $C_6$)alkyl group, a($C_2$ to $C_6$)alkenyl group, a($C_3$ to $C_6$)alkynyl group, hydroxymethyl group, chloromethyl group, $-CH_2CONH_2$, cyanomethyl group, $-CH_2CO_2$-($C_1$ to $C_4$)alkyl, a($C_3$ to $C_6$)cycloalkyl group, a($C_3$ to $C_6$)cycloalkyl($C_1$ to $C_2$)alkyl group, a($C_1$ to $C_2$)alkoxy($C_1$ to $C_2$)alkyl group, a phenyl group which may be substituted one or more substituent (the substituent is at least one selected from a($C_1$ to $C_6$)alkyl group, a($C_1$ to $C_6$)alkoxy group, a halogen atom, a($C_1$ to $C_6$)haloalkyl group, a($C_1$ to $C_6$)haloalkoxy group, a($C_3$ to $C_6$)cycloalkyl group, a($C_1$ to $C_4$)alkylamino group, a($C_1$ to $C_4$)alkoxycarbonyl group, a($C_2$ to $C_4$)alkylcarbonyl group, a($C_1$ to $C_4$)alkylsulfonyl group, a($C_2$ to $C_6$)alkenyl group and a($C_2$ to $C_6$)alkynyl group) or a benzyl group which may be substituted one or more substituent (the substituent is at least one selected from a($C_1$ to $C_6$)alkyl group, a($C_1$ to $C_6$)alkoxy group, a halogen atom, a($C_1$ to $C_6$)haloalkyl group, a($C_1$ to $C_6$)haloalkoxy group, a($C_3$ to $C_6$)cycloalkyl group, a($C_1$ to $C_4$)alkylamino group, a($C_1$ to $C_4$)alkoxycarbonyl group, a($C_2$ to $C_4$)alkylcarbonyl group, a($C_1$ to $C_4$)alkylsulfonyl group, a($C_2$ to $C_6$)alkenyl group and a($C_2$ to $C_6$)alkynyl group),

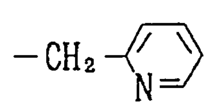 or 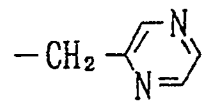 ;

provided that the glutarimide derivatives represented by the formula (A) are excluded,

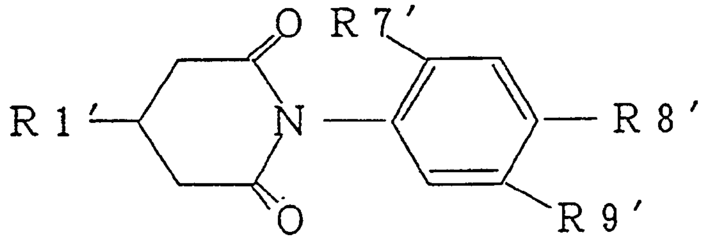 (A)

wherein R1′ represents hydrogen atom, a($C_1$ to $C_6$)alkyl group, a($C_3$ to $C_7$)cycloalkyl group, a($C_1$ to $C_6$)haloalkyl group, a($C_3$ to $C_6$)alkenyl group or a($C_3$ to $C_6$)alkynyl group;
R7′ represents hydrogen atom or a halogen atom;
R8′ represents hydrogen atom, cyano group, nitro group or a halogen atom;
R9′ represents hydrogen atom, cyano group, nitro group or hydroxyl group; -OR11 [wherein R11 is the same meaning as defined above], -O-CH(R12)$CO_2$R13 [wherein R12 and R13 are the same meanings as defined above], -$CO_2$R14 [wherein R14 is the same meaning as defined above], -C(R16′)=NO$CH_2CO_2$R17 [wherein R16′ represents hydrogen atom or a($C_1$ to $C_3$)alkyl group, and R17 is the same meaning as defined above], -SR23′ [wherein R23′ represents a($C_1$ to $C_6$)alkyl group, a($C_3$ to $C_6$)cycloalkyl group, a($C_2$ to $C_6$)alkenyl group, a($C_3$ to $C_6$)alkynyl group or cyanomethyl group], -SCH(R24)$CO_2$R25 [wherein R24 and R25 are the same meanings as defined above], or -$SCH_2(CH_2)_{n'}$-$CO_2$R25 [wherein n′ is an integer of 1 to 3 and R25 is the same meaning as defined above, and provided that when a compound of an optically active site, or a stereoisomer or geometric isomer is included in the glutarimide derivative, all these forms are included.

**2.** Glutaric acid amide derivatives represented by the formula (VII):

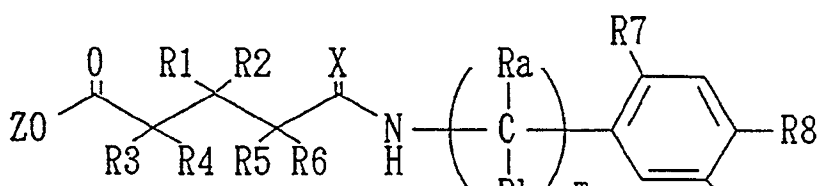

(VII)

wherein X represents oxygen atom or sulfur atom;
Z represents hydrogen atom, a($C_1$ to $C_6$)alkyl group, phenyl group or benzyl group;
R1, R2, R3, R4, R5 and R6 each independently represent hydrogen atom, a halogen atom, cyano group, a($C_1$ to $C_6$)alkyl group, a($C_3$ to $C_6$)cycloalkyl group, a($C_3$ to $C_6$)alkenyl group, a($C_3$ to $C_6$)alkynyl group, a($C_1$ to $C_6$)haloalkyl group, a($C_1$ to $C_6$)alkoxy group, a($C_1$ to $C_6$)haloalkoxy group, a($C_1$ to $C_6$)alkylthio group, a($C_1$ to $C_6$)haloalkylthio group, a($C_3$ to $C_6$)halocycloalkyl group, a phenyl group which may be substituted one or more substituent (the substituent is at least one selected from a halogen atom, a($C_1$ to $C_4$-)alkyl group, a($C_1$ to $C_4$)haloalkyl group, a($C_1$ to $C_4$)alkoxy group and a($C_1$ to $C_4$)haloalkoxy group) or a benzyl group which may be substituted one or more substituent (the substituent is at least one selected from a halogen atom, a($C_1$ to $C_4$)alkyl group, a($C_1$ to $C_4$)haloalkyl group, a($C_1$ to $C_4$)alkoxy group and a($C_1$ to $C_4$)haloalkoxy group), (two substituent groups on the same carbon atom may form a spiro ring by 2 to 5 methylene chains which may be optionally susbtituted by a halogen atom or two substituent groups on the adjacent carbon atom may form a 5- to 6-membered ring by 1 to 4 methylene chains which may be optionally susbtituted by a halogen atom);
m is an integer of 0 to 3;
Ra and Rb each independently represent hydrogen atom or a($C_1$ to $C_4$)alkyl group;

R7 represents hydrogen atom or a halogen atom;

(i) when R8 and R9 are taken separately,

R8 represents a halogen atom, a($C_1$ to $C_4$)alkyl group, hydrogen atom, cyano group or nitro group;

R9 represents hydrogen atom, a halogen atom, a($C_1$ to $C_6$)alkyl group, cyano group, a($C_1$ to $C_4$)haloalkyl group, nitro group, amino group, hydroxyl group, mercapto group, carboxyl group, OR11 [wherein R11 represents a($C_1$ to $C_6$)alkyl group, a($C_3$ to $C_6$)cycloalkyl group, a($C_3$ to $C_6$)cycloalkyl($C_1$ to $C_2$)alkyl group, a($C_2$ to $C_6$)alkenyl group, a($C_3$ to $C_6$)alkynyl group, a($C_1$ to $C_4$)haloalkyl group, phenyl group or benzyl group],

$$-O-\underset{R12}{\underset{|}{C}}HCO_2R13$$

[wherein R12 represents hydrogen atom, a($C_1$ to $C_3$)alkyl group, methylthio group, phenylthio group or methoxymethyl group, R13 represents hydrogen atom, sodium atom, potassium atom, ammonium group, a($C_1$ to $C_6$)alkyl group, a($C_2$ to $C_6$)alkenyl group, a($C_3$ to $C_6$)alkynyl group, a($C_3$ to $C_6$)cycloalkyl group, a($C_3$ to $C_6$)cycloalkyl($C_1$ to $C_2$)alkyl group, a($C_2$ to $C_4$)haloalkyl group, phenyl group, phenethyl group or benzyl group], $-CO_2R14$ [wherein R14 represents a($C_1$ to $C_6$)alkyl group, a($C_2$ to $C_6$)alkenyl group, a($C_3$ to $C_5$)alkynyl group, a($C_3$ to $C_6$)cycloalkyl group, a($C_2$ to $C_4$)haloalkyl group, phenyl group or benzyl group], $-CO_2CH_2CO_2R15$ [wherein R15 represents hydrogen atom or a($C_1$ to $C_4$)alkyl group],

$$-\overset{R16}{\overset{|}{C}}=NOCH_2CO_2R17$$

[wherein R16 represents hydrogen atom, a($C_1$ to $C_3$)alkyl group, a($C_1$ to $C_3$)alkoxy group or a($C_1$ to $C_3$)alkylthio group, R17 represents hydrogen atom, a($C_1$ to $C_6$)alkyl group, a($C_3$ to $C_6$)cycloalkyl group, a($C_2$ to $C_3$)haloalkyl group, phenyl group or benzyl group],

$$-\overset{R18}{\overset{|}{C}}=NN=\overset{R19}{\overset{|}{C}}CO_2R20$$

[wherein R18 represents hydrogen atom, a($C_1$ to $C_3$)alkyl group, a($C_1$ to $C_3$)alkoxy group or a($C_1$ to $C_3$)alkylthio group, R19 represents hydrogen atom or methyl group, R20 represents hydrogen atom, a($C_1$ to $C_4$)alkyl group, a($C_3$ to $C_6$)cycloalkyl group, a($C_2$ to $C_4$)haloalkyl group, phenyl group or benzyl group],

$$-CH_2Y\overset{R21}{\overset{|}{C}}HCO_2R22$$

[wherein Y represents oxygen atom or sulfur atom, R21 represents hydrogen or a($C_1$ to $C_3$)alkyl group, R22 represents hydrogen atom, a($C_1$ to $C_6$)alkyl group, a($C_3$ to $C_6$)cycloalkyl group, a($C_2$ to $C_4$)haloalkyl group, phenyl group or benzyl group], -SR23 [wherein R23 represents a($C_1$ to $C_6$)alkyl group, a($C_3$ to $C_6$)cycloalkyl group, a($C_2$ to $C_6$)alkenyl group, a($C_3$ to $C_6$)alkynyl group, cyanomethyl group, hydroxymethyl group or chloromethyl group],

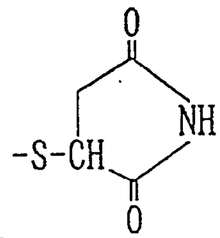 , 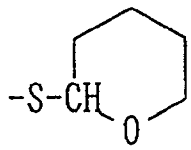 ,

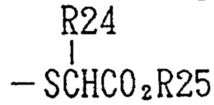

[wherein R24 represents hydrogen atom, a($C_1$ to $C_3$)alkyl group, methylthio group, phenylthio group or methoxymethyl group, R25 represents hydrogen atom, sodium atom, potassium atom, ammonium group, diisopropylammonium group, a($C_1$ to $C_6$)alkyl group, a($C_2$ to $C_6$)alkenyl group, a($C_3$ to $C_6$)alkynyl group, a($C_3$ to $C_6$)cycloalkyl group, a($C_3$ to $C_6$)cycloalkyl($C_1$ to $C_2$)alkyl group, a($C_2$ to $C_4$)haloalkyl group, phenyl group, benzyl group, α-methylbenzyl group, $-CH_2CH_2CH_2N(CH_3)_2$, a phenyl group which may be substituted one or more substituent (the substituent is at least one selected from a halogen atom, a($C_1$ to $C_4$)alkyl group, a($C_1$ to $C_4$)alkoxy group, a($C_1$ to $C_2$)alkylamino group, dimethylamino group, a($C_1$ to $C_2$)alkoxycarbonyl group, a($C_1$ to $C_2$)alkylcarbonyl group, a($C_1$ to $C_4$)haloalkyl group and a($C_1$ to $C_4$)haloalkyloxy group); or a benzyl group which may be substituted one or more substituent (the substituent is at least one selected from a halogen atom, a($C_1$ to $C_4$)alkyl group, a($C_1$ to $C_3$)alkoxy group, a($C_1$ to $C_2$)alkylamino group, dimethylamino group, a($C_1$ to $C_2$)alkylcarbony group, $-NHCOCF_3$, a($C_1$ to $C_2$)alkoxycarbonyl group, methylsulfonyl group, trifluoromethylsulfonyl group, a($C_1$ to $C_2$)haloalkyl group and a($C_1$ to $C_2$)haloalkyloxy group)], $-SCH_2(CH_2)_nCO_2R25$ [wherein n is an integer of 1 to 4, R25 is the same meaning as defined above], $-SCH_2$-Y-R26 [wherein Y is the same meaning as defined above, R26 represents hydrogen atom, a($C_1$ to $C_5$)alkyl group, cyanomethyl group, a($C_1$ to $C_4$)alkylcarbonyl group, $-COCH_2Cl$ or $CON(CH_3)_2$], $-SCH_2$-Y-CH(R24)$CO_2$R25 [wherein Y, R24 and R25 are the same meanings as defined above], $-SCH_2$-Y-$CH_2$-$(CH_2)_n$-$CO_2$R25 [wherein Y, n and R25 are the same meanings as defined above],

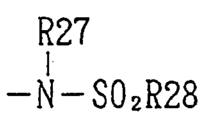

[wherein R27 represents hydrogen atom, sodium atom, a($C_1$ to $C_4$)alkyl group, a($C_1$ to $C_4$)alkylsulfonyl group or a($C_1$ to $C_3$)alkoxy($C_1$ to $C_2$)alkyl group, and R28 represents a($C_1$ to $C_4$)alkyl group or a($C_1$ to $C_3$)haloalkyl group], -N(R27)$CO_2$R25 [wherein R27 and R25 are the same meanings as defined above], -N(R26)CH(R12)$CO_2$R25 [wherein R12, R25 and R26 are the same meanings as defined above], -N(R27)COR11 [wherein R11 and R27 are the same meanings as defined above],

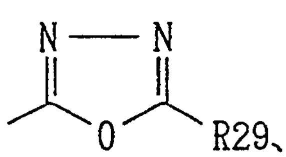 , 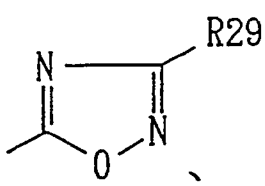 , 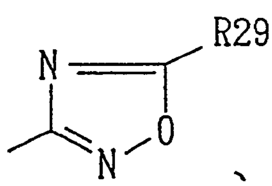 ,

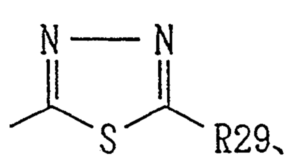 , 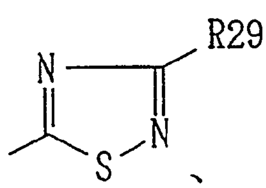 , 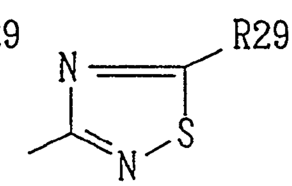

[wherein R29 represents hydrogen atom, a($C_1$ to $C_8$)alkyl group, a($C_3$ to $C_6$)cycloalkyl group, a($C_1$ to $C_3$)haloalkyl group, a($C_1$ to $C_5$)alkoxy group, a($C_1$ to $C_6$)alkylthio group, a($C_1$ to $C_4$)alkoxycarbonyl group, mercapto group, hydroxyl group, amino group, a($C_1$ to $C_3$)alkoxy($C_1$ to $C_2$)alkyl group, a($C_1$ to $C_6$)alkylamino

group, NR30(R31) {wherein R30 and R31 each independently represent a($C_1$ to $C_6$)alkyl group}, -$CON(CH_3)_2$, -$CONHSO_2CH_3$, a($C_1$ to $C_6$)alkylsulfonyl group, a halogen atom, phenyl group, benzyl group or -N(R32)$SO_2$R33 {wherein R32 represents hydrogen atom, sodium atom, a($C_1$ to $C_6$)alkyl group, a($C_1$ to $C_4$)alkylsulfonyl group, a($C_1$ to $C_3$)alkoxy($C_1$ to $C_2$)alkyl group, and R33 represents a($C_1$ to $C_6$)alkyl group or a($C_1$ to $C_4$)haloalkyl group}],

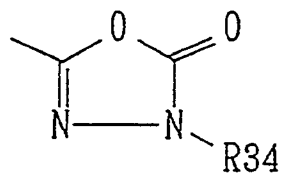

[wherein R34 represents a($C_1$ to $C_5$)alkyl group or a($C_3$ to $C_6$)cycloalkyl group],

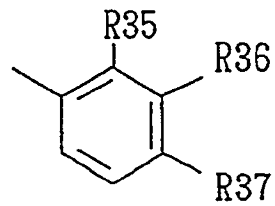

[wherein R35, R36 and R37 each independently represent hydrogen atom or a($C_1$ to $C_6$)alkyl group],

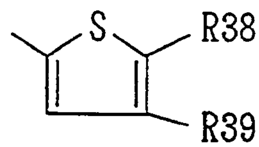

、

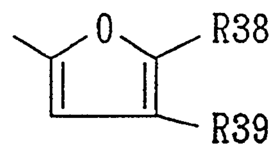

[wherein R38 and R39 each independently represent a halogen atom, hydrogen atom, a($C_1$ to $C_6$)alkyl group or dimethylamino group],

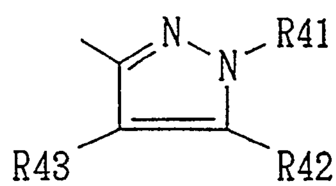

、

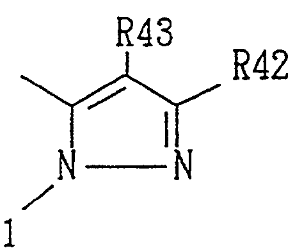

[wherein R41 represents hydrogen atom, a($C_1$ to $C_6$)alkyl group, a($C_3$ to $C_6$ )cycloalkyl group, phenyl group, benzyl group or 2-pyridyl group, R42 represents hydrogen atom, a halogen atom, hydroxyl group, amino group, mercapto group, a($C_1$ to $C_6$)alkyl group, a($C_1$ to $C_4$)alkylamino group, -NR30(R31) {wherein R 30 and R31 are the same meanings as defined above}, a($C_1$ to $C_4$)alkylthio group, -$CO_2$R30 {wherein R30 is the same meaning as defined above}, a($C_1$ to $C_4$)alkylsulfonyl group, a($C_1$ to $C_4$)alkoxy group or N(R27)$SO_2$R28 {wherein R27 and R28 are the same meanings as defined above}, R43 represents hydrogen atom, a halogen atom, nitro group, amino group, cyano group, a($C_1$ to $C_4$)alkylamino group, -NR30(R31) {wherein R30 and R31 are the same meanings as defined above}, a($C_1$ to $C_4$)alkylcarbonyl group or a($C_1$ to $C_4$)alkoxycarbonyl group],

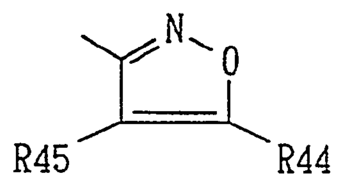
,
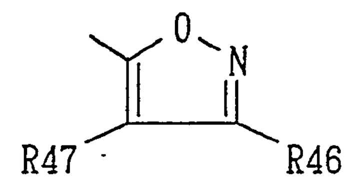

[wherein R44 represents hydrogen atom, hydroxyl group, methoxy group, amino group, benzoyl group or a($C_1$ to $C_6$)alkyl group, R45 represents hydrogen atom, cyano group, acetyl group, a($C_1$ to $C_6$)alkyl group, carboxyl group or a($C_1$ to $C_4$)alkoxycarbonyl group, R46 and R47 each independently represent hydrogen atom, phenyl group, hydroxyl group, methoxy group or a($C_1$ to $C_6$)alkyl group],

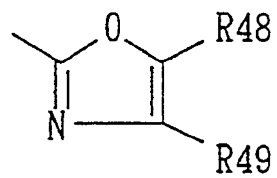

[wherein R48 and R49 each independently represent hydrogen atom or a($C_1$ to $C_6$)alkyl group], or

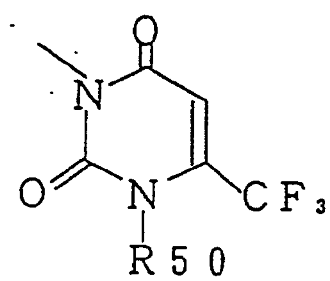

wherein R50 represents hydrogen atom, sodium atom or a($C_1$ to $C_4$)alkyl group;
(ii) when R8 and R9 form a ring,
the glutaric acid amide derivatives are represented by the formula (VIII), formula (IX), formula (X), formula (XI) or formula (XII):

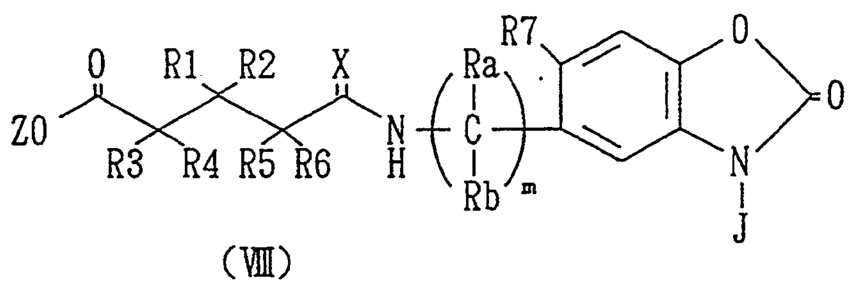

(VIII)

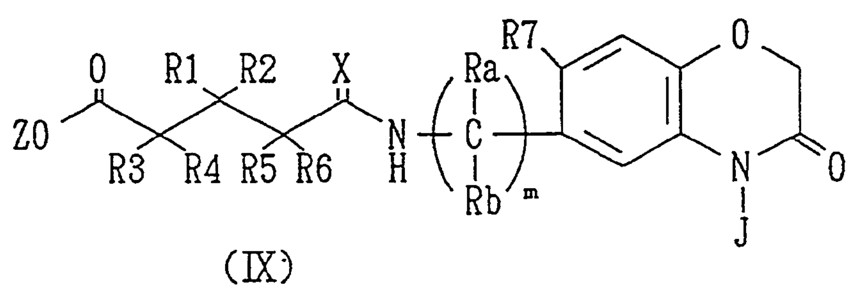

(IX)

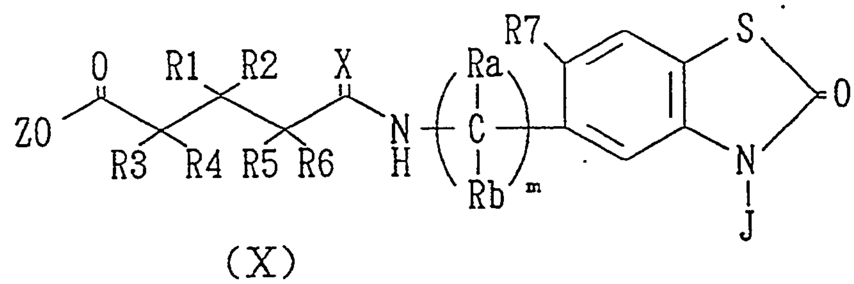

(X)

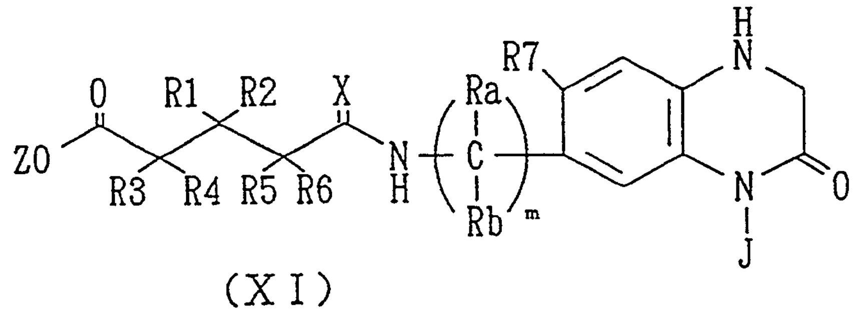

(XI)

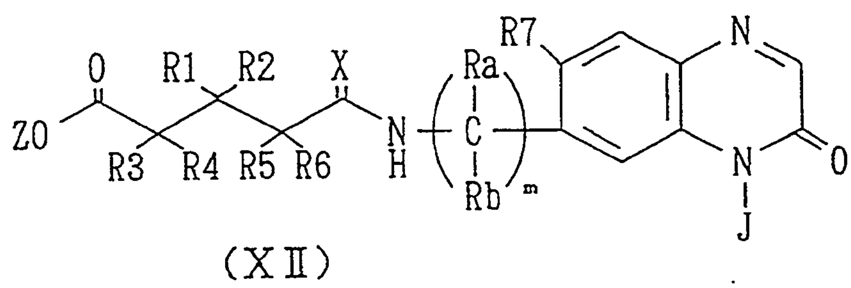

(XII)

wherein Ra, Rb, m, R1, R2, R3, R4, R5, R6, R7, X and Z are the same meanings as defined above;

J represents hydrogen atom, a($C_1$ to $C_6$)alkyl group, a($C_2$ to $C_6$)alkenyl group, a($C_3$ to $C_6$)alkynyl group, hydroxymethyl group, chloromethyl group, -$CH_2CONH_2$, cyanomethyl group, -$CH_2CO_2$($C_1$ to $C_4$)alkyl, a($C_3$ to $C_6$)cycloalkyl group, a($C_3$ to $C_6$)cycloalkyl($C_1$ to $C_2$)alkyl group, a($C_1$ to $C_2$)alkoxy($C_1$ to $C_2$)alkyl group, a phenyl group which may be substituted one or more substituent (the substituent is at least one selected from a($C_1$ to $C_6$)alkyl group, a($C_1$ to $C_6$)alkoxy group, a halogen atom, a($C_1$ to $C_6$)haloalkyl group, a($C_1$ to $C_6$) haloalkoxy group, a($C_3$ to $C_6$)cycloalkyl group, a($C_1$ to $C_4$)alkylamino group, a($C_1$ to $C_4$)alkoxycarbonyl group, a($C_2$ to $C_4$)alkylcarbonyl group, a($C_1$ to $C_4$)alkylsulfonyl group, a($C_2$ to $C_6$)alkenyl group and a($C_2$ to $C_6$)alkynyl group) or a benzyl group which may be substituted one or more substituent (the substituent is at least one selected from a($C_1$ to $C_6$)alkyl group, a($C_1$ to $C_6$)alkoxy group, a halogen atom, a($C_1$ to $C_6$)haloalkyl group, a($C_1$ to $C_6$)haloalkoxy group, a($C_3$ to $C_6$)cycloalkyl group, a($C_1$ to $C_4$)alkylamino group, a($C_1$ to $C_4$)alkoxycarbonyl group, a($C_2$ to $C_4$)alkylcarbonyl group, a($C_1$ to $C_4$)alkylsulfonyl group, a($C_2$ to $C_6$)alkenyl group and a($C_2$ to $C_6$)alkynyl group),

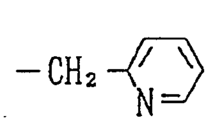 or 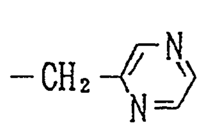 ;

provided that the glutaric acid amide derivatives represented by the formula (B) are excluded,

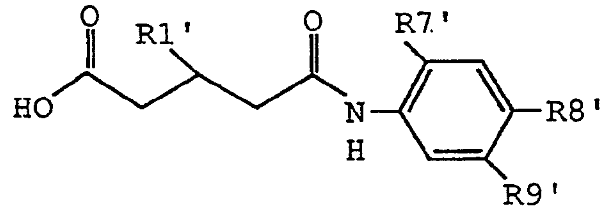

(B)

wherein R1′ represents hydrogen atom, a($C_1$ to $C_6$)alkyl group, a($C_3$ to $C_7$)cycloalkyl group, a($C_1$ to $C_6$)haloalkyl group, a($C_3$ to $C_6$)alkenyl group or a($C_3$ to $C_6$)alkynyl group;

R7′ represents hydrogen atom or a halogen atom;

R8′ represents hydrogen atom, cyano group, nitro group or a halogen atom;

R9′ represents hydrogen atom, cyano group, nitro group or hydroxyl group, -OR11 [wherein R11 is the same meaning as defined above], -O-CH(R12)$CO_2$R13 [wherein R12 and R13 are the same meanings as defined above], -$CO_2$R14 [wherein R14 is the same meaning as defined above], -C(R16′)=NOCH$_2$CO$_2$R17 [wherein R16′ represents hydrogen atom or a($C_1$ to $C_3$)alkyl group, and R17 is the same meaning as defined above], -SR23′ [wherein R23′ represents a($C_1$ to $C_6$)alkyl group, a($C_3$ to $C_6$)cycloalkyl group, a($C_2$ to $C_6$)alkenyl group, a($C_3$ to $C_6$)alkynyl group or cyanomethyl group], -SCH(R24)$CO_2$R25 [wherein R24 and R25 are the same meanings as defined above], or -$SCH_2(CH_2)_{n'}$-$CO_2$R25 [wherein n′ is an integer of 1 to 3 and R25 is the same meaning as defined above], and provided that when a compound of an optically active site, or a stereoisomer or geometric isomer is included in the glutaric acid amide derivative, all these forms are included.

3. A herbicide which comprises a glutarimide derivative as defined in Claim 1 or a glutaric acid amide derivative as defined in Claim 2 and a herbicidally acceptable carrier or diluent.

4. A method for killing weeds or inhibiting the growth of weeds at a locus, which comprises applying a herbicidally effective amount of a glutarimide derivative as defined in Claim 1 or a glutaric acid amide derivative as defined in Claim 2 thereto.

5. A method of preparing a glutarimide derivative as defined in claim 1, which comprises:
(i) reacting a compound of formula (a):

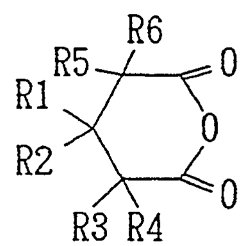

wherein R1, R2, R3, R4, R5 and R6 are as defined in claim 1 with a compound of formula (b):

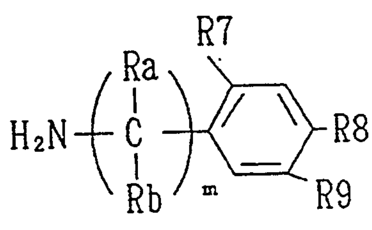

wherein R7, R8, R9, Ra, Rb and m are as defined in claim 1, to prepare a glutarimide derivative in which X as defined in claim 1 is oxygen and, if desired, converting the glutarimide derivative thus produced into a derivative in which X as defined in claim 1 is sulphur by means of a thiocarbonylation agent;
(ii) dehydrating and cyclizing a compound of formula (VII-i):

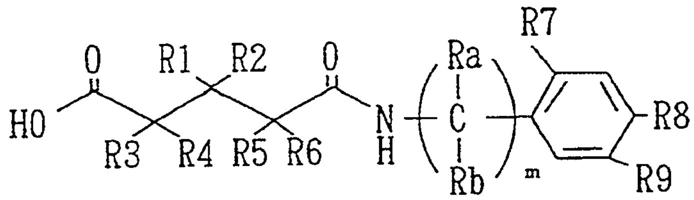

wherein R1, R2, R3, R4, R5, R6, R7, R8, R9, Ra, Rb and m are as defined in claim 1, to prepare a glutarimide derivative in which X as defined in claim 1 is oxygen and, if desired, converting the glutarimide derivative thus produced into a derivative in which X as defined in claim 1 is sulphur by means of a thiocarbonylation agent;

(iii) cyclizing a compound of formula (VII-2):

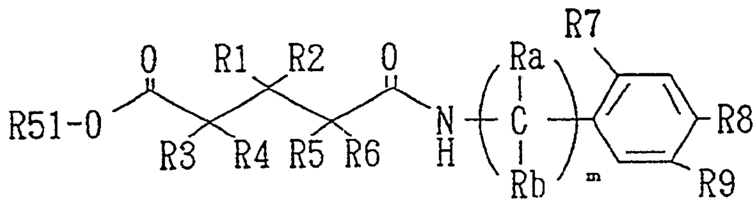

wherein R1, R2, R3, R4, R5, R6, R7, R8, R9, Ra, Rb and m are as defined in claim 1 and R51 is a $C_1$-$C_8$ alkyl group, phenyl group or benzyl group, to prepare a glutarimide derivative in which X as defined in claim 1 is oxygen and, if desired, converting the glutarimide derivative thus produced into a derivative in which X as defined in claim 1 is sulphur by means of a thiocarbonylation agent;

(iv) subjecting a compound of formula (d):

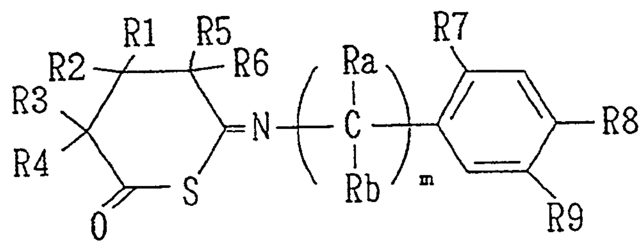

wherein R1, R2, R3, R4, R5, R6, R7, R8, R9, Ra, Rb and m are as defined in claim 1 to heat treatment or to treatment with an acid or base, to prepare a glutarimide derivative in which X as defined in claim 1 is sulphur.

**6.** A method of preparing a glutaric acid amide derivative as defined in claim 2, which comprises:

(i) reacting a compound of formula (a):

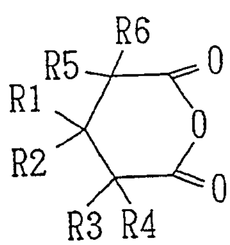

wherein R1, R2, R3, R4, R5 and R6 are as defined in claim 2 with a compound of formula (b):

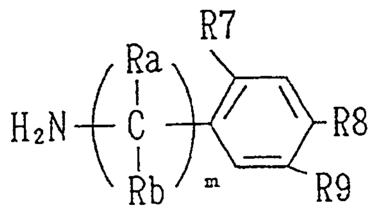

wherein R7, R8, R9, Ra, Rb and m are as defined in claim 2, to prepare a glutaric acid amide derivative as defined in claim 2 in which X is an oxygen atom and Z is a hydrogen atom and, if desired, converting the glutaric acid amide derivative thus produced into a derivative in which X is a sulphur atom by means of a thiocarbonylation agent; or

(ii) reacting a compound of formula (b) as defined above with a compound of formula (c):

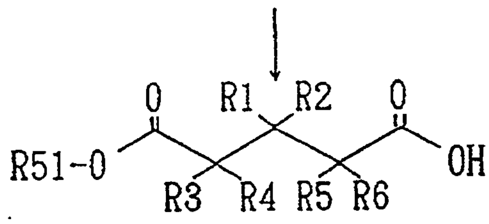

wherein R1, R2, R3, R4, R5 and R6 are as defined in claim 2 and R51 is a $C_1$-$C_6$ alkyl group, phenyl group or benzyl group, to prepare a glutaric acid amide derivative as defined in claim 2 in which X is an oxygen atom and Z is a $C_1$-$C_6$ alkyl group, phenyl group or benzyl group and, if desired, converting the glutaric acid amide derivative thus produced into a derivative in which X is a sulphur atom by means of a thiocarbonylation agent.

European Patent Office

# EUROPEAN SEARCH REPORT

Application Number

EP 91303698.4

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| X | DE - A - 1 445 652 (THE DIST. COMP.) * Claims 1,5; example 5 * -- | 1,2,5, 6 | C 07 D 211/88<br>C 07 D 221/20<br>C 07 D 265/36<br>C 07 D 271/107<br>C 07 D 273/02<br>C 07 D 401/10<br>C 07 D 413/04<br>C 07 D 413/10<br>C 07 C 233/15<br>C 07 C 233/13<br>C 07 C 233/43<br>C 07 C 233/54<br>C 07 C 239/54<br>A 01 N 43/40<br>A 01 N 37/30 |
| X | GB - A - 768 840 (CASS. FARBWERKE) * Claim 1; examples 1a,21,23 * --. | 1,2,5, 6 | |
| X | US - A - 2 673 205 (HOFFMANN) * Claim 1; examples 9,10 * -- | 1,2,5, 6 | |
| X | CHEMICAL ABSTRACTS, vol. 106, no. 11, March 16, 1987, Columbus, Ohio, USA KOMETANI T. et al. "A synthesis of succinimides and glutarimides from cyclic anhydrides" page 584, column 2, abstract-no. 84 339k & Tetrahedron Lett., 28(8), 919-22 (1986) -- | 1,2,5, 6 | TECHNICAL FIELDS SEARCHED (Int. Cl.5) |
| X | CHEMICAL ABSTRACTS, vol. 55, no. 20, October 2, 1961, Columbus, Ohio, USA NAKANO T. et al. "Structure of julocrotine" columns 19834,19835,abstract-no. 19 834c-19 835d & J. Org. Chem., 26, 1184-91 (1961) -- | 1 | C 07 D 211/00<br>C 07 D 221/00<br>C 07 D 265/00<br>C 07 D 271/00<br>C 07 D 273/00<br>C 07 D 401/00<br>C 07 D 413/00<br>C 07 C 233/00<br>C 07 C 239/00 |
| X | CHEMICAL ABSTRACTS, vol. 52, no. 1, January 10, 1958, Columbus, Ohio, USA MALLARD B.G. et al. "N-Allyl and N-aryl derivatives of 3-phenylglutarimides and | 1,5 | |

The present search report has been drawn up for all claims

| Place of search | Date of completion of the search | Examiner |
|---|---|---|
| VIENNA | 02-07-1991 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

-2-
EP 91303698.4

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| | 3-phenyl-2-methylglutarimides" column 317, abstract-no. 317b & J. Am. Pharm. Assoc., 46, 176-8 (1957) -- | | |
| X | CHEMICAL ABSTRACTS, vol. 49, no. 13, july 10, 1955, Columbus, Ohio, USA MALLARD B.G. et al. "N-Allyl and N-aryl-3,3-diethylglutari-mides" column 8815, abstract-no. 8 815 e & J. Am. Pharm. Assoc., 43, 246-7 (1954) -- | 1,5 | |
| X | CHEMICAL ABSTRACTS, vol. 31, no. 1, January 10, 1937, Columbus, Ohio, USA PADEN j.H. et al. " Synthesis of pyrrolidines, piperidines and hexahydroazepines" column 683, abstract-no. 683/2 & J. Am. Chem. Soc., 58, 2487-99 (1936) ---- | 1,5 | TECHNICAL FIELDS SEARCHED (Int. Cl.5) |

The present search report has been drawn up for all claims

| Place of search | Date of completion of the search | Examiner |
|---|---|---|
| VIENNA | 02-07-1991 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)